# EUROPEAN PATENT SPECIFICATION

(11) **EP 4 126 861 B1**
(45) Date of publication and mention of the grant of the patent: **18.06.2025**
(21) Application number: 21715855.9
(22) Date of filing: 25.03.2021
(51) Int. Cl.: C07D 471/04, A61K 31/437, A61K 31/444, A61K 31/5377, A61K 31/341, A61K 31/337, A61K 31/357, A61K 31/351, A61K 31/4545, A61P 35/00, A61P 35/02, A61P 35/04

(54) **3-(ANILINO)-2-[3-(3-ALKOXY-PYRIDIN-4-YL]-1,5,6,7-TETRAHYDRO-4H-PYRROLO[3,2-C]PYRIDIN-4-ONE DERIVATIVES AS EGFR INHIBITORS FOR THE TREATMENT OF CANCER**
3-(ANILINO)-2-[3-(3-ALKOXY-PYRIDIN-4-YL]-1,5,6,7-TETRAHYDRO-4H-PYRROLO[3,2-C]PYRIDIN-4-ON-DERIVATE ALS EGFR INHIBITOREN ZUR BEHANDLUNG VON KREBS
DÉRIVÉS DE 3-(ANILINO)-2-[3-(3-ALKOXY-PYRIDIN-4-YL]-1,5,6,7-TETRAHYDRO-4H-PYRROLO[3,2-C]PYRIDIN-4-ONE EN TANT QU'INHIBITEURS EGFR POUR LE TRAITEMENT DU CANCER

(30) Priority: 31.03.2020 US 202063002729 P
(43) Date of publication of application: 08.02.2023
(73) Proprietor: Bayer Aktiengesellschaft, 51373 Leverkusen (DE); The Broad Institute, Inc., Cambridge, MA 02142 (US); Dana-Farber Cancer Institute, Inc., Boston, Massachusetts 02215 (US)
(72) Inventor: SIEGEL, Stephan, 10779 Berlin (DE); SIEGEL, Franziska, Cambridge, MA 02139 (US); SCHULZE, Volker, 16562 Hohen Neuendorf, OT Bergfelde (DE); BERGER, Markus, 12167 Berlin (DE); MORTIER, Jeremie Xavier G., 12055 Berlin (DE); SÜLZLE, Detlev, 13465 Berlin (DE); BÖMER, Ulf, 16548 Glienicke/Nordbahn (DE); KORR, Daniel, 10247 Berlin (DE); SCHRÖDER, Jens, 10115 Berlin (DE); MÖNNING, Ursula, 15569 Woltersdorf (DE); NIEHUES, Michael, 12159 Berlin (DE); MEYERSON, Matthew, Boston, MA 02215 (US); GREULICH, Heidi, Cambridge, MA 02142 (US); KAPLAN, Bethany, Cambridge, MA 02142 (US)
(74) Representative: BIP Patents
(86) International application number: PCT/EP2021/057759
(87) International publication number: WO 2021/198020

(56) References cited:
- WO-A1-2019/081486

## Description

### Field of application of the invention

The invention relates to substituted 4H-pyrrolo[3,2-c]pyridin-4-one compounds, a process for their production and uses thereof.

### BACKGROUND OF THE INVENTION

The Epidermal Growth Factor Receptor (EGFR or EGF-receptor) receptor tyrosine kinase family consists of 4 members: EGFR (Erbb1, Her1), ERBB2 (Her2), ERBB3 (Her3), and ERBB4 (Her4). EGFR mediates activation of MAPK and PI3K signaling pathways and thereby regulates cell proliferation, differentiation, migration and survival (Pao et al., 2010). EGFR gene amplification, overexpression, and mutation are frequently observed in various cancer indications and are associated with a poor prognosis (Gridelli et al., 2015).

In lung adenocarcinoma, mutations of EGFR are prevalent in approximately 15% of Western patients and up to 50% of East Asian patients (Paez et al., 2004). These mutations typically occur in one of four exons, exons 18-21, in the kinase domain of EGFR (Paez et al., 2004). The most common activating mutations in EGFR are a point mutation in exon 21, substituting an arginine for a leucine (L858R), and a small in-frame deletion in exon 19 that removes four amino acids (del 19/del746-750) (Pao et al., 2010). The FDA-approved inhibitors gefitinib, erlotinib, and afatinib, targeting mutations in exons 18, 19, and 21 of EGFR, are effective in patients but the response is often not durable (Mok et al., 2009; Sequist et al., 2013). Resistance frequently occurs in these patients in response to acquisition of a second mutation, T790M (Pao et al., 2005). Second generation inhibitors, e.g. afatinib, irreversibly target this mutation but are still potent inhibitors of wild-type EGFR, leading to dose-limiting toxicity and lack of efficacy in patients. A third-generation irreversible inhibitor, osimertinib, that maximizes activity towards T790M while minimizing activity towards wild-type EGFR, is effective in T790M mutant patients and is currently the standard treatment for T790M positive patients (Mok et al., 2017). Osimertinib is also approved as a front-line therapy for patients with mutations of EGFR exons 19 or 21 (Soria et al., 2018).

However, patients also develop resistance to irreversible third-generation EGFR inhibitors, such as osimertinib. One of the major osimertinib resistance mechanisms identified is mutation of the cysteine in position 797 to a serine, resulting in loss of the covalently interacting cysteine and loss of sensitivity to irreversible EGFR inhibitors, at which point progressing patients have currently only limited treatment options (Thress et al., 2015; Oxnard et al., 2018). Such C797S mutations can also occur when osimertinib is used as a first-line therapy, in the absence of the T790M mutation (Ramalingham et al., 2018a; Ramalingham et al., 2018b). A novel targeted therapy that is able to specifically address the EGFR-C797S acquired resistance mutation would be highly beneficial for those patients.

By contrast, and with the exception of A763_Y764insFQEA, small in-frame insertions of EGFR exon20 are resistant to all clinically-approved EGFR inhibitors at doses achievable in lung cancer patients and comprise an unmet medical need (Yasuda et. al., 2013).

Patients with EGFR exon20 insertions, such as V769_D770insASV, D770_N771insSVD, D770_N771insNPG, N771_P772insH, H773_V774insH, H773_V774insNPH, V774_C775insHV show particular low response rates to all currently approved EGFR-targeted therapies, resulting in significantly reduced progression-free survival as well as overall survival (Chen et al., 2016). This has been shown for the first-generation inhibitors erlotinib and gefitinib as well as for the second-generation inhibitor afatinib (Chen et al., 2016; Yang et al., 2015).

Therefore, the standard treatment for EGFR exon20 insertion patients is currently chemotherapy.

The same resistance profile has been observed for exon20 insertion mutations in ERBB2 (e.g. ERBB2 A775_G776insYVMA with the highest prevalence), another member of the EGF-receptor family (Arcila et al., 2012) and some of the uncommon EGFR mutations like L681Q (Chiu et al., 2015).

Several irreversible inhibitors are currently in clinical trials for the treatment of EGFR exon20 insertion patients: Osimertinib, initially approved for the treatment of T790M mutant NSCLC patients (Floc'h et al., 2018); poziotinib (HM-781-36B), a non-approved pan-Her inhibitor targeting EGFR, Her2/neu, and Her4 (Robichaux et al., 2018); as well as TAK-788 (AP32788) (Doebele et al., ASCO 2018). Of these, the first clinical data have been published for poziotinib and TAK-788. Both compounds clearly show clinical efficacy in EGFR exon20 insertion patients. However, major adverse events, mediated by inhibition of wild-type EGFR, have been reported for both clinical trials and these adverse events may limit clinical utility.

More recently, new preclinical data has been published for two additional compounds showing activity on EGFR exon20 insertions: TAS6417 (TCP-064) and compound 1a (Hasako et al., 2018; Jang et al., 2018). No clinical results are yet available for these two compounds.

PCT patent application published as WO 2019/081486 A1 (Bayer AG) relates to substituted 4H-pyrrolo[3,2-c]pyridin-4-one compounds, a process for their production and uses thereof.

In summary, mutant EGFR is a promising drug target for cancer therapy. In particular, patients with primary resistance to approved anti-EGFR therapies, due to EGFR exon20 insertions, have only few treatment options to date and there is a great need for novel alternative and/or improved therapeutics to provide these patients with an efficacious, well-tolerable therapy (Oxnard et al., 2013). Therefore, potent inhibitors of mutant EGFR, particularly of mutant EGFR with exon20 insertion mutations that show improved selectivity versus wild-type EGFR, represent valuable compounds that should complement therapeutic options either as single agents or in combination with other drugs.

### SUMMARY OF THE INVENTION

The invention provides compounds that inhibit a mutant EGFR; specifically, an EGFR comprising one or more exon 20 insertion mutations, an L858R mutation, or a small in-frame deletion of exon 19, each of which may be in the presence or absence of a C797S mutation. These compounds furthermore have reduced activity towards the wild-type-EGFR.

The compounds of the present invention as defined in the claims herein differ from the compounds of WO 2019/081486 A1 in the proviso for the substituents R3 to R5, i.e. in that the 6- and/or the 7-position of the 1,5,6,7- tetrahydro-4H-pyrrolo[3,2-c]pyridin-4-one has to be substituted.

It has now been found that the compounds of the present invention have surprising and advantageous properties.

In particular, said compounds of the present invention have surprisingly been found to effectively inhibit mutant EGFR with exon 20 insertion mutations, particularly those harboring a D770_N771ins SVD exon 20 insertion with an IC₅₀ below 5 nM. Furthermore it has been found that these compounds additionally show cellular potency below 1 µM in EGFR V769_D770insASV, D770_N771insSVD, D770_N771insNPG, N771_P772insH, or H773_V774insNPH exon 20 insertion harboring BA/F3 cell lines. Furthermore, the here described compounds are active in BA/F3 cell lines harboring D770_N771insSVD C797S. In addition, the here described compounds potently inhibit proliferation of BA/F3 cell lines carrying EGFR activating mutations with or without C797S acquired resistance mutations (EGFR E746_A750del, L858R, E746_A750del C797S, L858R C797S), uncommon EGFR mutations (EGFR L681Q) or ERBB2 exon20 insertion A775_G776insYVMA.

Surprisingly these compounds additionally show at least 5 fold selectivity in an antiproliferative assay of EGFR D770_N771ins SVD exon 20 insertion harboring BA/F3 cell lines versus wild-type EGFR harboring BA/F3 cells and may therefore be used for the treatment or prophylaxis of diseases of uncontrolled cell growth, proliferation and/or survival, inappropriate cellular immune responses, or inappropriate cellular inflammatory responses or diseases which are accompanied with uncontrolled cell growth, proliferation and/or survival, inappropriate cellular immune responses, or inappropriate cellular inflammatory responses mediated by mutant EGFR with exon 20 insertion mutations and/or reduce (or block) proliferation in cells harboring EGFR exon 20 insertion mutations, for example, haematological tumours, solid tumours, and/or metastases thereof, *e.g.* leukaemias and myelodysplastic syndrome, malignant lymphomas, head and neck tumours including brain tumours and brain metastases, tumours of the thorax including non-small cell and small cell lung tumours, gastrointestinal tumours, endocrine tumours, mammary and other gynaecological tumours, urological tumours including renal, bladder and prostate tumours, skin tumours, and sarcomas, and/or metastases thereof.

### Description of the invention

In accordance with a first aspect, the invention relates to compounds of formula (I), 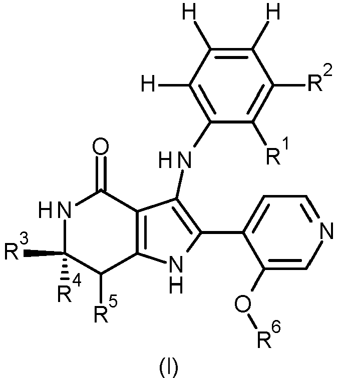 in which:
- R¹: represents methyl, ethyl, trifluoromethyl, 2,2-difluoroethyl, cyano, chloro, bromo, methoxy, or difluoromethoxy;
- R²: represents methyl, ethyl, fluoro, chloro, or bromo;
- R³: represents hydrogen, methyl, or trifluoromethyl;
- R⁴: represents hydrogen; or
- R³ and R⁴: together with the carbon atom to which they are attached form a 3- to 5-membered cycloalkyl ring;
- R⁵: represents hydrogen, C₁-C₃-alkyl, or C₂-C₃-alkenyl, wherein said alkyl groups are optionally substituted one or more times, independently of each other, with fluoro, hydroxy, or methoxy and said alkenyl groups are optionally substituted one or more times, with fluoro; or
- R³ and R⁵: together with the carbon atoms to which they are attached form a 4- to 6-membered cycloalkyl ring;

With the proviso that at least one of R³ and R⁵ is different from hydrogen;
- R⁶: represents C₂-C₅-alkyl, which is substituted once with hydroxy, C₁-C₄-alkoxy, or C₂-C₃-alkenyloxy,
or a group selected from the group: 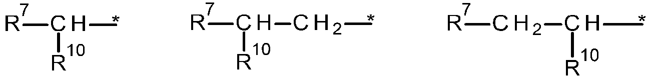
wherein * indicates the point of attachment of said group with the rest of the molecule;
- R⁷: represents a group selected from the group: 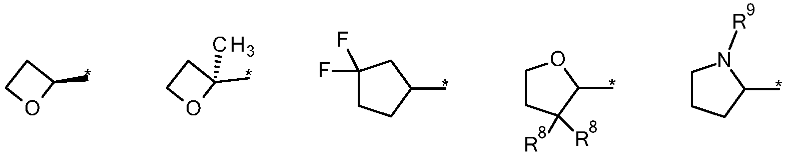 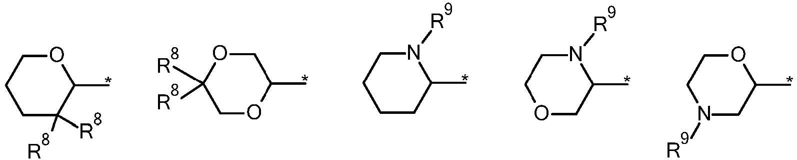 wherein * indicates the point of attachment of said group with the rest of the molecule;
- R⁸: independently represents hydrogen, methyl, or fluoro at each occurence;
- R⁹: represents C₁-C₃-alkyl or C₂-C₃-fluoroalkyl;
- R¹⁰: represents hydrogen or methyl;
or an N-oxide, a salt or a tautomer of said compound, or a salt of said N-oxide or tautomer.

In a second aspect, the invention relates to compounds of formula (I) as described *supra,* wherein:
- R¹: represents methyl, ethyl, trifluoromethyl, 2,2-difluoroethyl, methoxy, or difluoromethoxy;
- R²: represents methyl, fluoro, chloro, or bromo;
- R³: represents hydrogen, methyl, or trifluoromethyl;
- R⁴: represents hydrogen; or
- R³ and R⁴: together with the carbon atom to which they are attached form a 3- to 5-membered cycloalkyl ring;
- R⁵: represents hydrogen, C₁-C₃-alkyl, or C₂-C₃-alkenyl, wherein said alkyl groups are optionally substituted one, two, or three times, independently of each other, with fluoro, hydroxy, or methoxy and said alkenyl groups are optionally substituted one, two, or three times, with fluoro; or
- R³ and R⁵: together with the carbon atoms to which they are attached form a 5- to 6-membered cycloalkyl ring;

With the proviso that at least one of R³ and R⁵ is different from hydrogen;
- R⁶: represents C₂-C₅-alkyl, which is substituted once with hydroxy, C₁-C₃-alkoxy, or C₂-C₃-alkenyloxy,
or a group selected from the group: 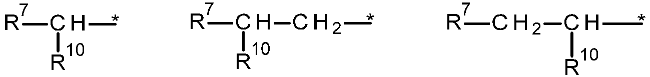
wherein * indicates the point of attachment of said group with the rest of the molecule;
- R⁷: represents a group selected from the group: 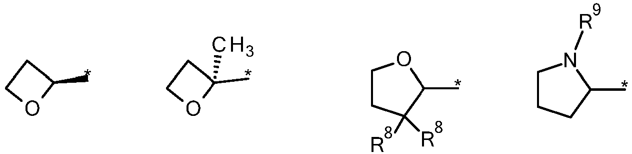 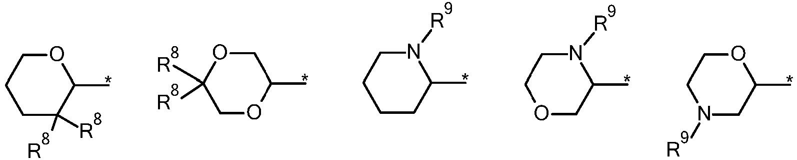 wherein * indicates the point of attachment of said group with the rest of the molecule;
- R³: represents hydrogen, methyl, or fluoro;
- R⁹: represents C₁-C₃-alkyl or C₂-C₃-fluoroalkyl;
- R¹⁰: represents hydrogen or methyl;
or an N-oxide, a salt or a tautomer of said compound, or a salt of said N-oxide or tautomer.

In a third aspect, the invention relates to compounds of formula (I) as described *supra,* wherein:
- R¹: represents methyl, ethyl, 2,2-difluoroethyl, or methoxy;
- R²: represents fluoro or chloro;
- R³: represents hydrogen, methyl, or trifluoromethyl;
- R⁴: represents hydrogen; or
- R³ and R⁴: together with the carbon atom to which they are attached form a 3- to 4-membered cycloalkyl ring;
- R⁵: represents hydrogen, C₁-C₃-alkyl, prop-2-en-1-yl or 3,3-difluoroprop-2-en-1-yl, wherein said alkyl groups are optionally substituted one, two, or three times with fluoro or once with hydroxy; or
- R³ and R⁵: together with the carbon atoms to which they are attached form a 5-membered cycloalkyl ring;

With the proviso that at least one of R³ and R⁵ is different from hydrogen;
- R⁶: represents C₂-C₄-alkyl, which is substituted once with hydroxy, methoxy, or (prop-2-en-1-yl)oxy,
or a group selected from the group: 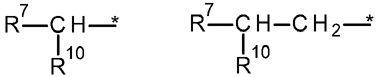
wherein * indicates the point of attachment of said group with the rest of the molecule;
- R⁷: represents a group selected from the group: 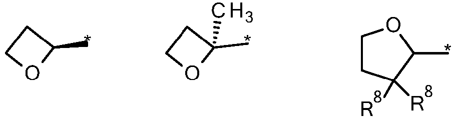 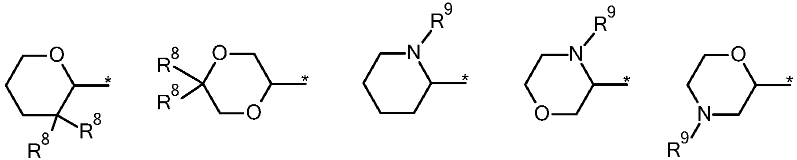 wherein * indicates the point of attachment of said group with the rest of the molecule;
- R⁸: represents hydrogen, methyl, or fluoro;
- R⁹: represents methyl or 2,2 difluoroethyl;
- R¹⁰: represents hydrogen;
or an N-oxide, a salt or a tautomer of said compound, or a salt of said N-oxide or tautomer.

In a fourth aspect, the invention relates to compounds of formula (I) as described *supra,* which is selected from the group consisting of:
(6*R*)-3-(3-chloro-2-methoxyanilino)-2-[3-(2-methoxy-2-methylpropoxy)pyridin-4-yl]-6-methyl-1,5,6,7-tetrahydro-4H-pyrrolo[3,2-c]pyridin-4-one
3-(3-chloro-2-methoxyanilino)-6-methyl-2-(3-{[(2*S*)-oxolan-2-yl]methoxy}pyridin-4-yl)-1,5,6,7-tetrahydro-4*H*-pyrrolo[3,2-c]pyridin-4-one
(6*R*)-3-(3-chloro-2-methoxyanilino)-6-methyl-2-(3-{[(2S)-oxolan-2-yl]methoxy}pyridin-4-yl)-1,5,6,7-tetrahydro-4H-pyrrolo[3,2-c]pyridin-4-one
(6*R*)-3-(3-chloro-2-methoxyanilino)-6-methyl-2-(3-{[(2*S*)-oxetan-2-yl]methoxy}pyridin-4-yl)-1,5,6,7-tetrahydro-4H-pyrrolo[3,2-c]pyridin-4-one
3-(3-fluoro-2-methoxyanilino)-6-methyl-2-(3-{[(2*S*)-oxolan-2-yl]methoxy}pyridin-4-yl)-1,5,6,7-tetrahydro-4*H*-pyrrolo[3,2-c]pyridin-4-one
(6*R*)-3-(3-fluoro-2-methoxyanilino)-6-methyl-2-(3-{[(2*S*)-oxolan-2-yl]methoxy}pyridin-4-yl)-1,5,6,7-tetrahydro-4*H*-pyrrolo[3,2-c]pyridin-4-one
(6*R*)-3-(3-chloro-2-methoxyanilino)-6-methyl-2-(3-{[(2*S*)-4-methylmorpholin-2-yl]methoxy}pyridin-4-yl)-1,5,6,7-tetrahydro-4*H*-pyrrolo[3,2-c]pyridin-4-one
(6*R*)-3-(3-chloro-2-methoxyanilino)-2-(3-{[(2*S*)-4-(2,2-difluoroethyl)morpholin-2-yl]methoxy}pyridin-4-yl)-6-methyl-1,5,6,7-tetrahydro-4*H*-pyrrolo[3,2-c]pyridin-4-one
3-(3-chloro-2-methoxyanilino)-7-(2-hydroxyethyl)-2-[3-(2-methoxy-2-methylpropoxy)pyridin-4-yl]-1,5,6,7-tetrahydro-4H-pyrrolo[3,2-c]pyridin-4-one
(7*R*)-3-(3-chloro-2-methoxyanilino)-7-(2-hydroxyethyl)-2-[3-(2-methoxy-2-methylpropoxy)pyridin-4-yl]-1,5,6,7-tetrahydro-4*H*-pyrrolo[3,2-c]pyridin-4-one
(7*S*)-3-(3-chloro-2-methoxyanilino)-7-(2-hydroxyethyl)-2-[3-(2-methoxy-2-methylpropoxy)pyridin-4-yl]-1,5,6,7-tetrahydro-4*H*-pyrrolo[3,2-c]pyridin-4-one
3-(3-chloro-2-methoxyanilino)-7-(2-hydroxyethyl)-2-(3-{[(2*S*)-oxolan-2-yl]methoxy}pyridin-4-yl)-1,5,6,7-tetrahydro-4*H*-pyrrolo[3,2-c]pyridin-4-one
(7*R*)-3-(3-chloro-2-methoxyanilino)-7-(2-hydroxyethyl)-2-(3-{[(2*S*)-oxolan-2-yl]methoxy}pyridin-4-yl)-1,5,6,7-tetrahydro-4*H*-pyrrolo[3,2-c]pyridin-4-one
(7*S*)-3-(3-chloro-2-methoxyanilino)-7-(2-hydroxyethyl)-2-(3-{[(2*S*)-oxolan-2-yl]methoxy}pyridin-4-yl)-1,5,6,7-tetrahydro-4*H*-pyrrolo[3,2-c]pyridin-4-one
3-(3-chloro-2-methoxyanilino)-7-(2-hydroxyethyl)-2-{3-[(2-methyloxetan-2-yl)methoxy]pyridin-4-yl}-1,5,6,7-tetrahydro-4*H*-pyrrolo[3,2-c]pyridin-4-one
(7*S*)-3-(3-chloro-2-methoxyanilino)-7-(2-hydroxyethyl)-2-(3-{[(2*S*)-2-methyloxetan-2-yl]methoxy}pyridin-4-yl)-1,5,6,7-tetrahydro-4*H*-pyrrolo[3,2-c]pyridin-4-one
(7*R*)-3-(3-chloro-2-methoxyanilino)-7-(2-hydroxyethyl)-2-(3-{[(2*S*)-2-methyloxetan-2-yl]methoxy}pyridin-4-yl)-1,5,6,7-tetrahydro-4*H*-pyrrolo[3,2-c]pyridin-4-one
(7*S*)-3-(3-chloro-2-methoxyanilino)-7-(2-hydroxyethyl)-2-(3-{[(2*R*)-2-methyloxetan-2-yl]methoxy}pyridin-4-yl)-1,5,6,7-tetrahydro-4*H*-pyrrolo[3,2-c]pyridin-4-one
(7*R*)-3-(3-chloro-2-methoxyanilino)-7-(2-hydroxyethyl)-2-(3-{[(2*R*)-2-methyloxetan-2-yl]methoxy}pyridin-4-yl)-1,5,6,7-tetrahydro-4*H*-pyrrolo[3,2-c]pyridin-4-one,
(6*R*)-3-(3-chloro-2-methoxy-anilino)-2-[3-(1,4-dioxan-2-ylmethoxy)-4-pyridyl]-6-methyl-1,5,6,7-tetrahydropyrrolo[3,2-c]pyridin-4-one
(6*R*)-3-(3-chloro-2-methoxy-anilino)-2-{3-[(2*S*)-1,4-dioxan-2-ylmethoxy]-4-pyridyl}-6-methyl-1,5,6,7-tetrahydropyrrolo[3,2-c]pyridin-4-one,
(6*R*)-3-(3-chloro-2-methoxy-anilino)-2-{3-[(2*R*)-1,4-dioxan-2-ylmethoxy]-4-pyridyl}-6-methyl-1,5,6,7-tetrahydropyrrolo[3,2-c]pyridin-4-one,
(6*R*)-3-(3-chloro-2-methoxy-anilino)-6-methyl-2-[3-(tetrahydropyran-2-ylmethoxy)-4-pyridyl]-1,5,6,7-tetrahydropyrrolo[3,2-c]pyridin-4-one
(6*R*)-3-(3-chloro-2-methoxy-anilino)-6-methyl-2-{3-[(2*S*)-tetrahydropyran-2-ylmethoxy]-4-pyridyl}-1,5,6,7-tetrahydropyrrolo[3,2-c]pyridin-4-one
(6*R*)-3-(3-chloro-2-methoxy-anilino)-6-methyl-2-{3-[(2*R*)-tetrahydropyran-2-ylmethoxy]-4-pyridyl}-1,5,6,7-tetrahydropyrrolo[3,2-c]pyridin-4-one
3-(3-chloro-2-methoxy-anilino)-7-methyl-2-[3-[[(2*S*)-tetrahydrofuran-2-yl]methoxy]-4-pyridyl]-1,5,6,7-tetrahydropyrrolo[3,2-c]pyridin-4-one
(7*S*)-3-(3-chloro-2-methoxy-anilino)-7-methyl-2-[3-[[(2*S*)-tetrahydrofuran-2-yl]methoxy]-4-pyridyl]-1,5,6,7-tetrahydropyrrolo[3,2-c]pyridin-4-one
(7*R*)-3-(3-chloro-2-methoxy-anilino)-7-methyl-2-[3-[[(2*S*)-tetrahydrofuran-2-yl]methoxy]-4-pyridyl]-1,5,6,7-tetrahydropyrrolo[3,2-c]pyridin-4-one
3-(3-chloro-2-methoxy-anilino)-2-[3-(1,4-dioxan-2-ylmethoxy)-4-pyridyl]-7-methyl-1,5,6,7-tetrahydropyrrolo[3,2-c]pyridin-4-one
(7*S*)-3-(3-chloro-2-methoxy-anilino)-2-{3-[(2*S*)-1,4-dioxan-2-ylmethoxy]-4-pyridyl}-7-methyl-1,5,6,7-tetrahydropyrrolo[3,2-c]pyridin-4-one
(7*S*)-3-(3-chloro-2-methoxy-anilino)-2-{3-[(2*R*)-1,4-dioxan-2-ylmethoxy]-4-pyridyl}-7-methyl-1,5,6,7-tetrahydropyrrolo[3,2-c]pyridin-4-one,
(7*R*)-3-(3-chloro-2-methoxy-anilino)-2-{3-[(2*S*)-1,4-dioxan-2-ylmethoxy]-4-pyridyl}-7-methyl-1,5,6,7-tetrahydropyrrolo[3,2-c]pyridin-4-one
(7*R*)-3-(3-chloro-2-methoxy-anilino)-2-{3-[(2*R*)-1,4-dioxan-2-ylmethoxy]-4-pyridyl}-7-methyl-1,5,6,7-tetrahydropyrrolo[3,2-c]pyridin-4-one
3-(3-chloro-2-methoxy-anilino)-2-[3-[[(2*S*)-tetrahydrofuran-2-yl]methoxy]-4-pyridyl]-6-(trifluoromethyl)-1,5,6,7-tetrahydropyrrolo[3,2-c]pyridin-4-one
(6*S*)-3-(3-chloro-2-methoxy-anilino)-2-[3-[[(2*S*)-tetrahydrofuran-2-yl]methoxy]-4-pyridyl]-6-(trifluoromethyl)-1,5,6,7-tetrahydropyrrolo[3,2-c]pyridin-4-one
(6*R*)-3-(3-chloro-2-methoxy-anilino)-2-{3-[(1-methoxypropan-2-yl)oxy]-4-pyridyl}-6-methyl-1,5,6,7-tetrahydropyrrolo[3,2-c]pyridin-4-one
(6*R*)-3-(3-chloro-2-methoxy-anilino)-2-(3-{[(2*S*)-1-methoxypropan-2-yl]oxy}-4-pyridyl)-6-methyl-1,5,6,7-tetrahydropyrrolo[3,2-c]pyridin-4-one
(6*R*)-3-(3-chloro-2-methoxy-anilino)-2-(3-{[(2*R*)-1-methoxypropan-2-yl]oxy}-4-pyridyl)-6-methyl-1,5,6,7-tetrahydropyrrolo[3,2-c]pyridin-4-one,
3-(3-chloro-2-methoxy-anilino)-7-methyl-2-[3-(tetrahydropyran-2-ylmethoxy)-4-pyridyl]-1,5,6,7-tetrahydropyrrolo[3,2-c]pyridin-4-one
(7*S*)-3-(3-chloro-2-methoxy-anilino)-7-methyl-2-{3-[(2*S*)-tetrahydropyran-2-ylmethoxy]-4-pyridyl}-1,5,6,7-tetrahydropyrrolo[3,2-c]pyridin-4-one
(7*S*)-3-(3-chloro-2-methoxy-anilino)-7-methyl-2-{3-[(2*R*)-tetrahydropyran-2-ylmethoxy]-4-pyridyl}-1,5,6,7-tetrahydropyrrolo[3,2-c]pyridin-4-one
(7*R*)-3-(3-chloro-2-methoxy-anilino)-7-methyl-2-{3-[(2*S*)-tetrahydropyran-2-ylmethoxy]-4-pyridyl}-1,5,6,7-tetrahydropyrrolo[3,2-c]pyridin-4-one
(7*R*)-3-(3-chloro-2-methoxy-anilino)-7-methyl-2-{3-[(2*R*)-tetrahydropyran-2-ylmethoxy]-4-pyridyl}-1,5,6,7-tetrahydropyrrolo[3,2-c]pyridin-4-one
(5a*R*,8a*S*)-3-(3-chloro-2-methoxyanilino)-2-{3-[(1-methoxypropan-2-yl)oxy]-4-pyridyl}-5,5a,6,7,8,8a-hexahydrocyclopenta[b]pyrrolo[2,3-d]pyridin-4(1*H*)-one
(5a*R*,8a*S*)-3-(3-chloro-2-methoxyanilino)-2-(3-{[(2*S*)-1-methoxypropan-2-yl]oxy}-4-pyridyl)-5,5a,6,7,8,8a-hexahydrocyclopenta[b]pyrrolo[2,3-d]pyridin-4(1*H*)-one
(5a*R*,8a*S*)-3-(3-chloro-2-methoxyanilino)-2-(3-{[(2*R*)-1-methoxypropan-2-yl]oxy}-4-pyridyl)-5,5a,6,7,8,8a-hexahydrocyclopenta[b]pyrrolo[2,3-d]pyridin-4(1*H*)-one
(5a*R*,8a*S*)-3-(3-chloro-2-methoxyanilino)-2-[3-(1,4-dioxan-2-ylmethoxy)-4-pyridyl]-5,5a,6,7,8,8a-hexahydrocyclopenta[b]pyrrolo[2,3-d]pyridin-4(1*H*)-one
(5a*R*,8a*S*)-3-(3-chloro-2-methoxyanilino)-2-{3-[(2*S*)-1,4-dioxan-2-ylmethoxy]-4-pyridyl}-5,5a,6,7,8,8a-hexahydrocyclopenta[b]pyrrolo[2,3-d]pyridin-4(1*H*)-one,
(5a*R*,8a*S*)-3-(3-chloro-2-methoxyanilino)-2-{3-[(2*R*)-1,4-dioxan-2-ylmethoxy]-4-pyridyl}-5,5a,6,7,8,8a-hexahydrocyclopenta[b]pyrrolo[2,3-d]pyridin-4(1*H*)-one,
(5a*R**,8a*S**)-3-(3-chloro-2-methoxyanilino)-2-(3-{[(2*S*)-oxolan-2-yl]methoxy}-4-pyridyl)-5,5a,6,7,8,8a-hexahydrocyclopenta[b]pyrrolo[2,3-d]pyridin-4(1H)-one
(5a*R*,8a*S*)-3-(3-chloro-2-methoxyanilino)-2-(3-{[(2*S*)-oxolan-2-yl]methoxy}-4-pyridyl)-5,5a,6,7,8,8a-hexahydrocyclopenta[b]pyrrolo[2,3-d]pyridin-4(1H)-one
3-(3-chloro-2-methyl-anilino)-7-methyl-2-(3-{[(2*S*)-oxolan-2-yl]methoxy}pyridin-4-yl)-1,5,6,7-tetrahydropyrrolo[3,2-c]pyridin-4-one
(7*S*)-3-(3-chloro-2-methyl-anilino)-7-methyl-2-(3-{[(2S)-oxolan-2-yl]methoxy}pyridin-4-yl)-1,5,6,7-tetrahydropyrrolo[3,2-c]pyridin-4-one
(7*R*)-3-(3-chloro-2-methyl-anilino)-7-methyl-2-(3-{[(2*S*)-oxolan-2-yl]methoxy}pyridin-4-yl)-1,5,6,7-tetrahydropyrrolo[3,2-c]pyridin-4-one
3-(3-chloro-2-methyl-anilino)-2-[3-[(3,3-difluorotetrahydropyran-2-yl)methoxy]-4-pyridyl]-7-methyl-1,5,6,7-tetrahydropyrrolo[3,2-c]pyridin-4-one
3-(3-chloro-2-methyl-anilino)-2-[3-[(5,5-dimethyl-1,4-dioxan-2-yl)methoxy]-4-pyridyl]-7-methyl-1,5,6,7-tetrahydropyrrolo[3,2-c]pyridin-4-one
(6*R*)-3-[2-(2,2-difluoroethyl)-3-fluoroanilino]-6-methyl-2-(3-{[(2*S*)-oxolan-2-yl]methoxy}pyridin-4-yl)-1,5,6,7-tetrahydro-4*H*-pyrrolo[3,2-c]pyridin-4-one
(5a*R*,8a*S*)-3-(3-chloro-2-methoxyanilino)-2-(3-{[(2*S*)-4-methylmorpholin-2-yl]methoxy}pyridin-4-yl)-5,5a,6,7,8,8a-hexahydrocyclopenta[b]pyrrolo[2,3-d]pyridin-4(1*H*)-one
(6*R*)-3-[2-(2,2-difluoroethyl)-3-fluoroanilino]-2-[3-(2-methoxy-2-methylpropoxy)pyridin-4-yl]-6-methyl-1,5,6,7-tetrahydro-4*H*-pyrrolo[3,2-c]pyridin-4-one
3-(3-chloro-2-methyl-anilino)-2-[3-(1,4-dioxan-2-ylmethoxy)-4-pyridyl]-7-methyl-1,5,6,7-tetrahydropyrrolo[3,2-c]pyridin-4-one
(7*S*)-3-(3-chloro-2-methyl-anilino)-2-[3-[(2*S*)-1,4-dioxan-2-ylmethoxy]-4-pyridyl]-7-methyl-1,5,6,7-tetrahydropyrrolo[3,2-c]pyridin-4-one
(7*R*)-3-(3-chloro-2-methyl-anilino)-2-[3-[(2*R*)-1,4-dioxan-2-ylmethoxy]-4-pyridyl]-7-methyl-1,5,6,7-tetrahydropyrrolo[3,2-c]pyridin-4-one
(7*S*)-3-(3-chloro-2-methyl-anilino)-2-[3-[(2*R*)-1,4-dioxan-2-ylmethoxy]-4-pyridyl]-7-methyl-1,5,6,7-tetrahydropyrrolo[3,2-c]pyridin-4-one
(7*R*)-3-(3-chloro-2-methyl-anilino)-2-[3-[(2*S*)-1,4-dioxan-2-ylmethoxy]-4-pyridyl]-7-methyl-1,5,6,7-tetrahydropyrrolo[3,2-c]pyridin-4-one
(6*R*)-3-(3-chloro-2-methoxy-anilino)-2-[3-[(3,3-difluorotetrahydrofuran-2-yl)methoxy]-4-pyridyl]-6-methyl-1,5,6,7-tetrahydropyrrolo[3,2-c]pyridin-4-one
(6*R*)-3-(3-chloro-2-methoxy-anilino)-2-(3-{[(2*S*)-3,3-difluorotetrahydrofuran-2-yl]methoxy}-4-pyridyl)-6-methyl-1,5,6,7-tetrahydropyrrolo[3,2-c]pyridin-4-one
(6*R*)-3-(3-chloro-2-methoxy-anilino)-2-(3-{[(2*R*)-3,3-difluorotetrahydrofuran-2-yl]methoxy}-4-pyridyl)-6-methyl-1,5,6,7-tetrahydropyrrolo[3,2-c]pyridin-4-one
(6*R*)-3-(3-chloro-2-methoxy-anilino)-2-[3-[(3,3-difluorotetrahydropyran-2-yl)methoxy]-4-pyridyl]-6-methyl-1,5,6,7-tetrahydropyrrolo[3,2-c]pyridin-4-one
(6*R*)-3-(3-chloro-2-methoxy-anilino)-2-(3-{[(2*S*)-3,3-difluorotetrahydropyran-2-yl]methoxy}-4-pyridyl)-6-methyl-1,5,6,7-tetrahydropyrrolo[3,2-c]pyridin-4-one
(6*R*)-3-(3-chloro-2-methoxy-anilino)-2-(3-{[(2*R*)-3,3-difluorotetrahydropyran-2-yl]methoxy}-4-pyridyl)-6-methyl-1,5,6,7-tetrahydropyrrolo[3,2-c]pyridin-4-one
(6*R*)-3-(3-chloro-2-methoxy-anilino)-2-[3-[(5,5-dimethyl-1,4-dioxan-2-yl)methoxy]-4-pyridyl]-6-methyl-1,5,6,7-tetrahydropyrrolo[3,2-c]pyridin-4-one
(6*R*)-3-(3-chloro-2-methoxy-anilino)-2(3-{[(2*S*)-5,5-dimethyl-1,4-dioxan-2-yl]methoxy}-4-pyridyl)-6-methyl-1,5,6,7-tetrahydropyrrolo[3,2-c]pyridin-4-one
(6*R*)-3-(3-chloro-2-methoxy-anilino)-2(3-{[(2*R*)-5,5-dimethyl-1,4-dioxan-2-yl]methoxy}-4-pyridyl)-6-methyl-1,5,6,7-tetrahydropyrrolo[3,2-c]pyridin-4-one
(6*R*)-3-(3-chloro-2-methoxy-anilino)-2-[3-(2-methoxypropoxy)-4-pyridyl]-6-methyl-1,5,6,7-tetrahydropyrrolo[3,2-c]pyridin-4-one
(6*R*)-3-(3-chloro-2-methoxy-anilino)-2-{3-[(2*R*)-2-methoxypropoxy]-4-pyridyl}-6-methyl-1,5,6,7-tetrahydropyrrolo[3,2-c]pyridin-4-one
(6*R*)-3-(3-chloro-2-methoxy-anilino)-2-{3-[(2*S*)-2-methoxypropoxy]-4-pyridyl}-6-methyl-1,5,6,7-tetrahydropyrrolo[3,2-c]pyridin-4-one
(6*R*)-3-(3-chloro-2-methoxy-anilino)-6-methyl-2-[3-[(1-methyl-2-piperidyl)methoxy]-4-pyridyl]-1,5,6,7-tetrahydropyrrolo[3,2-c]pyridin-4-one
(6*R*)-3-(3-chloro-2-methoxy-anilino)-6-methyl-2-(3-{[(2*S*)-1-methyl-2-piperidyl]methoxy}-4-pyridyl)-1,5,6,7-tetrahydropyrrolo[3,2-c]pyridin-4-one
(6*R*)-3-(3-chloro-2-methoxy-anilino)-6-methyl-2-(3-{[(2*R*)-1-methyl-2-piperidyl]methoxy}-4-pyridyl)-1,5,6,7-tetrahydropyrrolo[3,2-c]pyridin-4-one
(6*R*)-3-(3-chloro-2-methoxy-anilino)-6-methyl-2-[3-(2-tetrahydropyran-2-ylethoxy)-4-pyridyl]-1,5,6,7-tetrahydropyrrolo[3,2-c]pyridin-4-one
(6*R*)-3-(3-chloro-2-methoxy-anilino)-6-methyl-2-(3-{2-[(2*S*)-tetrahydropyran-2-yl]ethoxy}-4-pyridyl)-1,5,6,7-tetrahydropyrrolo[3,2-c]pyridin-4-one
(6*R*)-3-(3-chloro-2-methoxy-anilino)-6-methyl-2-(3-{2-[(2*R*)-tetrahydropyran-2-yl]ethoxy}-4-pyridyl)-1,5,6,7-tetrahydropyrrolo[3,2-c]pyridin-4-one
(7*S*)-3-(3-chloro-2-methoxyanilino)-7-(2-fluoroethyl)-2-(3-{[(3*S*)-4-methylmorpholin-3-yl]methoxy}pyridin-4-yl)-1,5,6,7-tetrahydro-4*H*-pyrrolo[3,2-c]pyridin-4-one
(7*R*)-3-(3-chloro-2-methoxyanilino)-7-(2-fluoroethyl)-2-(3-{[(3*S*)-4-methylmorpholin-3-yl]methoxy}pyridin-4-yl)-1,5,6,7-tetrahydro-4*H*-pyrrolo[3,2-c]pyridin-4-one
(6*R*)-3-(3-chloro-2-ethylanilino)-6-methyl-2-(3-{[(2*S*)-4-methylmorpholin-2-yl]methoxy}pyridin-4-yl)-1,5,6,7-tetrahydro-4*H*-pyrrolo[3,2-c]pyridin-4-one
(6*R*)-3-(3-chloro-2-methoxyanilino)-6-methyl-2-(3-{2-[(2*S*)-oxolan-2-yl]ethoxy}pyridin-4-yl)-1,5,6,7-tetrahydro-4*H*-pyrrolo[3,2-c]pyridin-4-one
(6*R*)-3-(3-chloro-2-methoxyanilino)-6-methyl-2-(3-{[(3*S*)-4-methylmorpholin-3-yl]methoxy}pyridin-4-yl)-1,5,6,7-tetrahydro-4*H*-pyrrolo[3,2-c]pyridin-4-one
(7*S*)-3-(3-chloro-2-methoxy-anilino)-2-(3-{[(2*S*)-5,5-dimethyl-1,4-dioxan-2-yl]methoxy}-4-pyridyl)-7-methyl-1,5,6,7-tetrahydropyrrolo[3,2-c]pyridin-4-one
(7*S*)-3-(3-chloro-2-methoxy-anilino)-2-(3-{[(2*R*)-5,5-dimethyl-1,4-dioxan-2-yl]methoxy}-4-pyridyl)-7-methyl-1,5,6,7-tetrahydropyrrolo[3,2-c]pyridin-4-one
(7*R*)-3-(3-chloro-2-methoxy-anilino)-2-(3-{[(2*S*)-5,5-dimethyl-1,4-dioxan-2-yl]methoxy}-4-pyridyl)-7-methyl-1,5,6,7-tetrahydropyrrolo[3,2-c]pyridin-4-one
(7*R*)-3-(3-chloro-2-methoxy-anilino)-2-(3-{[(2*R*)-5,5-dimethyl-1,4-dioxan-2-yl]methoxy}-4-pyridyl)-7-methyl-1,5,6,7-tetrahydropyrrolo[3,2-c]pyridin-4-one
(7*S*)-3-(3-chloro-2-methoxy-anilino)-2-(3-{[(2*S*)-tetrahydrofuran-2-yl]methoxy}-4-pyridyl)-7-(3,3,3-trifluoropropyl)-1,5,6,7-tetrahydropyrrolo[3,2-c]pyridin-4-one
(7*R*)-3-(3-chloro-2-methoxy-anilino)-2-(3-{[(2*S*)-tetrahydrofuran-2-yl]methoxy}-4-pyridyl)-7-(3,3,3-trifluoropropyl)-1,5,6,7-tetrahydropyrrolo[3,2-c]pyridin-4-one
(7*S*)-3-(3-chloro-2-methoxy-anilino)-7-(3,3-difluoroallyl)-2-(3-{[(2*S*)-tetrahydrofuran-2-yl]methoxy}-4-pyridyl)-1,5,6,7-tetrahydropyrrolo[3,2-c]pyridin-4-one
(7*R*)-3-(3-chloro-2-methoxy-anilino)-7-(3,3-difluoroallyl)-2-(3-{[(2*S*)-tetrahydrofuran-2-yl]methoxy}-4-pyridyl)-1,5,6,7-tetrahydropyrrolo[3,2-c]pyridin-4-one
(6*R*)-3-(3-chloro-2-methylanilino)-2-{3-[(1,4-dioxan-2-yl)methoxy]pyridin-4-yl}-6-methyl-1,5,6,7-tetrahydro-4*H*-pyrrolo[3,2-c]pyridin-4-one
(6*R*)-3-(3-chloro-2-methylanilino)-2-(3-{[(2*S*)-1,4-dioxan-2-yl]methoxy}pyridin-4-yl)-6-methyl-1,5,6,7-tetrahydro-4*H*-pyrrolo[3,2-c]pyridin-4-one
(6*R*)-3-(3-chloro-2-methylanilino)-6-methyl-2-(3-{[(2*S*)-oxolan-2-yl]methoxy}pyridin-4-yl)-1,5,6,7-tetrahydro-4*H*-pyrrolo[3,2-c]pyridin-4-one
(6*R*)-3-(3-chloro-2-methylanilino)-2-{3-[(1-methoxypropan-2-yl)oxy]pyridin-4-yl}-6-methyl-1,5,6,7-tetrahydro-4*H*-pyrrolo[3,2-c]pyridin-4-one
(6*R*)-3-(3-chloro-2-methylanilino)-6-methyl-2-(3-{[(2*R*)-4-methylmorpholin-2-yl]methoxy}pyridin-4-yl)-1,5,6,7-tetrahydro-4*H*-pyrrolo[3,2-c]pyridin-4-one
(6*R*)-3-(3-chloro-2-methylanilino)-6-methyl-2-(3-{[(2*S*)-4-methylmorpholin-2-yl]methoxy}pyridin-4-yl)-1,5,6,7-tetrahydro-4*H*-pyrrolo[3,2-c]pyridin-4-one
(6*R*)-3-[2-(2,2-difluoroethyl)-3-fluoro-anilino]-2-[3-(1,4-dioxan-2-ylmethoxy)-4-pyridyl]-6-methyl-1,5,6,7-tetrahydropyrrolo[3,2-c]pyridin-4-one
(6*R*)-3-(3-chloro-2-ethylanilino)-6-methyl-2-(3-{[(2*S*)-oxolan-2-yl]methoxy}pyridin-4-yl)-1,5,6,7-tetrahydro-4*H*-pyrrolo[3,2-c]pyridin-4-one
(6*R*)-3-(3-chloro-2-ethylanilino)-2-(3-{[(2*S*)-1,4-dioxan-2-yl]methoxy}pyridin-4-yl)-6-methyl-1,5,6,7-tetrahydro-4*H*-pyrrolo[3,2-c]pyridin-4-one
3'-(3-chloro-2-methoxyanilino)-2'-(3-{[(2*S*)-1,4-dioxan-2-yl]methoxy}pyridin-4-yl)-1',7'-dihydrospiro[cyclopropane-1,6'-pyrrolo[3,2-c]pyridin]-4'(5'*H*)-one
3'-(3-chloro-2-methoxyanilino)-2'-(3-{[(2*S*)-4-methylmorpholin-2-yl]methoxy}pyridin-4-yl)-1',7'-dihydrospiro[cyclopropane-1,6'-pyrrolo[3,2-c]pyridin]-4'(5'H)-one
3'-(3-chloro-2-methoxyanilino)-2'-(3-{[(2*S*)-oxolan-2-yl]methoxy}pyridin-4-yl)-1',7'-dihydrospiro[cyclopropane-1,6'-pyrrolo[3,2-c]pyridin]-4'(5'*H*)-one
7-allyl-2-[3-(2-allyloxyethoxy)-4-pyridyl]-3-(3-chloro-2-methoxy-anilino)-1,5,6,7-tetrahydropyrrolo[3,2-c]pyridin-4-one
(7*S*)-3-(3-chloro-2-methoxyanilino)-7-(2-hydroxyethyl)-2-(3-{[(3*S*)-4-methylmorpholin-3-yl]methoxy}pyridin-4-yl)-1,5,6,7-tetrahydro-4H-pyrrolo[3,2-c]pyridin-4-one
(7*R*)-3-(3-chloro-2-methoxyanilino)-7-(2-hydroxyethyl)-2-(3-{[(3*S*)-4-methylmorpholin-3-yl]methoxy}pyridin-4-yl)-1,5,6,7-tetrahydro-4H-pyrrolo[3,2-c]pyridin-4-one
3-(3-chloro-2-methyl-anilino)-7-methyl-2-[3-(tetrahydropyran-2-ylmethoxy)-4-pyridyl]-1,5,6,7-tetrahydropyrrolo[3,2-c]pyridin-4-one
(7*S*)-3-(3-chloro-2-methyl-anilino)-7-methyl-2-(3-{[(2*S*)-tetrahydropyran-2-yl]methoxy}-4-pyridyl)-1,5,6,7-tetrahydropyrrolo[3,2-c]pyridin-4-one
(7*R*)-3-(3-chloro-2-methyl-anilino)-7-methyl-2-(3-{[(2*S*)-tetrahydropyran-2-yl]methoxy}-4-pyridyl)-1,5,6,7-tetrahydropyrrolo[3,2-c]pyridin-4-one
(6*R*)-3-(3-chloro-2-methoxy-anilino)-2-[3-(3-methoxybutoxy)-4-pyridyl]-6-methyl-1,5,6,7-tetrahydropyrrolo[3,2-c]pyridin-4-one
(6*R*)-3-(3-chloro-2-methoxy-anilino)-2-{3-[(3*S*)-3-methoxybutoxy]-4-pyridyl}-6-methyl-1,5,6,7-tetrahydropyrrolo[3,2-c]pyridin-4-one,
(6*R*)-3-(3-chloro-2-methoxy-anilino)-2-{3-[(3*R*)-3-methoxybutoxy]-4-pyridyl}-6-methyl-1,5,6,7-tetrahydropyrrolo[3,2-c]pyridin-4-one
3-(3-chloro-2-methoxy-anilino)-2-[3-(1,4-dioxan-2-ylmethoxy)-4-pyridyl]spiro[5,7-dihydro-1H-pyrrolo[3,2-c]pyridine-6,1'-cyclobutane]-4-one
3-(3-chloro-2-methoxy-anilino)-2-{3-[(2*S*)-1,4-dioxan-2-ylmethoxy]-4-pyridyl}spiro[5,7-dihydro-1*H*-pyrrolo[3,2-c]pyridine-6,1'-cyclobutane]-4-one,
3-(3-chloro-2-methoxy-anilino)-2-{3-[(2*R*)-1,4-dioxan-2-ylmethoxy]-4-pyridyl}spiro[5,7-dihydro-1*H*-pyrrolo[3,2-c]pyridine-6,1'-cyclobutane]-4-one
3'-(3-chloro-2-methoxyanilino)-2'-(3-{[(2*S*)-oxolan-2-yl]methoxy}pyridin-4-yl)-1',7'-dihydrospiro[cyclobutane-1,6'-pyrrolo[3,2-c]pyridin]-4'(5'*H*)-one
3'-(3-chloro-2-methoxyanilino)-2'-(3-{[(2*S*)-oxan-2-yl]methoxy}pyridin-4-yl)-1',7'-dihydrospiro[cyclobutane-1,6'-pyrrolo[3,2-c]pyridin]-4'(5'*H*)-one
3-(3-chloro-2-methoxy-anilino)-2-[3-(2-tetrahydropyran-2-ylethoxy)-4-pyridyl]spiro[5,7-dihydro-1*H*-pyrrolo[3,2-c]pyridine-6,1'-cyclobutane]-4-one
3'-(3-chloro-2-methoxyanilino)-2'-{3-[(5,5-dimethyl-1,4-dioxan-2-yl)methoxy]pyridin-4-yl}-1',7'-dihydrospiro[cyclobutane-1,6'-pyrrolo[3,2-c]pyridin]-4'(5'*H*)-one
3'-(3-chloro-2-methoxyanilino)-2'-(3-{[(2*R*)-5,5-dimethyl-1,4-dioxan-2-yl]methoxy}pyridin-4-yl)-1',7'-dihydrospiro[cyclobutane-1,6'-pyrrolo[3,2-c]pyridin]-4'(5'*H*)-one
3'-(3-chloro-2-methoxyanilino)-2'-(3-{[(2*S*)-5,5-dimethyl-1,4-dioxan-2-yl]methoxy}pyridin-4-yl)-1',7'-dihydrospiro[cyclobutane-1,6'-pyrrolo[3,2-c]pyridin]-4'(5'*H*)-one
3'-(3-chloro-2-methoxyanilino)-2'-{3-[(5,5-dimethyl-1,4-dioxan-2-yl)methoxy]pyridin-4-yl}-1',7'-dihydrospiro[cyclopropane-1,6'-pyrrolo[3,2-c]pyridin]-4'(5'*H*)-one
3'-(3-chloro-2-methoxyanilino)-2'-(3-{[(2*R*)-5,5-dimethyl-1,4-dioxan-2-yl]methoxy}pyridin-4-yl)-1',7'-dihydrospiro[cyclopropane-1,6'-pyrrolo[3,2-c]pyridin]-4'(5'*H*)-oneand
3'-(3-chloro-2-methoxyanilino)-2'-(3-{[(2*S*)-5,5-dimethyl-1,4-dioxan-2-yl]methoxy}pyridin-4-yl)-1',7'-dihydrospiro[cyclopropane-1,6'-pyrrolo[3,2-c]pyridin]-4'(5'*H*)-one;or
or an N-oxide, a salt, a tautomer or a stereoisomer of said compound, or a salt of said N-oxide, tautomer or stereoisomer.

A further aspect of the invention relates to compounds of formula (I), which are present as their salts.

It is to be understood that the present invention relates to any sub-combination within any embodiment or aspect of the present invention of compounds of general formula (I), supra.

More particularly still, the present invention covers compounds of general formula (I) which are disclosed in the Example section of this text, infra.

In accordance with another aspect, the present invention covers methods of preparing compounds of the present invention, said methods comprising the steps as described in the Experimental Section herein.

Another embodiment of the invention are compounds according as disclosed in the Claims section or disclosed analogs of the exemplified compounds and subcombinations thereof.

### Definitions

Constituents which are optionally substituted as stated herein, may be substituted, unless otherwise noted, one or more times, independently of one another at any possible position. When any variable occurs more than one time in any constituent, each definition is independent. For example, when R¹, R^{1a}, R^{1b}, R^{1c}, R², R³ and/or R⁴ occur more than one time in any compound of formula (I) each definition of R¹, R^{1a}, R^{1b}, R^{1c}, R², R³ and R⁴ is independent.

Should a constituent be composed of more than one part, *e.g.* C₁-C₄-alkoxy-C₂-C₄-alkyl, the position of a possible substituent can be at any of these parts at any suitable position. A hyphen at the beginning or at the end of the constituent marks the point of attachment to the rest of the molecule. Should a ring be substituted the substituent could be at any suitable position of the ring, also on a ring nitrogen atom, if suitable.

The term "comprising" when used in the specification includes "consisting of".

If it is referred to "as mentioned above" or "mentioned above", *"supra"* within the description it is referred to any of the disclosures made within the specification in any of the preceding pages.

If it is referred to "as mentioned herein", "described herein", "provided herein," or "as mentioned in the present text," or "stated herein" within the description it is referred to any of the disclosures made within the specification in any of the preceding or subsequent pages.

"Suitable" within the sense of the invention means chemically possible to be made by methods within the knowledge of a skilled person.

The terms as mentioned in the present text may have the following meanings:
The term "halogen atom", "halo-" or "Hal-" is to be understood as meaning a fluorine, chlorine, bromine or iodine atom.

The term "C₁-C₆-alkyl" is to be understood as meaning a linear or branched, saturated, monovalent hydrocarbon group having 1, 2, 3, 4, 5, or 6 carbon atoms, e.g. a methyl, ethyl, propyl, butyl, pentyl, hexyl, iso-propyl, iso-butyl, sec-butyl, tert-butyl, iso-pentyl, 2-methylbutyl, 1-methylbutyl, 1-ethylpropyl, 1,2-dimethylpropyl, neo-pentyl, 1,1-dimethylpropyl, 4-methylpentyl, 3-methylpentyl, 2-methylpentyl, 1-methylpentyl, 2-ethylbutyl, 1-ethylbutyl, 3,3-dimethylbutyl, 2,2-dimethylbutyl, 1,1-dimethylbutyl, 2,3-dimethylbutyl, 1,3-dimethylbutyl or 1,2-dimethylbutyl group, or an isomer thereof. Particularly, said group has 1, 2, 3 or 4 carbon atoms ("C₁-C₄-alkyl"), e.g. a methyl, ethyl, propyl, butyl, iso-propyl, iso-butyl, sec-butyl, tert-butyl group, more particularly 1, 2 or 3 carbon atoms ("C₁-C₃-alkyl"), e.g. a methyl, ethyl, n-propyl or iso-propyl group.

The term "C₂-C₃-fluoroalkyl" is to be understood as meaning a linear or branched, saturated, monovalent hydrocarbon group in which the term "C₂-C₃-alkyl" is defined *supra,* and in which one or more hydrogen atoms is replaced by a fluoro atom. Said C₂-C₃-fluoroalkyl group is, for example, -CF₂CF₃, -CH₂CH₂F, -CH₂CHF₂, -CH₂CF₃, - CH₂CH₂CF₃, or -CH(CH₂F)₂.

The term "C₁-C₄-alkoxy" is to be understood as meaning a linear or branched, saturated, monovalent, hydrocarbon group of formula -O-alkyl, in which the term "alkyl" is defined *supra, e.g.* a methoxy, ethoxy, n-propoxy, iso-propoxy, n-butoxy, iso-butoxy, tert-butoxy or sec-butoxy group, or an isomer thereof.

The term "C₂-C₃-alkenyl" means a linear or branched, monovalent hydrocarbon group, which contains one double bond, and which has 2 or 3 carbon atoms. Said alkenyl group is, for example, an ethenyl (or "vinyl"), prop-2-en-1-yl (or "allyl"), prop-1-en-1-yl group. Particularly, said group is vinyl or allyl.

The term "C₂-C₃-alkenyloxy" means a linear or branched, monovalent hydrocarbon group of formula -O-alkenyl, in which the term "alkenyl" is defined *supra, e.g.* an ethenyloxy (or "vinyloxy"), prop-2-en-1-yloxy (or "allyloxy"), prop-1-en-1-yloxy group. Particularly, said group is vinyloxy or allyloxy.

The term "3- to 5-membered cycloalkyl ring" means a monocyclic, saturated carbocycle with 3, 4, or 5 ring atoms in total. Said 3- to 5-membered cycloalkyl group is for example, a monocyclic hydrocarbon ring, e.g. cyclopropyl, cyclobutyl, or cyclopentyl.

The term "4- to 6-membered cycloalkyl ring" means a monocyclic, saturated carbocycle with 4, 5 or 6 ring atoms in total. Said 4- to 6-membered cycloalkyl group is for example, a monocyclic hydrocarbon ring, e.g. cyclobutyl, cyclopentyl, or cyclohexyl.

The term "C₁-C₆", as used throughout this text, e.g. in the context of the definition of "C₁-C₆-alkyl" or "C₁-C₆-haloalkyl" is to be understood as meaning an alkyl group having a finite number of carbon atoms of 1 to 6, *i.e.* 1, 2, 3, 4, 5 or 6 carbon atoms. It is to be understood further that said term "C₁-C₆" is to be interpreted as any sub-range comprised therein, *e.g*. C₁-C₆, C₂-C₆, C₃-C₆, C₁-C₂ , C₁-C₃, particularly C₁-C₂ , C₁-C₃ , C₁-C₄,
The term "C₁-C₄", as used throughout this text, e.g. in the context of the definition of "C₁-C₄-alkyl", "C₁-C₄-haloalkyl", "C₁-C₄-alkoxy", or "C₁-C₄-haloalkoxy" is to be understood as meaning an alkyl group having a finite number of carbon atoms of 1 to 4, *i.e.* 1, 2, 3 or 4 carbon atoms. It is to be understood further that said term "C₁-C₄" is to be interpreted as any sub-range comprised therein, e.g. C₁-C₄ , C₂-C₄ , C₂-C₃, C₃-C₄ , C₁-C₂ , C₁-C₃ , particularly C₁-C₂ , C₁-C₃ , C₁-C₄, in the case of "C₁-C₆-haloalkyl" or "C₁-C₄-haloalkoxy" even more particularly C₁-C₂.

Further, as used herein, the term "C₃-C₆", as used throughout this text, e.g. in the context of the definition of "C₃-C₆-cycloalkyl", is to be understood as meaning a cycloalkyl group having a finite number of carbon atoms of 3 to 6, *i.e.* 3, 4, 5 or 6 carbon atoms. It is to be understood further that said term "C₃-C₆" is to be interpreted as any sub-range comprised therein, e.g. C₃-C₆ , C₄-C₅ , C₃-C₅ , C₃-C₄ , C₄-C₆, C₅-C₆; particularly C₃-C₆.

The term "substituted" means that one or more hydrogens on the designated atom is replaced with a selection from the indicated group, provided that the designated atom's normal valency under the existing circumstances is not exceeded, and that the substitution results in a stable compound. Combinations of substituents and/or variables are permissible only if such combinations result in stable compounds.

The term "optionally substituted" means optional substitution with the specified groups, radicals or moieties.

Ring system substituent means a substituent attached to an aromatic or nonaromatic ring system which, for example, replaces an available hydrogen on the ring system.

As used herein, the term "one or more", *e.g.* in the definition of the substituents of the compounds of the general formulae of the present invention, is understood as meaning "one, two, three, four, five, etc. particularly one, two, three or four, more particularly one, two or three, even more particularly one or two".

The compounds of general formula (I) may exist as isotopic variants. The invention therefore includes one or more isotopic variant(s) of the compounds of general formula (I), particularly deuterium-containing compounds of general formula (I).

The term "isotopic variant" of a compound or a reagent is defined as a compound exhibiting an unnatural proportion of one or more of the isotopes that constitute such a compound.

The term "isotopic variant of the compound of general formula (I)" is defined as a compound of general formula (I) exhibiting an unnatural proportion of one or more of the isotopes that constitute such a compound.

The expression "unnatural proportion" is to be understood as meaning a proportion of such isotope which is higher than its natural abundance. The natural abundances of isotopes to be applied in this context are described in "Isotopic Compositions of the Elements 1997", Pure Appl. Chem., 70(1), 217-235, 1998. Examples of such isotopes include stable and radioactive isotopes of hydrogen, carbon, nitrogen, oxygen, phosphorus, sulfur, fluorine, chlorine, bromine and iodine, such as ²H (deuterium), ³H (tritium), ¹¹C, ¹³C, ¹⁴C, ¹⁵N, ¹⁷O, ¹⁸O, ³²P, ³³P, ³³S, ³⁴S, ³⁵S, ³⁶S, ¹⁸F, ³⁶Cl, ⁸²Br, ¹²³I, ¹²⁴I, ¹²⁵I, ¹²⁹I and ¹³¹I, respectively.

With respect to the treatment and/or prophylaxis of the disorders specified herein the isotopic variant(s) of the compounds of general formula (I) in one embodiment contain deuterium ("deuterium-containing compounds of general formula (I)"). Isotopic variants of the compounds of general formula (I) in which one or more radioactive isotopes, such as ³H or ¹⁴C, are incorporated are useful e.g. in drug and/or substrate tissue distribution studies. These isotopes are particularly preferred for the ease of their incorporation and detectability. Positron emitting isotopes such as ¹⁸F or ¹¹C may be incorporated into a compound of general formula (I). These isotopic variants of the compounds of general formula (I) are useful for *in vivo* imaging applications. Deuterium-containing and ¹³C-containing compounds of general formula (I) can be used in mass spectrometry analyses (H. J. Leis et al., Curr. Org. Chem., 1998, 2, 131) in the context of preclinical or clinical studies.

Isotopic variants of the compounds of general formula (I) can generally be prepared by methods known to a person skilled in the art, such as those described in the schemes and/or examples herein, by substituting a reagent for an isotopic variant of said reagent, in one embodiment for a deuterium-containing reagent. Depending on the desired sites of deuteration, in some cases deuterium from D₂O can be incorporated either directly into the compounds or into reagents that are useful for synthesizing such compounds (Esaki et al., Tetrahedron, 2006, 62, 10954; Esaki et al., Chem. Eur. J., 2007, 13, 4052). Deuterium gas is also a useful reagent for incorporating deuterium into molecules. Catalytic deuteration of olefinic bonds (H. J. Leis et al., Curr. Org. Chem., 1998, 2, 131; J. R. Morandi et al., J. Org. Chem., 1969, 34 (6), 1889) and acetylenic bonds (N. H. Khan, J. Am. Chem. Soc., 1952, 74 (12), 3018; S. Chandrasekhar et al., Tetrahedron, 2011, 52, 3865) is a rapid route for incorporation of deuterium. Metal catalysts (i.e. Pd, Pt, and Rh) in the presence of deuterium gas can be used to directly exchange deuterium for hydrogen in functional groups containing hydrocarbons (J. G. Atkinson et al., US Patent 3966781). A variety of deuterated reagents and synthetic building blocks are commercially available from companies such as for example C/D/N Isotopes, Quebec, Canada; Cambridge Isotope Laboratories Inc., Andover, MA, USA; and CombiPhos Catalysts, Inc., Princeton, NJ, USA. Further information on the state of the art with respect to deuterium-hydrogen exchange is given for example in Hanzlik et al., J. Org. Chem. 55, 3992-3997, 1990; R. P. Hanzlik et al., Biochem. Biophys. Res. Commun. 160, 844, 1989; P. J. Reider et al., J. Org. Chem. 52, 3326-3334, 1987; M. Jarman et al., Carcinogenesis 16(4), 683-688, 1993; J. Atzrodt et al., Angew. Chem., Int. Ed. 2007, 46, 7744; K. Matoishi et al., J. Chem. Soc, Chem. Commun. 2000, 1519-1520; K. Kassahun et al., WO2012/112363.

The term "deuterium-containing compound of general formula (I)" is defined as a compound of general formula (I), in which one or more hydrogen atom(s) is/are replaced by one or more deuterium atom(s) and in which the abundance of deuterium at each deuterated position of the compound of general formula (I) is higher than the natural abundance of deuterium, which is about 0.015%. Particularly, in a deuterium-containing compound of general formula (I) the abundance of deuterium at each deuterated position of the compound of general formula (I) is higher than 10%, 20%, 30%, 40%, 50%, 60%, 70% or 80%, in one embodiment higher than 90%, 95%, 96% or 97%, in other embodiments higher than 98% or 99% at said position(s). It is understood that the abundance of deuterium at each deuterated position is independent of the abundance of deuterium at other deuterated position(s).

The selective incorporation of one or more deuterium atom(s) into a compound of general formula (I) may alter the physicochemical properties (such as for example acidity [A. Streitwieser et al., J. Am. Chem. Soc., 1963, 85, 2759; C. L. Perrin, et al., J. Am. Chem. Soc., 2007, 129, 4490], basicity [C. L. Perrin, et al., J. Am. Chem. Soc., 2003, 125, 15008; C. L. Perrin in Advances in Physical Organic Chemistry, 44, 144; C. L. Perrin et al., J. Am. Chem. Soc., 2005, 127, 9641], lipophilicity [B. Testa et al., Int. J. Pharm., 1984, 19(3), 271]) and/or the metabolic profile of the molecule and may result in changes in the ratio of parent compound to metabolites or in the amounts of metabolites formed. Such changes may result in certain therapeutic advantages and hence may be preferred in some circumstances. Reduced rates of metabolism and metabolic switching, where the ratio of metabolites is changed, have been reported (D. J. Kushner et al., Can. J. Physiol. Pharmacol., 1999, 77, 79; A. E. Mutlib et al., Toxicol. Appl. Pharmacol., 2000, 169, 102). These changes in the exposure to parent drug and metabolites can have important consequences with respect to the pharmacodynamics, tolerability and efficacy of a deuterium-containing compound of general formula (I). In some cases deuterium substitution reduces or eliminates the formation of an undesired or toxic metabolite and enhances the formation of a desired metabolite (e.g. Nevirapine: A. M. Sharma et al., Chem. Res.Toxicol., 2013, 26, 410; Uetrecht et al., Chemical Research in Toxicology, 2008, 21, 9, 1862; Efavirenz: A. E. Mutlib et al., Toxicol. Appl. Pharmacol., 2000, 169, 102). In other cases the major effect of deuteration is to reduce the rate of systemic clearance. As a result, the biological half-life of the compound is increased. The potential clinical benefits would include the ability to maintain similar systemic exposure with decreased peak levels and increased trough levels. This could result in lower side effects and enhanced efficacy, depending on the particular compound's pharmacokinetic/ pharmacodynamic relationship. Indiplon (A. J. Morales et al., Abstract 285, The 15th North American Meeting of the International Society of Xenobiotics, San Diego, CA, October 12-16, 2008), ML-337 (C. J. Wenthur et al., J. Med. Chem., 2013, 56, 5208), and Odanacatib (K. Kassahun et al., WO2012/112363) are examples for this deuterium effect. Still other cases have been reported in which reduced rates of metabolism result in an increase in exposure of the drug without changing the rate of systemic clearance (e.g. Rofecoxib: F. Schneider et al., Arzneim. Forsch. Drug. Res., 2006, 56, 295; Telaprevir: F. Maltais et al., J. Med. Chem., 2009, 52, 7993). Deuterated drugs showing this effect may have reduced dosing requirements (e.g. lower number of doses or lower dosage to achieve the desired effect) and/or may produce lower metabolite loads.

A compound of general formula (I) may have multiple potential sites of attack for metabolism. To optimize the above-described effects on physicochemical properties and metabolic profile, deuterium-containing compounds of general formula (I) having a certain pattern of one or more deuterium-hydrogen exchange(s) can be selected. Particularly, the deuterium atom(s) of deuterium- containing compound(s) of general formula (I) is/are attached to a carbon atom and/or is/are located at those positions of the compound of general formula (I), which are sites of attack for metabolizing enzymes such as e.g. cytochrome P₄₅₀.

Where the plural form of the word compounds or salts is used herein, this is taken to mean also a single compound or salt.

By "stable compound' or "stable structure" is meant a compound that is sufficiently robust to survive isolation to a useful degree of purity from a reaction mixture, and formulation into an efficacious therapeutic agent.

The compounds of this invention may contain one or more asymmetric centre, depending upon the location and nature of the various substituents desired. Asymmetric carbon atoms may be present in the (R) or (S) configuration, resulting in racemic mixtures in the case of a single asymmetric centre, and diastereomeric mixtures in the case of multiple asymmetric centres. In certain instances, asymmetry may also be present due to restricted rotation about a given bond, for example, the central bond adjoining two substituted aromatic rings of the specified compounds.

Substituents on a ring may also be present in either cis or trans form. It is intended that all such configurations (including enantiomers and diastereomers), are included within the scope of the present invention.

Preferred compounds are those which produce the more desirable biological activity. Separated, pure or partially purified isomers and stereoisomers or racemic or diastereomeric mixtures of the compounds of this invention are also included within the scope of the present invention. The purification and the separation of such materials can be accomplished by standard techniques known in the art.

The optical isomers can be obtained by resolution of the racemic mixtures according to conventional processes, for example, by the formation of diastereoisomeric salts using an optically active acid or base or formation of covalent diastereomers. Examples of appropriate acids are tartaric, diacetyltartaric, ditoluoyltartaric and camphorsulfonic acid. Mixtures of diastereoisomers can be separated into their individual diastereomers on the basis of their physical and/or chemical differences by methods known in the art, for example, by chromatography or fractional crystallisation. The optically active bases or acids are then liberated from the separated diastereomeric salts. A different process for separation of optical isomers involves the use of chiral chromatography (e.g., chiral HPLC columns), with or without conventional derivatisation, optimally chosen to maximise the separation of the enantiomers. Suitable chiral HPLC columns are manufactured by Daicel, e.g., Chiracel OD and Chiracel OJ among many others, all routinely selectable. Enzymatic separations, with or without derivatisation, are also useful. The optically active compounds of this invention can likewise be obtained by chiral syntheses utilizing optically active starting materials.

In order to limit different types of isomers from each other reference is made to IUPAC Rules Section E (Pure Appl Chem 45, 11-30, 1976).

The present invention includes all possible stereoisomers of the compounds of the present invention as single stereoisomers, or as any mixture of said stereoisomers, *e.g.* R- or S- isomers, or E- or Z-isomers, in any ratio. Isolation of a single stereoisomer, *e.g.* a single enantiomer or a single diastereomer, of a compound of the present invention may be achieved by any suitable state of the art method, such as chromatography, especially chiral chromatography, for example.

Further, the compounds of the present invention may exist as tautomers. For example, any compound of the present invention which contains a pyrazole moiety as a heteroaryl group for example can exist as a 1H tautomer, or a 2H tautomer, or even a mixture in any amount of the two tautomers, or a triazole moiety for example can exist as a 1H tautomer, a 2H tautomer, or a 4H tautomer, or even a mixture in any amount of said 1H, 2H and 4H tautomers, namely :

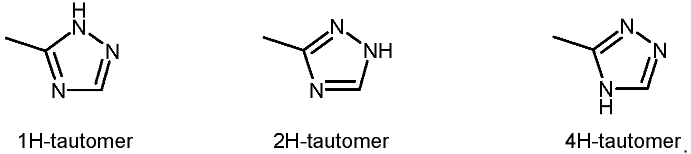

The present invention includes all possible tautomers of the compounds of the present invention as single tautomers, or as any mixture of said tautomers, in any ratio.

Further, the compounds of the present invention can exist as N-oxides, which are defined in that at least one nitrogen of the compounds of the present invention is oxidised. The present invention includes all such possible N-oxides.

Further, the compounds of the present invention can exist in free form, *e.g.* as a free base, or as a free acid, or as a zwitterion, or can exist in the form of a salt. Said salt may be any salt, either an organic or inorganic addition salt, particularly any pharmaceutically acceptable organic or inorganic addition salt, customarily used in pharmacy.

The term "pharmaceutically acceptable salt" refers to a relatively non-toxic, inorganic or organic acid addition salt of a compound of the present invention. For example, see S. M. Berge, et al. "Pharmaceutical Salts," J. Pharm. Sci. 1977, 66, 1-19.

A suitable pharmaceutically acceptable salt of the compounds of the present invention may be, for example, an acid-addition salt of a compound of the present invention bearing a nitrogen atom, in a chain or in a ring, for example, which is sufficiently basic, such as an acid-addition salt with an inorganic acid, such as hydrochloric, hydrobromic, hydroiodic, sulfuric, bisulfuric, phosphoric or nitric acid, for example, or with an organic acid, such as formic, acetic, acetoacetic, pyruvic, trifluoroacetic, propionic, butyric, hexanoic, heptanoic, undecanoic, lauric, benzoic, salicylic, 2-(4-hydroxybenzoyl)-benzoic, camphoric, cinnamic, cyclopentanepropionic, digluconic, 3-hydroxy-2-naphthoic, nicotinic, pamoic, pectinic, persulfuric, 3-phenylpropionic, picric, pivalic, 2-hydroxyethanesulfonate, itaconic, sulfamic, trifluoromethanesulfonic, dodecylsulfuric, ethansulfonic, benzenesulfonic, para-toluenesulfonic, methansulfonic, 2-naphthalenesulfonic, naphthalinedisulfonic, camphorsulfonic acid, citric, tartaric, stearic, lactic, oxalic, malonic, succinic, malic, adipic, alginic, maleic, fumaric, D-gluconic, mandelic, ascorbic, glucoheptanoic, glycerophosphoric, aspartic, sulfosalicylic, hemisulfuric or thiocyanic acid, for example.

Further, another suitably pharmaceutically acceptable salt of a compound of the present invention which is sufficiently acidic, is an alkali metal salt, for example a sodium or potassium salt, an alkaline earth metal salt, for example a calcium or magnesium salt, an ammonium salt or a salt with an organic base which affords a physiologically acceptable cation, for example a salt with N-methyl-glucamine, dimethyl-glucamine, ethyl-glucamine, lysine, dicyclohexylamine, 1,6-hexadiamine, ethanolamine, glucosamine, sarcosine, serinol, tris-hydroxy-methyl-aminomethane, aminopropandiol, sovak-base, 1-amino-2,3,4-butantriol. Additionally, basic nitrogen containing groups may be quaternised with such agents as lower alkyl halides such as methyl, ethyl, propyl, and butyl chlorides, bromides and iodides; dialkyl sulfates like dimethyl, diethyl, and dibutyl sulfate; and diamyl sulfates, long chain halides such as decyl, lauryl, myristyl and strearyl chlorides, bromides and iodides, aralkyl halides like benzyl and phenethyl bromides and others.

Those skilled in the art will further recognise that acid addition salts of the claimed compounds may be prepared by reaction of the compounds with the appropriate inorganic or organic acid via any of a number of known methods. Alternatively, alkali and alkaline earth metal salts of acidic compounds of the invention are prepared by reacting the compounds of the invention with the appropriate base via a variety of known methods.

The present invention includes all possible salts of the compounds of the present invention as single salts, or as any mixture of said salts, in any ratio.

In the present text, in particular in the Experimental Section, for the synthesis of intermediates and of examples of the present invention, when a compound is mentioned as a salt form with the corresponding base or acid, the exact stoichiometric composition of said salt form, as obtained by the respective preparation and/or purification process, is, in most cases, unknown.

Unless specified otherwise, suffixes to chemical names or structural formulae such as "hydrochloride", "trifluoroacetate", "sodium salt", or "x HCl", "x CF₃COOH", "x Na⁺", for example, are to be understood as not a stoichiometric specification, but solely as a salt form.

This applies analogously to cases in which synthesis intermediates or example compounds or salts thereof have been obtained, by the preparation and/or purification processes described, as solvates, such as hydrates with (if defined) unknown stoichiometric composition.

The salts include water-insoluble and, particularly, water-soluble salts.

Furthermore, the present invention includes all possible crystalline forms, or polymorphs, of the compounds of the present invention, either as single polymorphs, or as a mixture of more than one polymorphs, in any ratio.

In the context of the properties of the compounds of the present invention the term "pharmacokinetic profile" means one single parameter or a combination thereof including permeability, bioavailability, exposure, and pharmacodynamic parameters such as duration, or magnitude of pharmacological effect, as measured in a suitable experiment. Compounds with improved pharmacokinetic profiles can, for example, be used in lower doses to achieve the same effect, may achieve a longer duration of action, or a may achieve a combination of both effects.

The term "combination" in the present invention is used as known to persons skilled in the art and may be present as a fixed combination, a non-fixed combination or kit-of-parts.

A "fixed combination" in the present invention is used as known to persons skilled in the art and is defined as a combination wherein the said first active ingredient and the said second active ingredient are present together in one unit dosage or in a single entity. One example of a "fixed combination" is a pharmaceutical composition wherein the said first active ingredient and the said second active ingredient are present in admixture for simultaneous administration, such as in a formulation. Another example of a "fixed combination" is a pharmaceutical combination wherein the said first active ingredient and the said second active ingredient are present in one unit without being in admixture.

A non-fixed combination or "kit-of-parts" in the present invention is used as known to persons skilled in the art and is defined as a combination wherein the said first active ingredient and the said second active ingredient are present in more than one unit. One example of a non-fixed combination or kit-of-parts is a combination wherein the said first active ingredient and the said second active ingredient are present separately. The components of the non-fixed combination or kit-of-parts may be administered separately, sequentially, simultaneously, concurrently or chronologically staggered. Any such combination of a compound of formula (I) of the present invention with an anti-cancer agent as defined below is an embodiment of the invention.

The term "(chemotherapeutic) anti-cancer agents" relates to any agent that reduces the survival or proliferation of a cancer cell, and includes but is not limited to 131I-chTNT, abarelix, abiraterone, aclarubicin, ado-trastuzumab emtansine, afatinib, aflibercept, aldesleukin, alemtuzumab, Alendronic acid, alitretinoin, altretamine, amifostine, aminoglutethimide, Hexyl aminolevulinate, amrubicin, amsacrine, anastrozole, ancestim, anethole dithiolethione, angiotensin II, antithrombin III, aprepitant, arcitumomab, arglabin, arsenic trioxide, asparaginase, axitinib, azacitidine, basiliximab, belotecan, bendamustine, belinostat, bevacizumab, bexarotene, bicalutamide, bisantrene, bleomycin, bortezomib, buserelin, bosutinib, brentuximab vedotin, busulfan, cabazitaxel, cabozantinib, calcium folinate, calcium levofolinate, capecitabine, capromab, carboplatin, carfilzomib, carmofur, carmustine, catumaxomab, celecoxib, celmoleukin, ceritinib, cetuximab, chlorambucil, chlormadinone, chlormethine, cidofovir, cinacalcet, cisplatin, cladribine, clodronic acid, clofarabine, copanlisib, crisantaspase, cyclophosphamide, cyproterone, cytarabine, dacarbazine, dactinomycin, darbepoetin alfa, dabrafenib, dasatinib, daunorubicin, decitabine, degarelix, denileukin diftitox, denosumab, depreotide, deslorelin, dexrazoxane, dibrospidium chloride, dianhydrogalactitol, diclofenac, docetaxel, dolasetron, doxifluridine, doxorubicin, doxorubicin + estrone, dronabinol, eculizumab, edrecolomab, elliptinium acetate, eltrombopag, endostatin, enocitabine, enzalutamide, epirubicin, epitiostanol, epoetin alfa, epoetin beta, epoetin zeta, eptaplatin, eribulin, erlotinib, esomeprazole, estradiol, estramustine, etoposide, everolimus, exemestane, fadrozole, fentanyl, filgrastim, fluoxymesterone, floxuridine, fludarabine, fluorouracil, flutamide, folinic acid, formestane, fosaprepitant, fotemustine, fulvestrant, gadobutrol, gadoteridol, gadoteric acid meglumine, gadoversetamide, gadoxetic acid, gallium nitrate, ganirelix, gefitinib, gemcitabine, gemtuzumab, Glucarpidase, glutoxim, GM-CSF, goserelin, granisetron, granulocyte colony stimulating factor, histamine dihydrochloride, histrelin, hydroxycarbamide, I-125 seeds, lansoprazole, ibandronic acid, ibritumomab tiuxetan, ibrutinib, idarubicin, ifosfamide, imatinib, imiquimod, improsulfan, indisetron, incadronic acid, ingenol mebutate, interferon alfa, interferon beta, interferon gamma, iobitridol, iobenguane (123I), iomeprol, ipilimumab, irinotecan, Itraconazole, ixabepilone, lanreotide, lapatinib, lasocholine, lenalidomide, lenograstim, lentinan, letrozole, leuprorelin, levamisole, levonorgestrel, levothyroxine sodium, lisuride, lobaplatin, lomustine, lonidamine, masoprocol, medroxyprogesterone, megestrol, melarsoprol, melphalan, mepitiostane, mercaptopurine, mesna, methadone, methotrexate, methoxsalen, methylaminolevulinate, methylprednisolone, methyltestosterone, metirosine, mifamurtide, miltefosine, miriplatin, mitobronitol, mitoguazone, mitolactol, mitomycin, mitotane, mitoxantrone, mogamulizumab, molgramostim, mopidamol, morphine hydrochloride, morphine sulfate, nabilone, nabiximols, nafarelin, naloxone + pentazocine, naltrexone, nartograstim, nedaplatin, nelarabine, neridronic acid, nivolumabpentetreotide, nilotinib, nilutamide, nimorazole, nimotuzumab, nimustine, nitracrine, nivolumab, obinutuzumab, octreotide, ofatumumab, omacetaxine mepesuccinate, omeprazole, ondansetron, oprelvekin, orgotein, orilotimod, osimertinib, oxaliplatin, oxycodone, oxymetholone, ozogamicine, p53 gene therapy, paclitaxel, palifermin, palladium-103 seed, palonosetron, pamidronic acid, panitumumab, pantoprazole, pazopanib, pegaspargase, PEG-epoetin beta (methoxy PEG-epoetin beta), pembrolizumab, pegfilgrastim, peginterferon alfa-2b, pemetrexed, pentazocine, pentostatin, peplomycin, Perflubutane, perfosfamide, Pertuzumab, picibanil, pilocarpine, pirarubicin, pixantrone, plerixafor, plicamycin, poliglusam, polyestradiol phosphate, polyvinylpyrrolidone + sodium hyaluronate, polysaccharide-K, pomalidomide, ponatinib, porfimer sodium, poziotinib, pralatrexate, prednimustine, prednisone, procarbazine, procodazole, propranolol, quinagolide, rabeprazole, racotumomab, radium-223 chloride, radotinib, raloxifene, raltitrexed, ramosetron, ramucirumab, ranimustine, rasburicase, razoxane, refametinib, regorafenib, risedronic acid, rhenium-186 etidronate, rituximab, romidepsin, romiplostim, romurtide, roniciclib, samarium (153Sm) lexidronam, sargramostim, satumomab, secretin, sipuleucel-T, sizofiran, sobuzoxane, sodium glycididazole, sorafenib, stanozolol, streptozocin, sunitinib, talaporfin, tamibarotene, tamoxifen, tapentadol, tasonermin, teceleukin, technetium (99mTc) nofetumomab merpentan, 99mTc-HYNIC-[Tyr3]-octreotide, tegafur, tegafur + gimeracil + oteracil, temoporfin, temozolomide, temsirolimus, teniposide, testosterone, tetrofosmin, thalidomide, thiotepa, thymalfasin, thyrotropin alfa, tioguanine, tocilizumab, topotecan, toremifene, tositumomab, trabectedin, tramadol, trastuzumab, trastuzumab emtansine, treosulfan, tretinoin, trifluridine + tipiracil, trilostane, triptorelin, trametinib, trofosfamide, thrombopoietin, tryptophan, ubenimex, valatinib, valrubicin, vandetanib, vapreotide, vemurafenib, vinblastine, vincristine, vindesine, vinflunine, vinorelbine, vismodegib, vorinostat, vorozole, yttrium-90 glass microspheres, zinostatin, zinostatin stimalamer, zoledronic acid, zorubicin.

By "Epidermal Growth Factor Receptor (EGFR) Polypeptide" is meant a polypeptide having at least about 95% amino acid sequence identity to the sequence provided at UniProt Accession No. P00533-1 or a fragment thereof. In some embodiments, the EGFR fragment binds an EFGR ligand and/or has kinase activity. Mutant EGFR polypeptides include those having an insertion between, for example, amino acids V769 and D770 or between D770 and N771. In other embodiments, the amino acid sequence identity is 96, 97, 98, 99, or 100% to UniProt Accession No. P00533-1.

An exemplary full length sequence of human EGFR, which indicates V769, D770, and N771 in bold, is provided at UniProt Accession No. P00533-1, which is reproduced below:

10          20          30          40          50

60          70          80          90          100

110         120         130         140         150

160         170         180         190         200

210         220         230         240         250

260         270         280         290         300

310         320         330         340         350


360 370 380 390 400

410 420 430 440 450

460 470 480 490 500

510 520 530 540 550

560 570 580 590 600

610 620 630 640 650

660 670 680 690 700

710 720 730 740 750

760 770 780 790 800

810 820 830 840 850

860 870 880 890 900

910 920 930 940 950

960 970 980 990 1000

1010 1020 1030 1040 1050

1060 1070 1080 1090 1100

1110 1120 1130 1140 1150

1160 1170 1180 1190 1200

1210


An exemplary polynucleotide encoding EGFR is provided at NCBI Reference Sequence: NM_001346897.1, which is reproduced below:

1 gtccgggcag cccccggcgc agcgcggccg cagcagcctc cgccccccgc
acggtgtgag
61 cgcccgacgc ggccgaggcg gccggagtcc cgagctagcc ccggcggccg
ccgccgccca
121 gaccggacga caggccacct cgtcggcgtc cgcccgagtc cccgcctcgc
cgccaacgcc
181 acaaccaccg cgcacggccc cctgactccg tccagtattg atcgggagag
ccggagcgag
241 ctcttcgggg agcagcgatg cgaccctccg ggacggccgg ggcagcgctc
ctggcgctgc
301 tggctgcgct ctgcccggcg agtcgggctc tggaggaaaa gaaagtttgc
caaggcacga
361 gtaacaagct cacgcagttg ggcacttttg aagatcattt tctcagcctc
cagaggatgt
421 tcaataactg tgaggtggtc cttgggaatt tggaaattac ctatgtgcag
aggaattatg
481 atctttcctt cttaaagacc atccaggagg tggctggtta tgtcctcatt
gccctcaaca
541 cagtggagcg aattcctttg gaaaacctgc agatcatcag aggaaatatg
tactacgaaa
601 attcctatgc cttagcagtc ttatctaact atgatgcaaa taaaaccgga
ctgaaggagc
661 tgcccatgag aaatttacag ggccaaaagt gtgatccaag ctgtcccaat
gggagctgct
721 ggggtgcagg agaggagaac tgccagaaac tgaccaaaat catctgtgcc
cagcagtgct
781 ccgggcgctg ccgtggcaag tcccccagtg actgctgcca caaccagtgt
gctgcaggct
841 gcacaggccc ccgggagagc gactgcctgg tctgccgcaa attccgagac
gaagccacgt
901 gcaaggacac ctgcccccca ctcatgctct acaaccccac cacgtaccag
atggatgtga

961 accccgaggg caaatacagc tttggtgcca cctgcgtgaa gaagtgtccc cgtaattatg
1021 tggtgacaga tcacggctcg tgcgtccgag cctgtggggc cgacagctat gagatggagg
1081 aagacggcgt ccgcaagtgt aagaagtgcg aagggccttg ccgcaaagtg tgtaacggaa
1141 taggtattgg tgaatttaaa gactcactct ccataaatgc tacgaatatt aaacacttca
1201 aaaactgcac ctccatcagt ggcgatctcc acatcctgcc ggtggcattt aggggtgact
1261 ccttcacaca tactcctcct ctggatccac aggaactgga tattctgaaa accgtaaagg
1321 aaatcacagg gtttttgctg attcaggctt ggcctgaaaa caggacggac ctccatgcct
1381 ttgagaacct agaaatcata cgcggcagga ccaagcaaca tggtcagttt tctcttgcag
1441 tcgtcagcct gaacataaca tccttgggat tacgctccct caaggagata agtgatggag
1501 atgtgataat ttcaggaaac aaaaatttgt gctatgcaaa tacaataaac tggaaaaaac
1561 tgtttgggac ctccggtcag aaaaccaaaa ttataagcaa cagaggtgaa aacagctgca
1621 aggccacagg ccaggtctgc catgccttgt gctcccccga gggctgctgg ggcccggagc
1681 ccagggactg cgtctcttgc cggaatgtca gccgaggcag ggaatgcgtg gacaagtgca
1741 accttctgga gggtgagcca agggagtttg tggagaactc tgagtgcata cagtgccacc
1801 cagagtgcct gcctcaggcc atgaacatca cctgcacagg acggggacca gacaactgta
1861 tccagtgtgc ccactacatt gacggccccc actgcgtcaa gacctgcccg gcaggagtca
1921 tgggagaaaa caacaccctg gtctggaagt acgcagacgc cggccatgtg tgccacctgt
1981 gccatccaaa ctgcacctac ggatgcactg ggccaggtct tgaaggctgt ccaacgaatg

2041 ggcctaagat cccgtccatc gccactggga tggtgggggc cctcctcttg
ctgctggtgg
2101 tggccctggg gatcggcctc ttcatgcgaa ggcgccacat cgttcggaag
cgcacgctgc
2161 ggaggctgct gcaggagagg gagcttgtgg agcctcttac acccagtgga
gaagctccca
2221 accaagctct cttgaggatc ttgaaggaaa ctgaattcaa aaagatcaaa
gtgctgggct
2281 ccggtgcgtt cggcacggtg tataagggac tctggatccc agaaggtgag
aaagttaaaa
2341 ttcccgtcgc tatcaaggaa ttaagagaag caacatctcc gaaagccaac
aaggaaatcc
2401 tcgatgaagc ctacgtgatg gccagcgtgg acaaccccca cgtgtgccgc
ctgctgggca
2461 tctgcctcac ctccaccgtg cagctcatca cgcagctcat gcccttcggc
tgcctcctgg
2521 actatgtccg ggaacacaaa gacaatattg gctcccagta cctgctcaac
tggtgtgtgc
2581 agatcgcaaa gggcatgaac tacttggagg accgtcgctt ggtgcaccgc
gacctggcag
2641 ccaggaacgt actggtgaaa acaccgcagc atgtcaagat cacagatttt
gggctggcca
2701 aactgctggg tgcggaagag aaagaatacc atgcagaagg aggcaaagtg
cctatcaagt
2761 ggatggcatt ggaatcaatt ttacacagaa tctataccca ccagagtgat
gtctggagct
2821 acggggtgac tgtttgggag ttgatgacct ttggatccaa gccatatgac
ggaatccctg
2881 ccagcgagat ctcctccatc ctggagaaag gagaacgcct ccctcagcca
cccatatgta
2941 ccatcgatgt ctacatgatc atggtcaagt gctggatgat agacgcagat
agtcgcccaa
3001 agttccgtga gttgatcatc gaattctcca aaatggcccg agacccccag
cgctaccttg
3061 tcattcaggg ggatgaaaga atgcatttgc caagtcctac agactccaac
ttctaccgtg

3121 ccctgatgga tgaagaagac atggacgacg tggtggatgc cgacgagtac
ctcatcccac
3181 agcagggctt cttcagcagc ccctccacgt cacggactcc cctcctgagc
tctctgagtg
3241 caaccagcaa caattccacc gtggcttgca ttgatagaaa tgggctgcaa
agctgtccca
3301 tcaaggaaga cagcttcttg cagcgataca gctcagaccc cacaggcgcc
ttgactgagg
3361 acagcataga cgacaccttc ctcccagtgc ctggtgagtg gcttgtctgg
aaacagtcct
3421 gctcctcaac ctcctcgacc cactcagcag cagccagtct ccagtgtcca
agccaggtgc
3481 tccctccagc atctccagag ggggaaacag tggcagattt gcagacacag
tgaagggcgt
3541 aaggagcaga taaacacatg accgagcctg cacaagctct ttgttgtgtc
tggttgtttg
3601 ctgtacctct gttgtaagaa tgaatctgca aaatttctag cttatgaagc
aaatcacgga
3661 catacacatc tgtgtgtgtg agtgttcatg atgtgtgtac atctgtgtat
gtgtgtgtgt
3721 gtatgtgtgt gtttgtgaca gatttgatcc ctgttctctc tgctggctct
atcttgacct
3781 gtgaaacgta tatttaacta attaaatatt agttaatatt aataaatttt
aagctttatc
3841 cagaaaaaaa aaaaaaaaa

The intermediates used for the synthesis of the compounds of claims 1-4 as described below, as well as their use for the synthesis of the compounds of claims 1-4, are one further aspect of the present invention. Preferred intermediates are the Intermediate Examples as disclosed below.

### General Procedures

The compounds according to the invention can be prepared according to the following schemes 1 - 5.

The schemes and procedures described below illustrate synthetic routes to the compounds of general formula (I) of the invention and are not intended to be limiting. It is obvious to the person skilled in the art that the order of transformations as exemplified in the schemes can be modified in various ways. The order of transformations exemplified in the schemes is therefore not intended to be limiting. In addition, interconversion of any of the substituents R¹, R², R³, R⁴, R⁵, R⁶, and PG can be achieved before and/or after the exemplified transformations. These modifications can be such as the introduction of protecting groups, cleavage of protecting groups, reduction or oxidation of functional groups, halogenation, metallation, substitution or other reactions known to the person skilled in the art. These transformations include those which introduce a functionality which allows for further interconversion of substituents. Appropriate protecting groups and their introduction and cleavage are well-known to the person skilled in the art. Specific examples are described in the subsequent paragraphs.

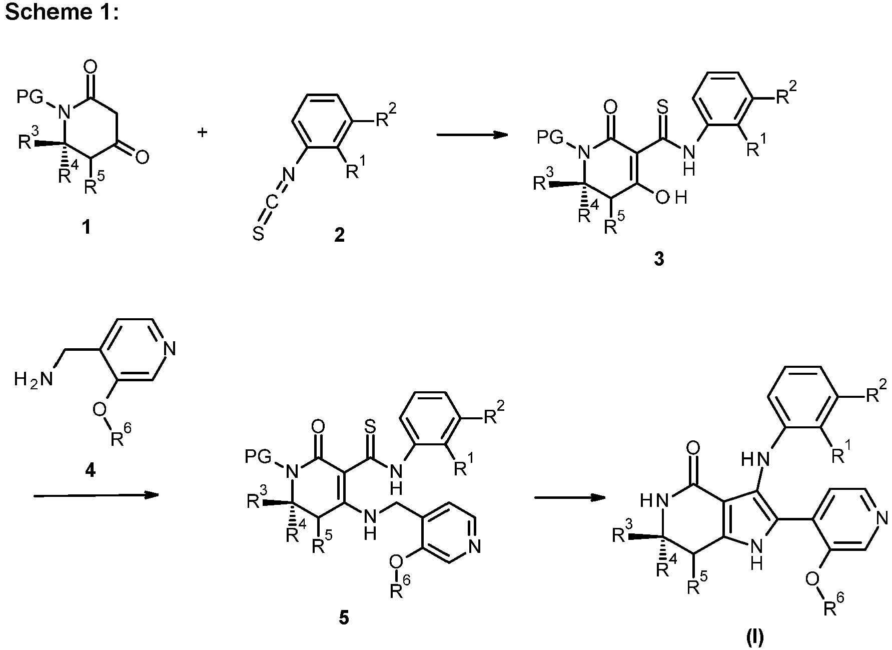

*Scheme* 1: Route for the preparation of compounds of general formula (I), wherein R¹, R², R³, R⁴, R⁵, and R⁶ have the meaning as given for general formula (I) and PG can be hydrogen or optionally a suitable protecting group, e.g. tert-butoxycarbonyl (Boc).

Compound of formula 1, **2,** and 4 are either commercially available or can be prepared according to procedures available from the public domain, as understandable to the person skilled in the art. Specific examples are described in the subsequent paragraphs.

A suitably substituted piperadine-2,4-diones of general formula (Compound of formula **1),** such as, for example, 2,4-piperadinedione, can be reacted with a suitably substituted isothiocyanate (Compound of formula **2),** such as, for example, 3-fluorophenylisothiocyanate, in a suitable solvent system, such as, for example, acetonitrile, in the presence of a suitable base, such as, for example, triethylamine or DBU, at temperatures ranging from -78°C to +100°C, in some embodiments the reaction is carried out at 0°C or +100°C, to furnish general formula (3). Similar reactions have been performed in the literature (D. E. Worrall, *J. Am. Chem. Soc.,* 1940, 62, 675).

Intermediates of general formula **(3)** can be converted to Intermediates of general formula **(5)** by reaction with a suitable amine (compounds of general formula **4),** such as, for example 4-(aminomethyl)pyridine, in a suitable solvent system, such as, for example, ethanol and ethyl acetate, at a temperature between room temperature and the boiling point of the respective solvents, in some embodiments the reaction is carried out at the boiling point of the respective solvents, whereby the water formed in the reaction is removed from the reaction by methods known to those skilled in the art, such as, for example, azeotropic removal of water (Dean-Stark conditions) or with molecular sieves, to furnish general formula **(5).**

Intermediates of general formula **(3)** and intermediates of general formula **(5)** in which PG represents a protecting group can be converted to Intermediates in which PG represents a hydrogen atom using standard deprotection conditions known to those skilled in the art. When PG is a protecting group such as, for example, tert-butoxycarbonyl (Boc), the deprotection can be carried out using acids, such as, for example, hydrochloric acid and trifluoroacetic acid, in a suitable solvent system, such as, for example, dichloromethane and dioxane, at a temperature between 0°C and the boiling point of the respective solvents, in one embodiment the reaction is carried out at the room temperature, to furnish compounds of general formula (3) and intermediates of general formula **(5)** whereby PG is hydrogen atom.

Intermediates of general formula **(5)** are reacted with a base and/or oxidizing reagent, in one embodiment an oxidizing agent, such as, for example hydrogen peroxide or SIBX (stabilized iodoxybenoic acid, in a suitable solvent system, such as, for example, methanol, in a temperature range from -30°C to the boiling point of the respective solvent, in one embodiment the reaction is carried out at the boiling point of the respective solvent, to furnish compounds of general formula **(I).** Optionally, these types of reactions can be carried on with an additive, such as, for example, an acid or base, such as, for example, acetic acid or trifluoroacetic acid (not-limiting), and triethylamine or diispropylethylamine (not-limiting).

Intermediates of general formula **(5)** could be converted to compounds of general formula **(I)** by thermal heating them in a suitable solvent at elevated temperatures, which could be above the boiling point of the said solvent, such as, for example, RT to +250°C. These reactions could optionally be carried out in vessel whereby the pressure can be increased, such as, for example, in an autoclave. Intermediates of general formula **(5)** can also be converted to compounds of general formula **(I)** by thermal heating in the presence of a metal catalyst, such as, for example, palladium on activated charcoal, in a suitable solvent, such as, for example, DMF, DMA, EtOH, MeOH, NMP (not-limiting) at elevated temperatures, such as, for example, RT to +150°C. Optionally, these types of reactions can be carried on with an additive, such as, for example, an acid or base, such as, for example, acetic acid or trifluoroacetic acid (not-limiting), and triethylamine or diispropylethylamine (not-limiting), to furnish compounds of general formula (I).

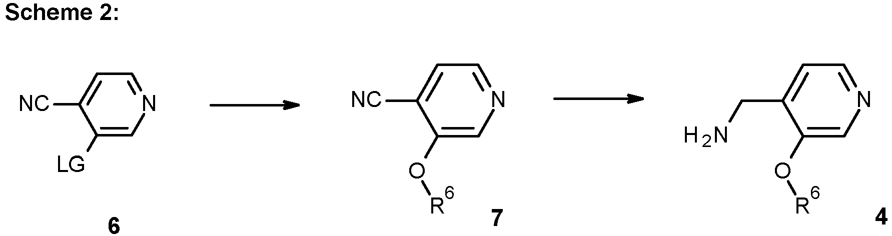

*Scheme 2:* Process for the preparation of compounds of general formula **(4),** wherein R⁶ have the meaning as given for general formula (I).

Compounds of general formula **(6)** can be converted to compounds of general formula **(7)** by treatment with a suitable nucleophile, such as for example, amines, alcohols, metal alkoxides, azides, thiols or metal thiolates, under either basic, neutral, acidic, catalytic conditions, in one embodiment basic conditions, in a suitable solvent or using the nucleophile as solvent, such as, for example, DMF, tetrahydrofuran (THF), in a temperature range from -78°C to the boiling point of the respective solvent, in one embodiment the reaction is carried out -10°C to the boiling point of the respective solvent, to furnish general formula **(7).** Such substitution reactions have been previously reported (Clark et al., J. Med. Chem., 2008, 51, 6631 - 6634; Guo et al., Tetrahedron Letts., 2013, 54, 3233 - 3237; Watterson et al., J. Med. Chem., 2007, 50, 3730 - 3742; Bellale et al., J. Med. Chem., 2014, 57, 6572 - 6582; Klimesova et al., Eur. J. Med. Chem., 1996, 31, 389 - 395; Leroy et al., Synth. Commun., 1997, 27, 2905 - 2916; LaMattina et al., J. Org. Chem., 1981, 46, 4179 - 4182; Beugelmans et al., Tetrahedron, 1983, 39, 4153 - 4162).

Compounds of general formula **(7)** can be converted to compounds of general formula **(4)** by many reducing methods known to those skilled in the art, using numerous different reagents and reaction conditions; such methods and reagents can be carried out with metal hydrides, such as, for example, lithium aluminum hydride in THF (Bullock et al., J. Am. Chem. Soc., 1956, 78, 490, Wang et al., J. Org. Chem., 2006, 71, 4021 - 3160), or using zinc in acetic acid (Rabe, Chem. Ber., 1913, 46, 1024), or using diborane (De Munno et al., Heterocycles, 1996, 43, 1893 - 1900), or using catalytic hydrogenation methods, for example, hydrogen and palladium on carbon under acidic conditions (Stokker et al., J. Med. Chem., 1981, 24, 115 - 117; Bertini et al., J. Med. Chem., 2005, 48, 664 - 670), hydrogen and nickel under basic conditions (Walpole et al., J. Med. Chem., 1993, 36, 2362 - 2372, Kuramochi et al., Bioorg. Med. Chem., 2005, 13, 4022 - 4036.)

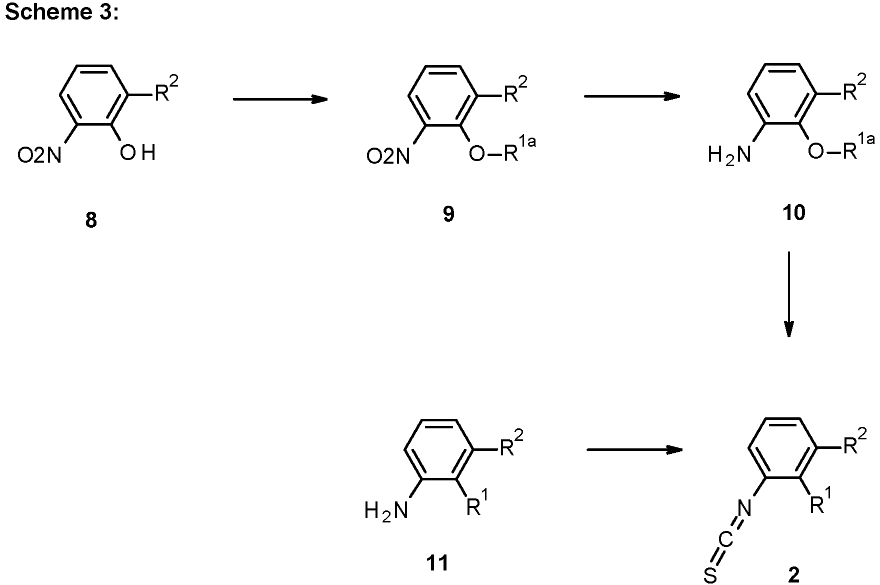

*Scheme 3:* Process for the preparation of compounds of general formula **2,** wherein R^{1a} represents methyl or difluoromethyl corresponding to R¹ in the general formula (I) with the meaning of methoxy and difluoromethoxy. The synthesis of compounds **9** and **10** relates to alkoxy substitution of the phenyl ring. However, the isothiocyanate containing product **2** and the synthesis thereof (*i.e.,* **10 → 2** or **11 → 2)** is general to R¹ groups according to general formula (I).

Compounds of general formula **(8),** can be converted to compounds of general formula **(9),** using various methods which are known to those skilled in the art. Such transformations could be, for example, to alkylate the phenolic alcohol with alkylating reagents, such as, for example, alkyl halides, alkyl sulfonates, in which these alkyl groups can optionally contain fluorides, alkoxyl groups. These alkylation reactions are known to those skilled in the art using a variety of methods: i) K₂CO₃ in a solvent such as, DMF, acetone, DMFA (see the teachings of Muro et al., J. Med. Chem., 2009, 52, 7974 and WO2009/20990 A1); ii) KOH in EtOH (see the teachings of Macias et al., J. Agric. Food Chem., 2006, 54, 9843); iii) Mitsunobu reaction (see the teachings of US2006/122168 A1 and EP2151431 A1) to furnish intermediates of general formula **(9).**

Compounds of general formula **(9)** can be converted to compounds of general formula **(10)** by reduction methods and these methods are known to those skilled in the art. These reductions can be carried using: i) hydrogen gas and a catalyst (for Pd/C as catalyst see the teachings of Chan et al., J. Am. Chem. Soc., 2011, 133, 2989; for platinum see the teachings of Niemann et al., J. Am. Chem Soc., 1941, 63, 2204; for Raney-Nickel see the teachings of US2009/253767 A1); ii) iron and ammonium chloride (see the teachings of Sweeney et al., Bioorg. Med. Chem. Lett., 2008, 18, 4348); iii) sodium dithionite (see the teachings of Chong et al., J. Med. Chem., 2012, 55, 10601); iv) zinc and ammonium chloride (see the teachings of WO2010/42699 A1) to furnish intermediates of general formula **(10).**

Compounds of general formula **(10)** can be converted to compounds of general formula **(2)** by using reagents such as, for example, thiophosgene, carbon disulphide, 1,1"-thiocarbonyldi-2(1H)-pyridone or 1,1'-thiocarbonyldiimidazole, in one embodiment thiophosgene, under basic conditions, in a suitable solvent, such as, for example, dichloromethane, chloroform, acetone, or biphasic mixtures, such as, for example, dichloromethane, chloroform with aqueous basic solutions, in another embodiment, dichloromethane with an aqueous saturated solution of sodium hydrogen carbonate or sodium carbonate, in a temperature range from -78°C to the boiling point of the respective solvent, in another embodiment the reaction is carried out 0°C to room temperature, to furnish compounds of general formula **(2).** Such transformations reactions have been previously reported (Harris et al., J. Med. Chem., 2005, 48, 1610; Degorce et al., Tetrahedron Lett., 2011, 52, 6719; WO2016/91845 A1; Fairhurst et al., Org. Lett., 2005, 7, 4697; Chaskar et al., Synth. Commun., 2008, 38, 16940; US2004/122237 A1).

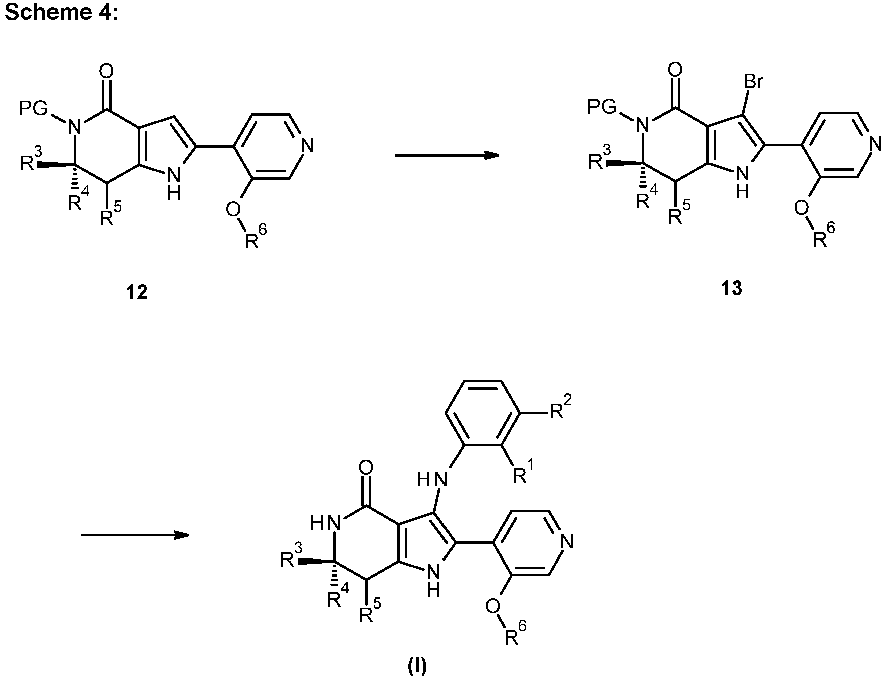

*Scheme 4:* Route for the preparation of compounds of general formula (I), wherein R¹, R², R³, R⁴, R⁵, and R⁶ have the meaning as given for general formula (I) and PG represents hydrogen or a suitable protecting group, *e.g.* tert-butoxycarbonyl (Boc).

Compounds similar to those of general formula **12** are known to those skilled in the art and their syntheses have been reported in the literature (see the teachings of Voss et al., WO2015/22073 A1; Hart et al., WO2016/100166 A1; Anderson et al., J. Med. Chem., 2007, 50, 2647; Vanotti et al., J. Med. Chem., 2008, 51, 487).

Compounds of general formula **(12)** could be converted to compounds of general formula **(13)** using standard bromination methods which are known to those skilled in the art (WO2016/100166 A1). Such brominations could be carried out using a brominating agent, such as, for example, N-bromosuccinimide, in a suitable solvent, such as, for example, DMF, in a temperature range from -78°C to the boiling point of said solvent, in one embodiment the temperature range is 0°C to RT.

Intermediates of general formula **(13)** can be reacted with suitable anilines, such as, for example, 2-difluoromethoxyaniline, in the presence of a base, such as, for example, lithium bis(trimethylsilyl)amide (LHMDS), in the presence of a catalyst, such as, for example a suitable ligand, in one embodiment 2-(di-tert-butylphosphino)-2',4',6'-triisopropyl-3,6-dimethoxy-1,1'-biphenyl (tBuBrettPhos) and in the presence of a pre-catalyst, such as, for example a palladium pre-catalyst, in another embodiment chloro[2-(dicyclohexylphosphino)-3,6-dimethoxy-2',4',6'-triisopropyl-1,1'-biphenyl][2-(2-aminoethyl)phenyl]palladium(II) (BrettPhos-PreCat MTBE ether adduct) in a suitable solvent system, such as, for example, tetrahydrofuran (THF), at a temperature range of 0°C to 200°C. In one embodiment, the reaction is carried out at 80°C, to furnish compounds of general formula (I). Similar transformations have been carried out and have been reported (WO2015/193339 A1).

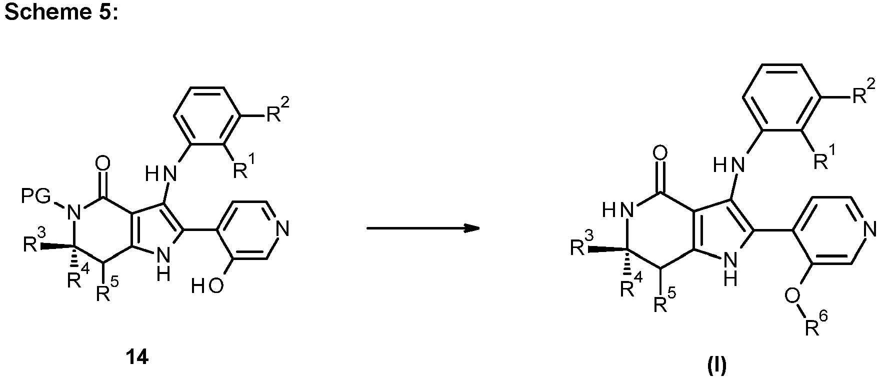

*Scheme 5:* Route for the preparation of compounds of general formula (I), wherein R¹, R², R³, R⁴, R⁵, and R⁶ have the meaning as given for general formula (I) and PG represents hydrogen or a suitable protecting group, *e.g.* tert-butoxycarbonyl (Boc).

Compounds similar to those of general formula **(14)** can be prepared according to the procedure described by Scheme 1 under the use of 4-(aminomethyl)-3-hydroxypyridine instead of intermediate **(4).** Intermediates of general formula **(14)** can be converted to compounds of general formula (I) by reaction with a suitable alcohol under Mitsunobu conditions (the teachings of Oyo Mitsunobu, Synthesis, 1981, 1-28 or Tsunoda et al., Tetrahedron Lett., 1994, 35, 5081) such as, for example oxetan-3-ylmethanol, in the presence of (tributylphosphoranylidene)acetonitrile or triphenylphosphin together with diisopropyl azodicarboxylate in a suitable solvent system, such as, for example, dioxane or THF, at a temperature between room temperature and the boiling point of the respective solvents.

It is known to the person skilled in the art that, if there are a number of reactive centers on a starting or intermediate compound, it may be necessary to block one or more reactive centers temporarily by protective groups in order to allow a reaction to proceed specifically at the desired reaction center.

The compounds according to the invention are isolated and purified in a manner known per se, *e.g.* by distilling off the solvent *in vacuo* and recrystallizing the residue obtained from a suitable solvent or subjecting it to one of the customary purification methods, such as chromatography on a suitable support material. Furthermore, reverse phase preparative HPLC may be applied. The compounds of the present invention which possess a sufficiently basic or acidic functionality, may result as a salt, such as, in the case of a compound of the present invention which is sufficiently basic, a trifluoroacetate or formate salt for example, or, in the case of a compound of the present invention which is sufficiently acidic, an ammonium salt for example. Salts of this type can either be transformed into its free base or free acid form, respectively, by various methods known to the person skilled in the art, or be used as salts in subsequent biological assays. Additionally, the drying process during the isolation of the compounds of the present invention may not fully remove traces of cosolvents, especially such as formic acid or trifluoroacetic acid, to give solvates or inclusion complexes. The person skilled in the art will recognise which solvates or inclusion complexes are acceptable to be used in subsequent biological assays. It is to be understood that the specific form (*e.g.* salt, free base, free acid, solvate, inclusion complex) of a compound of the present invention as isolated and described herein is not necessarily the only form in which said compound can be applied to a biological assay in order to quantify the specific biological activity.

Salts of the compounds of formula (I) according to the invention can be obtained by dissolving the free compound in a suitable solvent (for example a ketone such as acetone, methylethylketone or methyl isobutyl ketone, an ether such as diethyl ether, tetrahydrofuran or dioxane, a chlorinated hydrocarbon such as methylene chloride or chloroform, or a low molecular weight aliphatic alcohol such as methanol, ethanol or isopropanol) which contains the desired acid or base, or to which the desired acid or base is then added. The acid or base can be employed in salt preparation, depending on whether a mono- or polybasic acid or base is concerned and depending on which salt is desired, in an equimolar ratio or one differing therefrom. The salts are obtained by filtering, reprecipitating, precipitating with a non-solvent for the salt or by evaporating the solvent. Salts obtained can be converted into the free compounds which, in turn, can be converted into salts. In this manner, pharmaceutically unacceptable salts, which can be obtained, for example, as process products in the manufacturing on an industrial scale, can be converted into pharmaceutically acceptable salts by processes known to the person skilled in the art. Especially preferred are hydrochlorides and the process used in the example section.

Pure diastereomers and pure enantiomers of the compounds and salts according to the invention can be obtained *e.g.* by asymmetric synthesis, by using chiral starting compounds in synthesis or by splitting up enantiomeric and diasteriomeric mixtures obtained in synthesis.

Enantiomeric and diastereomeric mixtures can be split up into the pure enantiomers and pure diastereomers by methods known to the person skilled in the art. In one embodiment, diastereomeric mixtures are separated by crystallization, in particular fractional crystallization, or chromatography. Enantiomeric mixtures can be separated *e.g.* by forming diastereomers with a chiral auxiliary agent, resolving the diastereomers obtained and removing the chiral auxiliary agent. As chiral auxiliary agents, for example, chiral acids can be used to separate enantiomeric bases such as *e.g.* mandelic acid and chiral bases can be used to separate enantiomeric acids by formation of diastereomeric salts. Furthermore, diastereomeric derivatives such as diastereomeric esters can be formed from enantiomeric mixtures of alcohols or enantiomeric mixtures of acids, respectively, using chiral acids or chiral alcohols, respectively, as chiral auxiliary agents. Additionally, diastereomeric complexes or diastereomeric clathrates may be used for separating enantiomeric mixtures. Alternatively, enantiomeric mixtures can be split up using chiral separating columns in chromatography. Another suitable method for the isolation of enantiomers is the enzymatic separation.

One preferred aspect of the invention is the process for the preparation of the compounds of claims 1-4 according to the examples as well as the intermediates used for their preparation.

Optionally, compounds of the formula (I) can be converted into their salts, or, optionally, salts of the compounds of the formula (I) can be converted into the free compounds. Corresponding processes are customary for the skilled person.

### Commercial utility

As mentioned supra, the compounds of the present invention have surprisingly been found to effectively inhibit mutant EGFR in a cell (*e.g.,* a cancer cell) contacted with the compound, thereby inducing cell death (*e.g.,* apoptosis) and may therefore be used for the treatment or prophylaxis of diseases of uncontrolled cell growth, proliferation and/or survival, inappropriate cellular immune responses, or inappropriate cellular inflammatory responses, or diseases which are accompanied with uncontrolled cell growth, proliferation and/or survival, inappropriate cellular immune responses, or inappropriate cellular inflammatory responses, particularly in which the uncontrolled cell growth, proliferation and/or survival, inappropriate cellular immune responses, or inappropriate cellular inflammatory responses is mediated by mutant EGFR, such as, for example, benign and malignant neoplasia, more specifically haematological tumours, solid tumours, and/or metastases thereof, *e.g.* leukaemias and myelodysplastic syndrome, malignant lymphomas, head and neck tumours including brain tumours and brain metastases, tumours of the thorax including non-small cell and small cell lung tumours, gastrointestinal tumours, endocrine tumours, mammary and other gynaecological tumours, urological tumours including renal, bladder and prostate tumours, skin tumours, and sarcomas, and/or metastases thereof, especially haematological tumours, solid tumours, and/or metastases of breast, bladder, bone, brain, central and peripheral nervous system, cervix, colon, endocrine glands (*e.g.,* thyroid and adrenal cortex), endocrine tumours, endometrium, esophagus, gastrointestinal tumours, germ cells, kidney, liver, lung, larynx and hypopharynx, mesothelioma, ovary, pancreas, prostate, rectum, renal, small intestine, soft tissue, stomach, skin, testis, ureter, vagina and vulva as well as malignant neoplasias including primary tumours in said organs and corresponding secondary tumours in distant organs ("tumour metastases"). Haematological tumours can, e.g., be exemplified by aggressive and indolent forms of leukemia and lymphoma, namely non-Hodgkins disease, chronic and acute myeloid leukemia (CML / AML), acute lymphoblastic leukemia (ALL), Hodgkins disease, multiple myeloma and T-cell lymphoma. Also included are myelodysplastic syndrome, plasma cell neoplasia, paraneoplastic syndromes, and cancers of unknown primary site as well as AIDS related malignancies.

In accordance with an aspect of the present invention therefore the invention relates to a compound of general formula I as defined in the claims hereinfor use in the treatment or prophylaxis of a disease, especially for use in the treatment of a disease.

In accordance with an embodiment as defined in claim 6 herein, the present invention relates to said compound for use, wherein the diseases are hyperproliferative diseases and/or disorders responsive to induction of cell death.

In accordance with an embodiment as defined in claim 7 herein, the present invention relates to said compound for use, wherein the hyperproliferative diseases and/or disorders responsive to induction of cell death are haematological tumours, solid tumours and/or metastases thereof.

In accordance with an embodiment as defined in claim 8 herein, the present invention relates to said compound for use, wherein the tumour harbors a mutant EGFR and/or metastases thereof.

In accordance with an embodiment as defined in claim 9 herein, the present invention relates to said compound for use, wherein the tumour is lung cancer, particularly lung cancer harboring a mutant EGFR with exon 20 insertion mutation, and/or metastases thereof.

In accordance with an embodiment as defined in claim 10 herein, the present invention relates to said compound for use, wherein the tumour is lung cancer, particularly lung cancer harboring a mutant EGFR with in-frame deletions in exon 19 (such as EGFR E746_A750del) or point mutations in exon 21 (e.g. L858R), and/or metastases thereof.

In accordance with an embodiment as defined in claim 11 herein, the present invention relates to said compound for use, wherein the tumour is lung cancer, particularly lung cancer harboring a mutant EGFR with a D770_N771insSVD C797S, E746_A750del C797S, or L858R C797S acquired resistance mutation, and/or metastases thereof.

In accordance with an embodiment as defined in claim 12 herein, the present invention relates to said compound for use, wherein the tumour is lung cancer, particularly lung cancer harboring a mutant ERBB2 with exon 20 insertion mutations (such as ERBB2 A775_G776insYVMA), and/or metastases thereof.

By "hyperproliferative disease" is meant a disease, such as cancer, associated with inappropriately high levels of cell division, inappropriately low levels of apoptosis, or both. The term "inappropriate" within the context of the present invention, in particular in the context of "inappropriate cellular immune responses, or inappropriate cellular inflammatory responses", as used herein, is to be understood as generally meaning a response which is less than, or greater than normal, and which is associated with, responsible for, or results in, the pathology of said diseases.

Hyper-proliferative disorders include psoriasis, keloids, and other hyperplasias affecting the skin, benign prostate hyperplasia (BPH), solid tumours, such as cancers of the breast, respiratory tract, brain, reproductive organs, digestive tract, urinary tract, eye, liver, skin, head and neck, thyroid, parathyroid and their distant metastases. Those disorders also include lymphomas, sarcomas, and leukaemias.

Examples of breast cancer include invasive ductal carcinoma, invasive lobular carcinoma, ductal carcinoma in situ, and lobular carcinoma *in situ.*

Examples of cancers of the respiratory tract small-cell and non-small-cell lung carcinoma, as well as bronchial adenoma and pleuropulmonary blastoma.

Examples of brain cancers include brain stem and hypothalmic glioma, cerebellar and cerebral astrocytoma, medulloblastoma, ependymoma, as well as neuroectodermal and pineal tumour.

Tumours of the male reproductive organs include prostate and testicular cancer. Tumours of the female reproductive organs include endometrial, cervical, ovarian, vaginal, and vulvar cancer, as well as sarcoma of the uterus.

Tumours of the digestive tract include anal, colon, colorectal, oesophageal, gallbladder, gastric, pancreatic, rectal, small-intestine, and salivary gland cancers.

Tumours of the urinary tract include bladder, penile, kidney, renal pelvis, ureter, urethral and human papillary renal cancers.

Eye cancers include intraocular melanoma and retinoblastoma.

Examples of liver cancers include hepatocellular carcinoma (liver cell carcinomas with or without fibrolamellar variant), cholangiocarcinoma (intrahepatic bile duct carcinoma), and mixed hepatocellular cholangiocarcinoma.

Skin cancers include squamous cell carcinoma, Kaposi's sarcoma, malignant melanoma, inverted sinonasal papilloma, inverted sinonasal papilloma-associated sinonasal squamous cell carcinoma, Merkel cell skin cancer, and non-melanoma skin cancer.

Head-and-neck cancers include laryngeal, hypopharyngeal, nasopharyngeal, oropharyngeal cancer, inverted sinonasal papilloma, inverted sinonasal papilloma-associated sinonasal squamous cell carcinoma, lip and oral cavity cancer and squamous cell. Lymphomas include AIDS-related lymphoma, non-Hodgkin's lymphoma, cutaneous T-cell lymphoma, Burkitt lymphoma, Hodgkin's disease, and lymphoma of the central nervous system.

Sarcomas include sarcoma of the soft tissue, osteosarcoma, malignant fibrous histiocytoma, lymphosarcoma, and rhabdomyosarcoma.

Leukemias include acute myeloid leukemia, acute lymphoblastic leukemia, chronic lymphocytic leukemia, chronic myelogenous leukemia, and hairy cell leukemia.

These disorders have been well characterized in humans, but also exist with a similar etiology in other mammals, and can be treated by administering pharmaceutical compositions of the present invention.

The term "treating" or "treatment" as stated throughout this document is used conventionally, e.g., the management or care of a subject for the purpose of combating, alleviating, reducing, relieving, improving the condition of, *etc.,* of a disease or disorder, such as a carcinoma.

### Pharmaceutical compositions of the compounds of the invention

This invention also relates to pharmaceutical compositions containing one or more compounds of the present invention as defined in the claims herein. These compositions can be utilised to achieve the desired pharmacological effect by administration to a patient in need thereof. A patient, for the purpose of this invention, is a mammal, including a human, in need of treatment for the particular condition, disorder, or disease.

Therefore, the present invention includes pharmaceutical compositions that are comprised of a pharmaceutically acceptable carrier or auxiliary and a pharmaceutically effective amount of a compound, or salt thereof, of the present invention.

Another aspect of the invention is a pharmaceutical composition comprising a pharmaceutically effective amount of a compound of formula (I) and a pharmaceutically acceptable auxiliary for the treatment of a disease mentioned supra, especially for the treatment of haematological tumours, solid tumours and/or metastases thereof.

A pharmaceutically acceptable carrier or auxiliary is preferably a carrier that is non-toxic and innocuous to a patient at concentrations consistent with effective activity of the active ingredient so that any side effects ascribable to the carrier do not vitiate the beneficial effects of the active ingredient. Carriers and auxiliaries are all kinds of additives assisting to the composition to be suitable for administration.

A pharmaceutically effective amount of compound is preferably that amount which produces a result or exerts the intended influence on the particular condition being treated.

The compounds of the present invention can be administered with pharmaceutically-acceptable carriers or auxiliaries well known in the art using any effective conventional dosage unit forms, including immediate, slow and timed release preparations, orally, parenterally, topically, nasally, ophthalmically, optically, sublingually, rectally, vaginally, and the like.

For oral administration, the compounds can be formulated into solid or liquid preparations such as capsules, pills, tablets, troches, lozenges, melts, powders, solutions, suspensions, or emulsions, and may be prepared according to methods known to the art for the manufacture of pharmaceutical compositions. The solid unit dosage forms can be a capsule that can be of the ordinary hard- or soft-shelled gelatine type containing auxiliaries, for example, surfactants, lubricants, and inert fillers such as lactose, sucrose, calcium phosphate, and corn starch.

In another embodiment, the compounds of this invention may be tableted with conventional tablet bases such as lactose, sucrose and cornstarch in combination with binders such as acacia, corn starch or gelatine, disintegrating agents intended to assist the break-up and dissolution of the tablet following administration such as potato starch, alginic acid, corn starch, and guar gum, gum tragacanth, acacia, lubricants intended to improve the flow of tablet granulation and to prevent the adhesion of tablet material to the surfaces of the tablet dies and punches, for example talc, stearic acid, or magnesium, calcium or zinc stearate, dyes, colouring agents, and flavouring agents such as peppermint, oil of wintergreen, or cherry flavouring, intended to enhance the aesthetic qualities of the tablets and make them more acceptable to the patient. Suitable excipients for use in oral liquid dosage forms include dicalcium phosphate and diluents such as water and alcohols, for example, ethanol, benzyl alcohol, and polyethylene alcohols, either with or without the addition of a pharmaceutically acceptable surfactant, suspending agent or emulsifying agent. Various other materials may be present as coatings or to otherwise modify the physical form of the dosage unit. For instance tablets, pills or capsules may be coated with shellac, sugar or both.

Dispersible powders and granules are suitable for the preparation of an aqueous suspension. They provide the active ingredient in admixture with a dispersing or wetting agent, a suspending agent and one or more preservatives. Suitable dispersing or wetting agents and suspending agents are exemplified by those already mentioned above. Additional excipients, for example those sweetening, flavouring and colouring agents described above, may also be present.

The pharmaceutical compositions of this invention may also be in the form of oil-in-water emulsions. The oily phase may be a vegetable oil such as liquid paraffin or a mixture of vegetable oils. Suitable emulsifying agents may be (1) naturally occurring gums such as gum acacia and gum tragacanth, (2) naturally occurring phosphatides such as soy bean and lecithin, (3) esters or partial esters derived from fatty acids and hexitol anhydrides, for example, sorbitan monooleate, (4) condensation products of said partial esters with ethylene oxide, for example, polyoxyethylene sorbitan monooleate. The emulsions may also contain sweetening and flavouring agents.

Oily suspensions may be formulated by suspending the active ingredient in a vegetable oil such as, for example, arachis oil, olive oil, sesame oil or coconut oil, or in a mineral oil such as liquid paraffin. The oily suspensions may contain a thickening agent such as, for example, beeswax, hard paraffin, or cetyl alcohol. The suspensions may also contain one or more preservatives, for example, ethyl or n-propyl p-hydroxybenzoate; one or more colouring agents; one or more flavouring agents; and one or more sweetening agents such as sucrose or saccharin.

Syrups and elixirs may be formulated with sweetening agents such as, for example, glycerol, propylene glycol, sorbitol or sucrose. Such formulations may also contain a demulcent, and preservative, such as methyl and propyl parabens and flavouring and colouring agents.

The compounds of this invention may also be administered parenterally, that is, subcutaneously, intravenously, intraocularly, intrasynovially, intramuscularly, or interperitoneally, as injectable dosages of the compound in preferably a physiologically acceptable diluent with a pharmaceutical carrier which can be a sterile liquid or mixture of liquids such as water, saline, aqueous dextrose and related sugar solutions, an alcohol such as ethanol, isopropanol, or hexadecyl alcohol, glycols such as propylene glycol or polyethylene glycol, glycerol ketals such as 2,2-dimethyl-1,1-dioxolane-4-methanol, ethers such as poly(ethylene glycol) 400, an oil, a fatty acid, a fatty acid ester or, a fatty acid glyceride, or an acetylated fatty acid glyceride, with or without the addition of a pharmaceutically acceptable surfactant such as a soap or a detergent, suspending agent such as pectin, carbomers, methycellulose, hydroxypropylmethylcellulose, or carboxymethylcellulose, or emulsifying agent and other pharmaceutical adjuvants.

Illustrative of oils which can be used in the parenteral formulations of this invention are those of petroleum, animal, vegetable, or synthetic origin, for example, peanut oil, soybean oil, sesame oil, cottonseed oil, corn oil, olive oil, petrolatum and mineral oil. Suitable fatty acids include oleic acid, stearic acid, isostearic acid and myristic acid. Suitable fatty acid esters are, for example, ethyl oleate and isopropyl myristate. Suitable soaps include fatty acid alkali metal, ammonium, and triethanolamine salts and suitable detergents include cationic detergents, for example dimethyl dialkyl ammonium halides, alkyl pyridinium halides, and alkylamine acetates; anionic detergents, for example, alkyl, aryl, and olefin sulfonates, alkyl, olefin, ether, and monoglyceride sulfates, and sulfosuccinates; non-ionic detergents, for example, fatty amine oxides, fatty acid alkanolamides, and poly(oxyethylene-oxypropylene)s or ethylene oxide or propylene oxide copolymers; and amphoteric detergents, for example, alkyl-beta-aminopropionates, and 2-alkylimidazoline quarternary ammonium salts, as well as mixtures.

The parenteral compositions of this invention will typically contain from about 0.5% to about 25% by weight of the active ingredient in solution. Preservatives and buffers may also be used advantageously. In order to minimise or eliminate irritation at the site of injection, such compositions may contain a non-ionic surfactant having a hydrophile-lipophile balance (HLB) in one embodiment of from about 12 to about 17. The quantity of surfactant in such formulation in one embodiment ranges from about 5% to about 15% by weight. The surfactant can be a single component having the above HLB or can be a mixture of two or more components having the desired HLB.

Illustrative of surfactants used in parenteral formulations are the class of polyethylene sorbitan fatty acid esters, for example, sorbitan monooleate and the high molecular weight adducts of ethylene oxide with a hydrophobic base, formed by the condensation of propylene oxide with propylene glycol.

The pharmaceutical compositions may be in the form of sterile injectable aqueous suspensions. Such suspensions may be formulated according to known methods using suitable dispersing or wetting agents and suspending agents such as, for example, sodium carboxymethylcellulose, methylcellulose, hydroxypropylmethyl-cellulose, sodium alginate, polyvinylpyrrolidone, gum tragacanth and gum acacia; dispersing or wetting agents which may be a naturally occurring phosphatide such as lecithin, a condensation product of an alkylene oxide with a fatty acid, for example, polyoxyethylene stearate, a condensation product of ethylene oxide with a long chain aliphatic alcohol, for example, heptadeca-ethyleneoxycetanol, a condensation product of ethylene oxide with a partial ester derived form a fatty acid and a hexitol such as polyoxyethylene sorbitol monooleate, or a condensation product of an ethylene oxide with a partial ester derived from a fatty acid and a hexitol anhydride, for example polyoxyethylene sorbitan monooleate.

The sterile injectable preparation may also be a sterile injectable solution or suspension in a non-toxic parenterally acceptable diluent or solvent. Diluents and solvents that may be employed are, for example, water, Ringer's solution, isotonic sodium chloride solutions and isotonic glucose solutions. In addition, sterile fixed oils are conventionally employed as solvents or suspending media. For this purpose, any bland, fixed oil may be employed including synthetic mono- or diglycerides. In addition, fatty acids such as oleic acid can be used in the preparation of injectables.

A composition of the invention may also be administered in the form of suppositories for rectal administration of the drug. These compositions can be prepared by mixing the drug with a suitable non-irritation excipient which is solid at ordinary temperatures but liquid at the rectal temperature and will therefore melt in the rectum to release the drug. Such materials are, for example, cocoa butter and polyethylene glycol.

Controlled release formulations for parenteral administration include liposomal, polymeric microsphere and polymeric gel formulations that are known in the art.

It may be desirable or necessary to introduce the pharmaceutical composition to the patient via a mechanical delivery device. The construction and use of mechanical delivery devices for the delivery of pharmaceutical agents is well known in the art. Direct techniques for administration, for example, administering a drug directly to the brain usually involve placement of a drug delivery catheter into the patient's ventricular system to bypass the blood-brain barrier. One such implantable delivery system, used for the transport of agents to specific anatomical regions of the body, is described in US Patent No. 5,011,472, issued April 30, 1991.

The compositions of the invention can also contain other conventional pharmaceutically acceptable compounding ingredients, generally referred to as carriers or diluents, as necessary or desired. Conventional procedures for preparing such compositions in appropriate dosage forms can be utilized.

Such ingredients and procedures include those described in the following references: Powell, M.F. et al., "Compendium of Excipients for Parenteral Formulations" PDA Journal of Pharmaceutical Science & Technology 1998, 52(5), 238-311; Strickley, R.G "Parenteral Formulations of Small Molecule Therapeutics Marketed in the United States (1999)-Part-1" PDA Journal of Pharmaceutical Science & Technology 1999, 53(6), 324-349; and Nema, S. et al., "Excipients and Their Use in Injectable Products" PDA Journal of Pharmaceutical Science & Technology 1997, 51(4), 166-171.

Commonly used pharmaceutical ingredients that can be used as appropriate to formulate the composition for its intended route of administration include:
acidifying agents (examples include but are not limited to acetic acid, citric acid, fumaric acid, hydrochloric acid, nitric acid);
alkalinizing agents (examples include but are not limited to ammonia solution, ammonium carbonate, diethanolamine, monoethanolamine, potassium hydroxide, sodium borate, sodium carbonate, sodium hydroxide, triethanolamine, trolamine);
adsorbents (examples include but are not limited to powdered cellulose and activated charcoal);
aerosol propellants (examples include but are not limited to carbon dioxide, CCl₂F₂, F₂ClC-CClF₂ and CClF₃);
air displacement agents (examples include but are not limited to nitrogen and argon);
antifungal preservatives (examples include but are not limited to benzoic acid, butylparaben, ethylparaben, methylparaben, propylparaben, sodium benzoate);
antimicrobial preservatives (examples include but are not limited to benzalkonium chloride, benzethonium chloride, benzyl alcohol, cetylpyridinium chloride, chlorobutanol, phenol, phenylethyl alcohol, phenylmercuric nitrate and thimerosal);
antioxidants (examples include but are not limited to ascorbic acid, ascorbyl palmitate, butylated hydroxyanisole, butylated hydroxytoluene, hypophosphorus acid, monothioglycerol, propyl gallate, sodium ascorbate, sodium bisulfite, sodium formaldehyde sulfoxylate, sodium metabisulfite);
binding materials (examples include but are not limited to block polymers, natural and synthetic rubber, polyacrylates, polyurethanes, silicones, polysiloxanes and styrenebutadiene copolymers);
buffering agents (examples include but are not limited to potassium metaphosphate, dipotassium phosphate, sodium acetate, sodium citrate anhydrous and sodium citrate dihydrate);
carrying agents (examples include but are not limited to acacia syrup, aromatic syrup, aromatic elixir, cherry syrup, cocoa syrup, orange syrup, syrup, corn oil, mineral oil, peanut oil, sesame oil, bacteriostatic sodium chloride injection and bacteriostatic water for injection);
chelating agents (examples include but are not limited to edetate disodium and edetic acid);
colourants (examples include but are not limited to FD&C Red No. 3, FD&C Red No. 20, FD&C Yellow No. 6, FD&C Blue No. 2, D&C Green No. 5, D&C Orange No. 5, D&C Red No. 8, caramel and ferric oxide red);
clarifying agents (examples include but are not limited to bentonite);
emulsifying agents (examples include but are not limited to acacia, cetomacrogol, cetyl alcohol, glyceryl monostearate, lecithin, sorbitan monooleate, polyoxyethylene 50 monostearate);
encapsulating agents (examples include but are not limited to gelatin and cellulose acetate phthalate);
flavourants (examples include but are not limited to anise oil, cinnamon oil, cocoa, menthol, orange oil, peppermint oil and vanillin);
humectants (examples include but are not limited to glycerol, propylene glycol and sorbitol);
levigating agents (examples include but are not limited to mineral oil and glycerin);
oils (examples include but are not limited to arachis oil, mineral oil, olive oil, peanut oil, sesame oil and vegetable oil);
ointment bases (examples include but are not limited to lanolin, hydrophilic ointment, polyethylene glycol ointment, petrolatum, hydrophilic petrolatum, white ointment, yellow ointment, and rose water ointment);
penetration enhancers (transdermal delivery) (examples include but are not limited to monohydroxy or polyhydroxy alcohols, mono-or polyvalent alcohols, saturated or unsaturated fatty alcohols, saturated or unsaturated fatty esters, saturated or unsaturated dicarboxylic acids, essential oils, phosphatidyl derivatives, cephalin, terpenes, amides, ethers, ketones and ureas);
plasticizers (examples include but are not limited to diethyl phthalate and glycerol);
solvents (examples include but are not limited to ethanol, corn oil, cottonseed oil, glycerol, isopropanol, mineral oil, oleic acid, peanut oil, purified water, water for injection, sterile water for injection and sterile water for irrigation);
stiffening agents (examples include but are not limited to cetyl alcohol, cetyl esters wax, microcrystalline wax, paraffin, stearyl alcohol, white wax and yellow wax);
suppository bases (examples include but are not limited to cocoa butter and polyethylene glycols (mixtures));
surfactants (examples include but are not limited to benzalkonium chloride, nonoxynol 10, oxtoxynol 9, polysorbate 80, sodium lauryl sulfate and sorbitan mono-palmitate);
suspending agents (examples include but are not limited to agar, bentonite, carbomers, carboxymethylcellulose sodium, hydroxyethyl cellulose, hydroxypropyl cellulose, hydroxypropyl methylcellulose, kaolin, methylcellulose, tragacanth and veegum);
sweetening agents (examples include but are not limited to aspartame, dextrose, glycerol, mannitol, propylene glycol, saccharin sodium, sorbitol and sucrose);
tablet anti-adherents (examples include but are not limited to magnesium stearate and talc);
tablet binders (examples include but are not limited to acacia, alginic acid, carboxymethylcellulose sodium, compressible sugar, ethylcellulose, gelatin, liquid glucose, methylcellulose, non-crosslinked polyvinyl pyrrolidone, and pregelatinized starch);
tablet and capsule diluents (examples include but are not limited to dibasic calcium phosphate, kaolin, lactose, mannitol, microcrystalline cellulose, powdered cellulose, precipitated calcium carbonate, sodium carbonate, sodium phosphate, sorbitol and starch);
tablet coating agents (examples include but are not limited to liquid glucose, hydroxyethyl cellulose, hydroxypropyl cellulose, hydroxypropyl methylcellulose, methylcellulose, ethylcellulose, cellulose acetate phthalate and shellac);
tablet direct compression excipients (examples include but are not limited to dibasic calcium phosphate);
tablet disintegrants (examples include but are not limited to alginic acid, carboxymethylcellulose calcium, microcrystalline cellulose, polacrillin potassium, crosslinked polyvinylpyrrolidone, sodium alginate, sodium starch glycollate and starch);
tablet glidants (examples include but are not limited to colloidal silica, corn starch and talc);
tablet lubricants (examples include but are not limited to calcium stearate, magnesium stearate, mineral oil, stearic acid and zinc stearate);
tablet/capsule opaquants (examples include but are not limited to titanium dioxide);
tablet polishing agents (examples include but are not limited to carnuba wax and white wax);
thickening agents (examples include but are not limited to beeswax, cetyl alcohol and paraffin);
tonicity agents (examples include but are not limited to dextrose and sodium chloride);
viscosity increasing agents (examples include but are not limited to alginic acid, bentonite, carbomers, carboxymethylcellulose sodium, methylcellulose, polyvinyl pyrrolidone, sodium alginate and tragacanth); and
wetting agents (examples include but are not limited to heptadecaethylene oxycetanol, lecithins, sorbitol monooleate, polyoxyethylene sorbitol monooleate, and polyoxyethylene stearate).

Pharmaceutical compositions according to the present invention can be illustrated as follows:
Sterile i.v. solution: A 5 mg/ml solution of the desired compound of this invention can be made using sterile, injectable water, and the pH is adjusted if necessary. The solution is diluted for administration to 1 - 2 mg/ml with sterile 5% dextrose and is administered as an i.v. infusion over about 60 minutes.
Lyophilised powder for i.v. administration: A sterile preparation can be prepared with (i) 100 - 1000 mg of the desired compound of this invention as a lyophilised powder, (ii) 32-327 mg/ml sodium citrate, and (iii) 300 - 3000 mg Dextran 40. The formulation is reconstituted with sterile, injectable saline or dextrose 5% to a concentration of 10 to 20 mg/ml, which is further diluted with saline or dextrose 5% to 0.2 - 0.4 mg/ml, and is administered either IV bolus or by IV infusion over 15 - 60 minutes.
Intramuscular suspension: The following solution or suspension can be prepared, for intramuscular injection:
   50 mg/ml of the desired, water-insoluble compound of this invention
   5 mg/ml sodium carboxymethylcellulose
   4 mg/ml TWEEN 80
   9 mg/ml sodium chloride
   9 mg/ml benzyl alcohol
Hard Shell Capsules: A large number of unit capsules are prepared by filling standard two-piece hard galantine capsules each with 100 mg of powdered active ingredient, 150 mg of lactose, 50 mg of cellulose and 6 mg of magnesium stearate.
Soft Gelatin Capsules: A mixture of active ingredient in a digestible oil such as soybean oil, cottonseed oil or olive oil is prepared and injected by means of a positive displacement pump into molten gelatin to form soft gelatin capsules containing 100 mg of the active ingredient. The capsules are washed and dried. The active ingredient can be dissolved in a mixture of polyethylene glycol, glycerin and sorbitol to prepare a water miscible medicine mix.
Tablets: A large number of tablets are prepared by conventional procedures so that the dosage unit is 100 mg of active ingredient, 0.2 mg. of colloidal silicon dioxide, 5 mg of magnesium stearate, 275 mg of microcrystalline cellulose, 11 mg. of starch, and 98.8 mg of lactose. Appropriate aqueous and non-aqueous coatings may be applied to increase palatability, improve elegance and stability or delay absorption.
Immediate Release Tablets/Capsules: These are solid oral dosage forms made by conventional and novel processes. These units are taken orally without water for immediate dissolution and delivery of the medication. The active ingredient is mixed in a liquid containing ingredient such as sugar, gelatin, pectin and sweeteners. These liquids are solidified into solid tablets or caplets by freeze drying and solid state extraction techniques. The drug compounds may be compressed with viscoelastic and thermoelastic sugars and polymers or effervescent components to produce porous matrices intended for immediate release, without the need of water.

### Dose and administration

Based upon standard laboratory techniques known to evaluate compounds useful for the treatment of hyper-proliferative disorders and angiogenic disorders, by standard toxicity tests and by standard pharmacological assays for the determination of treatment of the conditions identified above in mammals, and by comparison of these results with the results of known medicaments that are used to treat these conditions, the effective dosage of the compounds of this invention can readily be determined for treatment of each desired indication. The amount of the active ingredient to be administered in the treatment of one of these conditions can vary widely according to such considerations as the particular compound and dosage unit employed, the mode of administration, the period of treatment, the age and sex of the patient treated, and the nature and extent of the condition treated.

The total amount of the active ingredient to be administered will generally range from about 0.001 mg/kg to about 200 mg/kg body weight per day, and in particular embodiments from about 0.01 mg/kg to about 20 mg/kg body weight per day. Clinically useful dosing schedules will range from one to three times a day dosing to once every four weeks dosing. In addition, "drug holidays" in which a patient is not dosed with a drug for a certain period of time, may be beneficial to the overall balance between pharmacological effect and tolerability. A unit dosage may contain from about 0.5 mg to about 1500 mg of active ingredient, and can be administered one or more times per day or less than once a day. The average daily dosage for administration by injection, including intravenous, intramuscular, subcutaneous and parenteral injections, and use of infusion techniques will in other embodiments be from 0.01 to 200 mg/kg of total body weight. The average daily rectal dosage regimen will in particular embodiments be from 0.01 to 200 mg/kg of total body weight. The average daily vaginal dosage regimen will in other embodiments be from 0.01 to 200 mg/kg of total body weight. The average daily topical dosage regimen will in still other embodiments be from 0.1 to 200 mg administered between one to four times daily. The transdermal concentration will in other embodiments be that required to maintain a daily dose of from 0.01 to 200 mg/kg. The average daily inhalation dosage regimen will in other embodiments be from 0.01 to 100 mg/kg of total body weight.

Of course the specific initial and continuing dosage regimen for each patient will vary according to the nature and severity of the condition as determined by the attending diagnostician, the activity of the specific compound employed, the age and general condition of the patient, time of administration, route of administration, rate of excretion of the drug, drug combinations, and the like. The desired mode of treatment and number of doses of a compound of the present invention or a pharmaceutically acceptable salt or ester or composition thereof can be ascertained by those skilled in the art using conventional treatment tests.

### Combination Therapies

The compounds of this invention can be administered as the sole pharmaceutical agent or in combination with one or more other pharmaceutical agents where the combination causes no unacceptable adverse effects. Those combined pharmaceutical agents can be other agents having antiproliferative effects such as for example for the treatment of haematological tumours, solid tumours and/or metastases thereof and/or agents for the treatment of undesired side effects. The present invention relates also to such combinations.

Other anti-hyper-proliferative agents suitable for use with the composition of the invention include but are not limited to those compounds acknowledged to be used in the treatment of neoplastic diseases in Goodman and Gilman's The Pharmacological Basis of Therapeutics (Ninth Edition), editor Molinoff et al., publ. by McGraw-Hill, pages 1225-1287, (1996) especially (chemotherapeutic) anti-cancer agents as defined supra. The combination can be a non-fixed combination or a fixed-dose combination as the case may be.

Methods of testing for a particular pharmacological or pharmaceutical property are well known to persons skilled in the art.

The example testing experiments described herein serve to illustrate the present invention.

As will be appreciated by persons skilled in the art, the invention is as defined by the appended claims.

The following examples illustrate the invention in greater detail. Further compounds according to the invention, of which the preparation is not explicitly described, can be prepared in an analogous way.

The term "according to" within the experimental section is used in the sense that the procedure referred to is to be used "analogously to".

### EXPERIMENTAL SECTION

Chemical names were generated using the ACD/Name software from ACD/Labs. In some cases generally accepted names of commercially available reagents were used in place of ACD/Name generated names.

The following table 1 lists the abbreviations used in this paragraph and in the Examples section as far as they are not explained within the text body. Other abbreviations have their meanings customary *per se* to the skilled person.

**Table 1: Abbreviations**

| **Abbreviation** | **Meaning** |
|---|---|
| ACN | Acetonitrile |
| AcOH | acetic acid |
| aq. | Aqueous |
| br | broad signal (NMR) |
| d | doublet (NMR) |
| DAD | Diode Array Detector |
| DAST | Diethylaminosulfur trifluoride |
| DBU | 1,8-Diazabicyclo(5.4.0)undec-7-ene |
| DCM | Dichloromethane |
| dd | doublet of doublet (NMR) |
| DIPEA | Diisopropylethylamine |
| DMA | N,N-dimethylacetamide |
| DMAP | 4-Dimethylaminopyridine |
| DMF | N,N-dimethylformamide |
| DMSO | Dimethylsulfoxide |
| EDC.HCl | N-(3-Dimethylaminopropyl)-N'-ethylcarbodiimide hydrochloride salt |
| ESI | electrospray (ES) ionization |
| EtOAc | Ethyl acetate |
| EtOH | Ethanol |
| h, hr (hrs) | hour(s) |
| HCl | hydrogen chloride, hydrochloric acid |
| HPLC | high performance liquid chromatography |
| LC-MS | liquid chromatography-mass spectrometry |
| m | multiplet (NMR) |
| mCPBA | meta-Chloroperoxybenzoic acid |
| MeCN | Acetonitrile |
| MeOH | Methanol |
| min | minute(s) |
| MS | mass spectrometry |
| MTBE | Methyl-*tert*-butylether |
| MWD | Multiple wavelength detector |
| NaCL | Sodium chloride |
| NMR | Nuclear Magnetic Resonance spectroscopy : chemical shifts (δ) are given in ppm. The chemical shifts were corrected by setting the DMSO signal to 2.50 ppm using unless otherwise stated. |
| q | quartet (NMR) |
| RT | room temperature |
| Rₜ | retention time |
| s | singulet (NMR) |
| sat. | Saturated |
| SFC | Supercritical Fluid Chromatography |
| t | triplet (NMR) |
| td | triplet of doublet (NMR) |
| TEA | Triethylamine |
| TFA | Trifluoroacetic acid |
| THF | Tetrahydrofuran |
| δ | chemical shift |

Other abbreviations have their meanings customary *per se* to the skilled person.

The various aspects of the invention described in this application are illustrated by the following examples.

The example testing experiments described herein serve to illustrate the present invention.

### EXPERIMENTAL SECTION - GENERAL PART

All reagents, for which the synthesis is not described in the experimental part, are either commercially available, or are known compounds or may be formed from known compounds by known methods by a person skilled in the art.

The compounds and intermediates produced according to the methods of the invention may require purification. Purification of organic compounds is well known to the person skilled in the art and there may be several ways of purifying the same compound. In some cases, no purification may be necessary. In some cases, the compounds may be purified by crystallization. In some cases, impurities may be removed by trituration using a suitable solvent. In some cases, the compounds may be purified by chromatography, particularly flash column chromatography, using for example prepacked silica gel cartridges, e.g. Biotage SNAP cartridges KP-Sil^{®} or KP-NH^{®} in combination with a Biotage autopurifier system (SP4^{®} or Isolera Four^{®}) and eluents such as gradients of hexane/ethyl acetate or DCM/methanol. In flash column chromatography, unmodified ("regular") silica gel may be used as well as aminophase functionalized silica gel. If reference is made to flash column chromatography or to flash chromatography in the experimental section without specification of a stationary phase, regular silica gel was used.

In some cases, the compounds may be purified by preparative HPLC using for example a Waters autopurifier equipped with a diode array detector and/or on-line electrospray ionization mass spectrometer in combination with a suitable prepacked reverse phase column and eluents such as gradients of water and acetonitrile which may contain additives such as trifluoroacetic acid, formic acid or aqueous ammonia.

In some cases, purification methods as described above can provide those compounds of the present invention which possess a sufficiently basic or acidic functionality in the form of a salt, such as, in the case of a compound of the present invention which is sufficiently basic, a trifluoroacetate or formate salt for example, or, in the case of a compound of the present invention which is sufficiently acidic, an ammonium salt for example. A salt of this type can either be transformed into its free base or free acid form, respectively, by various methods known to the person skilled in the art, or be used as salts in subsequent biological assays. It is to be understood that the specific form (e.g. salt, free base etc.) of a compound of the present invention as isolated and as described herein is not necessarily the only form in which said compound can be applied to a biological assay in order to quantify the specific biological activity.

### Analytical LC-MS Methods:

### Method 1:

Instrument: Waters Acquity UPLCMS SingleQuad; Column: Acquity UPLC BEH C18 1.7 µm, 50x2.1mm; eluent A: water + 0.1 vol. % formic acid (99 %), eluent B: acetonitrile; gradient: 0-1.6 min. 1-99 % B, 1.6-2.0 min. 99 % B; flow 0.8 ml/min; temperature: 60 °C; DAD scan: 210-400 nm.

### Method 2:

Instrument: Waters Acquity UPLCMS SingleQuad; Column: Acquity UPLC BEH C18 1.7 µm, 50 x 2.1mm; eluent A: water + 0.2 vol.% aqueous ammonia (32%), eluent B: acetonitrile; gradient: 0-1.6 min. 1-99% B, 1.6-2.0 min. 99% B; flow 0.8 ml/min; temperature: 60 °C; DAD scan: 210-400 nm.

### Method 3:

Instrument: SHIMADZU LCMS-2020; Column: Kinetex EVO C18 30x2.1mm, 5µm, eluent A: 0.0375% TFA in water, eluent B: 0.01875% TFA in acetonitrile, gradient: 0.0-0.80 min 5-95% B 0.80-1.20 min 95% B; flow 1.5 mL/min, temperature: 50°C; UV detection: 220 nm & 254 nm)

### Method 4:

Instrument: SHIMADZU LC-20AB; Column: Kinetex C18 LC Column 4.6x50mm, 5µm, eluent A: 0.025% ammonia in water, eluent B: acetonitrile; gradient: 0.0-2.40 min 10-80% B, 2.40-3.30 min 80% B; flow 1.5 mL/min, temperature: 50 °C; UV detection: 220 nm & 215 nm & 254 nm

### Method 5:

MS instrument type: Agilent 1200\ G1956A; column: Kinetex EVO C18 30x2.1mm, 5µm; eluent A: 0.0375% TFA in water (v/v), eluent B: 0.01875% TFA in acetonitrile: gradient: 0.0-3.00 min 0-60% B, 3.00-3.50 min 60% B; flow rate: 0.8 mL/mix; oven temperature: 50 °C; UV detection: 220 nm & 254 nm.

### Method 6:

Instrument: Agilent 1290 UPLCMS 6230 TOF; Säule: BEH C 18 1.7 µm, 50x2.1mm; Eluent A: Wasser + 0.05 % Ameisensäure (99%); Eluent B: Acetonitril + 0.05 % Ameisensäure (99%); Gradient: 0-1.7 2-90% B, 1.7-2.0 90% B; Fluss 1.2 ml/min; Temperatur: 60°C; DAD scan: 190-400 nm.

### Preparative LC-MS Methods:

### Method 7:

Instrument: Waters Autopurification MS SingleQuad; Column: Waters XBrigde C18 5µ 100x30mm; eluent A: water + 0.2 vol. % aqueous ammonia (32 %), eluent B: acetonitrile; gradient: 0-5.5 min. 5-100 % B; flow 70 ml/min; temperature: 25 °C; DAD scan: 210-400 nm

### Method 8:

Instrument: Waters Autopurification MS SingleQuad; Column: Waters XBrigde C18 5µ 50x50mm; eluent A: water + 0.1 vol% formic acid, eluent B: methanol; gradient: 0-0.50 min. 20 % B; flow 50 to 100 ml/min, 0.50-8.00 min. 20 - 60% B; flow 100 ml/min, temperature: 25 °C; DAD scan: 210-400 nm

### Method 9:

Instrument: Labomatic HD-5000, pump head HDK-280, gradient module NDB-1000, fraction collector Labomatic Labocol Vario 2000, Knauer UV detector Azura UVD 2.1S, Prepcon 5 software. Column: Chromatorex C18 10µM 120x30 mm; Eluent A: water + 0.1% formic acid; Eluent B: acetonitrile; gradient: given for intermediates and examples, rate 150 mL/min, temperature 25°C.; UV 220 nm

### Method 10 :

Instrument: Labomatic HD-5000, pump head HDK-280, gradient module NDB-1000, fraction collector Labomatic Labocol Vario 2000, Knauer UV detector Azura UVD 2.1S, Prepcon 5 software. Column: Chromatorex C18 10µM 120x30 mm; Eluent A: 0.1% ammonia in water; Eluent B: acetonitrile; gradient: given for intermediates and examples, rate 150 mL/min, temperature 25°C.; UV 220 nm

### Method 11 :

Instrument: Labomatic HD-5000, pump head HDK-280, gradient module NDB-1000, fraction collector Labomatic Labocol Vario 2000, Knauer UV detector Azura UVD 2.1S, Prepcon 5 software. Column: Chromatorex C18 10µM 300x50 mm; Eluent A: 0.1% ammonia in water; Eluent B: acetonitrile; gradient: given for intermediates and examples, rate 250 mL/min, temperature 25°C.; UV 220 nm

### NMR Spectra:

The multiplicities of proton signals in ¹H NMR spectra given in the following paragraphs reflect the observed signal form and do not take into account any higher-order signal phenomena. As a rule, the chemical shift data refers to the center of the signal in question. In the case of wide multiplets, a range is specified. Signals hidden by solvent or water were either assigned tentatively or are not listed. Strongly broadened signals - *e.g.* caused by rapid rotation of molecular moieties or by interchanging protons - have also been assigned tentatively (often referred to as a broad multiplet or broad singlet) or are not shown.

The ¹H-NMR data of selected compounds are listed in the form of ¹H-NMR peaklists. Therein, for each signal peak the δ value in ppm is given, followed by the signal intensity, reported in round brackets. The δ value-signal intensity pairs from different peaks are separated by commas. Therefore, a peaklist is described by the general form: δ₁ (intensity₁), δ₂ (intensity₂), ... , δᵢ (intensityᵢ), ... , δₙ (intensityₙ).

The intensity of a sharp signal correlates with the height (in cm) of the signal in a printed NMR spectrum. When compared with other signals, this data can be correlated to the real ratios of the signal intensities. In the case of broad signals, more than one peak, or the center of the signal along with their relative intensity, compared to the most intense signal displayed in the spectrum, are shown. A ¹H-NMR peaklist is similar to a classical ¹H-NMR readout, and thus usually contains all the peaks listed in a classical NMR interpretation. Moreover, similar to classical ¹H-NMR printouts, peaklists can show solvent signals, signals derived from stereoisomers of the particular target compound, peaks of impurities, ¹³C satellite peaks, and/or spinning sidebands. The peaks of stereoisomers, and/or peaks of impurities are typically displayed with a lower intensity compared to the peaks of the target compound (e.g., with a purity of >90%). Such stereoisomers and/or impurities may be typical for the particular manufacturing process, and therefore their peaks may help to identify a reproduction of the manufacturing process on the basis of "by-product fingerprints". An expert who calculates the peaks of the target compound by known methods (MestReC, ACD simulation, or by use of empirically evaluated expectation values), can isolate the peaks of the target compound as required, optionally using additional intensity filters. Such an operation would be similar to peak-picking in classical ¹H-NMR interpretation. A detailed description of the reporting of NMR data in the form of peaklists can be found in the publication "Citation of NMR Peaklist Data within Patent Applications" (cf. http://www.researchdisclosure.com/searching-disclosures, Research Disclosure Database Number 605005, 2014, 01 Aug 2014). In the peak picking routine, as described in the Research Disclosure Database Number 605005, the parameter "MinimumHeight" can be adjusted between 1% and 4%. However, depending on the chemical structure and/or depending on the concentration of the measured compound it may be reasonable to set the parameter "MinimumHeight" <1%.

### Syntheses of Intermediate 1 Compounds

### Intermediate 1-1

### 3-(2-methoxy-2-methylpropoxy)pyridine-4-carbonitrile

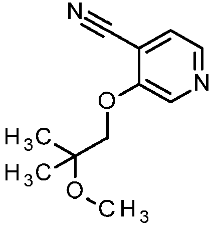

2-Methoxy-2-methylpropan-1-ol (16.5 g, 159 mmol, CAS 22665-67-4) was dissolved in THF (640 ml), cooled to 0 °C and sodium hydride (6.93 g, 60 % purity, 173 mmol) was added. The mixture was stirred for 1 h at RT. 3-chloropyridine-4-carbonitrile (20.0 g, 144 mmol, CAS 68325-15-5) dissolved in THF (130 ml) was added dropwise and the mixture was stirred overnight at RT. The mixture was quenched with sat. ammonium chloride solution and extracted 2 times with EtOAc. The combined organic layers were dried and concentrated under reduced pressure. The residue was purified by flash chromatography (amino phase silica, hexane / EtOAc gradient 20-60 %) to give 23.3 g (90% purity, 70% yield) of the title compound.

¹H-NMR (400 MHz, DMSO-d₆) δ [ppm]: 1.24 (s, 6H), 3.17 (s, 3H), 4.19 (s, 2H), 7.78 (d, 1H), 8.38 (d, 1H), 8.72 (s, 1H).

### Intermediate 1-3

### 3-[(2S)-tetrahydrofuran-2-ylmethoxy]isonicotinonitrile

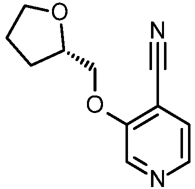

According to the method described for Intermediate 1-1 using 3-chloropyridine-4-carbonitrile (1.36 g, 9.79 mmol, CAS 68325-15-5) and (2S)-tetrahydrofuran-2-ylmethanol (1.00 g, 9.79 mmol, CAS 57203-01-7) as starting materials, 1.71 g (99% purity, 85% yield) of the title compound were prepared.

¹H-NMR (400 MHz, DMSO-d₆) δ [ppm]: 1.154 (2.31), 1.172 (4.65), 1.190 (2.40), 1.700 (0.67), 1.716 (1.62), 1.720 (1.19), 1.729 (1.38), 1.731 (1.35), 1.736 (2.07), 1.745 (2.56), 1.749 (1.39), 1.753 (1.52), 1.761 (1.81), 1.765 (2.68), 1.782 (1.96), 1.788 (0.78), 1.805 (1.06), 1.817 (1.15), 1.824 (1.48), 1.833 (1.88), 1.838 (1.69), 1.841 (1.45), 1.854 (2.48), 1.871 (2.12), 1.888 (1.38), 1.902 (1.61), 1.909 (0.84), 1.919 (2.04), 1.934 (1.89), 1.938 (1.69), 1.949 (1.19), 1.952 (1.13), 1.955 (1.41), 1.964 (0.76), 1.969 (1.02), 1.983 (2.72), 1.987 (9.66), 2.000 (2.04), 2.010 (1.89), 2.015 (1.57), 2.020 (1.52), 2.024 (1.46), 2.031 (1.86), 2.043 (1.46), 2.049 (1.19), 2.062 (0.82), 2.331 (0.56), 2.518 (3.41), 2.522 (2.11), 2.673 (0.58), 3.664 (1.92), 3.684 (3.84), 3.700 (4.51), 3.702 (3.81), 3.718 (2.78), 3.769 (2.68), 3.785 (5.28), 3.789 (2.72), 3.802 (3.67), 3.806 (3.47), 3.822 (1.96), 3.999 (0.68), 4.017 (2.07), 4.035 (2.07), 4.053 (0.70), 4.189 (0.87), 4.198 (1.10), 4.204 (1.82), 4.213 (2.20), 4.220 (2.49), 4.229 (2.98), 4.238 (1.78), 4.244 (6.81), 4.257 (1.50), 4.269 (8.40), 4.283 (5.50), 4.319 (6.52), 4.327 (6.36), 4.344 (3.74), 4.353 (2.58), 7.770 (8.92), 7.782 (9.31), 8.373 (11.59), 8.385 (11.19), 8.708 (16.00).

### Intermediate 1-6

### 3-{[(2S)-oxetan-2-yl]methoxy}pyridine-4-carbonitrile

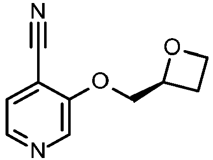

3-Chloropyridine-4-carbonitrile (27.1 g, 195 mmol, CAS 68325-15-5) and [(2S)-oxetan-2-yl]methanol (18.6 g, 211 mmol, CAS 2090778-00-8) were dissolved in THF (350 ml). Potassium *tert*-butoxide (26.32 g, 234 mmol) was added and the mixture was stirred for 2 h at RT. The reaction mixture was diluted with cooled sat. ammonium chloride solution and extracted with EtOAc. The organic phase was washed with brine and dried, concentrated under reduced pressure and purified by flash chromatography (amine functionalized silica KP-NH^{™}, hexane / EtOAc gradient 0-100%) to give 22.9 g (62% yield) of the title compound.

LC-MS (method 2): Rₜ = 0.68 min; MS (ESlpos): m/z = 191 [M+H]^{+ 1}H-NMR (400 MHz, DMSO-d₆) δ [ppm]: 8.75 (s, 1H), 8.40 (d, 1H), 7.75 - 7.85 (m, 1H), 5.05 (ddt, 1H), 4.50 - 4.59 (m, 2H), 4.46 (d, 2H), 2.57 - 2.82 (m, 2H).

### Intermediate 1-11

### tert-butyl (2S)-2-{[(4-cyanopyridin-3-yl)oxy]methyl}morpholine-4-carboxylate

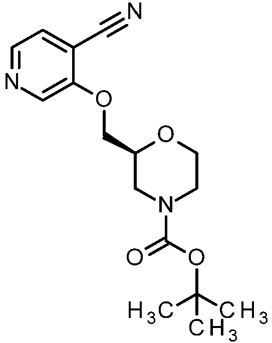

According to the method described for Intermediate 1-6 using 3-chloropyridine-4-carbonitrile (2.55 g, 18.4 mmol, CAS 68325-15-5) and *tert-butyl* (2S)-2-(hydroxymethyl)morpholine-4-carboxylate (4.00 g, 18.4 mmol, CAS 135065-76-8) as starting materials, 4.82 g (90% purity, 74% yield) of the title compound were prepared.

LC-MS (method 2): Rₜ = 1.06 min; MS (ESlpos): m/z = 320 [M+H]⁺

¹H-NMR (400 MHz, DMSO-d₆) δ [ppm]: 1.41 (s, 9H), 2.72 - 3.05 (m, 2H), 3.46 (br d, 1H), 3.67 - 3.82 (m, 2H), 3.82 - 3.88 (m, 1H), 4.00 (s, 1H), 4.37 (br d, 2H), 7.79 (d, 1H), 8.40 (d, 1H), 8.73 (s, 1H).

### Intermediate 1-19

### 3-[(2-methyloxetan-2-yl)methoxy]pyridine-4-carbonitrile3-[(methyloxetan-2-yl)methoxy]pyridine-4-carbonitrile

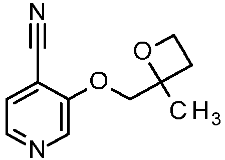

According to the method described for Intermediate 1-6 using 3-chloropyridine-4-carbonitrile (1.23 g, 8.90 mmol, CAS 68325-15-5) and (2-methyloxetan-2-yl)methanol (1.00 g, 9.79 mmol, CAS 61266-71-5) as starting materials; 1.30 g (68% yield) of the title compound were prepared after purification by flash chromatography (silica, hexane / EtOAc gradient 0-100%, EtOAc / EtOH gradient 0-35%).

¹H-NMR (400 MHz, DMSO-d₆) δ [ppm]: 8.71 - 8.82 (m, 1H), 8.40 (d, 1H), 7.75 - 7.82 (m, 1H), 4.35 - 4.50 (m, 1H), 4.33 - 4.49 (m, 1H), 4.24 - 4.50 (m, 2H), 2.68 - 2.77 (m, 1H), 2.40 - 2.47 (m, 1H), 1.41 - 1.47 (m, 3H).

### Intermediate 1-24

### 3-[(1,4-dioxan-2-yl)methoxy]pyridine-4-carbonitrile

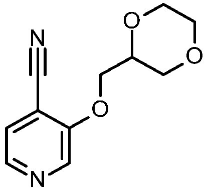

3-Chloropyridine-4-carbonitrile (CAS 68325-15-5, 1.40 g, 10.1 mmol) and (1,4-dioxan-2-yl)methanol (CAS 143669-41-4, 1.31 g, 11.1 mmol) were dissolved in THF (45 ml). Potassium tert-butoxide (1.03 g, 9.14 mmol) was added and the mixture was stirred for 1 h at 0°C. The reaction mixture was diluted slowly with sat. ammonium chloride solution and extracted with EtOAc (3x). The organic phase was washed with brine and filtered over a water-repellent filter, concentrated under reduced pressure and purified by flash chromatography (silica, hexane / EtOAc gradient 0-100 %; EtOAc / EtOH gradient 0-35%) to give 1.18 g of the title compound (53% yield).

LC-MS (method 2): Rₜ = 0.77 min; MS (ESlpos): m/z = 221 [M+H]⁺

¹H-NMR (400 MHz, DMSO-d₆) δ [ppm]: 8.72 (s, 1H), 8.23 - 8.46 (m, 1H), 7.68 - 7.86 (m, 1H), 4.32 (d, 2H), 3.40 - 3.99 (m, 7H).

### Intermediate 1-27

### 3-(tetrahydro-2H-pyran-2-ylmethoxy)isonicotinonitrile

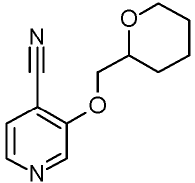

According to the method described for Intermediate 1-1 using 3-chloropyridine-4-carbonitrile (4.68 g, 33.7 mmol, CAS 68325-15-5) and tetrahydro-2*H*-pyran-2-ylmethanol (4.00 g, 98% purity, 33.7 mmol, CAS 100-72-1) as starting materials, 4.44 g (99% purity, 60% yield) of the title compound were prepared.

¹H-NMR (400 MHz, DMSO-d₆) δ [ppm]: 1.317 (0.67), 1.327 (0.58), 1.347 (2.01), 1.357 (1.66), 1.376 (2.31), 1.379 (2.27), 1.385 (2.15), 1.399 (0.91), 1.407 (2.07), 1.416 (2.07), 1.422 (2.00), 1.432 (2.43), 1.438 (1.54), 1.454 (2.00), 1.463 (2.87), 1.473 (3.60), 1.481 (6.00), 1.488 (6.43), 1.503 (2.42), 1.512 (3.34), 1.521 (1.76), 1.533 (1.58), 1.543 (1.96), 1.553 (1.37), 1.573 (0.60), 1.643 (2.27), 1.649 (2.31), 1.678 (1.99), 1.757 (0.58), 1.773 (0.43), 1.813 (2.16), 1.842 (1.42), 1.907 (1.48), 2.331 (0.61), 2.518 (4.00), 2.522 (2.49), 3.200 (0.40), 3.209 (0.63), 3.214 (0.94), 3.222 (0.73), 3.228 (0.72), 3.233 (0.84), 3.241 (1.31), 3.253 (0.75), 3.256 (1.12), 3.268 (0.84), 3.278 (0.43), 3.299 (1.01), 3.306 (1.13), 3.317 (1.96), 3.343 (1.42), 3.364 (1.69), 3.374 (1.87), 3.392 (2.78), 3.400 (3.57), 3.419 (1.76), 3.428 (1.88), 3.651 (1.04), 3.656 (1.19), 3.665 (1.97), 3.669 (1.70), 3.680 (1.96), 3.693 (1.87), 3.703 (1.19), 3.709 (1.06), 3.821 (0.66), 3.830 (0.58), 3.849 (0.51), 3.854 (0.58), 3.875 (2.31), 3.879 (2.81), 3.884 (2.03), 3.903 (2.12), 3.907 (2.13), 3.913 (1.94), 4.241 (0.79), 4.257 (16.00), 4.267 (9.01), 4.272 (8.57), 4.298 (0.64), 4.522 (1.30), 4.537 (2.06), 4.551 (0.91), 7.766 (8.21), 7.778 (8.57), 8.370 (10.48), 8.382 (10.13), 8.710 (15.12).

### Intermediate 1-41

### 3-(2-methoxy-1-methyl-ethoxy)pyridine-4-carbonitrile

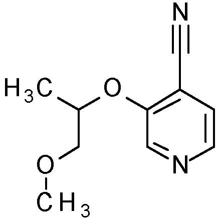

3-Hydroxypyridine-4-carbonitrile (1.00 g, 8.33 mmol; CAS 87032-82-4) and 1-methoxypropan-2-ol (1.1 ml, 11 mmol; CAS 107-98-2) were dissolved in dioxane (43 ml). (tributyl-lambda⁵-phosphanylidene)acetonitrile (3.3 ml, 12 mmol) was added and the mixture was stirred for 2 h at 105°C. The reaction mixture was concentrated under reduced pressure, hexane and methanol were added. The residue was purified by flash chromatography (silica, hexane / EtOAc gradient 50-100 %; EtOAc / EtOH gradient 0-10 %) to give 1.65 g of the title compound (98 % yield).

LC-MS (method 2): Rₜ = 0.82 min; MS (ESlpos): m/z = 193 [M+H]⁺

### Intermediate 1-61

### 3-[(3,3-difluorotetrahydropyran-2-yl)methoxy]pyridine-4-carbonitrile

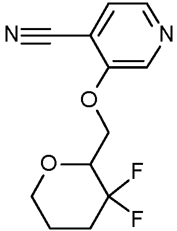

A mixture of 2-(benzyloxymethyl)-3,3-difluoro-2,6-dihydropyran (22.0 g, 91.6 mmol, CAS 275377-34-9) and Pd(OH)₂/C (6.60 g, 10% purity, wet) in MeOH (400 mL) was degassed and purged with H₂ for 3 times and the mixture was stirred at 40 °C for 12 hrs under H₂ atmosphere(45psi). TLC (Petroleum ether/Ethyl acetate=5/1, KMnO₄, R_{f} of Cpd. 4 = 0.4, product R_{f} = 0.1) showed most of the starting material was consumed. The mixture was filtered and concentrated under reduced pressure to give the (3,3-difluorotetrahydropyran-2-yl)methanol (13.5 g, crude) as a black liquid. A solution of (3,3-difluorotetrahydropyran-2-yl)methanol (8.00 g, 52.6 mmol, 1.0 eq) in DMF (8 mL) was added drop-wise to a suspension of NaH (3.15 g, 78.8 mmol, 60% purity, 1.5 eq) in DMF (48 mL). The mixture was stirred at 20 °C for 1 h. Then 3-chloropyridine-4-carbonitrile (8.01 g, 57.8 mmol, 1.1 *eq*) in DMF (24 mL) was added drop-wise with mild cooling to maintain a temperature of 30-40 °C. The solution was stirred at 30-40 °C for 3 hrs. The reaction mixture was quenched by addition sat.NH₄Cl (360 mL), and extracted with EtOAc (360 mL). The combined organic layer was dried over Na₂SO₄, filtered and concentrated under reduced pressure to give a residue. The residue was purified by column chromatography (SiO2, Petroleum ether/Ethyl acetate=1/0 to 3/1) to give the title compound (5.00 g, 19.4 mmol, 98.7% purity) as a yellow solid.

LC-MS (method 5): Rₜ = 0.689 min, (ESlpos): m/z = 255.1[M+H]⁺

¹H-NMR (400 MHz, CDCl₃) δ [ppm]: 8.56 (s, 1 H), 8.40 (d, 1 H), 7.45 (d, 1 H), 4.61 - 4.59 (m, 1 H), 4.45 - 4.40 (m, 1 H), 4.10-4.06 (m, 1 H), 4.00-3.89 (m, 1 H), 3.55-3.50 (m, 1 H), 2.35 - 2.33 (m, 1 H), 2.01-1.97 (m, 3 H).

### Intermediate 1-62

### 3-[(5,5-dimethyl-1,4-dioxan-2-yl)methoxy]pyridine-4-carbonitrile

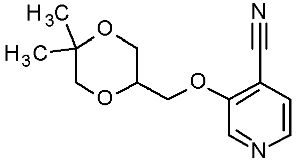

According to the method described for Intermediate 1-1 using 3-chloropyridine-4-carbonitrile (4.68 g, 33.7 mmol, CAS 68325-15-5) and (5,5-dimethyl-1,4-dioxan-2-yl)methanol (4.00 g, 98 % purity, 33.7 mmol, CAS 54321-57-2) as starting materials, 4.0 g (80% purity, 38% yield) of the title compound were prepared after purification by flash chromatography (amino phase silica, hexane / EtOAc gradient 0-100%).

LC-MS (method 2): Rₜ = 0.86 min; MS (ESlpos): m/z = 249 [M+H]⁺

¹H-NMR (400 MHz, DMSO-d₆) δ [ppm]: 1.016 (3.25), 1.051 (11.22), 1.175 (0.58), 1.203 (2.48), 1.265 (9.35), 1.990 (1.07), 2.521 (0.45), 3.315 (1.29), 3.326 (0.52), 3.334 (16.00), 3.343 (1.78), 3.464 (0.68), 3.478 (0.49), 3.486 (0.51), 3.492 (0.77), 3.498 (0.75), 3.573 (1.69), 3.607 (1.07), 3.629 (1.43), 3.736 (0.83), 3.763 (2.03), 3.784 (1.60), 3.789 (1.97), 3.793 (1.14), 3.799 (0.55), 3.804 (0.44), 4.359 (2.63), 4.368 (3.08), 4.693 (0.53), 7.784 (2.28), 7.796 (2.36), 7.798 (2.14), 8.390 (2.99), 8.402 (2.86), 8.718 (3.88).

### Intermediate 1-71

### 3-[(3,3-difluorotetrahydrofuran-2-yl)methoxy]pyridine-4-carbonitrile

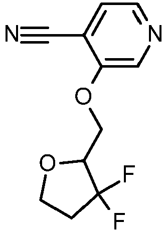

To a solution of 2-(benzyloxymethyl)-3,3-difluoro-2H-furan( 18.0 g, CAS 958232-09-2) in THF (145 mL) was added Pd/C (3.60 g, 10% purity) at 15 - 20 °C under H₂, the mixture was warmed to 40 - 45 °C over 15 min, then stirred at 40 - 45 °C under H₂ (45 Psi) for 10 hrs. The mixture was filtered and the filter cake was washed with THF (20.0 mL), then the filtrate was concentrated under reduced pressure to give (3,3-difluorotetrahydrofuran-2-yl)methanol (6.90 g, yield: 62.8%) as brown oil.To a suspension of NaH (2.82 g, 70.6 mmol, 60% purity) in DMF (25 mL) was dropwise added a solution of (3,3-difluorotetrahydrofuran-2-yl)methanol (6.50 g, 47.06 mmol, 1.00 eq) in DMF (20 mL) at 10 - 20 °C under N₂ (15 min), the solution was stirred at 20 - 30 °C under N₂ for 1 hr. Then a solution of 3-chloropyridine-4-carbonitrile (7.17 g, 51.8 mmol) in DMF (20 mL) was added the mixture at 10 - 20 °C over 15 min, the mixture was stirred for further 3 hrs at 45 - 50 °C under N₂. The mixture was poured into saturated NH₄Cl aqueous solution (250 mL), extracted with ethyl acetate (200 mL x 3), the organic phase was combined and washed with brine (300 mL x 3), then dried with Na₂SO₄, filtered, concentrated under reduced pressure to give brown oil. This brown oil was purified by column chromatography (silica, Petroleum ether/Ethyl acetate 0 - 33%) to give the title compound (6.00 g, 53.1 % yield) as yellow oil.

LC-MS

¹H NMR: (400 MHz, CDCl₃) δ 8.50 (s, 1 H), 8.39 (d, *J =* 4.8 Hz, 1 H), 7.44 (d, *J* = 5.2 Hz, 1 H), 4.41 - 4.37 (m, 2 H), 4.27 - 4.26 (m, 2 H), 4.03 (td, *J* = 8.4, 7.2 Hz, 1 H), 2.66 - 2.60 (m, 1 H), 2.49 - 2.47 (m, 1 H).

### Intermediate 1-80

### 3-(2-methoxypropoxy)pyridine-4-carbonitrile

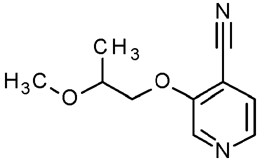

According to the method described for Intermediate 1-1 using 3-chloropyridine-4-carbonitrile (4.68 g, 33.7 mmol, CAS 68325-15-5) and 2-methoxypropan-1-ol (3.12 g, 34.6 mmol, CAS 1589-47-5) as starting materials, 5.24 g (95 % purity, 90 % yield) of the title compound were prepared after purification by flash chromatography (silica, Hexane / EE 15-100%).

LC-MS (method 2): Rₜ = 0.80 min; MS (ESlpos): m/z = 193 [M+H]⁺

¹H-NMR (400 MHz, DMSO-d₆) δ [ppm]: 0.000 (0.40), 1.190 (4.58), 1.206 (4.45), 3.336 (16.00), 3.338 (15.69), 3.712 (0.44), 3.723 (0.47), 4.224 (0.51), 4.239 (0.47), 4.250 (0.96), 4.265 (0.92), 4.302 (0.89), 4.311 (0.88), 4.328 (0.47), 4.337 (0.44), 7.777 (1.09), 7.790 (1.18), 8.382 (1.50), 8.394 (1.47), 8.718 (1.89).

### Intermediate 1-83

### 3-[(1-methyl-2-piperidyl)methoxy]pyridine-4-carbonitrile

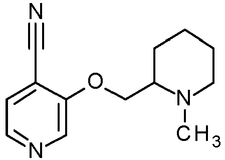

According to the method described for Intermediate 1-1 using 3-chloropyridine-4-carbonitrile (10.0 g, 72.2 mmol, CAS 68325-15-5) and (1-methylpiperidin-2-yl)methanol (11 ml, 87 mmol, CAS 20845-34-5) as starting materials, 11.5 g (95 % purity, 65 % yield) of the title compound were prepared after purification by flash chromatography (amino phase silica, hexane / EtOAc 50-100%).

LC-MS (method 2): Rₜ = 0.92 min; MS (ESlpos): m/z = 232 [M+H]⁺

¹H-NMR (400 MHz, DMSO-d₆) δ [ppm]: 1.23 - 1.50 (m, 4H), 1.52 - 1.57 (m, 1H), 1.66 - 1.83 (m, 2H), 1.99 - 2.09 (m, 1H), 2.23 - 2.29 (m, 4H), 2.73 - 2.79 (m, 1H), 4.21 (dd, 1H), 4.38 (dd, 1H), 7.77 (d, 1H), 8.38 (d, 1H), 8.73 (s, 1H).

### Intermediate 1-86

### 3-(2-tetrahydropyran-2-ylethoxy)pyridine-4-carbonitrile

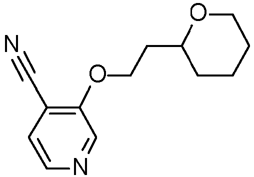

According to the method described for Intermediate 1-1 using 3-chloropyridine-4-carbonitrile (5.06 g, 36.5 mmol, CAS 68325-15-5) and 2-(tetrahydro-2H-pyran-2-yl)ethanol (5.00 g, 95 % purity, 36.5 mmol, CAS 38786-79-7) as starting materials, 7.75 g (99 % purity, 91 % yield) of the title compound were prepared after purification by flash chromatography (amino phase silica, hexane / EE 0-30%).

LC-MS method 1): Rₜ = 1.01 min; MS (ESlpos): m/z = 233 [M+H]⁺

¹H-NMR (400 MHz, CDCl₃) δ [ppm]: 1.176 (0.53), 1.233 (0.94), 1.254 (1.20), 1.265 (1.98), 1.282 (1.22), 1.286 (1.49), 1.292 (2.29), 1.313 (0.95), 1.323 (1.36), 1.399 (0.55), 1.408 (1.01), 1.416 (0.80), 1.431 (2.32), 1.439 (2.97), 1.447 (2.78), 1.462 (6.73), 1.468 (5.66), 1.476 (3.22), 1.485 (2.53), 1.493 (2.46), 1.495 (2.66), 1.501 (2.00), 1.510 (0.76), 1.518 (1.22), 1.529 (1.21), 1.556 (7.53), 1.566 (2.85), 1.586 (0.78), 1.592 (1.98), 1.596 (2.34), 1.737 (0.42), 1.751 (1.19), 1.757 (1.53), 1.762 (1.81), 1.773 (1.91), 1.782 (2.08), 1.787 (2.09), 1.795 (1.14), 1.832 (0.80), 1.846 (1.13), 1.855 (0.75), 1.858 (0.93), 1.869 (2.86), 1.881 (3.36), 1.891 (2.34), 1.895 (2.05), 1.904 (2.90), 1.910 (2.10), 1.917 (2.38), 1.920 (2.36), 1.926 (2.12), 1.931 (2.29), 1.935 (2.36), 1.941 (2.30), 1.947 (2.62), 1.956 (2.65), 1.962 (0.92), 1.968 (1.01), 1.971 (0.88), 1.977 (0.81), 1.983 (0.86), 1.992 (0.74), 3.318 (1.90), 3.324 (1.26), 3.331 (0.70), 3.336 (0.48), 3.347 (4.27), 3.354 (2.48), 3.360 (0.95), 3.374 (2.41), 3.382 (1.46), 3.437 (1.24), 3.443 (1.45), 3.446 (1.49), 3.452 (1.41), 3.459 (1.37), 3.465 (2.56), 3.469 (2.38), 3.474 (2.54), 3.479 (1.34), 3.487 (1.18), 3.492 (1.30), 3.496 (1.24), 3.501 (1.09), 3.862 (2.05), 3.867 (2.90), 3.873 (2.20), 3.876 (1.49), 3.891 (2.24), 3.895 (2.47), 3.901 (2.38), 4.199 (1.68), 4.211 (1.99), 4.214 (2.16), 4.222 (3.52), 4.226 (2.14), 4.234 (3.78), 4.237 (3.79), 4.249 (3.10), 4.269 (3.40), 4.283 (3.62), 4.290 (4.23), 4.304 (3.93), 4.313 (2.02), 4.326 (1.80), 7.347 (9.30), 7.359 (9.52), 8.264 (11.42), 8.276 (11.12), 8.432 (16.00).

### Intermediate 1-89

### tert-butyl (3S)-3-{[(4-cyanopyridin-3-yl)oxy]methyl}morpholine-4-carboxylate

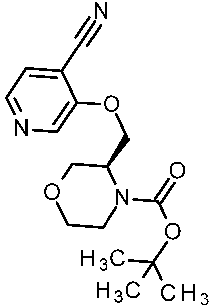

Tert-butyl (3*R*)-3-(hydroxymethyl)ymorpholine-4-carboxylate (676 mg, 3.11 mmol; CAS 215917-99-0) was dissolved in THF (4.4 ml) and cooled to 0°C. Sodium hydride (244 mg, 55 % purity, 5.60 mmol) was added in 4 portions and stirring was continued for 1h at 0°C. 3-Fluoropyridine-4-carbonitrile (380 mg, 3.11 mmol, CAS 113770-88-0) in THF (2.4 ml) was added at 0°C and the mixture was stirred for additional 2 h at 0°. The reaction mixture was diluted with 2N HCl-solution until a pH of 6-7 was reached and extracted with EtOAc. Combined organic phases were dried, concentrated under reduced pressure and purified by flash chromatography (silica, hexane / EtOAc gradient 0-100% and EtOAc /EtOH gradient 0-35%) to give 540 mg (95% purity, 51% yield) of the desired product.

¹H NMR (400 MHz, DMSO-d₆) δ [ppm]: 1.21 - 1.43 (m, 9 H) 3.11 - 3.29 (m, 1 H) 3.34 - 3.42 (m, 1 H) 3.48 - 3.57 (m, 1 H) 3.59 - 3.77 (m, 1 H) 3.77 - 3.86 (m, 1 H) 3.88 - 3.96 (m, 1 H) 4.21 - 4.64 (m, 3 H) 7.76 - 7.84 (m, 1 H) 8.37 - 8.43 (m, 1 H) 8.81 - 8.87 (m, 1 H).

### Intermediate 1-92

### 3-{2-[(2S)-oxolan-2-yl]ethoxy}pyridine-4-carbonitrile

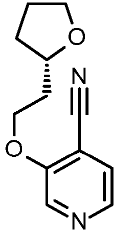

According to the method described for Intermediate 1-1 using 3-chloropyridine-4-carbonitrile (2.43 g, 17.2 mmol, CAS 68325-15-5) and 2-[(2S)-oxolan-2-yl]ethan-1-ol (2.00 g, 17.2 mmol, CAS 149818-53-1) as starting materials, 2.70 g (95 % purity, 68 % yield) of the title compound were prepared after purification by flash chromatography (amino phase silica, Hexane / EE 0-100%).

LC-MS (method 2): Rₜ = 0.80 min; MS (ESlpos): m/z = 193 [M+H]⁺

¹H-NMR (400 MHz, DMSO-d₆) δ [ppm]: 1.154 (2.56), 1.172 (5.24), 1.190 (2.58), 1.233 (0.85), 1.352 (2.12), 1.391 (0.61), 1.483 (1.05), 1.501 (2.33), 1.504 (1.53), 1.513 (1.30), 1.520 (1.48), 1.523 (2.73), 1.531 (2.65), 1.535 (1.66), 1.542 (1.48), 1.549 (1.65), 1.553 (2.96), 1.572 (1.56), 1.761 (0.46), 1.776 (0.52), 1.780 (0.68), 1.791 (1.34), 1.795 (0.82), 1.800 (0.82), 1.806 (1.57), 1.810 (2.48), 1.819 (0.70), 1.827 (4.09), 1.831 (4.34), 1.846 (4.59), 1.849 (3.69), 1.862 (1.93), 1.865 (2.94), 1.870 (1.24), 1.883 (1.59), 1.891 (0.50), 1.896 (1.34), 1.900 (0.73), 1.910 (0.81), 1.916 (2.41), 1.931 (4.66), 1.947 (4.01), 1.950 (7.39), 1.964 (5.52), 1.967 (3.06), 1.978 (2.23), 1.981 (2.86), 1.987 (8.71), 1.994 (2.08), 1.998 (2.24), 2.003 (1.01), 2.007 (2.91), 2.011 (1.51), 2.015 (1.93), 2.021 (1.59), 2.024 (1.72), 2.028 (2.45), 2.037 (1.32), 2.042 (1.24), 2.045 (1.38), 2.058 (0.99), 2.518 (3.31), 2.523 (2.25), 3.581 (2.58), 3.597 (2.89), 3.601 (4.66), 3.616 (4.66), 3.620 (3.61), 3.636 (3.05), 3.743 (3.12), 3.758 (3.60), 3.761 (3.85), 3.776 (3.56), 3.781 (2.79), 3.796 (2.36), 3.913 (1.05), 3.931 (2.59), 3.946 (2.96), 3.948 (2.92), 3.962 (2.58), 3.980 (0.99), 3.999 (0.61), 4.017 (1.77), 4.035 (1.72), 4.051 (2.65), 4.319 (0.70), 4.327 (0.44), 4.335 (0.57), 4.338 (0.70), 4.344 (4.45), 4.350 (4.12), 4.360 (5.15), 4.363 (8.01), 4.378 (4.75), 4.381 (4.76), 4.388 (0.69), 7.771 (9.17), 7.773 (10.23), 7.783 (9.44), 7.785 (10.67), 7.791 (0.59), 8.375 (12.64), 8.387 (12.32), 8.403 (0.44), 8.704 (16.00).

### Intermediate 1-93

### 3-{[(3S)-4-methylmorpholin-3-yl]methoxy}pyridine-4-carbonitrile

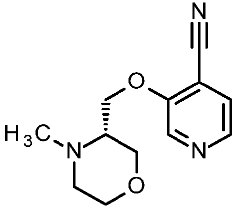

According to the method described for Intermediate 1-1 using 3-chloropyridine-4-carbonitrile (4.13 g, 29.8 mmol, CAS 68325-15-5) and [(3R)-4-methylmorpholin-3-yl]methanol-hydrogen chloride (1/1) (5.00 g, 29.8 mmol) as starting materials, 1.66 g (93 % purity, 22 % yield) of the title compound were prepared after purification by flash chromatography (amino phase silica, Hexane / EE 0-100%).

LC-MS (method 2): Rₜ = 0.69 min; MS (ESlpos): m/z = 234 [M+H]⁺

¹H-NMR (400 MHz, DMSO-d6) δ [ppm]: 2.191 (0.41), 2.215 (0.53), 2.223 (0.64), 2.240 (0.69), 2.244 (0.73), 2.248 (0.73), 2.252 (0.74), 2.270 (0.70), 2.278 (0.68), 2.310 (16.00), 2.475 (0.51), 2.478 (0.48), 2.521 (0.90), 2.526 (0.47), 2.661 (0.59), 2.667 (1.28), 2.674 (0.69), 2.690 (0.51), 2.697 (1.03), 2.704 (0.51), 2.854 (0.47), 3.359 (1.19), 3.382 (1.21), 3.387 (1.34), 3.410 (1.23), 3.475 (0.51), 3.482 (0.59), 3.503 (0.87), 3.509 (0.88), 3.529 (0.73), 3.535 (0.65), 3.688 (0.48), 3.695 (0.95), 3.702 (0.49), 3.723 (0.72), 3.860 (0.87), 3.867 (0.89), 3.888 (0.78), 3.895 (0.79), 4.212 (1.04), 4.227 (1.06), 4.238 (1.32), 4.252 (1.29), 4.420 (1.29), 4.432 (1.31), 4.446 (1.06), 4.458 (1.02), 7.781 (2.35), 7.793 (2.48), 8.394 (3.23), 8.406 (3.11), 8.748 (4.14).

### Intermediate 1-103

### 3-{[(2S)-1,4-dioxan-2-yl]methoxy}pyridine-4-carbonitrile

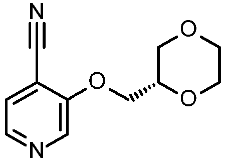

Using an analogous method as described for intermediate 1-6 with 3-chloropyridine-4-carbonitrile (CAS 68325-15-5, 1.00 g, 7.22 mmol) and [(2*R*)-1,4-dioxan-2-yl]methanol (CAS 406913-88-0, 938 mg, 7.94 mmol) as the starting materials; 490 mg (95% purity, 29% yield) of the title compound were prepared.

Optical rotation:[α]_{D} = - 1.68° +/- 0.35° (c = 7 mg/ml, methanol)

¹H-NMR (400 MHz, DMSO-d₆) δ [ppm]: 3.41 - 3.53 (m, 2 H), 3.59 - 3.72 (m, 2 H), 3.75 - 3.81 (m, 1 H), 3.82 - 3.87 (m, 1 H), 3.87 - 3.95 (m, 1 H), 4.27 - 4.37 (m, 2 H), 7.77 - 7.80 (m, 1 H), 8.38 - 8.41 (m, 1 H), 8.71 - 8.73 (m, 1 H).

### Intermediate 1-106

### tert-butyl (2R)-2-{[(4-cyanopyridin-3-yl)oxy]methyl}morpholine-4-carboxylate

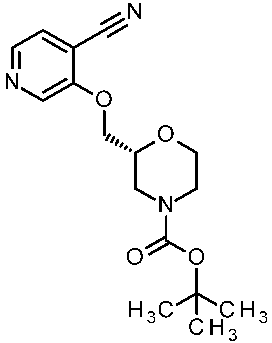

Using an analogous method as described for intermediate 1-6 with 3-chloropyridine-4-carbonitrile (CAS 68325-15-5, 3.25 g, 23.5 mmol) and *tert-butyl* (2R)-2-(hydroxymethyl)morpholine-4-carboxylate (CAS 135065-71-3, 5.10 g, 23.5 mmol) as the starting materials; 6.92 g (90% purity, 83% yield) of the title compound were prepared.

¹H-NMR (400 MHz, DMSO-d₆) δ [ppm]: 1.41 (s, 9H), 2.73 - 3.00 (m, 2H), 3.39 - 3.50 (m, 1H), 3.75 (m, 2H), 3.82 - 3.89 (m, 1H), 3.95 - 4.00 (m, 1H), 4.37 (br d, 2H), 7.79 (d, 1H), 8.40 (d, 1H), 8.73 (s, 1H).

LC-MS (method 2): Rₜ = 1.04 min; MS (ESlpos): m/z = 320 [M+H]⁺

### Intermediate 1-114

### 3-(2-allyloxyethoxy)pyridine-4-carbonitrile

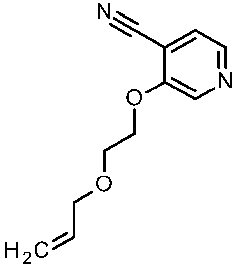

2-[(Prop-2-en-1-yl)oxy]ethan-1-ol (4.42 g, 43.3 mmol) was provided in THF (120 ml) and degassed with argon. To mixture was added sodium hydride (2.16 g, 60 % purity, 54.10 mmol) in portions at 0°C. 3-Chloropyridine-4-carbonitrile (5.00 g, 36.1 mmol) was added and the mixture was stirred at RT over night. The reaction mixture was treated with water and extracted with EtOAc. Combined organic phases were dried, concentrated under reduced pressure. The residue was purified by flash chromatography (silica, hexane / EtOAc gradient 20-100%) to give 5.67 g (77% yield) of the desired product.

LC-MS (method 2): Rₜ = 0.87 min; MS (ESlpos): m/z = 205 [M+H]⁺

¹H-NMR (400 MHz, DMSO-d₆) δ [ppm]: 2.523 (0.83), 2.527 (0.52), 3.399 (0.74), 3.401 (0.41), 3.413 (0.54), 3.494 (0.58), 3.508 (0.49), 3.773 (9.75), 3.780 (6.04), 3.783 (10.45), 3.787 (6.23), 3.795 (10.81), 3.939 (0.59), 3.952 (0.62), 3.956 (0.41), 4.040 (8.71), 4.044 (14.28), 4.047 (8.57), 4.053 (8.83), 4.057 (14.46), 4.061 (8.92), 4.455 (10.16), 4.462 (5.86), 4.467 (9.72), 4.470 (5.91), 4.477 (9.64), 4.607 (0.73), 5.132 (2.04), 5.136 (4.92), 5.141 (5.18), 5.144 (2.26), 5.158 (2.23), 5.161 (4.85), 5.166 (5.20), 5.170 (2.52), 5.246 (2.08), 5.251 (4.96), 5.255 (4.93), 5.260 (2.00), 5.289 (2.56), 5.294 (6.10), 5.298 (5.39), 5.303 (2.12), 5.839 (2.06), 5.852 (4.22), 5.865 (4.22), 5.878 (4.24), 5.882 (2.09), 5.891 (1.98), 5.895 (3.66), 5.908 (3.54), 5.921 (3.74), 5.935 (1.61), 7.779 (9.76), 7.791 (10.49), 8.384 (12.54), 8.396 (12.08), 8.729 (16.00).

### Intermediate 1-120

### 3-(3-methoxybutoxy)pyridine-4-carbonitrile

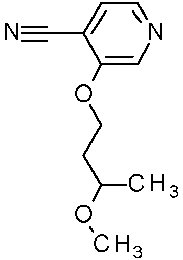

According to the method described for Intermediate 1-1 using 3-chloropyridine-4-carbonitrile (4.00 g, 28.9 mmol; CAS-RN:[68325-15-5]) and 3-methoxybutan-1-ol (3.9 ml, 35 mmol; CAS-RN:[2517-43-3]) as starting materials, 4.57 g (77 % yield) of the title compound were prepared after purification by flash chromatography (silica, Hexane / EE 70-100%; DCM / EtOH 0-20%).

LC-MS (method 1): Rₜ = 0.88 min; MS (ESlpos): m/z = 206 [M+H]⁺

¹H-NMR (400 MHz, DMSO-d₆) δ [ppm]: -0.025 (0.56), 1.036 (1.55), 1.051 (1.54), 1.141 (15.01), 1.156 (16.00), 1.876 (1.80), 1.892 (5.37), 1.908 (5.21), 1.923 (1.98), 3.182 (3.34), 3.351 (9.41), 3.489 (1.25), 3.504 (2.38), 3.520 (2.33), 3.535 (1.18), 4.322 (3.53), 4.330 (0.52), 4.338 (7.11), 4.354 (3.67), 7.749 (4.30), 7.751 (3.68), 7.761 (4.51), 7.763 (3.96), 8.363 (5.59), 8.375 (5.44), 8.692 (6.66).

### Intermediate 1-127

### 3-{[(2S)-oxan-2-yl]methoxy}pyridine-4-carbonitrile

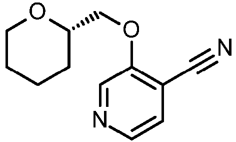

According to the method described for Intermediate 1-1 using 3-chloropyridine-4-carbonitrile (1.19 g, 8.61 mmol, CAS 68325-15-5) and [(2S)-oxan-2-yl]methanol (1.00 g, 8.61 mmol, CAS 51450-44-3) as starting materials, 1.26 g (99 % purity, 66 % yield) of the title compound were prepared after purification by flash chromatography (amino phase silica, hexane / EtOAc gradient 0-25%).

LC-MS (method 2): Rₜ = 0.95 min; MS (ESlpos): m/z = 219 [M+H]⁺

¹H-NMR (400 MHz, DMSO-d6) δ [ppm]: 1.316 (0.67), 1.326 (0.55), 1.347 (1.95), 1.356 (1.56), 1.375 (2.05), 1.379 (1.93), 1.385 (1.78), 1.399 (0.45), 1.406 (1.58), 1.416 (1.13), 1.421 (0.79), 1.431 (1.08), 1.440 (0.71), 1.453 (1.43), 1.463 (2.50), 1.472 (3.16), 1.481 (5.30), 1.488 (5.77), 1.502 (2.21), 1.511 (3.11), 1.520 (1.28), 1.533 (1.14), 1.542 (1.84), 1.552 (0.99), 1.572 (0.50), 1.643 (2.00), 1.649 (2.04), 1.677 (1.72), 1.812 (1.90), 1.817 (1.78), 1.825 (1.31), 1.842 (1.24), 2.518 (1.37), 2.522 (0.88), 3.364 (1.62), 3.373 (1.75), 3.391 (2.61), 3.400 (3.47), 3.419 (1.70), 3.428 (1.85), 3.650 (1.02), 3.656 (1.12), 3.664 (1.78), 3.669 (1.60), 3.675 (1.33), 3.680 (1.76), 3.692 (1.68), 3.697 (1.42), 3.702 (1.11), 3.708 (1.00), 3.870 (1.06), 3.875 (2.08), 3.879 (2.58), 3.884 (1.84), 3.898 (1.06), 3.902 (1.98), 3.907 (1.96), 3.912 (1.77), 4.240 (0.82), 4.257 (16.00), 4.267 (8.77), 4.271 (8.76), 4.298 (0.70), 7.764 (8.92), 7.766 (8.52), 7.776 (9.22), 7.778 (9.07), 8.369 (11.66), 8.381 (11.17), 8.710 (14.63).

### Syntheses of Intermediate 2 Compounds

### Intermediate 2-1

### 1-[3-(2-methoxy-2-methylpropoxy)pyridin-4-yl]methanamine

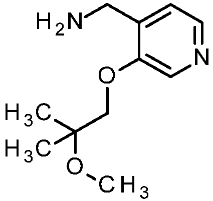

An autoclave was charged with 3-(2-methoxy-2-methylpropoxy)pyridine-4-carbonitrile (intermediate 1-1, 23.3 g, 113 mmol), ammonia (390 ml, 7.0 M in methanol, 18 mol) and Raney-Nickel (16.5 g, 50 %, 282 mmol, CAS 7440-02-0) and the mixture was stirred under 20 bar hydrogen atmosphere at RT for 17 h. The mixture was filtered through a pad of celite, washed with methanol and the filtrate was concentrated under reduced pressure to give 23.5 g (90 % purity, 89 % yield) of the title compound, which was used without further purification.

LC-MS (method 2): Rₜ = 0.68 min; MS (ESlpos): m/z = 211 [M+H]⁺

¹H-NMR (400 MHz, DMSO-d₆) δ [ppm]: 1.22 (s, 6H), 1.66 - 1.92 (br s, 2H), 3.17 (s, 3H), 3.67 - 3.78 (br s, 2H), 3.97 (s, 2H), 7.38 (br d, 1H), 8.17 (br d, 1H), 8.21 - 8.27 (s, 1H).

### Intermediate 2-3

### 1-(3-{[(2S)-oxolan-2-yl]methoxy}pyridin-4-yl)methanamine

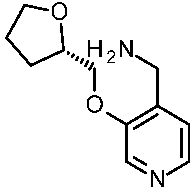

According to the method described for intermediate 2-1 using 3-[(2S)-tetrahydrofuran-2-ylmethoxy]isonicotinonitrile (intermediate 1-5, 7.00 g, 34.3 mmol) as a starting material, 5.84 g (99 % purity, 81 % yield) of the title compound were prepared.

¹H-NMR (400 MHz, DMSO-d₆) δ [ppm]: 1.676 (1.31), 1.692 (2.78), 1.714 (3.82), 1.721 (3.72), 1.726 (2.84), 1.730 (2.81), 1.737 (2.71), 1.742 (4.24), 1.759 (2.68), 1.782 (0.91), 1.799 (1.63), 1.819 (2.42), 1.829 (3.72), 1.850 (5.16), 1.867 (6.66), 1.883 (5.55), 1.898 (3.72), 1.903 (3.76), 1.919 (2.91), 1.934 (2.06), 1.949 (1.73), 1.963 (3.49), 1.976 (3.33), 1.981 (4.28), 1.993 (4.41), 2.002 (3.66), 2.005 (3.53), 2.011 (3.95), 2.024 (3.20), 2.030 (2.58), 2.044 (1.86), 2.518 (7.41), 2.523 (5.19), 3.661 (4.73), 3.681 (16.00), 3.696 (10.19), 3.715 (4.87), 3.760 (4.57), 3.776 (8.29), 3.793 (5.68), 3.796 (5.88), 3.813 (2.91), 3.881 (0.59), 4.014 (3.46), 4.029 (4.34), 4.040 (6.63), 4.054 (7.84), 4.091 (6.86), 4.100 (8.46), 4.117 (3.72), 4.126 (4.67), 4.152 (2.16), 4.168 (4.38), 4.178 (4.15), 4.184 (4.15), 4.193 (3.36), 4.209 (1.31), 7.371 (5.09), 7.383 (5.26), 8.159 (5.13), 8.170 (5.26), 8.236 (7.44).

### Intermediate 2-6

### 1-(3-{[(2S)-oxetan-2-yl]methoxy}pyridin-4-yl)methanamine

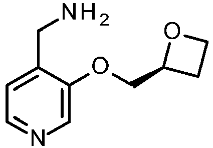

According to the method described for intermediate 2-1 using 3-{[(2S)-oxetan-2-yl]methoxy}pyridine-4-carbonitrile (intermediate 1-6, 22.9 g, 120 mmol) as a starting material, 24.4 g (quantitative) of the title compound were prepared.

¹H-NMR (400 MHz, DMSO-d₆) δ [ppm]: 1.009 (0.61), 1.078 (1.26), 1.753 (4.53), 1.762 (5.28), 2.522 (1.38), 2.553 (1.22), 2.570 (2.13), 2.575 (1.62), 2.581 (1.92), 2.587 (1.69), 2.592 (2.58), 2.597 (3.45), 2.603 (2.26), 2.609 (1.68), 2.615 (2.37), 2.620 (3.12), 2.637 (1.68), 2.675 (1.83), 2.692 (2.24), 2.696 (3.47), 2.703 (1.65), 2.713 (3.41), 2.717 (3.08), 2.724 (2.48), 2.733 (2.04), 2.740 (2.27), 2.744 (1.60), 2.761 (1.10), 3.160 (12.15), 3.173 (11.97), 3.726 (4.01), 3.743 (7.32), 3.760 (4.23), 4.097 (1.03), 4.110 (2.66), 4.123 (2.72), 4.136 (0.93), 4.197 (1.18), 4.204 (1.72), 4.224 (10.26), 4.231 (16.00), 4.242 (9.87), 4.258 (1.33), 4.270 (1.68), 4.459 (0.55), 4.469 (0.80), 4.475 (2.27), 4.489 (4.80), 4.497 (2.78), 4.505 (4.81), 4.511 (5.45), 4.518 (4.65), 4.527 (4.64), 4.532 (3.89), 4.535 (5.15), 4.539 (4.93), 4.549 (2.83), 4.553 (3.43), 4.556 (4.11), 4.570 (1.93), 4.991 (1.52), 5.002 (2.28), 5.011 (3.32), 5.019 (3.60), 5.022 (2.90), 5.027 (3.14), 5.036 (1.94), 5.039 (1.99), 5.047 (1.37), 7.398 (6.36), 7.409 (6.40), 8.177 (7.51), 8.189 (7.56), 8.210 (0.42), 8.272 (12.32), 8.304 (0.47).

### Intermediate 2-11

### tert-butyl (2S)-2-({[4-(aminomethyl)pyridin-3-yl]oxy}methyl)morpholine-4-carboxylate

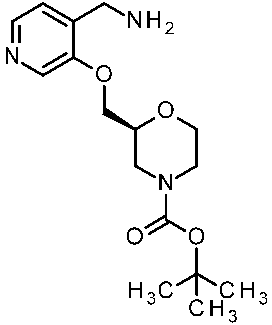

According to the method described for intermediate 2-1 using *tert-butyl* (2S)-2-{[(4-cyanopyridin-3-yl)oxy]methyl}morpholine-4-carboxylate (intermediate 1-11, 5.75 g, 18.0 mmol) as starting material, 6.00 g (95 % purity, 98 % yield) of the title compound were prepared.

LC-MS (method 2): Rₜ = 0.84 min; MS (ESlpos): m/z = 324 [M+H]⁺

¹H-NMR (400 MHz, DMSO-d₆) δ [ppm]: 1.41 (s, 9H), 3.16 (s, 2H), 3.39 - 3.49 (m, 2H), 3.66 - 3.79 (m, 5H), 3.79 - 3.98 (m, 3H), 4.07 - 4.20 (m, 3H), 7.39 (d, 1H), 8.19 (d, 1H), 8.27 (s, 1H).

### Intermediate 2-19

### 1-{3-[(2-methyloxetan-2-yl)methoxy]pyridin-4-yl}methanamine

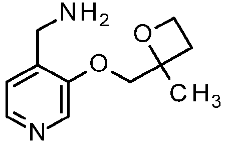

According to the method described for intermediate 2-1 using 3-[(methyloxetan-2-yl)methoxy]pyridine-4-carbonitrile (intermediate 1-19, 1.30 g, 6.37 mmol) as starting material, 1.31 g (89 % yield) of the title compound were prepared.

LC-MS (method 2): Rₜ = 0.60 min; MS (ESlpos): m/z = 209.1 [M+H]⁺

¹H-NMR (400 MHz, DMSO-d₆) δ [ppm]: 8.16 - 8.31 (m, 2H), 7.41 (d, 1H), 4.27 - 4.46 (m, 2H), 4.02 - 4.20 (m, 2H), 3.69 - 3.83 (m, 2H), 2.64 - 2.73 (m, 1H), 2.42 (ddd, 1H), 1.82 (br s, 2H), 1.35 - 1.45 (m, 3H).

### Intermediate 2-24

### 1-{3-[(1,4-dioxan-2-yl)methoxy]pyridin-4-yl}methanamine

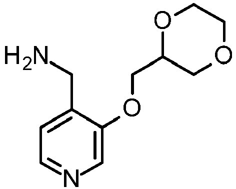

An autoclave was charged with 3-[(1,4-dioxan-2-yl)methoxy]pyridine-4-carbonitrile (intermediate 1-24, 1.17 g, 5.34 mmol), ammonia (19 ml, 7.0 M in methanol, 850 mmol) and Raney-Nickel (CAS 7440-02-0, 783 mg, 50 % wetted) and the mixture was stirred under 25 bar hydrogen atmosphere at RT for 22 h. The mixture was filtered through a pad of celite, eluted with methanol and the combined filtrates were concentrated under reduced pressure to give 1.13 g (94% yield) of the title compound.

¹H-NMR (400 MHz, DMSO-d₆) δ [ppm]: 8.24 (s, 1H), 8.17 (d, 1H), 7.38 (d, 1H), 4.09 (d, 2H), 3.73 - 3.91 (m, 3H), 3.59 - 3.73 (m, 4H), 3.46 - 3.52 (m, 2H), 3.39 - 3.45 (m, 2H). 2.06 (br., 2H).

LC-MS (method 2): Rₜ = 0.54 min; MS (ESlpos): m/z = 225 [M+H]⁺

### Intermediate 2-27

### 1-(3-{[oxan-2-yl]methoxy}pyridin-4-yl)methanamine

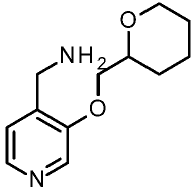

According to the method described for intermediate 2-1 using 3-(tetrahydro-2H-pyran-2-ylmethoxy)isonicotinonitrile (intermediate 1-27, 11.2 g, 95 % purity, 48.9 mmol) as a starting material, 11.5 g (85 % purity, 90 % yield) of the title compound were prepared.

¹H-NMR (400 MHz, DMSO-d₆) δ [ppm]: 1.297 (1.17), 1.306 (0.99), 1.327 (2.73), 1.337 (2.15), 1.357 (3.31), 1.364 (2.43), 1.387 (2.27), 1.425 (1.48), 1.433 (2.03), 1.480 (10.86), 1.487 (10.16), 1.499 (5.66), 1.521 (1.89), 1.530 (2.33), 1.540 (1.54), 1.560 (0.80), 1.571 (0.79), 1.642 (3.88), 1.672 (3.45), 1.774 (3.32), 1.805 (5.13), 1.831 (3.06), 2.326 (0.49), 2.522 (1.70), 2.668 (0.50), 3.166 (5.13), 3.358 (3.14), 3.367 (3.28), 3.386 (3.64), 3.394 (4.38), 3.412 (2.25), 3.421 (2.41), 3.608 (1.72), 3.615 (2.86), 3.620 (3.08), 3.632 (3.01), 3.643 (3.34), 3.648 (3.50), 3.678 (10.04), 3.774 (1.58), 3.807 (0.47), 3.840 (0.45), 3.879 (4.30), 3.907 (3.68), 4.019 (2.24), 4.033 (16.00), 4.045 (15.26), 4.104 (0.50), 4.256 (2.18), 4.266 (1.32), 4.271 (1.35), 7.364 (4.44), 7.374 (4.57), 7.395 (1.04), 7.407 (0.83), 7.765 (0.82), 7.777 (0.85), 8.153 (4.69), 8.164 (5.07), 8.179 (1.50), 8.228 (6.98), 8.267 (1.68), 8.369 (0.91), 8.381 (0.87), 8.710 (1.36).

### Intermediate 2-61

### [3-[(3,3-difluorotetrahydropyran-2-yl)methoxy]-4-pyridyl]methanamine

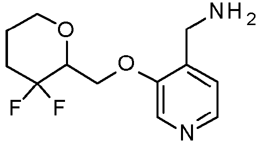

According to the method described for intermediate 2-24 using 3-[(3,3-difluorotetrahydropyran-2-yl)methoxy]pyridine-4-carbonitrile (intermediate 1-61, 2.86 g, 11.2 mmol) as starting material, 1.6 g (76% purity, 60% yield) of the title compound were prepared.

¹H-NMR (400 MHz, DMSO-d₆) δ [ppm]: 1.725 (0.76), 1.736 (1.59), 1.755 (2.90), 1.760 (3.77), 1.763 (3.99), 1.774 (3.54), 1.784 (2.91), 1.794 (1.34), 1.819 (0.64), 1.861 (0.73), 1.975 (0.55), 1.989 (0.56), 1.994 (0.50), 2.009 (0.82), 2.023 (0.75), 2.034 (0.69), 2.054 (0.59), 2.062 (0.50), 2.068 (0.50), 2.076 (0.43), 2.082 (0.41), 2.097 (0.74), 2.110 (0.64), 2.116 (0.59), 2.121 (0.68), 2.132 (0.49), 2.142 (0.63), 2.156 (0.99), 2.177 (0.94), 2.182 (1.02), 2.192 (1.22), 2.204 (1.18), 2.518 (1.22), 2.523 (0.87), 2.539 (4.62), 3.514 (1.21), 3.530 (1.18), 3.542 (1.49), 3.551 (1.37), 3.558 (1.20), 3.578 (1.21), 3.680 (16.00), 3.904 (1.64), 3.908 (1.70), 3.912 (1.69), 3.924 (1.06), 3.935 (1.52), 3.943 (1.42), 3.969 (1.34), 3.976 (1.49), 3.986 (1.73), 3.993 (1.64), 4.029 (1.25), 4.036 (1.46), 4.047 (1.77), 4.053 (1.74), 4.138 (2.83), 4.155 (2.22), 4.166 (3.33), 4.183 (2.70), 4.405 (3.20), 4.413 (3.10), 4.432 (2.64), 4.440 (2.52), 7.386 (4.59), 7.397 (4.66), 8.184 (7.04), 8.195 (6.87), 8.279 (11.15).

### Intermediate 2-41

### [3-(2-methoxy-1-methyl-ethoxy)-4-pyridyl]methanamine

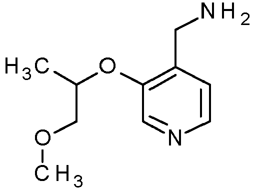

According to the method described for intermediate 2-24 using 3-(2-methoxy-1-methyl-ethoxy)pyridine-4-carbonitrile (intermediate 1-41, 1.78 g, 9.28 mmol) as starting material, 1.83 g (85% purity, 85% yield) of the title compound were prepared.

LC-MS (method 2): Rₜ = 0.61 min; MS (ESlpos): m/z = 197 [M+H]⁺

¹H-NMR (400 MHz, DMSO-d₆) δ [ppm]: 1.25 (d, 3H), 1.61 - 1.92 (s, 2H), 3.26 - 3.31 (m, 3H), 3.42 - 3.56 (m, 2H), 3.66 (br s, 2H), 3.67 (s, 1H), 4.67 - 4.75 (m, 1H), 7.38 (br s, 1H), 8.09 - 8.22 (br s, 1H), 8.29 (br s, 1H).

### Intermediate 2-62

### [3-[(5,5-dimethyl-1,4-dioxan-2-yl)methoxy]-4-pyridyl]methanamine

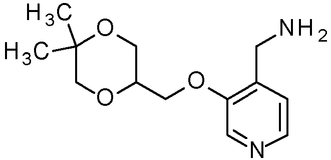

According to the method described for intermediate 2-24 using 3-[(5,5-dimethyl-1,4-dioxan-2-yl)methoxy]pyridine-4-carbonitrile (intermediate 1-62, 1.30 g, 5.24 mmol) as starting material, 1.26 g (90% purity, 86% yield) of the title compound were prepared.

¹H-NMR (400 MHz, DMSO-d₆) δ [ppm]: 1.014 (0.96), 1.049 (16.00), 1.201 (0.72), 1.262 (13.58), 1.751 (0.48), 2.518 (2.17), 2.523 (1.47), 3.308 (2.76), 3.539 (2.43), 3.567 (1.99), 3.601 (1.13), 3.623 (1.75), 3.694 (1.97), 3.720 (2.07), 3.746 (2.38), 3.783 (0.46), 4.128 (3.01), 4.136 (2.80), 7.386 (0.85), 8.181 (0.83), 8.237 (0.96).

### Intermediate 2-71

### [3-[(3,3-difluorotetrahydrofuran-2-yl)methoxy]-4-pyridyl]methanamine

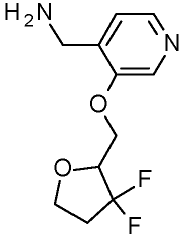

According to the method described for intermediate 2-24 using 3-[(3,3-difluorooxolan-2-yl)methoxy]pyridine-4-carbonitrile (intermediate 1-71; 6.00 g, 25.0 mmol) as starting material, 6.05 g (95% purity, 94% yield) of the title compound were prepared.

¹H-NMR (400 MHz, DMSO-d₆) δ [ppm]: 1.747 (10.29), 2.411 (0.51), 2.432 (0.98), 2.446 (1.80), 2.452 (1.67), 2.467 (3.90), 2.476 (3.52), 2.528 (9.20), 2.535 (5.84), 2.548 (6.51), 2.557 (3.54), 2.564 (3.43), 2.568 (3.28), 2.576 (2.55), 2.690 (0.45), 3.339 (10.40), 3.768 (2.32), 3.919 (4.35), 3.940 (10.45), 3.961 (11.55), 3.982 (5.12), 4.096 (5.30), 4.108 (5.81), 4.116 (8.28), 4.129 (8.19), 4.138 (4.58), 4.150 (3.93), 4.218 (1.47), 4.237 (6.93), 4.244 (5.84), 4.258 (13.04), 4.262 (13.41), 4.275 (16.00), 4.291 (10.75), 4.308 (5.70), 4.325 (1.53), 7.397 (8.68), 7.408 (8.82), 8.196 (8.97), 8.207 (9.54), 8.287 (13.08).

### Intermediate 2-80

### [3-(2-methoxypropoxy)-4-pyridyl]methanamine

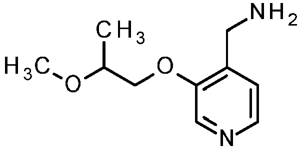

An autoclave was charged with 3-(2-methoxypropoxy)pyridine-4-carbonitrile (intermediate 1-80, 5.24 g, 95% purity, 25.9 mmol), ethanol (82 ml), TEA (20 ml, 150 mmol) and palladium on carbon (1.93 g, 10 % purity, 1.81 mmol) were added. The mixture was stirred under 15 bar hydrogen atmosphere at RT for 18 h. The mixture was filtered through a pad of celite, washed with ethanol (30 ml) and methanol (30 ml). The filtrate was concentrated under reduced pressure to give 4.51 g (90 % purity, 80 % yield) of the title compound, which was used without further purification.

LC-MS method 1): Rₜ = 0.48 min; MS (ESlpos): m/z = 197 [M+H]⁺

¹H-NMR (400 MHz, DMSO-d₆) δ [ppm]: 0.000 (4.49), 1.051 (0.46), 1.057 (0.52), 1.066 (0.53), 1.072 (0.51), 1.087 (0.49), 1.103 (0.49), 1.179 (15.56), 1.190 (1.59), 1.195 (16.00), 1.206 (0.93), 2.012 (0.55), 2.521 (0.55), 3.267 (2.30), 3.289 (1.38), 3.308 (0.44), 3.335 (2.56), 3.658 (1.00), 3.669 (1.25), 3.672 (1.31), 3.685 (1.67), 3.688 (1.57), 3.702 (12.04), 3.715 (0.43), 3.883 (1.13), 4.020 (1.18), 4.034 (1.02), 4.045 (3.71), 4.059 (3.67), 4.066 (3.60), 4.076 (3.54), 4.091 (1.14), 4.102 (0.97), 7.382 (2.86), 7.394 (2.92), 8.164 (4.66), 8.176 (4.66), 8.240 (8.01).

### Intermediate 2-83

### [3-[(1-methyl-2-piperidyl)methoxy]-4-pyridyl]methanamine

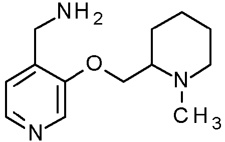

According to the method described for intermediate 2-24 using 3-[(1-methyl-2-piperidyl)methoxy]pyridine-4-carbonitrile (intermediate 1-83, 12.8 g, 55.4 mmol) as starting material, 12.9 g (95% purity, 94% yield) of the title compound were prepared.

LC-MS (method 2): Rₜ = 0.72 min; MS (ESlpos): m/z = 236 [M+H]⁺

¹H-NMR (400 MHz, DMSO-d₆) δ [ppm]: 1.18 - 1.60 (m, 4H), 1.62 - 1.85 (m, 4H), 2.03 (td, 1H), 2.16 - 2.26 (m, 4H), 2.76 (br d, 1H), 3.60 - 3.79 (m, 2H), 4.01 (dd, 1H), 4.18 (dd, 1H), 7.31 - 7.43 (m, 1H), 8.12 - 8.20 (br s, 1H), 8.24 (br s, 1H).

### Intermediate 2-86

### [3-(2-tetrahydropyran-2-ylethoxy)-4-pyridyl]methanamine

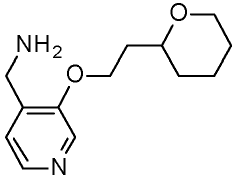

According to the method described for intermediate 2-24 using 3-(2-tetrahydropyran-2-ylethoxy)pyridine-4-carbonitrile (intermediate 1-86, 7.55 g, 32.5 mmol) as starting material, 7.62 g (95% purity, 94% yield) of the title compound were prepared.

¹H-NMR (400 MHz, CDCl₃) δ [ppm]: 0.016 (0.74), 0.094 (0.83), 1.177 (0.46), 1.227 (0.52), 1.236 (1.08), 1.258 (1.36), 1.268 (2.32), 1.286 (1.68), 1.289 (1.66), 1.295 (2.59), 1.298 (1.98), 1.317 (1.21), 1.325 (1.81), 1.378 (3.47), 1.387 (3.61), 1.396 (2.58), 1.409 (2.44), 1.419 (4.38), 1.427 (3.30), 1.441 (3.32), 1.450 (6.87), 1.456 (6.04), 1.459 (5.73), 1.471 (3.19), 1.479 (2.42), 1.489 (3.05), 1.495 (1.75), 1.499 (1.93), 1.503 (1.39), 1.513 (1.17), 1.522 (1.59), 1.533 (1.60), 1.541 (2.46), 1.547 (3.07), 1.566 (1.14), 1.579 (2.30), 1.583 (2.10), 1.604 (0.48), 1.640 (0.46), 1.656 (0.42), 1.662 (0.54), 1.677 (0.54), 1.714 (0.61), 1.751 (1.74), 1.758 (2.21), 1.763 (2.33), 1.766 (2.30), 1.774 (2.17), 1.787 (2.41), 1.791 (2.20), 1.795 (1.90), 1.831 (0.49), 1.853 (4.59), 1.868 (7.75), 1.870 (8.37), 1.885 (10.49), 1.901 (4.37), 3.306 (2.14), 3.313 (1.89), 3.334 (4.93), 3.341 (3.74), 3.355 (0.53), 3.362 (2.74), 3.370 (1.89), 3.393 (1.30), 3.398 (1.32), 3.413 (2.10), 3.421 (1.57), 3.425 (2.25), 3.430 (1.63), 3.440 (1.96), 3.452 (1.29), 3.457 (1.16), 3.770 (7.31), 3.829 (1.09), 3.873 (2.32), 3.878 (2.62), 3.883 (2.49), 3.887 (1.62), 3.901 (2.01), 3.906 (2.83), 3.911 (2.19), 3.915 (1.40), 4.078 (1.30), 4.092 (2.67), 4.101 (3.54), 4.106 (1.82), 4.115 (6.36), 4.128 (4.48), 4.144 (5.93), 4.150 (1.80), 4.161 (3.33), 4.168 (2.39), 4.184 (1.36), 7.118 (6.66), 7.129 (6.85), 8.134 (9.44), 8.146 (9.69), 8.153 (16.00).

### Intermediate 2-89

### tert-butyl (3S)-3-({[4-(aminomethyl)pyridin-3-yl]oxy}methyl)morpholine-4-carboxylate

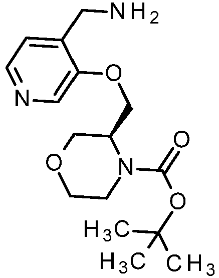

According to the method described for intermediate 2-24 using *tert-butyl* (3S)-3-{[(4-cyanopyridin-3-yl)oxy]methyl}morpholine-4-carboxylate (intermediate 1-89, 2.17 g, 6.78 mmol) as starting material, 1.41 g (95% purity, 65% yield) of the title compound were prepared.

¹H-NMR (400 MHz, DMSO-d₆) δ [ppm]:1.36 (br.s, 9H), 1.94 (br. s, 2H), 3.17 (d, 2H), 3.38 (dd, 1H), 3.52 (br d, 1H), 3.67 (s, 3H), 3.76 - 3.85 (m, 1H), 3.91 (d, 1H), 4.15 - 4.28 (m, 2H), 4.29 - 4.38 (m, 1H), 7.40 (d, 1H), 8.18 (d, 1H), 8.33 (s, 1H).

### Intermediate 2-92

### [3-[2-[(2S)-tetrahydrofuran-2-yl]ethoxy]-4-pyridyl]methanamine

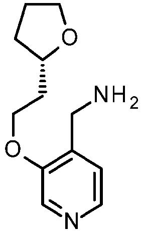

According to the method described for intermediate 2-24 using 3-{2-[(2S)-oxolan-2-yl]ethoxy}pyridine-4-carbonitrile (intermediate 1-91, 2.70 g, 12.4 mmol) as starting material, 2.5 g (92% purity, 84 % yield) of the title compound were prepared.

¹H-NMR (400 MHz, DMSO-d₆) δ [ppm]: 0.806 (0.60), 0.833 (0.93), 0.852 (1.11), 1.167 (0.87), 1.234 (2.81), 1.330 (0.69), 1.353 (1.38), 1.366 (0.48), 1.454 (1.47), 1.473 (3.14), 1.484 (1.85), 1.495 (3.68), 1.503 (3.47), 1.514 (2.06), 1.525 (3.65), 1.544 (1.88), 1.751 (10.32), 1.784 (3.08), 1.804 (4.93), 1.823 (6.94), 1.839 (7.18), 1.853 (4.19), 1.857 (4.81), 1.874 (2.78), 1.883 (3.92), 1.889 (3.98), 1.905 (8.10), 1.916 (8.01), 1.919 (7.87), 1.931 (3.62), 1.936 (3.35), 1.960 (2.45), 1.976 (2.75), 1.981 (2.42), 1.989 (3.65), 2.010 (3.17), 2.019 (1.94), 2.024 (1.91), 2.039 (1.23), 2.085 (0.42), 2.518 (7.93), 2.523 (5.14), 3.546 (0.45), 3.571 (3.26), 3.587 (4.46), 3.591 (6.55), 3.607 (6.82), 3.626 (4.07), 3.679 (12.44), 3.734 (4.28), 3.753 (6.19), 3.769 (5.68), 3.789 (3.20), 3.863 (0.51), 3.880 (2.42), 3.907 (1.38), 3.924 (3.71), 3.940 (5.26), 3.957 (3.80), 3.974 (1.29), 4.100 (0.51), 4.139 (8.82), 4.155 (16.00), 4.171 (8.61), 4.364 (0.42), 7.379 (5.41), 7.390 (5.56), 8.155 (5.62), 8.166 (5.83), 8.224 (8.46), 8.260 (1.02), 8.706 (0.48).

### Intermediate 2-93

### 1-(3-{[(3S)-4-methylmorpholin-3-yl]methoxy}pyridin-4-yl)methanamine

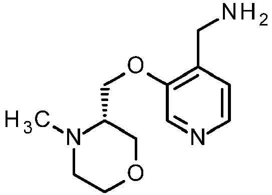

According to the method described for intermediate 2-24 using 3-{[(3S)-4-methylmorpholin-3-yl]methoxy}pyridine-4-carbonitrile (intermediate 1-93, 1.66 g, 7.12 mmol) as starting material, 1.60 g (96% purity, 91% yield) of the title compound were prepared.

¹H-NMR (400 MHz, DMSO-d₆) δ [ppm]: 1.793 (0.53), 2.191 (0.47), 2.201 (0.62), 2.210 (0.80), 2.212 (0.60), 2.228 (0.87), 2.231 (0.89), 2.236 (0.88), 2.239 (0.91), 2.257 (1.07), 2.265 (0.85), 2.272 (0.50), 2.287 (16.00), 2.419 (0.50), 2.423 (0.46), 2.428 (0.53), 2.432 (0.77), 2.442 (0.79), 2.447 (0.54), 2.450 (0.56), 2.455 (0.52), 2.650 (0.68), 2.657 (1.30), 2.663 (0.70), 2.680 (0.64), 2.687 (1.15), 2.693 (0.57), 2.853 (0.46), 3.363 (1.57), 3.368 (1.61), 3.391 (1.29), 3.472 (0.61), 3.478 (0.67), 3.499 (1.09), 3.505 (1.08), 3.526 (0.78), 3.532 (0.68), 3.683 (3.37), 3.688 (3.64), 3.700 (0.82), 3.715 (0.54), 3.722 (0.87), 3.729 (0.62), 3.848 (0.98), 3.856 (0.97), 3.875 (0.87), 3.883 (0.84), 3.983 (1.07), 3.997 (1.08), 4.008 (1.30), 4.023 (1.23), 4.216 (1.32), 4.226 (1.31), 4.241 (1.09), 4.251 (1.03), 7.383 (1.62), 7.394 (1.65), 8.169 (2.52), 8.181 (2.53), 8.259 (3.86).

### Intermediate 2-103

### 1-(3-{[(2S)-1,4-dioxan-2-yl]methoxy}pyridin-4-yl)methanamine

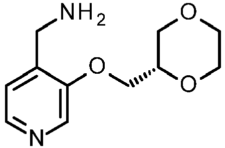

Using an analogous method as described for intermediate 2-1 with 3-{[(2S)-1,4-dioxan-2-yl]methoxy}pyridine-4-carbonitrile (intermediate 1-103, 1.24 g, 5.63 mmol) as the starting material; 1.56 g (80% purity, 99% yield) of the title compound were prepared.

¹H-NMR (400 MHz, DMSO-d₆): δ [ppm]= 1.232 (0.52), 1.845 (1.06), 2.518 (2.37), 2.523 (1.54), 3.159 (14.24), 3.171 (14.33), 3.376 (0.69), 3.379 (0.65), 3.404 (2.41), 3.419 (0.41), 3.428 (3.79), 3.431 (3.86), 3.439 (0.59), 3.447 (0.69), 3.455 (2.71), 3.465 (1.97), 3.474 (0.57), 3.486 (2.71), 3.493 (2.97), 3.508 (1.01), 3.520 (2.61), 3.592 (0.45), 3.601 (1.92), 3.608 (2.10), 3.630 (2.77), 3.636 (3.65), 3.659 (5.73), 3.661 (5.45), 3.683 (16.00), 3.700 (1.95), 3.754 (3.49), 3.762 (2.18), 3.784 (2.51), 3.794 (0.57), 3.812 (0.61), 3.823 (2.48), 3.829 (3.16), 3.842 (0.81), 3.849 (2.07), 3.856 (6.45), 3.868 (1.93), 3.874 (1.37), 3.880 (1.69), 3.887 (1.30), 3.892 (1.13), 3.899 (0.89), 4.087 (11.44), 4.099 (10.46), 4.111 (2.62), 4.125 (1.15), 7.380 (4.02), 7.391 (4.11), 7.411 (0.67), 7.423 (0.67), 8.168 (6.28), 8.179 (6.26), 8.184 (1.58), 8.196 (1.02), 8.230 (10.21), 8.274 (1.76).

### Intermediate 2-106

### tert-butyl (2R)-2-({[4-(aminomethyl)pyridin-3-yl]oxy}methyl)morpholine-4-carboxylate

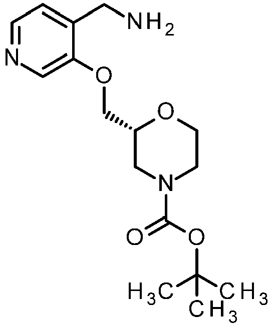

Using an analogous method as described for intermediate 2-1 with *tert*-butyl (2*R*)-2-{[(4-cyanopyridin-3-yl)oxy]methyl}morpholine-4-carboxylate (intermediate 1-106, 6.92 g, 21.7 mmol) as the starting material; 6.95 g of the title compound were prepared (90% purity, 89% yield).

¹H-NMR (400 MHz, DMSO-d₆) δ [ppm]: 1.41 (s, 9H), 2.70 - 3.01 (m, 2H), 3.17 (d, 3H), 3.41 - 3.52 (m, 1H), 3.63 - 3.70 (m, 2H), 3.70 - 3.81 (m, 2H), 3.82 - 4.00 (m, 2H), 4.08 - 4.18 (m, 3H), 7.39 (d, 1H), 8.18 (d, 1H), 8.25 (s, 1H).

LC-MS (method 2): Rₜ = 0.84 min; MS (ESlpos): m/z = 324 [M+H]⁺

### Intermediate 2-114

### [3-(2-allyloxyethoxy)-4-pyridyl]methanamine

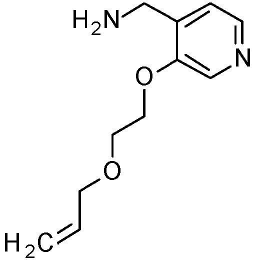

3-(2-Allyloxyethoxy)pyridine-4-carbonitrile (Intermediate 1-114, 5.65 g, 27.7 mmol) in THF (93 mL) was cooled to 0°C. Lithium aluminum hydride solution in THF (27.7 ml, 1M, 27.7 mmol) was added and the mixture was stirred for 2 h at 0°C. To the reaction mixture was added dropwise some water. The mixture was extracted with DCM. The organic layer was dried and concentrated under reduced pressure. The residue was purified by flash chromatography (amino phase silica, hexane / EtOAc gradient 40-100%) to give 1.55 g (27% yield) of the title compound.

LC-MS (method 2): Rₜ = 0.65 min; MS (ESlpos): m/z = 209 [M+H]⁺

¹H-NMR (400 MHz, DMSO-d₆) δ [ppm]: 1.80 (br s, 2H), 3.68 (s, 2H), 3.70 - 3.78 (m, 2H), 4.03 (dt, 2H), 4.18 - 4.29 (m, 2H), 5.11 - 5.19 (m, 1H), 5.27 (dq, 1H), 5.89 (ddt, 1H), 7.38 (d, 1H), 8.17 (d, 1H), 8.25 (s, 1H).

### Intermediate 2-120

### [3-(3-methoxybutoxy)-4-pyridyl]methanamine

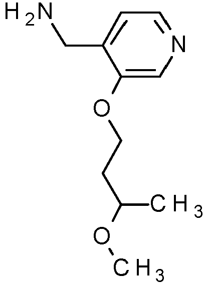

According to the method described for intermediate 2-80 using 3-(3-methoxybutoxy)pyridine-4-carbonitrile (Intermediate 1-120, 5.45 g, 26.4 mmol) as starting material, 5.44 g (97 % yield) of the title compound were prepared.

LC-MS (method 2): Rₜ = 0.71 min; MS (ESlpos): m/z = 211 [M+H]⁺

¹H-NMR (400 MHz, DMSO-d₆) δ [ppm]: 1.051 (0.81), 1.067 (0.91), 1.071 (0.88), 1.077 (1.12), 1.087 (0.88), 1.093 (1.02), 1.138 (15.60), 1.154 (16.00), 1.836 (0.52), 1.840 (1.04), 1.846 (0.41), 1.857 (2.52), 1.871 (3.76), 1.889 (2.51), 1.903 (1.06), 1.924 (0.52), 3.195 (2.02), 3.208 (2.65), 3.212 (0.49), 3.220 (2.28), 3.490 (1.10), 3.505 (1.62), 3.513 (0.60), 3.520 (1.56), 3.529 (0.45), 3.536 (1.04), 3.700 (12.28), 3.885 (0.49), 4.126 (2.71), 4.141 (4.53), 4.155 (1.98), 4.159 (1.97), 5.764 (2.28), 7.390 (3.00), 7.401 (3.05), 8.162 (4.81), 8.173 (4.79), 8.236 (8.17).

### Intermediate 2-127

### 1-(3-{[(2S)-oxan-2-yl]methoxy}pyridin-4-yl)methanamine

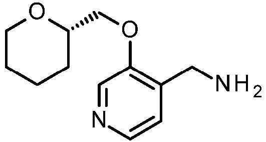

According to the method described for intermediate 2-1 using 3-{[(2S)-oxan-2-yl]methoxy}pyridine-4-carbonitrile (Intermediate 1-127, 1.26 g, 5.77 mmol) as a starting material, 1.21 g (95% purity, 90% yield) of the title compound were prepared.

¹H-NMR (400 MHz, DMSO-d₆) δ [ppm]: 1.297 (0.84), 1.307 (0.76), 1.328 (2.27), 1.337 (1.84), 1.359 (2.63), 1.365 (2.13), 1.387 (1.89), 1.396 (1.24), 1.425 (1.06), 1.434 (1.49), 1.443 (1.20), 1.456 (2.39), 1.467 (4.65), 1.481 (8.46), 1.489 (8.05), 1.500 (4.93), 1.509 (2.99), 1.522 (1.74), 1.531 (2.27), 1.541 (1.37), 1.551 (0.62), 1.562 (0.75), 1.572 (0.61), 1.644 (2.97), 1.674 (2.68), 1.806 (4.74), 1.810 (4.76), 1.824 (3.62), 1.831 (3.40), 1.886 (0.88), 2.522 (5.06), 3.166 (0.40), 3.358 (3.31), 3.367 (3.11), 3.386 (3.31), 3.395 (3.99), 3.413 (2.11), 3.422 (2.33), 3.604 (1.16), 3.609 (1.35), 3.616 (2.45), 3.621 (2.73), 3.633 (2.48), 3.643 (2.81), 3.648 (2.92), 3.655 (2.25), 3.661 (2.50), 3.678 (12.41), 3.880 (3.35), 3.903 (2.59), 3.908 (2.97), 3.913 (2.54), 4.019 (0.83), 4.034 (15.36), 4.046 (16.00), 7.365 (4.62), 7.376 (5.07), 7.407 (0.43), 8.152 (5.27), 8.163 (5.66), 8.227 (8.69), 8.268 (0.76), 8.474 (0.46).

### Syntheses of Intermediate 3 Compounds

### Intermediate 3-1

### tert-butyl (2R)-2-methyl-4,6-dioxopiperidine-1-carboxylate

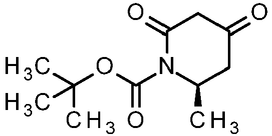

(3*R*)-3-[(*Tert*-butoxycarbonyl)amino]butanoic acid (3.00 g, 14.8 mmol, CAS 159991-23-8) was dissolved in dichloromethane (14 ml), cooled to 0 °C, EDC.HCl (4.24 g, 22.1 mmol), DMAP (2.7 g, 22.1 mmol) and 2,2-dimethyl-1,3-dioxane-4,6-dione (2.13 g, 14.8 mmol, CAS 2033-24-1) were added and the mixture was stirred for 3h at RT. The mixture was washed with potassium bisulfate (1 M), the organic layer was dried and concentrated under reduced pressure. The residue was dissolved in EtOAc (60 ml) and stirred for 5 h at reflux. The mixture was purified by flash chromatography (silica, DCM / EtOH gradient 0-8%) to give 3.12 g (90% purity, 84% yield) of the target compound.

According to the method described for intermediate 3-10 using 2,2-dimethyl-1,3-dioxane-4,6-dione (2.13 g, 14.8 mmol, CAS 2033-24-1) and (3R)-3-[(tert-butoxycarbonyl)amino]butanoic acid (3.00 g, 14.8 mmol, CAS 159991-23-8) as starting materials, 3.12 g (90 % purity, 84 % yield) of the title compound were prepared.

¹H-NMR (400 MHz, DMSO-d₆) δ [ppm]: 1.182 (2.28), 1.199 (2.39), 1.363 (2.20), 1.366 (2.21), 1.424 (16.00), 1.449 (0.70), 1.473 (0.83), 2.052 (0.45), 2.055 (0.46), 2.064 (0.63), 2.096 (0.49), 2.099 (0.48), 4.948 (0.97), 4.953 (0.96), 11.100 (0.64).

### Intermediate 3-2

### tert-butyl (2S)-2-methyl-4,6-dioxopiperidine-1-carboxylate

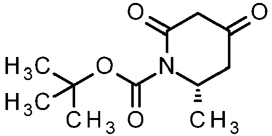

(3S)-3-[(*Tert*-butoxycarbonyl)amino]butanoic acid (1.76 g, 8.67 mmol, CAS 158851-30-0) was dissolved in dichloromethane (14 ml), cooled to 0 °C, EDC.HCl (2.49 g, 13.0 mmol), DMAP (1.59 g, 13.0 mmol) and 2,2-dimethyl-1,3-dioxane-4,6-dione (1.25 g, 8.67 mmol, CAS 2033-24-1) were added and the mixture was stirred for 3h at RT. The mixture was washed with potassium bisulfate (1 M), the organic layer was dried and concentrated under reduced pressure. The residue was dissolved in EtOAc (35 ml) and stirred for 5 h at reflux. The mixture was purified by flash chromatography (silica, DCM / EtOH gradient 0-8 %) to give 890 mg (90 % purity, 41 % yield) of the target compound.

LC-MS (method 2): Rₜ = 0.46 min; MS (ESlneg): m/z = 226 [M-H]⁻

¹H-NMR (400 MHz, DMSO-d₆) δ [ppm]: 1.182 (2.11), 1.199 (2.10), 1.237 (0.63), 1.253 (0.52), 1.362 (1.54), 1.425 (16.00), 1.450 (3.87), 1.473 (1.10), 2.055 (0.41), 2.064 (0.77), 2.096 (0.43), 2.099 (0.42), 2.210 (1.52), 3.335 (0.75), 4.948 (0.88), 4.953 (0.89), 11.098 (1.59).

### Intermediate 3-3.1

### ethyl 3-{[(2,4-dimethoxyphenyl)methyl]amino}butanoate

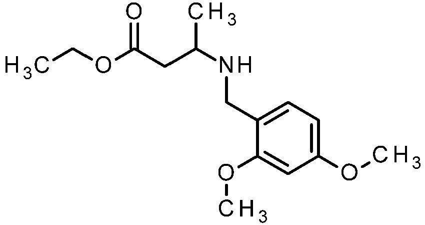

1-(2,4-dimethoxyphenyl)methanamine (13 ml, 90 mmol, CAS 20781-20-8) was dissolved in ethanol (51 ml), cooled to 0°C, ethyl (2E)-but-2-enoate (17 ml, 130 mmol, CAS 623-70-1) was added and the mixture was stirred overnight at RT. The mixture was evaporated and purified by flash chromatography (silica, hexane / EtOAc gradient 50-100 %) to give 10.2 g (90 % purity, 36 % yield) of the target compound.

LC-MS (method 2): Rₜ = 1.12 min; MS (ESlpos): m/z = 282 [M+H]⁺

¹H-NMR (400 MHz, DMSO-d₆) δ [ppm]: 1.01 (d, 3H), 1.15 (t, 3H), 1.78 (br s, 1H), 2.21 (dd, 1H), 2.43 (dd, 1H), 2.91 (sxt, 1H), 3.57 (q, 2H), 3.73 (s, 3H), 3.75 (s, 3H), 4.03 (q, 2H), 6.45 (dd, 1H), 6.51 (d, 1H), 7.16 (d, 1H).

### Intermediate 3-3.2

### ethyl 3-{[(2,4-dimethoxyphenyl)methyl](3-ethoxy-3-oxopropanoyl)amino}butanoate

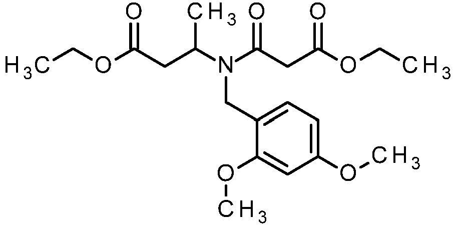

Ethyl 3-{[(2,4-dimethoxyphenyl)methyl]amino}butanoate (intermediate 3-12.1, 10.2 g, 36.3 mmol) was dissolved in dichloromethane (48 ml), cooled to 0°C, N,N-diisopropylethylamine (9.5 ml, 54 mmol) and DMAP (443 mg, 3.63 mmol) were added. ethyl 3-chloro-3-oxopropanoate (7.0 ml, 54 mmol, CAS 36239-09-5) was added dropwise (temperature < 15°C) and the mixture was stirred for 30 min at 0°C and for 24h at RT. The mixture was diluted with ammonium chloride solution, stirred for 15 min and the organic layer was separated. The aqueous phase was extracted with DCM and the combined organic layers were dried and evaporated. The residue was purified by flash chromatography (silica, hexane / EtOAc gradient 10-60%) to give 9.70 g (68% yield) of the target compound.

LC-MS (method 2): Rₜ = 1.18 min; MS (ESlpos): m/z = 396 [M+H]⁺

¹H-NMR (400 MHz, DMSO-d₆) δ [ppm]: 1.021 (4.55), 1.038 (4.55), 1.078 (4.32), 1.089 (4.59), 1.096 (9.95), 1.106 (4.48), 1.113 (4.56), 1.124 (4.47), 1.142 (9.83), 1.152 (4.41), 1.154 (2.41), 1.160 (4.65), 1.170 (9.50), 1.172 (4.21), 1.187 (4.57), 1.190 (2.20), 1.201 (4.42), 1.219 (9.90), 1.237 (4.60), 1.987 (5.16), 2.442 (0.66), 2.461 (0.98), 2.482 (2.24), 2.518 (0.93), 2.522 (0.58), 2.552 (1.10), 2.569 (1.02), 2.591 (0.55), 2.608 (0.53), 3.329 (9.77), 3.395 (5.36), 3.565 (1.63), 3.604 (1.99), 3.733 (0.56), 3.754 (16.00), 3.802 (14.13), 3.806 (14.06), 3.848 (1.95), 3.888 (1.51), 3.924 (0.51), 3.932 (0.90), 3.942 (1.66), 3.951 (1.85), 3.959 (1.82), 3.968 (1.81), 3.973 (1.34), 3.977 (1.00), 3.986 (0.82), 3.991 (3.52), 3.999 (0.63), 4.009 (3.31), 4.017 (1.32), 4.025 (1.76), 4.035 (1.30), 4.042 (4.24), 4.053 (0.54), 4.060 (4.18), 4.078 (1.32), 4.103 (1.07), 4.120 (3.04), 4.137 (2.80), 4.155 (0.89), 4.251 (0.66), 4.267 (3.84), 4.294 (1.79), 4.305 (0.67), 4.325 (2.09), 4.368 (0.78), 4.385 (0.63), 4.402 (0.61), 5.758 (3.05), 6.390 (1.07), 6.396 (1.13), 6.411 (1.13), 6.418 (1.21), 6.508 (1.10), 6.514 (1.49), 6.518 (2.40), 6.523 (2.17), 6.529 (1.21), 6.535 (1.34), 6.580 (2.30), 6.586 (1.89), 6.892 (1.65), 6.913 (1.50), 7.016 (1.60), 7.037 (1.44).

### Intermediate 3-3.3

### ethyl 1-[(2,4-dimethoxyphenyl)methyl]-6-methyl-2,4-dioxopiperidine-3-carboxylate

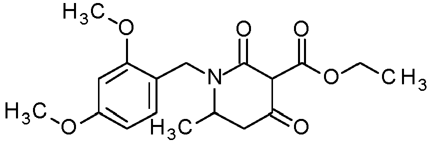

sodium ethanolate (10 ml, 21 % purity, 27 mmol, CAS 141-52-6) was cooled to 0°C, ethyl 3-{[(2,4-dimethoxyphenyl)methyl](3-ethoxy-3-oxopropanoyl)amino}butanoate (intermediate 3-12.2, 9.70 g, 24.5 mmol) dissolved in ethanol (49 ml) was added dropwise (temperature < 5°C). The mixture was stirred for 2h at RT. The mixture was quenched with 2M HCl (pH 4), extracted with DCM and the organic layer was dried and evaporated. The residue was purified by flash chromatography (silica, hexane / EtOAc gradient 20-100%) to give 6.30 g (74% yield) of the target compound.

LC-MS (method 2): Rₜ = 0.54 min; MS (ESlpos): m/z = 350 [M+H]⁺

¹H-NMR (400 MHz, DMSO-d₆) δ [ppm]: 1.096 (5.49), 1.112 (5.54), 1.154 (1.53), 1.172 (3.32), 1.190 (1.76), 1.213 (4.16), 1.231 (9.08), 1.249 (4.27), 1.987 (5.61), 2.084 (0.43), 2.518 (1.08), 2.522 (0.74), 3.733 (1.37), 3.741 (16.00), 3.746 (3.62), 3.780 (0.93), 3.790 (14.77), 3.796 (3.47), 3.999 (0.64), 4.017 (1.41), 4.035 (1.33), 4.053 (0.46), 4.165 (1.03), 4.183 (2.94), 4.201 (2.81), 4.219 (0.93), 4.740 (1.13), 4.779 (1.02), 5.759 (5.28), 6.464 (0.96), 6.470 (1.06), 6.485 (1.04), 6.490 (1.13), 6.556 (1.68), 6.561 (1.53), 6.569 (0.54), 6.575 (0.43), 7.046 (0.52), 7.065 (0.50).

### Intermediate 3-13

### tert-butyl 5-(2-fluoroethyl)-2,4-dioxopiperidine-1-carboxylate

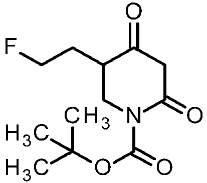

*Tert*-butyl 2,4-dioxopiperidine-1-carboxylate (5.00 g, 23.4 mmol) and 1-bromo-2-fluoroethane (6.6 ml, 88 mmol) were dissolved in THF (120 ml), cooled to -20°C, LiHMDS (70 ml, 1.0 M in THF, 70 mmol) was added dropwise and the mixture was stirred for 1h. The mixture was diluted with ammonium chloride solution and the organic layer was separated. The aqueous phase was adjusted to pH 6 with HCl (2M), extracted 2 times with DCM and the combined organic layers were dried and evaporated to give 4.10 g (67% yield) of the target compound.

LC-MS (method 2): Rₜ = 0.49 min; MS (ESlpos): m/z = 260 [M+H]⁺

### Intermediate 3-49

### tert-butyl (4aS,7aR)-2,4-dioxooctahydro-1H-cyclopenta[b]pyridine-1-carboxylate

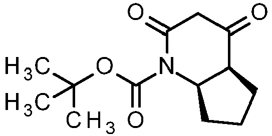

To an icecooled mixture of (1S,2R)-2-[(tert-butoxycarbonyl)amino]cyclopentane-1-carboxylic acid (1.00 g, 4.36 mmol, CAS 137170-89-9) in DCM (7.0 ml) was added 1-ethyl-3-(3-dimethylaminopropyl)carbodiimid (1.25 g, 6.54 mmol; CAS 25952-53-8), 4-dimethylaminopyridine (799 mg, 6.54 mmol; CAS 1122-58-3) and 2,2-dimethyl-1,3-dioxane-4,6-dione (629 mg, 4.36 mmol, CAS 2033-24-1). The mixture was stirred at room temperature for 3 h. The reaction mixture was washed with aqueous potassiumhydrogensulfate solution (1 M). The organic layer was dried over a water repellant filter and concentrated under reduced pressure. The residue was dissolved in EtOAc and stirred under reflux for 5 h. The mixture was evaporated and purified by flash chromatography (silica, DCM / EtOH gradient 0-10 %) to give 834 mg (75 % yield) of the title compound.

LC-MS (method 2): Rₜ = 0.60 min; MS (ESlneg): m/z = 252 [M-H]⁻

### Intermediate 3-55.1

### Ethyl cis-2-[(2,4-dimethoxybenzyl)amino]cyclopentanecarboxylate

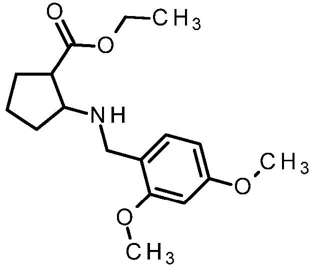

To a solution of ethyl cis-2-aminocyclopentanecarboxylate (40.0 g, 254 mmol, CAS 114745-45-8) in THF (200 ml) was added sodium triacetoxyborohydride (107 g, 508 mmol), AcOH (15.9 g, 266 mmol) and 2,4-dimethoxybenzaldehyde (42.2 g, 254 mmol) at 0 °C. The mixture was stirred at 50°C for 12 h. The reaction mixture was partitioned between water 500 ml and ethyl acetate 600 ml. The organic phase was separated, washed with NaHCO₃ (200 ml x 3) and brine 300 ml, dried over Na₂SO₄, filtered and concentrated under reduced pressure to give the title compound (20.0 g, 25.5% yield) as a brown oil.

LC-MS (MS instrument type: SHIMADZU LCMS-2020, Column: Kinetex EVO C18 2.1 x 30mm, 5um, mobile phase A: 0.025% ammonia in water , B: acetonitrile, gradient: 0.0 min 5% B→0.8 min 95% B→1.2 min 95% B→1.21 min 5% B→1.55 min 5%B, flow rate: 1.5 mL/min, oven temperature: 40°C; PDA detection: 220 nm & 254 nm.): Rₜ = 1.091 min; MS (ESlpos): m/z = 308.3 [M+H]⁺

### Intermediate 3-55.2

### Ethyl cis-2-[(2,4-dimethoxybenzyl)(3-ethoxy-3-oxopropanoyl)amino]cyclopentanecarboxylate

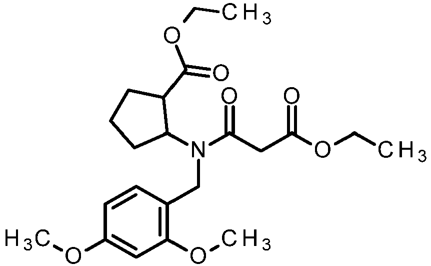

To a solution of ethyl cis-2-[(2,4-dimethoxybenzyl)amino]cyclopentanecarboxylate (20.0 g, 65.0 mmol, 1.00 *eq*) in DCM (100 mL) was added TEA (13.2 g, 130 mmol, 2.00 *eq*) and ethyl 3-chloro-3-oxopropanoate (10.7 g, 71.5 mmol, 1.10 *eq*) at 0 °C. The mixture was stirred at 25 °C for 12 hrs. The reaction mixture was quenched by addition of water (100 ml) at 25 °C, and extracted with DCM 150 ml. The combined organic layers were washed with brine, dried over Na₂SO₄, filtered and concentrated under reduced pressure to give a residue. The residue was purified by flash chromatography (silica, petroleum ether / EtOAc 0 - 100%) to give the title compound (12.0 g, 28.4 mmol, 43.7% yield) as a yellow oil.

¹H-NMR (400 MHz, CDCl₃) δ [ppm]: 7.08-6.92 (m, 1H), 6.46-6.40 (m, 2H), 4.88-4.85 (m, 1H), 4.42-4.41 (m, 1H), 4.18-4.12 (m, 5H), 3.82-3.77 (m, 6H), 3.37-3.22 (m, 2H), 2.03-1.92 (m, 1H), 1.88-1.83 (m, 4H), 1.32-1.30 (m, 1H), 1.27-1.24 (m, 7H).

### Intermediate 3-55.3

### Ethyl cis-1-(2,4-dimethoxybenzyl)-2,4-dioxooctahydro-1H-cyclopenta[b]pyridine-3-carboxylate

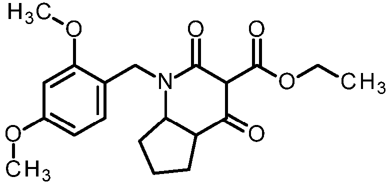

To a solution of ethyl cis-2-[(2,4-dimethoxybenzyl)(3-ethoxy-3-oxopropanoyl)amino]cyclo-pentanecarboxylate (16.0 g, 37.9 mmol) in DMF (300 mL) was added Cs₂CO₃ (24.7 g, 75.9 mmol). The mixture was stirred at 80 °C for 2 h. The reaction mixture was filtered and concentrated under reduced pressure to give the title compound (15.0 g, crude) as a yellow liquid, which was used directly in the next step.

LC-MS (method 4): Rₜ = 0.780 min; MS (ESlpos): m/z = 367.3 [M+H]⁺

### Intermediate 3-63

### 2-(2,2-difluoroethyl)-1-fluoro-3-isothiocyanatobenzene

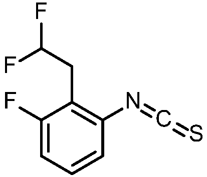

To oxalic acid-2-(2,2-difluoroethyl)-3-fluoroaniline (1/1) (1.60 g, 6.03 mmol, CAS 2311903-06-5) in DCM (20 ml) and sodium bicarbonate solution (40 mL) was slowly added thiophosgene (509 µl, 6.6 mmol) over 30 minutes at 0°C. The reaction mixture was stirred for 2 h at 0°C and then warmed to RT. Phases were separated and the organic phase was wasched with brine, dried and evaporated to give 1.03 g (79 % yield) of the title compound.

¹H-NMR (400 MHz, DMSO-d₆) δ [ppm]: 3.25 - 3.38 (m, 2H), 6.18 - 6.52 (m, 1H), 7.27 - 7.34 (m, 1H), 7.36 - 7.42 (m, 1H), 7.42 - 7.51 (m, 1H).

### Intermediate 3-98

### tert-butyl 2,4-dioxo-5-(3,3,3-trifluoropropyl)piperidine-1-carboxylate

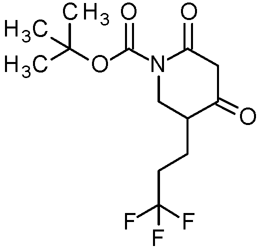

*Tert*-butyl 2,4-dioxopiperidine-1-carboxylate (2.00 g, 9.38 mmol) and lithium chloride (56 ml, 0.50 M, 28 mmol) were dissolved in THF (80 ml), cooled to -20°C, LiHMDS (28 ml, 1.0 M in THF, 28 mmol) was added dropwise and the mixture was stirred for 30 min. 1,1,1-trifluoro-3-iodopropane (5.5 ml, 47 mmol) was added and the mixture was stirred for 30 min at 0°C and at RT for overnight. The mixture was diluted with ammonium chloride solution to adjust pH 6 and was extracted with EtOAc. The combined organic layers were dried and evaporated. The residue was purified by flash chromatography (silica, Hex / EtOAc gradient 30-100%) to give 620 mg (21% yield, impure with tert-butyl 5-(3,3-difluoroallyl)-2,4-dioxo-piperidine-1-carboxylate) of the title compound.

LC-MS (method 2): Rₜ = 0.59 min; MS (ESlneg): m/z = 308 [M-H]⁻

¹H-NMR (400 MHz, DMSO-d₆) δ [ppm]: 1.157 (0.58), 1.175 (1.21), 1.193 (0.61), 1.365 (1.67), 1.392 (0.54), 1.406 (0.97), 1.422 (9.65), 1.428 (16.00), 1.466 (1.90), 1.989 (2.17), 2.325 (0.52), 2.339 (0.52), 2.354 (0.58), 2.368 (0.45), 2.382 (0.41), 2.525 (0.54), 3.682 (0.58), 3.693 (0.58), 3.750 (0.61), 3.763 (0.57), 4.020 (0.52), 4.037 (0.51), 4.944 (1.05), 4.952 (1.84).

### Intermediate 3-114

### tert-butyl 5-allyl-2,4-dioxo-piperidine-1-carboxylate

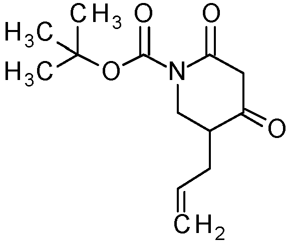

According to the method described for Intermediate 3-13 using *tert*-butyl 2,4-dioxopiperidine-1-carboxylate (250 mg, 1.17 mmol) and 3-bromoprop-1-ene (410 µl, 4.7 mmol) as starting materials, 105 mg (35% yield) of the title compound were prepared after purification by flash chromatography (silica, hexane / EtOAc gradient 20-100 %).

LC-MS (method 2): Rₜ = 0.58 min; MS (ESlpos): m/z = 252 [M-H]⁺

¹H-NMR (400 MHz, DMSO-d₆) δ [ppm]: 1.42 (s, 9H), 1.88 - 2.14 (m, 1H), 2.38 (br d, 2H), 3.50 - 3.78 (m, 2H), 4.92 (br s, 1H), 5.00 - 5.12 (m, 2H), 5.68 - 5.85 (m, 1H), 11.25 (br s, 1H).

### Syntheses of Intermediate 4 Compounds

### Intermediate 4-1

### tert-butyl (2R)-5-[(3-chloro-2-methoxyphenyl)carbamothioyl]-4-hydroxy-2-methyl-6-oxo-3,6-dihydropyridine-1(2H)-carboxylate

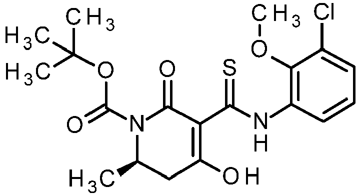

1-Chloro-3-isothiocyanato-2-methoxybenzene (780 mg, 3.91 mmol, CAS 127142-64-7) and *tert*-butyl (2*R*)-2-methyl-4,6-dioxopiperidine-1-carboxylate (intermediate 3-1, 3.12 g, 13.7 mmol) were dissolved in acetonitrile (18 ml), cooled to 0°C, DBU (870 µl, 5.9 mmol) was added and the mixture was stirred for 4h at RT. The mixture was poured into aqueous HCl (1%) and stirred for 20 min. The phases were separated and the aqueous layer was extracted with DCM, the combined organic layers were dried and evaporated. The residue was purified by flash chromatography (silica, hexane / EtOAc gradient 0-80%) to give 3.24 g (95% purity, 53% yield) of the title compound.

LC-MS (method 2): Rₜ = 0.71 min; MS (ESlneg): m/z = 425 [M-H]⁻

¹H-NMR (400 MHz, DMSO-d₆) δ [ppm]: 1.29 (d, 3H), 1.49 (s, 9H), 2.52 - 2.59 (m, 1H), 3.37 - 3.45 (dd, 1H), 3.77 (s, 3H), 4.32 -4.38 (m, 1H), 7.22 (t, 1H), 7.47 (dd, 1H), 7.74 - 7.80 (dd, 1H), 13.47 (br s, 1H), 16.42 (s, 1H).

### Intermediate 4-2

### tert-butyl (2S)-5-[(3-chloro-2-methoxyphenyl)carbamothioyl]-4-hydroxy-2-methyl-6-oxo-3,6-dihydropyridine-1(2H)-carboxylate

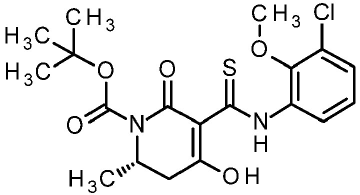

1-Chloro-3-isothiocyanato-2-methoxybenzene (780 mg, 3.91 mmol, , CAS 127142-64-7) and *tert*-butyl (2*S*)-2-methyl-4,6-dioxopiperidine-1-carboxylate (intermediate 3-2, 888 mg, 3.91 mmol) were dissolved in acetonitrile (18 ml), cooled to 0°C, DBU (870 µl, 5.9 mmol) was added and the mixture was stirred for 4h at RT. The mixture was poured into HCl (1%) and stirred for 20 min. The phases were separated and the aqueous layer was extracted with DCM, the combined organic layers were dried and evaporated. The residue was purified by flash chromatography (silica, hexane / EtOAc gradient 0-100 %) to give 1.40 g (85 % purity, 71 % yield) of the target compound.

LC-MS (method 2): Rₜ = 0.71 min; MS (ESlneg): m/z = 425 [M-H]⁻

¹H-NMR (400 MHz, DMSO-d₆) δ [ppm]: 1.30 (d, 3H), 1.49 (s, 9H), 2.52 - 2.60 (m, 1H), 3.41 (dd, 1H), 3.77 (s, 3H), 4.32 - 4.39 (m, 1H), 7.22 (t, 1H), 7.46 (dd, 1H), 7.72 - 7.80 (m, 1H), 13.47 (br s, 1H), 16.42 (br s, 1H).

### Intermediate 4-3

### 1-[(2,4-dimethoxyphenyl)methyl]-6-methylpiperidine-2,4-dione

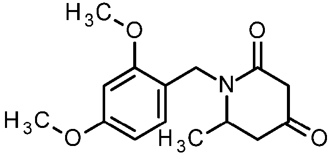

Ethyl 1-[(2,4-dimethoxyphenyl)methyl]-6-methyl-2,4-dioxopiperidine-3-carboxylate (intermediate 3-3.3, 6.30 g, 18.0 mmol) was dissolved in dichloromethane (43 ml), HCI (43 ml, 2.0 M, 86 mmol) was added and the mixture was stirred for 15 min at RT. The phases were separated and the aqueous phase was washed with DCM. The combined organic layers were dried and evaporated. The residue was dissolved in acetonitrile (61 ml) and water (8.3 ml) and stirred for 2h at reflux. The mixture was concentrated under reduced pressure to give 4.68 g (90 % purity, 84 % yield) of the target compound, which was used without further purification.

LC-MS (method 2): Rₜ = 0.60 min; MS (ESlpos): m/z = 278 [M+H]⁺

¹H-NMR (400 MHz, DMSO-d₆) δ [ppm]: 1.085 (1.53), 1.092 (4.76), 1.102 (1.69), 1.109 (4.79), 2.354 (0.67), 2.359 (0.68), 2.394 (0.81), 2.399 (0.81), 2.780 (0.67), 2.794 (0.71), 2.819 (0.58), 2.834 (0.58), 3.237 (1.17), 3.288 (2.10), 3.404 (1.88), 3.456 (1.05), 3.701 (0.44), 3.708 (0.48), 3.717 (0.63), 3.725 (0.69), 3.734 (3.76), 3.747 (16.00), 3.781 (2.91), 3.797 (13.19), 4.179 (1.36), 4.217 (1.52), 4.754 (1.52), 4.792 (1.33), 4.919 (0.58), 4.924 (0.58), 5.758 (5.33), 6.465 (1.20), 6.471 (1.24), 6.486 (1.24), 6.492 (1.29), 6.540 (0.53), 6.545 (0.50), 6.570 (2.24), 6.576 (2.01), 6.982 (0.41), 7.089 (1.79), 7.110 (1.65), 10.404 (0.41).

### Intermediate 4-13

### tert-butyl 5-[(3-chloro-2-methoxyphenyl)carbamothioyl]-3-(2-fluoroethyl)-4-hydroxy-6-oxo-3,6-dihydropyridine-1(2H)-carboxylate

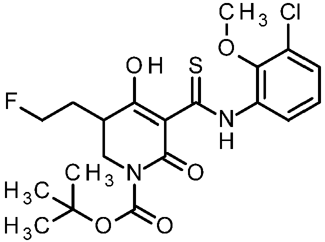

According to the method described for intermediate 4-1 using 1-chloro-3-isothiocyanato-2-methoxybenzene (1.85 g, 9.26 mmol, CAS 127142-64-7) and *tert-*butyl 5-(2-fluoroethyl)-2,4-dioxopiperidine-1-carboxylate (intermediate 3-13, 2.40 g, 9.26 mmol) as starting materials, 1.78 g (70% purity, 29% yield) of the title compound were prepared after purification by flash chromatography (silica, EtOAc / EtOH gradient 0-10%).

LC-MS (method 2): Rₜ = 0.74 min; MS (ESlpos): m/z = 459 [M+H]⁺

### Intermediate 4-30

### tert-butyl 5-[(3-chloro-2-methoxy-phenyl)carbamothioyl]-4-hydroxy-3-methyl-6-oxo-2,3-dihydropyridine-1-carboxylate

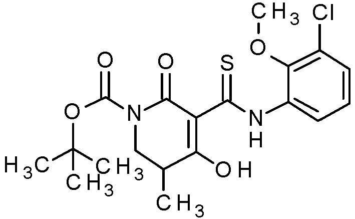

According to the method described for intermediate 4-1 using 1-chloro-3-isothiocyanato-2-methoxybenzene (1.67 g, 8.36 mmol, CAS 127142-64-7) and *tert-*butyl 5-methyl-2,4-dioxo-piperidine-1-carboxylate (2.00 g, 95 % purity, 8.36 mmol, CAS 942425-69-6) as starting materials, 1.2 g (80% purity, 27% yield) of the title compound were prepared after purification by flash chromatography (silica, Hexane / EtOAc gradient 0-20%).

LC-MS (method 2): Rₜ = 0.68 min; MS (ESlpos): m/z = 427 [M+H]⁺

¹H-NMR (400 MHz, DMSO-d₆) δ [ppm]: 1.156 (0.74), 1.174 (1.44), 1.192 (0.79), 1.211 (1.65), 1.228 (1.78), 1.238 (0.76), 1.364 (0.99), 1.491 (16.00), 1.501 (1.09), 1.989 (2.45), 2.520 (0.79), 2.525 (0.51), 3.672 (0.59), 3.776 (6.64), 3.801 (1.68), 3.805 (1.69), 3.808 (0.88), 3.890 (0.51), 4.020 (0.56), 4.037 (0.56), 7.203 (0.48), 7.224 (0.98), 7.244 (0.55), 7.458 (0.52), 7.462 (0.54), 7.479 (0.46), 7.482 (0.44).

### Intermediate 4-49

### tert-butyl (4aS,7aR)-3-[(3-chloro-2-methoxyphenyl)carbamothioyl]-4-hydroxy-2-oxo-2,4a,5,6,7,7a-hexahydro-1H-cyclopenta[b]pyridine-1-carboxylate

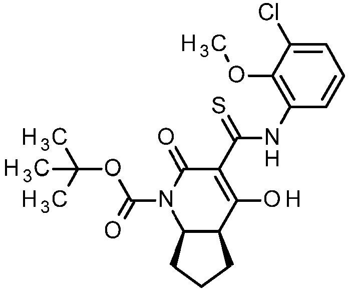

According to the method described for intermediate 4-10 using 1-chloro-3-isothiocyanato-2-methoxybenzene (654 mg, 3.28 mmol, CAS 127142-64-7) and *tert-*butyl (4a*S*,7a*R*)-2,4-dioxooctahydro-1*H*-cyclopenta[b]pyridine-1-carboxylate (intermediate 3-49, 830 mg, 3.28 mmol) as starting materials, 908 mg (80% purity, 49% yield) of the title compound were prepared after purification by flash chromatography (silica, DCM / EtOH gradient 0-10 %).

LC-MS (method 2): Rₜ = 0.81 min; MS (ESlpos): m/z = 453 [M+H]⁺

¹H-NMR (400 MHz, DMSO-d₆) δ [ppm]: 1.42 - 1.55 (s, 9H), 1.56 - 1.69 (m, 3H), 1.99 - 2.18 (m, 3H), 3.62 - 3.70 (s, 1H), 3.74 - 3.78 (s, 3H), 7.22 (t, 1H), 7.47 (d, 1H), 7.74 (br d, 1H), 13.63 (br s, 1H), 16.33 - 16.67 (br s, 1H).

### Intermediate 4-55

### cis-1-(2,4-dimethoxybenzyl)tetrahydro-1H-cyclopenta[b]pyridine-2,4(3H,4aH)-dione

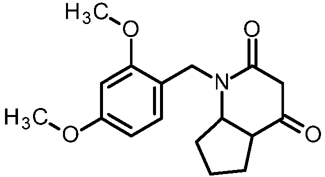

To a solution of ethyl *cis*-1-(2,4-dimethoxybenzyl)-2,4-dioxooctahydro-1*H*-cyclopenta[b] pyridine-3-carboxylate (intermediate 3-55.3, 15.0 g, 39.9 mmol) in DMF (200 ml) was added aq. HCI (1 M, 39.9 ml). The mixture was stirred at 90 °C for 1 h. The reaction mixture was partitioned between water (500 ml) and EtOAc (800 ml). The organic phase was separated, washed with brine, dried over Na₂SO₄, filtered and concentrated under reduced pressure to give a residue. The crude product was purified by reversed-phase HPLC (0.1% HCI in water / ACN) followed by flash chromatography (silica, petroleum ether / EtOAc 0 - 100%) to give the title compound (11.0 g, 35.3 mmol, 88.3% yield, 97.4% purity) as a yellow oil.

LC-MS (method 3): Rₜ = 0.862 min; MS (ESlpos): m/z = 304.2 [M+H]⁺

¹H-NMR (400 MHz, CDCl₃) δ [ppm]: 7.21-7.18 (m, 1H), 6.48-6.43 (m, 2H), 5.02 (d, 1H), 4.36 (d, 1H), 3.86-3.84 (m, 1H), 3.82 (s, 3H), 3.81 (s, 3H), 3.49-3.39 (m, 2H), 2.88-2.85 (m, 1H), 2.23-2.22 (m, 1H), 2.21-2.05 (m, 1H), 1.79-1.73 (m, 2H), 1.29-1.26 (m, 1H), 1.26-1.25 (m, 1H).

### Intermediate 4-58

### tert-butyl 5-[(3-chloro-2-methyl-phenyl)carbamothioyl]-4-hydroxy-3-methyl-6-oxo-2,3-dihydropyridine-1-carboxylate

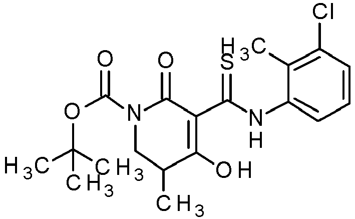

According to the method described for intermediate 4-1 using 1-chloro-3-isothiocyanato-2-methylbenzene (2.44 g, 13.3 mmol, CAS 19241-35-1) and *tert-*butyl 5-methyl-2,4-dioxopiperidine-1-carboxylate (3.02 g, 13.3 mmol, CAS 942425-69-6) as starting materials, 4.00 g (95% purity, 70% yield) of the title compound were prepared.

LC-MS (method 2): Rₜ = 0.70 min; MS (ESlpos): m/z = 411 [M+H]⁺

¹H-NMR (400 MHz, DMSO-d₆) δ [ppm]: 1.21 (d, 3H), 1.49 (s, 9H), 2.20 (s, 3H), 2.91 - 3.02 (m, 1H), 3.59 (dd, 1H), 3.85 (dd, 1H), 7.18 - 7.22 (m, 1H), 7.30 (t, 1H), 7.46 (d, 1H), 12.85 (br s, 1H), 16.06 (br s, 1H).

### Intermediate 4-63

### tert-butyl (2R)-5-{[2-(2,2-difluoroethyl)-3-fluorophenyl]carbamothioyl}-4-hydroxy-2-methyl-6-oxo-3,6-dihydropyridine-1(2H)-carboxylate

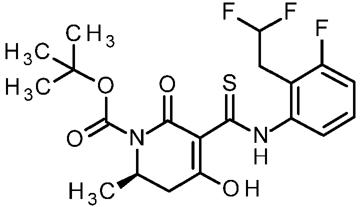

According to the method described for intermediate 4-1 using 2-(2,2-difluoroethyl)-1-fluoro-3-isothiocyanatobenzene (intermediate 3-63, 813 mg, 3.74 mmol) and *tert-*butyl (2*R*)-2-methyl-4,6-dioxopiperidine-1-carboxylate (intermediate 3-1, 654 mg, 2.88 mmol) as starting materials, 1.31 g (quantitative) of the title compound were prepared after purification by flash chromatography (silica Hexane / EtOAc gradient 50-100% and DCM / EtOH gradient 0-50%).

LC-MS (method 2): Rₜ = 0.68 min; MS (ESlneg): m/z = 443 [M-H]⁻

¹H-NMR (400 MHz, DMSO-d₆) δ ppm: 1.31 (d, 3 H), 1.48 (s, 9 H), 2.47 (d, 1 H), 3.02 - 3.32 (m, 3 H), 4.28 - 4.43 (m, 1 H), 6.05 - 6.39 (m, 1 H), 7.19 (d, 1 H), 7.25 - 7.32 (m, 1 H), 7.42 - 7.52 (m, 1 H), 12.78 (br s, 1 H), 15.24 (br s, 1 H).

### Intermediate 4-91

### tert-butyl (2R)-5-[(3-chloro-2-ethylphenyl)carbamothioyl]-4-hydroxy-2-methyl-6-oxo-3,6-dihydropyridine-1(2H)-carboxylate

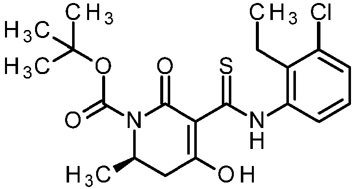

According to the method described for intermediate 4-1 using 1-chloro-2-ethyl-3-isothiocyanatobenzene (intermediate 3-91, 2.00 g, 10.1 mmol) and *tert-*butyl (2R)-2-methyl-4,6-dioxopiperidine-1-carboxylate (intermediate 3-1, 2.30 g, 10.1 mmol) as starting materials, 4.15 g (90 % purity, 87 % yield)of the title compound were prepared after purification by flash chromatography (silica, hexane / EtOAc gradient 20-60%).

LC-MS (method 2): Rₜ = 0.81 min; MS (ESlpos): m/z = 423.3 [M+H]⁺

¹H-NMR (400 MHz, DMSO-d₆) δ [ppm]: 1.04 - 1.13 (t, 3H), 1.27 - 1.35 (d, 3H), 1.48 (s, 9H), 2.52 - 2.56 (m, 1H), 2.57 - 2.71 (m, 2H), 3.35 - 3.40 (m, 1H), 4.36 (td, 1H), 7.25 - 7.34 (m, 2H), 7.45 (dd, 1H), 13.06 (br s, 1H), 16.05 (br s, 1H).

### Intermediate 4-98

### tert-butyl 5-[(3-chloro-2-methoxy-phenyl)carbamothioyl]-4-hydroxy-6-oxo-3-(3,3,3-trifluoropropyl)-2,3-dihydropyridine-1-carboxylate

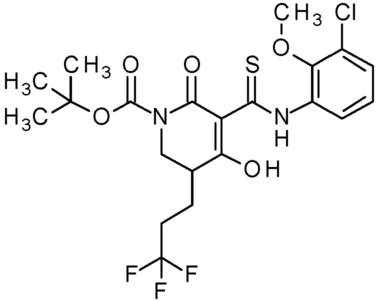

According to the method described for intermediate 4-1 using 1-chloro-3-isothiocyanato-2-methoxybenzene (438 mg, 1.97 mmol, CAS 127142-64-7) and *tert-*butyl 2,4-dioxo-5-(3,3,3-trifluoropropyl)piperidine-1-carboxylate (intermediate 3-98, 610 mg, 1.97 mmol, impure with *tert-*butyl 5-(3,3-difluoroallyl)-2,4-dioxo-piperidine-1-carboxylate) as starting materials, 670 mg (67% yield, impure with *tert-*butyl 5-[(3-chloro-2-methoxyphenyl)carbamothioyl]-3-(3,3-difluoroprop-2-en-1-yl)-4-hydroxy-6-oxo-3,6-dihydropyridine-1(2*H*)-carboxylate) of the title compound were prepared after purification by flash chromatography (silica, hexane / EtOAc gradient 20-60%).

LC-MS (method 2): Rₜ = 0.84 min; MS (ESlneg): m/z = 507 [M-H]⁻

### Intermediate 4-102

### tert-butyl (2R)-5-[(3-chloro-2-methylphenyl)carbamothioyl]-4-hydroxy-2-methyl-6-oxo-3,6-dihydropyridine-1(2H)-carboxylate

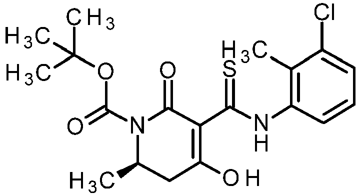

According to the method described for intermediate 4-1 using 1-chloro-3-isothiocyanato-2-methylbenzene (2.8 ml, 18 mmol; CAS 19241-35-1) and *tert-*butyl (2*R*)-2-methyl-4,6-dioxopiperidine-1-carboxylate (intermediate 3-1, 4.00 g, 17.6 mmol) as starting materials, 7.1 g (98% yield) of the title compound were prepared.

LC-MS (method 2): Rₜ = 0.81 min; MS (ESlpos): m/z = 411.4 [M+H]⁺

### Intermediate 4-114

### tert-butyl 3-allyl-5-[(3-chloro-2-methoxy-phenyl)carbamothioyl]-4-hydroxy-6-oxo-2,3-dihydropyridine-1-carboxylate

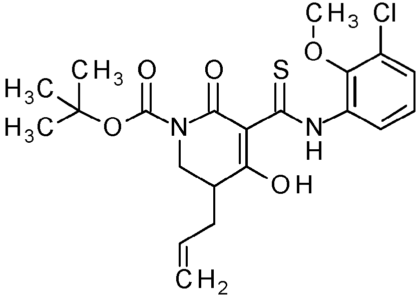

According to the method described for Intermediate 4-1 using 1-chloro-3-isothiocyanato-2-methoxybenzene (1.36 g, 6.12 mmol, CAS 127142-64-7) and *tert-*butyl 5-allyl-2,4-dioxo-piperidine-1-carboxylate (Intermediate 3-114, 1.55 g, 6.12 mmol) as starting materials, 2.32 g (90% purity, 75% yield) of the title compound were prepared after purification by flash chromatography (silica, hexane / EtOAc gradient 20-60%).

LC-MS (method 2): Rₜ = 0.79 min; MS (ESlneg): m/z = 451 [M-H]⁻

¹H-NMR (400 MHz, DMSO-d₆) δ [ppm]: 0.000 (3.19), 1.175 (0.18), 1.481 (16.00), 1.990 (0.34), 2.270 (0.19), 2.305 (0.24), 2.329 (0.17), 2.521 (0.43), 2.526 (0.29), 2.542 (0.18), 2.906 (0.20), 2.917 (0.26), 2.929 (0.25), 2.941 (0.19), 3.752 (0.55), 3.763 (0.89), 3.772 (0.65), 3.782 (7.69), 3.802 (0.37), 3.890 (0.23), 5.095 (0.37), 5.134 (0.74), 5.157 (0.45), 5.760 (4.53), 5.812 (0.19), 5.834 (0.18), 7.205 (0.54), 7.225 (1.16), 7.245 (0.65), 7.460 (0.63), 7.463 (0.68), 7.480 (0.55), 7.484 (0.55), 7.712 (0.47), 7.714 (0.48), 7.731 (0.45), 7.734 (0.43), 13.308 (0.19).

### Syntheses of Intermediate 5 Compounds

### Intermediate 5-1

### (6R)-N-(3-chloro-2-methoxyphenyl)-4-hydroxy-6-methyl-2-oxo-1,2,5,6-tetrahydropyridine-3-carbothioamide

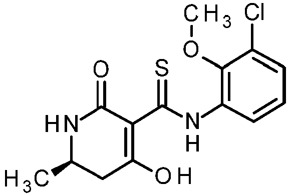

*Tert*-butyl(2*R*)-5-[(3-chloro-2-methoxyphenyl)carbamothioyl]-4-hydroxy-2-methyl-6-oxo-3,6-dihydropyridine-1(2*H*)-carboxylate (intermediate 4-1, 3.24 g, 7.59 mmol) was dissolved in dichloromethane (45 ml), TFA (5.9 ml, 75.9 mmol) was added and the mixture was stirred for 1h at RT. The mixture was evaporated, diluted with EtOAc, washed with sodium bicarbonate and sodium chloride solution, dried and concentrated under reduced pressure. The residue was purified by flash chromatography (silica, hexane / EtOAc gradient 40-100%) to give 2.01 g (95% purity, 77% yield) of the title compound.

LC-MS (method 2): Rₜ = 0.55 min; MS (ESlpos): m/z = 327 [M+H]⁺

¹H-NMR (400 MHz, DMSO-d₆) δ [ppm]: 1.20 (dd, 3H), 2.53 - 2.78 (m, 2H), 3.57 - 3.84 (m, 4H), 7.19 (dt, 1H), 7.41 (ddd, 1H), 7.83 (dd, 1H), 8.14 - 8.27 (br s, 1H), 9.29 - 9.45 (br s, 1H), 14.25 - 14.35 (br s, 0,5H), 14.69 - 14.81 (br s, 0,5H), 16.46 (s, 1H).

### Intermediate 5-2

### (6S)-N-(3-chloro-2-methoxyphenyl)-4-hydroxy-6-methyl-2-oxo-1,2,5,6-tetrahydropyridine-3-carbothioamide

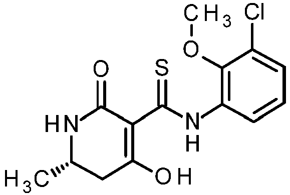

*Tert-*butyl (2*S*)-5-[(3-chloro-2-methoxyphenyl)carbamothioyl]-4-hydroxy-2-methyl-6-oxo-3,6-dihydropyridine-1(2*H*)-carboxylate (intermediate 4-2, 1.40 g, 3.28 mmol) was dissolved in dichloromethane (19 ml), TFA (2.5 ml, 33 mmol) was added and the mixture was stirred for 2h at RT. The mixture was evaporated, diluted with EtOAc, washed with sodium bicarbonate and sodium chloride solution, dried and concentrated under reduced pressure. The residue was purified by flash chromatography (silica, hexane / EtOAc gradient 20-100%) to give 730 mg (95% purity, 65% yield) of the title compound.

LC-MS (method 2): Rₜ = 0.58 min; MS (ESlpos): m/z = 327 [M+H]⁺

¹H-NMR (400 MHz, DMSO-d₆) δ [ppm]: 1.17 - 1.24 (dd, 3H), 2.53 - 2.78 (m, 2H), 3.62 - 3.71 (m, 0,5H), 3.74 (d, 3H), 3.76 - 3.82 (m, 0,5H), 7.19 (dt, 1H), 7.41 (ddd, 1H), 7.83 (ddd, 1H), 8.14 - 8.29 (s, 0,5H), 9.31 - 9.43 (s, 0,5H), 14.25 - 14.35 (s, 0,5H), 14.75 (s, 0,5H), 16.46 (s, 1H).

### Intermediate 5-3

### N-(3-chloro-2-methoxyphenyl)-1-[(2,4-dimethoxyphenyl)methyl]-4-hydroxy-6-methyl-2-oxo-1,2,5,6-tetrahydropyridine-3-carbothioamide

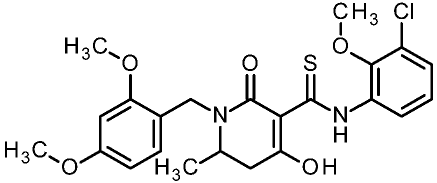

1-Chloro-3-isothiocyanato-2-methoxybenzene (560 mg, 2.52 mmol, CAS 127142-64-7) and 1-[(2,4-dimethoxyphenyl)methyl]-6-methylpiperidine-2,4-dione (intermediate 4-3, 700 mg, 2.52 mmol) were dissolved in acetonitrile (50 ml), cooled to 0 °C and DBU (0.69 ml, 4.6 mmol) was added dropwise. The mixture was stirred at RT for 2 h. The reaction was quenched with aq. HCI (1 M), stirred for 20 min and extracted with EtOAc. The organic layer was dried and concentrated under reduced pressure. The residue was purified by flash chromatography (silica, hexane / EtOAc gradient 20-60%) to give 1.15 g (96% yield) of the title compound.

LC-MS (method 2): Rₜ = 0.97 min; MS (ESlpos): m/z = 477 [M+H]⁺

¹H-NMR (400 MHz, DMSO-d₆) δ [ppm]: 0.000 (5.66), 1.157 (3.29), 1.166 (3.90), 1.175 (7.08), 1.183 (4.21), 1.193 (3.58), 1.203 (0.65), 1.991 (10.14), 2.459 (0.78), 2.464 (0.80), 2.521 (0.49), 3.238 (0.56), 3.255 (0.64), 3.281 (0.55), 3.298 (0.56), 3.334 (8.09), 3.673 (1.08), 3.685 (0.60), 3.690 (0.59), 3.702 (0.43), 3.735 (1.97), 3.750 (16.00), 3.755 (15.26), 3.769 (1.88), 3.803 (0.41), 3.816 (12.05), 3.835 (1.63), 4.002 (0.84), 4.020 (2.49), 4.038 (2.44), 4.056 (0.78), 4.168 (1.23), 4.206 (1.34), 4.812 (1.44), 4.850 (1.30), 6.489 (1.02), 6.495 (1.13), 6.510 (1.07), 6.516 (1.22), 6.588 (2.21), 6.594 (1.95), 7.158 (1.87), 7.179 (1.91), 7.191 (1.11), 7.211 (2.36), 7.232 (1.39), 7.427 (1.36), 7.431 (1.43), 7.448 (1.16), 7.451 (1.13), 7.820 (1.12), 7.822 (1.13), 7.840 (1.07), 7.843 (1.02), 14.564 (1.92), 16.515 (4.18), 17.239 (0.46).

### Intermediate 5-8

### 1-[(2,4-dimethoxyphenyl)methyl]-N-(3-fluoro-2-methoxyphenyl)-4-hydroxy-6-methyl-2-oxo-1,2,5,6-tetrahydropyridine-3-carbothioamide

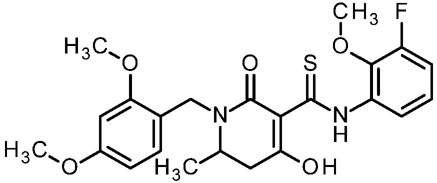

According to the method described for intermediate 5-3 using 1-fluoro-3-isothiocyanato-2-methoxybenzene (intermediate 3-17, 1.39 g, 7.57 mmol, CAS 1360955-02-7) and 1-[(2,4-dimethoxyphenyl)methyl]-6-methylpiperidine-2,4-dione (intermediate 4-3, 2.10 g, 7.57 mmol) as starting materials, 3.02 g (87% yield) of the title compound were prepared after purification by flash chromatography (silica, hexane / EtOAc gradient 20-60%).

LC-MS (method 2): Rₜ = 0.92 min; MS (ESlpos): m/z = 461 [M+H]⁺

¹H-NMR (400 MHz, DMSO-d6) δ [ppm]: 1.155 (0.63), 1.164 (3.80), 1.173 (1.57), 1.180 (4.36), 1.190 (0.80), 1.197 (0.63), 1.988 (1.39), 2.450 (0.67), 2.455 (0.66), 2.518 (1.65), 2.523 (1.12), 3.230 (0.53), 3.247 (0.59), 3.273 (0.51), 3.291 (0.51), 3.674 (0.52), 3.679 (0.52), 3.752 (16.00), 3.767 (1.88), 3.813 (12.69), 3.822 (1.73), 3.824 (1.79), 3.835 (9.12), 3.839 (8.67), 3.869 (0.44), 3.872 (0.44), 4.148 (1.18), 4.186 (1.30), 4.810 (1.43), 4.848 (1.31), 5.758 (9.32), 6.485 (1.05), 6.491 (1.16), 6.506 (1.10), 6.512 (1.26), 6.584 (2.20), 6.590 (1.99), 7.123 (0.42), 7.138 (0.50), 7.144 (1.00), 7.152 (1.89), 7.159 (1.08), 7.164 (0.84), 7.173 (1.79), 7.179 (0.79), 7.214 (0.70), 7.218 (0.76), 7.235 (0.49), 7.242 (0.71), 7.245 (0.70), 7.263 (0.43), 7.266 (0.40), 7.642 (1.04), 7.662 (0.92), 14.497 (1.68), 16.537 (4.37), 17.270 (0.57).

### Intermediate 5-13

### N-(3-chloro-2-methoxyphenyl)-5-(2-fluoroethyl)-4-hydroxy-2-oxo-1,2,5,6-tetrahydropyridine-3-carbothioamide

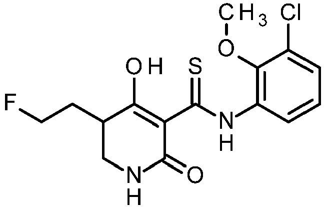

According to the method described for intermediate 5-1 using *tert-*butyl 5-[(3-chloro-2-methoxyphenyl)carbamothioyl]-3-(2-fluoroethyl)-4-hydroxy-6-oxo-3,6-dihydropyridine-1(2*H*)-carboxylate (intermediate 4-13, 1.78 g, 3.88 mmol) as starting material; 910 mg (65% purity, 42% yield) of the title compound were prepared.

¹H-NMR (400 MHz, DMSO-d₆) δ [ppm]: -0.008 (0.52), 0.008 (0.58), 0.866 (0.36), 0.873 (0.36), 0.887 (0.24), 0.904 (0.37), 0.923 (0.20), 1.096 (0.17), 1.174 (0.37), 1.181 (0.35), 1.234 (0.26), 1.355 (0.19), 1.703 (0.21), 1.721 (0.26), 1.736 (0.30), 1.754 (0.30), 1.783 (0.27), 1.797 (0.29), 1.816 (0.26), 2.068 (0.63), 2.087 (0.37), 2.103 (0.24), 2.118 (0.43), 2.132 (0.45), 2.154 (0.36), 2.169 (0.40), 2.185 (0.37), 2.202 (0.32), 2.223 (0.27), 2.238 (0.25), 2.521 (2.97), 2.525 (2.01), 2.681 (0.24), 2.737 (0.39), 2.760 (0.52), 2.776 (0.46), 2.793 (0.31), 2.880 (0.29), 3.154 (0.22), 3.173 (0.24), 3.184 (0.26), 3.234 (0.42), 3.265 (0.70), 3.294 (0.51), 3.307 (0.30), 3.363 (0.52), 3.369 (0.50), 3.460 (0.27), 3.493 (0.22), 3.529 (0.40), 3.545 (0.53), 3.555 (0.47), 3.562 (0.42), 3.572 (0.41), 3.586 (0.30), 3.696 (0.26), 3.711 (2.26), 3.741 (16.00), 3.751 (5.38), 3.759 (0.94), 3.801 (0.27), 3.809 (0.43), 3.827 (0.17), 4.493 (0.39), 4.510 (0.52), 4.524 (0.80), 4.540 (0.84), 4.554 (0.56), 4.562 (0.40), 4.578 (0.16), 4.588 (0.16), 4.611 (0.39), 4.628 (0.53), 4.643 (0.76), 4.658 (0.82), 4.667 (0.55), 4.673 (0.55), 4.680 (0.40), 4.696 (0.16), 5.760 (6.27), 7.149 (0.22), 7.158 (1.01), 7.170 (0.51), 7.179 (2.17), 7.190 (0.65), 7.199 (1.35), 7.212 (0.76), 7.232 (0.42), 7.391 (1.34), 7.408 (1.14), 7.429 (0.61), 7.449 (0.48), 7.724 (0.16), 7.764 (0.98), 7.783 (1.06), 7.829 (0.51), 7.849 (0.46), 8.230 (0.47), 9.457 (0.87), 14.247 (1.84), 14.393 (0.31), 14.686 (0.78), 16.458 (3.88), 16.523 (0.35), 16.655 (1.22), 16.770 (0.20).

### Intermediate 5-30

### N-(3-chloro-2-methoxy-phenyl)-4-hydroxy-3-methyl-6-oxo-2,3-dihydro-1H-pyridine-5-carbothioamide

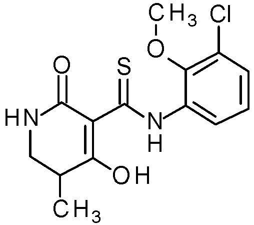

According to the method described for intermediate 5-1 using *tert-*butyl 5-[(3-chloro-2-methoxy-phenyl)carbamothioyl]-4-hydroxy-3-methyl-6-oxo-2,3-dihydropyridine-1-carboxylate (Intermediate 4-30, 1.54 g, 3.61 mmol) as starting material, 993 mg (90% purity, 76% yield) of the title compound were prepared.

LC-MS (method 2): Rₜ = 0.56 min; MS (ESlpos): m/z = 327 [M+H]⁺

¹H-NMR (400 MHz, DMSO-d6) δ [ppm]: 0.784 (0.91), 0.790 (0.68), 0.797 (2.32), 0.802 (3.11), 0.810 (1.85), 0.819 (0.67), 0.836 (0.42), 0.845 (0.80), 0.852 (1.99), 0.860 (3.10), 0.865 (2.26), 0.873 (0.68), 0.879 (0.90), 1.154 (2.56), 1.171 (5.42), 1.189 (2.70), 1.235 (0.64), 1.965 (0.44), 1.986 (8.74), 2.045 (0.70), 2.095 (16.00), 2.135 (0.60), 2.342 (0.41), 2.518 (1.55), 2.522 (1.01), 2.801 (7.66), 2.810 (0.61), 3.998 (0.65), 4.016 (1.91), 4.034 (1.93), 4.052 (0.64), 5.331 (6.51), 7.010 (1.56), 7.030 (1.63), 7.297 (0.83), 7.299 (0.82), 7.309 (0.88), 7.312 (0.93), 7.316 (0.98), 7.318 (0.91), 7.328 (0.93), 7.330 (0.88), 7.432 (6.84), 7.758 (0.92), 7.762 (0.94), 7.777 (1.58), 7.781 (1.55), 7.796 (0.79), 7.801 (0.80), 8.524 (1.08), 8.526 (1.20), 8.528 (1.31), 8.530 (1.15), 8.536 (1.24), 8.539 (1.32), 8.541 (1.30), 8.543 (1.11).

### Intermediate 5-38

### N-(3-chloro-2-methoxy-phenyl)-4-hydroxy-6-oxo-2-(trifluoromethyl)-2,3-dihydro-1H-pyridine-5-carbothioamide

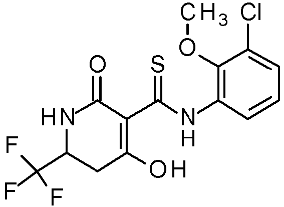

According to the method described for intermediate 5-3 using 1-chloro-3-isothiocyanato-2-methoxybenzene (1.32 g, 6.63 mmol, CAS 127142-64-7) and 6-(trifluoromethyl)piperidine-2,4-dione (1.20 g, 6.63 mmol, CAS 1552231-27-2) as starting materials, 1.70 g (67% yield) of the title compound were prepared after purification by flash chromatography (silica, Hexane / EtOAc gradient 20-80%).

LC-MS (method 2): Rₜ = 0.58 min; MS (ESlpos): m/z = 381 [M+H]⁺

¹H-NMR (400 MHz, DMSO-d₆) δ [ppm]: 2.08 (s, 1 H) 2.55 - 2.84 (m, 1 H) 3.38 - 3.67 (m, 1 H) 3.75 (s, 3 H) 4.34 - 4.67 (m, 1 H) 7.11 - 7.31 (m, 1 H) 7.45 (br d, 1 H) 7.86 (br d, 1 H) 13.85 - 14.32 (m, 1 H) 16.58 - 17.15 (m, 1 H).

### Intermediate 5-49

### (4aS,7aR)-N-(3-chloro-2-methoxyphenyl)-4-hydroxy-2-oxo-2,4a,5,6,7,7a-hexahydro-1H-cyclopenta[b]pyridine-3-carbothioamide

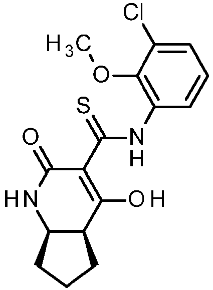

According to the method described for intermediate 5-1 using tert-butyl (4aS,7aR)-3-[(3-chloro-2-methoxyphenyl)carbamothioyl]-4-hydroxy-2-oxo-2,4a,5,6,7,7a-hexahydro-1*H-*cyclopenta[b]pyridine-1-carboxylate (Intermediate 4-49, 910 mg, 2.01 mmol) as starting material, 700 mg (85% purity, 84% yield) of the title compound were prepared after purification by flash chromatography (silica, hexane / EtOAc gradient 50-100%).

LC-MS (method 2): Rₜ = 0.71 min; MS (ESlpos): m/z = 353 [M+H]⁺

¹H-NMR (400 MHz, DMSO-d6) δ [ppm]: 1.54 - 1.94 (m, 5H), 2.02 - 2.31 (m, 1H), 2.73 - 2.98 (m, 1H), 3.71 - 3.77 (d, 3H), 3.95 - 4.05 (m, 1H), 7.14 - 7.24 (m, 1H), 7.36 - 7.46 (dd, 1H), 7.73 - 7.88 (dd, 1H), 7.93 - 9.26 (d, 1H), 14.40 - 14.98 (d, 1H), 16.51 (br d, 1H).

### Intermediate 5-55

### (4aR*,7aS*)-N-(3-chloro-2-methoxyphenyl)-1-(2,4-dimethoxybenzyl)-4-hydroxy-2-oxo-2,4a,5,6,7,7a-hexahydro-1H-cyclopenta[b]pyridine-3-carbothioamide

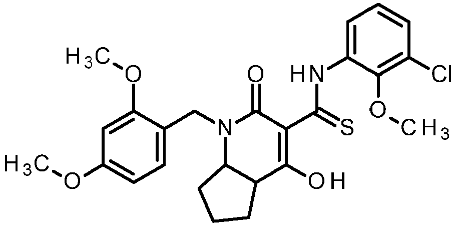

To a solution of *cis*-1-(2,4-dimethoxybenzyl)tetrahydro-1*H*-cyclopenta[b]pyridine-2,4(3*H*,4a*H*)-dione (intermediate 4-55, 5.00 g, 16.1 mmol, 1.00 eq) in ACN (100 ml) was added DBU (4.04 g, 26.5 mmol), the mixture was stirred at 0°C for 0.5 h, then 1-chloro-3-isothiocyanato-2-methoxybenzene (3.62 g, 18.1 mmol, CAS 127142-64-7) was added, the mixture was stirred at 25°C for 2 hrs. The reaction mixture was poured into ice water (200 ml) and extracted with ethyl acetate. The combined organic layers were washed with brine, dried over Na₂SO₄, filtered and concentrated under reduced pressure to give a residue. The residue was purified by flash chromatography (silica, petroleum ether / ethyl acetate 0 - 100%) followed by reversed-phase HPLC (0.1% TFA in water / ACN) to give the title compound (6.00 g, 11.9 mmol, 74.3% yield) as yellow gum.

LC-MS (method 3): Rₜ = 1.135 min; MS (ESlpos): m/z = 503.1 [M+H]⁺

¹H-NMR (400 MHz, CDCl₃) δ [ppm]: 7.88-7.86 (m, 1H), 7.30-7.28 (m, 1H), 7.20 (d, 1H), 7.09 (t, 1H), 6.49-6.45 (m, 2H), 5.00 (d, 1H), 4.38 (d, 1H), 3.88 (s, 3H), 3.86-3.85 (m, 1H), 3.83-3.81 (m, 1H), 3.77 (s, 3H), 3.75 (s, 3H), 3.74-3.73 (m, 1H), 3.10-3.07 (m, 1H), 2.32-2.03 (m, 1H), 1.98-1.97 (m, 1H), 1.74-1.71 (m, 1H), 1.69-1.65 (m, 2H).

### Intermediate 5-58

### N-(3-chloro-2-methyl-phenyl)-4-hydroxy-3-methyl-6-oxo-2,3-dihydro-1H-pyridine-5-carbothioamide

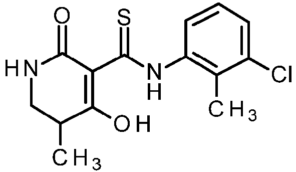

According to the method described for intermediate 5-1 using *tert-*butyl 5-[(3-chloro-2-methyl-phenyl)carbamothioyl]-4-hydroxy-3-methyl-6-oxo-2,3-dihydropyridine-1-carboxylate (intermediate 4-58, 2.00 g, 4.87 mmol) as starting material, 1.60 g (94% purity, 99% yield) of the title compound were prepared.

LC-MS (method 2): Rₜ = 0.56 min; MS (ESlpos): m/z = 311 [M+H]⁺

¹H-NMR (400 MHz, DMSO-d₆) δ [ppm]: -0.008 (0.53), 0.008 (0.56), 1.084 (6.64), 1.101 (6.76), 1.203 (4.07), 1.220 (4.11), 2.175 (16.00), 2.257 (0.73), 2.330 (0.46), 2.334 (0.41), 2.521 (1.94), 2.526 (1.13), 2.672 (0.47), 2.681 (0.52), 2.698 (0.64), 2.710 (0.57), 2.715 (0.53), 2.726 (0.70), 2.742 (0.44), 2.870 (0.51), 2.884 (0.51), 3.030 (0.44), 3.104 (0.55), 3.109 (0.55), 3.136 (1.08), 3.164 (0.55), 3.168 (0.54), 3.391 (0.41), 3.399 (0.41), 3.406 (0.41), 3.463 (0.54), 3.472 (0.61), 3.478 (0.64), 3.488 (0.63), 3.494 (0.58), 3.505 (0.53), 3.510 (0.49), 3.520 (0.43), 5.761 (0.71), 7.206 (1.10), 7.223 (1.93), 7.238 (0.72), 7.250 (1.55), 7.255 (1.43), 7.269 (2.12), 7.279 (1.00), 7.289 (0.93), 7.298 (1.35), 7.318 (0.54), 7.406 (1.56), 7.409 (1.56), 7.425 (1.30), 7.429 (1.27), 7.434 (1.11), 7.454 (0.81), 8.169 (0.79), 9.360 (0.96), 14.033 (2.11), 14.361 (1.21), 16.422 (6.50), 16.589 (3.84).

### Intermediate 5-63

### (6R)-N-[2-(2,2-difluoroethyl)-3-fluorophenyl]-4-hydroxy-6-methyl-2-oxo-1,2,5,6-tetrahydropyridine-3-carbothioamide

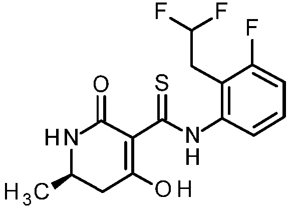

*Tert-*butyl (2*R*)-5-{[2-(2,2-difluoroethyl)-3-fluorophenyl]carbamothioyl}-4-hydroxy-2-methyl-6-oxo-3,6-dihydropyridine-1(2*H*)-carboxylate (1.29 g, 2.90 mmol) was dissolved in 32 ml DCM and 15 ml MeOH and HCI in dioxane (15 ml, 4.0 M, 58 mmol) was added. The was mixture stirred for 1 h at room temperature. To the reaction mixture was added halfconcentrated sodium bicarbonat solution. The mixture was extracted with ethylacetate, the organic layer was dried and concentrated under reduced pressure. The residue was purified by flash chromatography (silica, hexane / EtOAc gradient 35-80%) to give 752 mg (75% yield) of the title compound.

LC-MS (method 2): Rₜ = 0.55 min; MS (ESlpos): m/z = 345 [M+H]⁺

¹H-NMR (400 MHz, DMSO-d₆) δ [ppm]: 1.16 - 1.25 (m, 3 H), 2.53 - 2.79 (m, 2 H), 3.11 (br t, 2 H), 3.61 - 3.85 (m, 1 H), 6.03 - 6.37 (m, 1 H), 7.15 - 7.30 (m, 2 H), 7.35 - 7.52 (m, 1 H), 8.07 - 9.42 (m, 1 H), 13.84 - 14.61 (m, 1 H), 16.36 (d, 1 H).

### Intermediate 5-91

### (6R)-N-(3-chloro-2-ethylphenyl)-4-hydroxy-6-methyl-2-oxo-1,2,5,6-tetrahydropyridine-3-carbothioamide

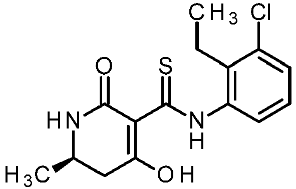

According to the method described for intermediate 5-1 using *tert-*butyl (2*R*)-5-[(3-chloro-2-ethylphenyl)carbamothioyl]-4-hydroxy-2-methyl-6-oxo-3,6-dihydropyridine-1(2*H*)-carboxylate (intermediate 4-91, 4.15 g, 9.77 mmol) as starting material, 2.80 g (90% purity, 79% yield) of the title compound were prepared after purification by flash chromatography (silica, hexane / EtOAc gradient 60 - 100%).

LC-MS (method 2): Rₜ = 0.74 min; MS (ESlpos): m/z = 323.2 [M+H]⁺

¹H-NMR (400 MHz, DMSO-d₆) δ [ppm]: 1.04 - 1.12 (t, 3H), 1.12 - 1.28 (dd, 3H), 2.53 - 2.78 (m, 4H), 3.58 - 3.87 (m, 1H), 7.25 - 7.36 (m, 2H), 7.36 - 7.48 (m, 1H), 8.11 - 9.47 (d, 1H), 14.02 - 14.58 (d, 1H), 16.44 (d, 1H).

### Intermediate 5-98

### N-(3-chloro-2-methoxyphenyl)-4-hydroxy-6-oxo-3-(3,3,3-trifluoropropyl)-2,3-dihydro-1H-pyridine-5-carbothioamide

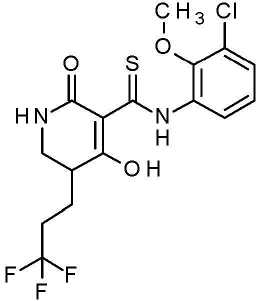

According to the method described for intermediate 5-63 using *tert-*butyl 5-[(3-chloro-2-methoxy-phenyl)carbamothioyl]-4-hydroxy-6-oxo-3-(3,3,3-trifluoropropyl)-2,3-dihydropyridine-1-carboxylate (intermediate 4-98, 665 mg, 1.36 mmol, impure with *tert-*butyl 5-[(3-chloro-2-methoxyphenyl)carbamothioyl]-3-(3,3-difluoroprop-2-en-1-yl)-4-hydroxy-6-oxo-3,6-dihydropyridine-1(2*H*)-carboxylate) as starting material, 520 mg of the title compound (94% yield, impure with *N*-(3-chloro-2-methoxyphenyl)-5-(3,3-difluoroprop-2-en-1-yl)-4-hydroxy-2-oxo-1,2,5,6-tetrahydropyridine-3-carbothioamide) were prepared after purification by flash chromatography (silica, hexane / EtOAc gradient 35-80%).

LC-MS (method 2): Rₜ = 0.69 min; MS (ESlpos): m/z = 409 [M+H]⁺

### Intermediate 5-102

### (6R)-N-(3-chloro-2-methylphenyl)-4-hydroxy-6-methyl-2-oxo-1,2,5,6-tetrahydropyridine-3-carbothioamide

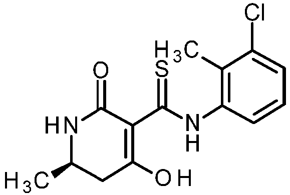

According to the method described for intermediate 5-1 using *tert-*butyl (2*R*)-5-[(3-chloro-2-methylphenyl)carbamothioyl]-4-hydroxy-2-methyl-6-oxo-3,6-dihydropyridine-1(2*H*)-carboxylate (intermediate 4-102, 7.00 g, 17.0 mmol) as starting material, 3.80 g (71% yield) of the title compound were prepared after purification by flash chromatography (silica, hexane / EtOAc gradient 40 - 100%).

LC-MS (method 2): Rₜ = 0.70 min; MS (ESlpos): m/z = 311.3 [M+H]⁺

¹H-NMR (400 MHz, DMSO-d₆) δ [ppm]: 1.20 (dd, 3H), 2.53 - 2.78 (m, 2H), 3.57 - 3.84 (m, 4H), 7.19 (dt, 1H), 7.41 (ddd, 1H), 7.83 (dd, 1H), 8.14 - 8.27 (br s, 1H), 9.29 - 9.45 (br s, 1H), 14.25 - 14.35 (br s, 0,5H), 14.69 - 14.81 (br s, 0,5H), 16.46 (s, 1H).

### Intermediate 5-111

### N-(3-chloro-2-methoxy-phenyl)-7-hydroxy-5-oxo-4-azaspiro[2.5]oct-6-ene-6-carbothioamide

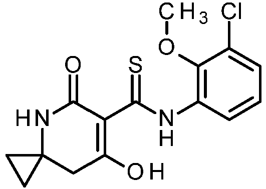

To a solution of 4-azaspiro[2.5]octane-5,7-dione (3.0 g, 21.56 mmol, CAS 1105663-34-0) in ACN (50 ml) was added DBU (4.80 g, 31.51 mmol) and 1-chloro-3-isothiocyanato-2-methoxybenzene (4.47 g, 22.39 mmol, CAS 127142-64-7) at 0 °C under N₂. The reaction was stirred at 30 °C for 12 h. The reaction mixture was adjusted to pH 3 with HCI (1M) and was extracted with ethyl acetate. The combined organic phase was dried over Na₂SO₄, filtered and concentrated under reduced pressure to give a residue. The crude product was purified by preparative HPLC (Column: Phenomenex luna C18 250*80mm*10 um; Eluent A :water (0.05%HCI); Eluent B: acetonitrile;0 - 20 min: 40% - 70% B) to give 1.9 g of the title compound (26% yield) as a white solid.

¹H-NMR (400 MHz, MeOD) δ [ppm]: 7.97-7.92 (m, 1H), 7.34-7.29 (m, 1H), 7.15-7.10 (m, 1H), 3.82 (s, 3H), 2.79 (s, 1H). 2.66 (s, 1H). 0.79-0.93 (m, 4H).

### Intermediate 5-114

### 3-allyl-N-(3-chloro-2-methoxy-phenyl)-4-hydroxy-6-oxo-2,3-dihydro-1H-pyridine-5-carbothioamide

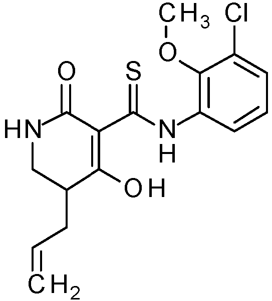

*Tert-*butyl 3-allyl-5-[(3-chloro-2-methoxy-phenyl)carbamothioyl]-4-hydroxy-6-oxo-2,3-dihydropyridine-1-carboxylate (intermediate 4-114, 2.32 g, 5.12 mmol) was dissolved in dichloromethane (24 ml) and methanol (12 ml), HCI in dioxane (13 ml, 4.0 M, 51 mmol) was added and the mixture was stirred for 2h at RT. The pH of the mixture was adjusted to 14 with sodium hydroxide solution and the aqueous phase was extracted with DCM. The organic layer was dried and concentrated under reduced pressure. The residue was purified by flash chromatography (silica, DCM / MeOH gradient 0-10%) to give 1.32 g (90% purity, 66% yield) of the title compound.

LC-MS (Method 2): Rₜ = 0.63 min; MS (ESlpos): m/z = 353 [M+H]⁺

¹H-NMR (400 MHz, DMSO-d6) δ [ppm]: 0.000 (5.34), 2.154 (0.50), 2.190 (0.60), 2.521 (1.27), 2.526 (0.87), 2.538 (0.50), 2.659 (0.48), 2.663 (0.42), 2.672 (0.83), 2.681 (0.43), 2.685 (0.45), 3.154 (0.42), 3.180 (0.60), 3.187 (0.49), 3.203 (0.44), 3.210 (0.43), 3.432 (0.67), 3.441 (0.55), 3.447 (0.59), 3.456 (0.62), 3.462 (0.78), 3.463 (0.67), 3.466 (0.57), 3.474 (1.00), 3.481 (0.44), 3.487 (0.56), 3.497 (0.53), 3.568 (0.81), 3.666 (0.54), 3.671 (0.46), 3.678 (0.60), 3.681 (0.72), 3.702 (0.69), 3.704 (0.61), 3.712 (0.51), 3.716 (0.58), 3.727 (0.50), 3.736 (16.00), 3.752 (7.90), 4.631 (0.41), 5.066 (0.99), 5.091 (1.81), 5.115 (0.67), 5.133 (1.50), 5.137 (1.50), 5.170 (0.57), 5.760 (14.24), 5.773 (0.43), 5.776 (0.52), 5.792 (0.49), 5.801 (0.65), 5.818 (0.77), 5.835 (0.52), 5.840 (0.48), 5.844 (0.43), 7.158 (1.11), 7.178 (2.40), 7.189 (0.69), 7.198 (1.43), 7.210 (1.26), 7.230 (0.69), 7.385 (1.42), 7.389 (1.52), 7.406 (1.28), 7.410 (1.25), 7.426 (0.81), 7.429 (0.84), 7.446 (0.67), 7.449 (0.66), 7.762 (1.14), 7.765 (1.17), 7.782 (1.09), 7.785 (1.06), 7.830 (0.72), 7.833 (0.72), 7.850 (0.68), 7.854 (0.65), 8.169 (0.55), 9.397 (0.90), 14.302 (2.08), 14.707 (1.08), 16.454 (4.97), 16.603 (2.44).

### Intermediate 5-123

### N-(3-chloro-2-methoxyphenyl)-8-hydroxy-6-oxo-5-azaspiro[3.5]non-7-ene-7-carbothioamide

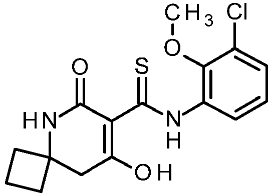

According to the method described for intermediate 5-3 using 1-chloro-3-isothiocyanato-2-methoxybenzene (4.47 g, 22.4 mmol, CAS 127142-64-7) and 5-azaspiro[3.5]nonane-6,8-dione (3.43 g, 22.4 mmol, CAS 1105665-46-0) as starting materials, 6.89 g (95% purity, 83% yield) of the title compound were prepared after precipitation from a mixture of ACN and aq. hydrochloric acid (2 M).

LC-MS (Method 2): Rₜ = 0.60 min; MS (ESlpos): m/z = 353 [M+H]⁺

¹H-NMR (400 MHz, DMSO-d₆) δ [ppm]: 1.715 (0.55), 1.737 (0.71), 1.761 (0.99), 1.782 (0.90), 1.805 (0.51), 2.024 (0.60), 2.033 (0.77), 2.040 (0.71), 2.055 (1.59), 2.065 (1.21), 2.074 (2.07), 2.085 (0.73), 2.133 (0.42), 2.157 (1.04), 2.162 (0.75), 2.181 (0.72), 2.187 (0.74), 2.205 (0.52), 2.210 (0.50), 2.228 (0.91), 2.234 (0.70), 2.252 (0.68), 2.258 (0.72), 2.518 (1.18), 2.523 (0.80), 2.862 (4.15), 3.001 (4.20), 3.740 (15.84), 3.747 (16.00), 7.153 (0.97), 7.173 (2.14), 7.185 (1.09), 7.193 (1.26), 7.206 (2.23), 7.226 (1.23), 7.379 (1.24), 7.383 (1.32), 7.399 (1.08), 7.403 (1.04), 7.421 (1.31), 7.425 (1.41), 7.441 (1.15), 7.445 (1.11), 7.803 (0.97), 7.806 (1.00), 7.824 (0.93), 7.827 (0.88), 7.860 (1.04), 7.863 (1.04), 7.880 (0.98), 7.883 (0.93), 8.670 (1.36), 9.774 (1.11), 14.249 (1.63), 14.703 (1.41), 16.499 (10.89).

### Syntheses of Intermediate 6 Compounds

### Intermediate 6-1

### (6R)-N-(3-chloro-2-methoxyphenyl)-4-({[3-(2-methoxy-2-methylpropoxy)pyridin-4-yl]methyl}amino)-6-methyl-2-oxo-1,2,5,6-tetrahydropyridine-3-carbothioamide

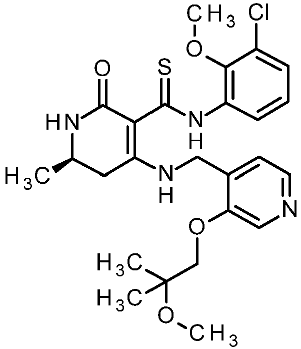

(6*R*)-*N*-(3-chloro-2-methoxyphenyl)-4-hydroxy-6-methyl-2-oxo-1,2,5,6-tetrahydropyridine-3-carbothioamide (intermediate 5-1, 200 mg, 612 µmol) and 1-[3-(2-methoxy-2-methylpropoxy)pyridin-4-yl]methanamine (intermediate 2-1, 154 mg, 734 µmol) were stirred for 3 h at 120°C. The reaction mixture was purified by flash chromatography (silica, DCM / EtOH gradient 0-10 %) to give 226 mg (80% purity, 57% yield) of the title compound.

LC-MS (method 2): Rₜ = 1.24 min; MS (ESlpos): m/z = 519 [M+H]⁺

¹H-NMR (400 MHz, DMSO-d₆) δ [ppm]: 1.10 - 1.15 (d, 3H), 1.20 - 1.30 (s, 6H), 2.40 - 2.47 (m, 1H), 2.88 - 2.98 (dd, 1H), 3.14 - 3.19 (s, 3H), 3.38 - 3.55 (m, 1H), 3.71 (s, 3H), 4.04 (s, 2H), 4.70 (d, 2H), 7.07 - 7.13 (t, 1H), 7.25 - 7.33 (m, 2H), 7.73 (s, 1H), 7.81 (dd, 1H), 8.24 (br d, 1H), 8.41 (s, 1H), 13.74 (t, 1H), 14.87 (s, 1H).

### Intermediate 6-2

### (6S)-N-(3-chloro-2-methoxyphenyl)-4-({[3-(2-methoxy-2-methylpropoxy)pyridin-4-yl]methyl}amino)-6-methyl-2-oxo-1,2,5,6-tetrahydropyridine-3-carbothioamide

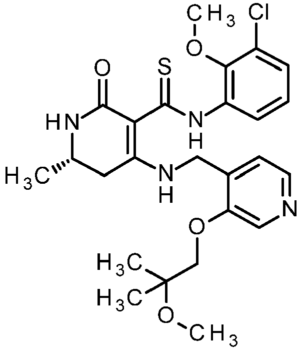

According to the method described for intermediate 6-1 using (6*S*)-N-(3-chloro-2-methoxyphenyl)-4-hydroxy-6-methyl-2-oxo-1,2,5,6-tetrahydropyridine-3-carbothioamide (intermediate 5-10, 200 mg, 612 µmol) and 1-[3-(2-methoxy-2-methylpropoxy)pyridin-4-yl]methanamine (intermediate 2-1, 129 mg, 612 µmol) as starting materials, 168 mg (85% purity, 45% yield) of the title compound were prepared.

LC-MS (method 2): Rₜ = 1.24 min; MS (ESlpos): m/z = 519 [M+H]⁺

¹H-NMR (400 MHz, DMSO-d₆) δ [ppm]: 1.13 (d, 3H), 1.21 - 1.26 (s, 6H), 2.40 - 2.46 (m, 1H), 2.87 - 2.97 (dd, 1H), 3.17 (s, 3H), 3.44 - 3.54 (m, 1H), 3.71 (s, 3H), 4.04 (s, 2H), 4.69 (d, 2H), 7.11 (t, 1H), 7.25 - 7.33 (m, 2H), 7.73 (s, 1H), 7.81 (dd, 1H), 8.24 (br d, 1H), 8.41 (s, 1H), 13.74 (t, 1H), 14.87 (s, 1H).

### Intermediate 6-3

### N-(3-chloro-2-methoxyphenyl)-1-(2,4-dimethoxybenzyl)-6-methyl-2-oxo-4-[({3-[(2S)-tetrahydrofuran-2-ylmethoxy]pyridin-4-yl}methyl)amino]-1,2,5,6-tetrahydropyridine-3-carbothioamide

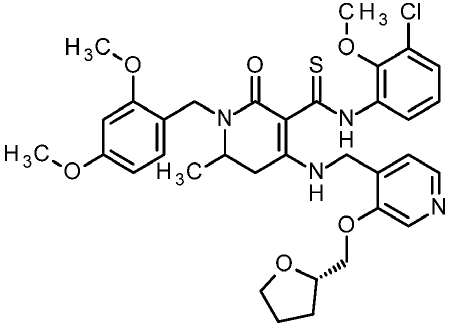

According to the method described for intermediate 6-1 using *N*-(3-chloro-2-methoxyphenyl)-1-[(2,4-dimethoxyphenyl)methyl]-4-hydroxy-6-methyl-2-oxo-1,2,5,6-tetrahydropyridine-3-carbothioamide (intermediate 5-3, 1.15 g, 2.41 mmol) and 1-(3-{[(2S)-oxolan-2-yl]methoxy}pyridin-4-yl)methanamine (intermediate 2-3, 1.00 g, 4.82 mmol) as starting materials, 831 mg (52% yield) of the title compound were prepared.

LC-MS (method 2): Rₜ = 1.47 min; MS (ESlpos): m/z = 667 [M+H]⁺

¹H-NMR (400 MHz, DMSO-d₆) δ [ppm]: 0.000 (4.47), 1.014 (2.99), 1.030 (3.03), 1.119 (1.52), 1.136 (1.56), 1.234 (0.47), 1.711 (0.41), 1.733 (0.62), 1.740 (0.59), 1.749 (0.60), 1.761 (1.00), 1.779 (1.08), 1.798 (0.82), 1.814 (0.61), 1.826 (0.80), 1.845 (0.93), 1.861 (0.86), 1.881 (0.72), 1.893 (0.64), 1.910 (0.46), 1.974 (0.60), 1.986 (0.58), 2.005 (0.60), 2.017 (0.51), 2.764 (0.47), 2.805 (0.74), 2.895 (0.64), 2.910 (0.60), 3.585 (0.64), 3.600 (0.52), 3.631 (0.59), 3.652 (1.11), 3.673 (3.36), 3.679 (2.99), 3.714 (13.99), 3.730 (1.53), 3.744 (16.00), 3.757 (1.75), 3.773 (1.79), 3.793 (12.71), 3.797 (6.60), 3.816 (0.60), 3.935 (0.45), 3.945 (0.47), 3.955 (0.66), 4.014 (0.40), 4.026 (0.71), 4.064 (0.69), 4.077 (0.61), 4.094 (1.02), 4.109 (0.79), 4.120 (1.31), 4.134 (2.73), 4.144 (1.38), 4.154 (1.53), 4.171 (1.63), 4.178 (1.11), 4.191 (0.84), 4.206 (0.84), 4.658 (1.17), 4.772 (1.35), 4.810 (1.21), 4.838 (0.54), 4.876 (0.46), 5.761 (1.69), 6.472 (1.15), 6.477 (1.32), 6.493 (1.15), 6.498 (1.36), 6.566 (3.20), 6.572 (2.71), 7.086 (0.93), 7.096 (1.63), 7.106 (2.91), 7.115 (2.50), 7.126 (1.87), 7.136 (1.34), 7.146 (0.42), 7.289 (1.85), 7.293 (2.09), 7.302 (1.53), 7.309 (2.24), 7.313 (2.05), 7.809 (1.32), 7.812 (1.28), 7.829 (1.26), 8.191 (0.49), 8.233 (0.82), 8.245 (0.82), 8.259 (0.60), 8.395 (1.15), 13.775 (0.73), 14.637 (2.00).

### Intermediate 6-6

### (6R)-N-(3-chloro-2-methoxyphenyl)-6-methyl-4-{[(3-{[(2S)-oxetan-2-yl]methoxy}pyridin-4-yl)methyl]amino}-2-oxo-1,2,5,6-tetrahydropyridine-3-carbothioamide

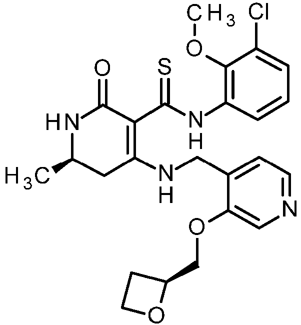

According to the method described for intermediate 6-1 using (6*R*)-*N*-(3-chloro-2-methoxyphenyl)-4-hydroxy-6-methyl-2-oxo-1,2,5,6-tetrahydropyridine-3-carbothioamide (intermediate 5-1, 200 mg, 612 µmol) and 1-(3-{[(2*S*)-oxetan-2-yl]methoxy}pyridin-4-yl)methanamine (intermediate 2-6, 143 mg, 734 µmol) as starting materials, 78.0 mg (95 % purity, 24% yield) of the title compound were prepared.

LC-MS (method 2): Rₜ = 1.13 min; MS (ESlpos): m/z = 503 [M+H]⁺

¹H-NMR (400 MHz, DMSO-d₆) δ [ppm]: 1.04 - 1.17 (d, 3H), 2.39 - 2.48 (m, 1H), 2.58 - 2.77 (m, 2H), 2.87 - 3.02 (dd, 1H), 3.39 - 3.54 (m, 1H), 3.68 - 3.74 (s, 3H), 4.26 - 4.41 (m, 2H), 4.48 - 4.58 (m, 2H), 4.66 - 4.79 (t, 2H), 4.99 - 5.10 (m, 1H), 7.06 - 7.15 (t, 1H), 7.19 - 7.36 (m, 2H), 7.69 - 7.77 (s, 1H), 7.77 - 7.86 (dd, 1H), 8.22 - 8.30 (d, 1H), 8.40 - 8.47 (s, 1H), 13.74 - 13.86 (t, 1H), 14.81 - 14.93 (s, 1H).

### Intermediate 6-7

### (6S)-N-(3-chloro-2-methoxyphenyl)-6-methyl-4-{[(3-{[(2S)-oxetan-2-yl]methoxy}pyridin-4-yl)methyl]amino}-2-oxo-1,2,5,6-tetrahydropyridine-3-carbothioamide

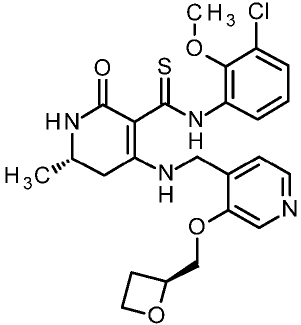

According to the method described for intermediate 6-1 using (6*S*)-*N*-(3-chloro-2-methoxyphenyl)-4-hydroxy-6-methyl-2-oxo-1,2,5,6-tetrahydropyridine-3-carbothioamide (intermediate 5-2, 200 mg, 612 µmol) and 1-(3-{[(2*S*)-oxetan-2-yl]methoxy}pyridin-4-yl)methanamine (intermediate 2-15, 119 mg, 612 µmol) as starting materials, 149 mg (85 % purity, 41% yield) of the title compound were prepared.

LC-MS (method 2): Rₜ = 1.12 min; MS (ESlpos): m/z = 503 [M+H]⁺

¹H-NMR (40 0MHz, DMSO-d₆) δ [ppm]: 1.11 - 1.15 (d, 3H), 2.37 - 2.48 (m, 1H), 2.56 - 2.78 (m, 3H), 2.90 - 2.98 (dd, 1H), 3.44 (m, 2H), 3.71 (s, 3H), 4.25 - 4.39 (m, 3H), 4.53 (ddd, 2H), 4.72 (d, 2H), 5.01 - 5.10 (m, 1H), 7.11 (t, 1H), 7.27 - 7.34 (m, 2H), 7.73 (s, 1H), 7.82 (dd, 1H), 8.23 - 8.28 (d, 1H), 8.44 (s, 1H), 13.71 - 13.84 (t, 1H), 14.88 (s, 1H).

### Intermediate 6-8

### 1-[(2,4-dimethoxyphenyl)methyl]-N-(3-fluoro-2-methoxyphenyl)-6-methyl-2-oxo-4-{[(3-{[(2S)-oxolan-2-yl]methoxy}pyridin-4-yl)methyl]amino}-1,2,5,6-tetrahydropyridine-3-carbothioamide

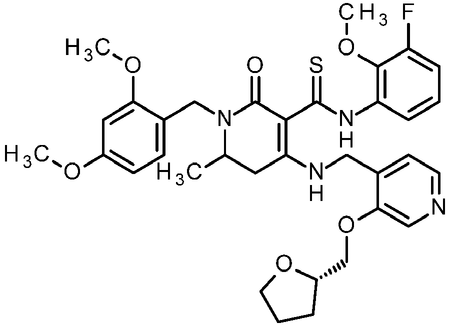

According to the method described for intermediate 6-1 using 1-[(2,4-dimethoxyphenyl)methyl]-*N*-(3-fluoro-2-methoxyphenyl)-4-hydroxy-6-methyl-2-oxo-1,2,5,6-tetrahydropyridine-3-carbothioamide (intermerdiate 5-8, 600 mg, 1.30 mmol) and 1-(3-{[(2*S*)-oxolan-2-yl]methoxy}pyridin-4-yl)methanamine (intermediate 2-3, 543 mg, 2.61 mmol) as starting materials, 545 mg (64% yield) of the title compound were prepared.

LC-MS (method 2): Rₜ = 1.41 min; MS (ESlpos): m/z = 651 [M+H]⁺

¹H-NMR (400 MHz, DMSO-d₆) δ [ppm]: 1.02 (d, 3 H), 1.67 - 1.93 (m, 3 H), 1.94 - 2.05 (m, 1 H), 2.72 - 2.83 (m, 1 H), 2.84 - 2.96 (m, 1 H), 3.57 (dt, 1 H), 3.62 - 3.70 (m, 1 H), 3.72 - 3.86 (m, 10 H), 4.06 - 4.26 (m, 4 H), 4.66 (br d, 2 H), 4.79 (d, 1 H), 6.48 (dd, 1 H), 6.57 (d, 1 H), 7.02 - 7.15 (m, 3 H), 7.32 (br s, 1 H), 7.63 - 7.71 (m, 1 H), 8.24 (br s, 1 H), 8.40 (br s, 1 H), 13.78 (br t, 1 H), 14.59 (s, 1 H)

### Intermediate 6-11

### tert-butyl (2S)-2-[({4-[({(2R)-5-[(3-chloro-2-methoxyphenyl)carbamothioyl]-2-methyl-6-oxo-1,2,3,6-tetrahydropyridin-4-yl}amino)methyl]pyridin-3-yl}oxy)methyl]morpholine-4-carboxylate

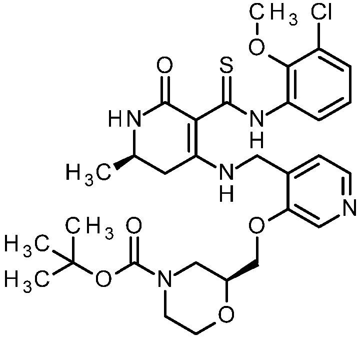

According to the method described for intermediate 6-1 using (6*R*)-*N*-(3-chloro-2-methoxyphenyl)-4-hydroxy-6-methyl-2-oxo-1,2,5,6-tetrahydropyridine-3-carbothioamide (intermediate 5-1, 231 mg, 706 µmol) and *tert-*butyl (2*S*)-2-({[4-(aminomethyl)pyridin-3-yl]oxy}methyl)morpholine-4-carboxylate (intermediate 2-11, 274 mg, 847 µmol) as starting materials, 192 mg (75% purity, 32% yield) of the title compound were prepared.

LC-MS (method 2): Rₜ = 1.31 min; MS (ESlpos): m/z = 632 [M+H]⁺

¹H-NMR (400 MHz, DMSO-d₆) δ [ppm]: 1.035 (5.26), 1.053 (10.50), 1.070 (5.14), 1.103 (0.49), 1.120 (3.35), 1.136 (3.07), 1.232 (0.40), 1.391 (16.00), 1.401 (12.08), 1.429 (1.83), 2.441 (0.44), 2.468 (0.55), 2.518 (1.88), 2.523 (1.26), 2.908 (0.58), 2.917 (0.59), 2.948 (0.42), 3.405 (1.04), 3.413 (0.50), 3.418 (1.02), 3.422 (2.70), 3.435 (3.25), 3.440 (3.00), 3.452 (2.84), 3.457 (1.18), 3.463 (0.67), 3.469 (1.20), 3.667 (1.42), 3.709 (13.77), 3.727 (0.75), 3.734 (0.89), 3.739 (0.84), 3.746 (0.77), 3.753 (0.62), 3.760 (0.52), 3.785 (2.22), 3.839 (0.56), 3.870 (0.52), 4.221 (1.89), 4.233 (1.74), 4.343 (1.63), 4.355 (3.18), 4.368 (1.57), 4.669 (1.10), 4.684 (1.11), 7.081 (0.91), 7.101 (2.08), 7.121 (1.23), 7.282 (1.33), 7.285 (1.34), 7.302 (1.60), 7.305 (1.67), 7.316 (0.80), 7.731 (1.10), 7.812 (0.91), 7.815 (0.91), 7.832 (0.87), 7.836 (0.82), 8.242 (1.14), 8.254 (1.13), 8.407 (1.75), 13.767 (0.59), 14.877 (1.63).

### Intermediate 6-13

### N-(3-chloro-2-methoxyphenyl)-5-(2-hydroxyethyl)-4-({[3-(2-methoxy-2-methylpropoxy)pyridin-4-yl]methyl}amino)-2-oxo-1,2,5,6-tetrahydropyridine-3-carbothioamide

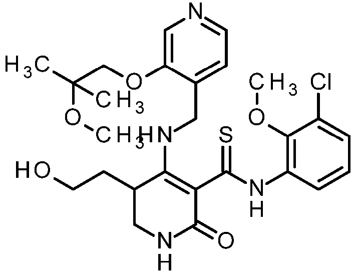

According to the method described for intermediate 6-1 using *N*-(3-chloro-2-methoxyphenyl)-5-(2-fluoroethyl)-4-hydroxy-2-oxo-1,2,5,6-tetrahydropyridine-3-carbothioamide (intermediate 5-13, 100 mg, 279 µmol) and 1-[3-(2-methoxy-2-methylpropoxy)pyridin-4-yl]methanamine (intermediate 2-1, 93.8 mg, 446 µmol) as starting materials, 78.9 mg (80% purity, 41% yield) of the title compound were prepared.

LC-MS (method 2): Rₜ = 1.14 min; MS (ESlpos): m/z = 551 [M+H]⁺

### Intermediate 6-16

### N-(3-chloro-2-methoxyphenyl)-5-(2-hydroxyethyl)-2-oxo-4-[({3-[(2S)-tetrahydrofuran-2-ylmethoxy]pyridin-4-yl}methyl)amino]-1,2,5,6-tetrahydropyridine-3-carbothioamide

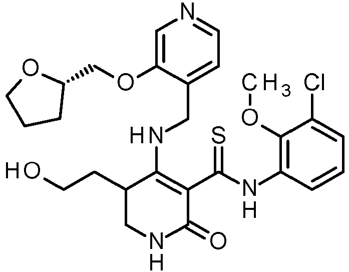

According to the method described for intermediate 6-1 using *N*-(3-chloro-2-methoxyphenyl)-5-(2-fluoroethyl)-4-hydroxy-2-oxo-1,2,5,6-tetrahydropyridine-3-carbothioamide (intermediate 5-13, 205 mg, 571 µmol) and 1-(3-{[(2*S*)-oxolan-2-yl]methoxy}pyridin-4-yl)methanamine (intermediate 2-3, 190 mg, 914 µmol) as starting materials, 141 mg (80% purity, 36% yield) of the title compound were prepared.

LC-MS (method 2): Rₜ = 1.10 min; MS (ESlpos): m/z = 550 [M+H]⁺

### Intermediate 6-19

### N-(3-chloro-2-methoxyphenyl)-5-(2-hydroxyethyl)-4-{[(3-{[2-methyloxetan-2-yl]methoxy}pyridin-4-yl)methyl]amino}-2-oxo-1,2,5,6-tetrahydropyridine-3-carbothioamide

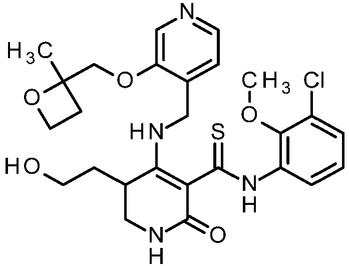

According to the method described for intermediate 6-1 using *N*-(3-chloro-2-methoxyphenyl)-5-(2-fluoroethyl)-4-hydroxy-2-oxo-1,2,5,6-tetrahydropyridine-3-carbothioamide (intermediate 5-13, 205 mg, 571 µmol) and 1-{3-[(2-methyloxetan-2-yl)methoxy]pyridin-4-yl}methanamine (intermediate 2-19, 190 mg, 914 µmol) as starting materials, 112.9 mg (80% purity, 29% yield) of the title compound were prepared.

LC-MS (method 2): Rₜ = 1.08 min; MS (ESlpos): m/z = 549 [M+H]⁺

### Intermediate 6-24

### (2R)-N-(3-chloro-2-methoxy-phenyl)-4-{[3-(1,4-dioxan-2-ylmethoxy)-4-pyridyl]methylamino}-2-methyl-6-oxo-2,3-dihydro-1H-pyridine-5-carbothioamide

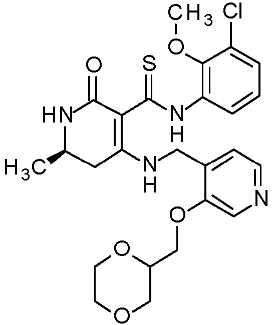

According to the method described for intermediate 6-1 using (6*R*)-*N*-(3-chloro-2-methoxyphenyl)-4-hydroxy-6-methyl-2-oxo-1,2,5,6-tetrahydropyridine-3-carbothioamide (intermediate 5-1, 200 mg, 612 µmol) and 1-{3-[(1,4-dioxan-2-yl)methoxy]pyridin-4-yl}methanamine (intermediate 2-24, 343 mg, 1.53 mmol) as starting materials, 220.0 mg (75% purity, 51% yield) of the title compound were prepared after purification by flash chromatography (silica, DCM / EtOH gradient 0-10%).

LC-MS (method 2): Rₜ = 1.13 min; MS (ESlpos): m/z = 533 [M+H]⁺

¹H-NMR (400 MHz, DMSO-d₆) δ [ppm]: 1.13 (d, 3H), 2.40 - 2.45 (m, 1H), 2.90 - 2.99 (m, 1H), 3.13 - 3.31 (m, 2H), 3.35 - 3.69 (m, 16H), 3.71 - 3.73 (m, 3H), 3.73 - 3.80 (m, 2H), 3.83 - 3.95 (m, 3H), 4.11 - 4.21 (m, 2H), 4.64 - 4.72 (m, 3H), 7.08 - 7.13 (m, 1H), 7.28 - 7.33 (m, 2H), 7.73 (s, 1H), 7.80 - 7.85 (m, 1H), 8.24 (s, 1H), 8.39 (s, 1H), 13.70 - 13.79 (m, 1H), 14.88 (s, 1H).

### Intermediate 6-27

### (2R)-N-(3-chloro-2-methoxy-phenyl)-2-methyl-6-oxo-4-{[3-(tetrahydropyran-2-ylmethoxy)-4-pyridyl]methylamino}-2,3-dihydro-1H-pyridine-5-carbothioamide

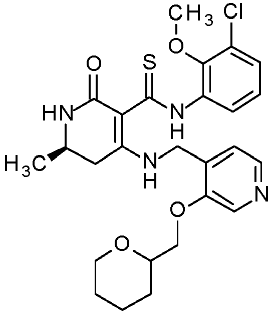

According to the method described for intermediate 6-1 using (6*R*)-N-(3-chloro-2-methoxyphenyl)-4-hydroxy-6-methyl-2-oxo-1,2,5,6-tetrahydropyridine-3-carbothioamide (intermediate 5-1, 200 mg, 612 µmol) and 1-(3-{[oxan-2-yl]methoxy}pyridin-4-yl)methanamine (intermediate 2-27, 204 mg, 918 µmol) as starting materials, 240 mg (74% yield) of the title compound were prepared after purification by flash chromatography (silica, DCM / EtOH gradient 0-10%).

LC-MS (method 2): Rₜ = 1.27 min; MS (ESlpos): m/z = 531 [M+H]⁺

¹H-NMR (400 MHz, DMSO-d₆) δ [ppm]: 1.13 (d, 3H), 1.30 - 1.40 (m, 1H), 1.45 - 1.54 (m, 4H), 1.63 - 1.71 (d, 1H), 1.76 - 1.86 (m, 1H), 2.37 - 2.47 (m, 1H), 2.90 - 2.97 (dd, 1H), 3.36 - 3.53 (m, 3H), 3.63 - 3.70 (m, 1H), 3.70 - 3.75 (s, 3H), 3.86 - 3.92 (m, 1H), 4.06 - 4.14 (m, 2H), 4.66 (d, 2H), 7.06 - 7.14 (t, 1H), 7.25 - 7.34 (m, 2H), 7.69 - 7.75 (s, 1H), 7.80 - 7.86 (dd, 1H), 8.20 - 8.29 (d, 1H), 8.39 (s, 1H), 13.70 - 13.78 (t, 1H), 14.88 (s, 1H).

### Intermediate 6-30

### N-(3-chloro-2-methoxy-phenyl)-3-methyl-6-oxo-4-[(3-{[(2S)-tetrahydrofuran-2-yl]methoxy}-4-pyridyl)methylamino]-2,3-dihydro-1H-pyridine-5-carbothioamide

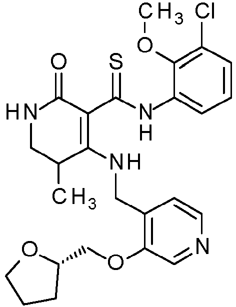

A mixture of *N*-(3-chloro-2-methoxy-phenyl)-4-hydroxy-3-methyl-6-oxo-2,3-dihydro-1*H-*pyridine-5-carbothioamide (intermediate 5-30, 200 mg, 612 µmol) and 1-(3-{[(2*S*)-oxolan-2-yl]methoxy}pyridin-4-yl)methanamine (intermediate 2-3, 129 mg, 99% purity, 612 µmol) in ACN (6 ml) was treated with *N,O*-bis(trimethylsilyl)trifluoroacetamide (378 µl, 1.530 mmol, CAS 10416-59-8) and stirred at 80°C for 3 h. The reaction was treated with another equivalent of *N,O*-bis(trimethylsilyl)trifluoroacetamide and was stirred at 80°C overnight. The reaction mixture was filtered and purified by preparative HPLC (method 10, gradient: 0.00-0.50 min 30% B, 0.50-6.00 min 30-70% B). The product containig fractions were pooled and freeze dried to give 168.0 mg (99% purity, 53% yield) of the desired product.

LC-MS (method 2): Rₜ = 1.19 min; MS (ESlpos): m/z = 517 [M+H]⁺

¹H-NMR (400 MHz, DMSO-d₆) δ [ppm]: 1.188 (2.04), 1.194 (2.13), 1.205 (2.17), 1.212 (2.09), 1.819 (0.41), 1.839 (0.64), 1.856 (0.69), 1.873 (0.48), 1.978 (0.46), 2.520 (2.11), 2.525 (1.43), 3.311 (0.61), 3.352 (0.46), 3.674 (0.73), 3.692 (0.84), 3.714 (16.00), 3.759 (0.42), 3.778 (0.79), 3.795 (0.67), 4.128 (0.55), 4.133 (0.60), 4.148 (1.52), 4.158 (0.89), 4.167 (0.51), 4.176 (0.88), 4.183 (0.45), 4.199 (0.88), 4.209 (0.45), 4.674 (1.03), 4.681 (0.99), 4.688 (1.03), 4.696 (0.88), 7.085 (1.02), 7.105 (2.23), 7.125 (1.27), 7.285 (1.42), 7.289 (1.50), 7.305 (1.22), 7.309 (1.17), 7.360 (1.20), 7.372 (1.22), 7.707 (0.68), 7.720 (0.69), 7.785 (1.07), 7.789 (1.09), 7.806 (1.01), 7.809 (0.97), 8.244 (2.36), 8.256 (2.26), 8.413 (2.73), 13.731 (0.68), 14.815 (1.75).

### Intermediate 6-33

### N-(3-chloro-2-methoxy-phenyl)-4-{[3-(1,4-dioxan-2-ylmethoxy)-4-pyridyl]methylamino}-3-methyl-6-oxo-2,3-dihydro-1H-pyridine-5-carbothioamide

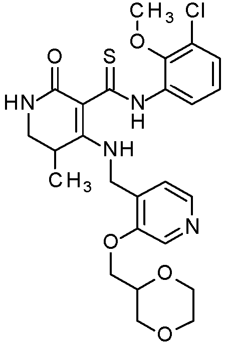

According to the method described for intermediate 6-30 using *N*-(3-chloro-2-methoxyphenyl)-4-hydroxy-3-methyl-6-oxo-2,3-dihydro-1*H*-pyridine-5-carbothioamide (Intermediate 5-30, 200 mg, 612 µmol) and 1-{3-[(1,4-dioxan-2-yl)methoxy]pyridin-4-yl}methanamine (Intermediate 2-24, 137 mg, 612 µmol) as starting materials, 82.2 mg (25% yield) of the title compound were prepared after purification by preparative HPLC (method 10, gradient: 0.00-0.50 min 30% B, 0.50-6.00 min 30-70% B).

LC-MS (method 2): Rₜ = 1.10 min; MS (ESlpos): m/z = 533 [M+H]⁺

¹H-NMR (400 MHz, DMSO-d₆) δ [ppm]: 1.196 (2.61), 1.198 (2.60), 1.213 (2.72), 2.338 (0.62), 2.520 (6.87), 2.525 (4.28), 2.680 (0.61), 2.966 (0.41), 3.307 (0.52), 3.351 (0.73), 3.360 (0.55), 3.397 (0.50), 3.410 (0.45), 3.422 (0.63), 3.425 (0.65), 3.434 (0.57), 3.437 (0.56), 3.449 (0.69), 3.462 (0.88), 3.489 (0.61), 3.518 (0.50), 3.521 (0.52), 3.588 (0.44), 3.593 (0.52), 3.616 (0.67), 3.622 (0.84), 3.651 (1.45), 3.657 (0.59), 3.679 (0.65), 3.714 (16.00), 3.725 (0.70), 3.747 (0.84), 3.757 (1.59), 3.781 (0.65), 3.849 (0.74), 3.878 (0.84), 3.893 (0.65), 3.900 (0.44), 3.906 (0.49), 3.912 (0.41), 3.918 (0.46), 4.155 (0.78), 4.169 (2.09), 4.183 (1.30), 4.680 (1.62), 4.694 (1.60), 7.084 (1.14), 7.104 (2.45), 7.125 (1.44), 7.284 (1.49), 7.288 (1.59), 7.304 (1.32), 7.308 (1.28), 7.364 (1.01), 7.376 (1.05), 7.707 (0.75), 7.720 (0.77), 7.784 (1.18), 7.788 (1.20), 7.805 (1.13), 7.808 (1.08), 8.253 (2.25), 8.265 (2.18), 8.408 (3.14), 13.725 (0.68), 14.816 (1.80).

### Intermediate 6-38

### N-(3-chloro-2-methoxy-phenyl)-6-oxo-4-[(3-{[(2S)-tetrahydrofuran-2-yl]methoxy}-4-pyridyl)methylamino]-2-(trifluoromethyl)-2,3-dihydro-1H-pyridine-5-carbothioamide

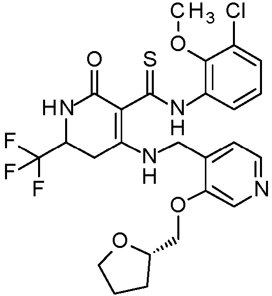

According to the method described for intermediate 6-1 using *N*-(3-chloro-2-methoxyphenyl)-4-hydroxy-6-oxo-2-(trifluoromethyl)-2,3-dihydro-1*H*-pyridine-5-carbothioamide (intermediate 5-38, 270 mg, 709 µmol) and 1-(3-{[(2*S*)-oxolan-2-yl]methoxy}pyridin-4-yl)methanamine (intermediate 2-3, 295 mg, 1.42 mmol) as starting materials, 236 mg (95% purity, 55% yield) of the title compound were prepared after purification by flash chromatography (silica, DCM / EtOH gradient 0-15 %).

LC-MS (method 2): Rₜ = 1.25 min; MS (ESlpos): m/z = 571 [M+H]⁺

¹H-NMR (400 MHz, DMSO-d₆) δ [ppm]: 1.818 (0.41), 1.836 (0.49), 1.855 (0.58), 1.872 (0.54), 2.084 (2.07), 2.518 (0.74), 2.523 (0.51), 2.933 (0.44), 2.973 (0.54), 3.303 (0.49), 3.347 (0.53), 3.675 (0.72), 3.693 (0.87), 3.716 (16.00), 3.770 (0.57), 3.781 (0.56), 3.786 (0.46), 4.120 (0.59), 4.129 (0.63), 4.135 (0.69), 4.143 (1.40), 4.152 (1.20), 4.161 (0.78), 4.178 (0.43), 4.185 (0.45), 4.195 (0.64), 4.206 (0.51), 4.212 (0.54), 4.222 (0.45), 4.697 (1.05), 4.702 (1.03), 4.712 (1.06), 4.717 (1.02), 5.758 (0.42), 7.105 (1.12), 7.125 (2.40), 7.145 (1.37), 7.253 (1.00), 7.264 (1.01), 7.308 (1.47), 7.312 (1.54), 7.328 (1.23), 7.332 (1.19), 7.815 (1.07), 7.818 (1.10), 7.835 (1.04), 7.838 (0.98), 8.231 (2.01), 8.243 (1.95), 8.366 (0.85), 8.378 (0.87), 8.405 (3.26), 13.952 (0.52), 14.486 (1.63).

### Intermediate 6-41

### (2R)-N-(3-chloro-2-methoxy-phenyl)-4-{[3-(2-methoxy-1-methylethoxy)-4-pyridyl]methylamino}-2-methyl-6-oxo-2,3-dihydro-1H-pyridine-5-carbothioamide

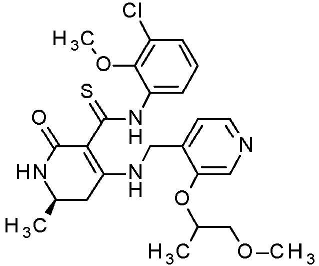

A mixture of (6*R*)-*N*-(3-chloro-2-methoxyphenyl)-4-hydroxy-6-methyl-2-oxo-1,2,5,6-tetrahydropyridine-3-carbothioamide (intermediate 5-1, 200 mg, 612 µmol) and [3-(2-methoxy-1-methyl-ethoxy)-4-pyridyl]methanamine (intermediate 2-41, 120 mg, 612 µmol) in ACN (9.6 ml) was treated with *N,O*-bis(trimethylsilyl)trifluoroacetamide (454 µl, 1.84 mmol, CAS 10416-59-8) and stirred at 80°C for 4 h. The reaction mixture was purified by flash chromatography (amino phase silica, DCM / EtOAc gradient 0-100% and DCM /EtOH gradient 0-10%) to give 210.0 mg (95% purity, 65% yield) of the desired product.

LC-MS (method 2): Rₜ = 1.22 min; MS (ESlpos): m/z = 505 [M+H]⁺

¹H-NMR (400 MHz, DMSO-d₆) δ [ppm]: 14.49 - 15.02 (m, 1H), 13.54 - 13.87 (m, 1H), 8.41 - 8.59 (m, 1H), 8.15 - 8.25 (m, 1H), 7.76 - 7.87 (m, 1H), 7.69 - 7.75 (m, 1H), 7.26 - 7.36 (m, 2H), 7.11 (s, 1H), 4.74 - 4.91 (m, 1H), 4.56 - 4.68 (m, 2H), 3.66 - 3.74 (m, 3H), 3.39 - 3.60 (m, 3H), 3.25 - 3.30 (m, 3H), 2.85 - 2.99 (m, 1H), 2.35 - 2.47 (m, 1H), 1.24 - 1.31 (m, 3H), 1.04 - 1.14 (m, 3H).

### Intermediate 6-44

### N-(3-chloro-2-methoxy-phenyl)-3-methyl-6-oxo-4-{[3-(tetrahydropyran-2-ylmethoxy)-4-pyridyl]methylamino}-2,3-dihydro-1H-pyridine-5-carbothioamide

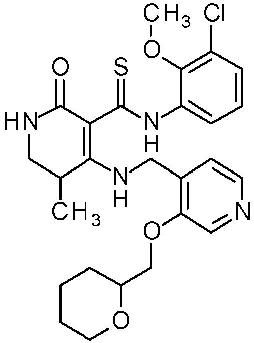

According to the method described for intermediate 6-30 using *N*-(3-chloro-2-methoxyphenyl)-4-hydroxy-3-methyl-6-oxo-2,3-dihydro-1*H*-pyridine-5-carbothioamide (intermediate 5-30, 200 mg, 612 µmol) and 1-(3-{[oxan-2-yl]methoxy}pyridin-4-yl)methanamine (intermediate 2-27, 136 mg, 612 µmol) as starting materials, 73.4 mg (99% purity, 22% yield) of the title compound were prepared after purification by preparative HPLC (method 10, gradient: 0.00-0.50 min 30% B, 0.50-6.00 min 30-70% B).

LC-MS (method 2): Rₜ = 1.23 min; MS (ESlpos): m/z = 531 [M+H]⁺

¹H-NMR (400 MHz, DMSO-d₆) δ [ppm]: 1.196 (3.37), 1.212 (3.32), 1.214 (3.29), 1.337 (0.48), 1.358 (0.41), 1.367 (0.41), 1.479 (2.04), 1.656 (0.68), 1.687 (0.58), 1.794 (0.64), 2.325 (0.67), 2.329 (0.81), 2.334 (0.69), 2.525 (2.16), 2.667 (0.50), 2.671 (0.63), 2.676 (0.49), 2.934 (0.54), 2.947 (0.60), 2.965 (0.65), 2.978 (0.59), 3.088 (0.60), 3.353 (1.26), 3.371 (0.71), 3.379 (0.85), 3.397 (0.45), 3.406 (0.50), 3.652 (0.67), 3.668 (0.64), 3.680 (0.67), 3.696 (0.51), 3.714 (16.00), 3.876 (0.77), 3.904 (0.72), 4.105 (0.94), 4.120 (2.42), 4.132 (1.96), 4.672 (2.17), 4.687 (2.16), 7.082 (1.27), 7.102 (2.71), 7.123 (1.57), 7.282 (1.69), 7.286 (1.82), 7.302 (1.46), 7.306 (1.47), 7.359 (1.76), 7.371 (1.82), 7.701 (1.00), 7.714 (1.03), 7.795 (1.33), 7.798 (1.39), 7.816 (1.29), 7.819 (1.27), 8.239 (2.69), 8.251 (2.56), 8.406 (4.25), 13.705 (0.50), 13.719 (0.96), 13.733 (0.51), 14.817 (1.73).

### Intermediate 6-49

### (4aS,7aR)-N-(3-chloro-2-methoxy-phenyl)-4-{[3-(2-methoxy-1-methyl-ethoxy)-4-pyridyl]methylamino}-2-oxo-1,4a,5,6,7,7a-hexahydrocyclopenta[b]pyridine-3-carbothioamide

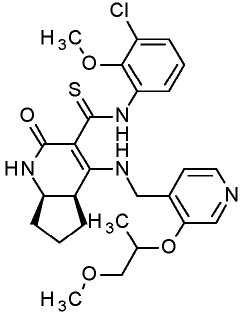

According to the method described for intermediate 6-41 using (4a*S*,7a*R*)-*N*-(3-chloro-2-methoxyphenyl)-4-hydroxy-2-oxo-2,4a,5,6,7,7a-hexahydro-1*H*-cyclopenta[b]pyridine-3-carbothioamide (intermediate 5-49, 200 mg, 567 µmol) and [3-(2-methoxy-1-methyl-ethoxy)-4-pyridyl]methanamine (intermediate 2-41, 171 mg, 65 % purity, 567 µmol) as starting materials, 157.7 mg (95% purity, 50% yield) of the title compound were prepared after purification by flash chromatography (silica, DCM / EtOH gradient 0-10%).

LC-MS (method 2): Rₜ = 1.25 min; MS (ESlpos): m/z = 531 [M+H]⁺

¹H-NMR (400 MHz, DMSO-d₆) δ [ppm]: 1.28 (d, 3H), 1.49 - 1.68 (m, 1H), 1.68 - 1.87 (m, 3H), 1.89 - 1.98 (m, 1H), 1.99 - 2.19 (m, 1H), 3.00 - 3.13 (m, 1H), 3.28 (s, 3H), 3.45 - 3.57 (m, 2H), 3.71 (s, 3H), 3.77 - 3.85 (m, 1H), 4.65 (m, 2H), 4.77 - 4.89 (m, 1H), 7.10 (t, 1H), 7.28 (dd, 1H), 7.34 - 7.38 (dd, 1H), 7.40 - 7.47 (s, 1H), 7.73 - 7.84 (d, 1H), 8.16 - 8.24 (d, 1H), 8.40 - 8.51 (s, 1H), 13.79 - 13.88 (q, 1H), 15.01 (s, 1H).

### Intermediate 6-52

### (4aS,7aR)-N-(3-chloro-2-methoxy-phenyl)-4-{[3-(1,4-dioxan-2-ylmethoxy)-4-pyridyl]methylamino}-2-oxo-1,4a,5,6,7,7a-hexahydrocyclopenta[b]pyridine-3-carbothioamide

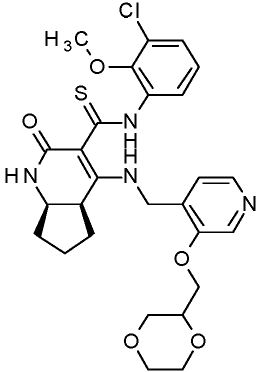

According to the method described for intermediate 6-41 using (4a*S*,7a*R*)-*N*-(3-chloro-2-methoxyphenyl)-4-hydroxy-2-oxo-2,4a,5,6,7,7a-hexahydro-1*H*-cyclopenta[b]pyridine-3-carbothioamide (intermediate 5-49, 200 mg, 567 µmol) and 1-{3-[(1,4-dioxan-2-yl)methoxy]pyridin-4-yl}methanamine (intermediate 2-24, 150 mg, 85% purity, 567 µmol) as starting materials, 105 mg (95% purity, 32% yield) of the title compound were prepared after purification by flash chromatography (silica, DCM / EtOH gradient 0-10%).

LC-MS (method 2): Rₜ = 1.16 min; MS (ESlpos): m/z = 559 [M+H]⁺

¹H-NMR (400 MHz, DMSO-d₆) δ [ppm]: 1.55 - 1.68 (m, 1H), 1.70 - 1.88 (m, 3H), 1.88 - 1.97 (m, 1H), 2.08 - 2.21 (m, 1H), 3.04 - 3.16 (m, 1H), 3.45 (m, 2H), 3.58 - 3.69 (m, 2H), 3.71 (s, 3H), 3.73 - 3.96 (m, 4H), 4.11 - 4.22 (m, 2H), 4.68 (br d, 2H), 7.07 - 7.14 (t, 1H), 7.29 (dd, 1H), 7.36 (d, 1H), 7.45 (s, 1H), 7.78 (dd, 1H), 8.25 (d, 1H), 8.40 (s, 1H), 13.75 - 13.90 (t, 1H), 15.02 (s, 1H).

### Intermediate 6-55

### (4aR*,7aS*)-N-(3-chloro-2-methoxyphenyl)-1-(2,4-dimethoxybenzyl)-2-oxo-4-[({3-[(2S)-tetrahydrofuran-2-ylmethoxy]pyridin-4-yl}methyl)amino]-2,4a,5,6,7,7a-hexahydro-1H-cyclopenta[b]pyridine-3-carbothioamide

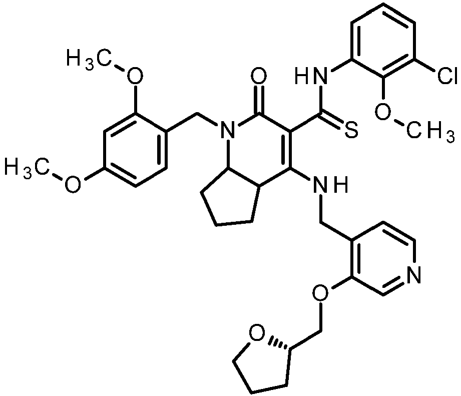

To a solution of 1-(3-{[(2*S*)-oxolan-2-yl]methoxy}pyridin-4-yl)methanamine (intermediate 2-3, 60 mg, 288 umol) in DME (0.5 ml) was added (4a*R**,7a*S**)-*N*-(3-chloro-2-methoxyphenyl)-1-(2,4-dimethoxybenzyl)-4-hydroxy-2-oxo-2,4a,5,6,7,7a-hexahydro-*1H-*cyclopenta[b]pyridine-3-carbothioamide (intermediate 5-55, 100 mg, 198 umol) and *N*,*O-*bis-(trimethylsiliyl)-acetamide (164 mg, 809 umol). The mixture was stirred at 80°C for 3 h. The reaction mixture was concentrated under reduced pressure to give a residue. The residue was purified by column chromatography (silica, petroleum ether / EtOAc 0-100%) to give the title compound (1.00 g, 1.44 mmol, 36% yield) as yellow oil.

LC-MS (method 4): Rₜ = 1.210 min; MS (ESlpos): m/z = 693.3 [M+H]⁺

### Intermediate 6-56

### (4aS,7aR)-N-(3-chloro-2-methoxyphenyl)-2-oxo-4-{[(3-{[(2S)-oxolan-2-yl]methoxy}pyridin-4-yl)methyl]amino}-2,4a,5,6,7,7a-hexahydro-1H-cyclopenta[b]pyridine-3-carbothioamide

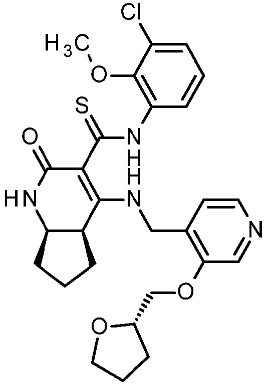

According to the method described for intermediate 6-41 using (4a*S*,7a*R*)-*N*-(3-chloro-2-methoxyphenyl)-4-hydroxy-2-oxo-2,4a,5,6,7,7a-hexahydro-1*H*-cyclopenta[b]pyridine-3-carbothioamide (intermediate 5-49, 100 mg, 283 µmol) and 1-(3-{[(2*S*)-oxolan-2-yl]methoxy}pyridin-4-yl)methanamine (intermediate 2-3, 59.0 mg, 283 µmol) as starting materials, 81.8 mg (95% purity, 50% yield) of the title compound were prepared after purification by flash chromatography (silica, DCM / EtOH gradient 0-10%).

LC-MS (method 2): Rₜ = 1.23 min; MS (ESlpos): m/z = 543 [M+H]⁺

### Intermediate 6-58

### N-(3-chloro-2-methyl-phenyl)-3-methyl-6-oxo-4-[({3-[(2S)-tetrahydrofuran-2-ylmethoxy]pyridin-4-yl}methyl)amino]-2,3-dihydro-1H-pyridine-5-carbothioamide

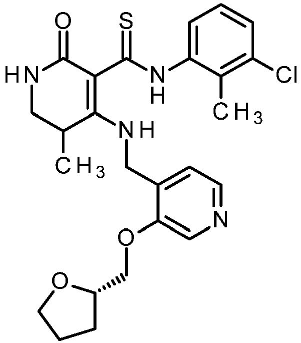

According to the method described for intermediate 6-30 using *N*-(3-chloro-2-methylphenyl)-4-hydroxy-3-methyl-6-oxo-2,3-dihydro-1H-pyridine-5-carbothioamide (intermediate 5-58, 200 mg, 643 µmol) and 1-(3-{[(2*S*)-oxolan-2-yl]methoxy}pyridin-4-yl)methanamine (intermediate 2-3, 134 mg, 643 µmol) as starting materials, 176 mg (99% purity, 54% yield) of the title compound were prepared after purification by preparative HPLC (method 11, 0.00 - 2.00 min 30% B, 2.00 - 14.00 min 30-70% B).

LC-MS (method 2): Rₜ = 1.17 min; MS (ESlpos): m/z = 501 [M+H]⁺

¹H-NMR (400 MHz, DMSO-d₆) δ [ppm]: 1.187 (3.94), 1.193 (4.14), 1.204 (4.16), 1.210 (3.96), 1.720 (0.43), 1.728 (0.62), 1.736 (0.73), 1.744 (0.64), 1.757 (0.63), 1.766 (0.43), 1.774 (0.45), 1.805 (0.45), 1.811 (0.42), 1.820 (0.79), 1.841 (1.24), 1.858 (1.23), 1.869 (0.89), 1.875 (0.89), 1.885 (0.67), 1.890 (0.65), 1.906 (0.46), 1.957 (0.58), 1.970 (0.50), 1.975 (0.72), 1.987 (0.75), 1.995 (0.63), 2.000 (0.55), 2.006 (0.68), 2.018 (0.53), 2.024 (0.40), 2.076 (0.42), 2.152 (16.00), 2.520 (2.58), 2.525 (1.64), 2.929 (0.63), 2.943 (0.72), 2.960 (0.79), 2.975 (0.71), 3.065 (0.68), 3.317 (1.17), 3.348 (1.10), 3.357 (0.84), 3.654 (0.73), 3.674 (1.38), 3.690 (1.65), 3.707 (0.92), 3.759 (0.79), 3.776 (1.66), 3.793 (1.27), 3.811 (0.51), 4.108 (0.67), 4.118 (1.04), 4.124 (1.25), 4.135 (2.06), 4.139 (2.07), 4.145 (1.80), 4.155 (1.02), 4.165 (1.52), 4.170 (0.60), 4.175 (0.44), 4.180 (0.65), 4.190 (1.78), 4.203 (0.91), 4.216 (0.60), 4.655 (2.01), 4.661 (1.88), 4.669 (2.05), 4.675 (1.68), 7.153 (1.20), 7.172 (2.50), 7.196 (1.92), 7.216 (2.91), 7.236 (1.24), 7.325 (2.24), 7.328 (2.21), 7.344 (3.93), 7.356 (2.60), 7.671 (1.29), 7.684 (1.32), 8.233 (4.50), 8.245 (4.32), 8.402 (5.12), 13.657 (0.63), 13.671 (1.16), 13.685 (0.60), 14.560 (2.80).

### Intermediate 6-61

### N-(3-chloro-2-methyl-phenyl)-4-({3-[(3,3-difluorotetrahydropyran-2-yl)methoxy]-4-pyridyl}methylamino)-3-methyl-6-oxo-2,3-dihydro-1H-pyridine-5-carbothioamide

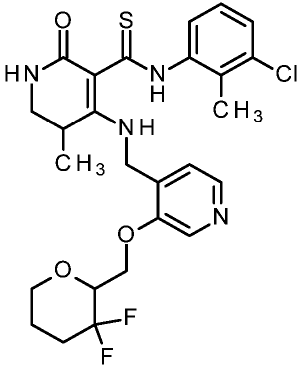

According to the method described for intermediate 6-41 using *N*-(3-chloro-2-methylphenyl)-4-hydroxy-3-methyl-6-oxo-2,3-dihydro-1*H*-pyridine-5-carbothioamide (intermediate 5-58, 200 mg, 643 µmol) and [3-[(3,3-difluorotetrahydropyran-2-yl)methoxy]-4-pyridyl]methanamine (intermediate 2-74, 166 mg, 643 µmol) as starting materials, 48.3 mg (95 % purity, 13 % yield) of the title compound were prepared after purification by preparative HPLC (method 10, gradient: 0.00-0.50 min 30% B, 0.50-6.00 min 30-70% B).

LC-MS (method 2): Rₜ = 1.24 min; MS (ESlpos): m/z = 551 [M+H]⁺

¹H-NMR (400 MHz, DMSO-d₆) δ [ppm]: 1.156 (1.64), 1.165 (3.83), 1.174 (3.03), 1.182 (3.87), 1.195 (3.64), 1.213 (3.60), 1.234 (0.52), 1.764 (1.66), 1.784 (1.08), 1.989 (3.93), 2.154 (16.00), 2.204 (0.55), 2.520 (3.32), 2.524 (2.18), 2.926 (0.63), 2.939 (0.72), 2.957 (0.80), 2.973 (0.71), 3.354 (0.85), 3.365 (0.53), 3.478 (0.54), 3.496 (0.55), 3.507 (0.72), 3.515 (0.64), 3.543 (0.54), 3.905 (0.79), 3.934 (0.70), 3.999 (0.60), 4.007 (0.63), 4.019 (1.14), 4.037 (0.85), 4.059 (0.58), 4.069 (0.59), 4.077 (0.70), 4.085 (0.42), 4.228 (0.67), 4.244 (1.07), 4.256 (0.84), 4.262 (0.65), 4.272 (1.30), 4.290 (0.66), 4.470 (0.78), 4.478 (0.81), 4.490 (0.85), 4.498 (1.36), 4.505 (0.67), 4.518 (0.66), 4.525 (0.64), 4.659 (2.83), 4.673 (2.78), 7.161 (1.06), 7.179 (2.57), 7.196 (2.13), 7.216 (2.88), 7.235 (1.14), 7.326 (2.25), 7.329 (2.18), 7.345 (1.87), 7.349 (2.04), 7.356 (1.83), 7.365 (1.82), 7.368 (1.74), 7.674 (1.32), 7.687 (1.33), 8.262 (4.76), 8.274 (4.46), 8.452 (5.79), 13.670 (0.86), 14.562 (2.70).

### Intermediate 6-62

### N-(3-chloro-2-methyl-phenyl)-4-({3-[(5,5-dimethyl-1,4-dioxan-2-yl)methoxy]-4-pyridyl}methylamino)-3-methyl-6-oxo-2,3-dihydro-1H-pyridine-5-carbothioamide

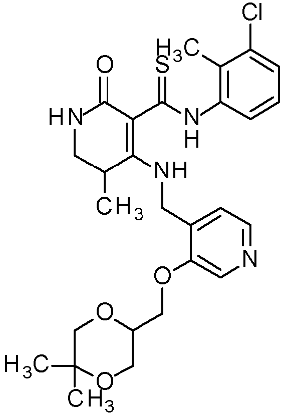

According to the method described for intermediate 6-41 using *N*-(3-chloro-2-methylphenyl)-4-hydroxy-3-methyl-6-oxo-2,3-dihydro-1*H*-pyridine-5-carbothioamide (intermediate 5-58, 200 mg, 643 µmol) and [3-[(5,5-dimethyl-1,4-dioxan-2-yl)methoxy]-4-pyridyl]methanamine (intermediate 2-62, 191 mg, 85% purity, 643 µmol) as starting materials, 165 mg (99 % purity, 47 % yield) of the title compound were prepared.

LC-MS (method 2): Rₜ = 1.21 min; MS (ESlpos): m/z = 545 [M+H]⁺

¹H-NMR (400 MHz, DMSO-d₆) δ [ppm]: 1.047 (15.23), 1.189 (4.61), 1.204 (4.66), 1.206 (4.60), 1.252 (12.49), 2.154 (16.00), 2.520 (1.89), 2.525 (1.19), 2.932 (0.62), 2.946 (0.70), 2.963 (0.77), 2.977 (0.68), 3.062 (0.71), 3.291 (1.83), 3.320 (3.42), 3.350 (1.03), 3.360 (0.80), 3.533 (3.08), 3.561 (2.48), 3.611 (0.57), 3.619 (0.80), 3.639 (1.60), 3.648 (1.83), 3.661 (1.07), 3.672 (0.98), 3.686 (1.30), 3.697 (1.23), 3.715 (0.70), 3.726 (0.71), 3.774 (0.42), 3.786 (0.88), 3.796 (0.98), 3.807 (0.76), 3.821 (0.59), 4.149 (0.41), 4.159 (0.44), 4.175 (1.17), 4.186 (1.16), 4.191 (1.48), 4.197 (1.68), 4.201 (2.09), 4.210 (1.40), 4.214 (1.21), 4.227 (0.46), 4.241 (0.41), 4.664 (2.17), 4.678 (2.14), 7.156 (1.01), 7.160 (1.19), 7.176 (2.76), 7.179 (2.49), 7.194 (2.03), 7.214 (2.79), 7.234 (1.13), 7.325 (2.20), 7.328 (2.11), 7.344 (1.78), 7.348 (1.67), 7.355 (2.55), 7.366 (2.56), 7.672 (1.32), 7.686 (1.34), 8.244 (4.07), 8.256 (3.92), 8.402 (3.95), 8.404 (3.78), 13.659 (0.84), 13.669 (0.85), 14.561 (2.20).

### Intermediate 6-63

### (6R)-N-[2-(2,2-difluoroethyl)-3-fluorophenyl]-6-methyl-2-oxo-4-{[(3-{[(2S)-oxolan-2-yl]methoxy}pyridin-4-yl)methyl]amino}-1,2,5,6-tetrahydropyridine-3-carbothioamide

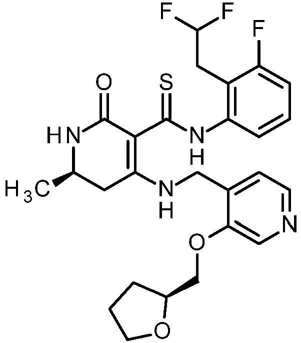

1-(3-{[(2*S*)-oxolan-2-yl]methoxy}pyridin-4-yl)methanamine (intermediate 2-3, 281 mg, 1.35 mmol) was three times triturated with toluene/chloroform and evaporated to dryness under reduced pressure. (6*R*)-*N*-[2-(2,2-difluoroethyl)-3-fluorophenyl]-4-hydroxy-6-methyl-2-oxo-1,2,5,6-tetrahydropyridine-3-carbothioamide (intermediate 5-63, 310 mg, 900 µmol) and dried 1-(3-{[(2*S*)-oxolan-2-yl]methoxy}pyridin-4-yl)methanamine were solved in 1,2-dimethoxyethane (1.3 ml) and cooled to 0°C. *N,O-*Bis(trimethylsilyl)trifluoroacetamide (440 µl, 1.8 mmol) was added and the mixture stirred for 1h at 80°C. The reaction mixture was diluted with ethylacetate and was washed three times with halfconcentrated sodium chlorid solution. The organic layer was dried and concentrated under reduced pressure. The residue was purified by flash chromatography (silica, DCM / EtOH 0-5%) to give 337 mg (70% yield) of the title compound.

LC-MS (method 2): Rₜ = 1.19 min; MS (ESlpos): m/z = 535 [M+H]⁺

¹H-NMR (400 MHz, DMSO-d₆) δ ppm: 1.13 (d, 3 H), 1.67 - 1.94 (m, 3 H), 1.95 - 2.08 (m, 1 H), 2.44 (dd, 1 H), 2.94 (dd, 1 H), 3.10 (td, 2 H), 3.48 (dt, 1 H), 3.63 - 3.72 (m, 1 H), 3.74 - 3.85 (m, 1 H), 4.07 - 4.27 (m, 3 H), 4.65 (d, 2 H), 5.99 - 6.32 (m, 1 H), 7.09 - 7.20 (m, 2 H), 7.29 (d, 1 H), 7.37 (td, 1 H), 7.72 (s, 1 H), 8.23 (d, 1 H), 8.39 (s, 1 H), 13.64 (br t, 1 H), 14.66 (s, 1 H).

### Intermediate 6-64

### tert-butyl (2S)-2-[({4-[({(4aS,7aR)-3-[(3-chloro-2-methoxyphenyl)carbamothioyl]-2-oxo-2,4a,5,6,7,7a-hexahydro-1H-cyclopenta[b]pyridin-4-yl}amino)methyl]pyridin-3-yl}oxy)methyl]morpholine-4-carboxylate

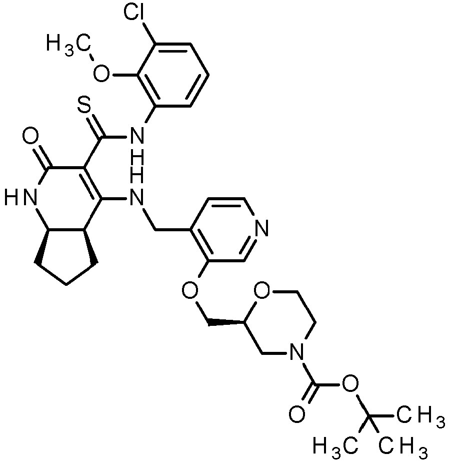

According to the method described for intermediate 6-41 using (4a*S*,7a*R*)-N-(3-chloro-2-methoxyphenyl)-4-hydroxy-2-oxo-2,4a,5,6,7,7a-hexahydro-1*H*-cyclopenta[b]pyridine-3-carbothioamide (intermediate 5-49, 200 mg, 567 µmol) and *tert-*butyl (2*S*)-2-({[4-(aminomethyl)pyridin-3-yl]oxy}methyl)morpholine-4-carboxylate (intermediate 2-11, 204 mg, 90% purity, 567 µmol) as starting materials, 144 mg (90% purity, 35% yield) of the title compound were prepared after purification by flash chromatography (silica, DCM / EtOH gradient 0-10%).

LC-MS (method 2): Rₜ = 1.34 min; MS (ESlpos): m/z = 658 [M+H]⁺

¹H-NMR (400 MHz, DMSO-d₆) δ [ppm]: 1.37 - 1.41 (s, 9H), 1.56 - 1.70 (m, 1H), 1.71 - 1.87 (m, 3H), 1.87 - 1.96 (m, 1H), 2.08 - 2.22 (m, 1H), 2.77 - 2.99 (m, 2H), 3.04 - 3.14 (m, 1H), 3.44 (m, 2H), 3.69 - 3.73 (s, 3H), 3.72 - 3.90 (m, 6H), 3.90 - 4.01 (m, 1H), 4.23 (d, 2H), 4.68 (br d, 2H), 7.09 (t, 1H), 7.28 (dd, 1H), 7.36 (d, 1H), 7.45 (s, 1H), 7.75 - 7.82 (dd, 1H), 8.25 (d, 1H), 8.42 (s, 1H), 13.78 - 13.93 (m, 1H), 15.02 (s, 1H).

### Intermediate 6-65

### (6R)-N-[2-(2,2-difluoroethyl)-3-fluorophenyl]-4-({[3-(2-methoxy-2-methylpropoxy)pyridin-4-yl]methyl}amino)-6-methyl-2-oxo-1,2,5,6-tetrahydropyridine-3-carbothioamide

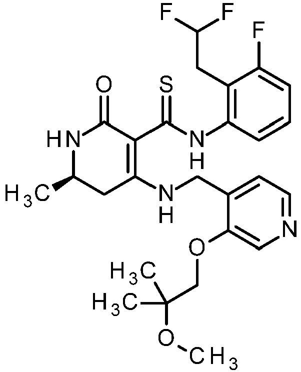

According to the method described for intermediate 6-1 using (6*R*)-*N*-[2-(2,2-difluoroethyl)-3-fluorophenyl]-4-hydroxy-6-methyl-2-oxo-1,2,5,6-tetrahydropyridine-3-carbothioamide (intermediate 5-63, 100 mg, 290 µmol) and 1-[3-(2-methoxy-2-methylpropoxy)pyridin-4-yl]methanamine (intermediate 2-1, 91.6 mg, 436 µmol) as starting materials, 163.5 mg (crude) of the title compound were prepared after purification by flash chromatography (amino phase silica, hexane / EtOAc gradient 25-100% and silica, DCM / EtOH gradient 1-10%).

LC-MS (method 2): Rₜ = 1.22 min; MS (ESlpos): m/z = 537 [M+H]⁺

¹H-NMR (400 MHz, DMSO-d₆) δ [ppm]: 14.65 (s, 1H), 13.54-13.70 (m, 1H), 8.40 (s, 1H), 8.23 (d, 1H), 7.72 (s, 1H), 7.36 (dt, 1H), 7.28 (d, 1H), 7.08-7.19 (m, 2H), 5.98-6.33 (m, 1H), 4.68 (d, 2H), 4.03 (s, 2H), 3.48 (td, 1H), 3.16 (s, 3H), 3.03-3.15 (m, 2H), 2.93 (dd, 1H), 2.40-2.47 (m, 1H), 1.23 (s, 6H), 1.13 (d, 3H).

### Intermediate 6-66

### N-(3-chloro-2-methyl-phenyl)-4-{[3-(1,4-dioxan-2-ylmethoxy)-4-pyridyl]methylamino}-3-methyl-6-oxo-2,3-dihydro-1H-pyridine-5-carbothioamide

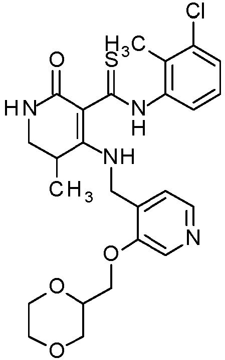

According to the method described for intermediate 6-41 using *N*-(3-chloro-2-methylphenyl)-4-hydroxy-3-methyl-6-oxo-2,3-dihydro-1H-pyridine-5-carbothioamide (intermediate 5-73, 200 mg, 643 µmol) and 1-{3-[(1,4-dioxan-2-yl)methoxy]pyridin-4-yl}methanamine (intermediate 2-36, 144 mg, 643 µmol) as starting materials, 58.7 mg (85% purity, 15% yield) of the title compound were prepared after purification by preparative HPLC (method 10, gradient: 0.00-0.50 min 30% B, 0.50-6.00 min 30-70% B).

LC-MS (method 2): Rₜ = 1.10 min; MS (ESlpos): m/z = 517 [M+H]⁺

¹H-NMR (400 MHz, DMSO-d₆) δ [ppm]: 1.157 (0.42), 1.174 (0.50), 1.194 (3.57), 1.200 (3.68), 1.212 (3.76), 1.217 (3.59), 1.239 (1.47), 1.257 (1.36), 1.989 (0.48), 2.076 (2.16), 2.093 (1.48), 2.120 (0.76), 2.132 (0.48), 2.153 (16.00), 2.520 (2.45), 2.525 (1.57), 2.936 (0.56), 2.950 (0.62), 2.967 (0.68), 2.981 (0.61), 3.078 (0.60), 3.356 (0.97), 3.366 (0.75), 3.393 (0.70), 3.406 (0.70), 3.417 (0.94), 3.420 (0.96), 3.431 (0.95), 3.434 (0.91), 3.445 (0.95), 3.458 (1.08), 3.467 (0.41), 3.484 (0.73), 3.490 (1.09), 3.495 (0.82), 3.510 (0.52), 3.514 (0.65), 3.518 (0.65), 3.522 (0.66), 3.585 (0.74), 3.591 (0.86), 3.613 (1.13), 3.619 (1.36), 3.645 (1.32), 3.653 (1.78), 3.660 (0.96), 3.683 (0.96), 3.746 (1.23), 3.753 (1.31), 3.780 (0.97), 3.838 (1.10), 3.845 (1.29), 3.873 (1.82), 3.885 (0.80), 3.892 (0.60), 3.897 (0.68), 3.909 (0.69), 3.916 (0.54), 4.143 (1.15), 4.159 (2.88), 4.171 (2.03), 4.660 (2.64), 4.675 (2.60), 7.154 (0.97), 7.157 (1.12), 7.173 (2.48), 7.177 (2.24), 7.187 (0.60), 7.196 (2.15), 7.215 (2.96), 7.235 (1.29), 7.325 (2.27), 7.328 (2.05), 7.347 (3.03), 7.360 (1.70), 7.672 (1.17), 7.686 (1.20), 8.242 (3.71), 8.254 (3.64), 8.397 (5.37), 13.662 (0.94), 14.460 (0.45), 14.563 (2.02).

### Intermediate 6-71

### (2R)-N-(3-chloro-2-methoxy-phenyl)-4-({3-[(3,3-difluorotetrahydrofuran-2-yl)methoxy]-4-pyridyl}methylamino)-2-methyl-6-oxo-2,3-dihydro-1H-pyridine-5-carbothioamide

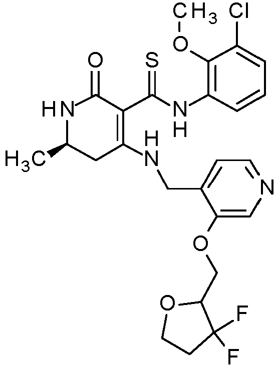

According to the method described for intermediate 6-41 using (6*R*)-*N*-(3-chloro-2-methoxyphenyl)-4-hydroxy-6-methyl-2-oxo-1,2,5,6-tetrahydropyridine-3-carbothioamide (intermediate 5-1, 300 mg, 918 µmol) and [3-[(3,3-difluorotetrahydrofuran-2-yl)methoxy]-4-pyridyl]methanamine (intermediate 2-71, 247 mg, 1.01 mmol) as starting materials, 467 mg (95% purity, 87% yield) of the title compound were prepared after purification by flash chromatography (silica, DCM / EtOH gradient 0-5 %).

LC-MS (method 2): Rₜ = 1.25 min; MS (ESlpos): m/z = 553 [M+H]⁺

¹H-NMR (400 MHz, DMSO-d₆) δ [ppm]: -0.116 (0.80), 0.000 (13.40), 0.008 (0.56), 0.012 (5.46), 0.059 (1.43), 0.118 (0.66), 0.127 (16.00), 0.135 (0.65), 1.113 (1.97), 1.129 (2.01), 1.753 (12.74), 1.850 (5.85), 2.076 (8.48), 2.477 (0.48), 2.520 (1.16), 2.525 (0.78), 2.536 (0.45), 3.716 (9.64), 3.927 (0.56), 3.949 (0.62), 4.349 (0.41), 4.364 (0.66), 4.659 (0.73), 4.673 (0.74), 5.294 (0.47), 7.094 (0.66), 7.114 (1.39), 7.134 (0.83), 7.290 (1.11), 7.294 (1.15), 7.310 (1.16), 7.314 (1.10), 7.741 (0.80), 7.814 (0.60), 7.835 (0.54), 14.883 (1.09).

### Intermediate 6-74

### (2R)-N-(3-chloro-2-methoxy-phenyl)-4-({3-[(3,3-difluorotetrahydropyran-2-yl)methoxy]-4-pyridyl}methylamino)-2-methyl-6-oxo-2,3-dihydro-1H-pyridine-5-carbothioamide

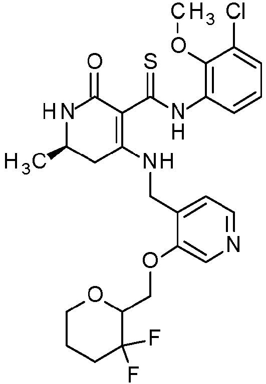

According to the method described for intermediate 6-41 using (6*R*)-*N*-(3-chloro-2-methoxyphenyl)-4-hydroxy-6-methyl-2-oxo-1,2,5,6-tetrahydropyridine-3-carbothioamide (intermediate 5-1, 300 mg, 918 µmol) and [3-[(3,3-difluorotetrahydropyran-2-yl)methoxy]-4-pyridyl]methanamine (intermediate 2-61, 261 mg, 1.01 mmol) as starting materials, 507 mg (90% purity, 88% yield) of the title compound were prepared after purification by flash chromatography (silica, DCM / EtOH gradient 0-5 %).

LC-MS (method 2): Rₜ = 1.29 min; MS (ESlpos): m/z = 567 [M+H]⁺

¹H-NMR (400 MHz, DMSO-d₆) δ [ppm]: 0.000 (5.51), 1.115 (1.18), 1.119 (1.27), 1.130 (1.25), 1.135 (1.23), 1.157 (4.11), 1.175 (8.61), 1.193 (4.32), 1.767 (0.50), 1.776 (0.42), 1.990 (16.00), 2.521 (1.24), 2.525 (0.80), 3.715 (7.15), 4.002 (1.24), 4.020 (3.61), 4.037 (3.46), 4.055 (1.12), 4.666 (0.89), 4.681 (0.93), 7.093 (0.59), 7.113 (1.26), 7.134 (0.73), 7.290 (0.67), 7.293 (0.70), 7.310 (1.37), 7.322 (0.87), 7.739 (0.53), 7.817 (0.62), 7.838 (0.57), 8.258 (1.28), 8.270 (1.23), 8.444 (1.99), 14.882 (0.94).

### Intermediate 6-77

### (2R)-N-(3-chloro-2-methoxy-phenyl)-4-({3-[(5,5-dimethyl-1,4-dioxan-2-yl)methoxy]-4-pyridyl}methylamino)-2-methyl-6-oxo-2,3-dihydro-1H-pyridine-5-carbothioamide

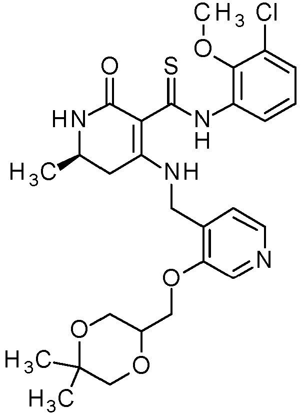

According to the method described for intermediate 6-41 using (6*R*)-*N*-(3-chloro-2-methoxyphenyl)-4-hydroxy-6-methyl-2-oxo-1,2,5,6-tetrahydropyridine-3-carbothioamide (intermediate 5-1, 300 mg, 918 µmol) and [3-[(5,5-dimethyl-1,4-dioxan-2-yl)methoxy]-4-pyridyl]methanamine (intermediate 2-62, 255 mg, 1.01 mmol) as starting materials, 446 mg (95 % purity, 82 % yield) of the title compound were prepared after purification by flash chromatography (silica, DCM / EtOH gradient 0-5 %).

LC-MS (method 2): Rₜ = 1.26 min; MS (ESlpos): m/z = 561 [M+H]⁺

¹H-NMR (400 MHz, DMSO-d₆) δ [ppm]: 1.042 (8.82), 1.123 (3.56), 1.139 (3.60), 1.154 (0.48), 1.172 (0.73), 1.250 (7.71), 1.987 (1.11), 2.423 (0.52), 2.465 (0.40), 2.518 (1.39), 2.523 (0.89), 2.911 (0.44), 2.921 (0.49), 2.952 (0.42), 3.298 (0.81), 3.466 (0.42), 3.535 (1.80), 3.563 (1.44), 3.648 (0.67), 3.654 (0.76), 3.657 (0.80), 3.662 (0.71), 3.670 (0.65), 3.677 (0.62), 3.695 (0.82), 3.702 (0.99), 3.712 (16.00), 3.725 (0.48), 3.731 (0.44), 3.800 (0.51), 3.811 (0.45), 4.181 (0.75), 4.184 (0.81), 4.191 (0.78), 4.194 (0.84), 4.199 (0.81), 4.205 (0.71), 4.212 (0.69), 4.219 (0.62), 4.667 (1.25), 4.683 (1.30), 7.087 (1.08), 7.107 (2.31), 7.127 (1.33), 7.285 (1.50), 7.289 (1.56), 7.305 (2.71), 7.308 (1.58), 7.317 (1.56), 7.733 (1.45), 7.815 (1.08), 7.818 (1.08), 7.835 (1.04), 7.838 (0.97), 8.241 (2.10), 8.252 (1.97), 8.398 (3.26), 13.747 (0.73), 14.879 (2.00).

### Intermediate 6-80

### (2R)-N-(3-chloro-2-methoxy-phenyl)-4-{[3-(2-methoxypropoxy)-4-pyridyl]methylamino}-2-methyl-6-oxo-2,3-dihydro-1H-pyridine-5-carbothioamide

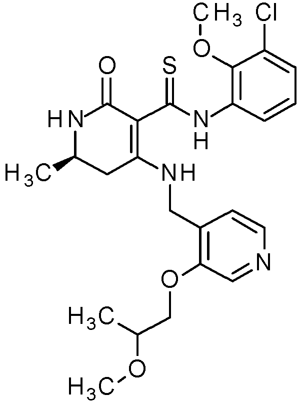

According to the method described for intermediate 6-41 using (6*R*)-*N*-(3-chloro-2-methoxyphenyl)-4-hydroxy-6-methyl-2-oxo-1,2,5,6-tetrahydropyridine-3-carbothioamide (intermediate 5-1, 300 mg, 918 µmol) and [3-(2-methoxypropoxy)-4-pyridyl]methanamine (intermediate 2-80, 198 mg, 1.01 mmol) as starting materials, 408 mg (95 % purity, 84 % yield) of the title compound were prepared after purification by flash chromatography (silica, DCM / EtOH gradient 0-5 %).

LC-MS (method 2): Rₜ = 1.23 min; MS (ESlpos): m/z = 505 [M+H]⁺

¹H-NMR (400 MHz, DMSO-d₆) δ [ppm]: 1.119 (1.55), 1.135 (1.57), 1.172 (0.46), 1.183 (1.75), 1.189 (1.87), 1.199 (1.77), 1.204 (1.83), 1.987 (0.64), 2.518 (0.51), 3.320 (5.28), 3.323 (5.65), 3.329 (16.00), 4.123 (1.31), 4.136 (1.07), 4.667 (0.85), 4.682 (0.86), 7.089 (0.56), 7.109 (1.21), 7.129 (0.70), 7.284 (0.78), 7.288 (0.83), 7.298 (0.79), 7.304 (0.77), 7.309 (1.15), 7.729 (0.70), 7.808 (0.55), 7.811 (0.57), 7.828 (0.54), 7.832 (0.50), 8.231 (1.28), 8.243 (1.18), 8.395 (1.90), 14.875 (0.95).

### Intermediate 6-83

### (2R)-N-(3-chloro-2-methoxy-phenyl)-2-methyl-4-({3-[(1-methyl-2-piperidyl)methoxy]-4-pyridyl}methylamino)-6-oxo-2,3-dihydro-1H-pyridine-5-carbothioamide

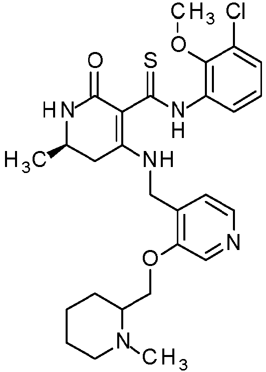

According to the method described for intermediate 6-30 using (6*R*)-N-(3-chloro-2-methoxyphenyl)-4-hydroxy-6-methyl-2-oxo-1,2,5,6-tetrahydropyridine-3-carbothioamide (intermediate 5-1, 300 mg, 918 µmol) and [3-[(1-methyl-2-piperidyl)methoxy]-4-pyridyl]methanamine (intermediate 2-83, 238 mg, 1.01 mmol) as starting materials, 400 mg (93% purity, 74% yield) of the title compound were prepared as precipitate from acetonitrile.

LC-MS (method 2): Rₜ = 1.27 min; MS (ESlpos): m/z = 544 [M+H]⁺

¹H-NMR (400 MHz, DMSO-d₆) δ [ppm]: 1.123 (2.28), 1.139 (2.29), 1.154 (0.89), 1.172 (1.71), 1.190 (0.87), 1.751 (16.00), 1.987 (2.76), 2.030 (0.43), 2.228 (1.29), 2.233 (3.95), 2.239 (4.00), 2.255 (0.46), 2.457 (0.42), 2.518 (0.81), 2.523 (0.53), 3.708 (9.50), 4.017 (0.71), 4.034 (0.85), 4.045 (0.51), 4.058 (0.55), 4.251 (0.49), 4.656 (0.97), 4.671 (0.96), 5.758 (0.95), 7.084 (0.81), 7.104 (1.73), 7.124 (1.01), 7.280 (1.37), 7.283 (1.43), 7.295 (1.13), 7.299 (1.34), 7.303 (1.35), 7.307 (1.09), 7.724 (0.99), 7.814 (0.78), 7.818 (0.80), 7.834 (0.75), 7.838 (0.72), 8.224 (1.05), 8.236 (1.05), 8.400 (1.61), 14.873 (0.82), 14.877 (0.83).

### Intermediate 6-86

### (2R)-N-(3-chloro-2-methoxy-phenyl)-2-methyl-6-oxo-4-{[3-(2-tetrahydropyran-2-ylethoxy)-4-pyridyl]methylamino}-2,3-dihydro-1H-pyridine-5-carbothioamide

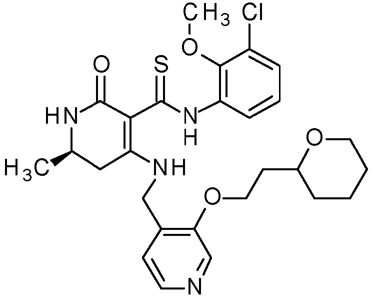

According to the method described for intermediate 6-30 using (6*R*)-*N*-(3-chloro-2-methoxyphenyl)-4-hydroxy-6-methyl-2-oxo-1,2,5,6-tetrahydropyridine-3-carbothioamide (intermediate 5-1, 300 mg, 918 µmol) and [3-(2-tetrahydropyran-2-ylethoxy)-4-pyridyl]methanamine (intermediate 2-86, 239 mg, 1.01 mmol) as starting materials, 400 mg (95% purity, 76% yield) of the title compound were prepared after purification by preparative HPLC (method 10, gradient: 0.00-0.50 min 30% B, 0.50-6.00 min 30-70% B).

LC-MS (method 2): Rₜ = 1.34 min; MS (ESlpos): m/z = 545 [M+H]⁺

¹H-NMR (400 MHz, DMSO-d₆) δ [ppm]: 0.000 (11.34), 1.130 (2.64), 1.144 (2.65), 1.425 (1.33), 1.434 (1.31), 1.441 (1.38), 1.584 (0.49), 1.617 (0.42), 1.728 (0.46), 1.853 (0.88), 1.871 (1.02), 1.886 (0.68), 2.077 (0.66), 2.423 (0.43), 2.466 (0.46), 2.520 (1.36), 2.525 (0.88), 2.941 (0.50), 2.982 (0.43), 3.311 (0.43), 3.443 (0.45), 3.457 (0.65), 3.464 (0.68), 3.471 (0.72), 3.476 (0.64), 3.483 (0.59), 3.710 (16.00), 3.826 (0.56), 3.831 (0.53), 3.857 (0.52), 4.191 (0.48), 4.201 (0.86), 4.206 (0.89), 4.217 (0.76), 4.646 (1.30), 4.661 (1.31), 7.085 (1.08), 7.106 (2.33), 7.126 (1.33), 7.282 (1.51), 7.286 (1.56), 7.302 (1.28), 7.306 (1.25), 7.315 (1.62), 7.327 (1.62), 7.727 (1.49), 7.823 (1.20), 7.827 (1.22), 7.844 (1.15), 7.847 (1.08), 8.218 (2.43), 8.230 (2.30), 8.373 (3.56), 13.750 (0.67), 14.884 (2.06).

### Intermediate 6-89

### tert-butyl (3S)-3-({[4-({5-[(3-chloro-2-methoxyphenyl)carbamothioyl]-3-(2-fluoroethyl)-6-oxo-1,2,3,6-tetrahydropyridin-4-yl}amino)methyl]pyridin-3-yl]oxy}methyl)morpholine-4-carboxylate

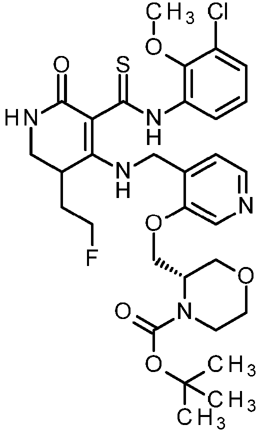

According to the method described for intermediate 6-1 using *N*-(3-chloro-2-methoxyphenyl)-5-(2-fluoroethyl)-4-hydroxy-2-oxo-1,2,5,6-tetrahydropyridine-3-carbothioamide (intermediate 5-13, 1.61 g, 4.50 mmol) and *tert-*butyl (3*S*)-3-({[4-(aminomethyl)pyridin-3-yl]oxy}methyl) morpholine-4-carboxylate (intermediate 2-89, 2.10 g, 90 % purity, 5.84 mmol) as starting materials, 590 mg (30% purity) of the title compound were prepared after purification by flash chromatography (amino phase silica, DCM / EtOH gradient 0-30%) next to 730 mg *tert-*butyl (3*S*)-3-({[4-({5-[(3-chloro-2-methoxy-phenyl)carbamothioyl]-3-(2-hydroxyethyl)-6-oxo-2,3-dihydro-1*H*-pyridin-4-yl}amino)methyl]-3-pyridyl}oxymethyl)morpholine-4-carboxylate (77% purity, 19% yield, see intermediate 6-115) as major side product of the reaction.

LC-MS (method 2): Rₜ = 1.32 min; MS (ESlpos): m/z = 664.3 [M+H]⁺

### Intermediate 6-91

### tert-butyl (2S)-2-[({4-[({(2R)-5-[(3-chloro-2-ethylphenyl)carbamothioyl]-2-methyl-6-oxo-1,2,3,6-tetrahydropyridin-4-yl}amino)methyl]pyridin-3-yl}oxy)methyl]morpholine-4-carboxylate

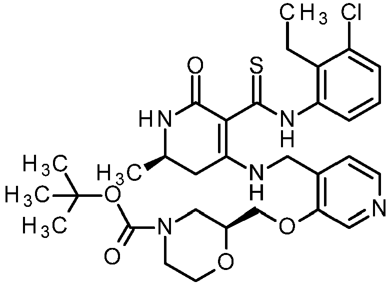

According to the method described for intermediate 6-41 using (6*R*)-N-(3-chloro-2-ethylphenyl)-4-hydroxy-6-methyl-2-oxo-1,2,5,6-tetrahydropyridine-3-carbothioamide (intermediate 5-91, 100 mg, 308 µmol) and *tert-*butyl (2*S*)-2-({[4-(aminomethyl)pyridin-3-yl]oxy}methyl)morpholine-4-carboxylate (intermediate 2-11, 99.6 mg, 308 µmol) as starting materials, 170 mg (90% purity, 78% yield) of the title compound were prepared after purification by flash chromatography (silica, DCM / EtOH gradient 0-10%).

LC-MS (method 2): Rₜ = 1.41 min; MS (ESlpos): m/z = 630 [M+H]⁺

¹H-NMR (400 MHz, DMSO-d₆): δ [ppm]= 1.06 (t, 3H), 1.13 (d, 3H), 1.39 (s, 10H), 2.60 (q, 2H), 2.91 (m, 3H), 3.45 (m, 2H), 3.68 - 3.77 (m, 2H), 3.80 - 3.88 (br d, 1H), 3.88 - 4.00 (br d, 1H), 4.22 (d, 2H), 4.66 (d, 2H), 7.18 - 7.25 (m, 2H), 7.28 - 7.33 (m, 2H), 7.71 (s, 1H), 8.24 (d, 1H), 8.40 (s, 1H), 13.73 (t, 1H), 14.71 (s, 1H).

### Intermediate 6-92

### (6R)-N-(3-chloro-2-methoxyphenyl)-6-methyl-2-oxo-4-{[(3-{2-[(2S)-oxolan-2-yl]ethoxy}pyridin-4-yl)methyl]amino}-1,2,5,6-tetrahydropyridine-3-carbothioamide

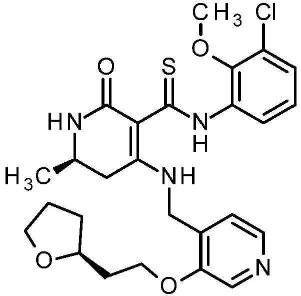

According to the method described for intermediate 6-41 using (6*R*)-N-(3-chloro-2-methoxyphenyl)-4-hydroxy-6-methyl-2-oxo-1,2,5,6-tetrahydropyridine-3-carbothioamide (intermediate 5-1, 300 mg, 918 µmol) and [3-[2-[(2*S*)-tetrahydrofuran-2-yl]ethoxy]-4-pyridyl]methanamine (intermediate 2-92, 224 mg, 1.01 mmol) as starting materials, 424 mg (95% purity, 83% yield) of the title compound were prepared after purification by flash chromatography (silica, DCM / EtOH gradient 0-5 %).

LC-MS (method 2): Rₜ = 1.27 min; MS (ESlpos): m/z = 531 [M+H]⁺

¹H-NMR (400 MHz, DMSO-d6) δ [ppm]: 1.124 (3.84), 1.140 (3.88), 1.450 (0.45), 1.472 (0.54), 1.479 (0.51), 1.501 (0.55), 1.781 (0.50), 1.802 (0.82), 1.817 (0.90), 1.836 (0.60), 1.917 (0.44), 1.921 (0.42), 1.933 (1.08), 1.937 (1.06), 1.950 (1.30), 1.960 (0.54), 1.964 (0.76), 1.969 (0.74), 1.977 (0.59), 1.990 (0.41), 1.997 (0.47), 2.391 (0.47), 2.418 (0.52), 2.432 (0.58), 2.459 (0.62), 2.518 (1.06), 2.522 (0.67), 2.920 (0.50), 2.929 (0.53), 2.960 (0.46), 2.970 (0.43), 3.459 (0.42), 3.562 (0.49), 3.578 (0.62), 3.582 (0.97), 3.598 (1.03), 3.601 (0.69), 3.618 (0.57), 3.708 (16.00), 3.725 (0.64), 3.740 (0.77), 3.744 (0.90), 3.759 (0.81), 3.763 (0.63), 3.779 (0.51), 3.947 (0.56), 3.963 (0.81), 3.979 (0.56), 4.202 (0.62), 4.217 (1.27), 4.230 (1.31), 4.246 (0.56), 4.649 (1.46), 4.664 (1.52), 5.758 (0.42), 7.086 (1.09), 7.106 (2.35), 7.126 (1.34), 7.282 (1.50), 7.286 (1.57), 7.302 (1.35), 7.306 (1.67), 7.309 (1.56), 7.321 (1.45), 7.724 (1.52), 7.817 (1.22), 7.821 (1.20), 7.837 (1.17), 7.841 (1.07), 8.220 (1.59), 8.232 (1.52), 8.379 (2.53), 13.732 (0.42), 13.747 (0.81), 14.877 (2.20).

### Intermediate 6-93

### (6R)-N-(3-chloro-2-methoxyphenyl)-6-methyl-4-{[(3-{[(3S)-4-methylmorpholin-3-yl]methoxy}pyridin-4-yl)methyl]amino}-2-oxo-1,2,5,6-tetrahydropyridine-3-carbothioamide

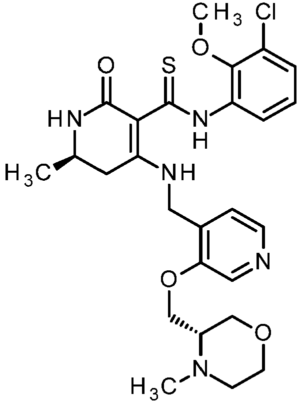

According to the method described for intermediate 6-41 using (6R)-N-(3-chloro-2-methoxyphenyl)-4-hydroxy-6-methyl-2-oxo-1,2,5,6-tetrahydropyridine-3-carbothioamide (intermediate 5-1, 300 mg, 918 µmol) and 1-(3-{[(3S)-4-methylmorpholin-3-yl]methoxy}pyridin-4-yl)methanamine (intermediate 2-93, 240 mg, 1.01 mmol) as starting materials, 420 mg (95 % purity, 80 % yield) of the title compound were prepared.

LC-MS (method 2): Rₜ = 1.13 min; MS (ESlpos): m/z = 546 [M+H]⁺

¹H-NMR (400 MHz, DMSO-d6) δ [ppm]: 0.000 (8.37), 1.126 (3.34), 1.142 (3.35), 2.220 (0.46), 2.223 (0.49), 2.228 (0.50), 2.231 (0.47), 2.249 (0.43), 2.288 (9.99), 2.407 (0.42), 2.435 (0.47), 2.448 (0.52), 2.476 (0.80), 2.520 (1.12), 2.525 (0.89), 2.542 (1.92), 2.652 (0.77), 2.658 (0.41), 2.671 (0.41), 2.675 (0.51), 2.681 (0.76), 2.924 (0.43), 2.933 (0.47), 2.964 (0.40), 3.349 (0.83), 3.373 (0.79), 3.377 (0.85), 3.401 (0.73), 3.476 (0.54), 3.483 (0.62), 3.503 (0.70), 3.509 (0.65), 3.529 (0.46), 3.686 (0.70), 3.693 (0.41), 3.712 (16.00), 3.891 (0.69), 3.899 (0.62), 3.919 (0.57), 3.927 (0.54), 4.022 (0.61), 4.038 (0.63), 4.047 (0.72), 4.063 (0.69), 4.304 (0.72), 4.315 (0.75), 4.330 (0.65), 4.340 (0.59), 4.667 (1.61), 4.682 (1.59), 7.088 (1.01), 7.108 (2.17), 7.129 (1.24), 7.284 (1.42), 7.288 (1.50), 7.298 (1.54), 7.304 (1.42), 7.309 (2.28), 7.729 (1.36), 7.810 (1.10), 7.814 (1.11), 7.831 (1.07), 7.834 (0.97), 8.237 (2.24), 8.249 (2.18), 8.417 (3.32), 13.728 (0.72), 14.874 (1.93).

### Intermediate 6-94

### N-(3-chloro-2-methoxy-phenyl)-4-({3-[(5,5-dimethyl-1,4-dioxan-2-yl)methoxy]-4-pyridyl}methylamino)-3-methyl-6-oxo-2,3-dihydro-1H-pyridine-5-carbothioamide

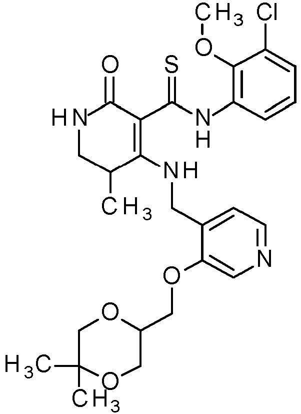

According to the method described for intermediate 6-41 using *N*-(3-chloro-2-methoxyphenyl)-4-hydroxy-3-methyl-6-oxo-2,3-dihydro-1H-pyridine-5-carbothioamide (intermediate 5-30, 200 mg, 612 µmol) and [3-[(5,5-dimethyl-1,4-dioxan-2-yl)methoxy]-4-pyridyl]methanamine (intermediate 2-62, 182 mg, 85% purity, 612 µmol) as starting materials, 150 mg (98 % purity, 43 % yield) of the title compound were prepared after purification by preparative HPLC (method 10, gradient: 0.00-0.50 min 30% B, 0.50-6.00 min 30-70% B).

LC-MS (method 2): Rₜ = 1.19 min; MS (ESlpos): m/z = 561 [M+H]⁺

¹H-NMR (400 MHz, DMSO-d6) δ [ppm]: 0.074 (1.10), 1.016 (1.81), 1.042 (8.37), 1.187 (2.15), 1.191 (2.31), 1.204 (3.41), 1.209 (2.31), 1.249 (7.21), 2.520 (1.39), 2.525 (1.00), 2.962 (0.42), 3.294 (0.99), 3.322 (2.20), 3.346 (0.72), 3.491 (0.41), 3.498 (0.41), 3.535 (1.52), 3.563 (1.22), 3.625 (0.48), 3.645 (0.99), 3.653 (1.09), 3.665 (0.60), 3.676 (0.55), 3.690 (0.74), 3.701 (0.77), 3.715 (16.00), 3.730 (0.46), 3.793 (0.48), 3.803 (0.55), 3.815 (0.42), 4.187 (0.67), 4.202 (0.91), 4.208 (1.07), 4.220 (0.83), 4.684 (1.18), 4.693 (1.19), 7.082 (1.06), 7.103 (2.31), 7.123 (1.32), 7.285 (1.37), 7.289 (1.42), 7.305 (1.20), 7.309 (1.14), 7.372 (1.28), 7.384 (1.29), 7.710 (0.73), 7.723 (0.74), 7.791 (1.14), 7.795 (1.13), 7.812 (1.10), 7.816 (0.99), 8.255 (2.33), 8.267 (2.15), 8.413 (2.19), 8.416 (2.12), 13.727 (0.51), 14.817 (1.56).

### Intermediate 6-98

### N-(3-chloro-2-methoxy-phenyl)-6-oxo-4-[(3-{[(2S)-tetrahydrofuran-2-yl]methoxy}-4-pyridyl)methylamino]-3-(3,3,3-trifluoropropyl)-2,3-dihydro-1H-pyridine-5-carbothioamide

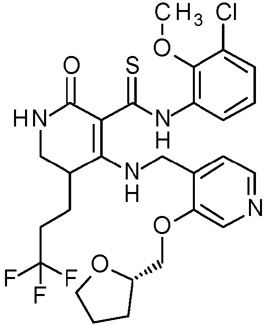

According to the method described for intermediate 6-63 using *N*-(3-chloro-2-methoxyphenyl)-4-hydroxy-6-oxo-3-(3,3,3-trifluoropropyl)-2,3-dihydro-1*H*-pyridine-5-carbothioamide (intermediate 5-98, 515 mg, 1.26 mmol, impure with N-(3-chloro-2-methoxyphenyl)-5-(3,3-difluoroprop-2-en-1-yl)-4-hydroxy-2-oxo-1,2,5,6-tetrahydropyridine-3-carbothioamide) and 1-(3-{[(2S)-oxolan-2-yl]methoxy}pyridin-4-yl)methanamine (intermediate 2-3, 393 mg, 1.89 mmol) as starting materials, 382 mg of the title compound (51% yield, impure with 3-(3-chloro-2-methoxy-anilino)-7-(3,3-difluoroallyl)-2-(3-{[(2*S*)-tetrahydrofuran-2-yl]methoxy}-4-pyridyl)-1,5,6,7-tetrahydropyrrolo[3,2-c]pyridin-4-one) were prepared after purification by flash chromatography (silica, DCM / EtOH gradient 0-5%).

LC-MS (method 2): Rₜ = 1.27 min; MS (ESlpos): m/z = 599 [M+H]⁺

### Intermediate 6-102

### (6R)-N-(3-chloro-2-methylphenyl)-4-[({3-[(1,4-dioxan-2-yl)methoxy]pyridin-4-yl}methyl)amino]-6-methyl-2-oxo-1,2,5,6-tetrahydropyridine-3-carbothioamide

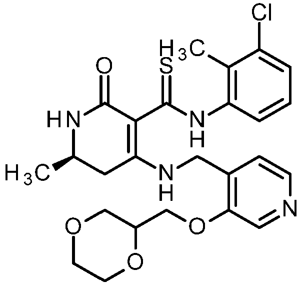

According to the method described for intermediate 6-30 using (6*R*)-*N-*(3-chloro-2-methylphenyl)-4-hydroxy-6-methyl-2-oxo-1,2,5,6-tetrahydropyridine-3-carbothioamide (Intermediate 5-102, 400 mg, 1.29 mmol) and 1-{3-[(1,4-dioxan-2-yl)methoxy]pyridin-4-yl}methanamine (Intermediate 2-24, 433 mg, 1.93 mmol) as starting materials, 417 mg (90% purity, 56% yield) of the title compound were prepared after purification by flash chromatography (silica, DCM / EtOH gradient 0-10 %).

LC-MS (method 2): Rₜ = 1.13 min; MS (ESlpos): m/z = 517 [M+H]⁺

¹H-NMR (400 MHz, DMSO-d₆): δ [ppm]: 1.13 (d, 3H), 2.16 (s, 3H), 2.37 - 2.46 (dd, 1H), 2.87 - 3.00 (dd, 1H), 3.39 - 3.54 (m, 4H), 3.58 - 3.70 (m, 2H), 3.75 (m, 1H), 3.82 - 3.97 (m, 2H), 4.07 - 4.24 (m, 2H), 4.65 (d, 2H), 7.14 - 7.24 (m, 3H), 7.30 (br d, 1H), 7.32 - 7.36 (dd, 1H), 7.63 - 7.73 (m, 1H), 8.20 - 8.29 (m, 1H), 8.34 - 8.45 (s, 1H), 13.63 - 13.75 (m, 1H), 14.62 (s, 1H).

### Intermediate 6-103

### (6R)-N-(3-chloro-2-methylphenyl)-4-{[(3-{[(2S)-1,4-dioxan-2-yl]methoxy}pyridin-4-yl)methyl]amino}-6-methyl-2-oxo-1,2,5,6-tetrahydropyridine-3-carbothioamide

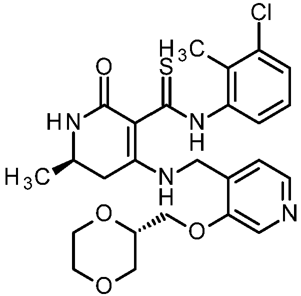

According to the method described for intermediate 6-41 using (6*R*)-N-(3-chloro-2-methylphenyl)-4-hydroxy-6-methyl-2-oxo-1,2,5,6-tetrahydropyridine-3-carbothioamide (intermediate 5-102, 158 mg, 510 µmol) and 1-(3-{[(2S)-1,4-dioxan-2-yl]methoxy}pyridin-4-yl)methanamine (200 mg, 80% purity, 713 µmol) as staring materials, 248 mg (90% purity, 85% yield) of the title compound were prepared after purification by flash chromatography (silica, DCM / EtOAc gradient 0-100% and DCM / EtOH gradient 0-30%).

LC-MS (method 2): Rₜ = 1.14 min; MS (ESlpos): m/z = 517.3 [M+H]⁺

¹H-NMR (400 MHz, DMSO-d₆): δ [ppm]: 14.55 - 14.67 (m, 1H), 13.63 - 13.75 (m, 1H), 8.36 - 8.43 (m, 1H), 8.20 - 8.27 (m, 1H), 7.65 - 7.72 (m, 1H), 7.28 - 7.37 (m, 2H), 7.15 - 7.24 (m, 2H), 4.62 - 4.69 (m, 2H), 4.10 - 4.21 (m, 2H), 3.84 - 3.96 (m, 2H), 3.73 - 3.79 (m, 1H), 3.58 - 3.69 (m, 2H), 3.39 - 3.54 (m, 4H), 2.89 - 2.99 (m, 1H), 2.38 - 2.46 (m, 1H), 2.13 - 2.18 (m, 3H), 1.11 - 1.17 (m, 3H).

### Intermediate 6-104

### (6R)-N-(3-chloro-2-methylphenyl)-6-methyl-2-oxo-4-{[(3-{[(2S)-oxolan-2-yl]methoxy}pyridin-4-yl)methyl]amino}-1,2,5,6-tetrahydropyridine-3-carbothioamide

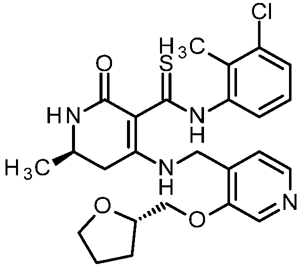

According to the method described for intermediate 6-41 using (6*R*)-N-(3-chloro-2-methylphenyl)-4-hydroxy-6-methyl-2-oxo-1,2,5,6-tetrahydropyridine-3-carbothioamide (intermediate 5-102, 300 mg, 965 µmol) and 1-(3-{[(2*S*)-oxolan-2-yl]methoxy}pyridin-4-yl)methanamine (intermediate 2-3, 201 mg, 965 µmol) as staring materials, 392 mg (97% purity, 79% yield) of the title compound were prepared after purification by flash chromatography (silica, DCM / EtOH gradient 0-10%).

LC-MS (method 2): Rₜ = 1.20 min; MS (ESlpos): m/z = 501 [M+H]⁺

¹H-NMR (400 MHz, DMSO-d₆) δ [ppm]: 1.13 (d, 3H), 1.69 - 1.79 (m, 1H), 1.79 - 1.95 (m, 2H), 1.95 - 2.05 (m, 1H), 2.16 (s, 3H), 2.39 - 2.47 (dd, 1H), 2.89 - 2.96 (dd, 1H), 3.41 - 3.51 (m, 1H), 3.65 - 3.71 (m, 1H), 3.75 - 3.83 (m, 1H), 4.09 - 4.15 (m, 2H), 4.15 - 4.23 (m, 1H), 4.65 (d, 2H), 7.15 - 7.19 (m, 1H), 7.19 - 7.26 (m, 1H), 7.29 (d, 1H), 7.34 (dd, 1H), 7.68 (s, 1H), 8.23 (br d, 1H), 8.39 (s, 1H), 13.70 (t, 1H), 14.61 (s, 1H).

### Intermediate 6-105

### (6R)-N-(3-chloro-2-methylphenyl)-4-[({3-[(1-methoxypropan-2-yl)oxy]pyridin-4-yl}methyl)amino]-6-methyl-2-oxo-1,2,5,6-tetrahydropyridine-3-carbothioamide

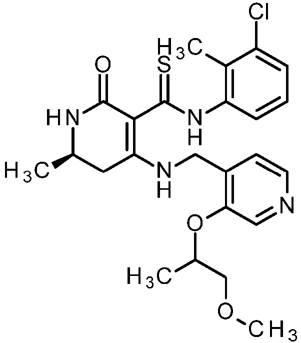

According to the method described for intermediate 6-41 using (6R)-N-(3-chloro-2-methylphenyl)-4-hydroxy-6-methyl-2-oxo-1,2,5,6-tetrahydropyridine-3-carbothioamide (intermediate 5-102, 206 mg, 662 µmol) and 1-{3-[(1-methoxypropan-2-yl)oxy]pyridin-4-yl}methanamine (200 mg, 65% purity, 662 µmol) as staring materials, 186 mg (87% purity, 50% yield) of the title compound were prepared after purification by flash chromatography (silica, DCM / EtOH gradient 0-10%).

LC-MS (method 2): Rₜ = 1.22 min; MS (ESlpos): m/z = 489 [M+H]⁺

¹H-NMR (400 MHz, DMSO-d₆) δ [ppm]: 1.11 - 1.15 (m, 3H), 1.27 (d, 3H), 2.13 - 2.17 (s, 3H), 2.34 - 2.45 (m, 1H), 2.85 - 3.01 (m, 1H), 3.28 (s, 3H), 3.43 - 3.58 (m, 3H), 4.62 (d, 2H), 4.75 - 4.86 (m, 1H), 7.13 - 7.25 (m, 2H), 7.27 - 7.37 (m, 2H), 7.67 (s, 1H), 8.20 (d, 1H), 8.44 (s, 1H), 13.59 - 13.76 (m, 1H), 14.58 - 14.63 (m, 1H).

### Intermediate 6-106

### tert-butyl (2R)-2-[({4-[({(2R)-5-[(3-chloro-2-methylphenyl)carbamothioyl]-2-methyl-6-oxo-1,2,3,6-tetrahydropyridin-4-yl}amino)methyl]pyridin-3-yl}oxy)methyl]morpholine-4-carboxylate

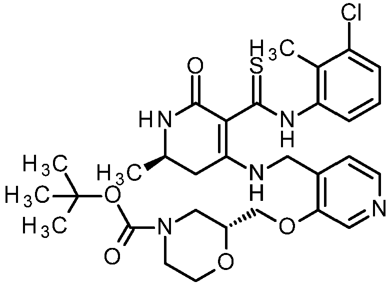

According to the method described for intermediate 6-30 using (6R)-N-(3-chloro-2-methylphenyl)-4-hydroxy-6-methyl-2-oxo-1,2,5,6-tetrahydropyridine-3-carbothioamide (intermediate 5-103, 158 mg, 510 µmol) and *tert-*butyl (2*R*)-2-({[4-(aminomethyl)pyridin-3-yl]oxy}methyl)morpholine-4-carboxylate (intermediate 2-106, 468 mg, 1.45 mmol) as starting materials, 592 mg (90% purity, 90% yield) of the title compound were prepared after purification by flash chromatography (silica, DCM / EtOH gradient 0-10%).

LC-MS (method 2): Rₜ = 1.31 min; MS (ESlpos): m/z = 616 [M+H]⁺

¹H-NMR (400 MHz, DMSO-d₆) δ [ppm]: 1.13 (d, 3H), 1.39 (s, 9H), 2.16 (s, 3H), 2.38 - 2.46 (dd, 1H), 2.88 - 2.96 (dd, 2H), 3.40 - 3.52 (m, 2H), 3.69 - 3.78 (m, 2H), 3.82 - 3.88 (m, 1H), 3.88 - 4.00 (m, 1H), 4.22 (d, 2H), 4.66 (d, 2H), 7.13 - 7.24 (m, 2H), 7.28 - 7.31 (d, 1H), 7.31 - 7.38 (dd, 1H), 7.68 (s, 1H), 8.19 - 8.29 (m, 1H), 8.38 - 8.47 (m, 1H), 13.65 - 13.76 (m, 1H), 14.61 (s, 1H).

### Intermediate 6-107

### tert-butyl (2S)-2-[({4-[({(2R)-5-[(3-chloro-2-methylphenyl)carbamothioyl]-2-methyl-6-oxo-1,2,3,6-tetrahydropyridin-4-yl}amino)methyl]pyridin-3-yl}oxy)methyl]morpholine-4-carboxylate

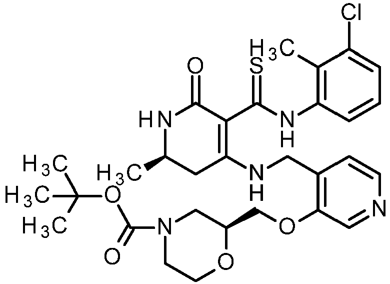

According to the method described for intermediate 6-41 using (6R)-N-(3-chloro-2-methylphenyl)-4-hydroxy-6-methyl-2-oxo-1,2,5,6-tetrahydropyridine-3-carbothioamide (intermediate 5-102, 300 mg, 965 µmol) and *tert-*butyl (2*S*)-2-({[4-(aminomethyl)pyridin-3-yl]oxy}methyl)morpholine-4-carboxylate (intermediate 2-11, 312 mg, 965 µmol) as staring materials, 505 mg (91% purity, 77% yield) of the title compound were prepared after purification by flash chromatography (silica, DCM / EtOH gradient 0-10%).

LC-MS (method 2): Rₜ = 1.31 min; MS (ESlpos): m/z = 616.4 [M+H]⁺

¹H-NMR (400 MHz, DMSO-d₆) δ [ppm]: 1.10 - 1.15 (d, 3H), 1.39 (s, 9H), 2.15 (s, 3H), 2.38 - 2.47 (dd, 1H), 2.88 - 2.97 (dd, 2H), 3.38 - 3.53 (m, 2H), 3.67 - 3.77 (m, 2H), 3.81 - 3.89 (m, 1H), 3.89 - 4.04 (m, 1H), 4.22 (d, 2H), 4.66 (d, 2H), 7.13 - 7.24 (m, 2H), 7.30 (br d, 1H), 7.32 (br d, 1H), 7.68 (s, 1H), 8.20 - 8.28 (d, 1H), 8.34 - 8.46 (d, 1H), 13.68 - 13.75 (m, 1H), 14.61 (s, 1H).

### Intermediate 6-108

### (2R)-N-[2-(2,2-difluoroethyl)-3-fluoro-phenyl]-4-{[3-(1,4-dioxan-2-ylmethoxy)-4-pyridyl]methylamino}-2-methyl-6-oxo-2,3-dihydro-1H-pyridine-5-carbothioamide

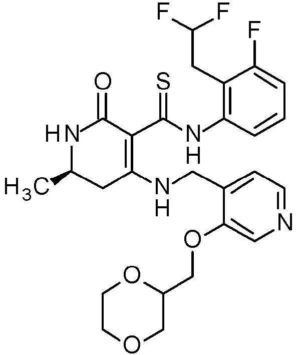

(6*R*)-*N*-[2-(2,2-difluoroethyl)-3-fluorophenyl]-4-hydroxy-6-methyl-2-oxo-1,2,5,6-tetrahydropyridine-3-carbothioamide (intermediate 5-63, 50 mg, 145 µmol) and 1-{3-[(1,4-dioxan-2-yl)methoxy]pyridin-4-yl}methanamine (intermediate 2-24, 48.8 mg, 218 µmol) were stirred for 2 h at 100°C under argon atmosphere. The reaction mixture was purified by flash chromatography (silica, DCM / EtOH gradient 0-20%) to give 58.0 mg (73% yield) of the title compound.

LC-MS (method 2): Rₜ = 1.08 min; MS (ESlpos): m/z = 551 [M+H]⁺

¹H-NMR (400 MHz, DMSO-d₆) δ [ppm]: 0.798 (0.99), 0.814 (1.12), 0.821 (1.18), 0.840 (0.99), 0.851 (1.59), 0.886 (0.58), 0.904 (1.07), 0.923 (0.55), 1.035 (4.37), 1.052 (9.06), 1.070 (4.63), 1.127 (15.74), 1.142 (16.00), 1.232 (6.51), 1.505 (0.47), 1.907 (1.01), 1.984 (0.73), 2.395 (2.20), 2.423 (2.51), 2.437 (2.78), 2.466 (4.83), 2.917 (2.28), 2.927 (2.49), 2.959 (2.13), 2.967 (1.99), 3.058 (2.09), 3.094 (3.81), 3.103 (3.90), 3.138 (2.04), 3.221 (0.61), 3.397 (2.21), 3.403 (2.19), 3.422 (5.36), 3.426 (4.59), 3.435 (3.78), 3.440 (3.16), 3.452 (5.88), 3.469 (3.90), 3.480 (2.87), 3.490 (4.80), 3.497 (4.64), 3.517 (2.11), 3.525 (2.99), 3.591 (1.99), 3.620 (3.65), 3.650 (6.39), 3.679 (3.18), 3.744 (3.79), 3.752 (4.08), 3.777 (3.11), 3.851 (3.92), 3.879 (5.54), 3.894 (2.52), 3.899 (2.41), 3.906 (2.41), 3.912 (2.27), 3.918 (2.02), 3.924 (1.59), 4.110 (1.17), 4.121 (1.93), 4.136 (3.35), 4.143 (4.93), 4.153 (6.15), 4.166 (3.59), 4.171 (3.13), 4.193 (0.84), 4.344 (1.57), 4.357 (2.96), 4.370 (1.50), 4.654 (8.16), 4.669 (8.23), 5.321 (0.58), 5.759 (13.29), 6.004 (0.68), 6.015 (1.34), 6.145 (1.37), 6.156 (2.70), 6.166 (1.38), 6.297 (1.28), 7.125 (5.34), 7.134 (3.21), 7.145 (6.19), 7.156 (4.80), 7.179 (3.36), 7.201 (0.97), 7.222 (0.79), 7.286 (6.38), 7.298 (6.43), 7.339 (2.23), 7.360 (3.78), 7.376 (3.78), 7.396 (1.77), 7.723 (6.82), 8.173 (0.62), 8.184 (0.61), 8.230 (7.67), 8.241 (7.33), 8.297 (1.25), 8.384 (12.84), 13.637 (3.03), 14.660 (6.94).

### Intermediate 6-109

### (6R)-N-(3-chloro-2-ethylphenyl)-6-methyl-2-oxo-4-{[(3-{[(2S)-oxolan-2-yl]methoxy}pyridin-4-yl)methyl]amino}-1,2,5,6-tetrahydropyridine-3-carbothioamide

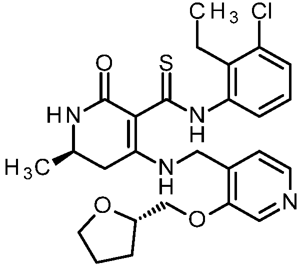

According to the method described for intermediate 6-41 using (6*R*)-*N*-(3-chloro-2-ethylphenyl)-4-hydroxy-6-methyl-2-oxo-1,2,5,6-tetrahydropyridine-3-carbothioamide (intermediate 5-91, 100 mg, 308 µmol) and 1-(3-{[(2S)-oxolan-2-yl]methoxy}pyridin-4-yl)methanamine (intermediate 2-3, 64.1 mg, 308 µmol) as staring materials, 130 mg (95% purity, 78% yield) of the title compound were prepared after purification by flash chromatography (silica, DCM / EtOH gradient 0-10%).

LC-MS (method 2): Rₜ = 1.29 min; MS (ESlpos): m/z = 515 [M+H]⁺

¹H-NMR (400 MHz, DMSO-d₆) δ [ppm]: 1.06 (t, 3H), 1.13 (d, 3H), 1.66 - 1.79 (m, 1H), 1.79 - 1.93 (m, 2H), 1.93 - 2.05 (m, 1H), 2.07 (d, 1H), 2.60 (q, 2H), 2.88 - 2.99 (dd, 1H), 3.41 - 3.54 (m, 1H), 3.61 - 3.73 (q, 1H), 3.73 - 3.85 (q, 1H), 4.14 (m, 2H), 4.16 - 4.26 (m, 1H), 4.65 (d, 2H), 7.19 - 7.25 (m, 2H), 7.30 (m, 2H), 7.70 (s, 1H), 8.15 - 8.28 (d, 1H), 8.39 (s, 1H), 13.67 - 13.76 (t, 1H), 14.71 (s, 1H).

### Intermediate 6-110

### (6R)-N-(3-chloro-2-ethylphenyl)-4-{[(3-{[(2S)-1,4-dioxan-2-yl]methoxy}pyridin-4-yl)methyl]amino}-6-methyl-2-oxo-1,2,5,6-tetrahydropyridine-3-carbothioamide

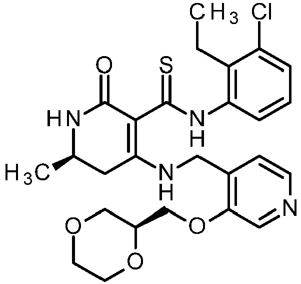

According to the method described for intermediate 6-41 using (6*R*)-*N*-(3-chloro-2-ethylphenyl)-4-hydroxy-6-methyl-2-oxo-1,2,5,6-tetrahydropyridine-3-carbothioamide (intermediate 5-91, 100 mg, 308 µmol) and 1-(3-{[(2S)-1,4-dioxan-2-yl]methoxy}pyridin-4-yl)methanamine (intermediate 2-103, 69.0 mg, 308 µmol) as staring materials, 180 mg (70% purity, 77% yield) of the title compound were prepared after purification by flash chromatography (silica, DCM / EtOH gradient 0-10%).

LC-MS (method 2): Rₜ = 1.24 min; MS (ESlpos): m/z = 531 [M+H]⁺

¹H-NMR (400 MHz, DMSO-d₆) δ [ppm]: 1.06 - 1.08 (m, 3H), 1.14 (d, 3H), 2.60 (q, 2H), 2.89 - 2.98 (dd, 1H), 3.40 - 3.42 (m, 1H), 3.46 - 3.47 (m, 2H), 3.49 - 3.53 (dd, 1H), 3.58 - 3.69 (m, 2H), 3.74 - 3.79 (m, 1H), 3.85 - 3.94 (m, 2H), 4.15 (dd, 2H), 4.66 (d, 2H), 7.19 - 7.25 (m, 2H), 7.28 - 7.33 (m, 2H), 7.71 (s, 1H), 8.24 (d, 1H), 8.38 (s, 1H), 13.71 (t, 1H), 14.72 (s, 1H).

### Intermediate 6-111

### N-(3-chloro-2-methoxyphenyl)-7-{[(3-{[(2S)-1,4-dioxan-2-yl]methoxy}pyridin-4-yl)methyl]amino}-5-oxo-4-azaspiro[2.5]oct-6-ene-6-carbothioamide

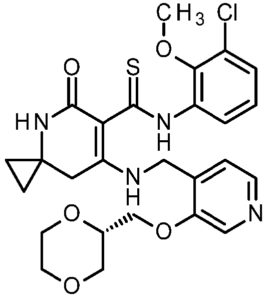

According to the method described for intermediate 6-41 using *N*-(3-chloro-2-methoxyphenyl)-7-hydroxy-5-oxo-4-azaspiro[2.5]oct-6-ene-6-carbothioamide (intermediate 5-111, 163 mg, 482 µmol) and 1-(3-{[(2*S*)-1,4-dioxan-2-yl]methoxy}pyridin-4-yl)methanamine (intermediate 2-103, 120 mg, 482 µmol) as staring materials, 220 mg (95% purity, 80% yield) of the title compound were prepared after purification by flash chromatography (silica, DCM / EtOH gradient 0-15%).

LC-MS (method 2): Rₜ = 1.15 min; MS (ESlpos): m/z = 545.3 [M+H]⁺

¹H-NMR (400 MHz, DMSO-d₆) δ [ppm]: 14.78 (s, 1H), 13.70 - 13.84 (m, 1H), 8.39 (s, 1H), 8.25 (d, 1H), 7.93 (s, 1H), 7.78 - 7.87 (m, 1H), 7.26 - 7.37 (m, 2H), 7.12 (t, 1H), 4.63 (d, 2H), 4.08 - 4.22 (m, 2H), 3.82 - 3.96 (m, 2H), 3.72 (s, 3H), 3.57 - 3.80 (m, 3H), 3.38 - 3.55 (m, 3H), 2.80 (s, 2H) 0.68 - 0.79 (m, 2H), 0.54 - 0.65 (m, 2H).

### Intermediate 6-112

### tert-butyl (2S)-2-[({4-[({6-[(3-chloro-2-methoxyphenyl)carbamothioyl]-5-oxo-4-azaspiro[2.5]oct-6-en-7-yl}amino)methyl]pyridin-3-yl}oxy)methyl]morpholine-4-carboxylate

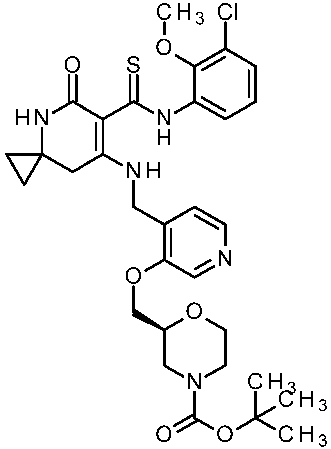

According to the method described for intermediate 6-41 using N-(3-chloro-2-methoxyphenyl)-7-hydroxy-5-oxo-4-azaspiro[2.5]oct-6-ene-6-carbothioamide (intermediate 5-111, 210 mg, 618 µmol) and *tert-*butyl (2*S*)-2-({[4-(aminomethyl)pyridin-3-yl]oxy}methyl)morpholine-4-carboxylate (intermediate 2-111, 200 mg, 618 µmol) as staring materials, 300,7 mg (95% purity, 71% yield) of the title compound were prepared after purification by flash chromatography (silica, DCM / EtOH gradient 0-15%).

LC-MS (method 2): Rₜ = 1.35 min; MS (ESlpos): m/z = 644.6 [M+H]⁺

¹H NMR (400 MHz, DMSO-d₆) δ [ppm]: 0.56 - 0.63 (m, 2H), 0.69 - 0.76 (m, 2H), 1.39 (s, 9H), 2.79 (s, 2H), 3.39 - 3.49 (m, 1 H), 3.70 - 3.77 (m, 2H) 3.72 (s, 3H), 3.80 - 3.97 (m, 2H), 4.22 (d, 2H), 4.63 (d, 2H), 7.10 (t, 1H), 7.26 - 7.32 (m, 2H), 7.83 (dd, 1H), 7.93 (s, 1H), 8.25 (d, 1H), 8.40 (s, 1H), 13.79 (t, 1 H), 14.78 (s, 1H).

### Intermediate 6-113

### N-(3-chloro-2-methoxyphenyl)-5-oxo-7-{[(3-{[(2S)-oxolan-2-yl]methoxy}pyridin-4-yl)methyl]amino}-4-azaspiro[2.5]oct-6-ene-6-carbothioamide

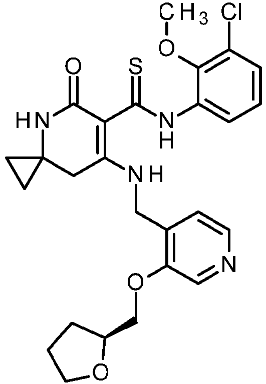

According to the method described for intermediate 6-41 using *N*-(3-chloro-2-methoxyphenyl)-7-hydroxy-5-oxo-4-azaspiro[2.5]oct-6-ene-6-carbothioamide (intermediate 5-111, 163 mg, 480 µmol) and 1-(3-{[(2*S*)-oxolan-2-yl]methoxy}pyridin-4-yl)methanamine (intermediate 2-3,100 mg, 480 µmol) as staring materials, 240 mg (95% purity, 85% yield) of the title compound were prepared after purification by flash chromatography (silica, DCM / EtOH gradient 1-15%).

LC-MS (method 2): Rₜ = 1.23 min; MS (ESlpos): m/z = 529.3 [M+H]⁺

1H NMR (400 MHz, DMSO-d₆) δ ppm] 0.57 - 0.61 (m, 2H), 0.70 - 0.75 (m, 2H) 1.69 - 1.94 (m, 3H) 1.96 - 2.04 (m, 1H), 2.79 (s, 2 H) 3.65 - 3.71 (m, 1H) 3.72 (s, 3H), 3.75 - 3.81 (m, 1H) 4.09 - 4.23 (m, 3H) 4.62 (d, 1H) 7.12 (t, 1H) 7.28 - 7.32 (m, 2H), 7.83 (dd, 1H) 7.93 (s, 1 H) 8.24 (d, 1H) 8.39 (s, 1H) 13.75 - 13.80 (m, 1H) 14.77 (s, 1H).

### Intermediate 6-114

### 3-allyl-4-{[3-(2-allyloxyethoxy)-4-pyridyl]methylamino}-N-(3-chloro-2-methoxy-phenyl)-6-oxo-2,3-dihydro-1H-pyridine-5-carbothioamide

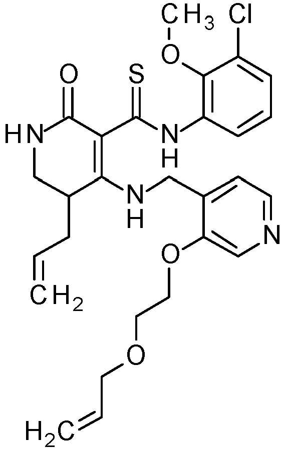

[3-(2-Allyloxyethoxy)-4-pyridyl]methanamine (Intermediate 2-114, 95.6 mg, 459 µmol) was treated three times with toluene/chloroform and dried under reduced pressure. 3-allyl-*N*-(3-chloro-2-methoxy-phenyl)-4-hydroxy-6-oxo-2,3-dihydro-1H-pyridine-5-carbothioamide (Intermediate 5-114, 108 mg, 306 µmol) and triethylamine (16 µL, 120 µmol) were added and the mixture was stirred for 75 min at 120°C. The reaction mixture was soluted in DCM and purified by flash chromatography (silica, DCM / EtOH gradient 0-7%) to give 17.0 mg (10% yield) of the title compound.

LC-MS (method 2): Rₜ = 1.26 min; MS (ESlpos): m/z = 543 [M+H]⁺

¹H+NMR (400 MHz, DMSO-d₆) δ [ppm]: 2.15 - 2.24 (m, 1 H), 2.25 - 2.36 (m, 1 H), 2.95 - 3.10 (m, 2 H), 3.24 (dd, 1 H), 3.71 (s, 3 H), 3.76 (t, 2 H), 4.01 (dt, 2 H), 4.25 - 4.39 (m, 2 H), 4.62 - 4.75 (m, 2 H), 5.11 - 5.19 (m, 3 H), 5.19 - 5.28 (m, 1 H), 5.77 - 5.95 (m, 2 H), 7.07 - 7.14 (m, 1 H), 7.30 (dd, 1 H), 7.39 (d, 1 H), 7.69 (d, 1 H), 7.81 (dd, 1 H), 8.25 (d, 1 H), 8.43 (s, 1 H), 13.79 (br t, 1 H), 14.78 (s, 1 H).

### Intermediate 6-115

### tert-butyl (3S)-3-({4-[({5-[(3-chloro-2-methoxy-phenyl)carbamothioyl]-3-(2-hydroxyethyl)-6-oxo-2,3-dihydro-1H-pyridin-4-yl}amino)methyl]-3-pyridyl}oxymethyl)morpholine-4-carboxylate

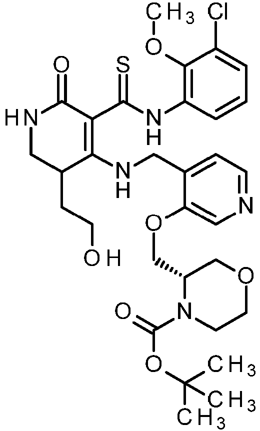

730 mg (77% purity, 19% yield) of the title compound were isolated as major side product in the preparation of intermediate 6-89 from *N*-(3-chloro-2-methoxyphenyl)-5-(2-fluoroethyl)-4-hydroxy-2-oxo-1,2,5,6-tetrahydropyridine-3-carbothioamide (1.61 g, 4.50 mmol) and *tert-*butyl (3S)-3-({[4-(aminomethyl)pyridin-3-yl]oxy}methyl)morpholine-4-carboxylate (2.10 g, 5.84 mmol).

LC-MS (method 2): Rₜ = 1.24 min; MS (ESlpos): m/z = 662.3 [M+H]⁺

¹H-NMR (400 MHz, DMSO-d₆) δ [ppm]: 14.77 (d, 1H), 13.64 - 13.79 (m, 1H), 8.51 (s, 1H), 8.27 (d, 1H), 7.79 - 7.87 (m, 1H), 7.67 (br t, 1H), 7.26 - 7.39 (m, 2H), 7.10 (t, 1H), 4.59 - 4.86 (m, 3H), 4.40 - 4.52 (m, 1H), 4.15 - 4.28 (m, 3H), 3.89 - 4.02 (m, 1H), 3.77 - 3.85 (m, 1H), 3.71 (s, 3H), 3.60 - 3.69 (m, 1H), 3.35 - 3.58 (m, 3H), 3.00 - 3.28 (m, 4H), 1.74 - 1.88 (m, 1H), 1.50 - 1.64 (m, 1H), 1.35 (br s, 9H).

### Intermediate 6-117

### N-(3-chloro-2-methyl-phenyl)-3-methyl-6-oxo-4-{[3-(tetrahydropyran-2-ylmethoxy)-4-pyridyl]methylamino}-2,3-dihydro-1H-pyridine-5-carbothioamide

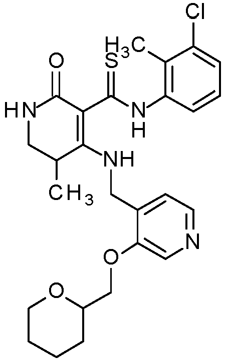

According to the method described for intermediate 6-41 using *N*-(3-chloro-2-methylphenyl)-4-hydroxy-3-methyl-6-oxo-2,3-dihydro-1*H*-pyridine-5-carbothioamide (intermediate 5-58, 200 mg, 643 µmol) and 1-(3-{[oxan-2-yl]methoxy}pyridin-4-yl)methanamine (intermediate 2-27, 143 mg, 643 µmol) as starting materials, 149 mg (98 % purity, 44 % yield) of the title compound were prepared after purification by preparative HPLC (method 10, gradient: 0.00-0.50 min 30% B, 0.50-6.00 min 30-70% B).

LC-MS (method 2): Rₜ = 1.25 min; MS (ESlpos): m/z = 515 [M+H]⁺

¹H-NMR (400 MHz, DMSO-d₆) δ [ppm]: 1.193 (4.27), 1.195 (4.45), 1.210 (4.44), 1.213 (4.41), 1.333 (0.60), 1.355 (0.51), 1.363 (0.46), 1.480 (2.76), 1.652 (0.88), 1.683 (0.74), 1.798 (0.83), 2.152 (16.00), 2.520 (2.60), 2.525 (1.65), 2.932 (0.61), 2.947 (0.70), 2.963 (0.75), 2.977 (0.69), 3.073 (0.67), 3.350 (1.32), 3.367 (1.12), 3.375 (1.08), 3.394 (0.48), 3.403 (0.60), 3.645 (0.73), 3.649 (0.65), 3.660 (0.68), 3.672 (0.70), 3.683 (0.42), 3.875 (0.97), 3.902 (0.94), 4.077 (0.43), 4.093 (1.21), 4.104 (1.40), 4.109 (2.65), 4.121 (2.72), 4.127 (1.32), 4.138 (0.45), 4.653 (2.84), 4.667 (2.82), 7.154 (1.13), 7.172 (2.46), 7.194 (1.88), 7.213 (2.77), 7.233 (1.16), 7.323 (2.21), 7.327 (2.22), 7.342 (3.86), 7.353 (2.68), 7.665 (1.27), 7.678 (1.30), 8.228 (4.42), 8.240 (4.09), 8.395 (6.58), 13.645 (0.60), 13.659 (1.11), 14.557 (2.47).

### Intermediate 6-120

### (2R)-N-(3-chloro-2-methoxy-phenyl)-4-{[3-(3-methoxybutoxy)-4-pyridyl]methylamino}-2-methyl-6-oxo-2,3-dihydro-1H-pyridine-5-carbothioamide

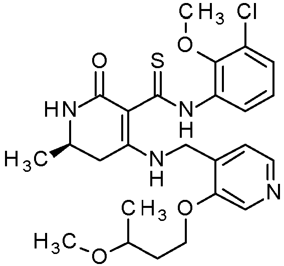

According to the method described for intermediate 6-108 using (6*R*)-*N*-(3-chloro-2-methoxyphenyl)-4-hydroxy-6-methyl-2-oxo-1,2,5,6-tetrahydropyridine-3-carbothioamide (Intermediate 5-1, 200 mg, 612 µmol) and [3-(3-methoxybutoxy)-4-pyridyl]methanamine (Intermediate 2-120, 193 mg, 918 µmol) as starting materials, 247 mg (95 % purity, 74 % yield) of the title compound were prepared after purification by flash chromatography (silica, DCM / EtOH gradient 0-5%).

LC-MS (method 2): Rₜ = 1.22 min; MS (ESlpos): m/z = 519 [M+H]⁺

¹H-NMR (400 MHz, DMSO-d₆) δ [ppm]: 0.902 (0.44), 1.036 (1.67), 1.053 (3.20), 1.071 (1.77), 1.119 (5.42), 1.122 (7.79), 1.135 (5.32), 1.137 (7.44), 1.885 (0.80), 1.902 (1.12), 1.917 (0.82), 2.066 (0.59), 2.084 (0.53), 2.418 (0.57), 2.518 (0.67), 2.523 (0.46), 2.928 (0.49), 2.968 (0.43), 3.218 (10.64), 3.220 (11.36), 3.423 (0.89), 3.435 (1.06), 3.440 (0.96), 3.453 (1.04), 3.457 (0.62), 3.470 (0.53), 3.511 (0.44), 3.525 (0.76), 3.542 (0.69), 3.709 (16.00), 4.185 (0.51), 4.199 (1.20), 4.214 (1.01), 4.226 (0.40), 4.232 (0.41), 4.343 (0.56), 4.355 (1.09), 4.368 (0.54), 4.651 (1.65), 4.665 (1.68), 5.758 (13.81), 7.086 (1.14), 7.106 (2.45), 7.126 (1.42), 7.281 (1.62), 7.285 (1.70), 7.301 (1.44), 7.305 (1.51), 7.310 (1.68), 7.321 (1.63), 7.724 (1.56), 7.815 (0.72), 7.818 (1.25), 7.822 (0.71), 7.835 (0.70), 7.839 (1.16), 7.842 (0.63), 8.220 (2.41), 8.231 (2.28), 8.383 (3.53), 13.733 (0.42), 13.747 (0.82), 14.877 (1.77).

### Intermediate 6-123

### N-(3-chloro-2-methoxy-phenyl)-8-{[3-(1,4-dioxan-2-ylmethoxy)-4-pyridyl]methylamino}-6-oxo-5-azaspiro[3.5]non-7-ene-7-carbothioamide

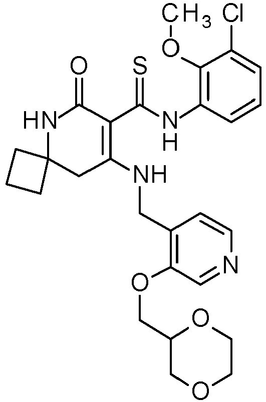

According to the method described for intermediate 6-30 using *N*-(3-chloro-2-methoxyphenyl)-8-hydroxy-6-oxo-5-azaspiro[3.5]non-7-ene-7-carbothioamide (Intermediate 5-123, 400 mg, 1.13 mmol) and 1-{3-[(1,4-dioxan-2-yl)methoxy]pyridin-4-yl}methanamine (Intermediate 2-24, 254 mg, 1.13 mmol) as starting materials, 634 mg (80% purity, 80% yield) of the title compound were prepared after filtering off the solid product followed by washing with acetonitrile.

LC-MS (method 2): Rₜ = 1.19 min; MS (ESlpos): m/z = 559 [M+H]⁺

### Intermediate 6-126

### N-(3-chloro-2-methoxyphenyl)-6-oxo-8-{[(3-{[(2S)-oxolan-2-yl]methoxy}pyridin-4-yl)methyl]amino}-5-azaspiro[3.5]non-7-ene-7-carbothioamide

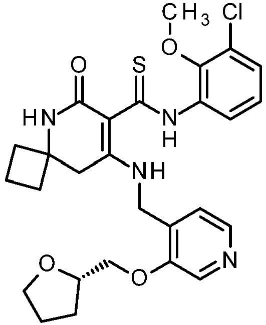

According to the method described for intermediate 6-30 using *N*-(3-chloro-2-methoxyphenyl)-8-hydroxy-6-oxo-5-azaspiro[3.5]non-7-ene-7-carbothioamide (Intermediate 5-123, 400 mg, 1.13 mmol) and 1-(3-{[(2S)-oxolan-2-yl]methoxy}pyridin-4-yl)methanamine (intermediate 2-3, 236 mg, 1.13 mmol) as starting materials, 560 mg (97% purity, 88% yield) of the title compound were prepared after purification by flash chromatography (silica, DCM / EtOH gradient 0-10%).

LC-MS (method 2): Rₜ = 1.26 min; MS (ESlpos): m/z = 543 [M+H]⁺

¹H-NMR (400 MHz, DMSO-d₆) δ [ppm]: 1.154 (0.56), 1.172 (1.21), 1.190 (0.61), 1.578 (0.46), 1.600 (0.42), 1.620 (0.44), 1.751 (0.49), 1.758 (0.41), 1.779 (0.45), 1.794 (0.59), 1.820 (0.42), 1.840 (0.64), 1.858 (0.95), 1.865 (0.68), 1.879 (1.29), 1.894 (0.81), 1.900 (0.78), 1.987 (2.29), 1.992 (0.45), 2.023 (0.40), 2.035 (0.68), 2.040 (0.49), 2.058 (0.96), 2.064 (0.74), 2.082 (0.70), 2.088 (0.73), 2.518 (1.07), 2.522 (0.66), 2.936 (3.05), 3.658 (0.44), 3.679 (0.81), 3.694 (0.85), 3.696 (0.87), 3.709 (16.00), 3.769 (0.66), 3.785 (0.99), 3.802 (0.64), 3.805 (0.68), 4.017 (0.46), 4.034 (0.44), 4.137 (0.46), 4.147 (1.02), 4.162 (1.66), 4.167 (1.29), 4.176 (1.55), 4.202 (0.83), 4.218 (0.60), 4.227 (0.50), 4.233 (0.49), 4.723 (1.67), 4.739 (1.66), 7.090 (1.13), 7.110 (2.40), 7.130 (1.38), 7.284 (1.52), 7.288 (1.59), 7.303 (1.23), 7.308 (1.18), 7.337 (1.41), 7.349 (1.45), 7.833 (1.15), 7.836 (1.15), 7.853 (1.11), 7.856 (1.02), 8.153 (1.82), 8.241 (1.98), 8.253 (1.78), 8.418 (3.02), 13.864 (0.68), 14.853 (1.84).

### Intermediate 6-127

### N-(3-chloro-2-methoxyphenyl)-8-{[(3-{[(2S)-oxan-2-yl]methoxy}pyridin-4-yl)methyl]amino}-6-oxo-5-azaspiro[3.5]non-7-ene-7-carbothioamide

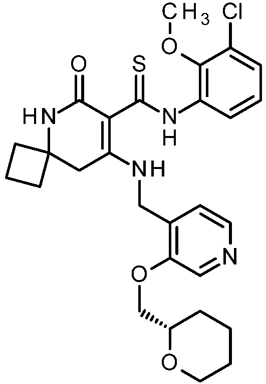

To a solution of *N*-(3-chloro-2-methoxyphenyl)-8-hydroxy-6-oxo-5-azaspiro[3.5]non-7-ene-7-carbothioamide (Intermediate 5-123, 400 mg, 1.13 mmol) and 1-(3-{[(2S)-oxan-2-yl]methoxy}pyridin-4-yl)methanamine (Intermediate 2-127, 252 mg, 1.13 mmol) in acetonitrile under argon atmosphere was added *N,O*-bis-(trimethylsiliyl)-acetamide (692 mg, 3.4 mmol; CAS 10416-59-8). The mixture was stirred over night at 80°C. The reaction mixture was concentrated under reduced pressure to give a residue. The residue was purified by column chromatography (silica, DCM / EtOH gradient, 0-10%) to give 562 mg (94% purity, 84% yield) of the title compound.

LC-MS (method 2): Rₜ = 1.34 min; MS (ESlpos): m/z = 557 [M+H]⁺

¹H-NMR (400 MHz, DMSO-d₆) δ [ppm]: 1.360 (0.45), 1.482 (1.75), 1.581 (0.55), 1.603 (0.52), 1.621 (0.51), 1.674 (0.63), 1.710 (0.50), 1.798 (0.53), 1.856 (0.54), 1.875 (0.91), 1.884 (0.79), 1.896 (0.60), 2.061 (0.89), 2.086 (0.77), 2.518 (2.69), 2.523 (1.94), 2.937 (3.47), 3.351 (0.49), 3.363 (0.44), 3.387 (0.54), 3.681 (0.49), 3.710 (16.00), 3.879 (0.61), 3.909 (0.62), 4.118 (1.41), 4.129 (2.57), 4.144 (1.30), 4.724 (1.82), 4.740 (1.83), 5.760 (1.42), 7.088 (1.09), 7.108 (2.35), 7.129 (1.34), 7.282 (1.52), 7.286 (1.58), 7.303 (1.27), 7.306 (1.22), 7.340 (1.51), 7.351 (1.53), 7.844 (1.24), 7.847 (1.25), 7.864 (1.21), 7.868 (1.13), 8.153 (2.08), 8.236 (2.06), 8.247 (1.91), 8.407 (3.33), 13.844 (0.43), 13.859 (0.80), 13.873 (0.40), 14.857 (2.11).

### Intermediate 6-128

### N-(3-chloro-2-methoxy-phenyl)-6-oxo-8-{[3-(2-tetrahydropyran-2-ylethoxy)-4-pyridyl]methylamino}-5-azaspiro[3.5]non-7-ene-7-carbothioamide

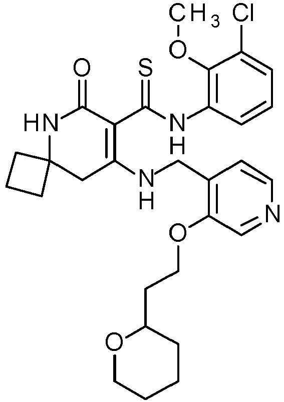

To a solution of *N*-(3-chloro-2-methoxyphenyl)-8-hydroxy-6-oxo-5-azaspiro[3.5]non-7-ene-7-carbothioamide (Intermediate 5-123, 400 mg, 1.13 mmol) and [3-(2-tetrahydropyran-2-ylethoxy)-4-pyridyl]methanamine (Intermediate 2-86, 268 mg, 1.13 mmol) in acetonitrile under argon atmosphere was added *N,O*-bis-(trimethylsiliyl)-acetamide (692 mg, 3.4 mmol; CAS 10416-59-8). The mixture was stirred over night at 80 °C. The reaction mixture was concentrated under reduced pressure to give a residue. The residue was purified by column chromatography (silica, DCM / EtOH gradient, 0-10%) to give 542 mg (95% purity, 80% yield) of the title compound.

LC-MS (method 2): Rₜ = 1.38 min; MS (ESlpos): m/z = 571 [M+H]⁺

¹H-NMR (400 MHz, DMSO-d₆) δ [ppm]: 1.222 (0.45), 1.230 (0.43), 1.421 (1.52), 1.431 (1.60), 1.439 (1.57), 1.567 (0.44), 1.588 (1.06), 1.614 (1.01), 1.646 (0.40), 1.724 (0.55), 1.864 (1.35), 1.883 (1.71), 1.895 (1.14), 1.913 (0.63), 2.066 (0.76), 2.086 (0.68), 2.518 (1.89), 2.523 (1.34), 2.947 (3.25), 3.309 (0.44), 3.355 (0.43), 3.485 (0.43), 3.676 (0.44), 3.706 (16.00), 3.825 (0.63), 3.830 (0.60), 3.855 (0.60), 4.207 (0.50), 4.220 (1.16), 4.235 (1.00), 4.252 (0.47), 4.698 (1.12), 4.711 (1.14), 5.759 (1.20), 7.084 (1.13), 7.104 (2.46), 7.124 (1.40), 7.278 (1.56), 7.282 (1.63), 7.298 (1.33), 7.302 (1.31), 7.853 (1.25), 7.856 (1.25), 7.873 (1.22), 7.877 (1.12), 8.147 (2.03), 13.871 (0.78), 14.863 (2.08).

### Intermediate 6-129

### N-(3-chloro-2-methoxyphenyl)-8-[({3-[(5,5-dimethyl-1,4-dioxan-2-yl]methoxy}pyridin-4-yl)methyl]amino}-6-oxo-5-azaspiro[3.5]non-7-ene-7-carbothioamide

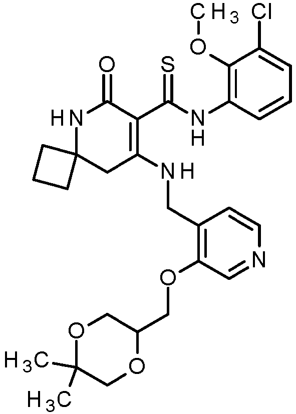

According to the method described for intermediate 6-30 using *N*-(3-chloro-2-methoxyphenyl)-8-hydroxy-6-oxo-5-azaspiro[3.5]non-7-ene-7-carbothioamide (intermediate 5-123, 400 mg, 1.13 mmol) and 1-{3-[(5,5-dimethyl-1,4-dioxan-2-yl)methoxy]pyridin-4-yl}methanamine (intermediate 2-62, 286 mg, 1.13 mmol) as starting materials, 530 mg (95% purity, 76% yield) of the title compound were prepared after reaction for 16h at 80°C and purification by flash chromatography (silica, DCM / EtOH gradient 0-10%).

LC-MS (method 2): Rₜ = 1.16 min; MS (ESlpos): m/z = 587 [M+H]⁺

¹H-NMR (400 MHz, DMSO-d6) δ [ppm]: 1.041 (7.96), 1.154 (0.54), 1.172 (1.14), 1.190 (0.57), 1.254 (7.02), 1.584 (0.42), 1.858 (0.41), 1.881 (0.80), 1.889 (0.59), 1.903 (0.46), 1.987 (1.93), 2.061 (0.77), 2.090 (0.63), 2.518 (0.92), 2.522 (0.57), 2.933 (2.68), 3.301 (0.91), 3.538 (1.58), 3.566 (1.28), 3.643 (0.44), 3.663 (0.99), 3.672 (1.02), 3.687 (1.01), 3.710 (16.00), 3.741 (0.66), 3.816 (0.42), 3.826 (0.50), 4.017 (0.41), 4.035 (0.41), 4.202 (0.96), 4.214 (0.94), 4.221 (1.00), 4.235 (0.92), 4.731 (1.27), 4.746 (1.27), 7.087 (1.00), 7.107 (2.12), 7.128 (1.23), 7.284 (1.38), 7.288 (1.45), 7.303 (1.13), 7.307 (1.10), 7.361 (0.55), 7.371 (0.58), 7.841 (1.07), 7.844 (1.09), 7.861 (1.02), 7.865 (0.97), 8.155 (1.74), 8.425 (0.46), 13.863 (0.65), 14.854 (1.76).

### Intermediate 6-132

### N-(3-chloro-2-methoxyphenyl)-7-[({3-[(5,5-dimethyl-1,4-dioxan-2-yl)methoxy]pyridin-4-yl}methyl]amino}-5-oxo-4-azaspiro[2.5]oct-6-ene-6-carbothioamide

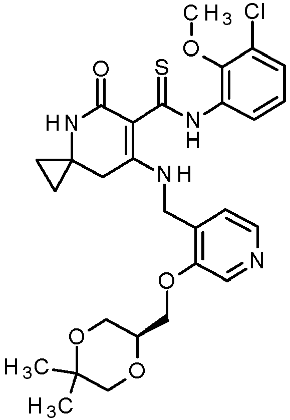

According to the method described for intermediate 6-41 using *N*-(3-chloro-2-methoxyphenyl)-7-hydroxy-5-oxo-4-azaspiro[2.5]oct-6-ene-6-carbothioamide (intermediate 5-111, 197 mg, 581 µmol) and 1-{3-[(5,5-dimethyl-1,4-dioxan-2-yl)methoxy]pyridin-4-yl}methanamine (intermediate 2-62, 191 mg, 756 µmol) as staring materials, 250 mg (95 % purity, 71 % yield) of the title compound were prepared after purification by flash chromatography (silica, DCM / EtOH gradient 0-10%).

LC-MS (method 2): Rₜ = 1.24 min; MS (ESlpos): m/z = 573.4 [M+H]⁺

¹H-NMR (400 MHz, DMSO-d6) δ [ppm]: 0.582 (0.72), 0.599 (2.46), 0.611 (1.13), 0.712 (1.04), 0.724 (2.58), 0.741 (0.80), 1.037 (9.92), 1.066 (0.47), 1.149 (1.01), 1.167 (2.00), 1.185 (0.99), 1.244 (9.16), 1.983 (3.69), 2.788 (3.96), 3.292 (1.25), 3.526 (1.98), 3.554 (1.60), 3.610 (0.42), 3.619 (0.56), 3.640 (1.28), 3.648 (1.38), 3.661 (1.27), 3.685 (1.55), 3.714 (16.00), 3.785 (0.64), 3.795 (0.73), 3.807 (0.60), 3.820 (0.45), 4.013 (0.84), 4.030 (0.87), 4.140 (0.43), 4.151 (0.47), 4.167 (1.33), 4.177 (1.32), 4.186 (1.39), 4.200 (1.26), 4.212 (0.49), 4.226 (0.42), 4.620 (2.09), 4.635 (2.12), 7.089 (1.10), 7.109 (2.36), 7.129 (1.37), 7.285 (3.26), 7.289 (2.39), 7.297 (1.94), 7.305 (1.52), 7.309 (1.43), 7.818 (1.37), 7.821 (1.40), 7.838 (1.33), 7.842 (1.28), 7.928 (2.52), 8.239 (2.24), 8.251 (2.15), 8.385 (3.64), 13.758 (0.50), 13.773 (0.95), 13.787 (0.51), 14.774 (2.38).

### Intermediate 7

### 3-(3-chloro-2-methoxyanilino)-5-[(2,4-dimethoxyphenyl)methyl]-6-methyl-2-(3-{[(2S)-oxolan-2-yl]methoxy}pyridin-4-yl)-1,5,6,7-tetrahydro-4H-pyrrolo[3,2-c]pyridin-4-one

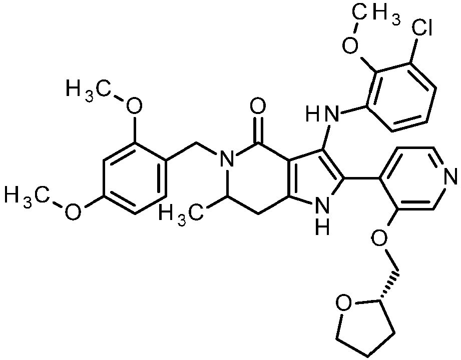

*N*-(3-chloro-2-methoxyphenyl)-1-(2,4-dimethoxybenzyl)-6-methyl-2-oxo-4-[({3-[(2*S*)-tetrahydrofuran-2-ylmethoxy]pyridin-4-yl}methyl)amino]-1,2,5,6-tetrahydropyridine-3-carbothioamide (intermediate 6-3, 825 mg, 1.24 mmol) was dissolved in methanol (5.0 ml), TFA (190 µl, 2.5 mmol) was added an the mixture was stirred fo 5min at RT. mCPBA (554 mg, 77 % purity, 2.47 mmol) was added and the mixture was stirred for 5h at 50°C. The mixture was diluted with half sat. sodium bicarbonate solution, extracted with DCM and the organic layer was dried and evaporated. The residue was purified by flash chromatography (amino phase silica, DCM / EtOH gradient 0-5%) to give 342 mg (44 %yield) of the title compound.

LC-MS (method 2): Rₜ = 1.42 min; MS (ESlpos): m/z = 633 [M+H]⁺

¹H-NMR (400 MHz, DMSO-d₆) δ [ppm]: -0.008 (0.59), 0.000 (15.08), 0.007 (0.51), 1.120 (0.51), 1.136 (0.55), 1.156 (1.98), 1.162 (2.16), 1.174 (5.04), 1.189 (2.15), 1.192 (2.38), 1.690 (0.47), 1.708 (0.41), 1.739 (0.41), 1.848 (0.60), 1.865 (1.04), 1.878 (1.01), 1.895 (0.71), 1.989 (5.55), 2.012 (0.55), 2.029 (0.49), 2.324 (0.48), 2.329 (0.65), 2.333 (0.46), 2.520 (2.70), 2.525 (1.71), 2.666 (0.75), 2.671 (0.74), 2.675 (0.53), 2.696 (0.60), 3.162 (0.50), 3.200 (0.42), 3.295 (0.88), 3.297 (0.88), 3.671 (2.28), 3.677 (0.53), 3.691 (0.88), 3.698 (1.03), 3.734 (9.16), 3.737 (9.27), 3.746 (2.64), 3.755 (1.28), 3.761 (1.21), 3.773 (1.42), 3.778 (1.54), 3.799 (11.86), 3.811 (1.51), 3.822 (1.07), 3.844 (1.00), 3.865 (0.85), 3.875 (6.91), 3.882 (7.24), 3.921 (0.73), 3.932 (0.71), 3.960 (0.76), 3.971 (0.73), 4.002 (0.49), 4.019 (1.36), 4.037 (1.77), 4.055 (1.07), 4.078 (0.45), 4.297 (0.45), 4.313 (1.18), 4.334 (0.80), 4.342 (0.68), 4.358 (0.42), 4.378 (0.66), 4.403 (0.49), 4.910 (0.79), 4.923 (0.72), 4.950 (0.73), 4.962 (0.66), 5.761 (16.00), 6.155 (0.63), 6.162 (0.63), 6.171 (1.14), 6.179 (0.96), 6.187 (0.67), 6.196 (0.65), 6.457 (0.60), 6.464 (1.06), 6.471 (0.78), 6.478 (0.75), 6.485 (1.13), 6.492 (0.80), 6.560 (1.42), 6.564 (2.18), 6.569 (1.37), 6.670 (2.71), 6.678 (1.49), 6.682 (2.94), 6.686 (1.43), 6.691 (1.42), 6.697 (1.13), 7.059 (0.97), 7.068 (1.03), 7.080 (0.94), 7.089 (0.96), 7.264 (1.21), 7.277 (1.35), 7.283 (1.23), 7.296 (1.17), 7.552 (1.57), 7.595 (1.58), 8.021 (1.47), 8.031 (1.74), 8.033 (1.64), 8.043 (1.36), 8.425 (2.34), 8.430 (2.40), 11.240 (0.94), 11.295 (0.97).

### Intermediate 8

### 5-[(2,4-dimethoxyphenyl)methyl]-3-(3-fluoro-2-methoxyanilino)-6-methyl-2-(3-{[(2S)-oxolan-2-yl]methoxy}pyridin-4-yl)-1,5,6,7-tetrahydro-4H-pyrrolo[3,2-c]pyridin-4-one

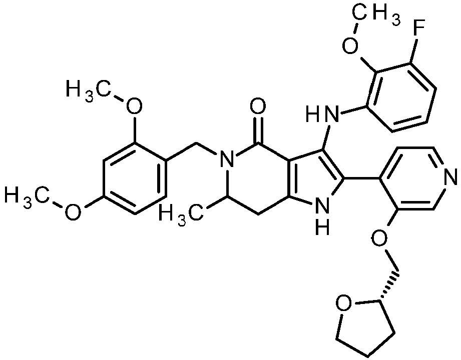

According to the method described for intermediate 3 using 1-[(2,4-dimethoxyphenyl)methyl]-*N*-(3-fluoro-2-methoxyphenyl)-6-methyl-2-oxo-4-{[(3-{[(2S)-oxolan-2-yl]methoxy}pyridin-4-yl)methyl]amino}-1,2,5,6-tetrahydropyridine-3-carbothioamide (intermediate 6-8, 540 mg, 830 µmol) as starting material, 224 mg (43% yield) of the title compound were prepared.

LC-MS (method 2): Rₜ = 1.37 min; MS (ESlpos): m/z = 617 [M+H]⁺

### Intermediate 11-1

### tert-butyl (2S)-2-[({4-[(6R)-3-(3-chloro-2-methoxyanilino)-6-methyl-4-oxo-4,5,6,7-tetrahydro-1H-pyrrolo[3,2-c]pyridin-2-yl]pyridin-3-yl}oxy)methyl]morpholine-4-carboxylate

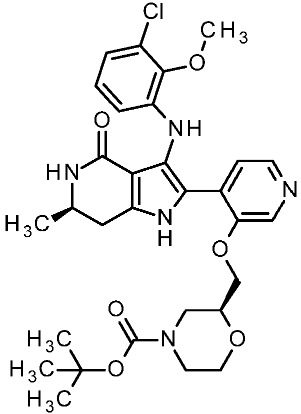

Tert-butyl (2*S*)-2-[({4-[({(2*R*)-5-[(3-chloro-2-methoxyphenyl)carbamothioyl]-2-methyl-6-oxo-1,2,3,6-tetrahydropyridin-4-yl}amino)methyl]pyridin-3-yl}oxy)methyl]morpholine-4-carboxylate (intermediate 6-11, 192 mg, 304 µmol) was dissolved in methanol (3.0 ml), TFA (24 µl, 305 µmol) and hydrogen peroxide (53 µl, 35% purity, 607 µmol) were added and the mixture was stirred for 15 h at 50°C. The mixture was quenched with sat. sodium bisulfit solution, concentrated under vacuum and purified by flash chromatography (silica, DCM / EtOH gradient 0-10%) to give 64.3 mg 117 mg (65% purity, 42% yield) of the title compound.

LC-MS (method 2): Rₜ = 1.25 min; MS (ESlpos): m/z = 598 [M+H]⁺

### Intermediate 11-2

### (6R)-3-(3-chloro-2-methoxyanilino)-6-methyl-2-(3-{[(2S)-morpholin-2-yl]methoxy}pyridin-4-yl)-1,5,6,7-tetrahydro-4H-pyrrolo[3,2-c]pyridin-4-one

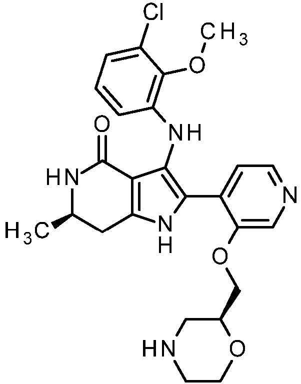

According to the method described for intermediate 5-1 using *tert-*butyl (2*S*)-2-[({4-[(6*R*)-3-(3-chloro-2-methoxyanilino)-6-methyl-4-oxo-4,5,6,7-tetrahydro-1*H*-pyrrolo[3,2-c]pyridin-2-yl]pyridin-3-yl}oxy)methyl]morpholine-4-carboxylate (intermediate 11-1, 117 mg, 196 µmol) as starting material, 67.0 mg (75% purity, 52% yield) of the title compound were prepared after purification by flash chromatography (amino phase silica, DCM / EtOH gradient 0-10%).

LC-MS (method 2): Rₜ = 0.96 min; MS (ESlpos): m/z = 498 [M+H]⁺

¹H-NMR (400 MHz, DMSO-d₆) δ [ppm]: -0.008 (0.50), 0.000 (16.00), 0.008 (0.48), 0.800 (0.59), 0.817 (0.61), 0.824 (0.65), 0.888 (0.50), 0.907 (0.92), 0.925 (0.43), 1.073 (0.52), 1.131 (0.70), 1.147 (0.74), 1.157 (3.09), 1.161 (0.77), 1.175 (6.21), 1.192 (3.19), 1.203 (0.48), 1.220 (0.65), 1.230 (3.48), 1.246 (3.28), 1.907 (0.98), 1.989 (10.21), 2.329 (0.50), 2.520 (1.74), 2.525 (1.15), 2.532 (0.67), 2.557 (0.69), 2.562 (0.72), 2.576 (0.56), 2.587 (0.74), 2.602 (0.51), 2.616 (0.70), 2.642 (0.63), 2.662 (0.42), 2.666 (0.54), 2.671 (0.68), 2.676 (0.53), 2.681 (0.41), 2.686 (0.46), 2.694 (0.45), 2.712 (0.57), 2.720 (0.54), 2.732 (0.71), 2.849 (0.56), 2.854 (0.60), 2.886 (0.94), 2.898 (0.60), 2.926 (0.49), 2.939 (0.45), 3.554 (0.41), 3.573 (0.55), 3.581 (0.55), 3.662 (1.81), 3.676 (1.40), 3.780 (0.41), 3.854 (0.46), 3.863 (0.53), 3.885 (11.08), 3.908 (0.46), 3.913 (0.48), 3.921 (0.44), 4.002 (0.80), 4.019 (2.41), 4.037 (2.38), 4.055 (0.94), 4.112 (0.67), 4.129 (0.55), 4.138 (0.70), 4.155 (0.60), 4.284 (0.65), 4.293 (0.69), 4.310 (0.55), 4.319 (0.50), 6.147 (0.94), 6.156 (0.82), 6.162 (0.98), 6.171 (0.96), 6.674 (0.41), 6.685 (4.07), 6.695 (1.93), 6.700 (1.63), 7.101 (0.64), 7.115 (0.55), 7.123 (1.35), 7.275 (1.77), 7.288 (1.75), 7.503 (2.29), 8.014 (2.39), 8.026 (2.18), 8.090 (0.49), 8.230 (0.48), 8.403 (3.09), 11.101 (1.12).

### Intermediate 55

### (5aR*,8aS*)-3-(3-chloro-2-methoxyanilino)-5-(2,4-dimethoxybenzyl)-2-{3-[(2S)-tetrahydrofuran-2-ylmethoxy]pyridin-4-yl}-5,5a,6,7,8,8a-hexahydrocyclopenta[b]pyrrolo[2,3-d]pyridin-4(1H)-one

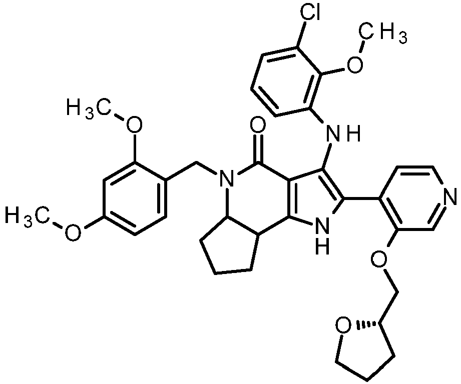

To a solution of (4a*R**,7a*S**)-*N*-(3-chloro-2-methoxyphenyl)-1-(2,4-dimethoxybenzyl)-2-oxo-4-[({3-[(2*S*)-tetrahydrofuran-2-ylmethoxy]pyridin-4-yl}methyl)amino]-2,4a,5,6,7,7a-hexahydro-1H-cyclopenta[b]pyridine-3-carbothioamide (400 mg, 576 umol, 1.00 eq) in MeOH (2 mL) was added H₂O₂ (0.26 g, 2.60 mmol, 4.50 eq). The mixture was stirred at 80 °C for 4 h. The reaction mixture was quenched by addition saturated NaHSO₃ 1 mL at 25 °C, and then diluted with water 100 mL and extracted with dichloromethane 150 mL (50 mL x 3). The combined organic layers were washed with brine 100 mL, dried over Na₂SO₄, filtered and concentrated under reduced pressure to give a residue. The residue was purified by flash silica gel chromatography (ISCO^{®}; 20 g SepaFlash^{®} Silica Flash Column, Eluent of 0~100% Ethyl acetate/Petroleum ether gradient @ 100 mL/min), TLC (Petroleum ether/Ethyl acetate = 0/1 Product R_{f} = 0.82) to give the title compound (350 mg, 530 umol, 92.0% yield) as yellow solid.

LC-MS (Method: MS instrument type: SHIMADZU LCMS-2020, Column: Kinetex EVO C18 2.1 x 30mm, 5um, mobile phase A: 0.025% NH₃•H₂O in Water (v/v) , B: Acetonitrile, gradient: 0.0 min 5% B→0.8 min 95% B→1.2 min 95% B→1.21 min 5% B→1.55 min 5%B, flow rate: 1.5 mL/min, oven temperature: 40°C; PDA detection: 220 nm & 254 nm): Rₜ = 1.249 min; MS (ESlpos): m/z = 659.3 [M+H]⁺

### Intermediate 64-1

### tert-butyl (2S)-2-[({4-[(5aR,8aS)-3-(3-chloro-2-methoxyanilino)-4-oxo-1,4,5,5a,6,7,8,8a-octahydrocyclopenta[b]pyrrolo[2,3-d]pyridin-2-yl]pyridin-3-yl}oxy)methyl]morpholine-4-carboxylate

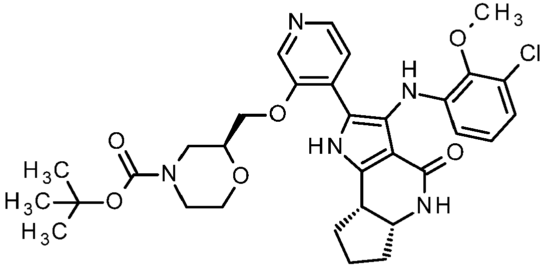

*Tert*-butyl (2*S*)-2-[({4-[({(4a*S*,7a*R*)-3-[(3-chloro-2-methoxyphenyl)carbamothioyl]-2-oxo-2,4a,5,6,7,7a-hexahydro-1*H*-cyclopenta[b]pyridin-4-yl}amino)methyl]pyridin-3-yl}oxy)methyl]morpholine-4-carboxylate (intermediate 6-64, 144 mg, 0.22 mmol)was dissolved in EtOH (7.3 ml). Palladium on carbon (233 mg, 10%, 0.22 mmol) and TFA (26 µl, 330 µmol; CAS 76-05-1) was added and the mixture was stirred at 90°C for 4 h. The reaction mixture was filtrated over celite and washed with MeOH. The filtrate was concentrated under reduced pressure and was purified by flash chromatography (silica, DCM / EtOH gradient 0-10 %) to give 57.0 mg (90% purity, 38% yield) of the title compound.

LC-MS (method 2): Rₜ = 1.27 min; MS (ESlpos): m/z = 624 [M+H]⁺

¹H-NMR (400 MHz, DMSO-d₆) δ [ppm]: 1.40 (s, 9H), 1.68 - 1.95 (m, 5H), 2.01 - 2.15 (m, 1H), 2.80 - 3.02 (m, 2H), 3.21 - 3.30 (m, 1H), 3.48 - 3.56 (td, 1H), 3.66 - 3.78 (m, 2H), 3.80 - 3.84 (m, 1H), 3.86 - 3.89 (m, 3H), 3.90 - 3.96 (m, 2H), 4.03 - 4.08 (m, 1H), 4.17 - 4.27 (m, 1H), 4.29 - 4.34 (dd, 1H), 6.07 - 6.19 (t, 1H), 6.57 - 6.75 (d, 2H), 6.98 - 7.10 (s, 1H), 7.15 - 7.31 (d, 1H), 7.54 (s, 1H), 8.02 (d, 1H), 8.41 (s, 1H), 10.82 - 11.01 (br s, 1H).

### Intermediate 64-2

### (5aR,8aS)-3-(3-chloro-2-methoxyanilino)-2-{3-[(2S)-morpholin-2-ylmethoxy]pyridin-4-yl}-5,5a,6,7,8,8a-hexahydrocyclopenta[b]pyrrolo[2,3-d]pyridin-4(1H)-one

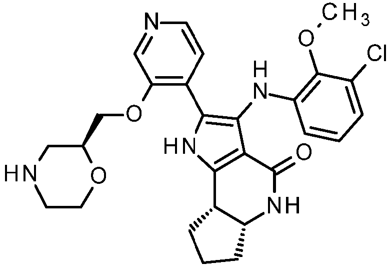

To *tert-*butyl (2*S*)-2-[({4-[(5a*R*,8a*S*)-3-(3-chloro-2-methoxyanilino)-4-oxo-1,4,5,5a,6,7,8,8a-octahydrocyclopenta[b]pyrrolo[2,3-d]pyridin-2-yl]pyridin-3-yl}oxy)methyl]morpholine-4-carboxylate (intermediate 64-1, 57 mg, 91 µmol) in DCM (0.5 ml) was added TFA (70 µl, 0.91 mmol) and the solution was stirred for 2h. The addition of TFA (70 µl, 0.91 mmol) was repeated and stirring was continued for 5h. The reaction mixture was concentrated under vacuum and the residue was purified by flash chromatography (amino phase silica, DCM / EtOH gradient 0-10%) to give 41.6 mg (90% purity, 48% yield) of the title compound were prepared.

LC-MS (method 2): Rₜ = 0.98 min; MS (ESlpos): m/z = 524.3 [M+H]⁺

¹H-NMR (400 MHz, DMSO-d₆) δ [ppm]: 1.62 - 1.82 (m, 5H), 1.82 - 1.95 (m, 1H), 2.00 - 2.18 (m, 2H), 2.54 - 2.61 (m, 1H), 2.67 (s, 4H), 2.82 - 2.95 (m, 1H), 3.19 - 3.28 (m, 1H), 3.54 - 3.62 (m, 1H), 3.81 - 3.87 (m, 2H), 3.88 (s, 3H), 4.03 - 4.11 (m, 1H), 4.11 - 4.19 (dd, 1H), 4.22 - 4.31 (dd, 1H), 6.14 (t, 1H), 6.67 - 6.70 (m, 2H), 7.04 - 7.15 (m, 1H), 7.25 (d, 1H), 7.58 (s, 1H), 8.01 (d, 1H), 8.40 (s, 1H), 10.94 (s, 1H).

### Intermediate 89-1

### tert-butyl (3S)-3-[({4-[3-(3-chloro-2-methoxyanilino)-7-(2-fluoroethyl)-4-oxo-4,5,6,7-tetrahydro-1H-pyrrolo[3,2-c]pyridin-2-yl]pyridin-3-yl}oxy)methyl]morpholine-4-carboxylate

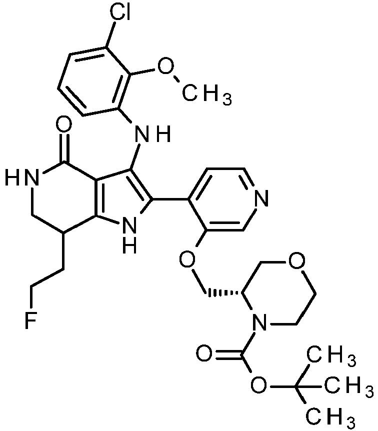

*Tert*-butyl (3*S*)-3-({[4-({5-[(3-chloro-2-methoxyphenyl)carbamothioyl]-3-(2-fluoroethyl)-6-oxo-1,2,3,6-tetrahydropyridin-4-yl}amino)methyl]pyridin-3-yl]oxy}methyl)morpholine-4-carboxylate (intermediate 6-89, 587 mg, 884 µmol) was suspended in methanol (9.0 ml). TFA (68 µl, 880 µmol and hydrogen peroxide (150 µl, 35% purity, 1.8 mmol) were added and the reaction micxture was heated for 17h to 50°C. The reaction mixture was carefully quenched with saturated sodium bisulfit solution and sodium bicarbonate solution and stirred for 15min. To the suspension was added solid NaCL and extracted with DCM. The combined organic phases were dried, concentrated and purified by flash chromatography (silica, DCM / EtOH gradient 0-20 %) to give 43.0 mg (85% purity, 7% yield) of the title compound.

LC-MS (method 2): Rₜ = 1.23 min; MS (ESlpos): m/z = 630.4 [M+H]⁺

### Intermediate 89-2

### 3-(3-chloro-2-methoxyanilino)-7-(2-fluoroethyl)-2-(3-{[(3S)-morpholin-3-yl]methoxy}pyridin-4-yl)-1,5,6,7-tetrahydro-4H-pyrrolo[3,2-c]pyridin-4-one

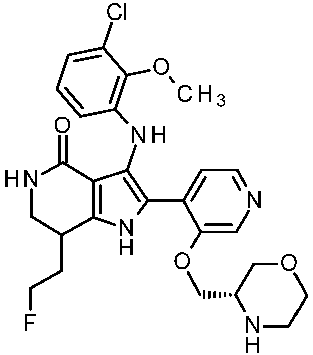

According to the method described for intermediate 5-1 using *tert-*butyl (3S)-3-[({4-[3-(3-chloro-2-methoxyanilino)-7-(2-fluoroethyl)-4-oxo-4,5,6,7-tetrahydro-1*H*-pyrrolo[3,2-c]pyridin-2-yl]pyridin-3-yl}oxy)methyl]morpholine-4-carboxylate (intermediate 89-1, 42.0 mg, 66.7 µmol) as starting material, 35 mg (85% purity, 84% yield) of the title compound were prepared after coevaporation with toluene.

LC-MS (method 2): Rₜ = 0.97 min; MS (ESlpos): m/z = 530.3 [M+H]⁺

¹H-NMR (400 MHz, DMSO-d₆): δ [ppm]= 1.19 - 1.33 (m, 7H), 2.27 - 2.40 (m, 5H), 3.57 - 4.10 (m, 22H), 3.85 - 3.90 (m, 4H), 4.20 - 4.74 (m, 7H), 6.05 - 6.16 (m, 1H), 6.60 - 6.70 (m, 2H), 6.88 - 7.32 (m, 1H), 7.10 - 7.31 (m, 7H), 7.12 - 7.28 (m, 1H), 7.37 - 7.45 (m, 1H), 7.59 - 7.72 (m, 1H), 8.16 - 8.22 (m, 1H), 8.50 (s, 1H), 9.04 - 9.45 (m, 2H), 11.12 - 11.23 (m, 1H).

### Intermediate 91-1

### tert-butyl (2S)-2-[({4-[(6R)-3-(3-chloro-2-ethylanilino)-6-methyl-4-oxo-4,5,6,7-tetrahydro-1H-pyrrolo[3,2-c]pyridin-2-yl]pyridin-3-yl}oxy)methyl]morpholine-4-carboxylate

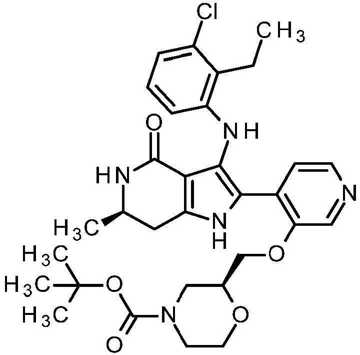

*Tert*-butyl (2*S*)-2-[({4-[({(2*R*)-5-[(3-chloro-2-ethylphenyl)carbamothioyl]-2-methyl-6-oxo-1,2,3,6-tetrahydropyridin-4-yl}amino)methyl]pyridin-3-yl}oxy)methyl]morpholine-4-carboxylate (intermediate 6-91, 170 mg, 270 µmol) was dissolved in acetic acid (2.8 ml, 49 mmol) and hydrogen peroxide (55 µl, 30% purity, 540 µmol) was added and the mixture was stirred for 4 h at 60°C. Saturated sodium bisulfit solution was added after cooling, the mixture was evaporated and purified by flash chromatography (silica, DCM / EtOH gradient 0-10 %) to give 49.2 mg (65% purity, 20% yield) of the title compound.

LC-MS (method 2): Rₜ = 1.34 min; MS (ESlpos): m/z = 596 [M+H]⁺

¹H-NMR (400 MHz, DMSO-d₆) δ [ppm]: 1.035 (0.94), 1.053 (1.97), 1.071 (0.85), 1.200 (1.27), 1.218 (2.74), 1.232 (3.37), 1.248 (2.68), 1.374 (0.84), 1.414 (16.00), 1.907 (2.67), 2.201 (0.78), 2.518 (1.70), 2.523 (1.22), 2.642 (0.40), 2.673 (0.44), 2.815 (0.44), 2.833 (0.93), 2.852 (0.92), 2.919 (0.44), 2.929 (0.43), 3.530 (0.42), 3.537 (0.44), 3.765 (0.52), 3.794 (0.68), 3.807 (0.51), 3.822 (0.45), 3.950 (0.41), 4.247 (0.48), 4.257 (0.48), 6.212 (0.68), 6.216 (0.60), 6.231 (0.61), 6.235 (0.66), 6.698 (0.48), 6.713 (1.34), 6.717 (1.22), 6.723 (0.95), 6.742 (0.83), 7.113 (0.62), 7.119 (0.95), 7.188 (0.67), 7.200 (0.68), 7.976 (1.07), 7.989 (0.99), 8.369 (1.55), 10.984 (0.66).

### Intermediate 91-2

### (6R)-3-(3-chloro-2-ethylanilino)-6-methyl-2-(3-{[(2S)-morpholin-2-yl]methoxy}pyridin-4-yl)-1,5,6,7-tetrahydro-4H-pyrrolo[3,2-c]pyridin-4-one

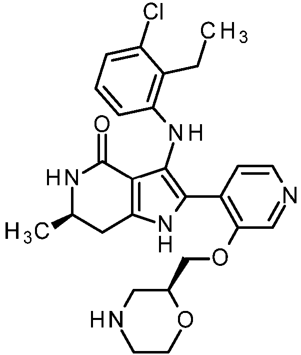

According to the method described for intermediate 5-1 using tert-butyl (2S)-2-[({4-[(6R)-3-(3-chloro-2-ethylanilino)-6-methyl-4-oxo-4,5,6,7-tetrahydro-1*H*-pyrrolo[3,2-c]pyridin-2-yl]pyridin-3-yl}oxy)methyl]morpholine-4-carboxylate (intermediate 91-1, 49.0 mg, 82.2 µmol) as starting material, 35 mg (45% purity, 41% yield) of the title compound were prepared after purification by flash chromatography (amino phase silica, DCM / EtOH gradient 0-10%).

LC-MS (method 2): Rₜ = 1.06 min; MS (ESlpos): m/z = 496.3 [M+H]⁺

### Intermediate 106-1

### tert-butyl (2R)-2-[({4-[(6R)-3-(3-chloro-2-methylanilino)-6-methyl-4-oxo-4,5,6,7-tetrahydro-1H-pyrrolo[3,2-c]pyridin-2-yl]pyridin-3-yl}oxy)methyl]morpholine-4-carboxylate

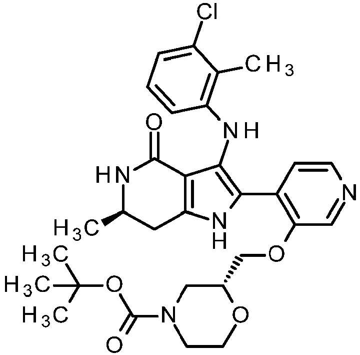

According to the method described for intermediate 64-1 using tert-butyl (2*R*)-2-[({4-[({(2*R*)-5-[(3-chloro-2-methylphenyl)carbamothioyl]-2-methyl-6-oxo-1,2,3,6-tetrahydropyridin-4-yl}amino)methyl]pyridin-3-yl}oxy)methyl]morpholine-4-carboxylate (intermediate 6-106, 200 mg, 325 µmol) as starting material, 92,7 mg (60% purity, 30% yield) of the title compound were prepared after purification by flash chromatography (silica, DCM / EtOH gradient 0-10%).

LC-MS (method 2): Rₜ = 1.26 min; MS (ESlpos): m/z = 582 [M+H]⁺

### Intermediate 106-2

### (6R)-3-(3-chloro-2-methylanilino)-6-methyl-2-(3-{[(2R)-morpholin-2-yl]methoxy}pyridin-4-yl)-1,5,6,7-tetrahydro-4H-pyrrolo[3,2-c]pyridin-4-one

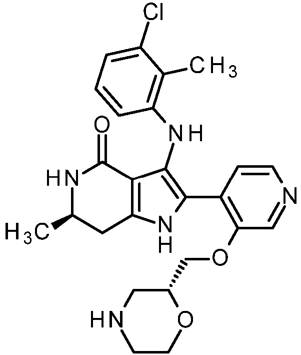

To *tert-*butyl (2*R*)-2-[({4-[(6*R*)-3-(3-chloro-2-methylanilino)-6-methyl-4-oxo-4,5,6,7-tetrahydro-1H-pyrrolo[3,2-c]pyridin-2-yl]pyridin-3-yl}oxy)methyl]morpholine-4-carboxylate (92.7 mg, 165 µmol) in DCM (1.0 ml) was added TFA (120 µl, 1.5 mmol) and the solution was stirred for 1h. The addition of TFA (130 µl, 1.6 mmol) was repeated and stirring was continued for 1h. The reaction mixture was concentrated under vacuum and the residue was purified by flash chromatography (amino phase silica, DCM / EtOH gradient 0-10%) to give 57 mg (60% yield) of the title compound.

LC-MS (method 2): Rₜ = 0.99 min; MS (ESlpos): m/z = 482.3 [M+H]⁺

¹H-NMR (400 MHz, DMSO-d₆) δ [ppm]: 1.25 (d, 3H), 2.36 (s, 3H), 2.55 (dd, 1H), 2.61 (dd, 1H) 2.65 - 2.93 (m, 4H), 3.39 - 3.45 (m, 1H), 3.62 (dt, 1H), 3.73 - 3.93 (m, 3H), 4.06 (dd, 1H), 4.32 (dd, 1H), 6.17 - 6.23 (m, 1H), 6.70 - 6.80 (m, 2H), 7.15 (s, 1H), 7.22 (d, 1H), 7.35 (s, 1H), 7.98 (d, 1H), 8.36 (s, 1H), 11.07 (s, 1H).

### Intermediate 107-1

### tert-butyl (2S)-2-[({4-[(6R)-3-(3-chloro-2-methylanilino)-6-methyl-4-oxo-4,5,6,7-tetrahydro-1H-pyrrolo[3,2-c]pyridin-2-yl]pyridin-3-yl}oxy)methyl]morpholine-4-carboxylate

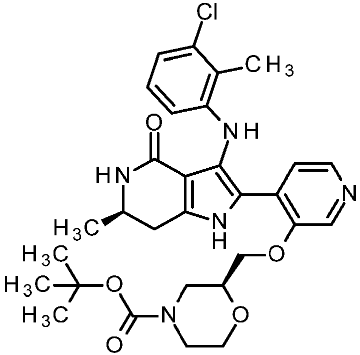

According to the method described for intermediate 64-1 using tert-butyl (2*S*)-2-[({4-[({(2*R*)-5-[(3-chloro-2-methylphenyl)carbamothioyl]-2-methyl-6-oxo-1,2,3,6-tetrahydropyridin-4-yl}amino)methyl]pyridin-3-yl}oxy)methyl]morpholine-4-carboxylate (intermediate 6-107, 200 mg, 325 µmol) as starting material, 96.0 mg (75% purity, 38% yield) of the title compound were prepared after purification by flash chromatography (silica, DCM / EtOH gradient 0-10%).

LC-MS (method 2): Rₜ = 1.26 min; MS (ESlpos): m/z = 582 [M+H]⁺

### Intermediate 107-2

### (6R)-3-(3-chloro-2-methylanilino)-6-methyl-2-(3-{[(2S)-morpholin-2-yl]methoxy}pyridin-4-yl)-1,5,6,7-tetrahydro-4H-pyrrolo[3,2-c]pyridin-4-one

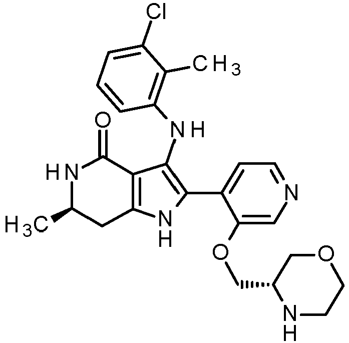

To *tert-*butyl (2*S*)-2-[({4-[(6*R*)-3-(3-chloro-2-methylanilino)-6-methyl-4-oxo-4,5,6,7-tetrahydro-1H-pyrrolo[3,2-c]pyridin-2-yl]pyridin-3-yl}oxy)methyl]morpholine-4-carboxylate (96.0 mg, 165 µmol) in DCM (1.0 ml) was added TFA (130 µl, 1.6 mmol) and the solution was stirred for 1h. The addition of TFA (130 µl, 1.6 mmol) was repeated and stirring was continued for 1h. The reaction mixture was concentrated under vacuum and the residue was purified by flash chromatography (amino phase silica, DCM / EtOH gradient 0-10%) to give 59 mg (80% purity, 59% yield) of the title compound were prepared.

LC-MS (method 2): Rₜ = 0.99 min; MS (ESlpos): m/z = 482.3 [M+H]⁺

¹H-NMR (400 MHz, DMSO-d₆): δ [ppm]: -1.24 (d, 3H), 2.36 (s, 3H), 2.55 - 2.77 (m, 5H), 2.85 (dd, 1H), 2.92 (dd, 1H), 3.58 (td, 1H), 3.75 - 3.93 (m, 3H), 4.10 (dd, 1H), 4.24 (dd, 1H), 6.18 - 6.25 (m, 1H), 6.71 - 6.80 (m, 2H), 7.14 (s, 1H), 7.24 (d, 1H), 7.33 (s, 1H), 7.98 (d, 1H), 8.37 (s, 1H), 11.08 (s, 1H).

### Intermediate 112-1

### tert-butyl (2S)-2-[({4-[3'-(3-chloro-2-methoxyanilino)-4'-oxo-1',4',5',7'-tetrahydrospiro[cyclopropane-1,6'-pyrrolo[3,2-c]pyridin]-2'-yl]pyridin-3-yl}oxy)methyl]morpholine-4-carboxylate

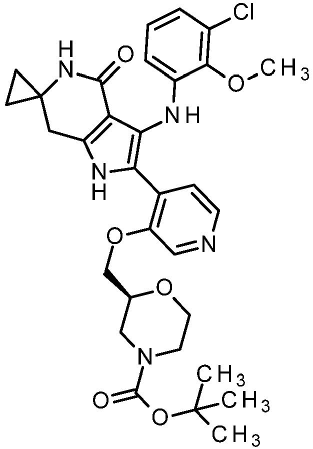

According to the method described for intermediate 89-1 using *tert-*butyl (2*S*)-2-[({4-[({6-[(3-chloro-2-methoxyphenyl)carbamothioyl]-5-oxo-4-azaspiro[2.5]oct-6-en-7-yl}amino)methyl]pyridin-3-yl}oxy)methyl]morpholine-4-carboxylate (intermediate 6-112, 301 mg, 467 µmol) as starting material, 105 mg (33% yield) of the title compound were prepared after purification by preparative HPLC (method 10, gradient: 0.00-0.50 min 15% B, 0.50-6.00 min 15-55% B).

LC-MS (method 2): Rₜ = 1.27 min; MS (ESlpos): m/z = 610.4 [M+H]⁺

¹H-NMR (400 MHz, DMSO-d₆) δ [ppm]: 0.67 - 079 (m, 4H), 1.41 (m, 9H) 2.80 - 2.93 (m, 3H), 3.54 (dt, 1H), 3.53 (td, 1H), 3.76 (d, 2H), 3.84 - 3.98 (m, 3H), 3.88 (s, 3H), 4.12 - 4.18 (m, 1H), 4.32 - 4.37 (m, 1H), 6.12 - 6.17 (m, 1H), 6.66 - 6.71 (m, 2H), 7.26 (s, 1H), 7.28 (d, 1H), 7.52 (s, 1H), 8.04 (d, 1H), 8.40 (s, 1H), 11.03 (s, 1H).

### Intermediate 112-2

### 3'-(3-chloro-2-methoxyanilino)-2'-(3-{[(2S)-morpholin-2-yl]methoxy}pyridin-4-yl)-1',7'-dihydrospiro[cyclopropane-1,6'-pyrrolo[3,2-c]pyridin]-4'(5'H)-one

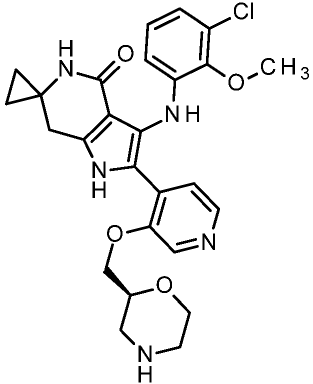

According to the method described for intermediate 5-1 using tert-butyl (2S)-2-[({4-[3'-(3-chloro-2-methoxyanilino)-4'-oxo-1',4',5',7'-tetrahydrospiro[cyclopropane-1,6'-pyrrolo[3,2-c]pyridin]-2'-yl]pyridin-3-yl}oxy)methyl]morpholine-4-carboxylate (105 mg, 171 µmol), 44.8 mg (90% purity, 46% yield) of the title compound were prepared after purification by flash chromatography (amino phase silica, DCM / EtOH gradient 1-10%).

LC-MS (method 2): Rₜ = 0.98 min; MS (ESlpos): m/z = 510.3 [M+H]⁺

¹H-NMR (400 MHz, DMSO-d₆) δ [ppm]: 0.59 - 0.82 (m, 4H), 2.52 - 2.58 (m, 1H), 2.52 - 2.93 (m, 6H), 3.57 (dt, 1H), 3.80 - 3.93 (m, 2H), 3.89 (s, 3H) 4.11 (dd, 1H), 4.34 (dd, 1H), 6.10 - 6.25 (m, 1H), 6.63 - 6.74 (m, 2H), 7.26 - 7.33 (m, 2H), 7.55 (s, 1H), 8.02 (d, 1H), 8.41 (s, 1H), 11.13 (br. s, 1H).

### Intermediate 115-1

### tert-butyl (3S)-3-[({4-[3-(3-chloro-2-methoxyanilino)-7-(2-hydroxyethyl)-4-oxo-4,5,6,7-tetrahydro-1H-pyrrolo[3,2-c]pyridin-2-yl]pyridin-3-yl}oxy)methyl]morpholine-4-carboxylate

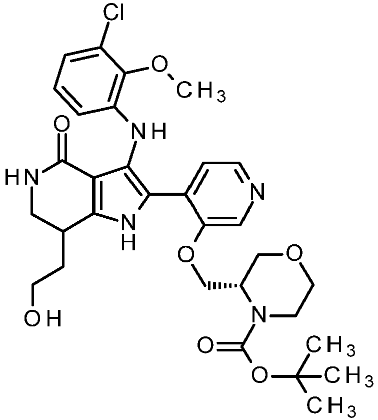

According to the method described for intermediate 89-1 using *tert-*butyl (3*S*)-3-({4-[({5-[(3-chloro-2-methoxy-phenyl)carbamothioyl]-3-(2-hydroxyethyl)-6-oxo-2,3-dihydro-1*H-*pyridin-4-yl}amino)methyl]-3-pyridyl}oxymethyl)morpholine-4-carboxylate (intermediate 6-115, 730 mg, 1.10 mmol) as starting material, 310 mg (95% purity, 43% yield) of the title compound were prepared after purification by flash chromatography (silica, DCM / EtOH gradient 1-20%).

LC-MS (method 2): Rₜ = 1.13 min; MS (ESlpos): m/z = 628.3 [M+H]⁺

¹H-NMR (400 MHz, DMSO-d₆) δ [ppm]: 1.27 (s, 4.5H), 1.30 (s, 4.5H), 1.61 - 1.77 (m, 1H), 1.82 - 1.95 (m, 1H), 2.97 - 3.25 (m, 2H), 3.36 (dd, 1H), 3.48 - 3.70 (m, 4H), 3.78 - 3.89 (m, 1H), 3.86 (s, 1.5H), 3.88 (s, 1.5H), 3.92 - 4.04 (m, 1H), 4.18 - 4.55 (m, 3H), 4.86 - 5.00 (m, 1H), 6.04 - 6.20 (m, 1H), 6.56 - 6.74 (m, 2H), 7.07 - 7.16 (m, 1H), 7.31 (d, 0.5H), 7.34 (d, 1H), 7.48 (s, 1H), 7.97 - 8.08 (m, 1H), 8.42 - 8.59 (m, 1H), 11.15 (s, 0.5H), 11.19 (br. S, 0.5H).

### Intermediate 115-2

### 3-(3-chloro-2-methoxyanilino)-7-(2-hydroxyethyl)-2-(3-{[(3S)-morpholin-3-yl]methoxy}pyridin-4-yl)-1,5,6,7-tetrahydro-4H-pyrrolo[3,2-c]pyridin-4-one

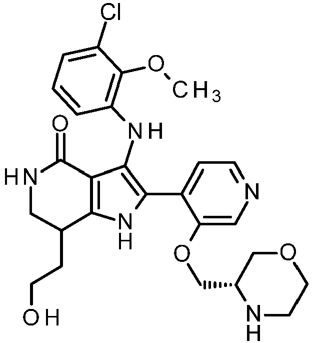

The title compound can be prepared in analogy to intermediate 5-1 from *tert-*butyl (3S)-3-[({4-[3-(3-chloro-2-methoxyanilino)-7-(2-hydroxyethyl)-4-oxo-4,5,6,7-tetrahydro-1*H-*pyrrolo[3,2-c]pyridin-2-yl]pyridin-3-yl}oxy)methyl]morpholine-4-carboxylate (Intermediate 115-1) by treatment with TFA.

LC-MS (method 2): Rₜ = 0.90 min; MS (ESlpos): m/z = 528.4 [M+H]⁺

¹H-NMR (400 MHz, DMSO-d₆): δ [ppm]= 1.67 - 1.84 (m, 2H), 2.77 - 2.88 (m, 1H), 2.95 - 3.10 (m, 2H), 3.11 - 3.30 (m, 2H), 3.38 - 3.47 (m, 2H), 3.48 - 3.58 (m, 3H), 3.65 - 3.81 (m, 3H), 3.89 (s, 3H), 4.07 -4.34 (m, 2H) 6.10 - 6.19 (m, 1H), 6.67 - 6.72 (m, 2H), 7.12 (br s, 1H), 7.27(dd, 1H), 7.61 (d, 1H), 7.99 (dd, 1H), 8.39 (d, 1H), 12.15 (br. S, 1H).

### Examples

### Example 1

### (6R)-3-(3-chloro-2-methoxyanilino)-2-[3-(2-methoxy-2-methylpropoxy)pyridin-4-yl]-6-methyl-1,5,6,7-tetrahydro-4H-pyrrolo[3,2-c]pyridin-4-one

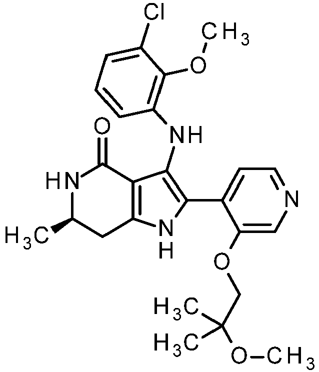

(6*R*)*-*N-(3-chloro-2-methoxyphenyl)-4-({[3-(2-methoxy-2-methylpropoxy)pyridin-4-yl]methyl}amino)-6-methyl-2-oxo-1,2,5,6-tetrahydropyridine-3-carbothioamide (intermediate 6-1, 226 mg, 435 µmol) was dissolved in methanol (2.5 ml), TFA (19 µl, 250 µmol) and hydrogen peroxide (43 µl, 35% purity, 500 µmol) were added and the mixture was stirred for 16h at 50°C. The mixture was evaporated and purified by preparative HPLC (method 10, gradient: 0.00-0.50 min 30% B, 0.50-6.00 min 30-70% B) to give 26.6 mg (95% purity, 12% yield) of the title compound.

LC-MS (method 2): Rₜ = 1.19 min; MS (ESlpos): m/z = 485 [M+H]⁺

¹H-NMR (400 MHz, DMSO-d₆) δ [ppm]: 1.24 (d, 3H), 1.27 (d, 6H), 2.55 - 2.64 (dd, 1H), 2.88 (dd, 1H), 3.24 (s, 3H), 3.73 - 3.83 (m, 1H), 3.89 (s, 3H), 4.13 - 4.24 (q, 2H), 6.17 (dd, 1H), 6.68 - 6.73 (m, 2H), 7.13 (s, 1H), 7.28 (d, 1H), 7.50 (s, 1H), 8.02 (d, 1H), 8.42 (s, 1H), 11.16 (s, 1H).

### Example 2

### (6S)-3-(3-chloro-2-methoxyanilino)-2-[3-(2-methoxy-2-methyl propoxy) pyridin-4-yl]-6-methyl-1,5,6,7-tetrahydro-4H-pyrrolo[3,2-c]pyridin-4-one

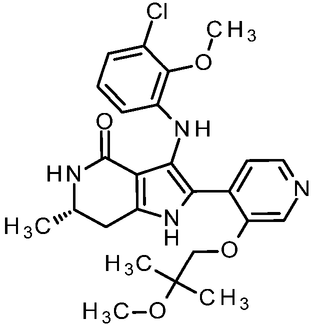

According to the method described for example 1 using (6*S*)-*N*-(3-chloro-2-methoxyphenyl)-4-({[3-(2-methoxy-2-methylpropoxy)pyridin-4-yl]methyl}amino)-6-methyl-2-oxo-1,2,5,6-tetrahydropyridine-3-carbothioamide (intermediate 6-2, 168 mg, 324 µmol) as starting material; 95.0 mg (90% purity, 54% yield) of the title compound were prepared after purification by preparative HPLC (method 10, gradient: 0.00-0.50 min 30% B, 0.50-6.00 min 30-70% B).

LC-MS (method 2): Rₜ = 1.18 min; MS (ESlpos): m/z = 485 [M+H]⁺

¹H-NMR (400 MHz, DMSO-d₆) δ [ppm]: 1.24 (d, 3H), 1.27 (d, 6H), 2.55 - 2.65 (m, 1H), 2.87 (dd, 1H), 3.24 (s, 3H), 3.74 - 3.87 (m, 1H), 3.89 (s, 3H), 4.14 - 4.23 (q, 2H), 6.14 - 6.19 (m, 1H), 6.67 - 6.73 (m, 2H), 7.12 (s, 1H), 7.27 (d, 1H), 7.49 (s, 1H), 8.02 (d, 1H), 8.42 (s, 1H), 11.16 (s, 1H).

### Example 3

### 3-(3-chloro-2-methoxyanilino)-6-methyl-2-(3-{[(2S)-oxolan-2-yl]methoxy}pyridin-4-yl)-1,5,6,7-tetrahydro-4H-pyrrolo[3,2-c]pyridin-4-one

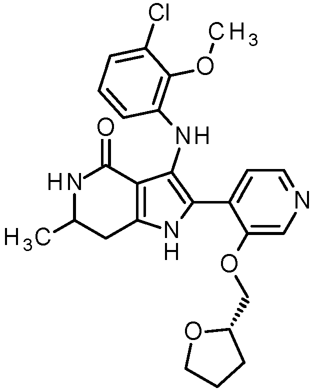

3-(3-Chloro-2-methoxyanilino)-5-[(2,4-dimethoxyphenyl)methyl]-6-methyl-2-(3-{[(2*S*)-oxolan-2-yl]methoxy}pyridin-4-yl)-1,5,6,7-tetrahydro-4H-pyrrolo[3,2-c]pyridin-4-one (intermediate 7, 340 mg, 537 µmol) was dissolved in ethanol (10 ml) and THF (5.0 ml), aqueous HCl (5.0 ml, 4.0 M, 20 mmol) was added and the mixture was stirred for 2 h at 80°C. The mixture was quenched with sat. sodium bicarbonate solution, extracted with EtOAc and the organic layer was washed with sat. sodium chloride solution, dried and evaporated. The residue was purified by preparative HPLC (method 10, gradient: 0.00-0.50 min 30% B, 0.50-6.00 min 30-70% B) to give 108 mg (95% purity, 40% yield) of the target compound.

LC-MS (method 2): Rₜ = 1.10 min; MS (ESlpos): m/z = 483 [M+H]⁺

¹H-NMR (400 MHz, DMSO-d₆) δ [ppm]: -0.008 (0.93), 0.008 (0.79), 1.227 (4.82), 1.234 (4.31), 1.243 (5.08), 1.250 (3.74), 1.654 (0.61), 1.672 (0.77), 1.684 (0.71), 1.689 (0.67), 1.701 (0.70), 1.856 (0.93), 1.872 (1.42), 1.876 (1.43), 1.887 (1.74), 1.893 (1.44), 1.903 (1.12), 1.915 (0.42), 1.991 (0.50), 1.996 (0.48), 2.004 (0.76), 2.013 (0.69), 2.022 (0.66), 2.032 (0.51), 2.036 (0.55), 2.324 (0.48), 2.329 (0.64), 2.333 (0.44), 2.520 (1.99), 2.525 (1.34), 2.554 (0.79), 2.579 (0.79), 2.584 (0.68), 2.594 (1.00), 2.620 (0.94), 2.624 (0.75), 2.666 (0.48), 2.671 (0.64), 2.676 (0.45), 2.856 (0.55), 2.868 (0.64), 2.880 (0.76), 2.894 (1.10), 2.908 (0.48), 2.921 (0.68), 2.934 (0.60), 3.310 (0.41), 3.741 (0.54), 3.761 (1.57), 3.778 (2.32), 3.786 (1.07), 3.798 (1.78), 3.815 (1.01), 3.821 (0.93), 3.827 (0.76), 3.838 (1.63), 3.844 (1.48), 3.854 (1.22), 3.859 (1.66), 3.868 (16.00), 3.875 (12.15), 3.992 (0.63), 4.011 (0.79), 4.017 (0.77), 4.028 (0.59), 4.037 (0.85), 4.048 (1.09), 4.054 (1.03), 4.075 (0.68), 4.299 (0.71), 4.307 (1.67), 4.314 (1.63), 4.324 (1.11), 4.333 (1.53), 4.341 (1.14), 4.379 (0.86), 4.388 (0.63), 4.404 (0.74), 4.413 (0.59), 5.761 (8.73), 6.151 (1.45), 6.158 (1.84), 6.167 (2.07), 6.172 (1.37), 6.175 (1.59), 6.181 (1.09), 6.653 (0.74), 6.666 (5.64), 6.673 (6.41), 6.682 (3.94), 6.689 (1.84), 6.702 (0.44), 7.094 (1.97), 7.108 (1.60), 7.259 (1.97), 7.270 (3.35), 7.282 (2.47), 7.449 (3.36), 7.486 (2.54), 8.021 (2.73), 8.027 (3.33), 8.034 (2.53), 8.040 (3.04), 8.422 (7.31), 11.216 (1.62), 11.242 (1.23).

### Example 4

### 3-(3-chloro-2-methoxyanilino)-6-methyl-2-(3-{[(2S)-oxolan-2-yl]methoxy}pyridin-4-yl)-1,5,6,7-tetrahydro-4H-pyrrolo[3,2-c]pyridin-4-one (stereoisomer 1)

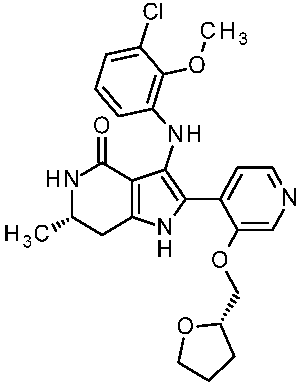

The title compound from example 3 (102 mg) was separated into enantiomers by preparative chiral HPLC to give 32.0 mg (95% purity, 30% yield) of the title compound (enantiomer 1, Rₜ = 12.8 - 15.2 min) and enantiomer 2 (43.0 mg, Rₜ = 9.8 - 12.1 min, see example 5).

### Preparative chiral HPLC method:

Instrument: PrepCon Labomatic HPLC; Column: YMC Amylose SA 5µ, 250x30; eluent A: hexane + 0.1 vol % diethylamine; eluent B: ethanol + 0.1 vol % diethylamine; isocratic: 80%A+20%B; flow: 50 ml/min; temperature: 25°C; UV: 254 nm

### Analytical chiral HPLC method:

Instrument: Waters Alliance 2695; Column: YMC Amylose SA 3µ, 100x4.6; eluent A: hexane + 0.1 vol % diethylamine; eluent B: ethanol; isocratic: 80%A+20%B; flow: 1.4 ml/min; temperature: 25°C; UV: 254 nm
Analytical chiral HPLC: Rₜ = 4.65 min.
Optical rotation:[α]_{D} = 6.0° +/- 0.49° (c = 1,36 mg/ml in DMSO)
LC-MS (method 2): Rₜ = 1.10 min; MS (ESlpos): m/z = 483 [M+H]⁺
¹H-NMR (400 MHz, DMSO-d₆) δ [ppm]: 1.24 (d, 3 H), 1.61 - 1.76 (m, 1 H), 1.82 - 1.94 (m, 2 H), 1.95 - 2.09 (m, 1 H), 2.59 (dd, 1 H), 2.88 (dd, 1 H), 3.70 - 3.92 (m, 6 H), 4.01 (dd, 1 H), 4.25 - 4.47 (m, 2 H), 6.17 (dd, 1 H), 6.62 - 6.76 (m, 2 H), 7.11 (s, 1 H), 7.26 (d, 1 H), 7.48 (s, 1 H), 8.03 (d, 1 H), 8.42 (s, 1 H), 11.24 (s, 1 H).

### Example 5

### 3-(3-chloro-2-methoxyanilino)-6-methyl-2-(3-{[(2S)-oxolan-2-yl]methoxy}pyridin-4-yl)-1,5,6,7-tetrahydro-4H-pyrrolo[3,2-c]pyridin-4-one (stereoisomer 2)

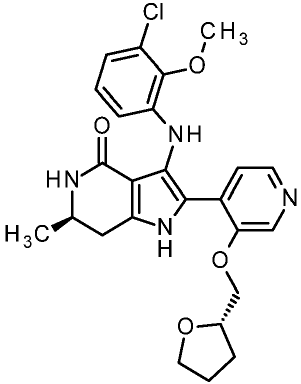

For the preparation of the title compound as mixture of two stereoisomers see example 3. Separation of diastereoomers by preparative chiral HPLC (method see example 4) gave 43.0 mg (95% purity, 40% yield) of the title compound (enantiomer 2, Rₜ = 9.8 - 12.1 min).
Analytical chiral HPLC (method see example 4): Rₜ = 3.78 min.
Optical rotation:[α]_{D} = 26.4° +/- 0.85° (c = 1,6 mg/ml in DMSO)
LC-MS (method 2): Rₜ = 1.10 min; MS (ESlpos): m/z = 483 [M+H]⁺
¹H-NMR (400 MHz, DMSO-d₆) δ [ppm]: 1.23 (d, 3 H), 1.61 - 1.74 (m, 1 H), 1.81 - 1.93 (m, 2 H), 1.94 - 2.08 (m, 1 H), 2.58 (dd, 1 H), 2.90 (dd, 1 H), 3.70 - 3.90 (m, 6 H), 4.01 - 4.11 (m, 1 H), 4.25 - 4.38 (m, 2 H), 6.16 (dd, 1 H), 6.60 - 6.76 (m, 2 H), 7.09 (s, 1 H), 7.27 (d, 1 H), 7.45 (s, 1 H), 8.03 (d, 1 H), 8.42 (s, 1 H), 11.21 (s, 1 H)

### Example 6

### (6R)-3-(3-chloro-2-methoxyanilino)-6-methyl-2-(3-{[(2S)-oxetan-2-yl]methoxy}pyridin-4-yl)-1,5,6,7-tetrahydro-4H-pyrrolo[3,2-c]pyridin-4-one

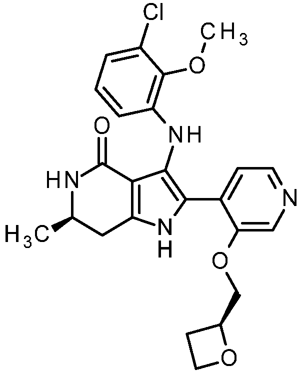

According to the method described for example 1 using (6*R*)-*N*-(3-chloro-2-methoxyphenyl)-6-methyl-4-{[(3-{[(2*S*)-oxetan-2-yl]methoxy}pyridin-4-yl)methyl]amino}-2-oxo-1,2,5,6-tetrahydropyridine-3-carbothioamide (intermediate 6-6, 78.0 mg, 155 µmol) as starting material, 39.2 mg (95% purity, 51 % yield) of the title compound were prepared after purification by preparative HPLC (method 10, gradient: 0.00-0.50 min 30% B, 0.50-6.00 min 30-70% B).

LC-MS (method 2): Rₜ = 1.03 min; MS (ESlpos): m/z = 469 [M+H]⁺

¹H-NMR (400 MHz, DMSO-d₆) δ [ppm]: 1.22 (d, 3H), 2.54 - 2.76 (m, 3H), 2.81 - 2.92 (dd, 1H), 3.72 - 3.81 (m, 1H), 3.86 (s, 3H), 4.28 (dd, 1H), 4.38 - 4.50 (m, 2H), 4.56 - 4.64 (m, 1H), 5.07 - 5.19 (m, 1H), 6.16 (dd, 1H), 6.63 - 6.69 (m, 2H), 7.07 (s, 1H), 7.28 (d, 1H), 7.44 (s, 1H), 8.06 (d, 1H), 8.46 (s, 1H), 11.20 (s, 1H).

### Example 7

### (6S)-3-(3-chloro-2-methoxyanilino)-6-methyl-2-(3-{[(2S)-oxetan-2-yl]methoxy}pyridin-4-yl)-1,5,6,7-tetrahydro-4H-pyrrolo[3,2-c]pyridin-4-one

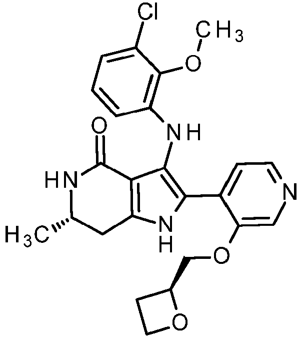

According to the method described for example 1 using (6*S*)-*N*-(3-chloro-2-methoxyphenyl)-6-methyl-4-{[(3-{[(2*S*)-oxetan-2-yl]methoxy}pyridin-4-yl)methyl]amino}-2-oxo-1,2,5,6-tetrahydropyridine-3-carbothioamide (intermediate 6-7, 149 mg, 296 µmol) as starting material, 46.0 mg (95% purity, 31% yield) of the title compound were prepared after purification by preparative HPLC (method 10, gradient: 0.00-0.50 min 30% B, 0.50-6.00 min 30-70% B).

LC-MS (method 2): Rₜ = 1.03 min; MS (ESlpos): m/z = 469 [M+H]⁺

¹H-NMR (400 MHz, DMSO-d₆) δ [ppm]: 1.24 (d, 3H), 1.27 (d, 6H), 2.55 - 2.65 (dd, 1H), 2.87 (dd, 1H), 3.24 (s, 3H), 3.74 - 3.87 (m, 1H), 3.89 (s, 3H), 4.14 - 4.23 (q, 2H), 6.14 - 6.19 (m, 1H), 6.67 - 6.73 (m, 2H), 7.12 (s, 1H), 7.27 (d, 1H), 7.49 (s, 1H), 8.02 (d, 1H), 8.42 (s, 1H), 11.16 (s, 1H).

### Example 8

### 3-(3-fluoro-2-methoxyanilino)-6-methyl-2-(3-{[(2S)-oxolan-2-yl]methoxy}pyridin-4-yl)-1,5,6,7-tetrahydro-4H-pyrrolo[3,2-c]pyridin-4-one

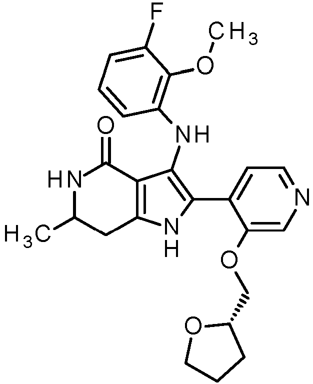

5-[(2,4-Dimethoxyphenyl)methyl]-3-(3-fluoro-2-methoxyanilino)-6-methyl-2-(3-{[(2*S*)-oxolan-2-yl]methoxy}pyridin-4-yl)-1,5,6,7-tetrahydro-4*H*-pyrrolo[3,2-c]pyridin-4-one (intermediate 8, 60.0 mg, 108 µmol) was stirred in polyphosphoric acid (600 µl) for 3 h at 100°C. The mixture was cooled to 0°C and diluted with ice water. The mixture was purified by preparative HPLC (method 10, gradient: 0.00-0.50 min 30% B, 0.50-6.00 min 30-70% B) to give 3.5 mg (85% purity, 6% yield) of the target compound.

LC-MS (method 2): Rₜ = 1.09 min; MS (ESlpos): m/z = 467 [M+H]⁺

¹H-NMR (400 MHz, DMSO-d₆) δ [ppm]: 0.876 (0.23), 1.241 (0.35), 1.257 (2.24), 1.264 (1.00), 1.273 (0.64), 1.281 (0.31), 1.374 (0.21), 1.897 (0.18), 1.913 (0.21), 1.918 (0.17), 2.543 (0.63), 2.548 (0.44), 2.565 (16.00), 3.786 (0.19), 3.803 (0.26), 3.823 (0.24), 3.862 (0.18), 3.868 (0.18), 3.922 (1.19), 3.928 (1.08), 3.940 (0.18), 4.335 (0.21), 4.356 (0.20), 6.038 (0.16), 6.058 (0.20), 6.506 (0.17), 6.649 (0.18), 6.670 (0.16), 7.120 (0.20), 7.133 (0.20), 7.286 (0.17), 7.296 (0.20), 7.308 (0.18), 7.469 (0.32), 7.511 (0.29), 8.442 (0.22).

### Example 9

### 3-(3-fluoro-2-methoxyanilino)-6-methyl-2-(3-{[(2S)-oxolan-2-yl]methoxy}pyridin-4-yl)-1,5,6,7-tetrahydro-4H-pyrrolo[3,2-c]pyridin-4-one (stereoisomer 1)

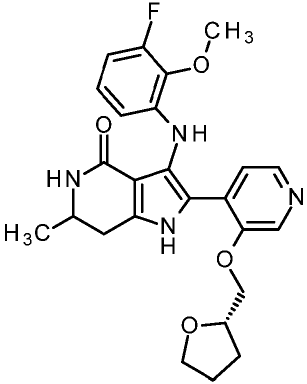

The title compound from example 8 (88 mg) was separated into enantiomers by preparative chiral HPLC to give 29.0 mg (95% purity, 31% yield) of the title compound (enantiomer 1, Rₜ = 13.8 - 15.7 min) and enantiomer 2 (37.0 mg, Rₜ = 16.5 - 19.2 min, see example 10).

### Preparative chiral HPLC method:

Instrument: Sepiatec: Prep SFC100; Column: Reprosil Chiral NR 8µ 250x30mm; eluent A: CO2; eluent B: methanol + 0.2 vol % aqueous ammonia (32%); isocratic: 35%B; flow: 100 ml/min; temperature: 40°C; BPR: 150bar; UV: 254 nm

### Analytical chiral HPLC method:

Instrument: Agilent: 1260, Aurora SFC-Modul; Column: Reprosil Chiral NR 5µ 100x4.6mm; eluent A: CO2; eluent B: methanol + 0.2 vol % aqueous ammonia (32%); isocratic: 35%B; flow: 4 ml/min; temperature: 37.5°C; BPR: 100bar; UV: 254 nm
Analytical chiral HPLC: Rₜ = 3.88 min.
Optical rotation:[α]_{D} = -28.5° +/- 0.87° (c = 1,55 mg/ml in DMSO)
LC-MS (method 2): Rₜ = 1.06 min; MS (ESlpos): m/z = 467 [M+H]⁺
¹H-NMR (400 MHz, DMSO-d₆) δ [ppm]: -0.009 (0.75), 0.007 (0.66), 1.162 (0.57), 1.234 (6.98), 1.249 (6.41), 1.656 (0.62), 1.675 (0.77), 1.684 (0.72), 1.689 (0.61), 1.706 (0.84), 1.723 (0.44), 1.852 (0.41), 1.858 (0.49), 1.873 (1.35), 1.889 (2.23), 1.906 (1.66), 1.925 (0.57), 1.985 (0.50), 2.003 (0.68), 2.017 (0.76), 2.023 (0.53), 2.034 (0.73), 2.049 (0.60), 2.324 (0.40), 2.329 (0.55), 2.333 (0.40), 2.525 (1.28), 2.542 (1.66), 2.555 (0.93), 2.582 (0.94), 2.595 (1.13), 2.622 (1.17), 2.671 (0.52), 2.851 (1.05), 2.863 (1.18), 2.891 (0.93), 2.903 (0.84), 3.763 (0.78), 3.772 (0.79), 3.783 (1.90), 3.800 (2.52), 3.816 (1.18), 3.828 (1.06), 3.845 (2.06), 3.862 (1.19), 3.865 (1.27), 3.882 (0.59), 3.905 (16.00), 3.983 (1.13), 4.002 (1.43), 4.007 (1.38), 4.026 (1.35), 4.313 (0.44), 4.323 (0.84), 4.330 (1.04), 4.340 (0.77), 4.349 (0.98), 4.366 (0.44), 4.382 (1.60), 4.389 (1.12), 4.406 (1.35), 4.414 (1.08), 6.017 (1.85), 6.037 (1.94), 6.463 (0.81), 6.466 (0.86), 6.484 (1.14), 6.487 (1.24), 6.493 (0.92), 6.511 (1.07), 6.514 (1.02), 6.612 (0.84), 6.627 (0.99), 6.632 (1.49), 6.647 (1.47), 6.653 (0.75), 6.668 (0.64), 7.111 (3.02), 7.263 (2.22), 7.276 (2.25), 7.489 (4.61), 8.018 (1.46), 8.031 (1.38), 8.419 (2.26), 11.227 (2.36).

### Example 10

### 3-(3-fluoro-2-methoxyanilino)-6-methyl-2-(3-{[(2S)-oxolan-2-yl]methoxy}pyridin-4-yl)-1,5,6,7-tetrahydro-4H-pyrrolo[3,2-c]pyridin-4-one (stereoisomer 2)

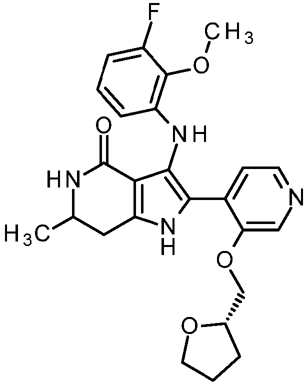

For the preparation of the title compound as mixture of two stereoisomers see example 8. Separation of diastereomers by preparative chiral HPLC (method see example 9) to give 37.0 mg (95 % purity, 40 % yield) of the title compound (enantiomer 2, Rt = 16.5 - 19.2 min).
Analytical chiral HPLC (method see example 13): Rₜ = 4.61 min.
Optical rotation:[α]_{D} = 20.7° +/- 1.48° (c = 1,4 mg/ml in DMSO)
LC-MS (method 2): Rt = 1.05 min; MS (ESlpos): m/z = 467 [M+H]⁺
¹H-NMR (400 MHz, DMSO-d₆) δ [ppm]: -0.008 (1.65), 0.008 (1.20), 1.161 (0.61), 1.226 (6.68), 1.242 (6.76), 1.653 (0.59), 1.673 (0.76), 1.681 (0.64), 1.690 (0.56), 1.703 (0.69), 1.850 (0.47), 1.858 (1.01), 1.866 (1.05), 1.877 (1.57), 1.895 (1.37), 1.902 (0.85), 1.912 (0.54), 1.918 (0.44), 1.976 (0.50), 1.993 (0.64), 2.006 (0.81), 2.013 (0.54), 2.025 (0.64), 2.038 (0.47), 2.329 (0.77), 2.333 (0.54), 2.520 (2.56), 2.525 (1.73), 2.542 (0.48), 2.550 (1.02), 2.576 (0.99), 2.590 (1.15), 2.616 (1.18), 2.671 (0.77), 2.676 (0.56), 2.878 (1.06), 2.891 (1.19), 2.918 (0.97), 2.931 (0.89), 3.310 (0.55), 3.742 (0.69), 3.759 (1.54), 3.779 (2.13), 3.796 (1.44), 3.813 (0.43), 3.823 (1.01), 3.840 (2.01), 3.857 (1.24), 3.860 (1.35), 3.877 (0.65), 3.899 (16.00), 4.023 (0.76), 4.044 (1.46), 4.050 (1.41), 4.071 (1.01), 4.295 (0.51), 4.313 (2.78), 4.325 (1.22), 4.333 (2.10), 4.341 (1.20), 6.011 (1.84), 6.032 (1.93), 6.452 (0.84), 6.456 (0.84), 6.473 (1.19), 6.477 (1.24), 6.483 (0.89), 6.500 (1.11), 6.504 (0.99), 6.605 (0.86), 6.621 (0.99), 6.626 (1.50), 6.641 (1.46), 6.647 (0.76), 6.662 (0.65), 7.097 (2.84), 7.271 (2.94), 7.283 (2.95), 7.446 (4.69), 8.024 (2.60), 8.036 (2.43), 8.418 (4.08), 11.198 (2.34).

### Example 11

### (6R)-3-(3-chloro-2-methoxyanilino)-6-methyl-2-(3-{[(2S)-4-methylmorpholin-2-yl]methoxy}pyridin-4-yl)-1,5,6,7-tetrahydro-4H-pyrrolo[3,2-c]pyridin-4-one

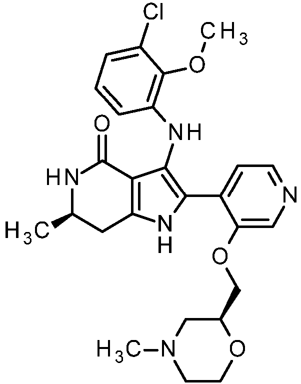

(6*R*)-3-(3-Chloro-2-methoxyanilino)-6-methyl-2-(3-{[(2*S*)-morpholin-2-yl]methoxy}pyridin-4-yl)-1,5,6,7-tetrahydro-4*H*-pyrrolo[3,2-c]pyridin-4-one (intermediate 11-2, 30.0 mg, 60.2 µmol) was dissolved in methanol (820 µl), formaldehyde (9.0 µl, 37% purity, 120 µmol) and acetic acid (3.4 µl, 60 µmol) were added and the mixture was stirred for 15 min at RT. Sodium triacetoxyborohydride (19.2 mg, 90.4 µmol) was added and the mixture was stirred for 1h at RT. The mixture was filtered trough a SCX column and washed with MeOH and ammonia (7M in MeOH). The ammonia filtrate was evaporated and purified by preparative HPLC (method 10, gradient: 0.00-0.50 min 30% B, 0.50-6.00 min 30-70% B) to give 16.7 mg (90% purity, 49% yield) of the title compound.

LC-MS (method 2): Rt = 1.03 min; MS (ESlpos): m/z = 512 [M+H]⁺

¹H-NMR (400 MHz, DMSO-d₆) δ [ppm]: 1.24 (d, 3H), 1.84 - 1.95 (m, 1H), 1.99 - 2.07 (m, 1H), 2.20 (s, 3H), 2.59 - 2.70 (m, 2H), 2.72 - 2.80 (d, 1H), 2.88 - 2.97 (dd, 1H), 3.59 - 3.69 (dt, 1H), 3.73 - 3.82 (m, 1H), 3.88 (s, 3H), 3.90 - 3.97 (m, 2H), 4.19 (dd, 1H), 4.28 (dd, 1H), 6.15 (dd, 1H), 6.65 - 6.71 (m, 2H), 7.12 (s, 1H), 7.28 (d, 1H), 7.50 (s, 1H), 8.03 (d, 1H), 8.40 (s, 1H), 11.04 (s, 1H).

### Example 12

### (6R)-3-(3-chloro-2-methoxyanilino)-2-(3-{[(2S)-4-(2,2-difluoroethyl)morpholin-2-yl]methoxy}pyridin-4-yl)-6-methyl-1,5,6,7-tetrahydro-4H-pyrrolo[3,2-c]pyridin-4-one

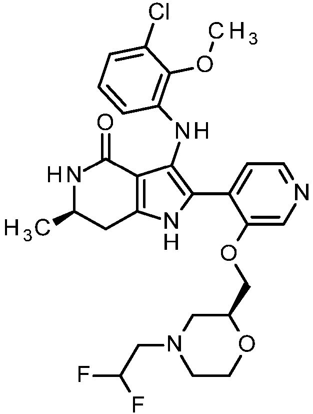

(6*R*)-3-(3-Chloro-2-methoxyanilino)-6-methyl-2-(3-{[(2S)-morpholin-2-yl]methoxy}pyridin-4-yl)-1,5,6,7-tetrahydro-4*H*-pyrrolo[3,2-c]pyridin-4-one (intermediate 11-2, 30.0 mg, 60.2 µmol) was dissolved in DMF (0.9 ml), triethylamine (34 µl, 240 µmol) and 2,2-difluoroethyl trifluoromethanesulfonate (16 µl, 120 µmol) were added and the mixture was stirred for 1h at RT. The mixture was filtered trough a SCX column and washed with MeOH and ammonia (7M in MeOH). The ammonia filtrate was evaporated and purified by preparative HPLC (method 10, gradient: 0.00-0.50 min 30% B, 0.50-6.00 min 30-70% B) to give 6.9 mg (85% purity, 17% yield) of the target compound.

LC-MS (method 2): Rt = 1.14 min; MS (ESlpos): m/z = 562 [M+H]⁺

¹H-NMR (400 MHz, DMSO-d₆) δ [ppm]: 1.24 (d, 3H), 2.23 (s, 1H), 2.29 - 2.36 (m, 1H), 2.57 - 2.65 (m, 1H), 2.72 - 2.86 (m, 3H), 2.86 - 2.97 (m, 2H), 3.65 (m, 2H), 3.88 (s, 3H), 3.91 - 4.00 (m, 2H), 4.12 - 4.29 (m, 2H), 5.95 - 6.35 (m, 1H), 6.12 - 6.19 (m, 1H), 6.65 - 6.71 (m, 2H), 7.08 - 7.15 (s, 1H), 7.28 (d, 1H), 7.52 (s, 1H), 8.03 (d, 1H), 8.40 (s, 1H), 11.02 (s, 1H).

### Example 13

### 3-(3-chloro-2-methoxyanilino)-7-(2-hydroxyethyl)-2-[3-(2-methoxy-2-methylpropoxy)pyridin-4-yl]-1,5,6,7-tetrahydro-4H-pyrrolo[3,2-c]pyridin-4-one

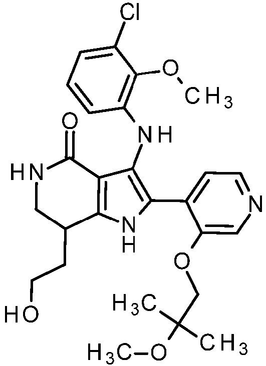

According to the method described for example 1 using *N*-(3-chloro-2-methoxyphenyl)-5-(2-hydroxyethyl)-4-({[3-(2-methoxy-2-methylpropoxy)pyridin-4-yl]methyl}amino)-2-oxo-1,2,5,6-tetrahydropyridine-3-carbothioamide (intermediate 6-13, 79.0 mg, 144 µmol) as starting material; 33.0 mg (90% purity, 40% yield) of the title compound were prepared after purification by preparative HPLC (method 10, gradient: 0.00-0.50 min 15% B, 0.50-6.00 min 15-55% B).

¹H-NMR (400 MHz, DMSO-d₆) δ [ppm]: 1.27 (d, 6H), 1.61 - 1.77 (m, 1H), 1.90 - 2.00 (m, 1H), 2.98 - 3.13 (m, 1H), 3.18 - 3.27 (m, 1H), 3.22 (s, 3H), 3.49 - 3.59 (m, 3H), 3.88 (s, 3H), 4.18 (s, 2H), 4.65 - 4.75 (t, 1H), 6.10 - 6.21 (quin, 1H), 6.68 (m, 2H), 7.12 - 7.20 (m, 1H), 7.26 - 7.32 (d, 1H), 7.50 - 7.56 (s, 1H), 7.99 - 8.04 (d, 1H), 8.43 (s, 1H), 10.93 - 11.00 (s, 1H).

### Example 14

### 3-(3-chloro-2-methoxyanilino)-7-(2-hydroxyethyl)-2-[3-(2-methoxy-2-methylpropoxy)pyridin-4-yl]-1,5,6,7-tetrahydro-4H-pyrrolo[3,2-c]pyridin-4-one (stereoisomer 1)

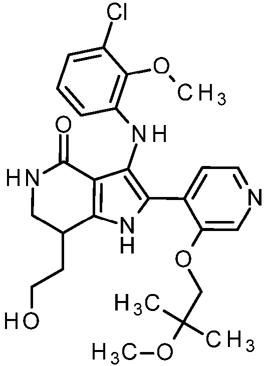

The title compound from example 13 (28 mg) was separated into enantiomers by preparative chiral HPLC to give 8.6 mg (95% purity, 11% yield) of the title compound (enantiomer 1, Rₜ = 7.1 - 9.6 min) and enantiomer 2 (8.5 mg, Rₜ = 10.4 - 13.8 min, see example 15).

### Preparative chiral HPLC method: MTBE

Instrument: PrepCon Labomatic HPLC; Column: YMC Amylose SA 5µ, 250x30; eluent A: methyl tert-butyl ether + 0.1 vol % diethylamine; eluent B: acetonitrile + 0.1 vol % diethylamine; isocratic: 60%A+40%B; flow: 50 ml/min; temperature: 25°C; UV: 254 nm Analytical chiral HPLC method: MTBE
Instrument: Waters Alliance 2695; Column: YMC Amylose SA 3µ, 100x4.6; eluent A: methyl tert-butyl ether + 0.1 vol % diethylamine; eluent B: acetonitrile; isocratic: 60%A+40%B; flow: 1.4 ml/min; temperature: 25°C; UV: 254 nm
Analytical chiral HPLC: Rₜ = 2.93 min.

### Example 15

### 3-(3-chloro-2-methoxyanilino)-7-(2-hydroxyethyl)-2-[3-(2-methoxy-2-methylpropoxy)pyridin-4-yl]-1,5,6,7-tetrahydro-4H-pyrrolo[3,2-c]pyridin-4-one (stereoisomer 2)

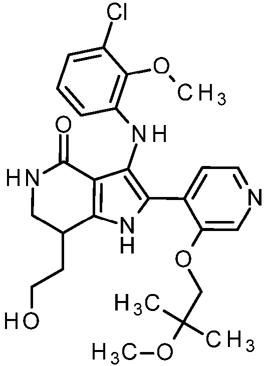

For the preparation of the title compound as mixture of stereoisomers see example 13. Separation of enantiomers by preparative chiral HPLC (method see example 14) gave 8.5 mg (95% purity, 11% yield) of the title compound (enantiomer 2, Rₜ = Rt = 10.4 - 13.8 min).

Analytical chiral HPLC (method see example 14): Rₜ = 4.28 min.

### Example 16

### 3-(3-chloro-2-methoxyanilino)-7-(2-hydroxyethyl)-2-(3-{[(2S)-oxolan-2-yl]methoxy}pyridin-4-yl)-1,5,6,7-tetrahydro-4H-pyrrolo[3,2-c]pyridin-4-one

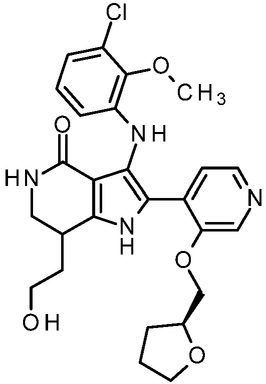

According to the method described for example 1 using *N*-(3-chloro-2-methoxyphenyl)-5-(2-hydroxyethyl)-2-oxo-4-[({3-[(2*S*)-tetrahydrofuran-2-ylmethoxy]pyridin-4-yl}methyl)amino]-1,2,5,6-tetrahydropyridine-3-carbothioamide (intermediate 6-16, 141 mg, 258 µmol) as starting material, 53.0 mg (90% purity, 36% yield) of the title compound were prepared after purification by preparative HPLC (method 10, gradient: 0.00-0.50 min 15% B, 0.50-6.00 min 15-55% B).

LC-MS (method 2): Rₜ = 1.01 min; MS (ESlpos): m/z = 513 [M+H]⁺

¹H-NMR (400 MHz, DMSO-d₆) δ [ppm]: 1.63 - 1.79 (m, 2H), 1.84 - 1.96 (m, 3H), 1.96 - 2.05 (m, 1H), 2.06 - 2.11 (m, 5H), 3.02 - 3.13 (m, 1H), 3.14 - 3.25 (m, 1H), 3.47 - 3.66 (m, 3H), 3.69 - 3.85 (m, 2H), 3.87 (d, 3H), 4.00 - 4.10 (m, 1H), 4.27 - 4.42 (m, 2H), 4.81 (t, 1H), 6.15 (m, 1H), 6.65 - 6.70 (m, 2H), 7.06 - 7.23 (s, 1H), 7.27 (dd, 1H), 7.52 (d, 1H), 8.02 (dd, 1H), 8.42 (d, 1H), 8.46 - 8.48 (m, 1H), 11.05 - 11.13 (d, 1H).

### Example 17

### 3-(3-chloro-2-methoxyanilino)-7-(2-hydroxyethyl)-2-(3-{[(2S)-oxolan-2-yl]methoxy}pyridin-4-yl)-1,5,6,7-tetrahydro-4H-pyrrolo[3,2-c]pyridin-4-one (stereoisomer 1)

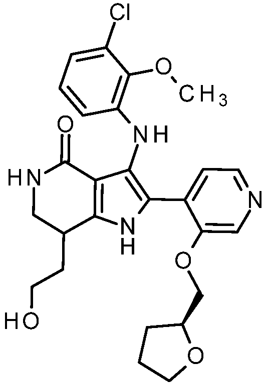

The title compound from example 16 (50 mg) was separated into enantiomers by preparative chiral HPLC to give 22.0 mg (90% purity, 17% yield) of the title compound (enantiomer 1, Rₜ = 12.5 - 15.0 min) and enantiomer 2 (26.0 mg, Rₜ = 18.5 - 24.0 min, see example 18).

### Preparative chiral HPLC method: SFC

Instrument: Sepiatec: Prep SFC100; Column: Chiralpak IA 5µ 250x30mm; eluent A: CO2; eluent B: ethanol + 0.2 vol % aqueous ammonia (32%); isocratic: 25%B; flow: 100 ml/min; temperature: 40°C; BPR: 150bar; UV: 254 nm

### Analytical chiral HPLC method: SFC

Instrument: Agilent: 1260, Aurora SFC-Modul; Column: Chiralpak IA 5µ 100x4.6mm; eluent A: CO2; eluent B: ethanol + 0.2 vol % aqueous ammonia (32%); isocratic: 25%B; flow: 4 ml/min; temperature: 37.5°C; BPR: 100bar; UV: 254 nm
Analytical chiral HPLC: Rₜ = 2.84 min.
¹H-NMR (400 MHz, DMSO-d₆) δ [ppm]: 1.63 - 1.73 (m, 2H), 1.84 - 1.92 (m, 2H), 1.93 - 2.05 (m, 2H), 3.08 (m, 1H), 3.16 - 3.25 (m, 1H), 3.47 - 3.55 (m, 2H), 3.56 (m, 1H), 3.76 - 3.86 (m, 2H), 3.87 (s, 3H), 4.03 - 4.11 (m, 1H), 4.25 - 4.37 (m, 2H), 4.81 (t, 1H), 6.13 - 6.17 (dd, 1H), 6.67 (s, 1H), 6.68 (d, 1H), 7.15 (m, 1H), 7.28 (d, 1H), 7.52 (s, 1H), 8.02 (d, 1H), 8.42 (s, 1H), 11.10 (s, 1H).

### Example 18

### 3-(3-chloro-2-methoxyanilino)-7-(2-hydroxyethyl)-2-(3-{[(2S)-oxolan-2-yl]methoxy}pyridin-4-yl)-1,5,6,7-tetrahydro-4H-pyrrolo[3,2-c]pyridin-4-one (stereoisomer 2)

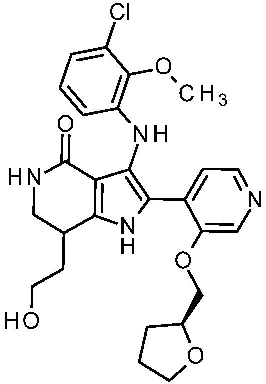

For the preparation of the title compound as mixture of two stereoisomers see example 16. Separation of diastereomers by preparative chiral HPLC (method see example 17) to give 26.0 mg (95% purity, 11% yield) of the title compound (enantiomer 2, Rₜ = 18.5 - 24.0 min).

Analytical chiral HPLC (method see example 17): Rₜ = 4.14 min.

¹H-NMR (400 MHz, DMSO-d₆) δ [ppm]: 1.62 - 1.74 (m, 2H), 1.84 - 1.94 (m, 3H), 1.98 - 2.06 (m, 1H), 3.06 (m, 1H), 3.22 (m, 1H), 3.48 - 3.60 (m, 3H), 3.76 - 3.86 (m, 2H), 3.87 (s, 3H), 4.03 (dd, 1H), 4.26 - 4.41 (m, 2H), 4.81 (t, 1H), 6.15 (dd, 1H), 6.67 (s, 1H), 6.68 (d, 1H), 7.11 - 7.17 (m, 1H), 7.27 (d, 1H), 7.53 (s, 1H), 8.02 (br d, 1H), 8.42 (s, 1H), 11.11 (s, 1H).

### Example 19

### 3-(3-chloro-2-methoxyanilino)-7-(2-hydroxyethyl)-2-{3-[(2-methyloxetan-2-yl)methoxy]pyridin-4-yl}-1,5,6,7-tetrahydro-4H-pyrrolo[3,2-c]pyridin-4-one

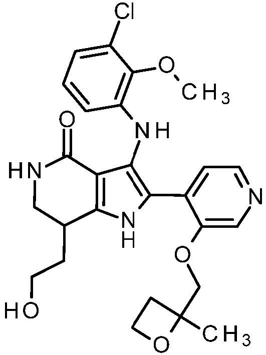

According to the method described for example 1 using *N*-(3-chloro-2-methoxyphenyl)-5-(2-hydroxyethyl)-4-{[(3-{[2-methyloxetan-2-yl]methoxy}pyridin-4-yl)methyl]amino}-2-oxo-1,2,5,6-tetrahydropyridine-3-carbothioamide (intermediate 6-19, 112 mg, 205 µmol) as starting material, 42.7 mg (95% purity, 39% yield) of the title compound were prepared after purification by preparative HPLC (method 10, gradient: 0.00-0.50 min 15% B, 0.50-6.00 min 15-55% B).

LC-MS (method 2): Rₜ = 0.98 min; MS (ESlpos): m/z = 513 [M+H]⁺

¹H-NMR (400 MHz, DMSO-d₆) δ [ppm]: 1.43 - 1.49 (d, 3H), 1.57 - 1.76 (m, 1H), 1.78 - 2.00 (m, 1H), 2.29 - 2.45 (m, 2H), 2.63 - 2.82 (m, 1H), 2.95 - 3.04 (m, 1H), 3.14 - 3.27 (m, 1H), 3.52 (m, 3H), 3.85 (d, 3H), 4.05 - 4.48 (m, 5H), 4.77 (m, 1H), 6.15 - 6.20 (m, 1H), 6.61 - 6.68 (m, 2H), 7.11 (d, 1H), 7.28 (d, 1H), 7.44 (d, 1H), 8.05 (dd, 1H), 8.48 (d, 1H), 11.19 (s, 1H).

### Example 20

### 3-(3-chloro-2-methoxyanilino)-7-(2-hydroxyethyl)-2-{3-[(2-methyloxetan-2-yl)methoxy]pyridin-4-yl}-1,5,6,7-tetrahydro-4H-pyrrolo[3,2-c]pyridin-4-one (stereoisomer 1)

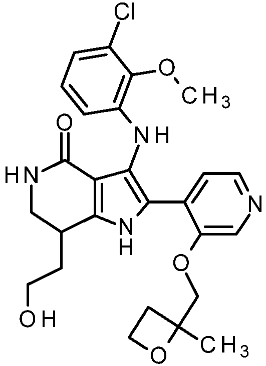

The title compound from example 19 (43 mg) was separated into enantiomers by preparative chiral HPLC to give 10.0 mg (99% purity, 10% yield) of the title compound (enantiomer 1, Rₜ = 27.4 - 32.6 min), enantiomer 2 (9.0 mg, Rₜ = 44.6 - 52.2 min, see example 21) and a mixture of enantiomer 3 and 4 (22 mg, Rₜ = 20.5 - 26.9 min, see example 22 and 23)

### Preparative chiral HPLC method: MTBE

Instrument: PrepCon Labomatic HPLC; Column: YMC Amylose SA 5µ, 250x30; eluent A: methyl tert-butyl ether + 0.1 vol % diethylamine; eluent B: acetonitrile + 0.1 vol % diethylamine; isocratic: 80%A+20%B; flow: 50 ml/min; temperature: 25°C; UV: 280 nm

### Analytical chiral HPLC method: MTBE

Instrument: Waters Alliance 2695; Column: YMC Amylose SA 3µ, 100x4.6; eluent A: methyl tert-butyl ether + 0.1 vol % diethylamine; eluent B: acetonitrile; isocratic: 80%A+20%B; flow: 1.4 ml/min; temperature: 25°C; UV: 280 nm
Analytical chiral HPLC: Rₜ = 10.73 min.

### Example 21

### 3-(3-chloro-2-methoxyanilino)-7-(2-hydroxyethyl)-2-{3-[(2-methyloxetan-2-yl)methoxy]pyridin-4-yl}-1,5,6,7-tetrahydro-4H-pyrrolo[3,2-c]pyridin-4-one (stereoisomer 2)

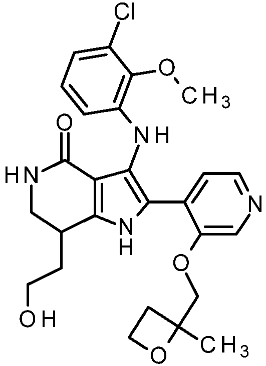

For the preparation of the title compound as mixture of stereoisomers see example 19. Separation of enantiomers by preparative chiral HPLC (method see example 20) to give 9.0 mg (99 % purity, 9 % yield) of the title compound (enantiomer 2, Rₜ = 44.6 - 52.2 min).

Analytical chiral HPLC (method see example 29): Rₜ = 17.38 min.

### Example 22

### 3-(3-chloro-2-methoxyanilino)-7-(2-hydroxyethyl)-2-{3-[(2-methyloxetan-2-yl)methoxy]pyridin-4-yl}-1,5,6,7-tetrahydro-4H-pyrrolo[3,2-c]pyridin-4-one (stereoisomer 3)

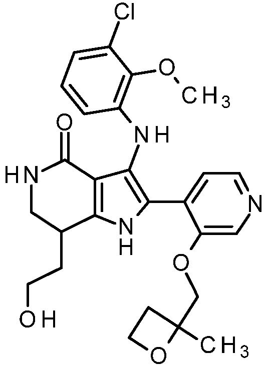

For the preparation of the title compound as the mixture of stereoisomers see example 19. Separation of enantiomers by preparative chiral HPLC (method see example 20) to give a mixture of enantiomer 3 and 4 (22 mg, Rₜ = 20.5 - 26.9 min). An additional preparative chiral HPLC separation gave 9.0 mg (99% purity, 9% yield) of the title compound (enantiomer 3, Rₜ = 6.4 - 8.4 min) and enantiomer 4 (11.0 mg, Rₜ = 9.5 - 11.3 min).

### Preparative chiral HPLC method: MTBE

Instrument: PrepCon Labomatic HPLC; Column: YMC Cellulose SC 5µ, 250x30; eluent A: methyl tert-butyl ether + 0.1 vol % diethylamine; eluent B: acetonitrile + 0.1 vol % diethylamine; isocratic: 50%A+50%B; flow: 50 ml/min; temperature: 25°C; UV: 280 nm

### Analytical chiral HPLC method: MTBE

Instrument: Waters Alliance 2695; Column: YMC Cellulose SC 3µ, 100x4.6; eluent A: methyl tert-butyl ether + 0.1 vol % diethylamine; eluent B: acetonitrile; isocratic: 50%A+50%B; flow: 1.4 ml/min; temperature: 25°C; UV: 280 nm
Analytical chiral HPLC: Rₜ = 2.57 min.

### Example 23

### 3-(3-chloro-2-methoxyanilino)-7-(2-hydroxyethyl)-2-{3-[(2-methyloxetan-2-yl)methoxy]pyridin-4-yl}-1,5,6,7-tetrahydro-4H-pyrrolo[3,2-c]pyridin-4-one (stereoisomer 4)

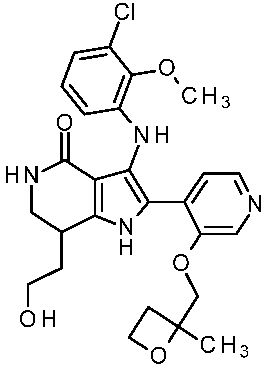

For the preparation of the title compound as the mixture of stereoisomers see example 19. Separation of enantiomers by preparative chiral HPLC (method see example 20) to give a mixture of enantiomer 3 and 4 (22 mg, Rₜ = 20.5 - 26.9 min). An additional preparative chiral HPLC (method see example 22) gave 11.0 mg (99 % purity, 10 % yield) of the title compound (enantiomer 4, Rₜ = 9.5 - 11.3 min).

Analytical chiral HPLC (method see example 22): Rₜ = 3.59 min.

### Example 24

### (6R)-3-(3-chloro-2-methoxy-anilino)-2-[3-(1,4-dioxan-2-ylmethoxy)-4-pyridyl]-6-methyl-1,5,6,7-tetrahydropyrrolo[3,2-c]pyridin-4-one

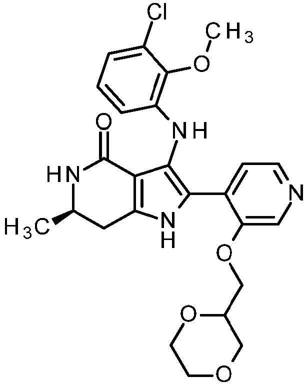

According to the method described for example 1 using (2*R*)-N-(3-chloro-2-methoxyphenyl)-4-{[3-(1,4-dioxan-2-ylmethoxy)-4-pyridyl]methylamino}-2-methyl-6-oxo-2,3-dihydro-1*H*-pyridine-5-carbothioamide (intermediate 6-24, 220 mg, 413 µmol) as starting material, 86.0 mg (95% purity, 40% yield) of the title compound were prepared after purification by preparative HPLC (method 10, gradient: 0.00-0.50 min 30% B, 0.50-7.00 min 30-70% B).

LC-MS (method 2): Rₜ = 1.05 min; MS (ESlneg): m/z = 497 [M-H]⁻

¹H-NMR (400 MHz, DMSO-d₆) δ [ppm]: 1.24 (dd, 3H), 2.55 - 2.65 (dd, 1H), 2.94 (m, 1H), 3.38 - 3.47 (m, 1H), 3.47 - 3.58 (m, 1H), 3.69 - 3.92 (m, 5H), 3.85 - 3.89 (d, 4H), 3.97 - 4.09 (m, 1H), 4.09 - 4.20 (dd, 1H), 4.20 - 4.35 (dd, 1H), 6.15 (m, 1H), 6.66 - 6.70 (m, 2H), 7.09 - 7.14 (m, 1H), 7.28 (dd, 1H), 7.50 (d, 1H), 8.04 (dd, 1H), 8.39 (d, 1H), 11.04 (d, 1H).

### Example 25

### (6R)-3-(3-chloro-2-methoxy-anilino)-2-[3-(1,4-dioxan-2-ylmethoxy)-4-pyridyl]-6-methyl-1,5,6,7-tetrahydropyrrolo[3,2-c]pyridin-4-one (stereoisomer 1)

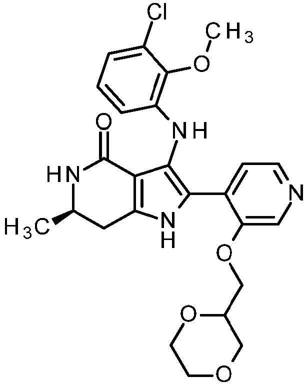

The title compound from example 24 (80 mg) was separated into enantiomers by preparative chiral HPLC to give 26.8 mg (95 % purity, 12 % yield) of the title compound (enantiomer 1, Rₜ = 12.0 - 14.7 min) and enantiomer 2 (30.0 mg, Rₜ = 16.3 - 19.8 min, see example 26).

### Preparative chiral HPLC method: NPB

Instrument: PrepCon Labomatic HPLC; Column: YMC Cellulose SB 5µ, 250x30; eluent A: hexane + 0.1 vol % diethylamine; eluent B: ethanol + 0.1 vol % diethylamine; isocratic: 50%A+50%B; flow: 30 ml/min; temperature: 25°C; UV: 280 nm

### Analytical chiral HPLC method: NPB

Instrument: Waters Alliance 2695; Column: YMC Cellulose SB 3µ, 100x4.6; eluent A: hexane + 0.1 vol % diethylamine; eluent B: ethanol; isocratic: 50%A+50%B; flow: 1.4 ml/min; temperature: 25°C; UV: 280 nm
Analytical chiral HPLC: Rₜ = 2.43 min.
¹H-NMR (400 MHz, DMSO-d₆) δ [ppm]: 0.816 (0.64), 0.824 (0.45), 0.833 (1.22), 0.838 (0.75), 0.854 (0.92), 0.955 (0.44), 1.109 (5.44), 1.174 (0.54), 1.232 (6.54), 1.247 (6.24), 1.397 (0.70), 1.989 (0.60), 2.571 (1.01), 2.597 (1.01), 2.611 (1.15), 2.637 (1.13), 2.889 (1.04), 2.902 (1.13), 2.929 (0.90), 2.943 (0.85), 3.402 (1.17), 3.428 (1.67), 3.456 (1.32), 3.501 (0.53), 3.524 (1.20), 3.530 (1.22), 3.554 (0.83), 3.561 (0.75), 3.693 (1.23), 3.704 (1.83), 3.714 (1.57), 3.721 (1.61), 3.727 (1.49), 3.742 (1.03), 3.766 (0.56), 3.780 (0.83), 3.809 (2.04), 3.833 (1.37), 3.838 (1.39), 3.876 (16.00), 3.895 (1.33), 4.007 (0.85), 4.016 (0.98), 4.024 (0.94), 4.032 (0.80), 4.038 (0.76), 4.143 (0.92), 4.159 (0.82), 4.169 (1.54), 4.184 (1.31), 4.194 (0.72), 4.229 (1.50), 4.239 (1.48), 4.255 (0.92), 4.265 (0.80), 6.137 (1.45), 6.147 (1.59), 6.151 (1.61), 6.161 (1.51), 6.673 (5.82), 6.683 (3.28), 6.687 (3.06), 7.115 (2.94), 7.278 (1.83), 7.290 (1.86), 7.498 (4.15), 8.036 (1.21), 8.047 (1.16), 8.398 (1.75), 11.048 (2.49).

### Example 26

### (6R)-3-(3-chloro-2-methoxy-anilino)-2-[3-(1,4-dioxan-2-ylmethoxy)-4-pyridyl]-6-methyl-1,5,6,7-tetrahydropyrrolo[3,2-c]pyridin-4-one (stereoisomer 2)

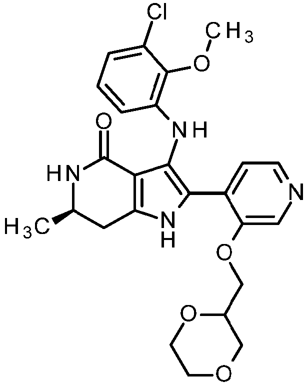

For the preparation of the title compound as mixture of two stereoisomers see example 24. Separation of enantiomers by preparative chiral HPLC (method see example 25) to give 30.0 mg (100% purity, 15% yield) of the title compound (enantiomer 2, Rₜ = 16.3 - 19.8 min).

Analytical chiral HPLC (method see example 25): Rₜ = 3.24 min.

¹H-NMR (400 MHz, DMSO-d₆) δ [ppm]: 0.750 (0.47), 0.768 (0.49), 1.110 (16.00), 1.158 (1.54), 1.235 (2.70), 1.251 (2.39), 2.525 (0.99), 2.613 (0.42), 2.639 (0.42), 2.895 (0.42), 3.393 (0.45), 3.418 (0.58), 3.422 (0.58), 3.446 (0.51), 3.516 (0.42), 3.521 (0.43), 3.712 (0.56), 3.745 (0.71), 3.774 (0.43), 3.780 (0.48), 3.810 (0.64), 3.816 (0.60), 3.839 (0.46), 3.845 (0.45), 3.881 (6.95), 4.104 (0.46), 4.130 (0.55), 4.195 (1.58), 4.291 (0.50), 4.301 (0.50), 4.317 (0.41), 6.138 (0.60), 6.151 (0.70), 6.162 (0.62), 6.678 (2.37), 6.688 (1.36), 6.691 (1.28), 7.122 (1.08), 7.274 (0.88), 7.287 (0.88), 7.514 (1.63), 8.032 (0.70), 8.045 (0.66), 8.394 (1.13), 11.040 (0.89).

### Example 27

### (6R)-3-(3-chloro-2-methoxy-anilino)-6-methyl-2-[3-(tetrahydropyran-2-ylmethoxy)-4-pyridyl]-1,5,6,7-tetrahydropyrrolo[3,2-c]pyridin-4-one

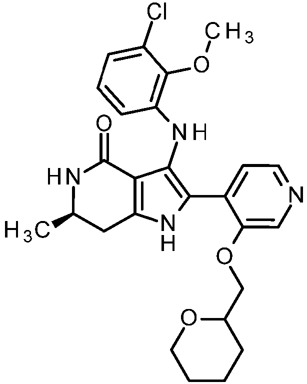

According to the method described for example 1 using (2R)-N-(3-chloro-2-methoxyphenyl)-2-methyl-6-oxo-4-{[3-(tetrahydropyran-2-ylmethoxy)-4-pyridyl]methylamino}-2,3-dihydro-1H-pyridine-5-carbothioamide (intermediate 6-27, 240 mg, 452 µmol) as starting material, 129.0 mg (95% purity, 55% yield) of the title compound were prepared after purification by preparative HPLC (method 10, gradient: 0.00-0.50 min 30% B, 0.50-7.00 min 30-70% B).

LC-MS (method 2): Rₜ = 1.22 min; MS (ESlneg): m/z = 495 [M-H]⁻

¹H-NMR (400 MHz, DMSO-d₆) δ [ppm]: 1.21 - 1.29 (dd, 3H), 1.29 - 1.43 (m, 1H), 1.47 - 1.60 (m, 3H), 1.60 - 1.70 (m, 1H), 1.80 - 1.89 (m, 1H), 2.56 - 2.66 (m, 1H), 2.86 - 2.95 (m, 1H), 3.47 - 3.61 (m, 1H), 3.75 - 3.86 (m, 2H), 3.87 - 3.92 (d, 3H), 4.01 - 4.13 (m, 2H), 4.28 - 4.43 (m, 1H), 6.15 (t, 1H), 6.67 - 6.72 (m, 2H), 7.14 (d, 1H), 7.28 (dd, 1H), 7.55 (d, 1H), 8.01 (dd, 1H), 8.41 (d, 1H), 11.04 (d, 1H).

### Example 28

### (6R)-3-(3-chloro-2-methoxy-anilino)-6-methyl-2-[3-(tetrahydropyran-2-ylmethoxy)-4-pyridyl]-1,5,6,7-tetrahydropyrrolo[3,2-c]pyridin-4-one (stereoisomer 1)

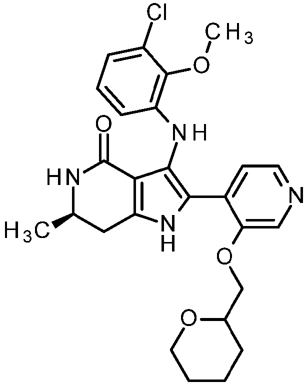

The title compound from example 27 (123 mg) was separated into enantiomers by preparative chiral HPLC, followed by triturating with hexane to give 39.5 mg (95 % purity, 17 % yield) of the title compound (enantiomer 1, Rₜ = 18.1 - 20.2 min) and enantiomer 2 (38.3 mg, Rₜ = 20.2 - 23.2 min, see example 29).

### Preparative chiral HPLC method: NPB

Instrument: PrepCon Labomatic HPLC; Column: YMC Cellulose SC 10µ, 250x50; eluent A: hexane + 0.1 vol % diethylamine; eluent B: ethanol; gradient: ; flow: 150 mL/min; temperature: 25°C; UV: 280 nm

### Analytical chiral HPLC method: NPB

Instrument: Waters Alliance 2695; Column: YMC Cellulose SC 3µ, 100x4.6; eluent A: hexane + 0.1 vol % diethylamine; eluent B: ethanol; gradient: ; flow: 1.4 ml/min; temperature: 25°C; UV: 280 nm
Analytical chiral HPLC: Rt = 6.00 min.
¹H-NMR (400 MHz, DMSO-d₆) δ [ppm]: 1.227 (5.37), 1.244 (5.36), 1.342 (0.46), 1.371 (0.59), 1.545 (2.02), 1.617 (0.78), 1.649 (0.65), 1.832 (0.71), 2.522 (0.87), 2.578 (0.81), 2.603 (0.81), 2.618 (0.97), 2.644 (1.00), 2.888 (0.90), 2.901 (0.98), 2.928 (0.80), 2.941 (0.72), 3.495 (0.48), 3.522 (0.83), 3.547 (0.41), 3.779 (0.96), 3.793 (1.28), 3.805 (0.99), 3.819 (0.80), 3.888 (16.00), 4.047 (0.84), 4.074 (1.70), 4.092 (0.99), 4.100 (1.20), 4.118 (1.01), 4.291 (1.12), 4.298 (1.16), 4.317 (0.96), 4.324 (0.88), 6.141 (1.49), 6.153 (2.22), 6.166 (1.55), 6.676 (0.47), 6.686 (5.52), 6.696 (3.67), 6.698 (3.51), 7.131 (2.44), 7.275 (1.89), 7.287 (1.89), 7.534 (3.95), 8.008 (1.26), 8.021 (1.19), 8.412 (1.94), 11.049 (2.10).

### Example 29

### (6R)-3-(3-chloro-2-methoxy-anilino)-6-methyl-2-[3-(tetrahydropyran-2-ylmethoxy)-4-pyridyl]-1,5,6,7-tetrahydropyrrolo[3,2-c]pyridin-4-one (stereoisomer 2)

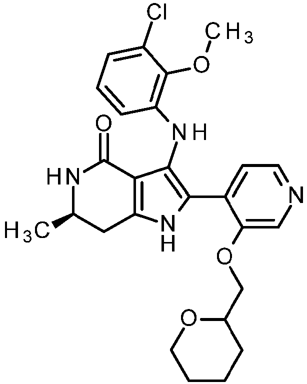

For the preparation of the title compound as mixture of two stereoisomers see example 27. Separation of enantiomers by preparative chiral HPLC (method see example 28) followed by stirring with hexane, filtering off and drying to give 38.3 mg (95% purity, 16% yield) of the title compound (enantiomer 2, Rₜ = 20.2 - 23.2 min).

Analytical chiral HPLC (method see example 28): Rₜ = 6.78 min.

¹H-NMR (400 MHz, DMSO-d₆) δ [ppm]: 1.232 (4.97), 1.249 (4.87), 1.325 (0.41), 1.354 (0.52), 1.515 (0.76), 1.543 (1.35), 1.631 (0.67), 1.662 (0.57), 1.829 (0.59), 2.518 (0.95), 2.522 (0.62), 2.579 (0.71), 2.606 (0.74), 2.619 (0.89), 2.646 (0.90), 2.873 (0.78), 2.886 (0.91), 2.913 (0.70), 2.926 (0.66), 3.539 (0.46), 3.567 (0.82), 3.780 (0.58), 3.795 (0.91), 3.810 (0.81), 3.822 (0.89), 3.839 (0.43), 3.845 (0.47), 3.891 (16.00), 4.026 (0.92), 4.047 (1.35), 4.052 (1.72), 4.071 (1.26), 4.360 (0.97), 4.368 (1.02), 4.385 (0.90), 4.393 (0.83), 6.143 (1.44), 6.155 (2.45), 6.167 (1.45), 6.691 (4.16), 6.702 (4.21), 7.141 (2.29), 7.269 (1.67), 7.282 (1.70), 7.557 (3.62), 8.003 (1.06), 8.015 (1.02), 8.404 (1.63), 11.038 (1.89).

### Example 30

### 3-(3-chloro-2-methoxy-anilino)-7-methyl-2-(3-{[(2S)-tetrahydrofuran-2-yl]methoxy}-4-pyridyl)-1,5,6,7-tetrahydropyrrolo[3,2-c]pyridin-4-one

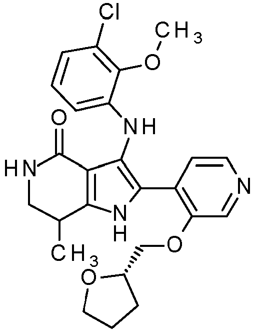

According to the method described for example 1 using *N*-(3-chloro-2-methoxy-phenyl)-3-methyl-6-oxo-4-[(3-{[(2*S*)-tetrahydrofuran-2-yl]methoxy}-4-pyridyl)methylamino]-2,3-dihydro-1H-pyridine-5-carbothioamide (intermediate 6-30, 198 mg, 383 µmol) as starting material, 108.0 mg (85% purity, 50% yield) of the title compound were prepared after purification by preparative HPLC (method 9, gradient: 0.00-0.50 min 15% B, 0.50-5.99 min 15-54.6% B, 5.99-6.59 min 54.6-55% B).

LC-MS (method 1): Rₜ = 0.92 min; MS (ESlpos): m/z = 483 [M+H]⁺

¹H-NMR (400 MHz, DMSO-d₆) δ [ppm]: 1.272 (1.93), 1.282 (2.54), 1.287 (2.74), 1.297 (2.07), 1.685 (0.44), 1.693 (0.43), 1.708 (0.44), 1.857 (0.84), 1.874 (1.31), 1.890 (0.97), 1.904 (0.60), 1.909 (0.56), 2.018 (0.43), 2.027 (0.40), 2.047 (0.64), 2.086 (0.51), 2.520 (1.59), 2.525 (1.07), 3.081 (1.06), 3.091 (1.00), 3.102 (1.08), 3.446 (0.42), 3.456 (0.55), 3.463 (0.58), 3.552 (0.86), 3.562 (0.91), 3.672 (1.39), 3.716 (0.94), 3.740 (0.59), 3.759 (0.89), 3.775 (0.73), 3.815 (0.58), 3.832 (1.24), 3.851 (0.95), 3.870 (16.00), 3.884 (0.44), 4.002 (0.55), 4.007 (0.55), 4.027 (0.50), 4.033 (0.58), 4.038 (0.52), 4.304 (0.41), 4.313 (0.55), 4.330 (1.08), 4.341 (0.59), 4.357 (0.98), 4.381 (0.48), 6.140 (1.32), 6.150 (1.20), 6.154 (1.19), 6.164 (1.30), 6.655 (0.46), 6.666 (5.66), 6.675 (2.54), 6.679 (2.44), 7.148 (0.75), 7.161 (0.67), 7.266 (1.60), 7.276 (1.58), 7.502 (1.76), 7.512 (1.81), 8.027 (2.59), 8.040 (2.39), 8.414 (2.73), 11.028 (0.82), 11.071 (0.81).

### Example 31

### 3-(3-chloro-2-methoxy-anilino)-7-methyl-2-(3-{[(2S)-tetrahydrofuran-2-yl]methoxy}-4-pyridyl)-1,5,6,7-tetrahydropyrrolo[3,2-c]pyridin-4-one (stereoisomer 1)

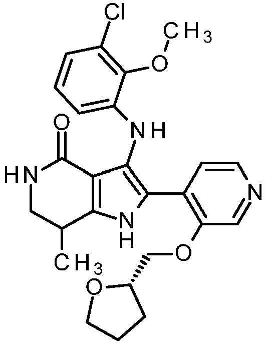

The title compound from example 31 (105 mg) was separated into enantiomers by preparative chiral HPLC, followed by trituration with hexane to give 31.5 mg (95% purity, 33% yield) of the title compound (enantiomer 1, Rₜ = 5.2 - 5.8 min) and enantiomer 2 (32.9 mg, Rₜ = 6.1 - 7.1 min, see example 32).

### Preparative chiral HPLC method: MTBE

Instrument: PrepCon Labomatic HPLC; Column: YMC Cellulose SB 5µ, 250x30; eluent A: methyl tert-butyl ether + 0.1 vol % diethylamine; eluent B: acetonitrile + 0.1 vol % diethylamine; isocratic: 80%A+20%B; flow: 50 ml/min; temperature: 25°C; UV: 254 nm

### Analytical chiral HPLC method: MTBE

Instrument: Waters Alliance 2695; Column: YMC Cellulose SB 3µ, 100x4.6; eluent A: methyl tert-butyl ether + 0.1 vol % diethylamine; eluent B: acetonitrile; isocratic: 80%A+20%B; flow: 1.4 ml/min; temperature: 25°C; UV: 254 nm
Analytical chiral HPLC: Rₜ = 2.25 min.
¹H-NMR (400 MHz, DMSO-d₆) δ [ppm]: 1.279 (4.44), 1.294 (4.51), 1.663 (0.45), 1.681 (0.58), 1.690 (0.64), 1.695 (0.58), 1.712 (0.67), 1.838 (0.48), 1.855 (1.60), 1.871 (2.27), 1.889 (1.55), 1.907 (0.60), 1.997 (0.55), 2.012 (0.62), 2.026 (0.67), 2.043 (0.54), 3.081 (1.21), 3.088 (1.48), 3.100 (1.91), 3.419 (0.46), 3.436 (0.82), 3.444 (0.97), 3.452 (0.89), 3.459 (0.77), 3.720 (0.49), 3.738 (1.17), 3.757 (1.59), 3.774 (0.72), 3.811 (0.76), 3.828 (1.69), 3.848 (1.34), 3.867 (16.00), 4.012 (0.64), 4.030 (1.24), 4.035 (1.22), 4.053 (0.82), 4.301 (0.68), 4.309 (0.99), 4.326 (2.43), 4.350 (1.24), 4.359 (0.89), 6.136 (1.43), 6.147 (1.47), 6.150 (1.52), 6.160 (1.48), 6.653 (0.55), 6.663 (5.92), 6.673 (3.15), 6.677 (2.91), 7.159 (1.72), 7.263 (2.67), 7.275 (2.68), 7.508 (3.92), 8.024 (2.75), 8.036 (2.62), 8.412 (4.31), 11.027 (2.16).

### Example 32

### 3-(3-chloro-2-methoxy-anilino)-7-methyl-2-(3-{[(2S)-tetrahydrofuran-2-yl]methoxy}-4-pyridyl)-1,5,6,7-tetrahydropyrrolo[3,2-c]pyridin-4-one (stereoisomer 2)

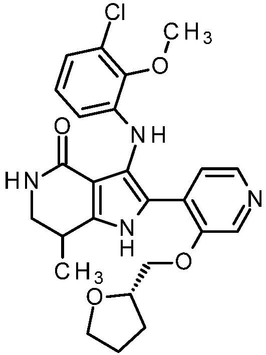

For the preparation of the title compound as mixture of two stereoisomers see example 30. Separation of enantiomers by preparative chiral HPLC (method see example 31) followed by trituration with hexane gave 32.9 mg (95% purity, 35% yield) of the title compound (enantiomer 2, Rₜ = 6.1 - 7.1 min).

Analytical chiral HPLC (method see example 31): Rₜ = 2.79 min.

¹H-NMR (400 MHz, DMSO-d₆) δ [ppm]: 1.268 (4.71), 1.284 (4.77), 1.656 (0.50), 1.674 (0.63), 1.683 (0.69), 1.704 (0.75), 1.854 (0.50), 1.869 (1.19), 1.884 (1.82), 1.901 (1.44), 1.917 (0.50), 2.002 (0.63), 2.016 (0.75), 2.034 (0.69), 2.048 (0.56), 2.326 (0.94), 2.668 (0.94), 3.079 (1.88), 3.090 (1.51), 3.103 (1.32), 3.440 (0.50), 3.454 (1.00), 3.461 (1.00), 3.471 (0.88), 3.734 (0.50), 3.753 (1.19), 3.771 (1.63), 3.788 (0.75), 3.812 (0.82), 3.829 (1.76), 3.849 (1.44), 3.866 (16.00), 3.979 (0.82), 4.000 (1.38), 4.003 (1.32), 4.023 (1.00), 4.319 (0.94), 4.337 (1.00), 4.354 (2.01), 4.378 (1.19), 4.386 (0.94), 6.136 (1.44), 6.147 (1.51), 6.151 (1.51), 6.160 (1.51), 6.652 (0.63), 6.662 (5.90), 6.672 (3.14), 6.677 (2.82), 7.143 (2.01), 7.260 (2.64), 7.272 (2.70), 7.497 (4.02), 8.023 (2.76), 8.036 (2.64), 8.409 (4.45), 11.071 (2.26).

### Example 33

### 3-(3-chloro-2-methoxy-anilino)-2-[3-(1,4-dioxan-2-ylmethoxy)-4-pyridyl]-7-methyl-1,5,6,7-tetrahydropyrrolo[3,2-c]pyridin-4-one

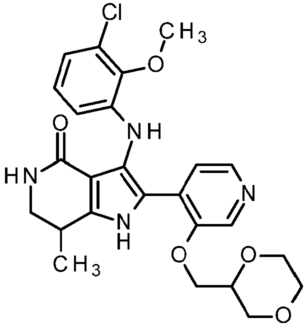

According to the method described for example 1 using *N*-(3-chloro-2-methoxy-phenyl)-4-{[3-(1,4-dioxan-2-ylmethoxy)-4-pyridyl]methylamino}-3-methyl-6-oxo-2,3-dihydro-1*H-*pyridine-5-carbothioamide (Intermediate 6-33, 75.0 mg, 141 µmol) as starting material, 17.1 mg (95% purity, 23% yield) of the title compound were prepared after purification by preparative HPLC (method 9, gradient: 0.00-0.50 min 15% B, 0.50-5.99 min 15-54.6% B, 5.99-6.59 min 54.6-55% B).

LC-MS (method 1): Rₜ = 0.86 min; MS (ESlpos): m/z = 499 [M+H]⁺

¹H-NMR (400 MHz, DMSO-d⁶) δ [ppm]: 1.234 (0.48), 1.273 (5.62), 1.289 (5.73), 2.047 (0.45), 2.198 (0.41), 2.525 (2.92), 2.542 (2.79), 3.086 (2.24), 3.102 (2.07), 3.375 (1.18), 3.400 (1.12), 3.404 (1.08), 3.414 (0.90), 3.428 (1.05), 3.443 (1.14), 3.467 (1.75), 3.481 (1.27), 3.508 (1.40), 3.529 (0.79), 3.540 (0.72), 3.656 (0.92), 3.685 (2.85), 3.715 (2.65), 3.801 (1.76), 3.809 (1.99), 3.821 (1.44), 3.830 (1.50), 3.844 (1.65), 3.851 (1.51), 3.866 (16.00), 4.002 (0.73), 4.045 (0.45), 4.095 (0.69), 4.113 (0.49), 4.121 (0.89), 4.139 (0.73), 4.188 (1.77), 4.203 (1.94), 4.225 (0.86), 4.235 (0.78), 4.251 (0.61), 4.260 (0.53), 6.117 (1.11), 6.121 (1.24), 6.131 (1.90), 6.136 (1.18), 6.141 (1.17), 6.145 (1.16), 6.660 (6.88), 6.670 (3.50), 6.674 (3.42), 7.145 (1.82), 7.261 (3.47), 7.273 (3.45), 7.503 (2.77), 7.518 (2.56), 8.042 (2.09), 8.046 (2.21), 8.054 (2.03), 8.059 (2.01), 8.396 (3.07), 8.402 (3.17), 10.938 (1.50), 10.957 (1.37).

### Example 34

### 3-(3-chloro-2-methoxy-anilino)-2-[3-(1,4-dioxan-2-ylmethoxy)-4-pyridyl]-7-methyl-1,5,6,7-tetrahydropyrrolo[3,2-c]pyridin-4-one (stereoisomer 1)

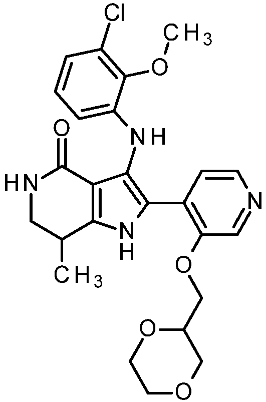

The title compound from example 33 (15 mg) was separated into enantiomers by preparative chiral HPLC to give 4.9 mg (90% purity, 33% yield) of the title compound (enantiomer 1, Rₜ = 11.7 - 12.5 min), enantiomer 2 (4.3 mg, Rₜ = 12.6 - 13.9 min, see example 35), enantiomer 3 (5.0 mg, Rₜ = 19.5 - 21.7 min, see example 36) and enantiomer 4 (5.4 mg, Rₜ = 25.0 - 27.8 min, see example 37).

### Preparative chiral HPLC method: MTBE

Instrument: PrepCon Labomatic HPLC; Column: YMC Cellulose SB 5µ, 250x30; eluent A: methyl tert-butyl ether + 0.1 vol % diethylamine; eluent B: acetonitrile + 0.1 vol % diethylamine; gradient: 0-10 min 10-20% B; flow: 50 ml/min; temperature: 25°C; UV: 254 nm

### Analytical chiral HPLC method: MTBE

Instrument: Waters Alliance 2695; Column: YMC Cellulose SB 3µ, 100x4.6; eluent A: methyl tert-butyl ether + 0.1 vol % diethylamine; eluent B: acetonitrile; isocratic: 80%A+20%B; flow: 1.4 ml/min; temperature: 25°C; UV: 254 nm
Analytical chiral HPLC: Rₜ = 2.72 min.
Optical rotation:[α]_{D} = 12.7 ° +/- 0.88° (c = 5.7 mg/ml in CHLOROFORM)
¹H-NMR (400 MHz, CDCl₃) δ [ppm]: 0.849 (0.57), 0.860 (0.63), 0.887 (0.77), 1.263 (4.64), 1.388 (5.26), 1.405 (5.34), 2.187 (0.58), 3.133 (0.56), 3.148 (0.58), 3.262 (0.49), 3.268 (0.51), 3.287 (0.46), 3.292 (0.63), 3.297 (0.56), 3.311 (0.45), 3.317 (0.44), 3.498 (1.61), 3.625 (0.50), 3.632 (0.56), 3.642 (1.01), 3.654 (0.55), 3.665 (1.08), 3.670 (1.22), 3.694 (1.11), 3.704 (0.43), 3.722 (0.49), 3.726 (0.63), 3.729 (0.64), 3.734 (0.64), 3.751 (0.66), 3.758 (0.63), 3.844 (1.03), 3.850 (0.76), 3.881 (1.00), 3.910 (0.92), 3.917 (1.33), 3.924 (1.04), 3.934 (0.77), 3.941 (0.69), 3.948 (0.92), 3.954 (1.40), 3.960 (1.05), 4.087 (16.00), 4.188 (0.71), 4.195 (0.63), 4.205 (0.42), 4.212 (0.67), 4.222 (1.06), 4.246 (1.39), 4.263 (0.88), 4.307 (1.16), 4.314 (1.17), 4.331 (0.74), 4.338 (0.56), 5.349 (1.02), 6.223 (1.16), 6.227 (1.23), 6.244 (1.34), 6.247 (1.33), 6.622 (0.99), 6.642 (2.21), 6.662 (1.29), 6.728 (1.72), 6.732 (1.83), 6.748 (1.22), 6.751 (1.11), 7.373 (0.60), 7.384 (0.60), 7.611 (2.18), 8.294 (0.40), 10.413 (0.71).

### Example 35

### 3-(3-chloro-2-methoxy-anilino)-2-[3-(1,4-dioxan-2-ylmethoxy)-4-pyridyl]-7-methyl-1,5,6,7-tetrahydropyrrolo[3,2-c]pyridin-4-one (stereoisomer 2)

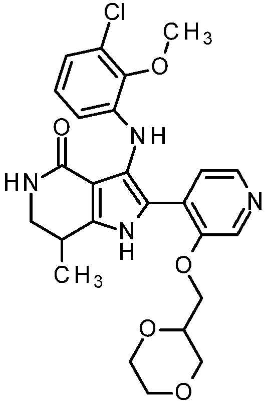

For the preparation of the title compound as mixture of stereoisomers see example 33. Separation of enantiomers by preparative chiral HPLC (method see example 34) to give 4.3 mg (90% purity, 29% yield) of the title compound (enantiomer 2, Rₜ = 12.6 - 13.9 min).

Analytical chiral HPLC (method see example 34): Rₜ = 2.99 min.

Optical rotation:[α]_{D} = 41.0° +/- 0.68° (c = 4.7 mg/ml in CHLOROFORM)

¹H-NMR (400 MHz, CDCl₃) δ [ppm]: 0.849 (0.65), 0.860 (0.74), 0.887 (0.92), 0.903 (0.46), 1.264 (5.29), 1.292 (0.73), 1.327 (0.41), 1.346 (0.41), 1.381 (5.08), 1.398 (5.05), 2.187 (0.56), 3.111 (0.62), 3.125 (0.62), 3.257 (0.46), 3.264 (0.49), 3.280 (0.42), 3.287 (0.56), 3.294 (0.51), 3.304 (0.44), 3.311 (0.42), 3.498 (1.06), 3.589 (0.75), 3.612 (0.91), 3.618 (0.87), 3.640 (0.92), 3.657 (0.49), 3.663 (0.54), 3.670 (0.53), 3.677 (0.53), 3.687 (0.47), 3.693 (0.47), 3.700 (0.43), 3.706 (0.42), 3.737 (0.43), 3.742 (0.59), 3.746 (0.57), 3.751 (0.60), 3.767 (0.64), 3.775 (0.64), 3.843 (1.02), 3.848 (0.71), 3.872 (0.60), 3.922 (0.94), 3.929 (1.50), 3.934 (1.06), 3.947 (0.81), 3.958 (1.44), 3.963 (1.67), 3.979 (0.73), 4.086 (16.00), 4.146 (0.54), 4.166 (1.19), 4.189 (1.34), 4.198 (0.71), 4.203 (0.43), 4.220 (0.72), 4.362 (0.85), 4.367 (1.09), 4.385 (0.93), 4.390 (0.72), 5.329 (0.98), 6.222 (1.13), 6.225 (1.21), 6.242 (1.28), 6.245 (1.29), 6.619 (1.00), 6.639 (2.24), 6.660 (1.30), 6.725 (1.70), 6.729 (1.84), 6.746 (1.24), 6.750 (1.15), 7.373 (0.80), 7.385 (0.86), 7.619 (2.15), 8.046 (0.49), 8.295 (0.68), 10.464 (0.69).

### Example 36

### 3-(3-chloro-2-methoxy-anilino)-2-[3-(1,4-dioxan-2-ylmethoxy)-4-pyridyl]-7-methyl-1,5,6,7-tetrahydropyrrolo[3,2-c]pyridin-4-one (stereoisomer 3)

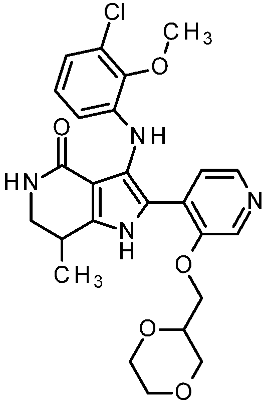

For the preparation of the title compound as mixture of stereoisomers see example 33. Separation of enantiomers by preparative chiral HPLC (method see example 34) to give 5.0 mg (90% purity, 17% yield) of the title compound (enantiomer 3, Rₜ = 19.5 - 21.7 min).

Analytical chiral HPLC (method see example 34): Rₜ = 4.66 min.

Optical rotation:[α]_{D} = - 41.4 ° +/- 0.72° (c = 5.7 mg/ml in chloroform)

¹H-NMR (400 MHz, CDCl₃) δ [ppm]: 0.842 (0.69), 0.849 (0.82), 0.860 (0.91), 0.869 (0.77), 0.876 (0.70), 0.887 (1.14), 0.903 (0.61), 1.210 (0.74), 1.226 (0.88), 1.263 (6.12), 1.281 (1.13), 1.292 (0.92), 1.309 (0.72), 1.326 (0.68), 1.344 (0.45), 1.379 (5.08), 1.396 (5.07), 2.131 (0.70), 2.187 (0.52), 3.109 (0.60), 3.123 (0.62), 3.259 (0.44), 3.266 (0.47), 3.283 (0.41), 3.289 (0.53), 3.296 (0.50), 3.306 (0.43), 3.313 (0.42), 3.589 (0.76), 3.612 (0.90), 3.618 (0.87), 3.640 (0.92), 3.658 (0.49), 3.664 (0.53), 3.671 (0.52), 3.678 (0.51), 3.689 (0.46), 3.694 (0.46), 3.702 (0.43), 3.708 (0.42), 3.737 (0.45), 3.742 (0.59), 3.746 (0.57), 3.751 (0.65), 3.767 (0.64), 3.775 (0.65), 3.839 (1.61), 3.843 (1.12), 3.848 (0.73), 3.872 (0.58), 3.922 (0.95), 3.929 (1.42), 3.933 (1.04), 3.947 (0.79), 3.957 (1.39), 3.963 (1.64), 3.971 (0.72), 4.086 (16.00), 4.145 (0.54), 4.165 (1.20), 4.188 (1.34), 4.198 (0.72), 4.203 (0.47), 4.214 (0.47), 4.220 (0.72), 4.362 (0.85), 4.367 (1.06), 4.384 (0.94), 4.390 (0.74), 5.454 (0.93), 6.224 (1.15), 6.227 (1.18), 6.244 (1.31), 6.248 (1.25), 6.620 (0.99), 6.641 (2.19), 6.660 (1.29), 6.726 (1.71), 6.730 (1.75), 6.746 (1.21), 6.750 (1.12), 7.373 (0.95), 7.386 (0.99), 7.616 (2.14), 8.047 (0.55), 8.058 (0.54), 8.294 (0.77), 10.465 (0.69).

### Example 37

### 3-(3-chloro-2-methoxy-anilino)-2-[3-(1,4-dioxan-2-ylmethoxy)-4-pyridyl]-7-methyl-1,5,6,7-tetrahydropyrrolo[3,2-c]pyridin-4-one (stereoisomer 4)

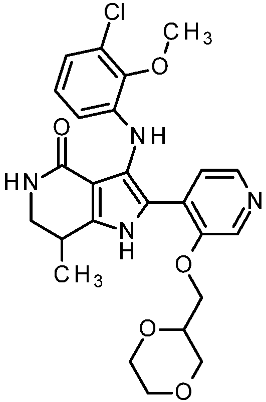

For the preparation of the title compound as mixture of stereoisomers see example 33. Separation of enantiomers by preparative chiral HPLC (method see example 34) to give 5.4 mg (90% purity, 36% yield) of the title compound (enantiomer 4, Rₜ = 25.0 - 27.8 min).

Analytical chiral HPLC (method see example 46): Rₜ = 6.80 min.

Optical rotation:[α]_{D} = 1.8 ° +/- 0.56° (c = 5.7 mg/ml in chloroform)

¹H-NMR (400 MHz, CDCl₃) δ [ppm]: 0.842 (0.88), 0.849 (1.04), 0.860 (1.18), 0.869 (1.00), 0.876 (0.91), 0.887 (1.48), 0.903 (0.81), 0.928 (0.54), 0.945 (0.48), 1.263 (7.94), 1.281 (1.53), 1.291 (1.28), 1.311 (1.03), 1.326 (0.72), 1.355 (0.48), 1.388 (5.36), 1.405 (5.48), 2.017 (0.47), 2.187 (0.61), 3.132 (0.56), 3.147 (0.58), 3.263 (0.48), 3.268 (0.50), 3.288 (0.46), 3.292 (0.62), 3.299 (0.56), 3.312 (0.46), 3.318 (0.45), 3.497 (4.25), 3.625 (0.48), 3.632 (0.55), 3.642 (1.01), 3.655 (0.53), 3.665 (1.07), 3.670 (1.27), 3.694 (1.14), 3.704 (0.44), 3.721 (0.50), 3.725 (0.62), 3.729 (0.62), 3.733 (0.63), 3.751 (0.66), 3.758 (0.64), 3.844 (1.03), 3.850 (0.77), 3.875 (0.59), 3.880 (0.97), 3.910 (0.89), 3.917 (1.38), 3.924 (1.03), 3.934 (0.78), 3.941 (0.66), 3.947 (0.88), 3.954 (1.40), 3.960 (1.06), 3.967 (0.75), 3.989 (0.41), 4.086 (16.00), 4.188 (0.73), 4.195 (0.60), 4.211 (0.70), 4.221 (1.14), 4.239 (0.40), 4.245 (1.50), 4.262 (1.05), 4.306 (1.22), 4.313 (1.25), 4.330 (0.79), 4.337 (0.60), 5.409 (1.00), 6.225 (1.17), 6.228 (1.24), 6.245 (1.33), 6.248 (1.32), 6.622 (1.03), 6.642 (2.31), 6.662 (1.35), 6.727 (1.68), 6.730 (1.83), 6.747 (1.25), 6.751 (1.18), 7.369 (1.16), 7.381 (1.20), 7.604 (2.21), 8.047 (0.67), 8.059 (0.67), 8.290 (0.99), 10.413 (0.71).

### Example 38

### 3-(3-chloro-2-methoxy-anilino)-2-(3-{[(2S)-tetrahydrofuran-2-yl]methoxy}-4-pyridyl)-6-(trifluoromethyl)-1,5,6,7-tetrahydropyrrolo[3,2-c]pyridin-4-one

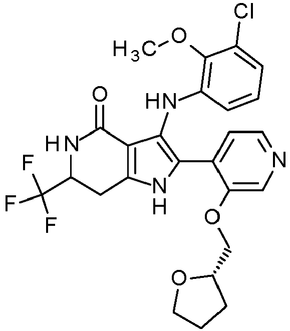

*N*-(3-Chloro-2-methoxy-phenyl)-6-oxo-4-[(3-{[(2*S*)-tetrahydrofuran-2-yl]methoxy}-4-pyridyl)methylamino]-2-(trifluoromethyl)-2,3-dihydro-1*H*-pyridine-5-carbothioamide (Intermediate 6-38, 135 mg, 236 µmol) was dissolved in methanol (2.7 ml), TFA (36 µl, 470 µmol) was added and the mixture was stirred fo 5 min at RT. mCPBA (106 mg, 77 % purity, 473 µmol) was added and the mixture was stirred for 3 h at 50°C. The mixture was diluted with half sat. sodium bicarbonate solution, extracted with DCM and the organic layer was dried over sodiumsulfate and evaporated. The residue was purified by two flash chromatographies (first: silica, DCM / EtOH gradient 0-10%, second: (amino phase silica, DCM / EtOH gradient 0-15 %) to give 110 mg (90% purity, 78 % yield) of the title compound.

LC-MS (method 2): Rₜ = 1.16 min; MS (ESlpos): m/z = 537 [M+H]⁺

¹H-NMR (400 MHz, DMSO-d6) δ [ppm]: 1.62 - 1.78 (m, 1 H), 1.85 (quin, 2 H), 1.94 - 2.04 (m, 1 H), 3.02 (br d, 1 H), 3.37 - 3.49 (m, 1 H), 3.65 - 3.89 (m, 5 H), 4.04 - 4.12 (m, 1 H), 4.25 - 4.49 (m, 3 H), 6.13 (dt, 1 H), 6.60 - 6.72 (m, 2 H), 7.28 (d, 1 H), 7.31 - 7.35 (m, 1 H), 7.83 (t, 1 H), 8.06 (dd, 1 H), 8.44 (s, 1 H), 11.38 (d, 1 H).

### Example 39

### 3-(3-chloro-2-methoxy-anilino)-2-(3-{[(2S)-tetrahydrofuran-2-yl]methoxy}-4-pyridyl)-6-(trifluoromethyl)-1,5,6,7-tetrahydropyrrolo[3,2-c]pyridin-4-one (stereoisomer 1)

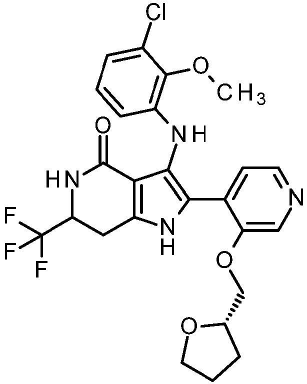

The title compound from example 38 (110 mg) was separated into enantiomers by preparative chiral HPLC to give 60.0 mg (90% purity, 50% yield) of the title compound (enantiomer 1, Rₜ = 4.9 - 6.1 min) and enantiomer 2 (55.0 mg, Rₜ = 9.4 - 10.9 min, see example 40).

### Preparative chiral HPLC method: NPB

Instrument: PrepCon Labomatic HPLC; Column: YMC Amylose SA 5µ, 250x30; eluent A: hexane + 0.1 vol % diethylamine; eluent B: ethanol + 0.1 vol % diethylamine; isocratic: 50%A+50%B; flow: 50 ml/min; temperature: 25°C; UV: 254 nm

### Analytical chiral HPLC method: NPB

Instrument: Waters Alliance 2695; Column: YMC Amylose SA 3µ, 100x4.6; eluent A: hexane + 0.1 vol % diethylamine; eluent B: ethanol; isocratic: 50% A + 50% B; flow: 1.4 ml/min; temperature: 25°C; UV: 254 nm
Analytical chiral HPLC: Rₜ = 1.46 min.
Optical rotation:[α]_{D} = 3.9 ° +/- 1.50° (c = 1.4 mg/ml in DMSO)
LC-MS (method 2): Rₜ = 1.14 min; MS (ESlpos): m/z = 537 [M+H]⁺
¹H-NMR (400 MHz, DMSO-d₆) δ [ppm]: 1.64 - 1.76 (m, 1 H), 1.79 - 1.92 (m, 2 H), 1.94 - 2.07 (m, 1 H), 2.96 - 3.09 (m, 1 H), 3.37 - 3.50 (m, 1 H), 3.68 - 3.88 (m, 5 H), 4.03 - 4.12 (m, 1 H), 4.26 - 4.36 (m, 2 H), 4.37 - 4.48 (m, 1 H), 6.13 (dd, 1 H), 6.53 - 6.77 (m, 2 H), 7.29 (s, 1 H), 7.32 (d, 1 H), 7.83 (d, 1 H), 8.06 (d, 1 H), 8.44 (s, 1 H), 11.38 (s, 1 H).

### Example 40

### 3-(3-chloro-2-methoxy-anilino)-2-(3-{[(2S)-tetrahydrofuran-2-yl]methoxy}-4-pyridyl)-6-(trifluoromethyl)-1,5,6,7-tetrahydropyrrolo[3,2-c]pyridin-4-one (stereoisomer 2)

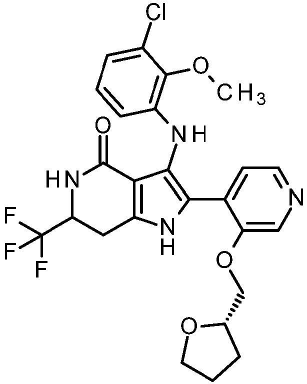

For the preparation of the title compound as mixture of two stereoisomers see example 38. Separation of enantiomers by preparative chiral HPLC (method see example 39) to give 55.0 mg (95% purity, 48% yield) of the title compound (enantiomer 2, Rₜ = 9.4 - 10.9 min).
Analytical chiral HPLC (method see example 39): Rₜ = 2.64 min.
Optical rotation:[α]_{D} = 22.4 ° +/- 1.88° (c = 1.7 mg/ml in DMSO)
LC-MS (method 2): Rₜ = 1.14 min; MS (ESlpos): m/z = 537 [M+H]⁺
¹H-NMR (400 MHz, DMSO-d₆) δ [ppm]: 1.61 - 1.74 (m, 1H), 1.77 - 1.91 (m, 2H), 1.92 - 2.06 (m, 1H), 3.02 (dd, 1H), 3.41 (dd, 1H), 3.69 - 3.89 (m, 5H), 4.02 - 4.14 (m, 1H), 4.22 - 4.35 (m, 2H), 4.36 - 4.49 (m, 1H), 6.12 (dd, 1H), 6.61 - 6.71 (m, 2H), 7.28 (s, 1H), 7.34 (d, 1H), 7.82 (d, 1H), 8.06 (d, 1H), 8.44 (s, 1H), 11.37 (s, 1H).

### Example 41

### (6R)-3-(3-chloro-2-methoxy-anilino)-2-{3-[(1-methoxypropan-2-yl)oxy]-4-pyridyl}-6-methyl-1,5,6,7-tetrahydropyrrolo[3,2-c]pyridin-4-one

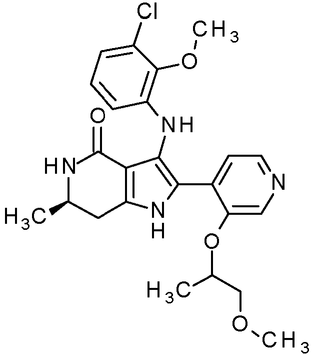

According to the method described for example 1 using (2*R*)-*N*-(3-chloro-2-methoxyphenyl)-4-{[3-(2-methoxy-1-methyl-ethoxy)-4-pyridyl]methylamino}-2-methyl-6-oxo-2,3-dihydro-1*H*-pyridine-5-carbothioamide (intermediate 6-41, 210 mg, 416 µmol) as starting material, 54.2 mg (95% purity, 26% yield) of the title compound were prepared after purification by preparative HPLC (method 10, gradient: 0.00-0.50 min 30% B, 0.50-6.00 min 30-70% B) followed by flash chromatography (silica, DCM / EtOH gradient 10-30 %).

LC-MS (method 2): Rₜ = 1.13 min; MS (ESlpos): m/z = 471 [M+H]⁺

¹H-NMR (400 MHz, DMSO-d₆) δ [ppm]: 11.04 (d, 1H), 8.38 - 8.44 (m, 1H), 7.96 - 8.06 (m, 1H), 7.43 - 7.53 (m, 1H), 7.25 - 7.33 (m, 1H), 7.01 - 7.12 (m, 1H), 6.61 - 6.72 (m, 2H), 6.10 - 6.20 (m, 1H), 4.71 - 4.86 (m, 1H), 3.84 - 3.99 (m, 3H), 3.73 - 3.84 (m, 1H), 3.49 - 3.66 (m, 2H), 2.86 - 3.00 (m, 1H), 2.55 - 2.57 (m, 1H), 2.54 - 2.61 (m, 1H), 2.11 - 2.24 (m, 1H), 1.15 - 1.46 (m, 7H).

### Example 42

### (6R)-3-(3-chloro-2-methoxy-anilino)-2-{3-[(1-methoxypropan-2-yl)oxy]-4-pyridyl}-6-methyl-1,5,6,7-tetrahydropyrrolo[3,2-c]pyridin-4-one (stereoisomer 1)

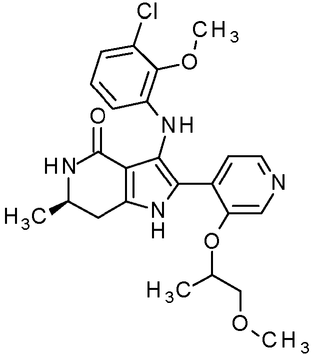

The title compound from example 41 (49.3 mg) was separated into enantiomers by preparative chiral HPLC to give 17.0 mg (95% purity, 8% yield) of the title compound (enantiomer 1, Rₜ = 13.9 - 17.5 min) and enantiomer 2 (10.0 mg, Rₜ = 18.1 - 24.3 min, see example 43).

### Preparative chiral HPLC method: MTBE

Instrument: PrepCon Labomatic HPLC; Column: YMC Amylose SA 5µ, 250x30; eluent A: methyl tert-butyl ether + 0.1 vol % diethylamine; eluent B: acetonitrile; isocratic: 80%A+20%B; flow: 60 ml/min; temperature: 25°C; UV: 254 nm

### Analytical chiral HPLC method: MTBE

Instrument: Waters Alliance 2695; Column: YMC Amylose SA 3µ, 100x4.6; eluent A: methyl tert-butyl ether + 0.1 vol % diethylamine; eluent B: acetonitrile; isocratic: 80%A+20%B; flow: 1.4 ml/min; temperature: 25°C; UV: 254 nm
Analytical chiral HPLC: Rₜ = 6.14 min.
Optical rotation:[α]_{D} = 5.58° +/- 0.27 ° (c = 4.48 mg/ml in METHANOL)

### Example 43

### (6R)-3-(3-chloro-2-methoxy-anilino)-2-{3-[(1-methoxypropan-2-yl)oxy]-4-pyridyl}-6-methyl-1,5,6,7-tetrahydropyrrolo[3,2-c]pyridin-4-one (stereoisomer 2)

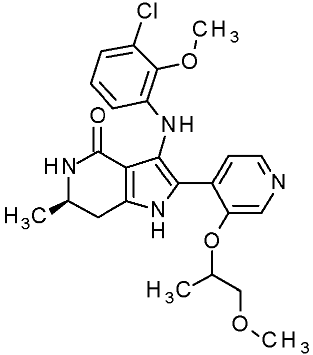

For the preparation of the title compound as mixture of two stereoisomers see example 41. Separation of enantiomers by preparative chiral HPLC (method see example 42) to give 10.0 mg (95% purity, 5% yield) of the title compound (enantiomer 2, Rₜ = 18.1 - 24.3 min).

Analytical chiral HPLC (method see example 42): Rₜ = 8.43 min.

Optical rotation:[α]_{D} = 24.04 ° +/- 0.92 ° (c = 3.55 mg/ml in METHANOL)

### Example 44

### 3-(3-chloro-2-methoxy-anilino)-7-methyl-2-[3-(tetrahydropyran-2-ylmethoxy)-4-pyridyl]-1,5,6,7-tetrahydropyrrolo[3,2-c]pyridin-4-one

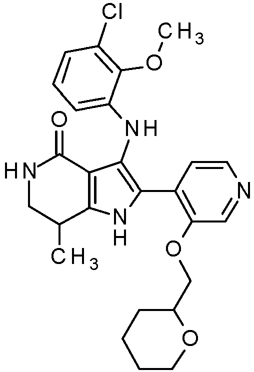

According to the method described for example 1 using *N*-(3-chloro-2-methoxy-phenyl)-3-methyl-6-oxo-4-{[3-(tetrahydropyran-2-ylmethoxy)-4-pyridyl]methylamino}-2,3-dihydro-1*H*-pyridine-5-carbothioamide (intermediate 6-44, 160 mg, 301 µmol) as starting material, 63.8 mg (90% purity, 38% yield) of the title compound were prepared after purification by preparative HPLC (method 9, gradient: 0.00-0.50 min 15% B, 0.50-5.20 min 15-48.9% B, 5.20-6.82 48.9% B, 6.82-7.63 48.9-55% B).

LC-MS (method 1): Rₜ = 0.97 min; MS (ESlpos): m/z = 497 [M+H]⁺

¹H-NMR (400 MHz, DMSO-d₆) δ [ppm]: 1.235 (0.48), 1.264 (3.69), 1.268 (4.58), 1.279 (4.68), 1.284 (4.45), 1.341 (0.55), 1.354 (0.65), 1.383 (0.61), 1.509 (1.19), 1.533 (2.97), 1.557 (1.11), 1.633 (1.10), 1.663 (0.92), 1.824 (0.96), 2.087 (1.19), 2.201 (0.65), 2.520 (4.98), 2.525 (3.30), 2.542 (0.52), 3.059 (0.47), 3.085 (1.96), 3.094 (1.85), 3.108 (1.39), 3.458 (0.52), 3.486 (1.55), 3.498 (1.40), 3.505 (1.48), 3.672 (2.47), 3.781 (1.00), 3.795 (0.83), 3.824 (0.44), 3.837 (0.54), 3.868 (1.88), 3.878 (16.00), 3.881 (14.89), 3.892 (1.46), 3.895 (1.16), 3.982 (0.84), 4.009 (0.76), 4.043 (0.76), 4.063 (0.79), 4.069 (0.91), 4.089 (0.74), 4.117 (0.68), 4.134 (0.76), 4.142 (1.05), 4.159 (1.07), 4.208 (1.02), 4.216 (1.04), 4.233 (0.66), 4.242 (0.59), 4.287 (0.81), 4.294 (0.85), 4.313 (0.69), 4.320 (0.65), 6.123 (1.42), 6.128 (1.56), 6.135 (2.09), 6.142 (1.54), 6.147 (1.45), 6.152 (1.37), 6.656 (0.49), 6.676 (8.10), 6.690 (5.52), 7.151 (1.42), 7.171 (1.29), 7.263 (1.86), 7.270 (2.19), 7.275 (2.04), 7.283 (2.06), 7.529 (3.04), 7.576 (2.78), 8.019 (1.73), 8.026 (2.07), 8.032 (1.85), 8.039 (1.80), 8.415 (4.45), 10.905 (1.56), 10.939 (1.42).

### Example 45

### 3-(3-chloro-2-methoxy-anilino)-7-methyl-2-[3-(tetrahydropyran-2-ylmethoxy)-4-pyridyl]-1,5,6,7-tetrahydropyrrolo[3,2-c]pyridin-4-one (stereoisomer 1)

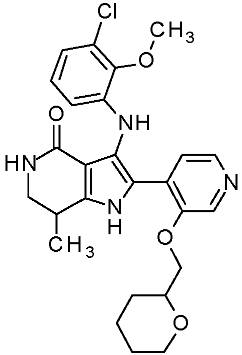

The title compound from example 44 (86 mg) was separated by preparative chiral HPLC into a mixture of isomers 1 (22 mg, Rt: 7.5 - 9.2 min) and a mixture of isomers 2 (22.1 mg, Rt = 10.4 - 12.4 min).

### Preparative chiral HPLC method: MTBE

Instrument: PrepCon Labomatic HPLC; Column: YMC Cellulose SB 10µ, 250x50; eluent A: methyl tert-butyl ether + 0.1 vol % diethylamine; eluent B: acetonitrile; isocratic: 70%A+30%B; flow: 100 mL/min; temperature: 25°C; UV: 280 nm

### Analytical chiral HPLC method: MTBE

Instrument: Waters Alliance 2695; Column: YMC Cellulose SB 3µ, 100x4.6; eluent A: methyl tert-butyl ether + 0.1 vol % diethylamine; eluent B: acetonitrile; isocratic: 70%A+30%B; flow: 1.4 ml/min; temperature: 25°C; UV: 280 nm
Analytical chiral HPLC: Rₜ (mixture of isomers 1) = 1.51 min + 1.64 min; Rₜ (mixture of isomers 2) = 2.04 min.

Mixture of isomers 1 was separated by preparative chiral HPLC into 14.0 mg (80% purity, 16% yield) of the title compound (enantiomer 1, Rt: 2.9 - 5.8 min) and enantiomer 2 (8 mg, Rt: 8.2 - 10.7 min).

### Preparative chiral HPLC method: POB

Instrument: PrepCon Labomatic HPLC; Column: Chiralcel OD-H 5µ, 250x20; eluent A: acetonitrile + 0.1 vol % diethylamine; eluent B: ethanol + 0.1 vol % diethylamine; isocratic: 90%A+10%B; flow: 20 ml/min; temperature: 25°C; UV: 254 nm

### Analytical chiral HPLC method: POB

Instrument: Waters Alliance 2695; Column: Chiralcel OD-H 5µ, 100x4.6; eluent A: acetonitrile + 0.1 vol % diethylamine; eluent B: ethanol ; isocratic: 90%A+10%B; flow: 1.4 ml/min; temperature: 25°C; UV: 254 nm
Analytical chiral HPLC: Rₜ = 2.90 min
Optical rotation:[α]_{D} = 21.06 ° +/- 1.40° (c = 2.8 mg/ml in chloroform)
¹H-NMR (400 MHz, DMSO-d₆) δ [ppm]: 0.696 (5.56), 0.730 (2.09), 0.797 (1.16), 0.802 (1.09), 0.814 (1.39), 0.821 (1.53), 0.832 (1.45), 0.840 (1.45), 0.850 (1.63), 0.886 (0.77), 0.904 (0.89), 0.922 (0.78), 0.974 (0.95), 0.991 (1.18), 1.009 (1.11), 1.069 (1.47), 1.087 (1.98), 1.138 (7.97), 1.157 (4.79), 1.175 (3.12), 1.230 (7.63), 1.261 (6.58), 1.277 (6.66), 1.292 (4.08), 1.337 (1.86), 1.366 (1.41), 1.505 (1.90), 1.531 (3.41), 1.632 (1.40), 1.667 (1.84), 1.753 (0.54), 1.766 (0.50), 1.826 (1.25), 1.908 (1.12), 1.959 (1.25), 1.981 (1.10), 2.062 (0.78), 2.084 (1.56), 2.116 (3.06), 2.197 (0.76), 2.209 (0.53), 2.327 (0.52), 2.539 (0.48), 2.911 (0.63), 2.929 (0.61), 3.059 (0.54), 3.085 (1.89), 3.092 (1.88), 3.107 (1.42), 3.123 (0.59), 3.475 (1.12), 3.493 (1.79), 3.504 (1.81), 3.521 (0.94), 3.588 (0.41), 3.773 (0.80), 3.794 (1.07), 3.819 (0.78), 3.868 (1.43), 3.881 (16.00), 3.973 (1.10), 4.000 (0.99), 4.040 (1.09), 4.060 (1.27), 4.065 (1.41), 4.085 (1.06), 4.286 (1.26), 4.293 (1.29), 4.311 (1.10), 4.318 (1.03), 5.757 (10.63), 6.124 (1.53), 6.135 (2.42), 6.148 (1.57), 6.653 (0.47), 6.675 (4.94), 6.687 (5.20), 6.936 (0.60), 6.957 (0.73), 7.013 (0.47), 7.170 (2.17), 7.197 (0.57), 7.218 (0.45), 7.263 (1.48), 7.275 (1.52), 7.575 (4.17), 8.019 (1.05), 8.413 (1.35), 10.937 (2.42).

### Example 46

### 3-(3-chloro-2-methoxy-anilino)-7-methyl-2-[3-(tetrahydropyran-2-ylmethoxy)-4-pyridyl]-1,5,6,7-tetrahydropyrrolo[3,2-c]pyridin-4-one (stereoisomer 2)

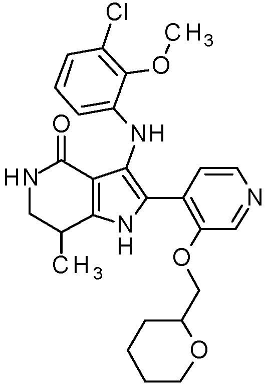

Separation of enantiomers by two preparative chiral HPLCs (methods see example 45) to give 8.0 mg (90% purity, 10% yield) of the title compound (enantiomer 2, Rₜ = 8.2 - 10.7 min).

Analytical chiral HPLC (method 2 of example 45): Rₜ = 4.44 min.

Optical rotation:[α]_{D} = 10.54 ° +/- 1.37° (c = 3.2 mg/ml in chloroform)

¹H-NMR (400 MHz, DMSO-d₆) δ [ppm]: 0.697 (0.44), 0.726 (0.45), 0.746 (0.48), 0.814 (0.46), 0.821 (0.52), 0.840 (0.44), 1.088 (0.43), 1.138 (2.62), 1.154 (1.05), 1.172 (0.68), 1.232 (1.48), 1.266 (4.54), 1.282 (4.64), 1.322 (0.42), 1.330 (0.40), 1.352 (0.72), 1.381 (0.84), 1.410 (0.50), 1.473 (0.50), 1.504 (1.04), 1.525 (2.25), 1.553 (0.84), 1.627 (0.87), 1.658 (0.74), 1.819 (0.78), 2.084 (2.26), 2.116 (0.76), 2.197 (0.50), 2.522 (1.16), 3.073 (0.99), 3.082 (1.50), 3.090 (1.47), 3.103 (1.10), 3.109 (1.04), 3.119 (0.41), 3.456 (0.58), 3.483 (1.31), 3.495 (1.03), 3.507 (1.09), 3.512 (1.04), 3.749 (0.44), 3.756 (0.53), 3.771 (0.63), 3.784 (0.52), 3.791 (0.46), 3.867 (1.39), 3.875 (16.00), 3.985 (0.82), 4.012 (0.76), 4.114 (0.81), 4.131 (0.88), 4.140 (1.31), 4.156 (1.23), 4.205 (1.25), 4.214 (1.30), 4.230 (0.80), 4.239 (0.73), 5.758 (4.98), 6.127 (1.46), 6.137 (1.49), 6.140 (1.63), 6.151 (1.48), 6.664 (0.54), 6.673 (5.89), 6.684 (3.15), 6.687 (3.03), 7.148 (1.83), 7.268 (1.70), 7.281 (1.73), 7.527 (3.91), 8.023 (1.08), 8.036 (1.05), 8.411 (1.64), 10.902 (2.10).

### Example 47

### 3-(3-chloro-2-methoxy-anilino)-7-methyl-2-[3-(tetrahydropyran-2-ylmethoxy)-4-pyridyl]-1,5,6,7-tetrahydropyrrolo[3,2-c]pyridin-4-one (stereoisomer 3)

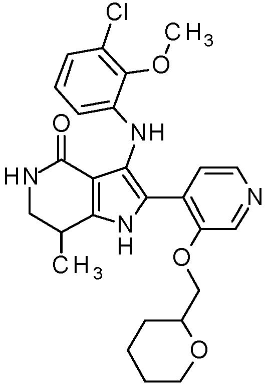

Mixture of isomers 2 from example 45 was separated by preparative chiral HPLC into 9.0 mg (90 % purity, 12 % yield) of the title compound (enantiomer 3, Rt: 6.9 - 8.9 min) and enantiomer 4 (10 mg, Rt: 9.5 - 11.6 min).

### Preparative chiral HPLC method: NPB

Instrument: PrepCon Labomatic HPLC; Column: YMC Amylose SA 5µ, 250x30; eluent A: hexane + 0.1 vol % diethylamine; eluent B: ethanol + 0.1 vol % diethylamine; isocratic: 50%A+50%B; flow: 40 ml/min; temperature: 25°C; UV: 254 nm

### Analytical chiral HPLC method: NPB

Instrument: Waters Alliance 2695; Column: YMC Amylose SA 3µ, 100x4.6; eluent A: hexane + 0.1 vol % diethylamine; eluent B: ethanol; isocratic: 50%A+50%B; flow: 1.4 ml/min; temperature: 25°C; UV: 254 nm
Analytical chiral HPLC: Rₜ = 1.82 min
Optical rotation:[α]_{D} = - 13.15 ° +/- 1.50° (c = 2.6 mg/ml in chloroform)
¹H-NMR (400 MHz, DMSO-d₆) δ [ppm]: 0.696 (0.51), 0.745 (0.72), 0.764 (0.74), 0.797 (0.58), 0.802 (0.41), 0.814 (0.61), 0.821 (0.67), 0.840 (0.50), 0.904 (0.50), 1.138 (2.28), 1.155 (3.04), 1.173 (1.45), 1.230 (1.45), 1.266 (4.79), 1.282 (4.86), 1.305 (0.63), 1.323 (0.65), 1.352 (0.82), 1.381 (0.95), 1.410 (0.58), 1.473 (0.53), 1.505 (1.07), 1.525 (2.42), 1.553 (0.92), 1.627 (0.94), 1.658 (0.81), 1.817 (0.85), 2.116 (0.43), 2.522 (0.67), 3.057 (0.41), 3.074 (1.08), 3.083 (1.60), 3.090 (1.59), 3.103 (1.21), 3.109 (1.10), 3.120 (0.43), 3.126 (0.40), 3.456 (0.63), 3.483 (1.38), 3.490 (1.04), 3.496 (1.14), 3.511 (1.53), 3.530 (0.62), 3.749 (0.48), 3.756 (0.57), 3.770 (0.70), 3.783 (0.58), 3.791 (0.51), 3.877 (16.00), 3.984 (0.88), 4.012 (0.80), 4.114 (0.85), 4.131 (0.88), 4.140 (1.35), 4.156 (1.23), 4.206 (1.32), 4.214 (1.34), 4.231 (0.84), 4.240 (0.76), 5.758 (11.55), 6.127 (1.50), 6.138 (1.57), 6.141 (1.64), 6.152 (1.52), 6.664 (0.59), 6.673 (5.99), 6.684 (3.25), 6.687 (3.10), 7.150 (1.93), 7.270 (1.75), 7.282 (1.74), 7.528 (4.06), 8.024 (0.99), 8.036 (0.95), 8.412 (1.42), 10.903 (2.24).

### Example 48

### 3-(3-chloro-2-methoxy-anilino)-7-methyl-2-[3-(tetrahydropyran-2-ylmethoxy)-4-pyridyl]-1,5,6,7-tetrahydropyrrolo[3,2-c]pyridin-4-one (stereoisomer 4)

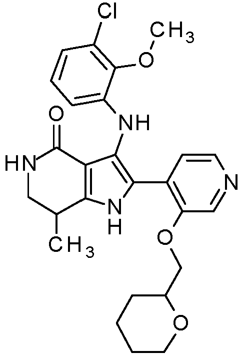

Separation of enantiomers by two preparative chiral HPLCs (first method see example 45, second method see example 47) to give 10.0 mg (90% purity, 13% yield) of the title compound (enantiomer 4, Rₜ = 9.5 - 11.6 min).

Analytical chiral HPLC (method see example 47): Rₜ = 4.44 min.

Optical rotation:[α]_{D} = - 61.28 ° +/- 0.86° (c = 2.4 mg/ml in chloroform)

¹H-NMR (400 MHz, DMSO-d₆) δ [ppm]: 0.697 (0.58), 0.744 (0.74), 0.763 (0.76), 0.814 (0.40), 0.821 (0.45), 0.840 (0.41), 1.138 (1.94), 1.154 (3.04), 1.172 (1.50), 1.231 (1.83), 1.261 (5.47), 1.277 (5.64), 1.307 (1.04), 1.337 (1.10), 1.366 (0.68), 1.532 (3.07), 1.633 (1.16), 1.665 (0.99), 1.827 (1.02), 3.058 (0.48), 3.085 (1.93), 3.092 (1.90), 3.106 (1.41), 3.122 (0.54), 3.492 (1.74), 3.502 (1.80), 3.522 (0.88), 3.773 (0.61), 3.794 (0.96), 3.820 (0.68), 3.880 (16.00), 3.973 (1.08), 4.000 (0.98), 4.040 (1.10), 4.060 (1.29), 4.066 (1.45), 4.085 (1.10), 4.285 (1.31), 4.292 (1.37), 4.311 (1.14), 4.318 (1.07), 5.758 (4.61), 6.122 (1.53), 6.134 (2.45), 6.147 (1.58), 6.675 (4.89), 6.687 (5.12), 7.169 (2.28), 7.262 (1.92), 7.275 (1.95), 7.573 (4.27), 8.017 (1.22), 8.028 (1.18), 8.414 (1.72), 10.937 (2.58).

### Example 49

### (5aR,8aS)-3-(3-chloro-2-methoxyanilino)-2-{3-[(1-methoxypropan-2-yl)oxy]-4-pyridyl}-5,5a,6,7,8,8a-hexahydrocyclopenta[b]pyrrolo[2,3-d]pyridin-4(1H)-one

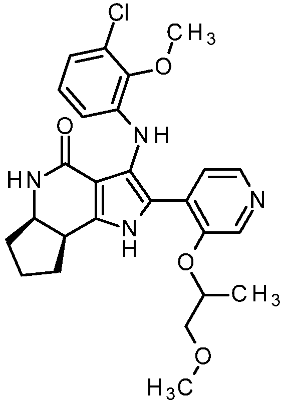

(4a*S*,7a*R*)-*N*-(3-Chloro-2-methoxy-phenyl)-4-{[3-(2-methoxy-1-methyl-ethoxy)-4-pyridyl]methylamino}-2-oxo-1,4a,5,6,7,7a-hexahydrocyclopenta[b]pyridine-3-carbothioamide (intermediate 6-49, 79.0 mg, 149 µmol) was dissolved in acetic acid (1.6 ml). Hydrogen peroxide (30 µl, 30% purity, 300 µmol) was added and the mixture stirred for 4 h at 60°C. The mixture was quenched with a few drops of sat. sodium bicarbonate solution and was evacuated under reduced pressure. The residue was purified by preparative HPLC (method 10, 0.00-0.50 min 30% B, 0.50--7.00 min 30-70% B) to give 61.5 mg (90% purity, 75% yield) of the title compound.

LC-MS (method 2): Rₜ = 1.16 min; MS (ESlpos): m/z = 497 [M+H]⁺

¹H-NMR (400 MHz, DMSO-d₆) δ [ppm]: 1.32 (t, 3H), 1.62 - 1.83 (m, 4H), 1.83 - 1.95 (m, 1H), 2.05 - 2.16 (m, 1H), 3.21 - 3.31 (m, 1H), 3.52 - 3.63 (m, 2H), 3.87 (s, 3H), 4.00 - 4.12 (m, 1H), 4.74 - 4.81 (m, 1H), 6.11 - 6.17 (m, 1H), 6.63 - 6.69 (d, 2H), 6.90 - 7.14 (m, 1H), 7.25 (t, 1H), 7.57 (d, 1H), 8.01 (dd, 1H), 8.40 - 8.47 (d, 1H), 10.96 (d, 1H).

### Example 50

### (5aR,8aS)-3-(3-chloro-2-methoxyanilino)-2-{3-[(1-methoxypropan-2-yl)oxy]-4-pyridyl}-5,5a,6,7,8,8a-hexahydrocyclopenta[b]pyrrolo[2,3-d]pyridin-4(1H)-one (stereoisomer 1)

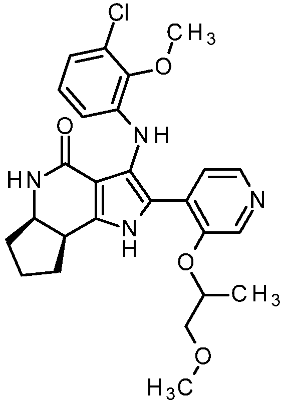

The title compound from example 49 (55.0 mg) was separated into enantiomers by preparative chiral HPLC followed by triturating with hexane to give 18.5 mg (95% purity, 22% yield) of the title compound (enantiomer 1, Rₜ = 7.4 - 9.3 min) and enantiomer 2 (20.6 mg, Rₜ = 9.6 - 12.4 min, see example 51).

### Preparative chiral HPLC method: MTBE

Instrument: PrepCon Labomatic HPLC; Column: YMC Amylose SA 5µ, 250x30; eluent A: methyl tert-butyl ether + 0.1 vol % diethylamine; eluent B: acetonitrile + 0.1 vol % diethylamine; isocratic: 40%A+60%B; flow: 60 ml/min; temperature: 25°C; UV: 254 nm

### Analytical chiral HPLC method: MTBE

Instrument: Waters Alliance 2695; Column: YMC Amylose SA 3µ, 100x4.6; eluent A: methyl tert-butyl ether + 0.1 vol % diethylamine; eluent B: acetonitrile; isocratic: 40%A+60%B; flow: 1.4 ml/min; temperature: 25°C; UV: 254 nm
Analytical chiral HPLC: Rₜ = 3.27 min.
Optical rotation:[α]_{D} = 104.18° +/- 0.35 ° (c = 3.3 mg/ml in chloroform)
¹H-NMR (400 MHz, DMSO-d₆) δ [ppm]: 10.86 - 11.01 (m, 1H), 8.42 (s, 1H), 7.97 - 8.04 (m, 1H), 7.47 - 7.61 (m, 1H), 7.23 - 7.31 (m, 1H), 7.00 - 7.10 (m, 1H), 6.63 - 6.69 (m, 2H), 6.06 - 6.19 (m, 1H), 4.66 - 4.88 (m, 1H), 4.00 - 4.16 (m, 1H), 3.77 - 3.90 (m, 3H), 3.53 - 3.64 (m, 2H), 3.20 - 3.27 (m, 1H), 2.05 - 2.15 (m, 1H), 1.83 - 1.94 (m, 1H), 1.61 - 1.83 (m, 4H), 1.29 - 1.38 (m, 3H).

### Example 51

### (5aR,8aS)-3-(3-chloro-2-methoxyanilino)-2-{3-[(1-methoxypropan-2-yl)oxy]-4-pyridyl}-5,5a,6,7,8,8a-hexahydrocyclopenta[b]pyrrolo[2,3-d]pyridin-4(1H)-one (stereoisomer 2)

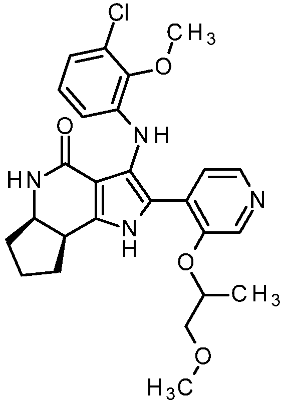

For the preparation of the title compound as mixture of two stereoisomers see example 49. Separation of enantiomers by preparative chiral HPLC (method see example 50) followed by trituration with hexane to give 20.6 mg (95% purity, 25% yield) of the title compound (enantiomer 2, Rₜ = 9.6 - 12.4 min).

Analytical chiral HPLC (method see example 64): Rₜ = 4.19 min.

Optical rotation:[α]_{D} = 31.42 ° +/- 0.82 ° (c = 4.8 mg/ml in chloroform)

¹H-NMR (400 MHz, DMSO-d₆) δ [ppm]: 10.99 (s, 1H), 8.42 (s, 1H), 7.93 - 8.08 (m, 1H), 7.49 - 7.62 (m, 1H), 7.20 - 7.30 (m, 1H), 7.06 - 7.14 (m, 1H), 6.62 - 6.69 (m, 2H), 6.01 - 6.21 (m, 1H), 4.72 - 4.87 (m, 1H), 3.87 (s, 4H), 3.58 (d, 2H), 3.24 - 3.28 (m, 1H), 2.07 - 2.19 (m, 1H), 1.58 - 1.95 (m, 6H), 1.31 (d, 4H).

### Example 52

### (5aR,8aS)-3-(3-chloro-2-methoxyanilino)-2-{3-[(1,4-dioxan-2-yl)methoxy]-4-pyridyl}-5,5a,6,7,8,8a-hexahydrocyclopenta[b]pyrrolo[2,3-d]pyridin-4(1H)-one

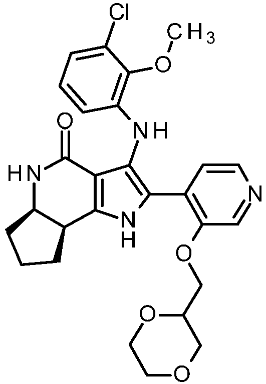

(4a*S*,7a*R*)-*N*-(3-chloro-2-methoxy-phenyl)-4-{[3-(1,4-dioxan-2-ylmethoxy)-4-pyridyl]methylamino}-2-oxo-1,4a,5,6,7,7a-hexahydrocyclopenta[b]pyridine-3-carbothioamide (intermediate 6-52, 47.4 mg, 84.8 µmol) was dissolved in EtOH (2.8 ml). Palladium on carbon (90.2 mg, 10%, 84.8 µmol) and TFA (9.8 µl, 130 µmol; CAS 76-05-1) were added and the mixture was stirred at 90°C for 4 h. The reaction mixture was filtrated over celite and washed with MeOH. The filtrate was concentrated under reduced pressure and purified by preparative HPLC (method 10, 0.00 - 0.50 30% B, 0.50 - 7.00 30-70% B) to give 18.8 mg (95% purity, 40% yield) of the title compound.

LC-MS (method 2): Rₜ = 1.07 min; MS (ESlpos): m/z = 525 [M+H]⁺

¹H-NMR (400 MHz, DMSO-d₆) δ [ppm]: 1.63 - 1.82 (m, 4H), 1.83 - 1.95 (m, 1H), 2.03 - 2.16 (m, 1H), 3.21 - 3.29 (m, 1H), 3.37 - 3.47 (m, 1H), 3.47 - 3.56 (m, 1H), 3.67 - 3.79 (m, 2H), 3.80 - 3.85 (m, 2H), 3.87 (s, 3H), 3.98 - 4.32 (m, 4H), 5.76 (s, 1H), 6.13 (t, 1H), 6.67 (d, 2H), 7.09 (s, 1H), 7.24 (d, 1H), 7.58 (d, 1H), 8.03 (dd, 1H), 8.39 (d, 1H), 10.95 (s, 1H).

### Example 53

### (5aR,8aS)-3-(3-chloro-2-methoxyanilino)-2-{3-[(1,4-dioxan-2-yl)methoxy]-4-pyridyl}-5,5a,6,7,8,8a-hexahydrocyclopenta[b]pyrrolo[2,3-d]pyridin-4(1H)-one (stereoisomer 1)

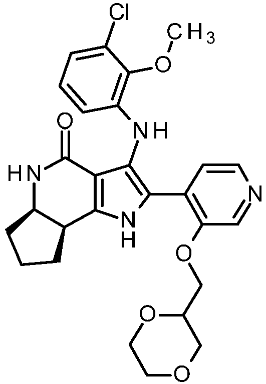

The title compound from example 52 (40.4 mg) was separated into enantiomers by preparative chiral HPLC to give 8.7 mg (95% purity) of the title compound (enantiomer 1, Rₜ = 11.8 - 13.7 min) and enantiomer 2 (6.3 mg, Rₜ = 15.7 - 18.2 min, see example 54).

### Preparative chiral HPLC method:

Instrument: PrepCon Labomatic HPLC; Column: YMC Cellulose SB 10µ, 250x50; eluent A: hexane + 0.1 vol % diethylamine; eluent B: ethanol; isocratic: 70% A + 30% B; flow: 150 mL/min; temperature: 25°C; UV: 254 nm

### Analytical chiral HPLC method:

Instrument: Waters Alliance 2695; Column: YMC Cellulose SB 3µ, 100x4.6; eluent A: hexane + 0.1 vol% diethylamine; eluent B: ethanol; isocratic: 70% A + 30% B; flow: 1.4 ml/min; temperature: 25°C; UV: 254 nm
Analytical chiral HPLC: Rₜ = 3.56 min.
Optical rotation:[α]_{D} = 46.55° +/- 0.46° (c = 4.55 mg/ml in chloroform)
¹H-NMR (400 MHz, DMSO-d₆) δ [ppm]: 10.82 - 11.03 (m, 1H), 8.39 (s, 1H), 7.99 - 8.05 (m, 1H), 7.53 - 7.58 (m, 1H), 7.19 - 7.29 (m, 1H), 7.06 - 7.12 (m, 1H), 6.62 - 6.72 (m, 2H), 6.08 - 6.17 (m, 1H), 4.12 - 4.26 (m, 2H), 3.94 - 4.07 (m, 2H), 3.87 (s, 5H), 3.64 - 3.75 (m, 2H), 3.40 - 3.57 (m, 2H), 2.06 - 2.16 (m, 1H), 1.62 - 1.95 (m, 5H).

### Example 54

### (5aR,8aS)-3-(3-chloro-2-methoxyanilino)-2-{3-[(1,4-dioxan-2-yl)methoxy]-4-pyridyl}-5,5a,6,7,8,8a-hexahydrocyclopenta[b]pyrrolo[2,3-d]pyridin-4(1H)-one (stereoisomer 2)

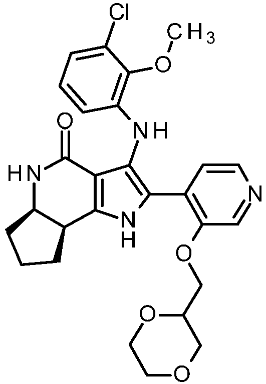

For the preparation of the title compound as mixture of two stereoisomers see example 52. Separation of enantiomers by preparative chiral HPLC (method see example 53) to give 6.3 mg (95% purity) of the title compound (enantiomer 2, Rₜ = 15.7 - 18.2 min).

Analytical chiral HPLC (method see example 53): Rₜ = 4.84 min.

Optical rotation:[α]_{D} = 46.49° +/- 0.46° (c = 3.49 mg/ml in chloroform)

¹H-NMR (400 MHz, DMSO-d₆) δ [ppm]: 10.89 - 11.00 (m, 1H), 8.32 - 8.44 (m, 1H), 7.97 - 8.06 (m, 1H), 7.49 - 7.65 (m, 1H), 7.20 - 7.29 (m, 1H), 7.03 - 7.09 (m, 1H), 6.59 - 6.76 (m, 2H), 6.09 - 6.22 (m, 1H), 4.23 - 4.34 (m, 1H), 3.96 - 4.18 (m, 3H), 3.78 - 3.91 (m, 5H), 3.67 - 3.76 (m, 2H), 3.48 - 3.58 (m, 1H), 3.37 - 3.43 (m, 1H), 3.20 - 3.28 (m, 1H), 2.02 - 2.15 (m, 2H), 1.61 - 1.93 (m, 5H).

### Example 55

### (5aR*,8aS*)-3-(3-chloro-2-methoxyanilino)-2-(3-{[(2S)-oxolan-2-yl]methoxy}-4-pyridyl)-5,5a,6,7,8,8a-hexahydrocyclopenta[b]pyrrolo[2,3-d]pyridin-4(1H)-one

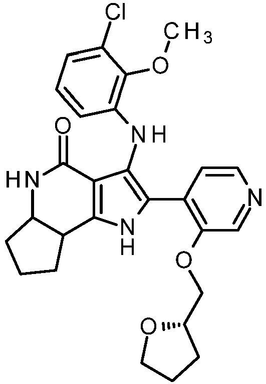

To a solution of intermediate 57-9 ((5a*R**,8a*S**)-3-(3-chloro-2-methoxyanilino)-5-(2,4-dimethoxybenzyl)-2-{3-[(2*S*)-tetrahydrofuran-2-ylmethoxy]pyridin-4-yl}-5,5a,6,7,8,8a-hexahydrocyclopenta[b]pyrrolo[2,3-d]pyridin-4(1*H*)-one) (300 mg, 455 umol) in dichloromethane (1 ml) was added TFA (1.54 g, 13.5 mmol). The mixture was stirred at 25 °C for 0.5 h. The reaction mixture was adjusted to pH = 8~9 with saturated NaHCO₃, and extracted with dichloromethane (50 ml x 3). The combined organic layers were washed with brine, dried over Na₂SO₄, filtered and concentrated under reduced pressure to give a residue. The residue was purified by prep-HPLC (column: Waters Xbridge C18 150x50 mm, 10µm; mobile phase: [water (0.05% ammonia hydroxide v/v)-ACN]; B%: 35%-65%, 11.5 min). The residue was purified by prep-TLC (SiO₂, DCM/MeOH = 10/1 Product R_{f} = 0.5) to give the title compound (220 mg, 425 umol, 93.4% yield, 98.4% purity) as yellow solid.

LC-MS (method 4): Rₜ = 1.026 min; MS (ESlpos): m/z = 509.2 [M+H]⁺

¹H-NMR (400 MHz, CDCl₃) δ [ppm]: 10.93 (d, 1H), 8.32 (s, 1H), 7.99 (s, 1H), 7.80 (d, 1H), 7.34 (d, 1H), 6.75 (d, 1H), 6.67-6.62 (m, 1H), 6.25 (d, 1H), 5.27-5.12 (m, 1H), 4.55-4.53 (m, 2H), 4.51 (s, 1H), 4.08 (s, 3H), 4.08-4.02 (m, 1H), 4.00-3.98 (m, 1H), 3.96-3.95 (m, 1H), 3.34-3.32 (m, 1H), 2.18-2.08 (m, 2H), 2.07-2.06 (m, 4H), 1.78-1.75 (m, 3H), 1.27-1.26 (m, 1H).

### Example 56

### (5aR,8aS)-3-(3-chloro-2-methoxyanilino)-2-(3-{[(2S)-oxolan-2-yl]methoxy}-4-pyridyl)-5,5a,6,7,8,8a-hexahydrocyclopenta[b]pyrrolo[2,3-d]pyridin-4(1H)-one

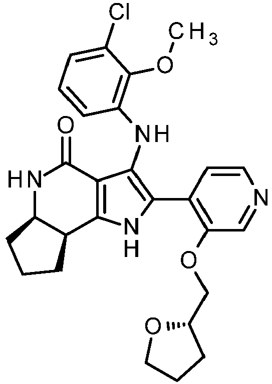

((5aR*,8aS*)-3-(3-chloro-2-methoxyanilino)-2-(3-{[(2*S*)-oxolan-2-yl]methoxy}pyridin-4-yl)-5,5a,6,7,8,8a-hexahydrocyclopenta[b]pyrrolo[2,3-d]pyridin-4(1*H*)-one) (example 55, 200 mg, 386.64 umol) was used for SFC isomer separation: The residue was purified by prep-SFC (column: DAICEL CHIRALPAK AD (250 mm * 30 mm, 10 µm); mobile phase: Phase A: CO₂; Phase B: 0.1% NH₃H₂O in MeOH; isocratic, 45 % B) to give the title compound (84.96 mg, 97.8% purity) as an off-white solid and a second isomer (see Example 57, 100.14 mg, 99.6% purity) as a yellow solid.

SFC: Rₜ = 0.737 min.

LC-MS (method 3): Rₜ = 0.848 min; MS (ESlpos): m/z = 509.0 [M+H]⁺

¹H NMR (400 MHz, DMSO-d₆) δ [ppm]: 11.12 (s, 1H), 8.42 (s, 1H), 8.02 (d, *J*=5.07 Hz, 1H), 7.58 (s, 1H), 7.24 (d, *J*=5.07 Hz, 1H), 7.13 (d, *J*=1.77 Hz, 1H), 6.61-6.73 (m, 2H), 6.09-6.22 (m, 1H), 4.27-4.41 (m, 2H), 3.98-4.08 (m, 2H), 3.82-3.92 (m, 4H), 3.72-3.81 (m, 1H), 3.23-3.31 (m, 1H), 1.96-2.16 (m, 2H), 1.77-1.94 (m, 4H), 1.58-1.76 (m, 4H),

To confirm the absolute configuration of (5a*R*,8a*S*)-3-(3-chloro-2-methoxyanilino)-2-(3-{[(2*S*)-oxolan-2-yl]methoxy}-4-pyridyl)-5,5a,6,7,8,8a-hexahydrocyclopenta[b]pyrrolo[2,3-d]pyridin-4(1*H*)-one example 56 was prepared by a second method starting from enantiopure intermediates:
According to the method described for example 1 using (4a*S*,7a*R*)-N-(3-chloro-2-methoxyphenyl)-2-oxo-4-{[(3-{[(2*S*)-oxolan-2-yl]methoxy}pyridin-4-yl)methyl]amino}-2,4a,5,6,7,7a-hexahydro-1*H*-cyclopenta[b]pyridine-3-carbothioamide (intermediate 6-56, 81.8 mg, 151 µmol) as starting material, 21.8 mg (95 % purity, 27 % yield) of the title compound were prepared after purification by preparative HPLC (method 10, gradient: 0.00-0.50 min 30% B, 0.50-7.00 min 30-70% B).

LC-MS (method 2): Rₜ = 1.18 min; MS (ESlpos): m/z = 509 [M+H]⁺

¹H-NMR (400 MHz, DMSO-d₆) δ [ppm]: 1.56 - 1.76 (m, 4H), 1.76 - 1.95 (m, 4H), 1.97 - 2.21 (m, 2H), 3.24 - 3.31 (m, 1H), 3.71 - 3.82 (m, 1H),3.87 (s, 3H) 3.82 - 3.92 (m, 1H), 3.96 - 4.08 (m, 2H), 4.27 - 4.38 (m, 2H), 6.12 - 6.19 (m, 1H), 6.67 (s, 1H), 6.68 (d, 1H), 7.12 (d, 1H), 7.20 - 7.29 (d, 1H), 7.57 (s, 1H), 7.98 - 8.08 (d, 1H), 8.41 (s, 1H), 11.08 - 11.17 (s, 1H).

### Example 57

### (5aS,8aR)-3-(3-chloro-2-methoxyanilino)-2-(3-{[(2S)-oxolan-2-yl]methoxy}pyridin-4-yl)-5,5a,6,7,8,8a-hexahydrocyclopenta[b]pyrrolo[2,3-d]pyridin-4(1H)-one

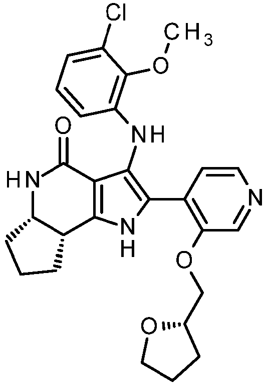

SFC purification (see Example 56) gave Example 57 (100.14 mg, 195 umol, 50.6% yield, 99.6% purity) as a yellow solid.

SFC: Rₜ = 1.108 min.

LC-MS (method 3): Rₜ = 0.844 min; MS (ESlpos): m/z = 509.0 [M+H]⁺

¹H-NMR (400 MHz, DMSO-d₆) δ [ppm]: 11.16 (s, 1H), 8.41 (s, 1H), 8.01 (d, 1H), 7.58 (s, 1H), 7.24 (d, 1H), 7.08 (s, 1H), 6.74-6.63 (m, 2H), 6.21-6.10 (m, 1H), 4.42 (dd, 1H), 4.34 (qd, 1H), 4.06 (br s, 1H), 3.98 (dd, 1H), 3.90-3.83 (m, 4H), 3.82-3.75 (m, 1H), 3.27-3.18 (m, 1H), 2.15-1.97 (m, 2H), 1.96-1.84 (m, 3H), 1.82-1.61 (m, 5H).

### Example 58

### 3-(3-chloro-2-methyl-anilino)-7-methyl-2-(3-{[(2S)-oxolan-2-yl]methoxy}pyridin-4-yl)-1,5,6,7-tetrahydropyrrolo[3,2-c]pyridin-4-one

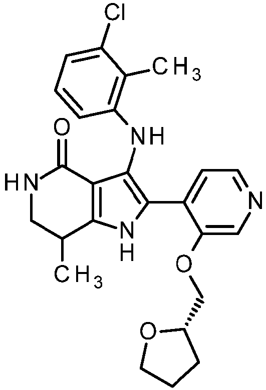

According to the method described for example 52 using *N*-(3-chloro-2-methyl-phenyl)-3-methyl-6-oxo-4-{[3-(tetrahydrofuran-2-ylmethoxy)-4-pyridyl]methylamino}-2,3-dihydro-1*H*-pyridine-5-carbothioamide (intermediate 6-58, 175 mg, 349 µmol) as starting material, 41.0 mg (90% purity, 23% yield) of the title compound were prepared after purification by preparative HPLC (method 10, gradient: 0.00-0.50 min 30% B, 0.50-6.00 min 30-70% B).

LC-MS (method 2): Rₜ = 1.13 min; MS (ESlpos): m/z = 467 [M+H]⁺

¹H-NMR (400 MHz, DMSO-d₆) δ [ppm]: -0.008 (0.88), 0.008 (1.06), 1.272 (3.04), 1.287 (4.62), 1.300 (2.69), 1.672 (0.60), 1.678 (0.63), 1.694 (0.66), 1.862 (1.06), 1.878 (1.68), 1.891 (1.28), 1.896 (1.34), 1.907 (0.97), 1.913 (0.75), 1.995 (0.59), 2.012 (0.67), 2.026 (0.65), 2.044 (0.45), 2.076 (0.57), 2.151 (1.36), 2.324 (0.63), 2.329 (0.96), 2.341 (16.00), 2.520 (2.88), 2.524 (1.82), 2.542 (1.06), 2.666 (0.54), 2.671 (0.71), 2.676 (0.50), 3.066 (0.47), 3.083 (1.76), 3.108 (1.55), 3.441 (0.53), 3.448 (0.60), 3.456 (0.60), 3.463 (0.80), 3.479 (0.59), 3.486 (0.45), 3.746 (0.76), 3.763 (1.15), 3.766 (1.09), 3.779 (1.14), 3.796 (0.62), 3.822 (0.98), 3.839 (2.14), 3.856 (1.21), 3.859 (1.42), 3.876 (0.61), 3.959 (0.50), 3.978 (0.90), 3.983 (0.98), 4.007 (0.93), 4.012 (0.76), 4.292 (0.57), 4.299 (0.74), 4.315 (1.84), 4.329 (0.87), 4.344 (1.36), 4.364 (0.80), 4.372 (0.61), 6.196 (1.80), 6.215 (1.90), 6.709 (1.22), 6.726 (2.83), 6.729 (2.49), 6.743 (2.27), 6.763 (2.50), 6.783 (0.85), 7.173 (1.40), 7.190 (0.92), 7.215 (2.55), 7.218 (2.23), 7.227 (2.37), 7.231 (2.06), 7.318 (2.46), 7.336 (2.15), 7.989 (5.23), 8.002 (4.79), 8.387 (4.36), 8.402 (0.49), 10.993 (1.16), 11.038 (1.31).

### Example 59

### 3-(3-chloro-2-methyl-anilino)-7-methyl-2-(3-{[(2S)-oxolan-2-yl]methoxy}pyridin-4-yl)-1,5,6,7-tetrahydropyrrolo[3,2-c]pyridin-4-one (stereoisomer 1)

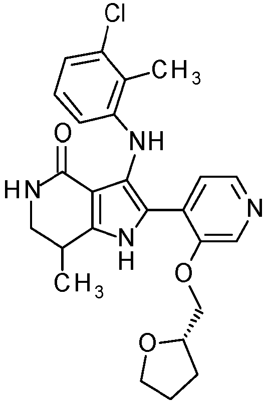

The title compound from example 58 (41 mg) was separated into enantiomers by preparative chiral HPLC to give 15.0 mg (97% purity, 35% yield) of the title compound (enantiomer 1, Rₜ = 5.5 - 6.6 min) and enantiomer 2 (17.0 mg, Rₜ = 8.5 - 10.2 min, see example 60).

### Preparative chiral HPLC method: NPB

Instrument: PrepCon Labomatic HPLC; Column: YMC Amylose SA 10µ, 250x50; eluent A: hexane + 0.1 vol % diethylamine; eluent B: ethanol; isocratic: 50%A+50%B; flow: 150 mL/min; temperature: 25°C; UV: 280 nm

### Analytical chiral HPLC method: NPB

Instrument: Waters Alliance 2695; Column: YMC Amylose SA 3µ, 100x4.6; eluent A: hexane + 0.1 vol % diethylamine; eluent B: ethanol; isocratic: 50% A + 50% B; flow: 1.4 ml/min; temperature: 25°C; UV: 280 nm
Analytical chiral HPLC: Rₜ = 1.66 min.
¹H-NMR (400 MHz, DMSO-d₆) δ [ppm]: 1.157 (0.44), 1.232 (0.89), 1.283 (5.39), 1.299 (5.41), 1.648 (0.54), 1.666 (0.66), 1.676 (0.68), 1.681 (0.64), 1.698 (0.77), 1.844 (0.53), 1.860 (1.82), 1.877 (2.70), 1.895 (1.76), 1.912 (0.72), 1.975 (0.40), 1.993 (0.65), 2.007 (0.66), 2.023 (0.69), 2.039 (0.57), 2.323 (0.65), 2.327 (0.99), 2.340 (16.00), 2.518 (2.08), 2.523 (1.41), 2.665 (0.48), 2.669 (0.62), 2.673 (0.45), 3.081 (1.39), 3.095 (1.71), 3.105 (1.95), 3.438 (0.82), 3.446 (0.96), 3.455 (1.00), 3.463 (0.80), 3.727 (0.63), 3.744 (1.40), 3.748 (1.24), 3.765 (1.96), 3.781 (0.89), 3.821 (0.97), 3.837 (2.12), 3.853 (1.19), 3.857 (1.46), 3.874 (0.63), 3.988 (0.56), 4.005 (1.54), 4.010 (1.48), 4.029 (0.74), 4.279 (0.44), 4.289 (0.76), 4.297 (1.17), 4.313 (3.41), 4.327 (0.62), 4.335 (1.70), 4.343 (1.07), 6.191 (1.80), 6.195 (1.84), 6.211 (1.98), 6.214 (1.86), 6.705 (1.10), 6.708 (1.37), 6.725 (3.31), 6.728 (2.75), 6.743 (2.35), 6.762 (2.61), 6.782 (0.88), 7.188 (1.84), 7.217 (3.06), 7.230 (3.05), 7.333 (4.38), 7.988 (1.94), 8.000 (1.85), 8.388 (3.04), 10.991 (2.43).

### Example 60

### 3-(3-chloro-2-methyl-anilino)-7-methyl-2-(3-{[(2S)-oxolan-2-yl]methoxy}pyridin-4-yl)-1,5,6,7-tetrahydropyrrolo[3,2-c]pyridin-4-one (stereoisomer 2)

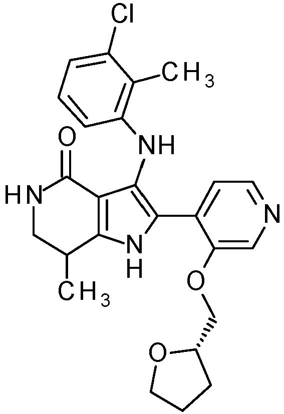

For the preparation of the title compound as mixture of stereoisomers see example 58. Separation of enantiomers by preparative chiral HPLC (method see example 59) to give 17.0 mg (97% purity, 40% yield) of the title compound (enantiomer 2, Rₜ = 8.5 - 10.2 min).

Analytical chiral HPLC (method see example 59): Rₜ = 2.50 min.

¹H-NMR (400 MHz, DMSO-d₆) δ [ppm]: 0.697 (0.54), 1.153 (0.49), 1.232 (1.18), 1.270 (5.58), 1.285 (5.57), 1.642 (0.52), 1.653 (0.51), 1.661 (0.66), 1.669 (0.81), 1.674 (0.67), 1.691 (0.86), 1.708 (0.47), 1.850 (0.41), 1.858 (0.49), 1.867 (1.05), 1.874 (1.15), 1.888 (1.92), 1.905 (1.61), 1.922 (0.55), 1.926 (0.53), 1.979 (0.42), 1.997 (0.61), 2.010 (0.77), 2.028 (0.71), 2.043 (0.57), 2.322 (0.72), 2.327 (1.10), 2.338 (16.00), 2.518 (2.42), 2.522 (1.51), 2.664 (0.52), 2.669 (0.72), 2.673 (0.52), 3.063 (0.49), 3.082 (2.08), 3.098 (1.66), 3.106 (1.32), 3.460 (0.93), 3.467 (0.92), 3.476 (0.98), 3.483 (0.76), 3.740 (0.63), 3.757 (1.22), 3.760 (1.29), 3.777 (1.83), 3.794 (0.94), 3.820 (0.99), 3.836 (2.13), 3.853 (1.22), 3.856 (1.39), 3.873 (0.58), 3.956 (0.91), 3.976 (1.58), 3.980 (1.52), 4.001 (1.12), 4.290 (0.43), 4.300 (0.75), 4.308 (1.05), 4.319 (0.66), 4.327 (1.12), 4.340 (1.85), 4.362 (1.46), 4.370 (1.12), 6.193 (1.84), 6.196 (1.82), 6.212 (2.01), 6.216 (1.87), 6.702 (1.12), 6.706 (1.41), 6.723 (3.32), 6.727 (2.70), 6.741 (2.37), 6.761 (2.64), 6.780 (0.88), 7.172 (2.12), 7.214 (2.69), 7.226 (2.71), 7.315 (4.47), 7.987 (1.46), 8.000 (1.40), 8.385 (2.25), 11.037 (2.47).

### Example 61

### 3-(3-chloro-2-methyl-anilino)-2-[3-[(3,3-difluorotetrahydropyran-2-yl)methoxy]-4-pyridyl]-7-methyl-1,5,6,7-tetrahydropyrrolo[3,2-c]pyridin-4-one

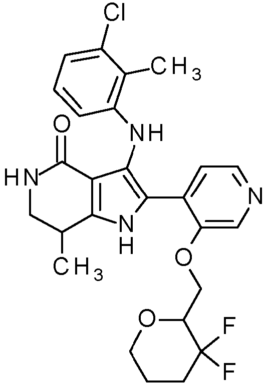

According to the method described for example 52 using N-(3-chloro-2-methyl-phenyl)-4-({3-[(3,3-difluorotetrahydropyran-2-yl)methoxy]-4-pyridyl}methylamino)-3-methyl-6-oxo-2,3-dihydro-1H-pyridine-5-carbothioamide (intermediate 6-61, 45.0 mg, 81.7 µmol) as starting material, 8.0 mg (98% purity, 19% yield) of the title compound were prepared after purification by preparative HPLC (method 10, gradient: 0.00-0.50 min 30% B, 0.50-6.00 min 30-70% B).

LC-MS (method 2): Rₜ = 1.16 min; MS (ESlpos): m/z = 517 [M+H]⁺

¹H-NMR (400 MHz, DMSO-d₆) δ [ppm]: 1.166 (0.49), 1.234 (1.04), 1.276 (9.40), 1.291 (9.46), 1.799 (3.46), 2.016 (0.66), 2.085 (0.68), 2.229 (1.27), 2.339 (16.00), 2.671 (1.27), 3.097 (3.37), 3.111 (3.61), 3.485 (1.93), 3.500 (1.82), 3.530 (1.23), 3.565 (1.59), 3.964 (1.80), 3.992 (1.63), 4.089 (1.19), 4.166 (0.83), 4.206 (1.08), 4.229 (1.83), 4.253 (1.91), 4.272 (0.96), 4.415 (1.27), 4.436 (1.02), 4.466 (1.48), 4.489 (1.13), 6.166 (3.03), 6.185 (3.23), 6.707 (1.48), 6.731 (5.16), 6.755 (2.67), 6.773 (0.93), 7.168 (2.40), 7.182 (2.50), 7.243 (2.63), 7.255 (4.65), 7.267 (2.52), 7.353 (3.37), 7.379 (3.57), 8.023 (2.69), 8.037 (4.84), 8.049 (2.46), 8.412 (7.55), 10.849 (2.97).

### Example 62

### 3-(3-chloro-2-methyl-anilino)-2-[3-[(5,5-dimethyl-1,4-dioxan-2-yl)methoxy]-4-pyridyl]-7-methyl-1,5,6,7-tetrahydropyrrolo[3,2-c]pyridin-4-one

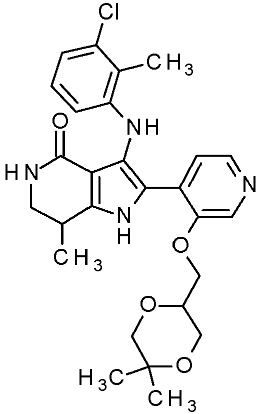

According to the method described for example 52 using N-(3-chloro-2-methyl-phenyl)-4-({3-[(5,5-dimethyl-1,4-dioxan-2-yl)methoxy]-4-pyridyl}methylamino)-3-methyl-6-oxo-2,3-dihydro-1H-pyridine-5-carbothioamide (intermediate 6-62, 160 mg, 294 µmol) as starting material, 39.0 mg (98% purity, 25% yield) of the title compound were prepared after purification by preparative HPLC (method 10, gradient: 0.00-0.50 min 35% B, 0.50-7.00 min 35-55% B).

LC-MS (method 2): Rₜ = 1.12 min; MS (ESlpos): m/z = 511 [M+H]⁺

¹H-NMR (400 MHz, DMSO-d₆) δ [ppm]: -0.008 (1.04), 0.008 (1.03), 1.062 (16.00), 1.213 (9.76), 1.229 (9.14), 1.260 (3.91), 1.270 (5.14), 1.276 (4.95), 1.285 (4.23), 2.077 (1.88), 2.328 (13.18), 2.333 (12.67), 2.520 (2.69), 2.525 (1.81), 2.667 (0.62), 2.671 (0.85), 2.676 (0.62), 3.076 (2.12), 3.094 (1.95), 3.347 (1.37), 3.367 (1.64), 3.375 (1.48), 3.464 (1.20), 3.473 (1.24), 3.481 (1.00), 3.553 (0.68), 3.567 (2.54), 3.575 (0.79), 3.583 (1.47), 3.591 (3.82), 3.595 (3.29), 3.605 (1.34), 3.619 (2.86), 3.634 (1.26), 3.643 (1.40), 3.659 (1.45), 3.673 (0.71), 3.689 (0.83), 3.824 (0.51), 3.837 (0.59), 3.848 (0.59), 3.863 (0.71), 3.871 (0.65), 3.880 (0.61), 3.888 (0.58), 3.897 (0.42), 4.100 (0.69), 4.117 (0.65), 4.126 (1.10), 4.144 (1.21), 4.170 (1.54), 4.179 (1.82), 4.184 (1.90), 4.189 (1.74), 4.198 (1.19), 4.206 (1.28), 4.224 (0.68), 4.232 (0.59), 6.178 (1.24), 6.185 (1.61), 6.189 (1.37), 6.197 (1.41), 6.204 (1.68), 6.208 (1.38), 6.686 (0.71), 6.690 (1.07), 6.696 (0.92), 6.705 (2.61), 6.711 (3.27), 6.716 (2.31), 6.719 (2.41), 6.740 (2.46), 6.760 (0.79), 7.154 (1.98), 7.217 (2.65), 7.225 (2.98), 7.230 (2.79), 7.237 (2.78), 7.305 (3.26), 7.337 (3.02), 8.016 (3.36), 8.023 (3.72), 8.028 (3.17), 8.036 (3.25), 8.369 (4.74), 8.374 (5.02), 10.944 (1.95), 10.963 (1.79).

### Example 63

### (6R)-3-[2-(2,2-difluoroethyl)-3-fluoroanilino]-6-methyl-2-(3-{[(2S)-oxolan-2-yl]methoxy}pyridin-4-yl)-1,5,6,7-tetrahydro-4H-pyrrolo[3,2-c]pyridin-4-one

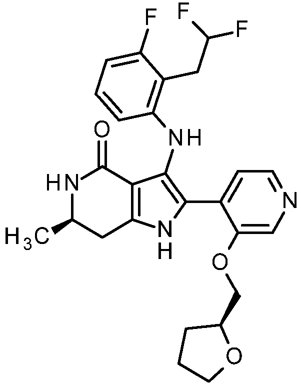

(6*R*)-*N*-[2-(2,2-difluoroethyl)-3-fluorophenyl]-6-methyl-2-oxo-4-{[(3-{[(2*S*)-oxolan-2-yl]methoxy}pyridin-4-yl)methyl]amino}-1,2,5,6-tetrahydropyridine-3-carbothioamide (intermediate 6-63, 90.0 mg, 168 µmol) was dissolved in methanol (430 µl), TFA (13 µl, 170 µmol), 2,6-di-tert-butyl-4-methylphenol (37.1 mg, 168 µmol, CAS 128-37-0) and hydrogen peroxide (34 µl, 30%, 340 µmol) were added and the mixture was stirred for 30 min at 100°C. To the mixture was added aqueous sodiumthiosulfate and potassiumcarbonate solution, followed by extraction with DCM / MeOH. The organic layer was separated, evaporated under reduced pressure and dried. The residue was purified by flash chromatography (silica, DCM / MeOH gradient 0-7 %) to give 21.0 mg (25% yield) of the title compound.

LC-MS (method 2): Rₜ = 1.08 min; MS (ESlpos): m/z = 501 [M+H]⁺

¹H-NMR (400 MHz, DMSO-d₆) δ ppm: 1.23 (d, 3H), 1.62 - 1.75 (m, 1H), 1.83 - 1.94 (m, 2H), 1.95 - 2.08 (m, 1H), 2.59 (dd, 1H), 2.91 (dd, 1H), 3.24 - 3.37 (m, 2H), 3.73 - 3.81 (m, 2H), 3.83 - 3.90 (m, 1H), 4.03 (dd, 1H), 4.26 - 4.40 (m, 2H), 6.13 (d, 1H), 6.25 - 6.62 (m, 2H), 6.83 - 6.93 (m, 1H), 7.03 (s, 1H), 7.27 (s, 1H), 7.35 (d, 1H), 8.00 (d, 1H), 8.41 (s, 1H), 11.22 (s, 1H).

### Example 64

### (5aR,8aS)-3-(3-chloro-2-methoxyanilino)-2-(3-{[(2S)-4-methylmorpholin-2-yl]methoxy}pyridin-4-yl)-5,5a,6,7,8,8a-hexahydrocyclopenta[b]pyrrolo[2,3-d]pyridin-4(1H)-one

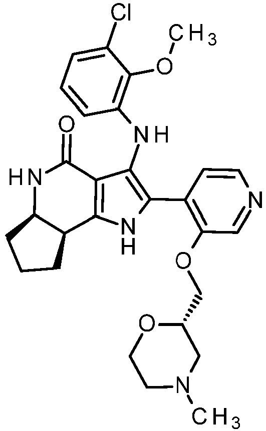

*Tert*-butyl (2*S*)-2-[({4-[(5a*R*,8a*S*)-3-(3-chloro-2-methoxyanilino)-4-oxo-1,4,5,5a,6,7,8,8a-octahydrocyclopenta[b]pyrrolo[2,3-d]pyridin-2-yl]pyridin-3-yl}oxy)methyl]morpholine-4-carboxylate (intermediate 64-2, 40.0 mg, 76.3 µmol) was supended in methanol (1.0 ml), formaldehyde (23 µl, 37% purity in water, 310 µmol) and acetic acid (4.4 µl, 76 µmol) was added and the mixture stirred for 15 min at room temperature. To the mixture was added sodium triacetoxyborohydride (48.5 mg, 229 µmol) and it was stirred for 12 h at room temperature. The reaction mixture was filtred through a SCX coloumn (2 g) and washed ammonia in methanol (7 M). The filtrate was concentrated and purified by preparative HPLC (method 10, gradient: 0.00-0.50 min 30% B, 0.50-7.00 min 30-70% B) to give 27.4 mg (95% purity, 63% yield) of the title compound.

LC-MS (method 2): Rₜ = 1.06 min; MS (ESlpos): m/z = 538 [M+H]⁺

¹H-NMR (400 MHz, DMSO-d₆) δ [ppm]: 1.63 - 1.85 (m, 5H), 1.85 - 1.96 (m, 2H), 1.99 - 2.15 (m, 2H), 2.19 (s, 3H), 2.65 (br d, 1H), 2.75 (br d, 1H), 3.21 - 3.29 (m, 1H), 3.64 (td, 1H), 3.88 (s, 3H), 3.88 - 4.00 (m, 2H), 4.04 - 4.11 (m, 1H), 4.16 - 4.29 (m, 2H), 6.13 (t, 1H), 6.68 (d, 2H), 7.07 - 7.13 (m, 1H), 7.25 (d, 1H), 7.59 (s, 1H), 8.02 (d, 1H), 8.40 (s, 1H), 10.93 (s, 1H).

### Example 65

### (6R)-3-[2-(2,2-difluoroethyl)-3-fluoroanilino]-2-[3-(2-methoxy-2-methylpropoxy)pyridin-4-yl]-6-methyl-1,5,6,7-tetrahydro-4H-pyrrolo[3,2-c]pyridin-4-one

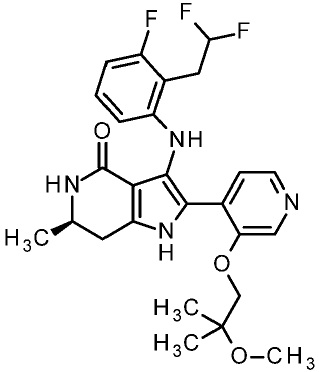

According to the method described for example 63 using (6R)-N-[2-(2,2-difluoroethyl)-3-fluorophenyl]-4-({[3-(2-methoxy-2-methylpropoxy)pyridin-4-yl]methyl}amino)-6-methyl-2-oxo-1,2,5,6-tetrahydropyridine-3-carbothioamide (intermediate 6-65, 110 mg, 205 µmol) as starting material, 25.0 mg (95% purity, 23% yield) of the title compound were prepared after purification by preparative HPLC (method 10, gradient: 0.00-0.50 min 30% B, 0.50-7.00 min 30-70% B) and following flash chromatography (silica, DCM / EtOH gradient 0-10 %).

LC-MS (method 2): Rₜ = 1.16 min; MS (ESlneg): m/z = 501 [M-H]⁻

¹H-NMR (400 MHz, DMSO-d₆) δ [ppm]: 1.224 (4.83), 1.240 (5.00), 1.263 (10.48), 1.276 (10.13), 2.522 (0.53), 2.568 (0.66), 2.594 (0.71), 2.608 (0.83), 2.634 (0.85), 2.862 (0.80), 2.874 (0.87), 2.902 (0.70), 2.914 (0.64), 3.247 (16.00), 3.278 (0.53), 3.287 (0.57), 3.361 (0.53), 3.769 (0.54), 3.782 (0.59), 3.795 (0.49), 4.135 (0.86), 4.160 (2.50), 4.179 (2.54), 4.203 (0.82), 6.113 (1.61), 6.133 (1.64), 6.452 (0.95), 6.473 (1.64), 6.495 (0.82), 6.867 (0.54), 6.887 (1.12), 6.905 (1.08), 6.925 (0.46), 7.070 (2.24), 7.349 (2.89), 7.374 (2.36), 7.386 (2.34), 7.977 (2.77), 7.990 (2.50), 8.401 (4.21), 11.154 (1.87).

### Example 66

### 3-(3-chloro-2-methyl-anilino)-2-[3-(1,4-dioxan-2-ylmethoxy)-4-pyridyl]-7-methyl-1,5,6,7-tetrahydropyrrolo[3,2-c]pyridin-4-one

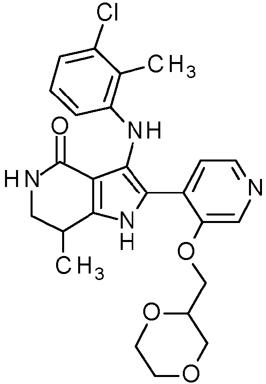

According to the method described for example 52 using N-(3-chloro-2-methyl-phenyl)-*4-{[3-(1,4-dioxan-2-ylmethoxy)-4-pyridyl]methylamino}-3-methyl-6-oxo-2,3-dihydro-1H-*pyridine-5-carbothioamide (intermediate 6-66, 144 mg, 219 µmol) as starting material, 20.7 mg (99% purity, 40% yield) of the title compound were prepared after purification by preparative HPLC (method 10, gradient: 0.00-0.50 min 15% B, 0.50-6.00 min 15-55% B)..

LC-MS (method 2): Rₜ = 1.03 min; MS (ESlpos): m/z = 483 [M+H]⁺

¹H-NMR (400 MHz, DMSO-d₆) δ [ppm]: -0.008 (1.05), 0.008 (0.96), 1.239 (0.64), 1.257 (0.73), 1.274 (6.36), 1.289 (6.41), 2.093 (0.68), 2.151 (0.85), 2.339 (16.00), 2.519 (3.48), 2.524 (2.23), 2.542 (0.49), 2.666 (0.60), 2.671 (0.82), 2.675 (0.59), 3.091 (2.46), 3.108 (2.37), 3.364 (0.92), 3.389 (1.03), 3.393 (1.07), 3.402 (0.86), 3.417 (1.01), 3.427 (1.05), 3.431 (1.05), 3.455 (1.31), 3.484 (1.34), 3.493 (1.16), 3.504 (1.18), 3.511 (1.72), 3.517 (1.14), 3.539 (0.94), 3.650 (0.45), 3.657 (0.71), 3.666 (0.57), 3.690 (2.81), 3.707 (0.83), 3.717 (1.88), 3.783 (0.88), 3.790 (1.04), 3.796 (1.04), 3.803 (1.70), 3.818 (1.98), 3.825 (1.37), 3.832 (1.38), 3.846 (0.75), 3.953 (0.48), 3.959 (0.53), 3.972 (0.64), 3.978 (0.70), 3.989 (0.71), 3.997 (0.78), 4.005 (0.56), 4.013 (0.42), 4.023 (0.40), 4.066 (0.81), 4.084 (0.52), 4.092 (1.07), 4.109 (0.77), 4.150 (2.94), 4.163 (2.42), 4.179 (1.05), 4.189 (1.00), 4.205 (0.66), 4.214 (0.62), 6.174 (2.03), 6.193 (2.18), 6.701 (1.04), 6.705 (1.38), 6.721 (3.70), 6.724 (3.18), 6.735 (2.90), 6.754 (2.96), 6.775 (1.00), 7.173 (1.96), 7.180 (1.93), 7.217 (3.82), 7.230 (3.78), 7.356 (2.87), 7.365 (2.82), 8.009 (2.79), 8.014 (2.97), 8.021 (2.75), 8.027 (2.81), 8.363 (3.98), 8.369 (4.16), 10.904 (1.51), 10.924 (1.51).

### Example 67

### 3-(3-chloro-2-methyl-anilino)-2-[3-(1,4-dioxan-2-ylmethoxy)-4-pyridyl]-7-methyl-1,5,6,7-tetrahydropyrrolo[3,2-c]pyridin-4-one (stereoisomer 1)

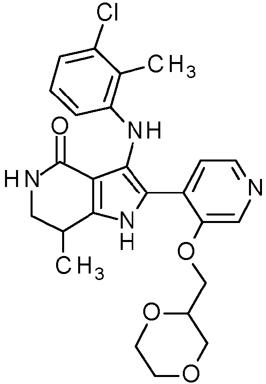

The title compound from example 66 (20.7 mg) was separated into enantiomers by preparative chiral HPLC to give 3.8 mg (90 % purity, 16 % yield) of the title compound (enantiomer 1, Rₜ = 5.8 - 7.1 min), enantiomer 2 (3.5 mg, Rₜ = 7.2 - 8.0 min, see example 68), enantiomer 3 (3.7 mg, Rt = 11.6 - 13.5 min, see example 69) and enantiomer 4 (3.4 mg, Rₜ = 18.4 - 21.1 min, see example 70).

### Preparative chiral HPLC method:

Instrument: PrepCon Labomatic HPLC; Column: YMC Cellulose SB 5µ, 250x30; eluent A: methyl *tert*-butyl ether + 0.1 vol % diethylamine; eluent B: acetonitrile + 0.1 vol % diethylamine; isocratic: 50%A+50%B; flow: 50 ml/min; temperature: 25°C; UV: 254 nm

### Analytical chiral HPLC method:

Instrument: Waters Alliance 2695; Column: YMC Cellulose SB 3µ, 100x4.6; eluent A: methyl tert-butyl ether + 0.1 vol % diethylamine; eluent B: acetonitrile; isocratic: 50%A+50%B; flow: 1.4 ml/min; temperature: 25°C; UV: 254 nm
Analytical chiral HPLC: Rₜ = 2.04 min.
Optical rotation:[α]_{D} = 12.14 ° +/- 0.26° (c = 7 mg/ml in CHLOROFORM)
¹H-NMR (400 MHz, DMSO-d₆) δ [ppm]: 1.27 - 1.30 (m, 3H), 2.34 (s, 3H), 3.07 - 3.13 (m, 2H), 3.43 - 3.55 (m, 3H), 3.64 - 3.73 (m, 2H), 3.82 (s, 2H), 3.93 - 4.02 (m, 1H), 4.13 - 4.20 (m, 2H), 6.19 (dd, 1H), 6.69 - 6.78 (m, 2H), 7.18 (s, 1H), 7.23 (d, 1H), 7.38 (s, 1H), 7.98 - 8.06 (m, 1H), 8.37 (s, 1H), 10.86 - 11.00 (m, 1H).

### Example 68

### 3-(3-chloro-2-methyl-anilino)-2-[3-(1,4-dioxan-2-ylmethoxy)-4-pyridyl]-7-methyl-1,5,6,7-tetrahydropyrrolo[3,2-c]pyridin-4-one (stereoisomer 2)

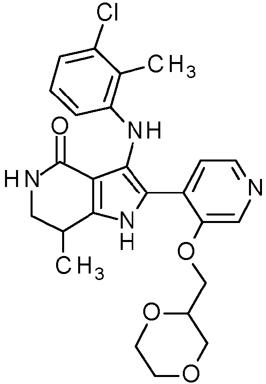

For the preparation of the title compound as mixture of stereoisomers see example 66. Separation of enantiomers by preparative chiral HPLC (method see example 67) to give 3.5 mg (90% purity, 15% yield) of the title compound (enantiomer 2, Rₜ = 7.2 - 8.0 min).

Analytical chiral HPLC (method see example 67): Rₜ = 2.31 min.

Optical rotation:[α]_{D} = 60.4 ° +/- 0.52° (c = 4 mg/ml in chloroform)

¹H-NMR (400 MHz, DMSO-d₆) δ [ppm]: 1.231 (0.59), 1.272 (1.50), 1.288 (1.52), 2.340 (4.06), 3.093 (0.55), 3.108 (0.57), 3.386 (0.66), 3.390 (0.63), 3.415 (0.49), 3.691 (0.82), 3.716 (0.50), 3.795 (0.40), 3.801 (0.73), 3.830 (0.52), 4.093 (0.45), 4.181 (0.41), 6.170 (0.45), 6.174 (0.45), 6.190 (0.49), 6.193 (0.47), 6.723 (0.87), 6.727 (0.72), 6.735 (0.67), 6.754 (0.63), 7.188 (0.56), 7.222 (0.76), 7.234 (0.77), 7.381 (1.01), 8.013 (0.60), 8.026 (0.57), 8.316 (16.00), 8.365 (0.97), 10.930 (0.67).

### Example 69

### 3-(3-chloro-2-methyl-anilino)-2-[3-(1,4-dioxan-2-ylmethoxy)-4-pyridyl]-7-methyl-1,5,6,7-tetrahydropyrrolo[3,2-c]pyridin-4-one (stereoisomer 3)

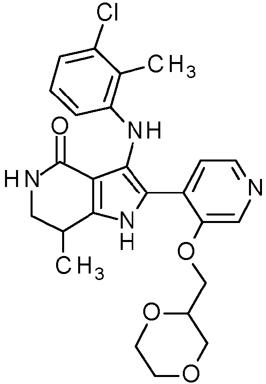

For the preparation of the title compound as mixture of stereoisomers see example 66. Separation of enantiomers by preparative chiral HPLC (method see example 67) to give 3.7 mg (90% purity, 16% yield) of the title compound (enantiomer 3, Rₜ = 11.6 - 13.5 min).

Analytical chiral HPLC (method see example 67): Rₜ = 3.51 min.

Optical rotation:[α]_{D} = - 46.77 ° +/- 0.74° (c = 5 mg/ml in chloroform)

¹H-NMR (400 MHz, DMSO-d₆) δ [ppm]: 0.833 (0.47), 0.851 (0.63), 0.864 (0.46), 1.013 (1.86), 1.087 (0.52), 1.138 (1.38), 1.156 (1.97), 1.174 (1.41), 1.200 (2.04), 1.232 (3.07), 1.272 (6.15), 1.288 (6.07), 1.752 (0.49), 2.340 (16.00), 2.522 (1.34), 2.669 (0.52), 3.093 (2.27), 3.108 (2.34), 3.126 (0.73), 3.361 (4.39), 3.386 (2.48), 3.390 (2.40), 3.415 (1.89), 3.461 (0.77), 3.480 (1.63), 3.489 (1.83), 3.495 (1.41), 3.509 (1.66), 3.516 (1.61), 3.539 (1.19), 3.657 (0.76), 3.664 (0.91), 3.691 (3.33), 3.715 (2.01), 3.795 (1.68), 3.801 (2.97), 3.811 (1.21), 3.823 (1.46), 3.830 (2.12), 3.982 (0.57), 3.990 (0.76), 3.998 (0.99), 4.006 (0.97), 4.015 (0.69), 4.023 (0.69), 4.030 (0.47), 4.067 (1.35), 4.085 (0.91), 4.092 (1.79), 4.110 (1.32), 4.181 (1.64), 4.189 (1.59), 4.206 (1.17), 4.215 (0.98), 6.170 (1.84), 6.174 (1.79), 6.189 (1.99), 6.193 (1.87), 6.703 (0.91), 6.707 (1.24), 6.723 (3.40), 6.727 (2.85), 6.735 (2.62), 6.754 (2.51), 6.775 (0.84), 7.188 (2.29), 7.222 (2.68), 7.235 (2.73), 7.379 (4.14), 8.014 (1.77), 8.026 (1.68), 8.317 (3.56), 8.366 (2.79), 10.930 (2.72).

### Example 70

### 3-(3-chloro-2-methyl-anilino)-2-[3-(1,4-dioxan-2-ylmethoxy)-4-pyridyl]-7-methyl-1,5,6,7-tetrahydropyrrolo[3,2-c]pyridin-4-one (stereoisomer 4)

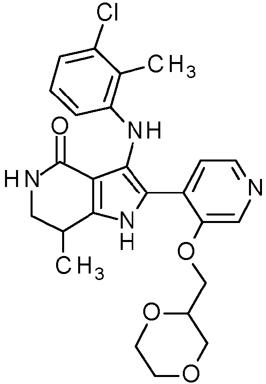

For the preparation of the title compound as mixture of stereoisomers see example 66. Separation of enantiomers by preparative chiral HPLC (method see example 67) to give 3.4 mg (90% purity, 15% yield) of the title compound (enantiomer 4, Rₜ = 18.6 - 21.1 min).

Analytical chiral HPLC (method see example 67): Rₜ = 5.89 min.

Optical rotation:[α]_{D} = - 12.63 ° +/- 0.69° (c = 6 mg/ml in chloroform)

¹H-NMR (400 MHz, DMSO-d₆) δ [ppm]: 1.28 (d, 3H), 2.34 (s, 3H), 3.06 - 3.14 (m, 2H), 3.43 - 3.54 (m, 3H), 3.64 - 3.74 (m, 2H), 3.77 - 3.86 (m, 2H), 3.93 - 4.01 (m, 1H), 4.16 (d, 2H), 6.18 (dd, 1H), 6.68 - 6.78 (m, 2H), 7.17 (s, 1H), 7.22 (d, 1H), 7.36 (s, 1H), 8.02 (d, 1H), 8.37 (s, 1H), 10.90 (s, 1H).

### Example 71

### (6R)-3-(3-chloro-2-methoxy-anilino)-2-{3-[(3,3-difluorotetrahydrofuran-2-yl)methoxy]-4-pyridyl}-6-methyl-1,5,6,7-tetrahydropyrrolo[3,2-c]pyridin-4-one

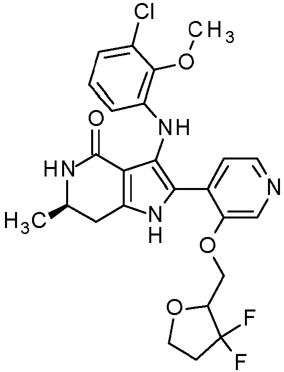

(2*R*)-*N*-(3-Chloro-2-methoxy-phenyl)-4-({3-[(3,3-difluorotetrahydrofuran-2-yl)methoxy]-4-pyridyl}methylamino)-2-methyl-6-oxo-2,3-dihydro-1*H*-pyridine-5-carbothioamide (intermediate 6-71, 450 mg, 814 µmol) was dissolved in methanol (14.1 ml), TFA (92 µl, 1.2 mmol) and hydrogen peroxide (139 µl, 35%, 1.6 mmol) were added and the mixture was stirred over night at 60°C. To the mixture were added two drops of ammonia in methanol (7 M) to adjust a basic pH. Sodiumthiosulfate solution (2 ml), water and DCM were added and the mixture stirred for 15 min at room temperature. The layers were separated, the aqueous layer was extracted with DCM, the combined organic layer was filtered over a water repellant filter and concentrated under reduced pressure. The residue was triturated with a small amount of EtOH and the resulting precipitate was filtered off and dried to give 260 mg (96% purity, 59% yield) of the title compound.

LC-MS (method 2): Rₜ = 1.10 min; MS (ESlpos): m/z = 519 [M+H]⁺

¹H-NMR (400 MHz, DMSO-d₆) δ [ppm]: 1.225 (5.43), 1.241 (5.49), 2.434 (0.40), 2.449 (0.50), 2.518 (1.66), 2.522 (1.22), 2.533 (0.80), 2.545 (0.72), 2.560 (0.57), 2.572 (0.91), 2.585 (0.59), 2.600 (0.55), 2.611 (0.55), 2.869 (0.66), 2.879 (0.72), 2.910 (0.56), 2.919 (0.53), 3.761 (0.54), 3.776 (0.60), 3.788 (0.50), 3.856 (16.00), 3.948 (0.70), 3.961 (0.79), 3.967 (0.78), 3.981 (0.77), 4.104 (0.57), 4.114 (0.64), 4.125 (1.04), 4.136 (1.03), 4.147 (0.47), 4.158 (0.42), 4.276 (0.50), 4.285 (0.61), 4.299 (0.89), 4.309 (0.53), 4.328 (0.52), 4.385 (0.80), 4.394 (1.05), 4.413 (0.99), 4.438 (0.82), 4.462 (0.55), 6.124 (0.85), 6.132 (0.99), 6.136 (1.22), 6.143 (1.39), 6.148 (0.98), 6.156 (0.93), 6.635 (2.06), 6.637 (2.63), 6.640 (2.58), 6.642 (2.55), 6.648 (3.18), 6.653 (3.03), 7.083 (2.51), 7.275 (2.42), 7.287 (2.46), 7.405 (2.17), 7.419 (2.09), 8.057 (3.78), 8.069 (3.36), 8.445 (2.79), 8.451 (2.97), 11.055 (1.13), 11.074 (1.08).

### Example 72

### (6R)-3-(3-chloro-2-methoxy-anilino)-2-{3-[(3,3-difluorotetrahydrofuran-2-yl)methoxy]-4-pyridyl}-6-methyl-1,5,6,7-tetrahydropyrrolo[3,2-c]pyridin-4-one (stereoisomer 1)

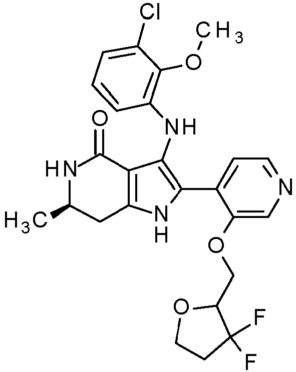

The title compound from example 71 (260 mg) was separated into enantiomers by preparative chiral HPLC to give 113.0 mg (90% purity, 39% yield) of the title compound (enantiomer 1, Rₜ = 5.3 - 5.9 min) and enantiomer 2 (114.0 mg, Rₜ = 6.5 - 7.3 min, see example 73).

### Preparative chiral HPLC method:

Instrument: PrepCon Labomatic HPLC; Column: YMC Cellulose SB 10µ, 250x50; eluent A: methyl tert-butyl ether + 0.1 vol % diethylamine; eluent B: acetonitrile; isocratic: 50%A+50%B; flow: 150 mL/min; temperature: 25°C; UV: 280 nm

### Analytical chiral HPLC method:

Instrument: Waters Alliance 2695; Column: YMC Cellulose SB 3µ, 100x4.6; eluent A: methyl tert-butyl ether + 0.1 vol % diethylamine; eluent B: acetonitrile; isocratic: 50%A+50%B; flow: 1.4 ml/min; temperature: 25°C; UV: 280 nm
Analytical chiral HPLC: Rₜ = 1.55 min.
Optical rotation:[α]_{D} = 29.88 ° +/- 0.55° (c = 5.2 mg/ml in chloroform)
¹H-NMR (400 MHz, DMSO-d₆) δ [ppm]: 1.137 (1.28), 1.156 (1.04), 1.225 (5.61), 1.241 (5.55), 2.074 (0.42), 2.084 (1.01), 2.322 (0.55), 2.327 (0.72), 2.331 (0.55), 2.392 (0.44), 2.413 (0.50), 2.434 (0.66), 2.456 (0.89), 2.522 (2.43), 2.532 (1.42), 2.559 (1.01), 2.572 (1.04), 2.600 (1.00), 2.664 (0.52), 2.668 (0.68), 2.673 (0.50), 2.869 (0.89), 2.882 (1.00), 2.910 (0.81), 2.922 (0.73), 3.763 (0.60), 3.776 (0.68), 3.789 (0.58), 3.854 (16.00), 3.926 (0.55), 3.948 (1.30), 3.967 (1.41), 3.988 (0.63), 4.104 (0.64), 4.115 (0.72), 4.125 (1.16), 4.137 (1.16), 4.148 (0.54), 4.158 (0.48), 4.283 (0.43), 4.298 (0.79), 4.308 (0.92), 4.327 (1.00), 4.384 (1.17), 4.393 (1.63), 4.412 (1.65), 4.434 (0.45), 6.123 (1.45), 6.135 (2.08), 6.147 (1.50), 6.635 (3.63), 6.637 (3.83), 6.648 (5.21), 7.084 (2.57), 7.275 (2.69), 7.287 (2.71), 7.418 (3.96), 8.056 (3.01), 8.069 (2.81), 8.444 (4.62), 11.075 (2.13).

### Example 73

### (6R)-3-(3-chloro-2-methoxy-anilino)-2-{3-[(3,3-difluorotetrahydrofuran-2-yl)methoxy]-4-pyridyl}-6-methyl-1,5,6,7-tetrahydropyrrolo[3,2-c]pyridin-4-one (stereoisomer 2)

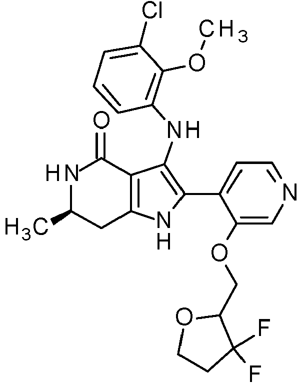

For the preparation of the title compound as mixture of stereoisomers see example 71. Separation of enantiomers by preparative chiral HPLC (method see example 72) to give 114.0 mg (90% purity, 39% yield) of the title compound (enantiomer 2, Rₜ = 6.5 - 7.3 min).

Analytical chiral HPLC (method see example 72): Rₜ = 1.92 min.

Optical rotation:[α]_{D} = 36.68 ° +/- 0.61° (c = 3.2 mg/ml in chloroform)

¹H-NMR (400 MHz, DMSO-d₆) δ [ppm]: 0.745 (0.48), 0.766 (0.49), 1.137 (1.00), 1.155 (1.48), 1.224 (5.47), 1.241 (5.43), 2.406 (0.41), 2.427 (0.48), 2.448 (0.73), 2.470 (1.13), 2.522 (1.83), 2.544 (1.09), 2.571 (0.92), 2.585 (1.00), 2.611 (0.96), 2.865 (0.88), 2.878 (0.96), 2.905 (0.78), 2.918 (0.72), 3.759 (0.57), 3.772 (0.65), 3.785 (0.54), 3.856 (16.00), 3.939 (0.53), 3.961 (1.28), 3.980 (1.37), 4.001 (0.63), 4.102 (0.61), 4.113 (0.71), 4.124 (1.13), 4.135 (1.11), 4.146 (0.53), 4.157 (0.46), 4.258 (0.49), 4.274 (0.83), 4.285 (0.81), 4.299 (0.94), 4.384 (0.48), 4.394 (0.50), 4.401 (0.49), 4.410 (0.44), 4.417 (0.51), 4.439 (1.33), 4.462 (0.89), 4.471 (0.66), 6.131 (1.42), 6.143 (2.26), 6.155 (1.47), 6.642 (3.98), 6.653 (4.95), 7.084 (2.44), 7.274 (2.62), 7.286 (2.64), 7.405 (3.92), 8.056 (2.84), 8.068 (2.65), 8.450 (4.39), 11.055 (2.05).

### Example 74

### (6R)-3-(3-chloro-2-methoxy-anilino)-2-{3-[(3,3-difluorotetrahydropyran-2-yl)methoxy]-4-pyridyl}-6-methyl-1,5,6,7-tetrahydropyrrolo[3,2-c]pyridin-4-one

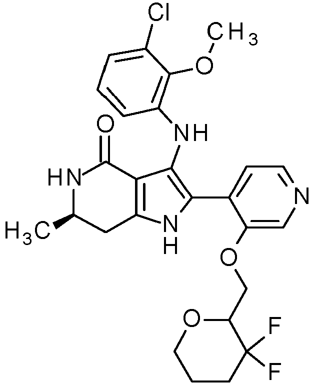

According to the method described for example 71 using (2R)-N-(3-chloro-2-methoxyphenyl)-4-({3-[(3,3-difluorotetrahydropyran-2-yl)methoxy]-4-pyridyl}methylamino)-2-methyl-6-oxo-2,3-dihydro-1H-pyridine-5-carbothioamide (intermediate 6-74, 450 mg, 794 µmol) as starting material, 309 mg (95% purity, 69% yield) of the title compound were prepared.

LC-MS (method 2): Rₜ = 1.20 min; MS (ESlpos): m/z = 533 [M+H]⁺

¹H-NMR (400 MHz, DMSO-d6) δ [ppm]: 0.851 (0.53), 1.232 (7.08), 1.248 (5.01), 1.807 (1.06), 1.829 (0.81), 2.518 (0.82), 2.522 (0.55), 2.570 (0.41), 2.575 (0.42), 2.596 (0.44), 2.601 (0.46), 2.610 (0.54), 2.615 (0.53), 2.636 (0.52), 2.641 (0.53), 2.891 (0.48), 2.896 (0.51), 2.903 (0.57), 2.908 (0.52), 2.931 (0.43), 2.936 (0.44), 2.944 (0.42), 3.623 (0.42), 3.777 (0.49), 3.791 (0.55), 3.804 (0.46), 3.880 (16.00), 4.008 (0.48), 4.037 (0.40), 4.139 (0.49), 4.198 (0.64), 4.214 (0.50), 4.241 (0.90), 4.260 (0.60), 4.269 (0.52), 4.530 (0.47), 4.536 (0.48), 4.557 (0.41), 4.595 (0.48), 4.600 (0.48), 4.620 (0.43), 6.122 (0.87), 6.133 (1.25), 6.143 (1.45), 6.155 (0.91), 6.671 (1.85), 6.673 (2.05), 6.680 (2.83), 6.684 (2.89), 6.690 (1.97), 6.692 (1.94), 7.126 (1.81), 7.292 (1.57), 7.295 (1.55), 7.305 (1.60), 7.308 (1.54), 7.531 (1.91), 7.545 (1.82), 8.044 (3.00), 8.057 (2.80), 8.447 (2.84), 8.450 (2.95), 10.923 (0.95), 10.960 (0.92).

### Example 75

### (6R)-3-(3-chloro-2-methoxy-anilino)-2-{3-[(3,3-difluorotetrahydropyran-2-yl)methoxy]-4-pyridyl}-6-methyl-1,5,6,7-tetrahydropyrrolo[3,2-c]pyridin-4-one (stereoisomer 1)

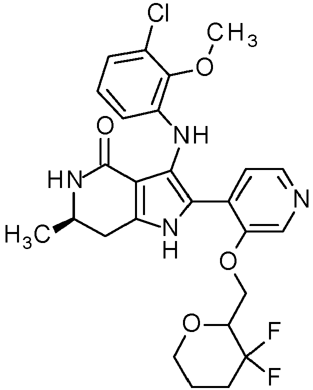

The title compound from example 74 (450 mg) was separated into enantiomers by preparative chiral HPLC to give 150 mg (98% purity, 33% yield) of the title compound (enantiomer 1, Rₜ = 6.7 - 9.4 min) and enantiomer 2 (15 mg, Rₜ = 13.8 - 19.2 min, see example 76).

### Preparative chiral HPLC method: MTBE

Instrument: PrepCon Labomatic HPLC; Column: Chiralpak IG 5µ, 250x30; eluent A: methyl tert-butyl ether + 0.1 vol % diethylamine; eluent B: acetonitrile + 0.1 vol % diethylamine; isocratic: 50% A + 50% B; flow: 50 ml/min; temperature: 25°C; UV: 254 nm

### Analytical chiral HPLC method: MTBE

Instrument: Waters Alliance 2695; Column: Chiralpak IG 3µ, 100x4.6; eluent A: methyl tert-butyl ether + 0.1 vol % diethylamine; eluent B: acetonitrile; isocratic: 50%A+50%B; flow: 1.4 ml/min; temperature: 25°C; UV: 254 nm
Analytical chiral HPLC: Rₜ = 3.03 min.
¹H-NMR (400 MHz, DMSO-d6) δ [ppm]: 1.233 (3.99), 1.249 (3.87), 1.808 (0.92), 1.829 (0.67), 2.074 (0.82), 2.084 (16.00), 2.518 (0.88), 2.522 (0.58), 2.539 (0.72), 2.569 (0.55), 2.595 (0.59), 2.609 (0.68), 2.635 (0.69), 2.896 (0.63), 2.909 (0.70), 2.936 (0.57), 2.948 (0.51), 3.585 (0.42), 3.594 (0.42), 3.776 (0.41), 3.789 (0.46), 3.879 (12.24), 4.000 (0.46), 4.028 (0.40), 4.136 (0.43), 4.143 (0.41), 4.197 (0.45), 4.203 (0.47), 4.240 (0.73), 4.259 (0.52), 4.267 (0.81), 4.286 (0.58), 4.528 (0.77), 4.535 (0.77), 4.555 (0.65), 4.561 (0.62), 5.758 (2.24), 6.122 (1.13), 6.133 (1.78), 6.146 (1.15), 6.670 (2.72), 6.672 (3.05), 6.683 (3.85), 7.119 (1.75), 7.293 (2.09), 7.306 (2.10), 7.539 (2.92), 8.043 (2.63), 8.055 (2.42), 8.445 (3.63), 10.957 (1.48).

### Example 76

### (6R)-3-(3-chloro-2-methoxy-anilino)-2-{3-[(3,3-difluorotetrahydropyran-2-yl)methoxy]-4-pyridyl}-6-methyl-1,5,6,7-tetrahydropyrrolo[3,2-c]pyridin-4-one (stereoisomer 2)

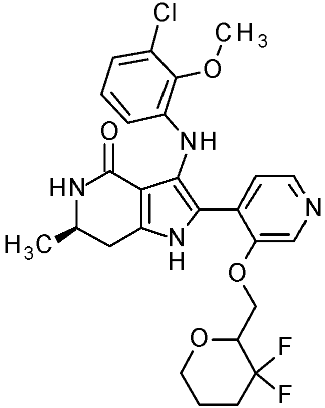

For the preparation of the title compound as mixture of stereoisomers see example 74. Separation of enantiomers by preparative chiral HPLC (method see example 75) to give 150 mg (98% purity, 33% yield) of the title compound (enantiomer 2, Rₜ = 13.8 - 19.2 min).

Analytical chiral HPLC (method see example 75): Rₜ = 7.41 min.

¹H-NMR (400 MHz, DMSO-d₆) δ [ppm]: 1.233 (3.43), 1.249 (3.34), 1.808 (0.71), 1.831 (0.58), 2.075 (0.75), 2.084 (16.00), 2.518 (0.82), 2.523 (0.54), 2.574 (0.47), 2.601 (0.50), 2.615 (0.59), 2.641 (0.60), 2.890 (0.53), 2.903 (0.61), 2.931 (0.48), 2.943 (0.44), 3.625 (0.41), 3.879 (10.65), 4.192 (0.44), 4.197 (0.49), 4.213 (0.64), 4.239 (0.70), 4.259 (0.44), 4.594 (0.63), 4.599 (0.65), 4.620 (0.57), 4.625 (0.52), 5.758 (2.16), 6.131 (0.95), 6.143 (1.53), 6.155 (0.99), 6.679 (3.44), 6.690 (2.39), 6.692 (2.31), 7.124 (1.53), 7.291 (1.56), 7.303 (1.57), 7.526 (2.51), 8.042 (1.71), 8.055 (1.58), 8.449 (2.55), 10.920 (1.28).

### Example 77

### (6R)-3-(3-chloro-2-methoxy-anilino)-2-{3-[(5,5-dimethyl-1,4-dioxan-2-yl)methoxy]-4-pyridyl}-6-methyl-1,5,6,7-tetrahydropyrrolo[3,2-c]pyridin-4-one

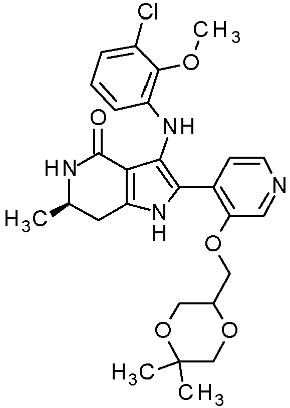

According to the method described for example 71 using (2*R*)-N-(3-chloro-2-methoxyphenyl)-4-({3-[(5,5-dimethyl-1,4-dioxan-2-yl)methoxy]-4-pyridyl}methylamino)-2-methyl-6-oxo-2,3-dihydro-1*H*-pyridine-5-carbothioamide (intermediate 6-77, 440 mg, 784 µmol) as starting material, 245 mg (93% purity, 55% yield) of the title compound were prepared.

LC-MS (method 2): Rₜ = 1.16 min; MS (ESlpos): m/z = 527 [M+H]⁺

¹H-NMR (400 MHz, DMSO-d₆) δ [ppm]: 0.975 (0.67), 1.035 (1.32), 1.052 (3.24), 1.066 (13.13), 1.145 (0.55), 1.198 (0.51), 1.226 (11.93), 1.232 (16.00), 1.246 (6.67), 2.518 (3.69), 2.522 (2.45), 2.542 (0.84), 2.549 (0.83), 2.569 (0.84), 2.576 (0.90), 2.583 (0.97), 2.591 (0.90), 2.610 (0.95), 2.615 (0.92), 2.863 (0.81), 2.876 (1.09), 2.884 (0.90), 2.903 (0.72), 2.912 (0.87), 2.924 (0.65), 3.371 (1.28), 3.398 (2.51), 3.426 (1.56), 3.569 (0.70), 3.578 (1.23), 3.587 (0.83), 3.599 (1.36), 3.608 (2.23), 3.622 (3.75), 3.629 (1.04), 3.642 (1.54), 3.649 (3.04), 3.659 (1.32), 3.668 (1.53), 3.684 (1.40), 3.697 (0.90), 3.714 (0.83), 3.748 (0.62), 3.763 (0.97), 3.777 (1.04), 3.789 (0.89), 3.802 (0.62), 3.863 (15.55), 3.867 (15.61), 3.883 (1.14), 3.891 (1.20), 3.899 (1.12), 3.908 (0.98), 3.916 (0.81), 3.924 (0.61), 4.154 (0.76), 4.165 (0.83), 4.170 (0.87), 4.181 (1.60), 4.191 (1.34), 4.196 (1.22), 4.207 (1.03), 4.255 (1.04), 4.264 (1.06), 4.281 (0.69), 4.290 (0.65), 4.299 (0.97), 4.309 (0.97), 4.326 (0.76), 4.335 (0.69), 5.759 (1.06), 6.139 (1.36), 6.148 (1.99), 6.154 (1.43), 6.159 (1.92), 6.163 (1.53), 6.171 (1.31), 6.647 (0.64), 6.657 (6.19), 6.661 (5.61), 6.667 (3.08), 6.672 (4.89), 7.093 (2.13), 7.103 (2.21), 7.269 (3.37), 7.280 (3.24), 7.449 (3.18), 7.472 (3.18), 8.033 (3.22), 8.037 (3.19), 8.045 (3.04), 8.049 (2.85), 8.404 (7.15), 11.038 (1.90), 11.051 (1.85).

### Example 78

### (6R)-3-(3-chloro-2-methoxy-anilino)-2-{3-[(5,5-dimethyl-1,4-dioxan-2-yl)methoxy]-4-pyridyl}-6-methyl-1,5,6,7-tetrahydropyrrolo[3,2-c]pyridin-4-one (stereoisomer 1)

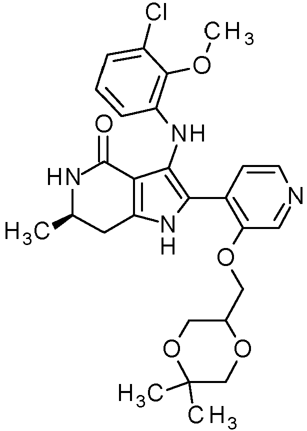

The title compound from example 77 (245 mg) was separated into enantiomers by preparative chiral HPLC to give 95.0 mg (98 % purity, 38 % yield) of the title compound (enantiomer 1, Rₜ = 18.0 - 21.5 min) and enantiomer 2 (125.0 mg, Rₜ = 22.0 - 26.8 min, see example 79).

### Preparative chiral HPLC method: NPB

Instrument: PrepCon Labomatic HPLC; Column: YMC Cellulose SB 10µ, 250x50; eluent A: hexane + 0.1 vol % diethylamine; eluent B: ethanol + 0.1 vol % diethylamine; isocratic: 80%A+20%B; flow: 100 mL/min; temperature: 25°C; UV: 254 nm

### Analytical chiral HPLC method: NPB

Instrument: Waters Alliance 2695; Column: YMC Cellulose SB 3µ, 100x4.6; eluent A: hexane + 0.1 vol % diethylamine; eluent B: ethanol; isocratic: 80%A+20%B; flow: 1.4 ml/min; temperature: 25°C; UV: 254 nm
Analytical chiral HPLC: Rₜ = 5.25 min.
¹H-NMR (400 MHz, DMSO-d₆) δ [ppm]: 0.747 (0.59), 0.766 (0.59), 1.065 (11.61), 1.156 (1.76), 1.232 (14.72), 1.246 (5.95), 2.084 (1.49), 2.522 (1.59), 2.550 (0.87), 2.575 (0.80), 2.590 (0.94), 2.616 (0.95), 2.871 (0.88), 2.884 (0.96), 2.912 (0.78), 2.924 (0.71), 3.308 (0.55), 3.370 (1.67), 3.378 (0.68), 3.399 (1.81), 3.569 (0.67), 3.577 (0.80), 3.598 (1.41), 3.606 (1.46), 3.622 (2.34), 3.643 (1.47), 3.649 (2.03), 3.668 (1.57), 3.698 (0.86), 3.762 (0.59), 3.774 (0.67), 3.788 (0.58), 3.863 (16.00), 3.882 (0.76), 3.891 (0.87), 3.899 (0.77), 3.907 (0.55), 3.915 (0.52), 4.164 (0.76), 4.180 (0.79), 4.190 (1.26), 4.206 (1.12), 4.254 (1.23), 4.263 (1.28), 4.280 (0.79), 4.289 (0.69), 6.139 (1.45), 6.148 (1.40), 6.153 (1.45), 6.163 (1.50), 6.646 (0.57), 6.657 (6.12), 6.666 (2.97), 6.672 (2.80), 7.089 (2.43), 7.269 (2.40), 7.282 (2.44), 7.445 (3.90), 8.035 (2.06), 8.048 (1.93), 8.404 (3.33), 11.051 (2.12).

### Example 79

### (6R)-3-(3-chloro-2-methoxy-anilino)-2-{3-[(5,5-dimethyl-1,4-dioxan-2-yl)methoxy]-4-pyridyl}-6-methyl-1,5,6,7-tetrahydropyrrolo[3,2-c]pyridin-4-one (stereoisomer 2)

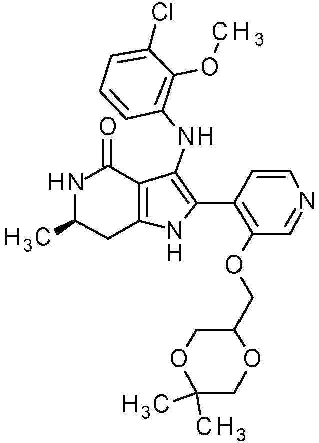

For the preparation of the title compound as mixture of stereoisomers see example 77. Separation of enantiomers by preparative chiral HPLC (method see example 78) to give 125 mg (88% purity, 45% yield) of the title compound (enantiomer 2, Rₜ = 22.0 - 26.8 min).

Analytical chiral HPLC (method see example 78): Rₜ = 6.19 min.

¹H-NMR (400 MHz, DMSO-d₆) δ [ppm]: 0.698 (0.42), 0.850 (0.50), 1.069 (13.03), 1.226 (13.85), 1.232 (10.38), 1.249 (6.84), 2.084 (0.51), 2.542 (1.43), 2.569 (1.11), 2.582 (1.19), 2.609 (1.13), 2.864 (1.01), 2.876 (1.15), 2.903 (0.89), 2.916 (0.85), 3.372 (0.64), 3.399 (1.85), 3.427 (1.98), 3.579 (0.89), 3.587 (0.97), 3.608 (1.79), 3.620 (3.09), 3.649 (2.20), 3.659 (1.47), 3.685 (1.72), 3.713 (0.90), 3.748 (0.51), 3.764 (0.81), 3.777 (0.91), 3.790 (0.77), 3.804 (0.50), 3.867 (16.00), 3.889 (1.02), 3.898 (1.09), 3.906 (0.98), 4.154 (0.93), 4.169 (0.95), 4.180 (1.36), 4.196 (1.16), 4.299 (1.28), 4.308 (1.29), 4.325 (0.97), 4.334 (0.89), 6.147 (1.59), 6.159 (2.13), 6.171 (1.54), 6.660 (5.84), 6.672 (4.03), 7.100 (2.93), 7.267 (2.66), 7.280 (2.67), 7.446 (0.59), 7.468 (4.12), 8.032 (2.46), 8.045 (2.25), 8.402 (4.08), 11.037 (2.45).

### Example 80

### (6R)-3-(3-chloro-2-methoxy-anilino)-2-[3-(2-methoxypropoxy)-4-pyridyl]-6-methyl-1,5,6,7-tetrahydropyrrolo[3,2-c]pyridin-4-one

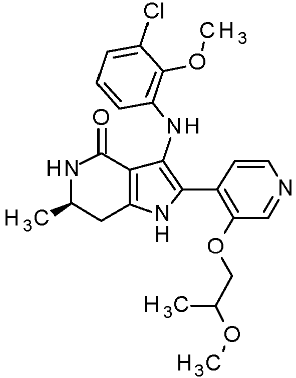

According to the method described for example 71 using (2R)-N-(3-chloro-2-methoxyphenyl)-4-{[3-(2-methoxypropoxy)-4-pyridyl]methylamino}-2-methyl-6-oxo-2,3-dihydro-1*H*-pyridine-5-carbothioamide (intermediate 6-80, 400 mg, 792 µmol) as starting material, 172 mg (90% purity, 42% yield) of the title compound were prepared.

LC-MS (method 2): Rₜ = 1.09 min; MS (ESlpos): m/z = 471 [M+H]⁺

¹H-NMR (400 MHz, DMSO-d₆) δ [ppm]: 1.035 (0.95), 1.052 (1.57), 1.070 (0.99), 1.183 (2.42), 1.193 (2.38), 1.199 (2.63), 1.209 (2.24), 1.224 (1.95), 1.229 (1.83), 1.240 (2.01), 1.246 (1.71), 2.518 (0.94), 2.523 (0.60), 2.905 (0.40), 3.329 (16.00), 3.350 (7.13), 3.356 (8.10), 3.801 (0.43), 3.812 (0.44), 3.816 (0.43), 3.828 (0.45), 4.091 (0.43), 4.251 (0.58), 4.259 (0.41), 5.758 (0.58), 6.136 (0.74), 6.146 (0.67), 6.151 (0.82), 6.160 (0.76), 6.670 (3.04), 6.680 (1.34), 6.686 (1.48), 7.104 (1.34), 7.271 (0.91), 7.275 (1.03), 7.283 (0.93), 7.287 (1.04), 7.488 (1.28), 7.504 (1.10), 8.024 (2.40), 8.037 (2.16), 8.406 (1.85), 8.410 (1.71), 11.053 (0.61), 11.086 (0.53).

### Example 81

### (6R)-3-(3-chloro-2-methoxy-anilino)-2-[3-(2-methoxypropoxy)-4-pyridyl]-6-methyl-1,5,6,7-tetrahydropyrrolo[3,2-c]pyridin-4-one (stereoisomer 1)

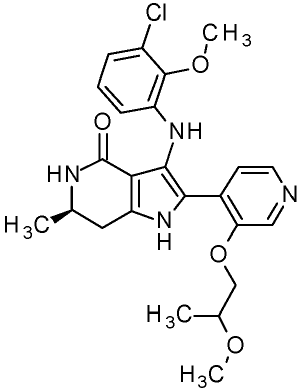

The title compound from example 80 (172 mg) was separated into enantiomers by preparative chiral HPLC to give 55.0 mg (99% purity, 32% yield) of the title compound (enantiomer 1, Rₜ = 6.1 - 6.9 min) and enantiomer 2 (64.0 mg, Rₜ = 7.1 - 8.5 min, see example 82).

### Preparative chiral HPLC method: MTBE

Instrument: PrepCon Labomatic HPLC; Column: YMC Cellulose SC 5µ, 250x30; eluent A: methyl tert-butyl ether + 0.1 vol % diethylamine; eluent B: acetonitrile + 0.1 vol % diethylamine; isocratic: 10% A + 90% B; flow: 50 ml/min; temperature: 25°C; UV: 254 nm

### Analytical chiral HPLC method: MTBE

Instrument: Waters Alliance 2695; Column: YMC Cellulose SC 3µ, 100x4.6; eluent A: methyl tert-butyl ether + 0.1 vol % diethylamine; eluent B: acetonitrile; isocratic: 10% A + 90% B; flow: 1.4 ml/min; temperature: 25°C; UV: 254 nm
Analytical chiral HPLC: Rₜ = 2.40 min.
Optical rotation:[α]_{D} = 44.27° +/- 0.38° (c = 8.1 mg/ml in CHLOROFORM)
¹H-NMR (400 MHz, DMSO-d₆) δ [ppm]: 1.138 (0.43), 1.161 (0.90), 1.194 (6.54), 1.210 (6.66), 1.231 (5.61), 1.246 (5.29), 2.573 (0.74), 2.600 (0.80), 2.613 (0.95), 2.639 (0.97), 2.868 (0.91), 2.881 (1.01), 2.908 (0.79), 2.921 (0.74), 3.334 (11.32), 3.350 (16.00), 3.757 (0.40), 3.772 (0.67), 3.785 (0.88), 3.801 (1.13), 3.811 (1.03), 3.816 (1.15), 3.826 (1.06), 3.832 (0.77), 3.841 (0.66), 4.115 (0.83), 4.130 (0.83), 4.140 (1.31), 4.155 (1.17), 4.218 (1.28), 4.227 (1.32), 4.243 (0.85), 4.252 (0.78), 6.139 (1.28), 6.148 (1.34), 6.153 (1.40), 6.163 (1.34), 6.662 (0.58), 6.672 (5.21), 6.682 (2.79), 6.687 (2.60), 7.108 (2.63), 7.272 (2.26), 7.284 (2.28), 7.505 (3.70), 8.025 (2.13), 8.037 (2.01), 8.411 (3.54), 11.087 (2.17).

### Example 82

### (6R)-3-(3-chloro-2-methoxy-anilino)-2-[3-(2-methoxypropoxy)-4-pyridyl]-6-methyl-1,5,6,7-tetrahydropyrrolo[3,2-c]pyridin-4-one (stereoisomer 2)

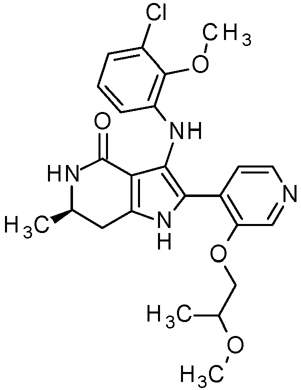

For the preparation of the title compound as mixture of stereoisomers see example 80. Separation of enantiomers by preparative chiral HPLC (method see example 81) to give 64.0 mg (99% purity, 37% yield) of the title compound (enantiomer 2, Rₜ = 7.1 - 8.5 min).

Analytical chiral HPLC (method see example 81): Rₜ = 2.77 min.

Optical rotation:[α]_{D} = 33.52 ° +/- 0.45° (c = 10.3 mg/ml in CHLOROFORM)

¹H-NMR (400 MHz, DMSO-d₆) δ [ppm]: 1.183 (5.84), 1.199 (5.95), 1.209 (0.75), 1.224 (4.41), 1.240 (4.37), 2.562 (0.64), 2.588 (0.66), 2.602 (0.77), 2.629 (0.79), 2.892 (0.70), 2.905 (0.80), 2.932 (0.64), 2.945 (0.59), 3.334 (6.76), 3.350 (1.57), 3.357 (16.00), 3.774 (0.47), 3.787 (0.54), 3.801 (0.55), 3.813 (0.68), 3.821 (0.62), 3.828 (0.80), 3.837 (0.78), 3.844 (0.55), 3.853 (0.56), 4.066 (0.82), 4.082 (0.78), 4.091 (1.04), 4.108 (0.92), 4.252 (0.99), 4.260 (1.00), 4.277 (0.79), 4.286 (0.74), 5.758 (1.03), 6.137 (1.24), 6.147 (1.14), 6.152 (1.25), 6.161 (1.27), 6.659 (0.48), 6.670 (5.31), 6.679 (2.46), 6.685 (2.23), 7.108 (2.02), 7.275 (1.98), 7.288 (1.99), 7.490 (3.17), 8.025 (1.77), 8.037 (1.66), 8.407 (2.68), 11.054 (1.62).

### Example 83

### (6R)-3-(3-chloro-2-methoxy-anilino)-6-methyl-2-{3-[(1-methyl-2-piperidyl)methoxy]-4-pyridyl}-1,5,6,7-tetrahydropyrrolo[3,2-c]pyridin-4-one

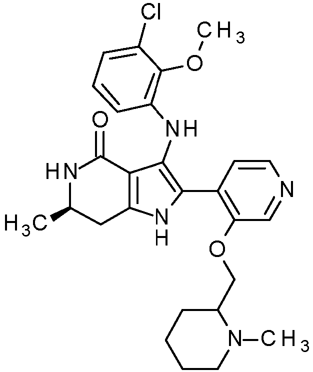

According to the method described for example 52 using (2*R*)-N-(3-chloro-2-methoxyphenyl)-2-methyl-4-({3-[(1-methyl-2-piperidyl)methoxy]-4-pyridyl}methylamino)-6-oxo-2,3-dihydro-1H-pyridine-5-carbothioamide (intermediate 6-83, 400 mg, 735 µmol) as starting material, 65.0 mg (95% purity, 16% yield) of the title compound were prepared after purification by preparative HPLC (method 10, gradient: 0.00-0.50 min 30% B, 0.50-6.00 min 30-70% B).

LC-MS (method 2): Rₜ = 1.25 min; MS (ESlpos): m/z = 510 [M+H]⁺

¹H-NMR (400 MHz, DMSO-d₆) δ [ppm]: 1.232 (8.38), 1.247 (8.29), 1.333 (0.53), 1.354 (0.58), 1.506 (0.45), 1.541 (0.45), 1.572 (0.43), 1.638 (1.53), 1.660 (1.69), 1.685 (1.37), 1.751 (0.79), 2.208 (8.43), 2.232 (0.88), 2.257 (8.96), 2.272 (1.26), 2.291 (1.36), 2.318 (0.77), 2.322 (0.93), 2.326 (1.03), 2.332 (0.70), 2.518 (2.23), 2.522 (1.40), 2.562 (0.55), 2.588 (0.63), 2.594 (0.69), 2.601 (0.74), 2.621 (0.65), 2.627 (0.86), 2.634 (0.80), 2.660 (0.94), 2.669 (0.80), 2.673 (0.58), 2.798 (0.65), 2.811 (0.72), 2.838 (0.55), 2.855 (0.75), 2.869 (0.71), 2.897 (0.56), 2.909 (0.54), 3.016 (0.52), 3.052 (0.79), 3.083 (0.47), 3.781 (0.45), 3.816 (0.56), 3.841 (0.43), 3.912 (15.15), 3.916 (16.00), 3.929 (1.09), 4.349 (0.60), 4.377 (3.14), 4.383 (2.59), 4.414 (0.82), 4.423 (0.92), 4.440 (0.44), 4.449 (0.42), 6.154 (2.00), 6.167 (2.83), 6.178 (1.98), 6.702 (0.81), 6.712 (5.84), 6.720 (3.73), 6.722 (6.06), 6.726 (2.91), 6.733 (4.74), 7.124 (1.83), 7.171 (1.87), 7.281 (2.37), 7.294 (2.58), 7.298 (2.59), 7.310 (2.32), 7.556 (3.15), 7.671 (2.81), 7.955 (4.80), 7.968 (4.47), 8.380 (6.87), 12.222 (1.42), 12.280 (1.37).

### Example 84

### (6R)-3-(3-chloro-2-methoxy-anilino)-6-methyl-2-{3-[(1-methyl-2-piperidyl)methoxy]-4-pyridyl}-1,5,6,7-tetrahydropyrrolo[3,2-c]pyridin-4-one (stereoisomer 1)

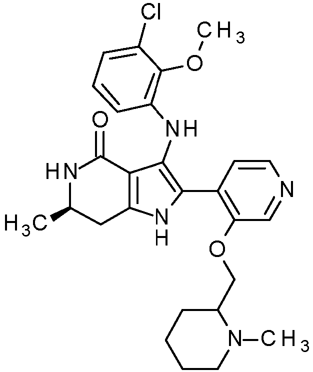

The title compound from example 83 (65.0 mg) was separated into enantiomers by preparative chiral HPLC to give 38.0 mg (95% purity, 56% yield) of the title compound (enantiomer 1, Rₜ = 8.8 - 11.4 min) and enantiomer 2 (40.0 mg, Rₜ = 15.8 - 29.4 min, see example 85).

### Preparative chiral HPLC method: MTBE

Instrument: PrepCon Labomatic HPLC; Column: YMC Amylose SA 10µ, 250x50; eluent A: methyl tert-butyl ether + 0.1 vol % diethylamine; eluent B: acetonitrile + 0.1 vol % diethylamine; isocratic: 30%A+70%B; flow: 150 mL/min; temperature: 25°C; UV: 280 nm

### Analytical chiral HPLC method: MTBE

Instrument: Waters Alliance 2695; Column: YMC Amylose SA 3µ, 100x4.6; eluent A: methyl tert-butyl ether + 0.1 vol % diethylamine; eluent B: acetonitrile; isocratic: 30% A + 70% B; flow: 1.4 ml/min; temperature: 25°C; UV: 280 nm
Analytical chiral HPLC: Rₜ = 3.48 min.
Optical rotation:[α]_{D} = 186.91 ° +/- 1.37° (c = 2.2 mg/ml in chloroform)
¹H-NMR (400 MHz, DMSO-d₆) δ [ppm]: 1.026 (0.50), 1.034 (0.84), 1.041 (0.50), 1.052 (1.65), 1.069 (0.89), 1.106 (0.45), 1.136 (1.30), 1.160 (0.43), 1.230 (5.56), 1.246 (5.12), 1.351 (0.45), 1.539 (0.43), 1.571 (0.46), 1.667 (1.44), 1.685 (1.53), 1.750 (0.62), 1.788 (0.46), 2.114 (0.52), 2.207 (9.53), 2.260 (0.80), 2.285 (0.87), 2.318 (0.62), 2.322 (0.98), 2.326 (1.26), 2.331 (0.91), 2.518 (5.31), 2.522 (3.34), 2.539 (1.08), 2.593 (0.69), 2.620 (0.70), 2.634 (0.92), 2.659 (1.34), 2.664 (1.15), 2.669 (1.32), 2.673 (0.96), 2.798 (0.80), 2.811 (0.90), 2.838 (0.63), 2.851 (0.58), 3.017 (0.61), 3.046 (0.55), 3.307 (0.56), 3.421 (0.92), 3.434 (0.78), 3.438 (0.71), 3.451 (0.67), 3.456 (0.42), 3.815 (0.57), 3.828 (0.61), 3.841 (0.54), 3.914 (16.00), 4.368 (0.98), 4.377 (3.07), 4.381 (3.24), 6.152 (1.40), 6.164 (1.88), 6.176 (1.43), 6.719 (3.31), 6.721 (3.68), 6.732 (5.31), 7.168 (2.33), 7.280 (2.52), 7.293 (2.61), 7.668 (3.39), 7.954 (2.83), 7.966 (2.70), 8.377 (4.16), 12.226 (1.67).

### Example 85

### (6R)-3-(3-chloro-2-methoxy-anilino)-6-methyl-2-{3-[(1-methyl-2-piperidyl)methoxy]-4-pyridyl}-1,5,6,7-tetrahydropyrrolo[3,2-c]pyridin-4-one (stereoisomer 2)

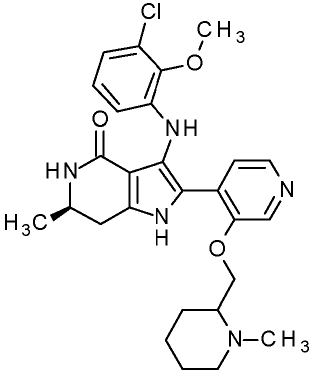

For the preparation of the title compound as mixture of stereoisomers see example 83. Separation of enantiomers by preparative chiral HPLC (method see example 84) to give 40.0 mg (95 % purity, 58 % yield) of the title compound (enantiomer 2, Rₜ = 15.8 - 29.4 min).

Analytical chiral HPLC (method see example 84): Rₜ = 6.63 min.

Optical rotation:[α]_{D} = - 96.75 ° +/- 0.35° (c = 7.3 mg/ml in chloroform)

¹H-NMR (400 MHz, DMSO-d₆) δ [ppm]: 1.035 (0.48), 1.052 (0.87), 1.070 (0.59), 1.107 (0.45), 1.137 (1.37), 1.164 (0.77), 1.231 (6.00), 1.247 (5.56), 1.332 (0.52), 1.353 (0.50), 1.423 (0.47), 1.472 (0.55), 1.504 (0.55), 1.638 (2.17), 1.734 (0.67), 1.764 (0.59), 2.115 (0.51), 2.209 (0.41), 2.234 (0.66), 2.256 (11.02), 2.291 (1.06), 2.322 (1.05), 2.326 (1.12), 2.522 (2.42), 2.561 (0.88), 2.587 (0.86), 2.601 (0.99), 2.627 (0.98), 2.665 (0.58), 2.669 (0.77), 2.673 (0.57), 2.856 (0.89), 2.869 (0.99), 2.896 (0.78), 2.908 (0.72), 3.054 (0.74), 3.083 (0.69), 3.781 (0.61), 3.794 (0.68), 3.806 (0.58), 3.911 (16.00), 3.928 (0.66), 4.348 (0.91), 4.361 (0.45), 4.374 (1.64), 4.415 (1.15), 4.422 (1.25), 4.441 (0.64), 4.449 (0.61), 6.152 (1.51), 6.162 (1.46), 6.166 (1.53), 6.176 (1.50), 6.702 (0.72), 6.712 (6.18), 6.722 (3.17), 6.726 (2.90), 7.124 (2.57), 7.298 (2.61), 7.310 (2.64), 7.555 (4.03), 7.954 (2.69), 7.966 (2.47), 8.380 (4.08), 12.282 (1.91).

### Example 86

### (6R)-3-(3-chloro-2-methoxy-anilino)-6-methyl-2-[3-(2-tetrahydropyran-2-ylethoxy)-4-pyridyl]-1,5,6,7-tetrahydropyrrolo[3,2-c]pyridin-4-one

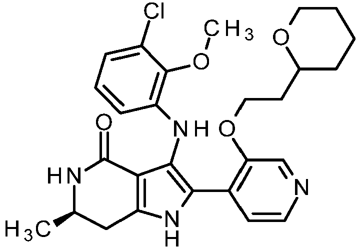

According to the method described for example 71 using (2R)-N-(3-chloro-2-methoxyphenyl)-2-methyl-6-oxo-4-{[3-(2-tetrahydropyran-2-ylethoxy)-4-pyridyl]methylamino}-2,3-dihydro-1H-pyridine-5-carbothioamide (intermediate 6-86, 390 mg, 715 µmol) as starting material, 179 mg (95% purity, 47% yield) of the title compound were prepared after trituration from EtOH.

LC-MS (method 2): Rₜ = 1.20 min; MS (ESlpos): m/z = 511 [M+H]⁺

¹H-NMR (400 MHz, DMSO-d₆) δ [ppm]: 1.222 (4.76), 1.233 (5.81), 1.238 (5.81), 1.249 (4.95), 1.289 (0.54), 1.401 (0.48), 1.425 (1.30), 1.446 (2.41), 1.546 (0.62), 1.576 (1.09), 1.605 (0.51), 1.755 (0.90), 1.866 (0.46), 1.884 (1.19), 1.900 (2.22), 1.911 (1.93), 1.926 (1.22), 1.941 (0.41), 2.331 (0.42), 2.518 (1.73), 2.522 (1.19), 2.553 (0.76), 2.557 (0.78), 2.578 (0.77), 2.585 (0.80), 2.593 (1.02), 2.619 (0.91), 2.625 (0.88), 2.673 (0.44), 2.869 (0.71), 2.881 (0.83), 2.894 (0.75), 2.908 (1.28), 2.921 (0.62), 2.934 (0.67), 2.947 (0.61), 3.278 (0.66), 3.297 (0.91), 3.305 (1.29), 3.404 (0.46), 3.417 (0.75), 3.431 (0.78), 3.438 (0.74), 3.445 (0.72), 3.768 (0.77), 3.782 (0.85), 3.859 (16.00), 3.867 (15.94), 3.882 (1.15), 4.217 (1.38), 4.232 (2.85), 4.244 (2.01), 4.257 (1.53), 4.271 (0.62), 6.106 (1.90), 6.116 (1.68), 6.119 (3.34), 6.130 (2.01), 6.141 (0.46), 6.640 (3.77), 6.645 (3.21), 6.650 (6.00), 6.659 (5.55), 7.079 (1.93), 7.094 (2.17), 7.287 (2.64), 7.293 (2.62), 7.300 (2.71), 7.306 (2.55), 7.450 (3.08), 7.491 (3.15), 8.011 (3.11), 8.023 (5.80), 8.035 (2.79), 8.343 (4.42), 8.350 (4.28), 11.200 (1.67), 11.240 (1.72).

### Example 87

### (6R)-3-(3-chloro-2-methoxy-anilino)-6-methyl-2-[3-(2-tetrahydropyran-2-ylethoxy)-4-pyridyl]-1,5,6,7-tetrahydropyrrolo[3,2-c]pyridin-4-one (stereoisomer 1)

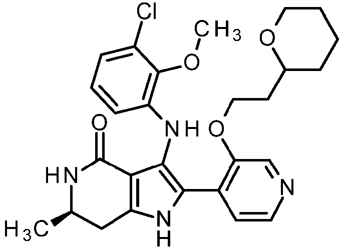

The title compound from example 86 (179.0 mg) was separated into enantiomers by preparative chiral HPLC to give 75 mg (98% purity, 41% yield) of the title compound (enantiomer 1, Rₜ = 8.7 - 10.0 min) and enantiomer 2 (110 mg, Rₜ = 10.8 - 14.0 min, see example 88).

### Preparative chiral HPLC method:

Instrument: PrepCon Labomatic HPLC; Column: Chiralpak IG 5µ, 250x30; eluent A: acetonitrile; eluent B: ethanol; isocratic: 90% A + 10% B; flow: 50 ml/min; temperature: 25°C; UV: 254 nm

### Analytical chiral HPLC method:

Instrument: Waters Alliance 2695; Column: Chiralpak IG 3µ, 100x4.6; eluent A: acetonitrile; eluent B: ethanol; isocratic: 90%A+10%B; flow: 1.4 ml/min; temperature: 25°C; UV: 254 nm
Analytical chiral HPLC: Rₜ = 3.85 min.
¹H-NMR (400 MHz, DMSO-d₆) δ [ppm]: 1.026 (0.61), 1.035 (4.02), 1.042 (0.70), 1.052 (7.99), 1.070 (4.01), 1.137 (1.11), 1.222 (5.15), 1.238 (5.47), 1.258 (0.58), 1.269 (0.54), 1.400 (0.46), 1.425 (1.21), 1.443 (1.78), 1.545 (0.75), 1.577 (0.61), 1.751 (1.65), 1.864 (0.43), 1.881 (1.11), 1.898 (1.52), 1.908 (1.03), 1.924 (0.45), 2.084 (4.85), 2.115 (0.48), 2.522 (1.32), 2.551 (0.78), 2.577 (0.78), 2.591 (0.89), 2.617 (0.92), 2.893 (0.82), 2.905 (0.92), 2.933 (0.75), 2.946 (0.68), 3.159 (1.11), 3.171 (1.09), 3.278 (0.44), 3.304 (0.86), 3.404 (1.13), 3.417 (1.30), 3.422 (2.44), 3.435 (2.38), 3.439 (2.21), 3.452 (2.04), 3.457 (0.72), 3.469 (0.66), 3.763 (0.55), 3.776 (0.58), 3.789 (0.52), 3.858 (16.00), 3.880 (0.70), 3.885 (0.68), 4.215 (1.37), 4.231 (2.84), 4.246 (1.33), 4.346 (1.40), 4.358 (2.70), 4.371 (1.29), 6.107 (1.40), 6.119 (2.37), 6.131 (1.42), 6.637 (4.36), 6.649 (4.20), 7.074 (2.24), 7.289 (2.47), 7.301 (2.50), 7.440 (3.76), 8.019 (2.61), 8.032 (2.48), 8.347 (3.88), 11.196 (1.97).

### Example 88

### (6R)-3-(3-chloro-2-methoxy-anilino)-6-methyl-2-[3-(2-tetrahydropyran-2-ylethoxy)-4-pyridyl]-1,5,6,7-tetrahydropyrrolo[3,2-c]pyridin-4-one (stereoisomer 2)

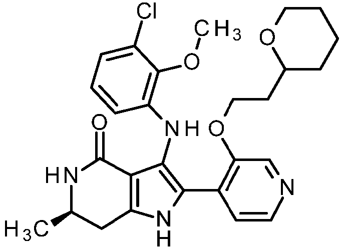

For the preparation of the title compound as mixture of stereoisomers see example 86. Separation of enantiomers by preparative chiral HPLC (method see example 87) to give 110 mg (90 % purity, 55 % yield) of the title compound (enantiomer 2, Rₜ = 15.8 - 29.4 min).

Analytical chiral HPLC (method see example 87): Rₜ = 4.81 min.

¹H-NMR (400 MHz, DMSO-d₆) δ [ppm]: 1.035 (3.07), 1.052 (5.89), 1.070 (3.14), 1.233 (5.40), 1.249 (5.34), 1.286 (0.49), 1.293 (0.49), 1.424 (0.44), 1.465 (1.94), 1.576 (0.78), 1.607 (0.65), 1.751 (0.73), 1.761 (0.65), 1.769 (0.63), 1.900 (1.17), 1.912 (1.42), 1.926 (1.09), 1.942 (0.44), 2.522 (1.22), 2.558 (0.76), 2.586 (0.80), 2.598 (0.93), 2.625 (0.94), 2.870 (0.85), 2.882 (0.96), 2.910 (0.77), 2.922 (0.70), 3.162 (1.91), 3.167 (1.91), 3.277 (0.48), 3.432 (1.17), 3.442 (1.22), 3.770 (0.59), 3.783 (0.71), 3.797 (0.57), 3.868 (16.00), 3.882 (0.87), 4.220 (0.44), 4.229 (0.74), 4.244 (1.74), 4.258 (1.76), 4.272 (0.74), 4.281 (0.42), 4.358 (0.44), 6.105 (1.49), 6.115 (1.54), 6.119 (1.44), 6.129 (1.51), 6.646 (3.01), 6.650 (3.35), 6.660 (5.84), 6.670 (0.46), 7.096 (2.60), 7.289 (2.82), 7.302 (2.84), 7.497 (3.70), 8.013 (3.38), 8.025 (3.19), 8.344 (4.80), 11.242 (2.11).

### Example 89

### 3-(3-chloro-2-methoxyanilino)-7-(2-fluoroethyl)-2-(3-{[(3S)-4-methylmorpholin-3-yl]methoxy}pyridin-4-yl)-1,5,6,7-tetrahydro-4H-pyrrolo[3,2-c]pyridin-4-one (stereoisomer 1)

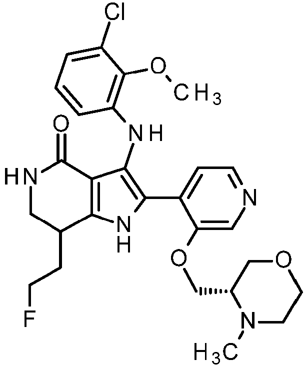

3-(3-Chloro-2-methoxyanilino)-7-(2-fluoroethyl)-2-(3-{[(3*S*)-morpholin-3-yl]methoxy}pyridin-4-yl)-1,5,6,7-tetrahydro-4*H*-pyrrolo[3,2-c]pyridin-4-one (Intermediate 89-2, 35 mg, 66 µmol) was supended in methanol (0.9 ml), formaldehyde (9.9 µl, 37% purity, 130 µmol) and acetic acid (3.8 µl, 66 µmol) was added and the mixture stirred for 15 min at room temperature. To the mixture was added sodium triacetoxyborohydride (21.0 mg, 99 µmol) and it was stirred for 12 h at room temperature. The reaction mixture was loaded on a SCX coloumn (2 g) and washed with methanol followed by ammonia in methanol (7 M). The basic filtrate was concentrated suspended in methanol (0.9 ml) and treated again with formaldehyde (9.9 µl, 37% purity, 130 µmol) and acetic acid (3.8 µl, 66 µmol) followed by sodium triacetoxyborohydride (21.0 mg, 99 µmol). Sodium hydroxid solution (2 M) was added and the aqueous phase extracted with DCM after addition of solid sodium chloride. The combined organics were dried, concentrated under reduced pressure and purified by preparative HPLC (method 10, gradient: 0.00-0.50 min 10% B, 0.50-10.00 min 10-50% B, 10.00 -13.00 min 50% B) to give 1,61 mg (95% purity, 4.4% yield) of the title compound and 2.62 mg of a second stereoisomer.

LC-MS (method 2): Rₜ = 1.07 min; MS (ESlpos): m/z = 544.3 [M+H]⁺

¹H-NMR (400 MHz, DMSO-d₆): δ [ppm]:1.83 - 2.04 (m, 2H), 2.24 - 2.31 (m, 3H), 2.35 - 2.41 (m, 1H), 2.80 - 2.93 (m, 1H), 2.98 - 3.08 (m, 1H), 3.52 - 3.67 (m, 4H), 3.72 - 3.96 (m, 6H), 4.25 - 4.43 (m, 2H), 4.44 - 4.59 (m, 1H), 4.59 - 4.73 (m, 1H), 6.09 - 6.16 (m, 1H), 6.71 (s, 2H), 7.14 - 7.23 (m, 1H), 7.27 - 7.34 (m, 1H), 7.67 (s, 1H), 7.95 - 8.03 (m, 1H), 8.33 - 8.40 (m, 1H), 11.38 - 11.55 (m, 1H).

### Example 90

### 3-(3-chloro-2-methoxyanilino)-7-(2-fluoroethyl)-2-(3-{[(3S)-4-methylmorpholin-3-yl]methoxy}pyridin-4-yl)-1,5,6,7-tetrahydro-4H-pyrrolo[3,2-c]pyridin-4-one (stereoisomer 2)

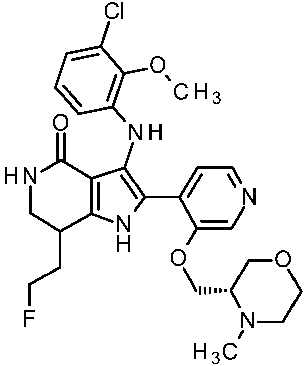

2,62 mg (95% purity, 6.7% yield) of the title compound were obtained as the second stereoisomer prepared and purified in example 89.

LC-MS (method 2): Rₜ = 1.05 min; MS (ESlpos): m/z = 544.3 [M+H]⁺

¹H-NMR (400 MHz, DMSO-d₆): δ [ppm]= 1.85 - 2.19 (m, 2H), 2.21 - 2.35 (m, 4H), 2.76 - 2.90 (m, 1H), 3.01 - 3.15 (m, 1H), 3.41 - 3.59 (m, 4H), 3.69 - 3.92 (m, 6H), 4.15 - 4.30 (m, 1H), 4.33 - 4.46 (m, 1H), 4.47 - 4.59 (m, 1H), 4.60 - 4.74 (m, 1H), 6.09 - 6.18 (m, 1H), 6.69 (d, 2H), 7.12 - 7.23 (m, 1H), 7.15 - 7.19 (m, 1H), 7.34 (d, 1H), 7.51 (s, 1H), 7.99 - 8.07 (m, 1H), 8.43 (s, 1H), 11.28 - 11.43 (m, 1H).

### Example 91

### (6R)-3-(3-chloro-2-ethylanilino)-6-methyl-2-(3-{[(2S)-4-methylmorpholin-2-yl]methoxy}pyridin-4-yl)-1,5,6,7-tetrahydro-4H-pyrrolo[3,2-c]pyridin-4-one

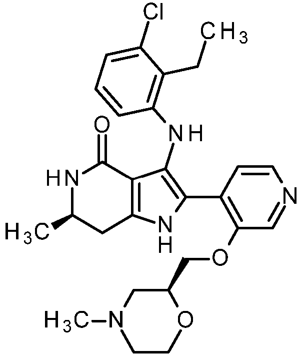

According to the method described for example 64 using (6R)-3-(3-chloro-2-ethylanilino)-6-methyl-2-(3-{[(2S)-morpholin-2-yl]methoxy}pyridin-4-yl)-1,5,6,7-tetrahydro-4H-pyrrolo[3,2-c]pyridin-4-one (intermediate 91-2, 33.0 mg, 66.5 µmol) as starting material, 18.9 mg (85% purity, 47% yield) of the title compound were prepared after purification by preparative HPLC (method 10, gradient: 0.00-0.50 min 30% B, 0.50-7.00 min 30-70% B).

LC-MS (method 2): Rₜ = 1.15 min; MS (ESlpos): m/z = 510 [M+H]⁺

¹H-NMR (400 MHz, DMSO-d₆): δ [ppm]= 1.21 - 1.30 (m, 6H), 1.88 - 2.00 (m, 1H), 2.01 - 2.13 (m, 1H), 2.20 - 2.26 (s, 3H), 2.56 - 2.66 (dd, 1H), 2.66 - 2.71 (m, 1H), 2.71 - 2.80 (m, 1H), 2.85 (q, 2H), 2.89 - 2.96 (dd, 1H), 3.60 - 3.70 (td, 1H), 3.75 - 3.86 (m, 1H), 3.94 (br s, 2H), 4.11 - 4.19 (m, 1H), 4.22 - 4.31 (dd, 1H), 6.20 - 6.26 (dd, 1H), 6.68 - 6.77 (m, 2H), 7.14 (s, 1H), 7.17 - 7.22 (d, 1H), 7.43 (s, 1H), 7.97 (d, 1H), 8.37 (s, 1H), 11.01 (s, 1H).

### Example 92

### (6R)-3-(3-chloro-2-methoxyanilino)-6-methyl-2-(3-{2-[(2S)-oxolan-2-yl]ethoxylpyridin-4-yl)-1,5,6,7-tetrahydro-4H-pyrrolo[3,2-c]pyridin-4-one

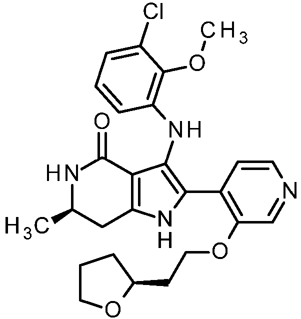

According to the method described for example 1 using (6*R*)-*N*-(3-chloro-2-methoxyphenyl)-6-methyl-2-oxo-4-{[(3-{2-[(2*S*)-oxolan-2-yl]ethoxy}pyridin-4-yl)methyl]amino}-1,2,5,6-tetrahydropyridine-3-carbothioamide (intermediate 6-92, 420 mg, 791 µmol) as starting material, 193.0 mg (93% purity, 46% yield) of the title compound were prepared after purification by preparative HPLC (method 10, gradient: 0.00-0.50 min 30% B, 0.50-6.00 min 30-70% B).

LC-MS (method 2): Rₜ = 1.14 min; MS (ESlpos): m/z = 497 [M+H]⁺

¹H-NMR (400 MHz, DMSO-d₆) δ [ppm]: 1.230 (4.54), 1.246 (4.51), 1.476 (0.50), 1.497 (0.62), 1.505 (0.56), 1.527 (0.58), 1.801 (0.59), 1.817 (0.74), 1.821 (0.73), 1.826 (0.50), 1.837 (0.85), 1.841 (0.69), 1.848 (0.52), 1.856 (0.62), 1.860 (0.51), 1.955 (0.44), 1.959 (0.51), 1.970 (0.80), 1.985 (1.09), 2.001 (1.12), 2.014 (0.88), 2.024 (0.88), 2.038 (0.40), 2.518 (0.98), 2.522 (0.63), 2.561 (0.66), 2.588 (0.70), 2.601 (0.80), 2.628 (0.82), 2.872 (0.73), 2.885 (0.83), 2.912 (0.66), 2.925 (0.61), 3.623 (0.55), 3.638 (0.71), 3.642 (1.17), 3.658 (1.21), 3.662 (0.77), 3.678 (0.60), 3.762 (0.51), 3.776 (0.64), 3.790 (0.50), 3.817 (0.71), 3.835 (1.16), 3.852 (1.16), 3.855 (1.08), 3.867 (16.00), 3.886 (0.40), 3.896 (0.56), 3.906 (0.59), 3.911 (0.56), 3.922 (0.56), 4.233 (0.63), 4.248 (1.41), 4.261 (1.49), 4.276 (0.66), 6.105 (1.40), 6.117 (2.34), 6.129 (1.42), 6.652 (4.10), 6.663 (3.93), 7.090 (2.20), 7.302 (2.55), 7.315 (2.53), 7.499 (3.37), 8.011 (3.36), 8.024 (3.17), 8.357 (4.24), 11.218 (1.79).

### Example 93

### (6R)-3-(3-chloro-2-methoxyanilino)-6-methyl-2-(3-{[(3S)-4-methylmorpholin-3-yl]methoxy}pyridin-4-yl)-1,5,6,7-tetrahydro-4H-pyrrolo[3,2-c]pyridin-4-one

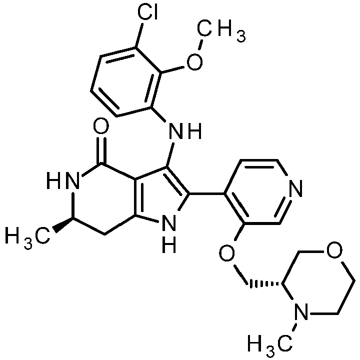

According to the method described for example 52 using (6R)-N-(3-chloro-2-methoxyphenyl)-6-methyl-4-{[(3-{[(3S)-4-methylmorpholin-3-yl]methoxy}pyridin-4-yl)methyl]amino}-2-oxo-1,2,5,6-tetrahydropyridine-3-carbothioamide (intermediate 6-93, 420 mg, 769 µmol) as starting material, 83.9 mg (92% purity, 20% yield) of the title compound were prepared after purification by preparative HPLC (method 10, gradient: 0.00-0.50 min 15% B, 0.50-7.00 min 15-55% B).

LC-MS (method 2): Rₜ = 1.11 min; MS (ESlpos): m/z = 512 [M+H]⁺

¹H-NMR (400 MHz, DMSO-d₆) δ [ppm]: 1.232 (4.68), 1.248 (4.71), 2.075 (1.05), 2.256 (9.73), 2.287 (1.11), 2.423 (0.69), 2.431 (0.73), 2.461 (0.89), 2.468 (0.97), 2.518 (1.87), 2.523 (1.28), 2.581 (0.62), 2.608 (0.64), 2.621 (0.84), 2.647 (0.91), 2.784 (0.75), 2.796 (0.85), 2.824 (0.60), 2.836 (0.54), 2.894 (0.84), 2.924 (0.77), 3.551 (0.73), 3.578 (1.50), 3.605 (1.43), 3.633 (0.47), 3.710 (1.64), 3.807 (0.53), 3.823 (1.04), 3.832 (1.11), 3.842 (0.98), 3.850 (1.46), 3.869 (0.77), 3.877 (0.64), 3.913 (16.00), 4.328 (0.52), 4.337 (0.53), 4.354 (1.00), 4.364 (0.93), 4.394 (1.42), 4.419 (0.59), 6.148 (1.38), 6.159 (1.51), 6.162 (1.26), 6.172 (1.39), 6.717 (2.96), 6.721 (3.33), 6.731 (5.63), 7.166 (2.10), 7.285 (2.65), 7.298 (2.69), 7.659 (3.27), 7.980 (3.07), 7.993 (2.91), 8.365 (4.18), 11.962 (1.67).

### Example 94

### 3-(3-chloro-2-methoxy-anilino)-2-{3-[(5,5-dimethyl-1,4-dioxan-2-yl)methoxy]-4-pyridyl}-7-methyl-1,5,6,7-tetrahydropyrrolo[3,2-c]pyridin-4-one (stereoisomer 1)

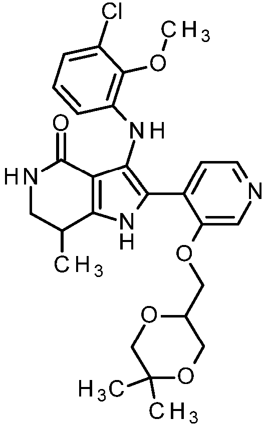

According to the method described for example 52 using N-(3-chloro-2-methoxy-phenyl)-4-({3-[(5,5-dimethyl-1,4-dioxan-2-yl)methoxy]-4-pyridyl}methylamino)-3-methyl-6-oxo-2,3-dihydro-1H-pyridine-5-carbothioamide (intermediate 6-94, 208 mg, 371 µmol) as starting material, 61.4 mg (98% purity, 31% yield) of the title compound as mixture of stereoisomers were prepared after purification by preparative HPLC (method 10, gradient: 0.00-0.50 min 30% B, 0.50-6.00 min 30-70% B).

LC-MS (method 2): Rₜ = 1.09 min; MS (ESlpos): m/z = 527 [M+H]⁺

¹H-NMR (400 MHz, DMSO-d₆) δ [ppm]: -0.008 (1.13), 0.008 (1.17), 1.056 (11.84), 1.205 (8.59), 1.225 (7.42), 1.259 (3.20), 1.268 (4.42), 1.275 (4.05), 1.283 (3.71), 2.520 (2.73), 2.524 (1.77), 3.071 (1.80), 3.082 (1.65), 3.088 (1.74), 3.099 (1.05), 3.368 (1.55), 3.458 (1.02), 3.467 (1.00), 3.475 (0.86), 3.561 (1.59), 3.573 (0.67), 3.583 (2.48), 3.588 (1.77), 3.596 (0.70), 3.602 (1.23), 3.611 (2.87), 3.616 (1.33), 3.626 (1.07), 3.636 (1.08), 3.652 (1.12), 3.660 (1.19), 3.678 (1.28), 3.690 (0.57), 3.707 (0.72), 3.856 (16.00), 3.860 (13.93), 3.888 (0.72), 3.897 (0.60), 3.905 (0.51), 3.913 (0.47), 4.138 (0.57), 4.155 (0.55), 4.164 (0.88), 4.182 (0.83), 4.199 (0.42), 4.212 (1.16), 4.226 (1.16), 4.234 (1.22), 4.244 (1.32), 4.250 (1.04), 4.258 (1.05), 4.271 (0.48), 4.276 (0.61), 4.284 (0.52), 6.130 (1.32), 6.142 (2.53), 6.151 (1.36), 6.155 (2.16), 6.165 (1.50), 6.634 (0.52), 6.645 (6.11), 6.650 (4.46), 6.654 (3.09), 6.660 (3.84), 6.663 (2.76), 7.126 (1.79), 7.132 (1.64), 7.260 (2.25), 7.265 (2.63), 7.273 (2.36), 7.278 (2.58), 7.439 (3.34), 7.479 (2.81), 8.046 (2.97), 8.051 (3.28), 8.058 (2.78), 8.064 (3.16), 8.405 (3.88), 8.410 (4.46), 10.978 (1.74), 10.994 (1.51).

The mixture of stereoisomers (53 mg) was separated into single enantiomers by preparative chiral HPLC to give 12.0 mg (92% purity, 21% yield) of the title compound (enantiomer 1, Rₜ = 5.7 - 6.8 min), enantiomer 2 (9.4 mg, Rₜ = 7.1 - 8.2 min, see example 94), and a mixture of enantiomer 3 and enantiomer 4 (16.0 mg, Rₜ = 11.6 - 13.2 min, see examples 95 and 96).

### Preparative chiral HPLC method: MTBE

Instrument: PrepCon Labomatic HPLC; Column: YMC Cellulose SB 5µ, 250x30; eluent A: methyl tert-butyl ether + 0.1 vol % diethylamine; eluent B: acetonitrile; isocratic: 55% B+45% A; flow: 80 ml/min; temperature: 25°C; UV: 254 nm

### Analytical chiral HPLC method: MTBE

Instrument: Waters Alliance 2695; Column: YMC Cellulose SB 3µ, 100x4.6; eluent A: methyl tert-butyl ether + 0.2 vol % diethylamine; eluent B: acetonitrile; isocratic: 90% A+10% B; flow: 1.4 ml/min; temperature: 25°C; UV: 254 nm
Analytical chiral HPLC: Rₜ = 3.61 min.
Optical rotation:[α]_{D} = 59.93 ° +/- 1.13° (c = 3.5 mg/ml in chloroform)

### Example 95

### 3-(3-chloro-2-methoxy-anilino)-2-[3-[(5,5-dimethyl-1,4-dioxan-2-yl)methoxy]-4-pyridyl]-7-methyl-1,5,6,7-tetrahydropyrrolo[3,2-c]pyridin-4-one (stereoisomer 2)

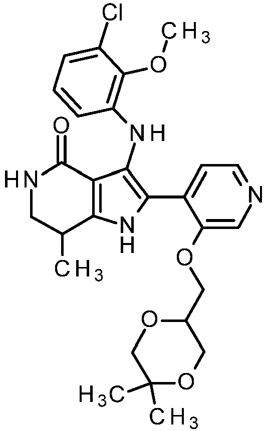

For the preparation of the title compound as mixture of stereoisomers see example. 94 Separation of enantiomers by preparative chiral HPLC (method see example 94) to give 9.4 mg (90 % purity, 16 % yield) of the title compound (enantiomer 2, Rₜ = 7.1 - 8.2 min).

Analytical chiral HPLC (method see example 93): Rₜ = 4.38 min.

Optical rotation:[α]_{D} = 9.81 ° +/- 0.96° (c = 4 mg/ml in chloroform)

### Example 96

### 3-(3-chloro-2-methoxy-anilino)-2-[3-[(5,5-dimethyl-1,4-dioxan-2-yl)methoxy]-4-pyridyl]-7-methyl-1,5,6,7-tetrahydropyrrolo[3,2-c]pyridin-4-one (stereoisomer 3)

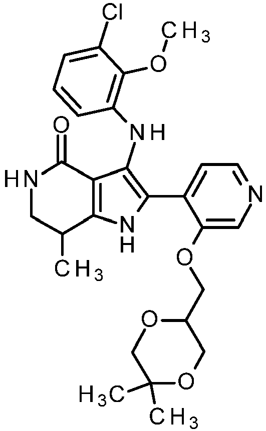

For the preparation of the title compound as the mixture of stereoisomers see example 94. Separation of enantiomers by preparative chiral HPLC (method see example 94) to give a mixture of enantiomer 3 and 4 (16 mg, Rₜ = 11.6 - 13.2 min). An additional preparative chiral HPLC gave 4.0 mg (94 % purity, 7 % yield) of the title compound (enantiomer 3, Rₜ = 5.9 - 6.9 min) and enantiomer 4 (2.3 mg, Rₜ = 7.2 - 8.3 min).

### Preparative chiral HPLC method: NPB

Instrument: PrepCon Labomatic HPLC; Column: YMC Amylose SA 10µ, 250x50; eluent A: hexane + 0.1 vol % diethylamine; eluent B: ethanol; isocratic: 50%A+50%B; flow: 150 mL/min; temperature: 25°C; UV: 220 nm

### Analytical chiral HPLC method: NPB

Instrument: Waters Alliance 2695; Column: YMC Amylose SA 3µ, 100x4.6; eluent A: hexane + 0.1 vol % diethylamine; eluent B: ethanol; isocratic: 50%A+50%B; flow: 1.4 ml/min; temperature: 25°C; UV: 220 nm
Analytical chiral HPLC: Rₜ = 1.73 min.
Optical rotation:[α]_{D} = - 8.97 ° +/- 1.78 ° (c = 1.6 mg/ml in chloroform)

### Example 97

### 3-(3-chloro-2-methoxy-anilino)-2-[3-[(5,5-dimethyl-1,4-dioxan-2-yl)methoxy]-4-pyridyl]-7-methyl-1,5,6,7-tetrahydropyrrolo[3,2-c]pyridin-4-one (stereoisomer 4)

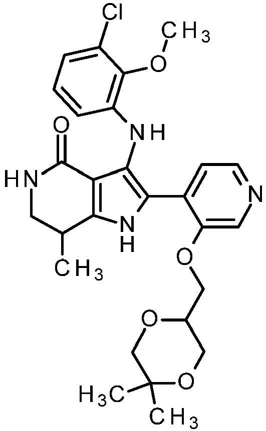

For the preparation of the title compound as the mixture of stereoisomers see intermediate 94. Separation of enantiomers by preparative chiral HPLC (method see example 94) to give a mixture of enantiomer 3 and 4 (16 mg, Rₜ = 11.6 - 13.2 min). An additional preparative chiral HPLC (method see example 96) gave 2.3 mg (90 % purity, 4 % yield) of the title compound (enantiomer 4, Rₜ = 7.2 - 8.3 min).

Analytical chiral HPLC (method see example 95): Rₜ = 2.02 min.

Optical rotation:[α]_{D} = - 38.15 ° +/- 1.40 ° (c = 3 mg/ml in chloroform)

### Example 98

### 3-(3-chloro-2-methoxy-anilino)-2-(3-{[(2S)-tetrahydrofuran-2-yl]methoxy}-4-pyridyl)-7-(3,3,3-trifluoropropyl)-1,5,6,7-tetrahydropyrrolo[3,2-c]pyridin-4-one (stereoisomer 1)

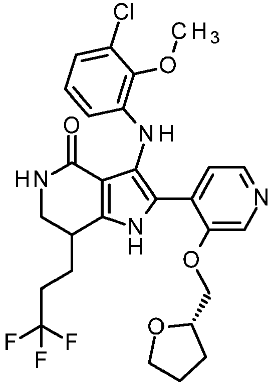

*N*-(3-chloro-2-methoxy-phenyl)-6-oxo-4-[(3-{[(2*S*)-tetrahydrofuran-2-yl]methoxy}-4-pyridyl)methylamino]-3-(3,3,3-trifluoropropyl)-2,3-dihydro-1*H*-pyridine-5-carbothioamide (intermediate 6-98, 375 mg, 626 µmol) was dissolved in MeOH (1.6 ml). TFA (48 µl, 626 µmol; CAS 76-05-1), 2,6-di-*tert*-butyl-4-methylphenole (138 mg, 626 µmol, CAS 128-37-0) and hydrogenperoxide (128 µl, 30%, 1.25 mmol) were added and the mixture was stirred at 100°C for 30 min. The reaction mixture was treated with sodiumthiosulfate solution and saturated potassiumcarbonate solution and extracted with ethylacetate. The organic layer was dried and concentrated under reduced pressure. The residue was purified by flash chromatography (silica, DCM / MeOH gradient 0-7%) to give 222 mg (63% yield) of the title compound as mixture of stereoisomers (impure with 25% of 3-(3-chloro-2-methoxy-anilino)-7-(3,3-difluoroallyl)-2-(3-{[(2S)-tetrahydrofuran-2-yl]methoxy}-4-pyridyl)-1,5,6,7-tetrahydropyrrolo[3,2-c]pyridin-4-one).

LC-MS (method 2): Rₜ = 1.21 min; MS (ESlpos): m/z = 565 [M+H]⁺

The title compound as mixture of stereoisomers (222 mg, impure with 25 % of 3-(3-chloro-2-methoxy-anilino)-7-(3,3-difluoroallyl)-2-(3-{[(2S)-tetrahydrofuran-2-yl]methoxy}-4-pyridyl)-1,5,6,7-tetrahydropyrrolo[3,2-c]pyridin-4-one) was separated into enantiomers by preparative chiral HPLC to give 57.0 mg (95 % purity, 35% yield) of the title compound (enantiomer 1, Rₜ = 8.2 - 9.4 min) and enantiomer 2 (56.0 mg, Rₜ = 9.6 - 10.7 min, see example 99).

### Preparative chiral HPLC method:

Instrument: PrepCon Labomatic HPLC; Column: YMC Cellulose SC 10µ, 250x50; eluent A: methyl tert-butyl ether + 0.1 vol% diethylamine; eluent B: acetonitrile + 0.1 vol% diethylamine; isocratic: 10% A + 90% B; flow: 100 mL/min; temperature: 25°C; UV: 280 nm

### Analytical chiral HPLC method:

Instrument: Waters Alliance 2695; Column: YMC Cellulose SC 3µ, 100x4.6; eluent A: methyl tert-butyl ether + 0.1 vol % diethylamine; eluent B: acetonitrile; isocratic: 10% A + 90% B; flow: 1.4 ml/min; temperature: 25°C; UV: 280 nm
Analytical chiral HPLC: Rₜ = 1.99 min.
Optical rotation:[α]_{D} = - 10.17 ° +/- 0.48 ° (c = 2.31 mg/ml in DMSO)
LC-MS (method 2): Rₜ = 1.21 min; MS (ESlpos): m/z = 565 [M+H]⁺
¹H-NMR (400 MHz, DMSO-d₆): δ [ppm]: 11.16 (s, 1H), 8.41 (s, 1H), 8.04 (d, 1H), 7.45 (s, 1H), 7.27 (d, 1H), 7.15 (br s, 1H), 6.61 - 6.72 (m, 2H), 6.11 - 6.20 (m, 1H), 4.24 - 4.39 (m, 2H), 3.94 - 4.07 (m, 1H), 3.86 (s, 3H), 3.70 - 3.81 (m, 2H), 3.56 (br dd, 1H), 3.19 - 3.29 (m, 1H), 3.03 (br dd, 1H), 2.24 - 2.45 (m, 2H), 1.94 - 2.09 (m, 2H), 1.60 - 1.93 (m, 4H).

### Example 99

### 3-(3-chloro-2-methoxy-anilino)-2-(3-{[(2S)-tetrahydrofuran-2-yl]methoxy}-4-pyridyl)-7-(3,3,3-trifluoropropyl)-1,5,6,7-tetrahydropyrrolo[3,2-c]pyridin-4-one (stereoisomer 2)

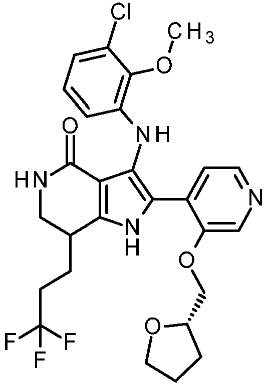

For the preparation of the title compound as mixture of stereoisomers see example 98. Separation of enantiomers by preparative chiral HPLC (method see example 98) to give 56.0 mg (95% purity, 32% yield) of the title compound (enantiomer 2, Rₜ = 9.6 - 10.7 min).
Analytical chiral HPLC (method see example 98): Rₜ = 2.25 min.
Optical rotation:[α]_{D} = 27.97 ° +/- 0.90 ° (c = 2.65 mg/ml in DMSO)
LC-MS (method 2): Rₜ = 1.21 min; MS (ESlpos): m/z = 565 [M+H]⁺
¹H-NMR (400 MHz, DMSO-d₆) δ [ppm]: 11.17 (s, 1H), 8.42 (s, 1H), 8.05 (d, 1H), 7.44 (s, 1H), 7.28 (d, 1H), 7.16 (br s, 1H), 6.62 - 6.74 (m, 2H), 6.15 (dd, 1H), 4.23 - 4.36 (m, 2H), 4.00 - 4.11 (m, 1H), 3.86 (s, 4H), 3.70 - 3.78 (m, 1H), 3.50 - 3.62 (m, 1H), 3.18 - 3.28 (m, 1H), 3.05 (br dd, 1H), 2.23 - 2.44 (m, 2H), 1.93 - 2.14 (m, 2H), 1.61 - 1.92 (m, 4H).

### Example 100

### 3-(3-chloro-2-methoxy-anilino)-7-(3,3-difluoroallyl)-2-(3-{[(2S)-tetrahydrofuran-2-yl]methoxy}-4-pyridyl)-1,5,6,7-tetrahydropyrrolo[3,2-c]pyridin-4-one (stereoisomer 1)

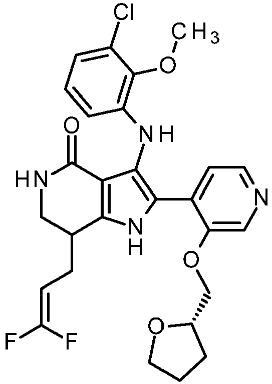

The side product from example 98 (222 mg, mixture of stereoisomers, 25% purity) was separated into enantiomers by preparative chiral HPLC (method see example 98) to give 25.5 mg (95% purity, 16% yield) of the title compound (enantiomer 1, Rₜ = 11.0 - 11.9 min) and enantiomer 2 (21.5 mg, Rₜ = 12.2 - 13.2 min, see example 101).
Analytical chiral HPLC (method see example 98): Rₜ = 2.50 min.
Optical rotation:[α]_{D} = - 34.95 ° +/- 2.67 ° (c = 1.33 mg/ml in DMSO)
LC-MS (method 2): Rₜ = 1.20 min; MS (ESlpos): m/z = 545 [M+H]⁺
¹H-NMR (400 MHz, DMSO-d₆) δ [ppm]: 11.18 (s, 1H), 8.43 (s, 1H), 8.05 (d, 1H), 7.45 (s, 1H), 7.29 (d, 1H), 7.14 (br s, 1H), 6.61 - 6.72 (m, 2H), 6.15 (dd, 1H), 4.53 - 4.67 (m, 1H), 4.24 - 4.40 (m, 2H), 4.03 (dd, 1H), 3.81 - 3.91 (m, 4H), 3.70 - 3.79 (m, 1H), 3.48 - 3.58 (m, 1H), 3.14 - 3.25 (m, 1H), 2.97 - 3.07 (m, 1H), 2.40 - 2.48 (m, 1H), 2.22 - 2.31 (m, 1H), 1.95 - 2.08 (m, 1H), 1.82 - 1.93 (m, 2H), 1.58 - 1.73 (m, 1H).

### Example 101

### 3-(3-chloro-2-methoxy-anilino)-7-(3,3-difluoroallyl)-2-(3-{[(2S)-tetrahydrofuran-2-yl]methoxy}-4-pyridyl)-1,5,6,7-tetrahydropyrrolo[3,2-c]pyridin-4-one (stereoisomer 2)

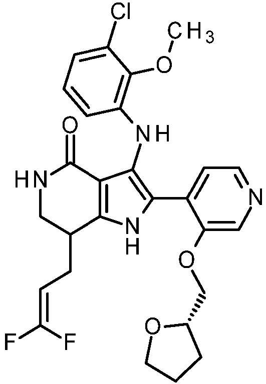

The side product from example 98 (222.0 mg, mixture of stereoisomers, 25% purity) was separated into enantiomers by preparative chiral HPLC (method see example 98) to give 21.5 mg (95% purity, 13% yield) of the title compound (enantiomer 2, Rₜ = 12.2 - 13.2 min).
Analytical chiral HPLC (method see example 98): Rₜ = 2.76 min.
Optical rotation:[α]_{D} = 45.40 ° +/- 2.09 ° (c = 1.49 mg/ml in DMSO)
LC-MS (method 2): Rₜ = 1.20 min; MS (ESlpos): m/z = 545 [M+H]⁺
¹H-NMR (400 MHz, DMSO-d₆) δ [ppm]: 11.17 (s, 1H), 8.42 (s, 1H), 8.04 (d, 1H), 7.47 (s, 1H), 7.27 (d, 1H), 7.14 (br s, 1H), 6.59 - 6.80 (m, 2H), 6.07 - 6.24 (m, 1H), 4.53 - 4.68 (m, 1H), 4.27 - 4.43 (m, 2H), 4.00 (dd, 1H), 3.86 (s, 3H), 3.72 - 3.85 (m, 2H), 3.53 (br dd, 1H), 3.20 (dt, 1H), 2.96 - 3.06 (m, 1H), 2.37 - 2.46 (m, 1H), 2.23 - 2.32 (m, 1H), 1.97 - 2.08 (m, 1H), 1.83 - 1.94 (m, 2H), 1.61 - 1.74 (m, 1H).

### Example 102

### (6R)-3-(3-chloro-2-methylanilino)-2-{3-[(1,4-dioxan-2-yl)methoxy]pyridin-4-yl}-6-methyl-1,5,6,7-tetrahydro-4H-pyrrolo[3,2-c]pyridin-4-one

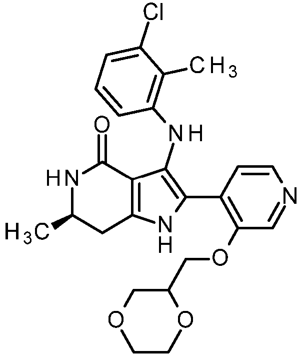

According to the method described for example 52 using (6R)-N-(3-chloro-2-methylphenyl)-4-[({3-[(1,4-dioxan-2-yl)methoxy]pyridin-4-yl}methyl)amino]-6-methyl-2-oxo-1,2,5,6-tetrahydropyridine-3-carbothioamide (intermediate 6-102, 200 mg, 387 µmol) as starting material, 35.8 mg (95% purity, 18% yield) of the title compound were prepared after purification by preparative HPLC (method 10, gradient: 0.00-0.50 min 30% B, 0.50-7.00 min 30-70% B).

LC-MS (method 2): Rₜ = 1.07 min; MS (ESlpos): m/z = 483 [M+H]⁺

¹H-NMR (400 MHz, DMSO-d₆) δ [ppm]: 1.22 - 1.27 (d, 3H), 2.34 - 2.37 (s, 3H), 2.61 (dd, 1H), 2.87 - 2.97 (m, 1H), 3.38 - 3.45 (m, 1H), 3.52 (m, 1H), 3.69 - 3.83 (m, 4H), 3.88 (m, 1H), 3.96 - 4.04 (m, 1H), 4.06 - 4.30 (m, 2H), 6.20 (d, 1H), 6.71 - 6.79 (m, 2H), 7.14 (s, 1H), 7.23 (dd, 1H), 7.33 (d, 1H), 8.00 (dd, 1H), 8.36 (d, 1H), 11.02 (d, 1H).

### Example 103

### (6R)-3-(3-chloro-2-methylanilino)-2-(3-{[(2S)-1,4-dioxan-2-yl]methoxy}pyridin-4-yl)-6-methyl-1,5,6,7-tetrahydro-4H-pyrrolo[3,2-c]pyridin-4-one

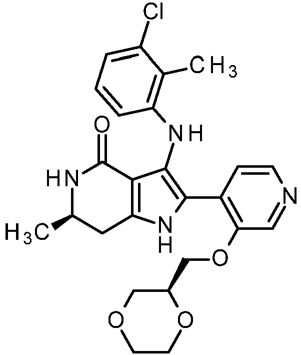

According to the method described for example 52 using (6*R*)-*N*-(3-chloro-2-methylphenyl)-4-{[(3-{[(2*S*)-1,4-dioxan-2-yl]methoxy}pyridin-4-yl)methyl]amino}-6-methyl-2-oxo-1,2,5,6-tetrahydropyridine-3-carbothioamide (intermediate 6-103, 246 mg, 476 µmol) as starting material, 26.7 mg (90% purity, 10% yield) of the title compound were prepared after purification by preparative HPLC (method 10, gradient: 0.00-0.50 min 30% B, 0.50-7.00 min 30-70% B).

LC-MS (method 2): Rₜ = 1.07 min; MS (ESlpos): m/z = 483 [M+H

¹H-NMR (400 MHz, DMSO-d₆) δ [ppm]: 10.99 - 11.11 (m, 1H), 8.34 - 8.40 (m, 1H), 7.93 - 8.02 (m, 1H), 7.31 - 7.36 (m, 1H), 7.21 - 7.26 (m, 1H), 7.11 - 7.16 (m, 1H), 6.66 - 6.82 (m, 2H), 6.15 - 6.24 (m, 1H), 4.10 - 4.26 (m, 2H), 3.96 - 4.04 (m, 1H), 3.85 - 3.93 (m, 1H), 3.67 - 3.82 (m, 4H), 3.49 - 3.56 (m, 1H), 3.38 - 3.47 (m, 1H), 2.88 - 2.97 (m, 1H), 2.56 - 2.65 (m, 1H), 2.34 - 2.38 (m, 3H), 1.19 - 1.29 (m, 3H).

### Analytical chiral HPLC method:

Instrument: Thermo Fisher UltiMate 3000; Column: YMC Cellulose SC 3µ, 100x4.6; eluent A: hexane + 0.1 vol % diethylamine; eluent B: ethanol; isocratic: 80%A+20%B; flow: 1.4 ml/min; temperature: 25°C; UV: 254 nm;
Analytical chiral HPLC: Rₜ = 8.86 min.

### Example 104

### (6R)-3-(3-chloro-2-methylanilino)-6-methyl-2-(3-{[(2S)-oxolan-2-yl]methoxy}pyridin-4-yl)-1,5,6,7-tetrahydro-4H-pyrrolo[3,2-c]pyridin-4-one

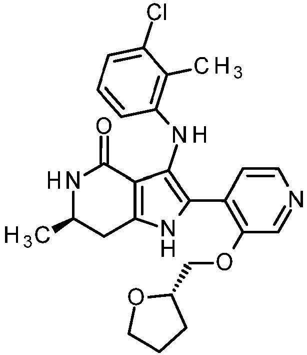

According to the method described for example 52 using (6*R*)-*N*-(3-chloro-2-methylphenyl)-6-methyl-2-oxo-4-{[(3-{[(2*S*)-oxolan-2-yl]methoxy}pyridin-4-yl)methyl]amino}-1,2,5,6-tetrahydropyridine-3-carbothioamide (intermediate 6-104, 200 mg, 399 µmol) as starting material, 54.0 mg (95% purity, 28% yield) of the title compound were prepared after purification by preparative HPLC (method 10, gradient: 0.00-0.50 min 30% B, 0.50-7.00 min 30-70% B).

LC-MS (method 2): Rₜ = 1.15 min; MS (ESlpos): m/z = 467 [M+H]⁺

¹H-NMR (400 MHz, DMSO-d₆) δ [ppm]: 1.24 (d, 3H), 1.66 (m, 1H), 1.84 - 1.92 (m, 2H), 1.99 (m, 1H), 2.34 (s, 3H), 2.54 - 2.63 (dd, 1H), 2.91 (dd, 1H), 3.74 - 3.82 (m, 2H), 3.82 - 3.88 (m, 1H), 4.00 - 4.06 (dd, 1H), 4.26 - 4.33 (m, 2H), 6.21 (dd, 1H), 6.70 - 6.79 (m, 2H), 7.11 (s, 1H), 7.22 - 7.27 (m, 2H), 8.00 (d, 1H), 8.39 (s, 1H), 11.18 (s, 1H).

### Example 105

### (6R)-3-(3-chloro-2-methylanilino)-2-{3-[(1-methoxypropan-2-yl)oxy]pyridin-4-yl}-6-methyl-1,5,6,7-tetrahydro-4H-pyrrolo[3,2-c]pyridin-4-one

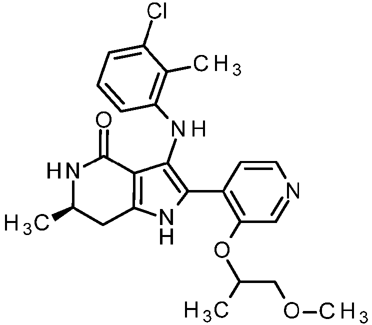

According to the method described for example 52 using ((6*R*)-*N*-(3-chloro-2-methylphenyl)-4-[({3-[(1-methoxypropan-2-yl)oxy]pyridin-4-yl}methyl)amino]-6-methyl-2-oxo-1,2,5,6-tetrahydropyridine-3-carbothioamide (intermediate 6-105, 186 mg, 380 µmol) as starting material, 38.1 mg (90% purity, 20% yield) of the title compound were prepared after purification by preparative HPLC (method 10, gradient: 0.00-0.50 min 30% B, 0.50-7.00 min 30-70% B).

LC-MS (method 2): Rₜ = 1.15 min; MS (ESlpos): m/z = 455 [M+H]⁺

¹H-NMR (400 MHz, DMSO-d₆) δ [ppm]: 1.22 - 1.32 (m, 6H), 2.34 (d, 3H), 2.55 - 2.66 (m, 1H), 2.84 - 2.98 (dd, 1H), 3.34 - 3.38 (m, 3H), 3.50 - 3.62 (m, 2H), 3.72 - 3.88 (m, 1H), 4.73 (br d, 1H), 6.19 - 6.23 (m, 1H), 6.69 - 6.78 (m, 2H), 7.12 (d, 1H), 7.24 (t, 1H), 7.31 (d, 1H), 7.98 (dd, 1H), 8.38 (d, 1H), 11.00 (br d, 1H).

### Example 106

### (6R)-3-(3-chloro-2-methylanilino)-6-methyl-2-(3-{[(2R)-4-methylmorpholin-2-yl]methoxy}pyridin-4-yl)-1,5,6,7-tetrahydro-4H-pyrrolo[3,2-c]pyridin-4-one

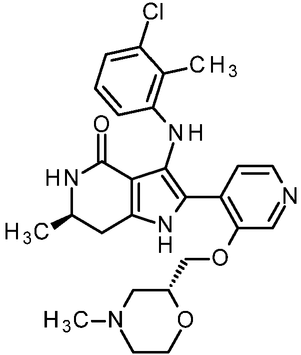

According to the method described for example 64 using (6*R*)-3-(3-chloro-2-methylanilino)-6-methyl-2-(3-{[(2*R*)-morpholin-2-yl]methoxy}pyridin-4-yl)-1,5,6,7-tetrahydro-4*H*-pyrrolo[3,2-c]pyridin-4-one (intermediate 106-2, 54.0 mg, 112 µmol) as starting material, 15.7 mg (95% purity, 27% yield) of the title compound were prepared after purification by preparative HPLC (method 10, gradient: 0.00-0.50 min 30% B, 0.50-6.00 min 30-70% B).

LC-MS (method 2): Rₜ = 1.08 min; MS (ESlpos): m/z = 496 [M+H]⁺

¹H-NMR (400 MHz, DMSO-d₆) δ [ppm]= 1.24 (d, 3H), 1.88 (t, 1H), 1.99 - 2.08 (td, 1H), 2.20 (s, 3H), 2.36 (s, 3H), 2.55 - 2.69 (m, 2H), 2.75 (br d, 1H), 2.89 (dd, 1H), 3.68 (td, 1H), 3.74 - 3.86 (m, 1H), 3.95 (m, 2H), 4.10 (dd, 1H), 4.30 (dd, 1H), 6.20 (dd, 1H), 6.71 - 6.79 (m, 2H), 7.14 (s, 1H), 7.23 (d, 1H), 7.35 (s, 1H), 7.99 (d, 1H), 8.36 (s, 1H), 11.01 (s, 1H).

### Example 107

### (6R)-3-(3-chloro-2-methylanilino)-6-methyl-2-(3-{[(2S)-4-methylmorpholin-2-yl]methoxy}pyridin-4-yl)-1,5,6,7-tetrahydro-4H-pyrrolo[3,2-c]pyridin-4-one

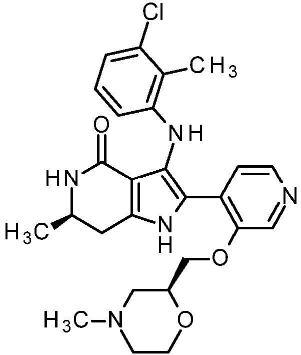

According to the method described for example 64 using (6*R*)-3-(3-chloro-2-methylanilino)-6-methyl-2-(3-{[(2*S*)-morpholin-2-yl]methoxy}pyridin-4-yl)-1,5,6,7-tetrahydro-4*H*-pyrrolo[3,2-c]pyridin-4-one (intermediate 107-2, 55.0 mg, 114 µmol) as starting material, 15.7 mg (95% purity, 27% yield) of the title compound were prepared after purification by preparative HPLC (method 10, gradient: 0.00-0.50 min 30% B, 0.50-6.00 min 30-70% B).

LC-MS (method 2): Rₜ = 1.08 min; MS (ESlpos): m/z = 496 [M+H]⁺

¹H-NMR (400 MHz, DMSO-d₆) δ [ppm]: 1.24 (d, 3H), 1.85 - 1.99 (t, 1H), 1.99 - 2.17 (td, 1H), 2.21 (s, 3H), 2.35 (s, 3H), 2.57 - 2.76 (m, 3H), 2.92 (dd, 1H), 3.60 - 3.69 (td, 1H), 3.75 - 3.85 (m, 1H), 3.88 - 3.98 (m, 2H), 4.11 - 4.25 (m, 2H), 6.20 (dd, 1H), 6.71 - 6.79 (m, 2H), 7.14 (s, 1H), 7.24 (d, 1H), 7.35 (s, 1H), 8.00 (d, 1H), 8.37 (s, 1H), 11.02 (s, 1H).

### Example 108

### (6R)-3-[2-(2,2-difluoroethyl)-3-fluoro-anilino]-2-[3-(1,4-dioxan-2-ylmethoxy)-4-pyridyl]-6-methyl-1,5,6,7-tetrahydropyrrolo[3,2-c]pyridin-4-one

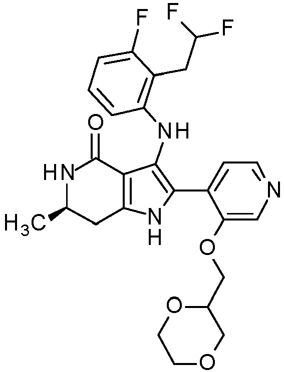

According to the method described for example 52 using (2*R*)-*N*-[2-(2,2-difluoroethyl)-3-fluoro-phenyl]-4-{[3-(1,4-dioxan-2-ylmethoxy)-4-pyridyl]methylamino}-2-methyl-6-oxo-2,3-dihydro-1*H*-pyridine-5-carbothioamide (Intermediate 6-108, 55.0 mg, 99.9 µmol) as starting material, 11.0 mg (90% purity, 19% yield) of the title compound were prepared after purification by flash chromatography (silica, DCM / MeOH gradient 0-8%).

LC-MS (method 2): Rₜ = 1.01 min; MS (ESlpos): m/z = 517 [M+H]⁺

¹H-NMR (400 MHz, DMSO-d₆) δ [ppm]: 0.798 (0.43), 0.816 (0.45), 0.822 (0.45), 0.852 (0.76), 0.905 (0.49), 1.083 (0.75), 1.118 (0.84), 1.134 (0.93), 1.143 (0.47), 1.184 (0.44), 1.228 (16.00), 1.242 (16.00), 2.337 (0.78), 2.518 (6.55), 2.523 (4.81), 2.573 (2.65), 2.599 (2.87), 2.613 (3.24), 2.639 (3.52), 2.678 (0.63), 2.897 (2.39), 2.908 (2.46), 2.935 (1.95), 2.948 (1.86), 3.277 (1.89), 3.351 (2.01), 3.393 (2.38), 3.399 (2.35), 3.418 (3.10), 3.423 (4.52), 3.427 (2.93), 3.447 (3.22), 3.452 (2.91), 3.475 (0.69), 3.488 (1.15), 3.494 (1.77), 3.501 (1.22), 3.517 (2.27), 3.523 (3.69), 3.530 (2.17), 3.544 (1.79), 3.552 (2.46), 3.559 (1.32), 3.639 (0.41), 3.660 (0.53), 3.694 (1.37), 3.701 (2.80), 3.709 (4.08), 3.716 (3.33), 3.722 (3.61), 3.729 (3.83), 3.746 (4.79), 3.751 (4.00), 3.764 (2.43), 3.774 (3.27), 3.790 (2.14), 3.805 (4.63), 3.832 (3.05), 3.879 (3.45), 3.883 (3.76), 3.913 (2.78), 3.992 (1.17), 4.001 (1.90), 4.008 (2.33), 4.017 (2.49), 4.026 (2.26), 4.033 (1.82), 4.041 (1.45), 4.049 (0.67), 4.102 (2.28), 4.118 (1.81), 4.127 (3.52), 4.144 (2.84), 4.151 (3.20), 4.167 (2.69), 4.247 (2.34), 4.256 (2.34), 4.273 (1.93), 4.279 (2.89), 4.288 (2.47), 4.305 (1.95), 4.314 (1.71), 5.759 (11.34), 6.113 (6.40), 6.134 (6.54), 6.303 (1.05), 6.444 (4.89), 6.465 (4.84), 6.468 (4.60), 6.488 (3.04), 6.585 (0.95), 6.857 (2.32), 6.877 (4.68), 6.894 (4.80), 6.915 (2.12), 7.057 (5.98), 7.249 (0.44), 7.260 (0.58), 7.326 (6.20), 7.335 (6.53), 7.358 (11.37), 7.371 (11.39), 7.995 (9.26), 7.998 (8.54), 8.008 (8.41), 8.010 (7.62), 8.202 (0.43), 8.227 (0.54), 8.238 (0.46), 8.379 (11.64), 8.383 (10.59), 11.046 (6.11).

### Example 109

### (6R)-3-(3-chloro-2-ethylanilino)-6-methyl-2-(3-{[(2S)-oxolan-2-yl]methoxy}pyridin-4-yl)-1,5,6,7-tetrahydro-4H-pyrrolo[3,2-c]pyridin-4-one

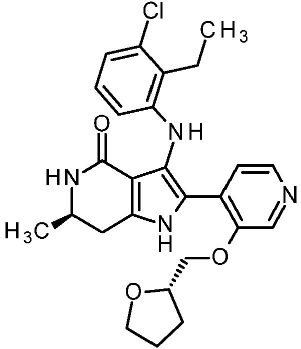

According to the method described for intermediate 91-1 using (6R)-N-(3-chloro-2-ethyl phenyl)-6-methyl-2-oxo-4-{[(3-{[(2*S*)-oxolan-2-yl]methoxy}pyridin-4-yl)methyl]amino}-1,2,5,6-tetrahydropyridine-3-carbothioamide (intermediate 6-109, 130 mg, 252 µmol) as starting material, 31.3 mg (85% purity, 22% yield) of the title compound were prepared after purification by preparative HPLC (method 10, gradient: 0.00-0.50 min 30% B, 0.50-7.00 min 30-70% B).

LC-MS (method 2): Rₜ = 1.23 min; MS (ESlpos): m/z = 481 [M+H]⁺

¹H-NMR (400 MHz, DMSO-d₆) δ [ppm]: 1.17 - 1.27 (m, 6H), 1.61 - 1.73 (m, 1H), 1.82 - 1.93 (m, 2H), 1.95 - 2.06 (m, 1H), 2.59 (dd, 1H), 2.80 - 2.88 (q, 2H), 2.91 (dd, 1H), 3.74 - 3.89 (m, 4H), 3.99 - 4.07 (m, 1H), 4.28 - 4.35 (m, 2H), 6.24 (dd, 1H), 6.66 - 6.77 (m, 2H), 7.11 (s, 1H), 7.19 (d, 1H), 7.34 (s, 1H), 7.97 (d, 1H), 8.39 (s, 1H), 11.18 (s, 1H).

### Example 110

### (6R)-3-(3-chloro-2-ethylanilino)-2-(3-{[(2S)-1,4-dioxan-2-yl]methoxy}pyridin-4-yl)-6-methyl-1,5,6,7-tetrahydro-4H-pyrrolo[3,2-c]pyridin-4-one

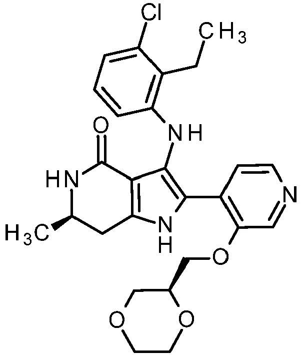

According to the method described for intermediate 91-1 using (6*R*)-*N*-(3-chloro-2-ethylphenyl)-4-{[(3-{[(2*S*)-1,4-dioxan-2-yl]methoxy}pyridin-4-yl)methyl]amino}-6-methyl-2-oxo-1,2,5,6-tetrahydropyridine-3-carbothioamide (intermediate 6-110, 144 mg, 271 µmol) as starting material, 37.4 mg (85% purity, 24% yield) of the title compound were prepared after purification by preparative HPLC (method 10, gradient: 0.00-0.50 min 30% B, 0.50-7.00 min 30-70% B).

LC-MS (method 2): Rₜ = 1.15 min; MS (ESlpos): m/z = 497 [M+H]⁺

¹H-NMR (400 MHz, DMSO-d₆) δ [ppm]: 1.20 - 1.26 (m, 6H), 2.61 (dd, 1H), 2.85 (q, 2H), 2.92 (dd, 1H), 3.42 (dd, 1H), 3.49 - 3.59 (td, 1H), 3.69 - 3.76 (m, 2H), 3.76 - 3.83 (m, 2H), 3.86 - 3.93 (dd, 1H), 4.01 (m, 1H), 4.11 - 4.18 (m, 1H), 4.18 - 4.24 (dd, 1H) 6.22 (dd, 1H), 6.67 - 6.77 (m, 2H), 7.13 (s, 1H), 7.20 (d, 1H), 7.42 (s, 1H), 7.98 (d, 1H), 8.36 (s, 1H), 11.01 (s, 1H).

### Example 111

### 3'-(3-chloro-2-methoxyanilino)-2'-(3-{[(2S)-1,4-dioxan-2-yl]methoxy}pyridin-4-yl)-1',7'-dihydrospiro[cyclopropane-1,6'-pyrrolo[3,2-c]pyridin]-4'(5'H)-one

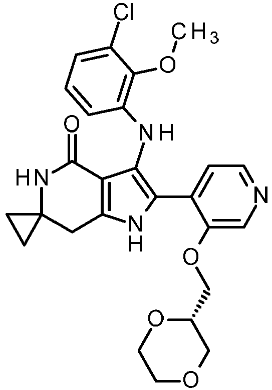

*N*-(3-chloro-2-methoxyphenyl)-7-{[(3-{[(2*S*)-1,4-dioxan-2-yl]methoxy}pyridin-4-yl)methyl]amino}-5-oxo-4-azaspiro[2.5]oct-6-ene-6-carbothioamide (intermediate 6-111, 220 mg, 404 µmol) was suspended in methanol (4.1 ml). TFA (34 µl, 440 µmol) and hydrogen peroxide (71 µl, 35%, 0.81 mmol) were added and the reaction mixture was heated for 17h to 50°C. The reaction mixture was quenched with saturated sodium bisulfit solution and sodium bicarbonate solution and stirred for 15min. To the suspension was diluted with water and extracted with DCM. The combined organic phases were dried, concentrated and purified by preparative HPLC (method 10, gradient: 0.00-0.50 min 5% B, 0.50-7.00 min 5-50% B) to give 121 mg (93% purity, 55% yield) of the title compound.

LC-MS (method 2): Rₜ = 1.09 min; MS (ESlpos): m/z = 511.2 [M+H]⁺

¹H-NMR (400 MHz, DMSO-d₆) δ [ppm]: 0.60 - 0.85 (m, 4H), 2.77 - 2.93 (m, 2H), 3.38 - 3.55 (m, 2H), 3.66 - 3.75 (m, 2H), 3.79 - 3.89 (m, 5H), 3.96 - 4.07 (m, 1H), 4.10 - 4.17 (m, 1H), 4.24 - 4.34 (m, 1H), 6.11 - 6.20 (m, 1H), 6.65 - 6.74 (m, 2H), 7.08 - 7.26 (m, 1H), 7.11 - 7.19 (m, 1H), 7.21 - 7.26 (m, 1H), 7.21 - 7.27 (m, 1H), 7.26 - 7.33 (m, 1H), 7.51 - 7.58 (m, 1H), 8.01 - 8.07 (m, 1H), 8.36 - 8.44 (m, 1H), 11.02 - 11.15 (m, 1H).

### Example 112

### 3'-(3-chloro-2-methoxyanilino)-2'-(3-{[(2S)-4-methylmorpholin-2-yl]methoxy}pyridin-4-yl)-1',7'-dihydrospiro[cyclopropane-1,6'-pyrrolo[3,2-c]pyridin]-4'(5'H)-one

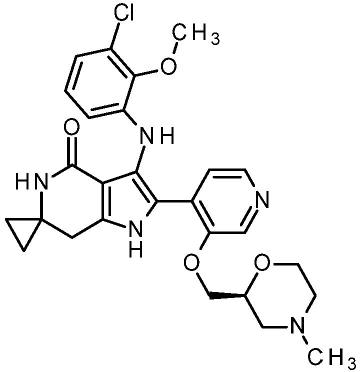

According to the method described for example 64 using 3'-(3-chloro-2-methoxyanilino)-2'-(3-{[(2*S*)-morpholin-2-yl]methoxy}pyridin-4-yl)-1',7'-dihydrospiro[cyclopropane-1,6'-pyrrolo[3,2-c]pyridin]-4'(5'H)-one (intermediate 112-2, 61.2 mg, 120 µmol) (intermediate 115-2, 46.0 mg, 87.1 µmol) as starting material, 20.2 mg (95 % purity, 30 % yield) of the title compound were prepared after purification by flash chromatography (amino phase silica, DCM / 2-propanol, gradient 1-4%).

LC-MS (method 2): Rₜ = 1.06 min; MS (ESlpos): m/z = 452.3 [M+H]⁺

¹H-NMR (400 MHz, DMSO-d₆) δ [ppm]: 0.61 - 0.83 (m, 4H), 1.84 - 2.05 (m, 2H), 2.16 - 2.22 (m, 3H), 2.60 - 2.66 (m, 1H), 2.72 - 2.77 (m, 1H), 2.78 - 2.92 (m, 2H), 3.60 - 3.71 (m, 1H), 3.82 - 4.01 (m, 5H), 3.86 - 3.90 (m, 3H), 4.10 - 4.20 (m, 1H), 4.28 - 4.39 (m, 1H), 4.62 - 4.83 (m, 1H), 6.11 - 6.23 (m, 1H), 6.63 - 6.73 (m, 2H), 7.23 - 7.32 (m, 2H), 7.55 (s, 1H), 7.96 - 8.11 (m, 1H), 8.03 (d, 1H), 8.40 (s, 1H), 10.97 - 11.15 (m, 1H).

### Example 113

### 3'-(3-chloro-2-methoxyanilino)-2'-(3-{[(2S)-oxolan-2-yl]methoxy}pyridin-4-yl)-1',7'-dihydrospiro[cyclopropane-1,6'-pyrrolo[3,2-c]pyridin]-4'(5'H)-one

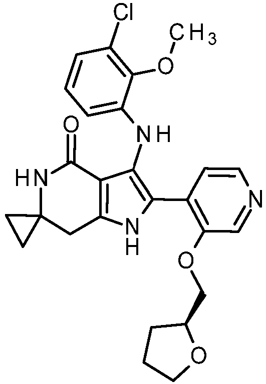

According to the method described for example 111 using *N*-(3-chloro-2-methoxyphenyl)-5-oxo-7-{[(3-{[(2*S*)-oxolan-2-yl]methoxy}pyridin-4-yl)methyl]amino}-4-azaspiro[2.5]oct-6-ene-6-carbothioamide (intermediate 6-113, 240 mg, 454 µmol) as starting material, 70.0 mg (90% purity, 28% yield) of the title compound were prepared after purification by preparative HPLC (method 10, gradient: 0.00-0.50 min 15% B, 0.50-6.00 min 15-55% B).

LC-MS (method 2): Rₜ = 1.15 min; MS (ESlpos): m/z = 495.2 [M+H]⁺

¹H-NMR (400 MHz, DMSO-d₆) δ [ppm]: 0.61 - 0.84 (m, 4H), 1.55 - 1.75 (m, 1H), 1.81 - 1.94 (m, 2H), 1.95 - 2.05 (m, 1H), 2.72 - 2.93 (m, 2H), 3.66 - 3.68 (m, 1H), 3.72 - 3.90 (m, 5H), 3.99 - 4.09 (m, 1H), 4.25 - 4.41 (m, 2H), 6.01 - 6.24 (m, 1H), 6.59 - 6.72 (m, 2H), 7.16 - 7.33 (m, 2H), 7.20 - 7.31 (m, 2H), 7.46 - 7.54 (m, 1H), 7.59 - 7.68 (m, 1H), 7.99 - 8.07 (m, 1H), 8.24 - 8.30 (m, 1H), 8.35 - 8.50 (m, 1H), 11.18 - 11.33 (m, 1H).

### Example 114

### 7-allyl-2-[3-(2-allyloxyethoxy)-4-pyridyl]-3-(3-chloro-2-methoxy-anilino)-1,5,6,7-tetrahydropyrrolo[3,2-c]pyridin-4-one

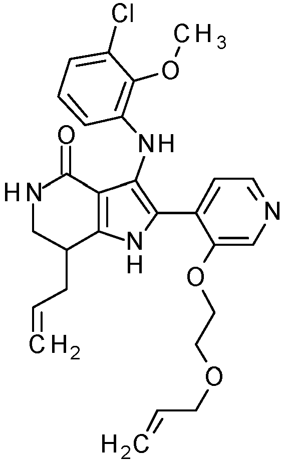

3-Allyl-4-{[3-(2-allyloxyethoxy)-4-pyridyl]methylamino}-*N*-(3-chloro-2-methoxy-phenyl)-6-oxo-2,3-dihydro-1*H*-pyridine-5-carbothioamide (Intermediate 6-114, 420 mg, 773 µmol) was suspended in methanol (2.0 mL) and TFA (60 µl, 770 µmol), 2,6-di-tert-butyl-4-methylphenole (170 mg, 773 µmol) and hydrogenperoxide (160 µl, 30%, 1.5 mmol) were added. The reaction mixture was stirred for 30 min at 100 °C. The reaction mixture was quenched with saturated sodiumbisulfit solution and sodiumbicarbonate solution and extracted with DCM/MeOH. The organic layer was dried and concentrated under reduced pressure. The residue was purified by flash chromatography (silica, DCM / MeOH gradient 0-7%) to give 258 mg (84% purity, 65% yield) of the title compound.

LC-MS (method 2): Rₜ = 1.18 min; MS (ESlpos): m/z = 509 [M+H]⁺

¹H-NMR (400 MHz, DMSO-d₆) δ [ppm]= 2.26 - 2.37 (m, 1 H), 2.51 - 2.58 (m, 1 H), 3.04 (br dd, 1 H), 3.18 (ddd, 1 H), 3.42 - 3.50 (m, 1 H), 3.79 - 3.84 (m, 2 H), 3.86 (s, 3 H), 4.05 (dt, 2 H), 4.28 - 4.38 (m, 2 H), 5.08 - 5.18 (m, 3 H), 5.19 - 5.31 (m, 1 H), 5.81 - 5.96 (m, 2 H), 6.09 - 6.18 (m, 1 H), 6.63 - 6.69 (m, 2 H), 7.11 (br s, 1 H), 7.29 (d, 1 H), 7.49 (s, 1 H), 8.05 (d, 1 H), 8.42 (s, 1 H), 10.96 (s, 1 H).

### Example 115

### 3-(3-chloro-2-methoxyanilino)-7-(2-hydroxyethyl)-2-(3-{[(3S)-4-methylmorpholin-3-yl]methoxy}pyridin-4-yl)-1,5,6,7-tetrahydro-4H-pyrrolo[3,2-c]pyridin-4-one stereoisomer 1

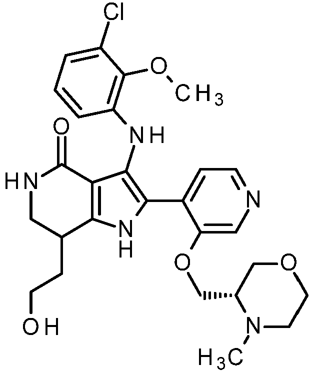

According to the method described for example 64 using 3-(3-chloro-2-methoxyanilino)-7-(2-hydroxyethyl)-2-(3-{[(3*S*)-morpholin-3-yl]methoxy}pyridin-4-yl)-1,5,6,7-tetrahydro-4H-pyrrolo[3,2-c]pyridin-4-one (intermediate 115-2, 46.0 mg, 87.1 µmol) as starting material, 9.54 mg (90% purity) of the title compound were prepared after purification by flash chromatography (amino phase silica, DCM / 2-propanol, gradient 1-4%) together with 7,60 mg; (70% purity, see example 116) of the second stereoisomer.

LC-MS (method 2): Rₜ = 0.94 min; MS (ESlpos): m/z = 442.3 [M+H]⁺

¹H-NMR (400 MHz, DMSO-d₆): δ [ppm]= 1.68 - 1.78 (m, 1H), 1.81 - 1.94 (m, 1H), 2.25 - 2.38 (m, 4H), 2.92 (d, 1H), 2.98 - 3.10 (m, 1H), 3.18 - 3.30 (m, 1H), 3.40 - 3.64 (m, 5H), 3.69 - 3.87 (m, 2H), 3.89 (s, 3H), 4.29 (dd, 1H) 4.40 (dd, 2H), 4.94 (t, 1H), 6.05 - 6.23 (m, 1H), 6.58 - 6.75 (m, 2H), 7.10 - 7.19 (m, 1H), 7.33 (d, 1H), 7.56 (s, 1H), 8.01 (d, 1H), 8.42 (s, 1H), 11.43 (s, 1H).

### Example 116

### 3-(3-chloro-2-methoxyanilino)-7-(2-hydroxyethyl)-2-(3-{[(3S)-4-methylmorpholin-3-yl]methoxy}pyridin-4-yl)-1,5,6,7-tetrahydro-4H-pyrrolo[3,2-c]pyridin-4-one, stereoisomer 2

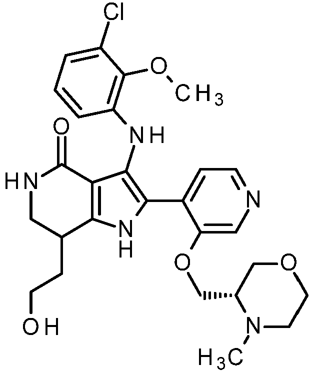

7,6 mg of the title compound (70% purity, see example 115) were isolated as second stereoisomer from the preparation of example 115.

LC-MS (method 2): Rₜ = 0.94 min; MS (ESlpos): m/z = 442.3 [M+H]⁺

¹H-NMR (400 MHz, DMSO-d₆) δ [ppm]: 1.68 - 1.78 (m, 2H), 2.25 - 2.38 (m, 4H), 2.98 - 3.10 (m, 1H), 3.08 (d, 1H), 3.18 - 3.30 (m, 1H), 3.40 - 3.64 (m, 5H), 3.69 - 3.87 (m, 2H), 3.90 (s, 3H), 4.31- 4.41 (m, 2H), 4.85 (t, 1H), 6.05 - 6.23 (m, 1H), 6.58 - 6.75 (m, 2H), 7.10 - 7.19 (m, 1H), 7.29 (d, 1H), 7.71 (s, 1H), 7.99 (d, 1H), 8.38 (s, 1H), 11.55 (s, 1H).

### Example 117

### 3-(3-chloro-2-methyl-anilino)-7-methyl-2-[3-(tetrahydropyran-2-ylmethoxy)-4-pyridyl]-1,5,6,7-tetrahydropyrrolo[3,2-c]pyridin-4-one

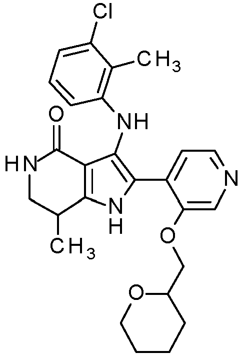

According to the method described for example 52 using *N*-(3-chloro-2-methyl-phenyl)-3-methyl-6-oxo-4-{[3-(tetrahydropyran-2-ylmethoxy)-4-pyridyl]methylamino}-2,3-dihydro-1*H*-pyridine-5-carbothioamide (intermediate 6-117, 145 mg, 282 µmol) as starting material, 51 mg (70% purity, 26% yield) of the title compound were prepared after purification by preparative HPLC (method 10, gradient: 0.00-0.50 min 15% B, 0.50-6.00 min 15-55% B).

LC-MS (method 2): Rₜ = 1.21 min; MS (ESlpos): m/z = 481 [M+H]⁺

### Example 118

### 3-(3-chloro-2-methyl-anilino)-7-methyl-2-[3-(tetrahydropyran-2-ylmethoxy)-4-pyridyl]-1,5,6,7-tetrahydropyrrolo[3,2-c]pyridin-4-one (stereoisomer 1 + 2)

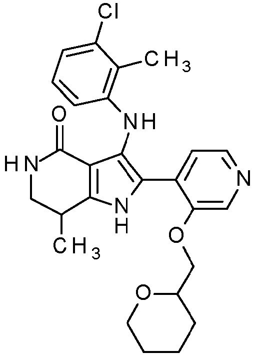

The mixture of stereoisomers of example 117 (50 mg) was separated into single streoisomers by preparative chiral HPLC to give 10.0 mg (95% purity, 27% yield) of the title compound (mixture of stereoisomers 1 + 2, Rₜ = 7.7 - 9.0 min) and mixture of stereoisomers 3 + 4 (10.0 mg, Rₜ = 9.3 - 10.6 min, (additional separation as example 119).

### Preparative chiral HPLC method: POB

Instrument: PrepCon Labomatic HPLC; Column: YMC Cellulose SB 10µ, 250x50; eluent A: acetonitrile; eluent B: ethanol; isocratic: 90%A+10%B; flow: 100 mL/min; temperature: 25°C; UV: 254 nm

### Analytical chiral HPLC method: POB

Instrument: Waters Alliance 2695; Column: YMC Cellulose SB 3µ, 100x4.6; eluent A: acetonitrile ; eluent B: ethanol ; isocratic: 90%A+10%B; flow: 1.4 ml/min; temperature: 25°C; UV: 254 nm
Analytical chiral HPLC: Rₜ = 1.97 min.
LC-MS (method 6): Rₜ = 0.86 min; MS (ESlpos): m/z = 481 [M+H]⁺
¹H-NMR (400 MHz, DMSO-d₆) δ [ppm]: 0.698 (3.09), 0.740 (2.13), 0.833 (2.30), 0.852 (2.64), 1.112 (4.99), 1.151 (5.93), 1.233 (14.22), 1.244 (10.15), 1.267 (6.07), 1.277 (5.77), 1.293 (3.75), 1.333 (2.31), 1.375 (2.13), 1.530 (2.80), 1.615 (1.33), 1.645 (1.14), 1.669 (0.89), 1.824 (1.04), 2.114 (0.76), 2.142 (3.95), 2.177 (1.08), 2.200 (0.71), 2.287 (2.40), 2.293 (1.09), 2.350 (7.93), 2.539 (16.00), 2.787 (1.29), 2.994 (1.09), 3.087 (1.48), 3.112 (1.05), 3.506 (1.19), 3.772 (0.62), 3.867 (0.59), 4.019 (0.81), 4.045 (0.62), 4.066 (0.53), 4.116 (0.79), 4.133 (0.62), 4.161 (0.61), 4.169 (0.60), 4.247 (0.58), 4.273 (0.46), 5.759 (10.66), 6.177 (1.39), 6.198 (1.37), 6.717 (0.87), 6.735 (2.25), 6.748 (1.68), 6.767 (1.66), 6.788 (0.62), 7.171 (0.98), 7.193 (1.07), 7.228 (1.04), 7.356 (1.62), 7.394 (1.72), 7.995 (0.61), 8.387 (0.65), 10.880 (0.97), 10.913 (1.00).

### Example 119

### 3-(3-chloro-2-methyl-anilino)-7-methyl-2-[3-(tetrahydropyran-2-ylmethoxy)-4-pyridyl]-1,5,6,7-tetrahydropyrrolo[3,2-c]pyridin-4-one (stereoisomer 3)

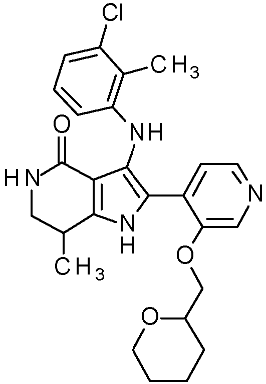

The isolated mixture of stereoisomers 3 +4 of example 118 (10 mg, Rₜ = 9.3 - 10.6 min) was separated into single stereoisomers by preparative chiral HPLC to give 1.2 mg (95 % purity, 3% yield) of the title compound (stereoisomer 3, Rₜ = 10.5 - 12.5 min) and stereoisomer 4 (Rₜ = 15.7 - 18.7 min).

### Preparative chiral HPLC method: SFC

Instrument: Sepiatec: Prep SFC100; Column: Chiralpak IA 5µ 250x30mm; eluent A: CO2; eluent B: methanol + 0.2 vol % aqueous ammonia (32%); isocratic: 30%B; flow: 100 ml/min; temperature: 40°C; BPR: 150bar; UV: 254 nm

### Analytical chiral HPLC method: SFC

Instrument: Agilent: 1260, Aurora SFC-Modul; Column: Chiralpak IA 5µ 100x4.6mm; eluent A: CO2; eluent B: methanol + 0.2 vol % aqueous ammonia (32%); isocratic: 30%B; flow: 4 ml/min; temperature: 37.5°C; BPR: 100bar; UV: 254 nm
Analytical chiral HPLC: Rₜ = 3.05 min.
Optical rotation:[α]_{D} = - 0.7 ° +/- 1.07 ° (c = 4.2 mg/ml in chloroform)
¹H-NMR (400 MHz, DMSO-d₆) δ [ppm]: 0.698 (8.70), 0.738 (3.97), 0.834 (2.06), 0.852 (2.78), 1.147 (9.18), 1.233 (14.60), 1.267 (7.55), 1.283 (7.35), 1.293 (5.02), 1.344 (1.79), 1.375 (1.89), 1.499 (1.77), 1.529 (3.00), 1.612 (1.42), 1.642 (1.24), 1.669 (1.44), 1.822 (1.06), 2.201 (0.65), 2.332 (2.43), 2.336 (1.55), 2.347 (16.00), 2.518 (13.06), 2.522 (8.36), 3.085 (1.84), 3.093 (1.63), 3.105 (1.30), 3.112 (1.32), 3.367 (0.42), 3.458 (0.67), 3.485 (1.47), 3.500 (1.38), 3.511 (1.36), 3.748 (0.75), 3.988 (0.94), 4.016 (0.91), 4.091 (0.85), 4.107 (1.01), 4.116 (1.66), 4.133 (1.43), 4.160 (1.52), 4.169 (1.56), 4.185 (0.81), 4.194 (0.70), 5.759 (3.43), 6.176 (1.86), 6.180 (1.81), 6.196 (1.95), 6.713 (1.12), 6.717 (1.38), 6.732 (3.34), 6.736 (2.71), 6.749 (2.33), 6.769 (2.49), 6.789 (0.84), 6.934 (0.57), 6.955 (0.64), 7.171 (2.18), 7.198 (0.74), 7.220 (0.91), 7.231 (0.95), 7.355 (4.30), 8.000 (0.49), 8.381 (0.47), 10.880 (2.40).

### Example 120

### (6R)-3-(3-chloro-2-methoxy-anilino)-2-[3-(3-methoxybutoxy)-4-pyridyl]-6-methyl-1,5,6,7-tetrahydropyrrolo[3,2-c]pyridin-4-one

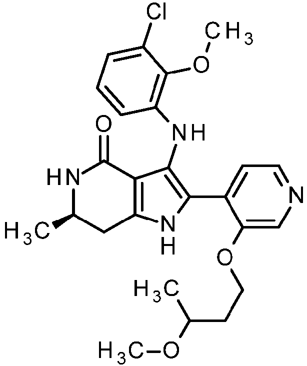

According to the method described for example 114 using (2*R*)-*N*-(3-chloro-2-methoxyphenyl)-4-{[3-(3-methoxybutoxy)-4-pyridyl]methylamino}-2-methyl-6-oxo-2,3-dihydro-1*H*-pyridine-5-carbothioamide (Intermediate 6-120, 243 mg, 468 µmol) as starting material, 66.0 mg (95% purity, 28% yield) of the title compound were prepared after purification by flash chromatography (silica, DCM / MeOH gradient 0-50 %).

LC-MS (method 2): Rₜ = 1.11 min; MS (ESlpos): m/z = 485 [M+H]⁺

¹H-NMR (400 MHz, DMSO-d₆) δ [ppm]: 1.105 (5.14), 1.121 (9.85), 1.137 (5.28), 1.221 (3.81), 1.227 (4.17), 1.237 (4.11), 1.243 (4.02), 1.900 (0.60), 1.916 (1.64), 1.923 (0.93), 1.933 (1.79), 1.948 (0.80), 2.518 (1.40), 2.523 (0.99), 2.541 (0.63), 2.546 (0.64), 2.567 (0.59), 2.573 (0.63), 2.582 (0.72), 2.586 (0.71), 2.608 (0.70), 2.613 (0.68), 2.869 (0.56), 2.881 (0.69), 2.889 (0.58), 2.902 (0.64), 2.909 (0.56), 2.922 (0.49), 2.929 (0.52), 2.942 (0.44), 3.208 (14.58), 3.217 (16.00), 3.443 (0.47), 3.459 (1.04), 3.474 (1.14), 3.488 (0.78), 3.761 (0.60), 3.774 (0.66), 3.786 (0.52), 3.855 (12.41), 3.860 (12.78), 4.189 (1.20), 4.206 (2.30), 4.213 (1.45), 4.225 (1.38), 4.241 (0.48), 6.106 (1.10), 6.110 (1.21), 6.119 (1.77), 6.122 (1.98), 6.130 (1.13), 6.134 (1.18), 6.632 (3.56), 6.639 (3.53), 6.642 (2.89), 6.644 (2.56), 6.650 (3.08), 7.061 (1.51), 7.071 (1.65), 7.283 (2.09), 7.287 (1.98), 7.295 (2.14), 7.300 (1.97), 7.442 (2.43), 7.474 (2.59), 8.023 (2.64), 8.031 (2.58), 8.036 (2.59), 8.043 (2.31), 8.361 (3.82), 8.363 (3.61), 11.189 (1.21), 11.214 (1.27).

### Example 121

### (6R)-3-(3-chloro-2-methoxy-anilino)-2-[3-(3-methoxybutoxy)-4-pyridyl]-6-methyl-1,5,6,7-tetrahydropyrrolo[3,2-c]pyridin-4-one (stereoisomer 1)

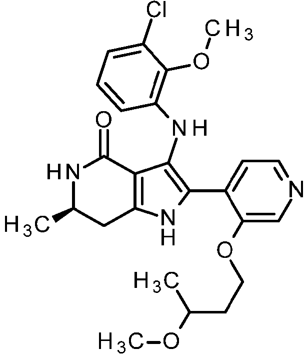

The title compound from example 120 (60 mg) was separated into enantiomers by preparative chiral HPLC to give 22.0 mg (95 % purity, 35 % yield) of the title compound (enantiomer 1, Rₜ = 6.0 - 7.1 min) and enantiomer 2 (22.0 mg, Rₜ = 8.6 - 10.3 min, see example 122).

### Preparative chiral HPLC method: MTBE

Instrument: Waters Alliance 2695; Column: YMC Cellulose SB 3µ, 100x4.6; eluent A: methyl tert-butyl ether + 0.1 vol % diethylamine; eluent B: acetonitrile; isocratic: 70%A+30%B; flow: 1.4 ml/min; temperature: 25°C; UV: 254 nm

### Analytical chiral HPLC method: MTBE

Instrument: Waters Alliance 2695; Column: YMC Cellulose SB 3µ, 100x4.6; eluent A: methyl tert-butyl ether + 0.1 vol % diethylamine; eluent B: acetonitrile; isocratic: 70%A+30%B; flow: 1.4 ml/min; temperature: 25°C; UV: 254 nm
Analytical chiral HPLC: Rₜ = 2.28 min.
Optical rotation:[α]_{D} = 3.66 ° +/- 2.69° (c = 1.3 mg/ml in DMSO)
LC-MS (method 1): Rₜ = 0.91 min; MS (ESlpos): m/z = 485 [M+H]⁺
¹H-NMR (400 MHz, DMSO-d₆) δ [ppm]: 0.831 (0.80), 0.836 (0.43), 0.853 (0.50), 1.105 (5.22), 1.121 (5.37), 1.221 (3.39), 1.237 (3.61), 1.900 (0.47), 1.915 (1.38), 1.931 (1.28), 1.947 (0.45), 2.518 (1.67), 2.523 (1.07), 2.541 (0.52), 2.567 (0.51), 2.581 (0.59), 2.606 (0.61), 2.888 (0.53), 2.901 (0.60), 2.928 (0.50), 2.941 (0.46), 3.208 (16.00), 3.458 (0.78), 3.473 (0.75), 3.854 (12.44), 4.188 (0.94), 4.205 (2.05), 4.221 (0.94), 6.105 (1.05), 6.117 (1.64), 6.129 (1.08), 6.632 (3.55), 6.642 (2.46), 6.644 (2.40), 7.060 (1.44), 7.287 (1.60), 7.299 (1.62), 7.441 (2.57), 8.030 (1.36), 8.043 (1.27), 8.363 (2.00), 11.188 (1.24).

### Example 122

### (6R)-3-(3-chloro-2-methoxy-anilino)-2-[3-(3-methoxybutoxy)-4-pyridyl]-6-methyl-1,5,6,7-tetrahydropyrrolo[3,2-c]pyridin-4-one (stereoisomer 2)

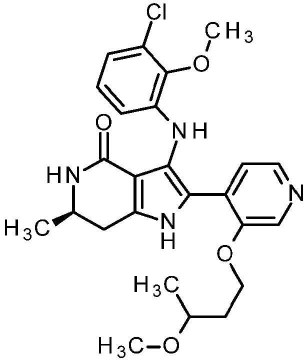

For the preparation of the title compound as mixture of stereoisomers see example 120. Separation of enantiomers by preparative chiral HPLC (method see example 121) to give 22.0 mg (95% purity, 35% yield) of the title compound (enantiomer 2, Rₜ = 8.6 - 10.3 min).
Analytical chiral HPLC (method see example 81): Rₜ = 3.28 min.
Optical rotation:[α]_{D} = 27.86 ° +/- 1.83° (c = 1.2 mg/ml in DMSO)
LC-MS (method 1): Rₜ = 0.91 min; MS (ESlneg): m/z = 483 [M-H]⁻
¹H-NMR (400 MHz, DMSO-d₆) δ [ppm]: 1.121 (5.75), 1.137 (5.82), 1.227 (3.86), 1.242 (3.88), 1.911 (0.66), 1.923 (0.68), 1.933 (0.74), 1.951 (0.68), 2.518 (0.75), 2.522 (0.46), 2.546 (0.57), 2.573 (0.58), 2.587 (0.68), 2.613 (0.71), 2.869 (0.62), 2.881 (0.70), 2.909 (0.56), 2.921 (0.52), 3.216 (16.00), 3.475 (0.52), 3.477 (0.54), 3.489 (0.51), 3.492 (0.49), 3.775 (0.46), 3.860 (13.63), 4.197 (0.52), 4.213 (1.27), 4.226 (1.25), 4.241 (0.53), 6.110 (1.20), 6.122 (2.01), 6.134 (1.21), 6.639 (3.40), 6.650 (3.43), 7.072 (1.78), 7.284 (1.48), 7.297 (1.50), 7.475 (2.96), 8.024 (0.93), 8.036 (0.89), 8.361 (1.43), 11.214 (1.46).

### Example 123

### 3-(3-chloro-2-methoxy-anilino)-2-[3-(1,4-dioxan-2-ylmethoxy)-4-pyridyl]spiro[5,7-dihydro-1H-pyrrolo[3,2-c]pyridine-6, 1'-cyclobutane]-4-one

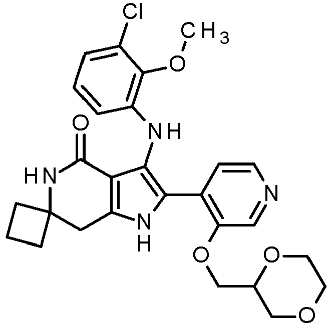

According to the method described for example 114 using N-(3-chloro-2-methoxyphenyl)-8-{[3-(1,4-dioxan-2-ylmethoxy)-4-pyridyl]methylamino}-6-oxo-5-azaspiro[3.5]non-7-ene-7-carbothioamide (Intermediate 6-123, 634 mg, 90% purity, 1.02 mmol) as starting material, 113.0 mg (95% purity, 21% yield) of the title compound were prepared after purification by preparative HPLC (method 10, gradient: 0.00-0.50 min 15% B, 0.50-6.00 min 15-55% B).

LC-MS (method 2): Rₜ = 1.11 min; MS (ESlpos): m/z = 525 [M+H]⁺

¹H-NMR (400 MHz, DMSO-d₆) δ [ppm]: 1.730 (0.64), 1.744 (1.16), 1.760 (0.75), 1.767 (0.72), 2.052 (1.02), 2.066 (1.12), 2.074 (2.07), 2.119 (0.88), 2.141 (0.99), 2.164 (0.54), 2.518 (2.19), 2.522 (1.36), 2.539 (9.99), 3.016 (4.03), 3.401 (1.02), 3.425 (1.24), 3.429 (1.25), 3.454 (1.17), 3.488 (0.47), 3.511 (0.87), 3.518 (0.88), 3.542 (0.44), 3.547 (0.57), 3.715 (1.55), 3.741 (2.57), 3.765 (0.99), 3.771 (0.51), 3.805 (1.00), 3.812 (1.10), 3.834 (0.90), 3.840 (0.96), 3.869 (16.00), 3.881 (1.25), 3.908 (0.64), 3.915 (0.68), 4.018 (0.57), 4.027 (0.60), 4.034 (0.63), 4.043 (0.54), 4.049 (0.45), 4.131 (0.93), 4.147 (0.72), 4.157 (1.25), 4.173 (1.00), 4.254 (1.16), 4.263 (1.13), 4.280 (0.82), 4.290 (0.73), 6.114 (1.47), 6.123 (1.29), 6.128 (1.48), 6.138 (1.49), 6.656 (0.56), 6.666 (6.14), 6.676 (2.92), 6.680 (2.66), 7.267 (2.52), 7.280 (2.57), 7.468 (3.76), 7.558 (2.81), 8.031 (3.13), 8.044 (2.90), 8.399 (4.51), 11.098 (1.87).

### Example 124

### 3-(3-chloro-2-methoxy-anilino)-2-[3-(1,4-dioxan-2-ylmethoxy)-4-pyridyl]spiro[5,7-dihydro-1H-pyrrolo[3,2-c]pyridine-6,1'-cyclobutane]-4-one (stereoisomer 1)

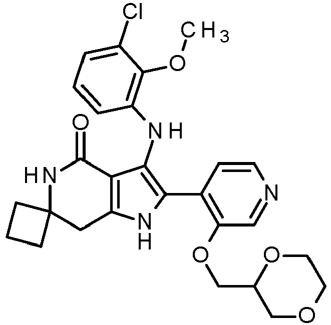

The title compound from example 123 (113.0 mg) was separated into enantiomers by preparative chiral HPLC to give 67.0 mg (99% purity, 59% yield) of the title compound (enantiomer 1, Rₜ = 13.6 - 16.4 min) and enantiomer 2 (31.0 mg, Rₜ = 20.9 - 25.2 min, see example 125).

### Preparative chiral HPLC method:

Instrument: PrepCon Labomatic HPLC-3; Column: YMC Cellulose SB 5µ, 250x30; eluent A: methyl tert-butyl ether + 0.1 vol % diethylamine; eluent B: ethanol; isocratic: 90%A + 10%B; flow: 60 ml/min; temperature: 25°C; UV: 280 nm

### Analytical chiral HPLC method:

Instrument: Thermo Fisher UltiMate 3000; Column: YMC Cellulose SB 3µ, 100x4.6; eluent A: methyl *tert*-butyl ether + 0.1 vol % diethylamine; eluent B: ethanol; isocratic: 90% A + 10% B; flow: 1.4 ml/min; temperature: 25°C; UV: 280 nm
Analytical chiral HPLC: Rₜ = 4.26 min.
Optical rotation:[α]_{D} = - 9.35 ° +/- 0.85° (c = 5.8 mg/ml in chloroform)
¹H-NMR (400 MHz, DMSO-d₆) δ [ppm]: 1.730 (0.65), 1.744 (1.13), 1.759 (0.76), 2.052 (1.01), 2.066 (1.10), 2.118 (0.87), 2.141 (0.99), 2.169 (0.56), 2.518 (1.92), 2.522 (1.30), 3.016 (4.00), 3.400 (0.98), 3.426 (1.19), 3.429 (1.20), 3.454 (1.15), 3.488 (0.48), 3.511 (0.83), 3.518 (0.86), 3.547 (0.55), 3.715 (1.49), 3.742 (2.49), 3.765 (0.66), 3.771 (0.47), 3.806 (0.92), 3.812 (1.03), 3.834 (0.87), 3.841 (0.88), 3.869 (16.00), 3.882 (1.24), 3.908 (0.65), 3.915 (0.71), 4.018 (0.56), 4.027 (0.61), 4.034 (0.60), 4.043 (0.53), 4.049 (0.45), 4.131 (0.89), 4.147 (0.70), 4.157 (1.21), 4.173 (0.99), 4.254 (1.10), 4.264 (1.09), 4.281 (0.80), 4.290 (0.73), 6.114 (1.36), 6.124 (1.27), 6.128 (1.42), 6.138 (1.41), 6.656 (0.48), 6.666 (6.00), 6.676 (2.88), 6.681 (2.61), 7.268 (1.98), 7.280 (2.01), 7.470 (3.54), 7.561 (2.69), 8.033 (1.39), 8.045 (1.33), 8.400 (2.24), 11.099 (1.85).

### Example 125

### 3-(3-chloro-2-methoxy-anilino)-2-[3-(1,4-dioxan-2-ylmethoxy)-4-pyridyl]spiro[5,7-dihydro-1H-pyrrolo[3,2-c]pyridine-6,1'-cyclobutane]-4-one (stereoisomer 2)

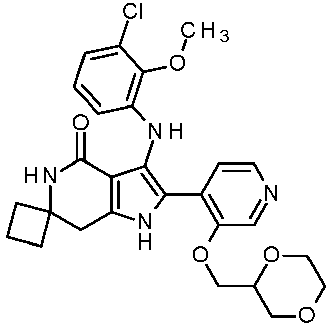

For the preparation of the title compound as mixture of stereoisomers see example 123. Separation of enantiomers by preparative chiral HPLC (method see example 124) to give 31.0 mg (99% purity, 27% yield) of the title compound (enantiomer 2, Rₜ = 20.9 - 25.2 min).
Analytical chiral HPLC (method see example 124): Rₜ = 6.54 min.
Optical rotation:[α]_{D} = 5.59 ° +/- 2.37° (c = 4.4 mg/ml in chloroform)
¹H-NMR (400 MHz, DMSO-d₆) δ [ppm]: 0.798 (0.56), 0.815 (0.54), 0.821 (0.56), 0.904 (0.52), 1.137 (1.09), 1.170 (0.61), 1.232 (0.58), 1.745 (1.16), 2.052 (1.02), 2.066 (1.11), 2.116 (1.11), 2.141 (1.02), 2.169 (0.60), 2.518 (2.98), 2.523 (2.06), 3.016 (4.11), 3.400 (1.00), 3.426 (1.23), 3.429 (1.23), 3.454 (1.16), 3.488 (0.50), 3.511 (0.85), 3.518 (0.92), 3.547 (0.57), 3.715 (1.55), 3.742 (2.55), 3.765 (0.66), 3.772 (0.47), 3.806 (0.96), 3.813 (1.07), 3.834 (0.90), 3.841 (0.89), 3.869 (16.00), 3.882 (1.29), 3.915 (0.72), 4.018 (0.56), 4.027 (0.63), 4.034 (0.60), 4.042 (0.58), 4.131 (0.93), 4.147 (0.74), 4.157 (1.24), 4.173 (0.99), 4.254 (1.14), 4.264 (1.16), 4.281 (0.83), 4.290 (0.75), 6.114 (1.39), 6.124 (1.26), 6.128 (1.44), 6.138 (1.42), 6.656 (0.53), 6.666 (5.93), 6.676 (2.83), 6.681 (2.60), 7.268 (2.09), 7.280 (2.14), 7.469 (3.66), 7.560 (2.75), 8.032 (1.67), 8.045 (1.61), 8.400 (2.70), 11.099 (1.86).

### Example 126

### 3'-(3-chloro-2-methoxyanilino)-2'-(3-{[(2S)-oxolan-2-yl]methoxy}pyridin-4-yl)-1',7'-dihydrospiro[cyclobutane-1,6'-pyrrolo[3,2-c]pyridin]-4'(5'H)-one

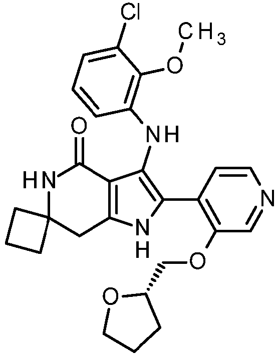

According to the method described for example 114 using *N*-(3-chloro-2-methoxyphenyl)-6-oxo-8-{[(3-{[(2*S*)-oxolan-2-yl]methoxy}pyridin-4-yl)methyl]amino}-5-azaspiro[3.5]non-7-ene-7-carbothioamide (Intermediate 6-126, 245 mg, 451 µmol) as starting material, 113 mg (95 % purity, 47% yield) of the title compound were prepared after purification by preparative HPLC (method 10, gradient: 0.00-0.50 min 15% B, 0.50-6.00 min 15-55% B).
Optical rotation:[α]_{D} = - 21.33 ° +/- 0.57° (c = 5.9 mg/ml in chloroform)
LC-MS (method 2): Rₜ = 1.21 min; MS (ESlpos): m/z = 509 [M+H]⁺
¹H-NMR (400 MHz, DMSO-d₆) δ [ppm]: 1.672 (0.42), 1.690 (0.51), 1.700 (0.59), 1.707 (0.59), 1.720 (1.01), 1.742 (1.16), 1.757 (0.77), 1.765 (0.69), 1.862 (1.19), 1.879 (1.87), 1.896 (1.18), 1.914 (0.51), 1.994 (0.48), 2.014 (0.72), 2.026 (0.97), 2.042 (1.24), 2.058 (1.10), 2.074 (0.58), 2.114 (0.71), 2.144 (0.94), 2.168 (0.52), 2.518 (1.75), 2.523 (1.14), 3.003 (4.33), 3.669 (0.63), 3.764 (0.43), 3.780 (0.88), 3.783 (0.90), 3.800 (1.43), 3.817 (0.70), 3.826 (0.76), 3.842 (1.64), 3.864 (16.00), 3.879 (0.48), 4.025 (0.50), 4.046 (1.08), 4.053 (1.06), 4.072 (0.63), 4.314 (0.51), 4.323 (0.98), 4.334 (1.93), 4.353 (1.29), 4.361 (0.78), 5.758 (4.68), 6.130 (1.42), 6.138 (1.21), 6.146 (1.44), 6.154 (1.43), 6.650 (0.63), 6.662 (6.02), 6.670 (2.79), 6.678 (2.31), 7.263 (2.53), 7.276 (2.55), 7.437 (3.53), 7.544 (2.66), 8.024 (3.40), 8.036 (3.02), 8.425 (4.62), 11.288 (1.67).

### Example 127

### 3'-(3-chloro-2-methoxyanilino)-2'-(3-{[(2S)-oxan-2-yl]methoxy}pyridin-4-yl)-1',7'-dihydrospiro[cyclobutane-1,6'-pyrrolo[3,2-c]pyridin]-4'(5'H)-one

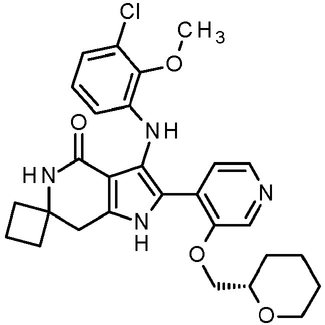

*N*-(3-chloro-2-methoxyphenyl)-8-{[(3-{[(2*S*)-oxan-2-yl]methoxy}pyridin-4-yl)methyl]amino}-6-oxo-5-azaspiro[3.5]non-7-ene-7-carbothioamide (Intermediate 6-127, 350 mg, 628 µmol) was dissolved in acetic acid (4 mL). Hydrogen peroxide (110 µl, 35 % purity, 1.3 mmol) was added and the mixture stirred for 70 min at 80°C. The mixture was quenched with sat. sodium thiosulfate solution and adjusted to pH 7-8 by addition of aq. sodium hydroxide solution (4 M, 17.5 mL). The mixture was stirred for 20 min with dichloromethane and water, was extracted two times and the combined organic layer was filtrated over a water repellent filter and concentrated under reduced pressure. The residue was triturated with a small amount of EtOH and the formed precipitate was filtered off and dried under reduced pressure to give 245 mg (90% purity, 67% yield) of the title compound.
Optical rotation:[α]_{D} = - 25.96 ° +/- 1.88° (c = 3.5 mg/ml in chloroform)
LC-MS (method 2): Rₜ = 1.29 min; MS (ESlpos): m/z = 523 [M+H]⁺
¹H-NMR (400 MHz, DMSO-d₆) δ [ppm]: 1.052 (0.78), 1.370 (0.46), 1.499 (0.43), 1.523 (0.77), 1.550 (1.08), 1.572 (1.03), 1.604 (0.55), 1.626 (0.68), 1.656 (0.57), 1.742 (1.18), 1.757 (0.85), 1.765 (0.78), 1.781 (0.44), 1.828 (0.56), 1.907 (0.63), 2.054 (1.05), 2.067 (1.13), 2.098 (0.47), 2.120 (0.87), 2.138 (0.94), 2.163 (0.56), 2.518 (1.83), 2.523 (1.37), 2.539 (1.11), 3.018 (4.48), 3.533 (0.46), 3.561 (0.84), 3.820 (0.56), 3.834 (0.44), 3.841 (0.46), 3.883 (16.00), 4.070 (1.44), 4.089 (1.05), 4.096 (1.58), 4.115 (1.05), 4.316 (0.99), 4.323 (1.04), 4.341 (0.89), 4.349 (0.83), 6.122 (1.45), 6.135 (2.44), 6.147 (1.50), 6.650 (0.43), 6.684 (4.90), 6.695 (3.42), 6.697 (3.36), 7.267 (2.57), 7.280 (2.58), 7.526 (3.35), 7.588 (2.71), 8.012 (3.29), 8.025 (3.10), 8.416 (4.66), 11.105 (1.85).

### Example 128

### 3-(3-chloro-2-methoxy-anilino)-2-[3-(2-tetrahydropyran-2-ylethoxy)-4-pyridyl]spiro[5,7-dihydro-1H-pyrrolo[3,2-c]pyridine-6,1'-cyclobutane]-4-one

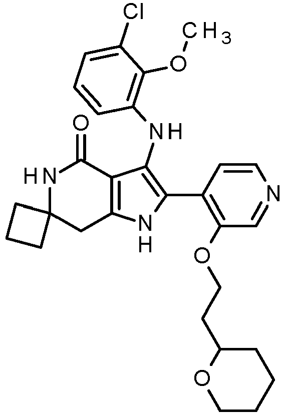

N-(3-chloro-2-methoxy-phenyl)-6-oxo-8-{[3-(2-tetrahydropyran-2-ylethoxy)-4-pyridyl]methyl amino}-5-azaspiro[3.5]non-7-ene-7-carbothioamide (Intermediate 6-128, 484 mg, 847 µmol) was dissolved in acetic acid (6 ml). Hydrogen peroxide (148 µl, 35%, 1.695 mmol) was added and the mixture stirred for 2 h at 60°C. The mixture was quenched with sat. sodiumthiosulfate solution and adjusted to pH 7-8 by addition of aqueos sodium hydroxide solution (4 M, 26 ml). The mixture was stirred for 20 min with dichloromethane and water, extracted two times and the combined organic layer was filtrated over a water repellent filter and concentrated under reduced pressure. The residue was purified by preparative HPLC (method 10, 0.00-0.50 min 30% B, 0.50-7.00 min 30-70% B) to give 358 mg (80% purity, 63% yield) of the title compound.

LC-MS (method 2): Rₜ = 1.30 min; MS (ESlpos): m/z = 537 [M+H]⁺

¹H-NMR (400 MHz, DMSO-d₆) δ [ppm]: 1.172 (0.61), 1.256 (0.60), 1.457 (1.68), 1.565 (0.77), 1.597 (0.67), 1.702 (0.75), 1.722 (1.33), 1.743 (1.58), 1.765 (1.25), 1.891 (0.73), 1.906 (1.84), 1.921 (2.00), 1.935 (0.77), 1.988 (1.24), 2.039 (1.40), 2.048 (1.14), 2.059 (0.95), 2.074 (2.52), 2.102 (0.51), 2.125 (0.97), 2.144 (1.08), 2.166 (0.61), 2.327 (0.51), 2.669 (0.51), 3.014 (4.64), 3.075 (0.56), 3.308 (0.77), 3.421 (0.60), 3.434 (0.58), 3.449 (0.58), 3.583 (2.34), 3.746 (0.40), 3.755 (0.43), 3.763 (0.61), 3.859 (16.00), 3.880 (0.81), 4.231 (1.39), 4.246 (2.78), 4.260 (1.34), 6.085 (1.36), 6.097 (1.87), 6.109 (1.40), 6.640 (3.62), 6.650 (4.89), 7.237 (0.41), 7.258 (0.46), 7.293 (1.20), 7.306 (1.27), 7.430 (3.71), 7.526 (2.90), 7.608 (0.51), 7.629 (0.41), 8.016 (1.13), 8.028 (1.11), 8.351 (1.86), 11.271 (1.98).

### Example 129

### 3'-(3-chloro-2-methoxyanilino)-2'-{3-[(5,5-dimethyl-1,4-dioxan-2-yl)methoxy]pyridin-4-yl}-1',7'-dihydrospiro[cyclobutane-1,6'-pyrrolo[3,2-c]pyridin]-4'(5'H)-one

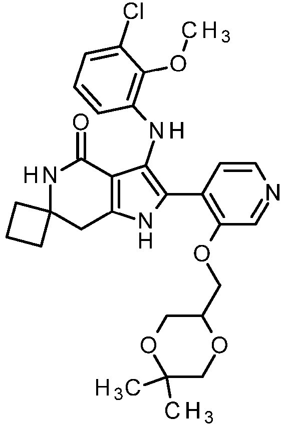

According to the method described for example 127 using N-(3-chloro-2-methoxyphenyl)-8-[({3-[(5,5-dimethyl-1,4-dioxan-2-yl]methoxy}pyridin-4-yl)methyl]amino}-6-oxo-5-azaspiro[3.5]non-7-ene-7-carbothioamide (intermediate 6-129, 476 mg, 811 µmol) as starting material, 260 mg (98% purity, 57% yield) of the title compound were prepared after purification by preparative HPLC (basic conditions).

LC-MS (method 2): Rₜ = 0.95 min; MS (ESlpos): m/z = 553 [M+H]⁺

¹H-NMR (400 MHz, DMSO-d6) δ [ppm]: 1.072 (9.72), 1.239 (8.80), 1.721 (0.58), 1.742 (0.98), 1.758 (0.78), 2.047 (0.94), 2.060 (0.95), 2.121 (0.72), 2.135 (0.84), 2.150 (0.64), 2.157 (0.51), 2.327 (0.44), 2.518 (1.83), 2.523 (1.16), 2.669 (0.45), 2.997 (3.45), 3.389 (1.14), 3.418 (1.39), 3.575 (0.60), 3.583 (0.69), 3.604 (1.12), 3.612 (1.10), 3.632 (1.85), 3.660 (1.65), 3.666 (1.22), 3.691 (1.32), 3.721 (0.82), 3.860 (14.82), 3.878 (0.55), 3.886 (0.53), 3.893 (0.59), 3.903 (0.52), 4.183 (0.59), 4.198 (0.59), 4.209 (1.10), 4.225 (1.01), 4.259 (1.07), 4.268 (1.13), 4.285 (0.62), 4.294 (0.54), 5.758 (16.00), 6.121 (1.32), 6.130 (1.19), 6.134 (1.34), 6.145 (1.36), 6.646 (0.46), 6.656 (5.73), 6.665 (2.76), 6.670 (2.42), 7.266 (2.47), 7.279 (2.48), 7.448 (3.42), 7.544 (2.59), 8.035 (3.24), 8.048 (2.97), 8.411 (4.37), 11.084 (1.78).

### Example 130

### 3'-(3-chloro-2-methoxyanilino)-2'-{3-[(5,5-dimethyl-1,4-dioxan-2-yl)methoxy]pyridin-4-yl}-1',7'-dihydrospiro[cyclobutane-1,6'-pyrrolo[3,2-c]pyridin]-4'(5'H)-one (stereoisomer 1)

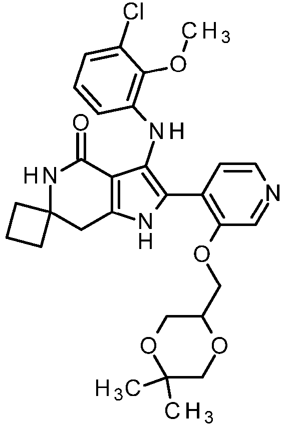

The title compound from example 129 (260 mg) was separated into enantiomers by preparative chiral HPLC to give 98 mg (98% purity of the title compound (enantiomer 1, Rₜ = 2.02 min) and enantiomer 2 (95 mg, Rₜ = 2.41 min, see example 131).

### Preparative chiral HPLC method:

Instrument: PrepCon Labomatic HPLC-3; Column: YMC Cellulose SB 10µ, 250x50; eluent A: hexane + 0.1 vol% diethylamine; eluent B: ethanol + 0.1 vol% diethylamine; isocratic: 50%A+50%B; flow: 100 mL/min; temperature: 25°C; UV: 280 nm

### Analytical chiral HPLC method:

Instrument: Thermo Fisher UltiMate 3000; Column: YMC Cellulose SB 3µ, 100x4.6; eluent A: hexane + 0.1 vol % diethylamine; eluent B: ethanol; isocratic: 50%A+50%B; flow: 1.4 ml/min; temperature: 25°C; UV: 280 nm
Analytical chiral HPLC: Rₜ = 2.02 min.
Optical rotation:[α]_{D} = 2.63° +/- 0.87° (c = 6.5 mg/ml in chloroform)
¹H-NMR (400 MHz, DMSO-d6) δ [ppm]: 1.071 (10.65), 1.239 (9.88), 1.722 (0.64), 1.742 (1.09), 1.757 (0.88), 1.765 (0.75), 2.032 (0.63), 2.048 (1.06), 2.060 (1.09), 2.074 (3.95), 2.084 (1.54), 2.097 (0.41), 2.121 (0.81), 2.134 (0.95), 2.150 (0.72), 2.159 (0.58), 2.522 (0.93), 2.997 (3.86), 3.389 (1.26), 3.418 (1.55), 3.575 (0.67), 3.583 (0.78), 3.604 (1.24), 3.612 (1.22), 3.632 (2.07), 3.660 (1.82), 3.665 (1.37), 3.691 (1.46), 3.721 (0.89), 3.860 (16.00), 3.879 (0.67), 3.886 (0.60), 3.893 (0.68), 3.903 (0.60), 3.910 (0.43), 3.919 (0.43), 4.183 (0.67), 4.198 (0.65), 4.209 (1.22), 4.225 (1.13), 4.258 (1.18), 4.268 (1.26), 4.285 (0.69), 4.294 (0.62), 6.121 (1.46), 6.131 (1.32), 6.135 (1.42), 6.145 (1.52), 6.645 (0.50), 6.656 (5.95), 6.665 (3.00), 6.670 (2.73), 7.267 (2.35), 7.280 (2.37), 7.449 (3.83), 7.544 (2.89), 8.035 (2.09), 8.048 (1.98), 8.411 (3.22), 11.084 (2.00).

### Example 131

### 3'-(3-chloro-2-methoxyanilino)-2'-{3-[(5,5-dimethyl-1,4-dioxan-2-yl)methoxy]pyridin-4-yl}-1',7'-dihydrospiro[cyclobutane-1,6'-pyrrolo[3,2-c]pyridin]-4'(5'H)-one (stereoisomer 2)

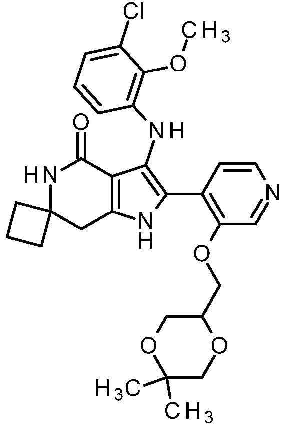

For the preparation of the title compound as mixture of stereoisomers see example 129. Separation of enantiomers by preparative chiral HPLC (method see example 130) to give 95.0 mg (98% purity, 36% yield) of the title compound (enantiomer 2, Rₜ = 2.41 min).
Analytical chiral HPLC (method see example 130): Rₜ = 2.41 min.
Optical rotation:[α]_{D} = -7.08° +/- 0.35° (c = 4.4 mg/ml in chloroform)
¹H-NMR (400 MHz, DMSO-d6) δ [ppm]: 1.071 (11.05), 1.239 (10.25), 1.721 (0.72), 1.743 (1.22), 1.757 (0.99), 1.780 (0.41), 2.048 (1.18), 2.060 (1.19), 2.097 (0.46), 2.121 (0.91), 2.135 (1.05), 2.150 (0.81), 2.157 (0.67), 2.997 (4.14), 3.389 (1.38), 3.417 (1.66), 3.575 (0.72), 3.583 (0.80), 3.605 (1.31), 3.613 (1.32), 3.632 (2.15), 3.660 (1.91), 3.666 (1.47), 3.692 (1.53), 3.721 (0.91), 3.860 (16.00), 3.879 (0.78), 3.886 (0.70), 3.893 (0.77), 3.904 (0.67), 3.910 (0.50), 3.919 (0.48), 4.183 (0.70), 4.198 (0.69), 4.210 (1.29), 4.224 (1.17), 4.258 (1.26), 4.268 (1.32), 4.285 (0.73), 4.294 (0.65), 6.121 (1.48), 6.131 (1.38), 6.135 (1.50), 6.145 (1.55), 6.645 (0.57), 6.656 (6.18), 6.666 (3.06), 6.670 (2.85), 7.267 (2.36), 7.279 (2.42), 7.449 (3.89), 7.546 (3.03), 8.035 (2.07), 8.048 (1.97), 8.411 (3.25), 11.085 (2.14).

### Example 132

### 3'-(3-chloro-2-methoxyanilino)-2'-{3-[(5,5-dimethyl-1,4-dioxan-2-yl)methoxy]pyridin-4-yl}-1',7'-dihydrospiro[cyclopropane-1,6'-pyrrolo[3,2-c]pyridin]-4'(5'H)-one

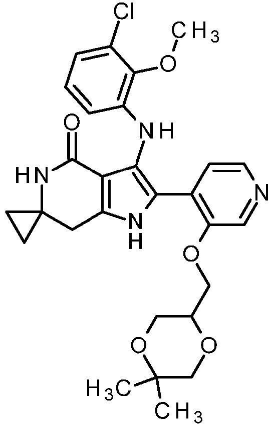

According to the method described for example 111 using N-(3-chloro-2-methoxyphenyl)-7-[({3-[(5,5-dimethyl-1,4-dioxan-2-yl)methoxy]pyridin-4-yl}methyl)amino]-5-oxo-4-azaspiro[2.5]oct-6-ene-6-carbothioamide (intermediate 6-132, 250 mg, 436 µmol) as starting material, 165 mg (90% purity, 63% yield) of the title compound were prepared after purification by flash column chromatography (eluent: DCM/ethanol 0-10%).

LC-MS (method 2): Rₜ = 0.87 min; MS (ESlpos): m/z = 539 [M+H]⁺

¹H-NMR (400 MHz, DMSO-d6) δ [ppm]: 0.696 (0.79), 0.708 (1.93), 0.718 (1.61), 0.736 (0.56), 0.753 (0.87), 0.764 (1.50), 0.774 (0.92), 0.785 (0.60), 0.798 (0.41), 0.977 (0.53), 1.039 (0.52), 1.060 (9.68), 1.071 (0.79), 1.149 (0.52), 1.154 (3.26), 1.172 (6.31), 1.190 (3.01), 1.223 (8.85), 1.988 (11.59), 2.518 (1.42), 2.523 (0.96), 2.774 (0.57), 2.815 (2.06), 2.838 (2.04), 2.879 (0.58), 3.025 (0.42), 3.027 (0.42), 3.372 (1.12), 3.401 (1.40), 3.572 (0.63), 3.580 (0.69), 3.603 (2.62), 3.609 (1.37), 3.632 (1.56), 3.646 (1.15), 3.653 (1.15), 3.672 (1.33), 3.701 (0.76), 3.857 (0.59), 3.870 (16.00), 3.886 (0.63), 3.895 (0.80), 3.903 (0.60), 3.911 (0.43), 3.919 (0.40), 4.000 (0.88), 4.017 (2.66), 4.035 (2.62), 4.053 (0.86), 4.159 (0.76), 4.175 (0.77), 4.185 (1.14), 4.201 (0.98), 4.274 (0.99), 4.283 (1.04), 4.300 (0.72), 4.309 (0.66), 6.160 (1.39), 6.171 (1.23), 6.174 (1.48), 6.185 (1.41), 6.658 (0.58), 6.668 (5.99), 6.678 (2.99), 6.682 (2.69), 7.253 (2.73), 7.278 (0.44), 7.283 (2.68), 7.295 (2.59), 7.508 (3.46), 8.038 (3.62), 8.050 (3.21), 8.407 (4.68), 11.060 (1.78).

### Example 133

### 3'-(3-chloro-2-methoxyanilino)-2'-(3-{[5,5-dimethyl-1,4-dioxan-2-yl]methoxy}pyridin-4-yl)-1',7'-dihydrospiro[cyclopropane-1,6'-pyrrolo[3,2-c]pyridin]-4'(5'H)-one (stereoisomer 1)

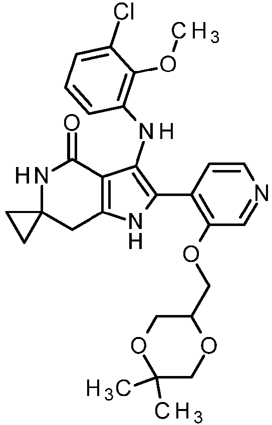

The title compound from example 132 (160 mg) was separated into enantiomers by preparative chiral HPLC to give 55.5 mg of the title compound (99% purity, 99% ee, enantiomer 1, Rₜ = 1.96 min) and enantiomer 2 (50.4 mg, Rₜ = 2.4 min, see example 134).

### Preparative chiral HPLC method:

Instrument: PrepCon Labomatic HPLC-3; Column: YMC Cellulose SB 10µ, 250x50; eluent A: hexane + 0.1 vol % diethylamine; eluent B: ethanol; isocratic: 50%A+50%B; flow: 100 mL/min; temperature: 25°C; UV: 220 nm.

### Analytical chiral HPLC method:

Instrument: Thermo Fisher UltiMate 3000; Column: YMC Cellulose SB 3µ, 100x4.6; eluent A: hexane + 0.1 vol % diethylamine; eluent B: ethanol; isocratic: 50%A+50%B; flow: 1.4 ml/min; temperature: 25°C; UV: 220 nm.
Analytical chiral HPLC: Rₜ = 1.99 min.
Optical rotation:[α]_{D} = -5.47° +/- 0.82° (c = 4.5 mg/ml in chloroform)
¹H-NMR (500 MHz, DMSO-d6) δ [ppm]: 0.697 (0.91), 0.708 (2.15), 0.717 (1.13), 0.733 (0.46), 0.752 (0.78), 0.761 (1.18), 0.775 (1.28), 0.784 (0.76), 0.795 (0.80), 0.800 (0.87), 0.813 (0.72), 0.821 (0.73), 0.837 (0.42), 0.905 (0.74), 1.034 (0.41), 1.060 (10.56), 1.084 (0.88), 1.222 (9.60), 1.259 (1.33), 2.514 (1.30), 2.518 (1.09), 2.522 (0.88), 2.780 (0.79), 2.813 (2.19), 2.838 (2.19), 2.872 (0.76), 3.374 (1.29), 3.397 (1.51), 3.577 (0.71), 3.583 (0.81), 3.600 (1.37), 3.606 (3.39), 3.628 (1.71), 3.650 (1.22), 3.671 (1.41), 3.695 (0.86), 3.857 (0.45), 3.870 (16.00), 3.879 (0.81), 3.887 (0.64), 3.895 (0.93), 3.901 (0.67), 3.908 (0.52), 3.915 (0.46), 4.163 (0.85), 4.175 (0.80), 4.184 (1.13), 4.197 (1.04), 4.276 (1.09), 4.284 (1.15), 4.298 (0.84), 4.305 (0.77), 6.162 (1.48), 6.171 (1.25), 6.174 (1.49), 6.182 (1.45), 6.660 (0.47), 6.669 (6.17), 6.676 (3.03), 6.680 (2.74), 7.252 (2.94), 7.284 (2.56), 7.294 (2.55), 7.507 (3.75), 8.039 (2.81), 8.049 (2.60), 8.407 (4.12), 10.850 (0.50), 11.060 (1.97).

### Example 134

### 3'-(3-chloro-2-methoxyanilino)-2'-(3-{[5,5-dimethyl-1,4-dioxan-2-yl]methoxy}pyridin-4-yl)-1',7'-dihydrospiro[cyclopropane-1,6'-pyrrolo[3,2-c]pyridin]-4'(5'H)-one (stereoisomer 2)

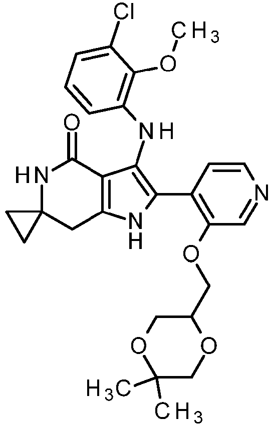

For the preparation of the title compound as mixture of stereoisomers see example 132. Separation of enantiomers by preparative chiral HPLC (method see example 133) to give 50.4 mg (99% purity, 98.8% ee) of the title compound (enantiomer 2).
Analytical chiral HPLC (method see example 133): Rₜ = 2.41 min.
Optical rotation:[α]_{D} = 4.86° +/- 0.24° (c = 4.9 mg/ml in chloroform)
¹H-NMR (500 MHz, DMSO-d6) δ [ppm]: 0.697 (0.92), 0.709 (2.20), 0.717 (1.14), 0.734 (0.46), 0.752 (0.77), 0.761 (1.23), 0.775 (1.28), 0.785 (0.79), 0.795 (0.80), 0.799 (0.57), 0.813 (0.40), 0.821 (0.51), 1.006 (0.40), 1.060 (10.65), 1.084 (0.80), 1.223 (9.80), 1.259 (1.35), 2.518 (0.66), 2.522 (0.52), 2.781 (0.78), 2.814 (2.23), 2.839 (2.24), 2.872 (0.78), 3.375 (1.31), 3.398 (1.52), 3.577 (0.72), 3.583 (0.80), 3.601 (1.39), 3.606 (3.41), 3.629 (1.70), 3.650 (1.19), 3.671 (1.43), 3.695 (0.82), 3.870 (16.00), 3.879 (0.73), 3.888 (0.62), 3.894 (0.70), 3.902 (0.66), 3.908 (0.49), 3.915 (0.47), 4.163 (0.83), 4.176 (0.80), 4.184 (1.14), 4.197 (1.03), 4.277 (1.10), 4.284 (1.14), 4.298 (0.82), 4.305 (0.76), 6.163 (1.42), 6.171 (1.24), 6.174 (1.41), 6.183 (1.39), 6.660 (0.48), 6.669 (5.90), 6.676 (2.83), 6.680 (2.60), 7.253 (2.95), 7.284 (2.51), 7.294 (2.53), 7.508 (3.72), 8.039 (2.68), 8.049 (2.48), 8.408 (4.01), 10.850 (0.48), 11.060 (2.00).

### EXPERIMENTAL SECTION - BIOLOGICAL ASSAYS

The pharmacological activity of the compounds according to the invention can be assessed using *in vitro-* and/or *in vivo*-assays, as known to the person skilled in the art. The following examples describe the biological activity of the compounds according to the invention, without the invention being limited to said examples.

Example compounds according to the invention were tested in selected biological assays one or more times. When tested more than once, data are reported as either average values or as median values, wherein
- the average value, also referred to as the arithmetic mean value, represents the sum of the values obtained divided by the number of times tested, and
- the median value represents the middle number of the group of values when ranked in ascending or descending order. If the number of values in the data set is odd, the median is the middle value. If the number of values in the data set is even, the median is the arithmetic mean of the two middle values.

Examples were synthesized one or more times. When synthesized more than once, data from biological assays represent average values or median values calculated utilizing data sets obtained from testing of one or more synthetic batch.

The *in vitro* activity of the compounds of the present invention can be demonstrated in the following assays:

### Expression and purification of the EGFR proteins used in the biochemical kinase assays

The different EGFR proteins used in the biochemical kinase activity inhibition assays were generated inhouse by expression in insect cells using Baculo Virus system and subsequent purification as described in the following paragraphs.

### Expression constructs:

The cDNAs encoding the various protein sequences from human EGFR human (P00533) were optimized for expression in eukaryotic cells and synthesized by the GeneArt Technology at Life Technologies.

These DNA sequences encoded the following sequence:
Construct EGFR #1 amino acid R669 to A1210
Construct EGFR #2 amino acid R669 to A1210 and the insertion of the amino acids sequence ASV between V769 and D770
Construct EGFR #3 amino acid R669 to A1210 and the insertion of the amino acids sequence SVD between D770 and N771
Additionally all constructs EGFR #1 to #3 encoded: at the N-terminus a TEV (Tobacco etch virus) protease cleavage site (DYDIPTTENLYFQG), at the C-terminus two stop codons and additionally 5' and 3' att-DNA sequences for Gateway Cloning.

Each of the four EFGR constructs was subcloned using the Gateway Technology into the Destination vector pD-Ins1. The vector pD-Ins1 is a Baculovirus transfer vector (based on vector pVL1393, Pharmingen) which provides a N-terminal fusion of a GST-tag to the integrated gene construct. The respective transfer vectors were termed pD-Ins1_ EGFR #1, pD-Ins1_ EGFR #2, pD-Ins1_ EGFR #3.

### EGFR amino acid sequences:

GST-EGFR #1 (Wild Type) 
MSPILGYWKIKGLVQPTRLLLEYLEEKYEEHLYERDEGDKWRNKKFELGLEFPNLPYYI
DGDVKLTQSMAIIRYIADKHNMLGGCPKERAEISMLEGAVLDIRYGVSRIAYSKDFETLK
VDFLSKLPEMLKMFEDRLCHKTYLNGDHVTHPDFMLYDALDVVLYMDPMCLDAFPKL
VCFKKRIEAIPQIDKYLKSSKYIAWPLQGWQATFGGGDHPPKSDPITSLYKKAGSDYDI
PTTTENLYFQGRRRHIVRKRTLRRLLQERELVEPLTPSGEAPNQALLRILKETEFKKIKV
LGSGAFGTVYKGLWIPEGEKVKIPVAIKELREATSPKANKEILDEAYVMASVDNPHVCR
LLGICLTSTVQLITQLMPFGCLLDYVREHKDNIGSQYLLNWCVQIAKGMNYLEDRRLVH
RDLAARNVLVKTPQHVKITDFGLAKLLGAEEKEYHAEGGKVPIKWMALESILHRIYTHQ
SDVWSYGVTVWELMTFGSKPYDGIPASEISSILEKGERLPQPPICTIDVYMIMVKCWMI
DADSRPKFRELIIEFSKMARDPQRYLVIQGDERMHLPSPTDSNFYRALMDEEDMDDVV
DADEYLIPQQGFFSSPSTSRTPLLSSLSATSNNSTVACIDRNGLQSCPIKEDSFLQRYS
SDPTGALTEDSIDDTFLPVPEYINQSVPKRPAGSVQNPVYHNQPLNPAPSRDPHYQDP
HSTAVGNPEYLNTVQPTCVNSTFDSPAHWAQKGSHQISLDNPDYQQDFFPKEAKPNG
IFKGSTAENAEYLRVAPQSSEFIGA
GST-EGFR #2 (ASV between V769 and D770) 
MSPILGYWKIKGLVQPTRLLLEYLEEKYEEHLYERDEGDKWRNKKFELGLEFPNLPYYI
DGDVKLTQSMAIIRYIADKHNMLGGCPKERAEISMLEGAVLDIRYGVSRIAYSKDFETLK
VDFLSKLPEMLKMFEDRLCHKTYLNGDHVTHPDFMLYDALDVVLYMDPMCLDAFPKL
 
VCFKKRIEAIPQIDKYLKSSKYIAWPLQGWQATFGGGDHPPKSDPITSLYKKAGSDYDI
PTTTENLYFQGRRRHIVRKRTLRRLLQERELVEPLTPSGEAPNQALLRILKETEFKKIKV
LGSGAFGTVYKGLWIPEGEKVKIPVAIKELREATSPKANKEILDEAYVMASVASVDNPH
VCRLLGICLTSTVQLITQLMPFGCLLDYVREHKDNIGSQYLLNWCVQIAKGMNYLEDRR
LVHRDLAARNVLVKTPQHVKITDFGLAKLLGAEEKEYHAEGGKVPIKWMALESILHRIYT
HQSDVWSYGVTVWELMTFGSKPYDGIPASEISSILEKGERLPQPPICTIDVYMIMVKCW
MIDADSRPKFRELIIEFSKMARDPQRYLVIQGDERMHLPSPTDSNFYRALMDEEDMDD
VVDADEYLIPQQGFFSSPSTSRTPLLSSLSATSNNSTVACIDRNGLQSCPIKEDSFLQR
YSSDPTGALTEDSIDDTFLPVPEYINQSVPKRPAGSVQNPVYHNQPLNPAPSRDPHYQ
DPHSTAVGNPEYLNTVQPTCVNSTFDSPAHWAQKGSHQISLDNPDYQQDFFPKEAKP
NGIFKGSTAENAEYLRVAPQSSEFIGA
GST-EGFR #3 (SVD between D770 and N771)

MSPILGYWKIKGLVQPTRLLLEYLEEKYEEHLYERDEGDKWRNKKFELGLEFPNLPYYI
DGDVKLTQSMAIIRYIADKHNMLGGCPKERAEISMLEGAVLDIRYGVSRIAYSKDFETLK
VDFLSKLPEMLKMFEDRLCHKTYLNGDHVTHPDFMLYDALDVVLYMDPMCLDAFPKL
VCFKKRIEAIPQIDKYLKSSKYIAWPLQGWQATFGGGDHPPKSDPITSLYKKAGSDYDI
PTTTENLYFQGRRRHIVRKRTLRRLLQERELVEPLTPSGEAPNQALLRILKETEFKKIKV
LGSGAFGTVYKGLWIPEGEKVKIPVAIKELREATSPKANKEILDEAYVMASVDSVDNPH
VCRLLGICLTSTVQLITQLMPFGCLLDYVREHKDNIGSQYLLNWCVQIAKGMNYLEDRR
LVHRDLAARNVLVKTPQHVKITDFGLAKLLGAEEKEYHAEGGKVPIKWMALESILHRIYT
HQSDVWSYGVTVWELMTFGSKPYDGIPASEISSILEKGERLPQPPICTIDVYMIMVKCW
MIDADSRPKFRELIIEFSKMARDPQRYLVIQGDERMHLPSPTDSNFYRALMDEEDMDD
VVDADEYLIPQQGFFSSPSTSRTPLLSSLSATSNNSTVACIDRNGLQSCPIKEDSFLQR
YSSDPTGALTEDSIDDTFLPVPEYINQSVPKRPAGSVQNPVYHNQPLNPAPSRDPHYQ
DPHSTAVGNPEYLNTVQPTCVNSTFDSPAHWAQKGSHQISLDNPDYQQDFFPKEAKP
NGIFKGSTAENAEYLRVAPQSSEFIGA

### Generation of recombinant Baculovirus:

In separate approaches each of the three transfer vectors was co-transfected in Sf9 cells with Baculovirus DNA (Flashbac Gold DNA, Oxford Expression Technologies) using Fugene HD (Roche). After 5 days the supernatant of the transfected cells containing the recombinant Baculovirus encoding the various EGFR proteins was used for further infection of Sf9 cells for virus amplification whereby the virus titer was monitored using qPCR.

### EGFR expression in Sf9 cells using bioreactor:

Sf9 cells cultured (Insect-xpress medium, Lonza, 27 °C) in a Wave-bioreactor with a disposable culture bag were infected at a cell density of 106 cells/ml with one of the recombinant baculovirus stocks at a multiplicity of infection of 1 and incubated for 48 h. Subsequently the cells were harvested by centrifugation and the cell pellet frozen at -80 °C.

### Purification of the GST-EGFR fusion proteins:

Purification of the GST-EGFR fusion proteins was achieved by affinity chromatography using Glutathion Sepharose 4B matrix (GE Healthcare Life Sciences).

The pelleted cells (from 4I cell culture) were resuspended in Lysis-Buffer (50 mM HEPES pH 7.4, 150 mM NaCl, 5 % Glycerol, 1 mM MgCl2, 1 mM MnCl2, 0.5 mM Na3VO4) and lysed by a freeze-thaw cycle followed by an incubation on ice for 60 min. The supernatant was centrifuged at 4000 x g for 30 min. at 4 °C. The supernatant was than incubated with Glutathion Sepharose 4B matrix (in a glass bottle rotating for 16 h, at 4 °C) for binding of the GST EGFR fusion protein, rinsed with Wash-Buffer and finally the bound protein was eluted using Elusion-Buffer (Lysis Buffer plus 25 mM Glutathione) and shock frozen with liquid nitrogen.

### WT-EGFR kinase assay

Inhibitory activity of compounds of the present invention against wild-type Epidermal Growth Factor Receptor (EGFR) was quantified employing the TR-FRET based EGFR assay as described in the following paragraphs.

Recombinant fusion protein of N-terminal Glutathion-S-Transferase (GST) and a fragment of human EGFR (amino acids R669 to A1210), expressed in Sf9 insect cells and purified via affinity chromatography using Glutathion Sepharose as described above, was used as a kinase. As substrate for the kinase reaction the biotinylated peptide biotin-Ahx-AEEEEYFELVAKKK (C-terminus in amide form) was used, which can be purchased e.g. form the company Biosynthan GmbH (Berlin-Buch, Germany).

For the assay 50 nl of a 100 fold concentrated solution of the test compound in DMSO was pipetted into either a black low volume 384 well microtiter plate or a black 1536 well microtiter plate (both Greiner Bio-One, Frickenhausen, Germany), 2 µl of a solution of EGFR in aqueous assay buffer [50 mM Hepes pH 7.0, 10 mM MgCl2, 1mM dithiothreitol, 0.5 mM EGTA, 0.3 mM activated sodium ortho-vanadate, 0.005 % (w/v) bovine serum albumin, 0.005% (v/v) Tween-20] were added and the mixture was incubated for 15 min at 22°C to allow pre binding of the test compounds to the enzyme before the start of the kinase reaction. Then the kinase reaction was started by the addition of 3 µL of a solution of adenosine tri phosphate (ATP, 3.33 mM => final conc. in the 5 µL assay volume is 2 mM) and substrate (1.67 µM => final conc. in the 5 µL assay volume is 1 µM) in assay buffer and the resulting mixture was incubated for a reaction time of 30 min at 22°C. The concentration of EGFR was adjusted depending of the activity of the enzyme lot and was chosen appropriate to have the assay in the linear range, typical concentration was 7.6 pg/µl. The reaction was stopped by the addition of 3 µl of a solution of HTRF detection reagents (83.3 nM streptavidine-XL665 [Cisbio Bioassays, Codolet, France] and 1.67 nM PT66-Tb-Cryptate, an terbium-cryptate labelled anti-phospho-tyrosine antibody from Cisbio Bioassays [instead of the PT66 Tb cryptate PT66 Eu Chelate from Perkin Elmer can also be used]) in an aqueous EDTA-solution (133.3 mM EDTA, 0.2 % (w/v) bovine serum albumin in 50 mM HEPES pH 7.5).

The resulting mixture was incubated 1 h at 22°C to allow the binding of the biotinylated phosphorylated peptide to the streptavidine-XL665 and the PT66-Tb-Cryptate. Subsequently the amount of phosphorylated substrate was evaluated by measurement of the resonance energy transfer from the PT66-Tb-Cryptate to the streptavidine-XL665. Therefore, the fluorescence emissions at 620 nm and 665 nm after excitation at 337 nm were measured in a HTRF reader, e.g. a Pherastar (BMG Labtechnologies, Offenburg, Germany) or a Viewlux (Perkin-Elmer). The ratio of the emissions at 665 nm and at 622 nm was taken as the measure for the amount of phosphorylated substrate. The data were normalised (enzyme reaction without inhibitor = 0 % inhibition, all other assay components but no enzyme = 100 % inhibition). Usually the test compounds were tested on the same microtiterplate in 11 different concentrations in the range of 20 µM to 0.07 nM (20 µM, 5.7 µM, 1.6 µM, 0.47 µM, 0.13 µM, 38 nM, 11 nM, 3.1 nM, 0.9 nM, 0.25 nM and 0.07 nM, the dilution series prepared separately before the assay on the level of the 100-fold concentrated solutions in DMSO by serial dilutions, exact concentrations may vary depending pipettors used) in duplicate values for each concentration and IC₅₀ values were calculated using Genedata Screener^{™} software.

### Exon20-mutant-EGFR(D770_N771insSVD) kinase assay

Inhibitory activity of compounds of the present invention against an Epidermal Growth Factor Receptor (EGFR) with an insertion of the amino acids sequence SVD between D770 and N771 was quantified employing the TR-FRET based kinase activity assay as described in the following paragraphs.

A recombinant fusion protein of N-terminal Glutathion-S-Transferase (GST) and a fragment of human EGFR variant (amino acids R669 to A1210 with insertion of the amino acids sequence SVD between D770 and N771 ("**EGFR ins SVD**"), expressed in Sf9 insect cells and purified via affinity chromatography using Glutathion Sepharose as described above, was used as a kinase. As substrate for the kinase reaction the biotinylated peptide biotin-Ahx-AEEEEYFELVAKKK (C-terminus in amide form) was used which can be purchased *e.g*. form the company Biosynthan GmbH (Berlin-Buch, Germany).

For the assay 50 nl of a 100-fold concentrated solution of the test compound in DMSO was pipetted into either a black low volume 384 well microtiter plate or a black 1536 well microtiter plate (both Greiner Bio-One, Frickenhausen, Germany), 2 µl of a solution of EGFR in aqueous assay buffer [50 mM Hepes pH 7.0, 10 mM MgCl2, 1 mM dithiothreitol, 0.5 mM EGTA, 0.3 mM activated sodium ortho-vanadate, 0.005 % (w/v) bovine serum albumin, 0.005% (v/v) Tween-20] were added and the mixture was incubated for 15 min at 22°C to allow pre binding of the test compounds to the enzyme before the start of the kinase reaction. Then the kinase reaction was started by the addition of 3 µL of a solution of adenosine tri phosphate (ATP, 3.33 mM => final conc. in the 5 µL assay volume is 2 mM) and substrate (1.67 µM => final conc. in the 5 µL assay volume is 1 µM) in assay buffer and the resulting mixture was incubated for a reaction time of 30 min at 22°C. The concentration of EGFR was adjusted depending of the activity of the enzyme lot and was chosen appropriate to have the assay in the linear range, typical concentration was 15 pg/µl. The reaction was stopped by the addition of 3 µl of a solution of HTRF detection reagents (83.3 nM streptavidine-XL665 [Cisbio Bioassays, Codolet, France] and 1.67 nM PT66-Tb-Cryptate, a terbium-cryptate labelled anti-phospho-tyrosine antibody from Cisbio Bioassays [instead of the PT66 Tb cryptate PT66 Eu Chelate from Perkin Elmer can also be used]) in an aqueous EDTA-solution (133.3 mM EDTA, 0.2 % (w/v) bovine serum albumin in 50 mM HEPES pH 7.5).

The resulting mixture was incubated 1 h at 22°C to allow the binding of the biotinylated phosphorylated peptide to the streptavidine-XL665 and the PT66-Tb-Cryptate.

Subsequently the amount of phosphorylated substrate was evaluated by measurement of the resonance energy transfer from the PT66-Tb-Cryptate to the streptavidine-XL665. Therefore, the fluorescence emissions at 620 nm and 665 nm after excitation at 337 nm were measured in a HTRF reader, *e.g*. a Pherastar (BMG Labtechnologies, Offenburg, Germany) or a Viewlux (Perkin-Elmer). The ratio of the emissions at 665 nm and at 622 nm was taken as the measure for the amount of phosphorylated substrate. The data were normalised (enzyme reaction without inhibitor = 0 % inhibition, all other assay components but no enzyme = 100 % inhibition). Usually the test compounds were tested on the same microtiterplate in 11 different concentrations in the range of 20 µM to 0.07 nM (20 µM, 5.7 µM, 1.6 µM, 0.47 µM, 0.13 µM, 38 nM, 11 nM, 3.1 nM, 0.9 nM, 0.25 nM and 0.07 nM, the dilution series prepared separately before the assay on the level of the 100fold concentrated solutions in DMSO by serial dilutions, exact concentrations may vary depending pipettors used) in duplicate values for each concentration and IC₅₀ values were calculated using Genedata Screener^{™} software.

### Exon20-mutant-EGFR(V769_D770insASV) kinase assay

Inhibitory activity of compounds of the present invention against an Epidermal Growth Factor Receptor (EGFR) with an insertion of the amino acids sequence ASV between V769 and D770 was quantified employing the TR-FRET based kinase activity assay as described in the following paragraphs.

A recombinant fusion protein of N-terminal Glutathion-S-Transferase (GST) and a fragment of human EGFR variant (amino acids R669 to A1210 with insertion of the amino acids sequence ASV between V769 and D770; ("**EGFR ins ASV**"), expressed in Sf9 insect cells and purified via affinity chromatography using Glutathion Sepharose as described above, was used as kinase. As substrate for the kinase reaction the biotinylated peptide biotin-Ahx-AEEEEYFELVAKKK (C-terminus in amide form) was used which can be purchased e.g. form the company Biosynthan GmbH (Berlin-Buch, Germany).

For the assay 50 nl of a 100-fold concentrated solution of the test compound in DMSO was pipetted into either a black low volume 384well microtiter plate or a black 1536 well microtiter plate (both Greiner Bio-One, Frickenhausen, Germany), 2 µl of a solution of EGFR in aqueous assay buffer [50 mM Hepes pH 7.0, 10 mM MgCl2, 1mM dithiothreitol, 0.5 mM EGTA, 0.3 mM activated sodium ortho-vanadate, 0.005 % (w/v) bovine serum albumin, 0.005% (v/v) Tween-20] were added and the mixture was incubated for 15 min at 22°C to allow pre binding of the test compounds to the enzyme before the start of the kinase reaction. Then the kinase reaction was started by the addition of 3 µL of a solution of adenosine tri phosphate (ATP, 3.33 mM => final conc. in the 5 µL assay volume is 2 mM) and substrate (1.67 µM => final conc. in the 5 µL assay volume is 1 µM) in assay buffer and the resulting mixture was incubated for a reaction time of 30 min at 22°C. The concentration of EGFR was adjusted depending of the activity of the enzyme lot and was chosen appropriate to have the assay in the linear range, typical concentration was 2.5 pg/µl. The reaction was stopped by the addition of 3 µl of a solution of HTRF detection reagents (83.3 nM streptavidine-XL665 [Cisbio Bioassays, Codolet, France] and 1.67 nM PT66-Tb-Cryptate, an terbium-cryptate labelled anti-phospho-tyrosine antibody from Cisbio Bioassays [instead of the PT66 Tb cryptate PT66 Eu Chelate from Perkin Elmer can also be used]) in an aqueous EDTA-solution (133.3 mM EDTA, 0.2 % (w/v) bovine serum albumin in 50 mM HEPES pH 7.5).

The resulting mixture was incubated 1 h at 22°C to allow the binding of the biotinylated phosphorylated peptide to the streptavidine-XL665 and the PT66-Tb-Cryptate. Subsequently the amount of phosphorylated substrate was evaluated by measurement of the resonance energy transfer from the PT66-Tb-Cryptate to the streptavidine-XL665. Therefore, the fluorescence emissions at 620 nm and 665 nm after excitation at 337 nm were measured in a HTRF reader, *e.g*. a Pherastar (BMG Labtechnologies, Offenburg, Germany) or a Viewlux (Perkin-Elmer). The ratio of the emissions at 665 nm and at 622 nm was taken as the measure for the amount of phosphorylated substrate. The data were normalised (enzyme reaction without inhibitor = 0 % inhibition, all other assay components but no enzyme = 100 % inhibition). Usually the test compounds were tested on the same microtiterplate in 11 different concentrations in the range of 20 µM to 0.07 nM (20 µM, 5.7 µM, 1.6 µM, 0.47 µM, 0.13 µM, 38 nM, 11 nM, 3.1 nM, 0.9 nM, 0.25 nM and 0.07 nM, the dilution series prepared separately before the assay on the level of the 100-fold concentrated solutions in DMSO by serial dilutions, exact concentrations may vary depending pipettors used) in duplicate values for each concentration and IC₅₀ values were calculated using Genedata Screener^{™} software. Table 2 shows the results of the inhibition in mutant EGFR biochemical assay.

**Table 2:**

| **Example No.** | **mutEGFR (D770_N771insSVD) kinase assay IC₅₀ [mol/l]** |
|---|---|
| **1** | 1.35 E-10 |
| **2** | 2.35 E-10 |
| **3** | 8.54 E-11 |
| **4** | 1.13 E-10 |
| **5** | 1.25 E-10 |
| **6** | 2.17 E-10 |
| **7** | 8.54 E-11 |
| | <7.25 E-11 |
| **8** | 2.46 E-10 |
| **9** | 1.01 E-10 |
| **10** | 9.57 E-11 |
| **11** | 1.79 E-10 |
| **12** | 1.72 E-10 |
| **13** | 1.89 E-10 |
| **14** | 1.39 E-10 |
| **15** | 1.58 E-10 |
| **16** | 4.13 E-10 |
| **17** | 2.38 E-10 |
| **18** | 1.13 E-10 |
| **19** | 2.79 E-10 |
| **20** | 3.16 E-10 |
| **21** | 4.73 E-10 |
| **22** | 6.60 E-10 |
| **23** | 7.96 E-10 |
| **24** | 1.11 E-10 |
| | <7.25 E-11 |
| **25** | 1.41 E-10 |
| **26** | 2.42 E-10 |
| **27** | 9.73 E-11 |
| **28** | 1.39 E-10 |
| **29** | 1.76 E-10 |
| **30** | 1.47 E-10 |
| **31** | 1.19 E-10 |
| **32** | 1.60 E-10 |
| | <7.25 E-11 |
| **33** | 2.01 E-10 |
| **34** | 1.75 E-10 |
| **35** | 2.72 E-10 |
| **36** | 3.31 E-10 |
| **37** | 3.18 E-10 |
| **38** | 1.88 E-10 |
| **39** | 1.88 E-8 |
| **40** | 9.81 E-11 |
| | <7.25 E-11 |
| **41** | 1.78 E-10 |
| **42** | 2.13 E-10 |
| **43** | 4.18 E-10 |
| **44** | 1.95 E-10 |
| **45** | 3.91 E-10 |
| **46** | 1.17 E-9 |
| **47** | 6.67 E-10 |
| **48** | 2.27 E-10 |
| **49** | 2.75 E-10 |
| **50** | 3.93 E-10 |
| **51** | 3.05 E-10 |
| **52** | 1.42 E-10 |
| **53** | 2.82 E-10 |
| **54** | 1.98 E-10 |
| **55** | 2.39 E-10 |
| **56** | 1.94 E-10 |
| **57** | 2.46 E-10 |
| **58** | 1.48 E-10 |
| **59** | 2.85 E-10 |
| **60** | 6.03 E-10 |
| **61** | 1.90 E-10 |
| **62** | 3.29 E-10 |
| **63** | 2.11 E-10 |
| **64** | 3.61 E-10 |
| **65** | 2.40 E-10 |
| **66** | 1.45 E-10 |
| **67** | 2.52 E-10 |
| **68** | 3.00 E-10 |
| **69** | 2.69 E-10 |
| **70** | 2.55 E-10 |
| **71** | 1.86 E-10 |
| **72** | 1.51 E-10 |
| **73** | 1.79 E-10 |
| **74** | 2.42 E-10 |
| **75** | 1.04 E-10 |
| **76** | 2.40 E-10 |
| **77** | 1.50 E-10 |
| **78** | 2.60 E-10 |
| **79** | 4.55 E-10 |
| **80** | 1.31 E-10 |
| **81** | 1.57 E-10 |
| **82** | 1.43 E-10 |
| **83** | 8.49 E-10 |
| **84** | 6.57 E-10 |
| **85** | 9.48 E-10 |
| **86** | 2.61 E-10 |
| **87** | 5.35 E-10 |
| **88** | 3.26 E-10 |
| **89** | 2.78 E-10 |
| **90** | 4.99 E-10 |
| **91** | 3.34 E-10 |
| **92** | 2.43 E-10 |
| **93** | 4.00 E-10 |
| **94** | 6.31 E-10 |
| **95** | 5.04 E-10 |
| **96** | 9.85 E-10 |
| **97** | 7.96 E-10 |
| **98** | 5.25 E-10 |
| **99** | 7.20 E-10 |
| **100** | 3.50 E-10 |
| **101** | 5.53 E-10 |
| **102** | 1.21 E-10 |
| **103** | 1.84 E-10 |
| **104** | 1.62 E-10 |
| **105** | 3.24 E-10 |
| **106** | 1.80 E-10 |
| **107** | 1.15 E-10 |
| **108** | 2.13 E-10 |
| **109** | 5.26 E-10 |
| **110** | 3.33 E-10 |
| **111** | 2.05 E-10 |
| **112** | 1.93 E-10 |
| **113** | 2.01 E-10 |
| **114** | 1.81 E-10 |
| **115** | 4.56 E-10 |
| **116** | 2.76 E-10 |
| **118** | 4.31 E-10 |
| **119** | 3.24 E-10 |
| **120** | 9.01 E-11 |
| **121** | 1.88 E-10 |
| **122** | 1.22 E-10 |
| **123** | 2.41 E-10 |
| **124** | 3.15 E-10 |
| **125** | 2.35 E-10 |
| **126** | 2.79 E-10 |
| **127** | 5.10 E-10 |
| **128** | 1.06 E-9 |
| **129** | 2,57 E-10 |
| **130** | 2,56 E-10 |
| **131** | 3,18 E-10 |
| **132** | 3,94 E-10 |
| **133** | 3,41 E-10 |
| **134** | 1,79 E-10 |

### Bub1 high ATP kinase assay

Bub1-inhibitory activity of compounds of the present invention at a high ATP concentration was quantified employing the Bub1 TR-FRET high ATP kinase assay as described in the following paragraphs.

N-terminally His₆-tagged recombinant catalytic domain of human Bub1 (amino acids 704-1085), expressed in insect cells (Hi5) and purified by Ni-NTA affinity chromatography and subsequent size exclusion chromatography, was used as enzyme. As substrate for the kinase reaction the biotinylated peptide biotin-Ahx-VLLPKKSFAEPG (C-terminus in amid form) was used which can be purchased e.g. form the company Biosyntan (Berlin, Germany).

For the assay 50 nl of a 100-fold concentrated solution of the test compound in DMSO was pipetted into either a black low volume 384well microtiter plate or a black 1536well microtiter plate (both Greiner Bio-One, Frickenhausen, Germany), 3 µl of a solution of adenosine-tri-phosphate (ATP, 3.33 mM => final conc. in the 5 µl assay volume is 2 mM) and substrate (1.67 µM => final conc. in the 5 µl assay volume is 1 µM) in aqueous assay buffer [50 mM Tris/HCl pH 7.5, 10 mM magnesium chloride (MgCl₂), 200 mM potassium chloride (KCI), 1.0 mM dithiothreitol (DTT), 0.1 mM sodium ortho-vanadate, 1% (v/v) glycerol, 0.01 % (w/v) bovine serum albumine (BSA), 0.005% (v/v) Trition X-100 (Sigma), 1x *Complete EDTA-free* protease inhibitor mixture (Roche)] were added. Then the kinase reaction was started by the addition of 2 µl of a solution of Bub1 in assay buffer and the resulting mixture was incubated for a reaction time of 60 min at 22°C. The concentration of Bub1 was adjusted depending of the activity of the enzyme lot and was chosen appropriate to have the assay in the linear range, a typical concentration is about 200 ng/ml. The reaction was stopped by the addition of 3 µl of a solution of TR-FRET detection reagents (0.167 µM streptavidine-XL665 [Cisbio Bioassays, Codolet, France] and 1.67 nM anti-phosho-Serine antibody [Merck Millipore, cat. # 35-002] and 0.67 nM LANCE EU-W1024 labeled anti-mouse IgG antibody [Perkin-Elmer, product no. AD0077, as an alternative a Terbium-cryptate-labeled anti-mouse IgG antibody from Cisbio Bioassays can be used]) in an aqueous EDTA-solution (83.3 mM EDTA, 0.2 % (w/v) bovine serum albumin in 100 mM HEPES pH 7.5).

The resulting mixture was incubated 1 h at 22°C to allow the formation of complex between the phosphorylated biotinylated peptide and the detection reagents. Subsequently the amount of phosphorylated substrate was evaluated by measurement of the resonance energy transfer from the Eu-chelate to the streptavidine-XL. Therefore, the fluorescence emissions at 620 nm and 665 nm after excitation at 350 nm was measured in a TR-FRET reader, e.g. a Pherastar or Pherastar FS (both from BMG Labtechnologies, Offenburg, Germany) or a Viewlux (Perkin-Elmer). The ratio of the emissions at 665 nm and at 622 nm was taken as the measure for the amount of phosphorylated substrate. The data were normalised (enzyme reaction without inhibitor = 0 % inhibition, all other assay components but no enzyme = 100 % inhibition). Usually the test compounds were tested on the same microtiterplate in 11 different concentrations in the range of 20 µM to 0.7 nM (20 µM, 5.7 µM, 1.6 µM, 0.47 µM, 0.13 µM, 38 nM, 11 nM, 3.1 nM, 0.9 nM, 0.25 nM and 0.07 nM, the dilution series prepared separately before the assay on the level of the 100fold concentrated solutions in DMSO by serial dilutions, exact concentrations may vary depending pipettors used) in duplicate values for each concentration and IC₅₀ values were calculated by a 4 parameter fit. Table 3 shows the results of the inhibition in Bub1 high ATP kinase assay.

**Table 3:**

| **Example No.** | **Bub1 high ATP (2 mM) IC₅₀ [mol/l]** |
|---|---|
| **1** | 1.32 E-6 |
| **2** | 3.93 E-7 |
| **3** | 5.39 E-6 |
| **4** | 3.01 E-6 |
| **5** | 9.70 E-6 |
| **6** | 1.02 E-5 |
| **7** | >2.0 E-5 |
| **8** | 1.28 E-5 |
| | 9.72 E-6 |
| | >2.0 E-5 |
| **9** | 8.24 E-6 |
| **10** | >2.0 E-5 |
| **11** | >2.0 E-5 |
| **12** | >2.0 E-5 |
| **13** | 3.63 E-6 |
| **14** | 5.39 E-6 |
| **15** | 1.39 E-6 |
| **16** | >2.0 E-5 |
| **17** | >2.0 E-5 |
| **18** | 1.92 E-5 |
| | >2.0 E-5 |
| **19** | 1.35 E-5 |
| **20** | 1.72 E-5 |
| **21** | >2.0 E-5 |
| **22** | 1.16 E-5 |
| **23** | >2.0 E-5 |
| **24** | 3.48 E-6 |
| **25** | 5.41 E-6 |
| **26** | 3.11 E-6 |
| **27** | >5.7 E-6 |
| **28** | 9.72 E-6 |
| **29** | 2.39 E-6 |
| **30** | 1.13 E-5 |
| **31** | >2.0 E-5 |
| **32** | 3.64 E-6 |
| **33** | 5.16 E-6 |
| **34** | >2.0 E-5 |
| **35** | 1.24 E-5 |
| **36** | 4.90 E-6 |
| **37** | 2.87 E-6 |
| **38** | 6.28 E-7 |
| **39** | >2.0 E-5 |
| **40** | 3.99 E-7 |
| **41** | 1.70 E-5 |
| **42** | 8.81 E-6 |
| **43** | >2.0 E-5 |
| **44** | 6.45 E-6 |
| **45** | >2.0 E-5 |
| **46** | >2.0 E-5 |
| **47** | 7.60 E-6 |
| **48** | 9.13 E-6 |
| **49** | 1.32 E-5 |
| **50** | >2.0 E-5 |
| **51** | 7.09 E-6 |
| **52** | 1.00 E-5 |
| **53** | 1.45 E-5 |
| **54** | 6.19 E-6 |
| **55** | 7.58 E-7 |
| **56** | 1.85 E-5 |
| | 1.88 E-5 |
| | >2.0 E-5 |
| **57** | 2.81 E-7 |
| **58** | 3.36 E-6 |
| **59** | 1.64 E-5 |
| **60** | 1.62 E-6 |
| **61** | 2.70 E-6 |
| **62** | 8.03 E-6 |
| **63** | 1.41 E-5 |
| **64** | >2.0 E-5 |
| **65** | 2.52 E-6 |
| **66** | 1.73 E-6 |
| **67** | 1.01 E-5 |
| **68** | 5.13 E-6 |
| **69** | 1.73 E-6 |
| **70** | 1.55 E-6 |
| **71** | 5.74 E-6 |
| **72** | 7.07 E-6 |
| **73** | 4.06 E-6 |
| **74** | >2.0 E-5 |
| **75** | 1.11 E-5 |
| **76** | >5.7 E-6 |
| **77** | >2.0 E-5 |
| **78** | >2.0 E-5 |
| **79** | 1.73 E-5 |
| | >2.0 E-5 |
| **80** | 2.67 E-6 |
| **81** | 4.87 E-6 |
| **82** | 2.71 E-6 |
| **83** | >2.0 E-5 |
| **84** | >2.0 E-5 |
| **85** | >2.0 E-5 |
| **86** | 2.40 E-6 |
| **87** | 1.91 E-5 |
| | >2.0 E-5 |
| **88** | 9.78 E-7 |
| **89** | 1.56 E-5 |
| **90** | 3.88 E-6 |
| **91** | >2.0 E-5 |
| **92** | 1.98 E-6 |
| **93** | 5.42 E-6 |
| **94** | >2.0 E-5 |
| **95** | >2.0 E-5 |
| **96** | >2.0 E-5 |
| **97** | >2.0 E-5 |
| **98** | 9.54 E-7 |
| **99** | >2.0 E-5 |
| **100** | 3.64 E-6 |
| **101** | >2.0 E-5 |
| **102** | 1.03 E-6 |
| **103** | 1.65 E-6 |
| **104** | 3.63 E-6 |
| **105** | 3.70 E-6 |
| **106** | 1.16 E-5 |
| **107** | >2.0 E-5 |
| **108** | 6.22 E-6 |
| **109** | 3.59 E-6 |
| **110** | 1.21 E-6 |
| **111** | 3.74 E-7 |
| **112** | 7.83 E-6 |
| **113** | 7.61 E-7 |
| **114** | 2.07 E-6 |
| **115** | 1.32 E-5 |
| **116** | 1.37 E-5 |
| **118** | >2.0 E-5 |
| | 1.94 E-5 |
| **119** | 1,52 E-6 |
| **120** | 1.48 E-6 |
| **121** | 1,83 E-6 |
| **122** | 7,41 E-7 |
| **123** | 4,39 E-7 |
| **124** | 4,79 E-7 |
| **125** | 4,46 E-7 |
| **126** | 1,10 E-6 |
| **127** | 1,44 E-6 |
| **128** | 7,26 E-7 |
| **129** | 1,31 E-6 |
| **130** | 7,68 E-7 |
| **131** | 1,67 E-6 |
| **132** | 1,48 E-6 |
| **133** | 1,65 E-6 |
| **134** | 1,16 E-6 |

Compounds of the present invention may show additional advantageous properties, such as, more potent inhibition of mutant EGFR with exon20 insertions than inhibition of wild-type EGFR, which may be useful to reduce potential toxicity arising from excessive inhibition of wild-type EGFR.

### Cellular Data Description (Ba/F3 cells overexpressing wt and mutant EGFR)

293T cells from ATCC were transfected with pBABEpuro expression constructs for WT EGFR or mutant EGFR (V769_D770insASV, D770_N771insSVD, D770_N771insNPG, N771_P772insH, H773_V774insNPH, E746_A750del, L858R, D770_N771insSVD C797S, E746_A750del C797S, L858R C797S, L861Q) or mutant ERBB2 (A775_G776insYVMA) and pCL-Eco packaging vector using Fugene-6 transfection reagent from Promega. Plates were incubated at at 37°C for 48 h. Retrovirus was harvested by filtering the media supernatant through a 0.45 µm filter.

Ba/F3 cells purchased from DSMZ were grown in RPMI + 10% FBS + 10 ng/mL IL-3 and infected with filtered retroviral supernatant at a 1:2 dilution. Polybrene was added to a concentration of 8 µg/mL, plates were spun for 90 min, and incubated overnight at 37°C. 2 µg/mL puromycin was added to the infected cells 24 h after infection and cells were continually grown in the presence of puromycin and 10 ng/mL IL-3. Following stably expressing Ba/F3 cell lines were generated: Ba/F3-EGFR-WT, Ba/F3-EGFR-V769_D770insASV, Ba/F3-EGFR-D770_N771insSVD, Ba/F3-EGFR-D770_N771insNPG, Ba/F3-EGFR-N771_P772insH, Ba/F3-EGFR-H773_V774insNPH, Ba/F3-EGFR-E746_A750del, Ba/F3-EGFR-L858R, Ba/F3-EGFR-D770_N771insSVD C797S, Ba/F3-EGFR-E746_A750del C797S, Ba/F3-EGFR-L858R C797S, Ba/F3-EGFR L861Q and Ba/F3-ERBB2-A775_G776insYVMA (Ba/F3- vector-control).

For cell survival assays, Ba/F3 cells were grown to a density of 1-2 million cells per mL, spun down and resuspended in media without IL-3, and replated at a concentration 200,000-500,000 cells per mL. The cells ectopically expressing WT EGFR plated with 10 ng/mL Millipore Culture grade EGF and Ba/F3 cells containing mutant EGFR or Mutant ERBB2 were cultivated without EGF. The cells ectopically expressing pBABEpuro empty vector were plated with 10 ng/mL IL-3.

2 days later, cells were plated in 50 µL in a 384 well plate at a concentration of 4000 cells per well for cells assayed in the absence of IL-3 and 2000 cells per well for cells assayed in the presence of IL-3. 100 nL of compound was added to each well using a 100 nL pin head, and plates were incubated at 37°C for 48 h.

Cell viability was measured by adding 20 µL of Cell Titer-Glo Luminescent Cell Viability Reagent diluted 1:3 in PBS. Plates were sealed with Perkin Elmer Top-Seal, inverted several times to mix, and immediately centrifuged at 1000 rpm for 2 min. Plates were incubated in low light conditions for 8-10 min and luminescence was measured. The IC₅₀ values for the examples are shown in Table 4, 6, 7, 8 and 9.

### Cellular Data Description (PC9 cells, EGFRex19del)

PC9 cells were purchased from Tokyo Medical College. 400 PC9 cells per well were seeded in growth medium (DMEM, 10% FCS) in a 384-well plate (CORNING #3571). Seed reference plate for time zero determination on the same day. All plates were incubated overnight at 37°C. After 24 hours, test compound were added in 7-step dilution using HP Compound printer and incubated at 37°C for 72h. After 3 days, 30 µL/well CTG solution (Promega Cell Titer Glo solution; catalog # G755B and G756B) were added to each well, incubated for 30 minutes and the plate were read on PheraStar. Proliferation is calculated after subtracting time zero luminescence values from day 4 values and comparing to untreated wells.

The IC₅₀ values were determined using the four parameter fit. The IC₅₀ values for the examples are shown in Table 9.

### Cellular Data Description (HCC-827 cells, EGFRex19del)

HCC-827 cells were purchased from ATCC. 400 HCC-829 cells per well were seeded in growth medium (RPMI1640, 10% FCS) in a 384-well plate (CORNING #3571). Seed reference plate for time zero determination on the same day. All plates were incubated overnight at 37°C. After 24 hours, test compound were added in 7-step dilution using HP Compound printer and incubated at 37°C for 72h. After 3 days, 30 µL/well CTG solution (Promega Cell Titer Glo solution; catalog # G755B and G756B) were added to each well, incubated for 30 minutes and the plate were read on PheraStar. Proliferation is calculated after subtracting time zero luminescence values from day 4 values and comparing to untreated wells.

The IC₅₀ values were determined using the four parameter fit. The IC₅₀ values for the examples are shown in Table 9.

**Table 4:**

| **Example No.** | **BA/F3 (insSVD) IC₅₀ [mol/l]** | **BA/F3 (wild type) IC₅₀ [mol/l]** |
|---|---|---|
| **1** | 1.78 E-8 | 4.17 E-7 |
| **2** | 6.46 E-8 | 5.75 E-7 |
| **3** | 2.85 E-8 | 3.28 E-7 |
| **4** | 9.53 E-8 | 8.83 E-7 |
| **5** | 2.02 E-8 | 3.31 E-7 |
| **6** | 8.64 E-8 | 6.23 E-7 |
| **7** | 2.57 E-8 | 3.84 E-7 |
| **8** | 7.33 E-8 | 6.23 E-7 |
| **9** | 1.04 E-7 | 6.44 E-7 |
| **10** | 3.17 E-8 | 3.82 E-7 |
| **11** | 1.78 E-8 | 2.64 E-7 |
| **12** | 4.87 E-8 | 7.19 E-7 |
| **13** | 3.50 E-8 | 3.39 E-7 |
| **14** | 2.10 E-8 | 2.62 E-7 |
| **15** | 1.92 E-7 | 8.95 E-7 |
| **16** | 6.38 E-8 | 6.44 E-7 |
| **17** | 2.30 E-7 | 1.58 E-6 |
| **18** | 2.64 E-8 | 3.62 E-7 |
| **19** | 8.65 E-8 | 6.84 E-7 |
| **20** | 1.22 E-7 | 1.17 E-6 |
| **21** | 6.79 E-8 | 9.43 E-7 |
| **22** | 6.16 E-7 | 2.63 E-6 |
| **23** | ! 9.20 **E-7** | 7.38 E-6 |
| **24** | 2.08 E-8 | 5.00 E-7 |
| **25** | 2.59 E-8 | 3.58 E-7 |
| **26** | 6.31 E-8 | 5.87 E-7 |
| **27** | 1.20 E-8 | 3.05 E-7 |
| **28** | 8.33 E-9 | 1.89 E-7 |
| **29** | 1.10 E-8 | 2.88 E-7 |
| **30** | 4.40 E-8 | 6.06 E-7 |
| **31** | 1.25 E-7 | ! 2.77 E-6 |
| **32** | 5.27 E-8 | 7.37 E-7 |
| **33** | 7.31 E-8 | 8.44 E-7 |
| **34** | 2.02 E-8 | 4.24 E-7 |
| **35** | 5.41 E-8 | 8.11 E-7 |
| **36** | 7.53 E-8 | 9.63 E-7 |
| **37** | 1.49 E-7 | 1.09 E-6 |
| **38** | 1.38 E-7 | 9.96 E-7 |
| **39** | 7.86 E-7 >1.0 E-6 | 4.12 E-6 |
| **40** | 6.43 E-8 | 4.80 E-7 |
| **41** | 3.98 E-8 | 4.79 E-7 |
| **42** | 1.53 E-8 | 5.31 E-7 |
| **43** | 2.19 E-7 | 1.86 E-6 |
| **44** | 4.05 E-8 | 5.94 E-7 |
| **45** | 4.73 E-8 | 8.40 E-7 |
| **46** | 1.25 E-7 | 2.75 E-6 |
| **47** | 1.06 E-7 | 1.99 E-6 |
| **48** | 2.57 E-8 | 6.11 E-7 |
| **49** | 3.31 E-8 | 2.91 E-7 |
| **50** | 3.49 E-7 | 1.65 E-6 |
| **51** | 1.36 E-8 | 4.12 E-7 |
| **52** | 3.89 E-8 | 4.07 E-7 |
| **53** | 3.49 E-8 | 7.12 E-7 |
| **54** | 4.24 E-8 | 6.75 E-7 |
| **55** | 7.51 E-8 | 9.78 E-7 |
| **56** | 5.15 E-8 | 6.93 E-7 |
| **57** | 1.95 E-7 | 1.71 E-6 |
| **58** | 7.29 E-8 | 1.25 E-6 |
| **59** | 4.19 E-8 | 6.09 E-7 |
| **60** | 1.75 E-7 | 1.07 E-6 |
| **61** | 2.05 E-8 | 5.34 E-7 |
| **62** | 5.26 E-8 | 1.10 E-6 |
| **63** | 1.17 E-7 | 1.17 E-6 |
| **64** | 4.76 E-8 | 6.43 E-7 |
| **65** | 7.20 E-8 | 7.95 E-7 |
| **66** | 4.28 E-8 | 7.66 E-7 |
| **67** | 2.71 E-8 | 1.15 E-6 |
| **68** | 6.37 E-8 | 1.38 E-6 |
| **69** | 7.24 E-8 | 2.18 E-6 |
| **70** | 2.24 E-7 | 4.63 E-6 |
| **71** | 4.29 E-8 | 7.31 E-7 |
| **72** | 3.66 E-8 | 6.28 E-7 |
| **73** | 5.53 E-8 | 6.43 E-7 |
| **74** | 1.40 E-8 | 4.57 E-7 |
| **75** | 1.82 E-8 | 2.64 E-7 |
| **76** | 1.77 E-7 | 2.44 E-6 |
| **77** | 1.53 E-8 | 3.20 E-6 |
| **78** | 2.07 E-8 | 4.88 E-7 |
| **79** | 1.51 E-7 | 1.91 E-6 |
| **80** | 1.99 E-8 | 6.28 E-7 |
| **81** | 4.40 E-8 | 5.83 E-7 |
| **82** | 1.39 E-8 | 2.48 E-7 |
| **83** | 2.46 E-8 | 6.69 E-7 |
| **84** | 1.61 E-8 | 3.79 E-7 |
| **85** | 1.03 E-7 | 1.17 E-6 |
| **86** | 1.93 E-8 | 4.80 E-7 |
| **87** | 1.50 E-8 | 4.21 E-7 |
| **88** | 7.04 E-8 | 6.34 E-7 |
| **89** | 1.82 E-8 | 4.25 E-7 |
| **90** | 8.38 E-8 | 1.17 E-6 |
| **91** | 3.02 E-8 | 5.56 E-7 |
| **92** | 2.02 E-8 | 5.19 E-7 |
| **93** | 8.67 E-9 | 2.54 E-7 |
| **94** | 6.66 E-8 | 1.08 E-6 |
| **95** | 1.49 E-8 | 4.15 E-7 |
| **96** | 3.81 E-7 | 3.43 E-6 |
| **97** | 3.21 E-8 | 1.13 E-6 |
| **98** | 1.81 E-8 | 4.44 E-7 |
| **99** | 4.59 E-8 | 8.46 E-7 |
| **100** | 5.89 E-8 | 9.90 E-7 |
| **101** | 4.21 E-8 | 6.03 E-7 |
| **102** | 3.87 E-8 | 8.69 E-7 |
| **103** | 3.22 E-8 | 5.29 E-7 |
| **104** | 3.23 E-8 | 9.83 E-7 |
| **105** | 4.47 E-8 | 8.84 E-7 |
| **106** | 3.18 E-7 | 3.02 E-6 |
| **107** | 2.50 E-8 | 5.46 E-7 |
| **108** | 1.41 E-7 | 1.40 E-6 |
| **109** | 6.69 E-8 | 9.44 E-7 |
| **110** | 4.03 E-8 | 7.80 E-7 |
| **111** | 1.94 E-8 | 5.74 E-7 |
| **112** | 2.04 E-8 | 5.53 E-7 |
| **113** | 2.37 E-8 | 8.49 E-7 |
| **114** | 2.87 E-8 | 3.47 E-7 |
| **115** | 4.94 E-7 >1.0 E-6 | 5.42 E-6 |
| **116** | 1.69 E-7 | 2.03 E-6 |
| **118** | 2.23 E-8 | 9.38 E-7 |
| **119** | 5.47 E-8 | 1.29 E-6 |
| **120** | 3.11E-8 | 3.58 E-7 |
| **121** | 8.81 E-9 | 2.67 E-7 |
| **122** | 2.38 E-8 | 5.06 E-7 |
| **123** | 3.39 E-8 | 6.04 E-7 |
| **124** | 2.60 E-8 | 5.13 E-7 |
| **125** | 4.04 E-8 | 9.48 E-7 |
| **126** | 4.64 E-8 | 8.14 E-7 |
| **127** | 1.21 E-8 | 4.06 E-7 |
| **128** | 3.84 E-8 | 9.42 E-7 |
| **129** | 1,40 E-8 | 2,93 E-7 |
| **130** | 8,01 E-9 | 4,18 E-7 |
| **131** | 4,84 E-8 | 9,80 E-7 |
| **132** | 4,49 E-8 | 9,86 E-7 |
| **133** | 2,22 E-8 | 4,84 E-7 |
| **134** | 9,28 E-8 | 7,18 E-7 |

In contrast to the claimed compounds of this invention the compounds claimed in the closest prior art WO 2016/120196 do not show the advantageous combined properties described above. This can be seen in Table 5.

**Table 5:**

| WO 2016/120196 **Example No.** | **mutEGFR (D770_N771insSVD) kinase assay** | **BA/F3 (insSVD)** | **BA/F3 (wild type)** |
|---|---|---|---|
| | **IC₅₀ [mol/l]** | **IC₅₀ [mol/l]** | **IC₅₀ [mol/l]** |
| **1** | 2.00 E-7 | > 1.00 E-6 | 1.53E-6 |
| **38** | 2.24 E-7 | > 2.00 E-6 | 5.91 E-6 |
| **41** | 3.43 E-8 | > 2.00 E-6 | 5.02 E-6 |
| **45** | 1.28 E-8 | > 2.00 E-6 | 4.04 E-6 |

**Table 6: (EGFR Exon20 insertion mutations)**

| **Example No.** | **BA/F3 (insNPG)** | **BA/F3 (ASV)** | **BA/F3 (EGFR N771_P772insH)** | **BA/F3 (EGFR H773_774insNPH)** |
|---|---|---|---|---|
| | **IC₅₀ [mol/l]** | **IC₅₀ [mol/l]** | **IC₅₀ [mol/l]** | **IC₅₀ [mol/l]** |
| **34** | 1.69 E-8 | 3.02 E-8 | 1.43 E-8 | 1.65 E-7 |
| **51** | 1.14 E-8 | 1.28 E-8 | 6.89 E-9 | 3.08 E-8 |
| **67** | 1.35 E-8 | 4.03 E-8 | 1.83 E-8 | 1.17 E-7 |
| **87** | 1.50 E-8 | 1.32 E-8 | 2.16 E-8 | 1.38 E-7 |
| **113** | 2.49 E-8 | 2.60 E-8 | 1.43 E-8 | 1.16 E-7 |
| **121** | 1.28 E-8 | 1.01 E-8 | 4.49 E-9 | 4.92 E-8 |

**Table 7: (classical activating and rare EGFR mutations)**

| **Example No.** | **BA/F3 (EGFR E746_A750del)** | **BA/F3 (EGFR L858R)** | **BA/F3 (EGFR L861Q)** |
|---|---|---|---|
| | **IC₅₀ [mol/l]** | **IC₅₀ [mol/l]** | **IC₅₀ [mol/l]** |
| **34** | 1.19 E-9 | 2.76 E-9 | 3.18 E-8 |
| **51** | 1.05 E-9 | 1.32 E-9 | 1.49 E-8 |
| **67** | 1.41 E-9 | 3.16 E-9 | 5.36 E-8 |
| **87** | 5.00 E-9 | 1.29 E-9 | 3.34 E-8 |
| **113** | 9.89 E-10 | 3.71 E-9 | 4.50 E-8 |
| **121** | 8.87 E-10 | 1.17 E-9 | 1.12 E-8 |

**Table 8: (aquired resistance space)**

| **Example No.** | **BA/F3 (EGFR 0770_N771 insSVD C797S)** | **BA/F3 (EGFR E746_A750del C797S)** | **BA/F3 (EGFR L858R C797S)** |
|---|---|---|---|
| | **IC₅₀ [mol/l]** | **IC₅₀ [mol/l]** | **IC₅₀ [mol/l]** |
| **34** | 3.55 E-8 | 7.95 E-10 | 3.07 E-9 |
| **51** | 1.95 E-8 | 5.64 E-10 | 1.32 E-9 |
| **67** | 4.06 E-8 | 5.81 E-10 | 2.49 E-9 |
| **87** | 2.21 E-8 | 8.04 E-10 | 1.38 E-9 |
| **113** | 7.14 E-8 | 1.10 E-9 | 5.07 E-9 |
| **121** | 2.17 E-8 | 3.84 E-10 | 1.07 E-9 |

**Table 9: (Errb2 mutans, PC9, HCC-827)**

| **Example No.** | **BA/F3 (ERBB2 A775_G776 insYVMA) IC₅₀ [mol/l]** | **PC9** | **HCC-827** |
|---|---|---|---|
| | | **IC₅₀ [mol/l]** | **IC₅₀ [mol/l]** |
| **34** | 3.31 E-8 | 3.43 E-9 | < 3.00 E-9 |
| **51** | 1.52 E-8 | 1.74 E-9 | < 3.00 E-9 |
| **67** | 2.92 E-8 | 4.09 E-9 | < 3.00 E-9 |
| **87** | 1.62 E-8 | 1.82 E-9 | < 3.00 E-9 |
| **113** | 2.22 E-8 | 6.46 E-9 | < 3.00 E-9 |
| **121** | 1.28 E-8 | 1.84 E-9 | < 3.00 E-9 |

### References:

Arcila et al., 2012: Arcila et al., Clin Cancer Res. 2012 Sep 15;18(18):4910-8.
Chen *et al.,* 2016: Chen et al., Onco Targets Ther. 2016 Jul 8;9:4181-6
Chiu et al., 2015 : Chiu et al., J Thorac Oncol. 2015;10: 793-799
Doebele et al., 2018 : Doebele et al.. Poster 338, presented at the 54th Annual Meeting of the American Society of Clinical Oncology, June 1-5, 2018, Chicago, Illinois
Floc'h et al., 2018 : Floc'h et al., Mol Cancer Ther. 2018 May 17(5) : 885-896
Hasako et al., 2018: Hasako et al., Mol Cancer Ther. 2018 Aug ;17(8):1648-1658
Jang et al., 2018: Jang et al., Angew Chem Int Ed Engl. 2018 Sep 3; 57(36): 11629-11633
Mok *et al.,* 2009 : Mok et al., N Engl J Med. 2009 Sep 3;361(10):947-57
Mok *et al.,* 2017: Mok et al., N Engl J Med. 2017 Feb 16;376(7):629-640
Oxnard *et al.,* 2013: Oxnard et al., J Thorac Oncol. 2013 Feb; 8(2): 179-184
Oxnard *et al.,* 2018: Oxnard et al., JAMA Oncol. 2018;4(11):1527-1534
Paez *et al.,* 2004 : Paez et al., Science. 2004 Jun 4;304(5676):1497-500
Pao *et al.,* 2005: Pao et al., PLoS Med. 2005 Mar;2(3):e73
Pao *et al.,* 2010: Pao and Chmielecki, Nat Rev Cancer. 2010 Nov;10(11):760-74
Ramalingam et al.,2018a: Ramalingam et al., J Clin Oncol. 2018 Mar 20;36(9):841-849.
Ramalingam et al., 2018b: Ramalingam et al., ESMO 2018; Annals Oncol. 2018 Oct : 29 (Suppl 8)
Robichaux et al., 2018 : Robichaux et al., Nat Med. 2018 May;24(5):638-646
Sequist *et al.,* 2013: Sequist et al., J Clin Oncol. 2013 Sep 20;31(27):3327-34
Soria et al., 2018: Soria et al., N Engl J Med. 2018 Jan 11;378(2):113-125.
Thress et al., 2015: Thress et al., Nat Med. 2015 Jun; 21(6): 560-562.
Yang *et al.,* 2015: Yang et al., Lancet Oncol. 2015 Jul;16(7):830-8
Yasuda, 2013: Yasuda, Sci Transl Med. 2013 Dec 18;5(216):216ra177

## Claims

1. A compound of formula (I) 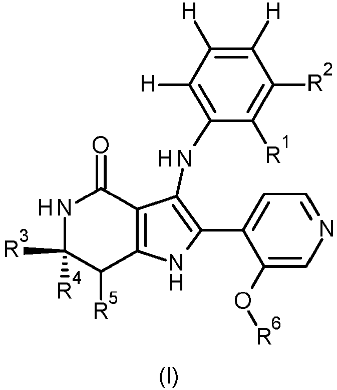 in which:
R¹ represents methyl, ethyl, trifluoromethyl, 2,2-difluoroethyl, cyano, chloro, bromo, methoxy, or difluoromethoxy;
R² represents methyl, ethyl, fluoro, chloro, or bromo;
R³ represents hydrogen, methyl, or trifluoromethyl;
R⁴ represents hydrogen; or
R³ and R⁴ together with the carbon atom to which they are attached form a 3- to 5-membered cycloalkyl ring;
R⁵ represents hydrogen, C₁-C₃-alkyl, or C₂-C₃-alkenyl, wherein said alkyl groups are optionally substituted one or more times, independently of each other, with fluoro, hydroxy, or methoxy and said alkenyl groups are optionally substituted one or more times with fluoro; or
R³ and R⁵ together with the carbon atoms to which they are attached form a 4- to 6-membered cycloalkyl ring;
With the proviso that at least one of R³ and R⁵ is different from hydrogen;
R⁶ represents C₂-C₅-alkyl, which is substituted once with hydroxy, C₁-C₄-alkoxy, or C₂-C₃-alkenyloxy, or a group selected from the group: 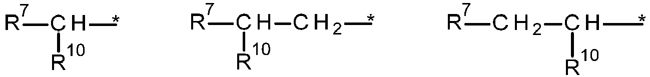 wherein * indicates the point of attachment of said group with the rest of the molecule;
R⁷ represents a group selected from the group: 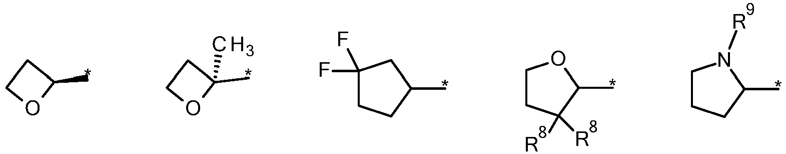 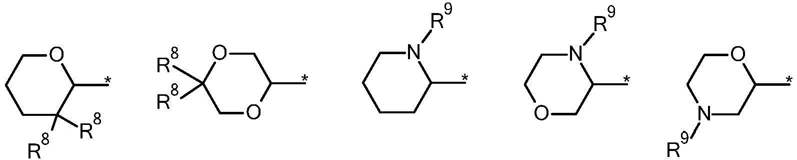 wherein * indicates the point of attachment of said group with the rest of the molecule;
R⁸ independently represents hydrogen, methyl, or fluoro at each occurence;
R⁹ represents C₁-C₃-alkyl or C₂-C₃-fluoroalkyl;
R¹⁰ represents hydrogen or methyl;
or an N-oxide, a salt, a tautomer or a stereoisomer of said compound, or a salt of said N-oxide, tautomer or stereoisomer.

2. The compound of formula (I) according to claim 1, wherein:
R¹ represents methyl, ethyl, trifluoromethyl, 2,2-difluoroethyl, methoxy, or difluoromethoxy;
R² represents methyl, fluoro, chloro, or bromo;
R³ represents hydrogen, methyl, or trifluoromethyl;
R⁴ represents hydrogen; or
R³ and R⁴ together with the carbon atom to which they are attached form a 3- to 5-membered cycloalkyl ring;
R⁵ represents hydrogen, C₁-C₃-alkyl, or C₂-C₃-alkenyl, wherein said alkyl groups are optionally substituted one, two, or three times, independently of each other, with fluoro, hydroxy, or methoxy and said alkenyl groups are optionally substituted one, two, or three times, with fluoro; or
R³ and R⁵ together with the carbon atoms to which they are attached form a 5- to 6-membered cycloalkyl ring;
With the proviso that at least one of R³ and R⁵ is different from hydrogen;
R⁶ represents C₂-C₅-alkyl, which is substituted once with hydroxy, C₁-C₃-alkoxy, or C₂-C₃-alkenyloxy, or a group selected from the group: 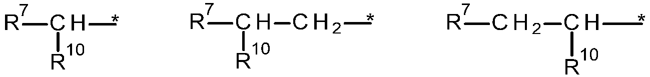 wherein * indicates the point of attachment of said group with the rest of the molecule;
R⁷ represents a group selected from the group: 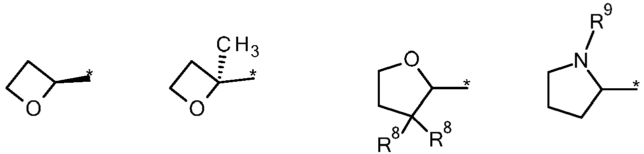 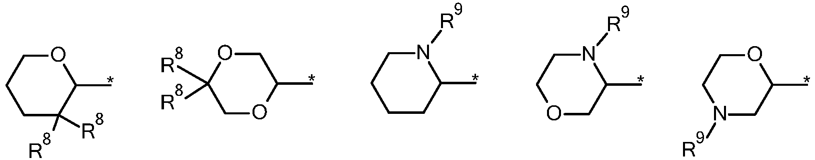 wherein * indicates the point of attachment of said group with the rest of the molecule;
R⁸ represents hydrogen, methyl, or fluoro;
R⁹ represents C₁-C₃-alkyl or C₂-C₃-fluoroalkyl;
R¹⁰ represents hydrogen or methyl;
or an N-oxide, a salt, a tautomer or a stereoisomer of said compound, or a salt of said N-oxide, tautomer or stereoisomer.

3. The compound of formula (I) according to claim 1 or 2, wherein:
R¹ represents methyl, ethyl, 2,2-difluoroethyl, or methoxy;
R² represents fluoro or chloro;
R³ represents hydrogen, methyl, or trifluoromethyl;
R⁴ represents hydrogen; or
R³ and R⁴ together with the carbon atom to which they are attached form a 3- to 4-membered cycloalkyl ring;
R⁵ represents hydrogen, C₁-C₃-alkyl, prop-2-en-1-yl or 3,3-difluoroprop-2-en-1-yl, wherein said alkyl groups are optionally substituted one, two, or three times with fluoro or once with hydroxy; or
R³ and R⁵ together with the carbon atoms to which they are attached form a 5-membered cycloalkyl ring;
With the proviso that at least one of R³ and R⁵ is different from hydrogen;
R⁶ represents C₂-C₄-alkyl, which is substituted once with hydroxy, methoxy, or (prop-2-en-1-yl)oxy,
or a group selected from the group: 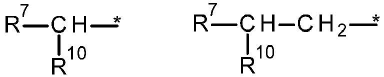
wherein * indicates the point of attachment of said group with the rest of the molecule;
R⁷ represents a group selected from the group: 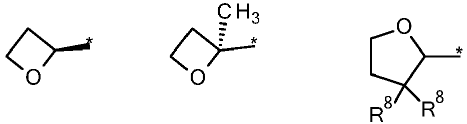 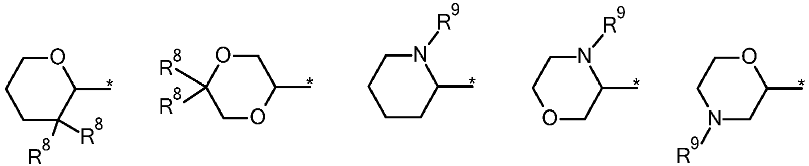 wherein * indicates the point of attachment of said group with the rest of the molecule;
R⁸ represents hydrogen, methyl, or fluoro;
R⁹ represents methyl or 2,2 difluoroethyl;
R¹⁰ represents hydrogen;
or an N-oxide, a salt, a tautomer or a stereoisomer of said compound, or a salt of said N-oxide, tautomer or stereoisomer.

4. The compound of formula (I) according to any of claims 1 to 3, which is selected from the group consisting of:
(6*R*)-3-(3-chloro-2-methoxyanilino)-2-[3-(2-methoxy-2-methylpropoxy)pyridin-4-yl]-6-methyl-1,5,6,7-tetrahydro-4H-pyrrolo[3,2-c]pyridin-4-one,
3-(3-chloro-2-methoxyanilino)-6-methyl-2-(3-{[(2*S*)-oxolan-2-yl]methoxy}pyridin-4-yl)-1,5,6,7-tetrahydro-4*H*-pyrrolo[3,2-c]pyridin-4-one,
(6*R*)-3-(3-chloro-2-methoxyanilino)-6-methyl-2-(3-{[(2*S*)-oxolan-2-yl]methoxy}pyridin-4-yl)-1,5,6,7-tetrahydro-4H-pyrrolo[3,2-c]pyridin-4-one,
(6*R*)-3-(3-chloro-2-methoxyanilino)-6-methyl-2-(3-{[(2*S*)-oxetan-2-yl]methoxy}pyridin-4-yl)-1,5,6,7-tetrahydro-4H-pyrrolo[3,2-c]pyridin-4-one,
3-(3-fluoro-2-methoxyanilino)-6-methyl-2-(3-{[(2*S*)-oxolan-2-yl]methoxy}pyridin-4-yl)-1,5,6,7-tetrahydro-4*H*-pyrrolo[3,2-c]pyridin-4-one,
(6*R*)-3-(3-fluoro-2-methoxyanilino)-6-methyl-2-(3-{[(2*S*)-oxolan-2-yl]methoxy}pyridin-4-yl)-1,5,6,7-tetrahydro-4*H*-pyrrolo[3,2-c]pyridin-4-one,
(6*R*)-3-(3-chloro-2-methoxyanilino)-6-methyl-2-(3-{[(2*S*)-4-methylmorpholin-2-yl]methoxy}pyridin-4-yl)-1,5,6,7-tetrahydro-4*H*-pyrrolo[3,2-c]pyridin-4-one,
(6*R*)-3-(3-chloro-2-methoxyanilino)-2-(3-{[(2*S*)-4-(2,2-difluoroethyl)morpholin-2-yl]methoxy}pyridin-4-yl)-6-methyl-1,5,6,7-tetrahydro-4*H*-pyrrolo[3,2-c]pyridin-4-one,
3-(3-chloro-2-methoxyanilino)-7-(2-hydroxyethyl)-2-[3-(2-methoxy-2-methylpropoxy)pyridin-4-yl]-1,5,6,7-tetrahydro-4*H*-pyrrolo[3,2-c]pyridin-4-one,
(7*R*)-3-(3-chloro-2-methoxyanilino)-7-(2-hydroxyethyl)-2-[3-(2-methoxy-2-methylpropoxy)pyridin-4-yl]-1,5,6,7-tetrahydro-4*H*-pyrrolo[3,2-c]pyridin-4-one,
(7*S*)-3-(3-chloro-2-methoxyanilino)-7-(2-hydroxyethyl)-2-[3-(2-methoxy-2-methylpropoxy)pyridin-4-yl]-1,5,6,7-tetrahydro-4*H*-pyrrolo[3,2-c]pyridin-4-one,
3-(3-chloro-2-methoxyanilino)-7-(2-hydroxyethyl)-2-(3-{[(2*S*)-oxolan-2-yl]methoxy}pyridin-4-yl)-1,5,6,7-tetrahydro-4*H*-pyrrolo[3,2-c]pyridin-4-one,
(7*R*)-3-(3-chloro-2-methoxyanilino)-7-(2-hydroxyethyl)-2-(3-{[(2*S*)-oxolan-2-yl]methoxy}pyridin-4-yl)-1,5,6,7-tetrahydro-4*H*-pyrrolo[3,2-c]pyridin-4-one,
(7*S*)-3-(3-chloro-2-methoxyanilino)-7-(2-hydroxyethyl)-2-(3-{[(2*S*)-oxolan-2-yl]methoxy}pyridin-4-yl)-1,5,6,7-tetrahydro-4*H*-pyrrolo[3,2-c]pyridin-4-one,
3-(3-chloro-2-methoxyanilino)-7-(2-hydroxyethyl)-2-{3-[(2-methyloxetan-2-yl)methoxy]pyridin-4-yl}-1,5,6,7-tetrahydro-4*H*-pyrrolo[3,2-c]pyridin-4-one,
(7*S*)-3-(3-chloro-2-methoxyanilino)-7-(2-hydroxyethyl)-2-(3-{[(2*S*)-2-methyloxetan-2-yl]methoxy}pyridin-4-yl)-1,5,6,7-tetrahydro-4*H*-pyrrolo[3,2-c]pyridin-4-one,
(7*R*)-3-(3-chloro-2-methoxyanilino)-7-(2-hydroxyethyl)-2-(3-{[(2*S*)-2-methyloxetan-2-yl]methoxy}pyridin-4-yl)-1,5,6,7-tetrahydro-4*H*-pyrrolo[3,2-c]pyridin-4-one,
(7*S*)-3-(3-chloro-2-methoxyanilino)-7-(2-hydroxyethyl)-2-(3-{[(2*R*)-2-methyloxetan-2-yl]methoxy}pyridin-4-yl)-1,5,6,7-tetrahydro-4*H*-pyrrolo[3,2-c]pyridin-4-one,
(7*R*)-3-(3-chloro-2-methoxyanilino)-7-(2-hydroxyethyl)-2-(3-{[(2*R*)-2-methyloxetan-2-yl]methoxy}pyridin-4-yl)-1,5,6,7-tetrahydro-4*H*-pyrrolo[3,2-c]pyridin-4-one,
(6*R*)-3-(3-chloro-2-methoxy-anilino)-2-[3-(1,4-dioxan-2-ylmethoxy)-4-pyridyl]-6-methyl-1,5,6,7-tetrahydropyrrolo[3,2-c]pyridin-4-one,
(6*R*)-3-(3-chloro-2-methoxy-anilino)-2-{3-[(2*S*)-1,4-dioxan-2-ylmethoxy]-4-pyridyl}-6-methyl-1,5,6,7-tetrahydropyrrolo[3,2-c]pyridin-4-one,
(6*R*)-3-(3-chloro-2-methoxy-anilino)-2-{3-[(2*R*)-1,4-dioxan-2-ylmethoxy]-4-pyridyl}-6-methyl-1,5,6,7-tetrahydropyrrolo[3,2-c]pyridin-4-one,
(6*R*)-3-(3-chloro-2-methoxy-anilino)-6-methyl-2-[3-(tetrahydropyran-2-ylmethoxy)-4-pyridyl]-1,5,6,7-tetrahydropyrrolo[3,2-c]pyridin-4-one,
(6*R*)-3-(3-chloro-2-methoxy-anilino)-6-methyl-2-{3-[(2*S*)-tetrahydropyran-2-ylmethoxy]-4-pyridyl}-1,5,6,7-tetrahydropyrrolo[3,2-c]pyridin-4-one,
(6*R*)-3-(3-chloro-2-methoxy-anilino)-6-methyl-2-{3-[(2*R*)-tetrahydropyran-2-ylmethoxy]-4-pyridyl}-1,5,6,7-tetrahydropyrrolo[3,2-c]pyridin-4-one,
3-(3-chloro-2-methoxy-anilino)-7-methyl-2-[3-[[(2*S*)-tetrahydrofuran-2-yl]methoxy]-4-pyridyl]-1,5,6,7-tetrahydropyrrolo[3,2-c]pyridin-4-one,
(7*S*)-3-(3-chloro-2-methoxy-anilino)-7-methyl-2-[3-[[(2*S*)-tetrahydrofuran-2-yl]methoxy]-4-pyridyl]-1,5,6,7-tetrahydropyrrolo[3,2-c]pyridin-4-one,
(7*R*)-3-(3-chloro-2-methoxy-anilino)-7-methyl-2-[3-[[(2*S*)-tetrahydrofuran-2-yl]methoxy]-4-pyridyl]-1,5,6,7-tetrahydropyrrolo[3,2-c]pyridin-4-one,
3-(3-chloro-2-methoxy-anilino)-2-[3-(1,4-dioxan-2-ylmethoxy)-4-pyridyl]-7-methyl-1,5,6,7-tetrahydropyrrolo[3,2-c]pyridin-4-one,
(7*S*)-3-(3-chloro-2-methoxy-anilino)-2-{3-[(2*S*)-1,4-dioxan-2-ylmethoxy]-4-pyridyl}-7-methyl-1,5,6,7-tetrahydropyrrolo[3,2-c]pyridin-4-one,
(7*S*)-3-(3-chloro-2-methoxy-anilino)-2-{3-[(2*R*)-1,4-dioxan-2-ylmethoxy]-4-pyridyl}-7-methyl-1,5,6,7-tetrahydropyrrolo[3,2-c]pyridin-4-one,
(7*R*)-3-(3-chloro-2-methoxy-anilino)-2-{3-[(2*S*)-1,4-dioxan-2-ylmethoxy]-4-pyridyl}-7-methyl-1,5,6,7-tetrahydropyrrolo[3,2-c]pyridin-4-one,
(7*R*)-3-(3-chloro-2-methoxy-anilino)-2-{3-[(2*R*)-1,4-dioxan-2-ylmethoxy]-4-pyridyl}-7-methyl-1,5,6,7-tetrahydropyrrolo[3,2-c]pyridin-4-one,
3-(3-chloro-2-methoxy-anilino)-2-[3-[[(2*S*)-tetrahydrofuran-2-yl]methoxy]-4-pyridyl]-6-(trifluoromethyl)-1,5,6,7-tetrahydropyrrolo[3,2-c]pyridin-4-one,
(6*S*)-3-(3-chloro-2-methoxy-anilino)-2-[3-[[(2*S*)-tetrahydrofuran-2-yl]methoxy]-4-pyridyl]-6-(trifluoromethyl)-1,5,6,7-tetrahydropyrrolo[3,2-c]pyridin-4-one,
(6*R*)-3-(3-chloro-2-methoxy-anilino)-2-{3-[(1-methoxypropan-2-yl)oxy]-4-pyridyl}-6-methyl-1,5,6,7-tetrahydropyrrolo[3,2-c]pyridin-4-one,
(6*R*)-3-(3-chloro-2-methoxy-anilino)-2-(3-{[(2*S*)-1-methoxypropan-2-yl]oxy}-4-pyridyl)-6-methyl-1,5,6,7-tetrahydropyrrolo[3,2-c]pyridin-4-one,
(6*R*)-3-(3-chloro-2-methoxy-anilino)-2-(3-{[(2*R*)-1-methoxypropan-2-yl]oxy}-4-pyridyl)-6-methyl-1,5,6,7-tetrahydropyrrolo[3,2-c]pyridin-4-one,
3-(3-chloro-2-methoxy-anilino)-7-methyl-2-[3-(tetrahydropyran-2-ylmethoxy)-4-pyridyl]-1,5,6,7-tetrahydropyrrolo[3,2-c]pyridin-4-one,
(7*S*)-3-(3-chloro-2-methoxy-anilino)-7-methyl-2-{3-[(2*S*)-tetrahydropyran-2-ylmethoxy]-4-pyridyl}-1,5,6,7-tetrahydropyrrolo[3,2-c]pyridin-4-one,
(7*S*)-3-(3-chloro-2-methoxy-anilino)-7-methyl-2-{3-[(2*R*)-tetrahydropyran-2-ylmethoxy]-4-pyridyl}-1,5,6,7-tetrahydropyrrolo[3,2-c]pyridin-4-one,
(7*R*)-3-(3-chloro-2-methoxy-anilino)-7-methyl-2-{3-[(2*S*)-tetrahydropyran-2-ylmethoxy]-4-pyridyl}-1,5,6,7-tetrahydropyrrolo[3,2-c]pyridin-4-one,
(7*R*)-3-(3-chloro-2-methoxy-anilino)-7-methyl-2-{3-[(2*R*)-tetrahydropyran-2-ylmethoxy]-4-pyridyl}-1,5,6,7-tetrahydropyrrolo[3,2-c]pyridin-4-one,
(5a*R*,8a*S*)-3-(3-chloro-2-methoxyanilino)-2-{3-[(1-methoxypropan-2-yl)oxy]-4-pyridyl}-5,5a,6,7,8,8a-hexahydrocyclopenta[b]pyrrolo[2, 3-d]pyridin-4(1*H*)-one,
(5a*R*,8a*S*)-3-(3-chloro-2-methoxyanilino)-2-(3-{[(2*S*)-1-methoxypropan-2-yl]oxy}-4-pyridyl)-5,5a,6,7,8,8a-hexahydrocyclopenta[b]pyrrolo[2,3-d]pyridin-4(1*H*)-one,
(5a*R*,8a*S*)-3-(3-chloro-2-methoxyanilino)-2-(3-{[(2*R*)-1-methoxypropan-2-yl]oxy}-4-pyridyl)-5,5a,6,7,8,8a-hexahydrocyclopenta[b]pyrrolo[2,3-d]pyridin-4(1*H*)-one,
(5a*R*,8a*S*)-3-(3-chloro-2-methoxyanilino)-2-[3-(1,4-dioxan-2-ylmethoxy)-4-pyridyl]-5,5a,6,7,8,8a-hexahydrocyclopenta[b]pyrrolo[2,3-d]pyridin-4(1*H*)-one,
(5a*R*,8a*S*)-3-(3-chloro-2-methoxyanilino)-2-{3-[(2*S*)-1,4-dioxan-2-ylmethoxy]-4-pyridyl}-5,5a,6,7,8,8a-hexahydrocyclopenta[b]pyrrolo[2, 3-d]pyridin-4(1*H*)-one,
(5a*R*,8a*S*)-3-(3-chloro-2-methoxyanilino)-2-{3-[(2*R*)-1,4-dioxan-2-ylmethoxy]-4-pyridyl}-5,5a,6,7,8,8a-hexahydrocyclopenta[b]pyrrolo[2,3-d]pyridin-4(1*H*)-one,
(5a*R**,8a*S**)-3-(3-chloro-2-methoxyanilino)-2-(3-{[(2*S*)-oxolan-2-yl]methoxy}-4-pyridyl)-5,5a,6,7,8,8a-hexahydrocyclopenta[b]pyrrolo[2,3-d]pyridin-4(1H)-one,
(5a*R*,8a*S*)-3-(3-chloro-2-methoxyanilino)-2-(3-{[(2*S*)-oxolan-2-yl]methoxy}-4-pyridyl)-5,5a,6,7,8,8a-hexahydrocyclopenta[b]pyrrolo[2,3-d]pyridin-4(1H)-one,
3-(3-chloro-2-methyl-anilino)-7-methyl-2-(3-{[(2*S*)-oxolan-2-yl]methoxy}pyridin-4-yl)-1,5,6,7-tetrahydropyrrolo[3,2-c]pyridin-4-one,
(7*S*)-3-(3-chloro-2-methyl-anilino)-7-methyl-2-(3-{[(2*S*)-oxolan-2-yl]methoxy}pyridin-4-yl)-1,5,6,7-tetrahydropyrrolo[3,2-c]pyridin-4-one,
(7*R*)-3-(3-chloro-2-methyl-anilino)-7-methyl-2-(3-{[(2*S*)-oxolan-2-yl]methoxy}pyridin-4-yl)-1,5,6,7-tetrahydropyrrolo[3,2-c]pyridin-4-one,
3-(3-chloro-2-methyl-anilino)-2-[3-[(3,3-difluorotetrahydropyran-2-yl)methoxy]-4-pyridyl]-7-methyl-1,5,6,7-tetrahydropyrrolo[3,2-c]pyridin-4-one,
3-(3-chloro-2-methyl-anilino)-2-[3-[(5,5-dimethyl-1,4-dioxan-2-yl)methoxy]-4-pyridyl]-7-methyl-1,5,6,7-tetrahydropyrrolo[3,2-c]pyridin-4-one,
(6*R*)-3-[2-(2,2-difluoroethyl)-3-fluoroanilino]-6-methyl-2-(3-{[(2*S*)-oxolan-2-yl]methoxy}pyridin-4-yl)-1,5,6,7-tetrahydro-4*H*-pyrrolo[3,2-c]pyridin-4-one,
(5a*R*,8a*S*)-3-(3-chloro-2-methoxyanilino)-2-(3-{[(2*S*)-4-methylmorpholin-2-yl]methoxy}pyridin-4-yl)-5,5a,6,7,8,8a-hexahydrocyclopenta[b]pyrrolo[2,3-d]pyridin-4(1*H*)-one,
(6*R*)-3-[2-(2,2-difluoroethyl)-3-fluoroanilino]-2-[3-(2-methoxy-2-methylpropoxy)pyridin-4-yl]-6-methyl-1,5,6,7-tetrahydro-4*H*-pyrrolo[3,2-c]pyridin-4-one,
3-(3-chloro-2-methyl-anilino)-2-[3-(1,4-dioxan-2-ylmethoxy)-4-pyridyl]-7-methyl-1,5,6,7-tetrahydropyrrolo[3,2-c]pyridin-4-one,
(7*S*)-3-(3-chloro-2-methyl-anilino)-2-[3-[(2*S*)-1,4-dioxan-2-ylmethoxy]-4-pyridyl]-7-methyl-1,5,6,7-tetrahydropyrrolo[3,2-c]pyridin-4-one,
(7*R*)-3-(3-chloro-2-methyl-anilino)-2-[3-[(2*R*)-1,4-dioxan-2-ylmethoxy]-4-pyridyl]-7-methyl-1,5,6,7-tetrahydropyrrolo[3,2-c]pyridin-4-one,
(7*S*)-3-(3-chloro-2-methyl-anilino)-2-[3-[(2*R*)-1,4-dioxan-2-ylmethoxy]-4-pyridyl]-7-methyl-1,5,6,7-tetrahydropyrrolo[3,2-c]pyridin-4-one,
(7*R*)-3-(3-chloro-2-methyl-anilino)-2-[3-[(2*S*)-1,4-dioxan-2-ylmethoxy]-4-pyridyl]-7-methyl-1,5,6,7-tetrahydropyrrolo[3,2-c]pyridin-4-one,
(6*R*)-3-(3-chloro-2-methoxy-anilino)-2-[3-[(3,3-difluorotetrahydrofuran-2-yl)methoxy]-4-pyridyl]-6-methyl-1,5,6,7-tetrahydropyrrolo[3,2-c]pyridin-4-one,
(6*R*)-3-(3-chloro-2-methoxy-anilino)-2-(3-{[(2*S*)-3,3-difluorotetrahydrofuran-2-yl]methoxy}-4-pyridyl)-6-methyl-1,5,6,7-tetrahydropyrrolo[3,2-c]pyridin-4-one,
(6*R*)-3-(3-chloro-2-methoxy-anilino)-2-(3-{[(2*R*)-3,3-difluorotetrahydrofuran-2-yl]methoxy}-4-pyridyl)-6-methyl-1,5,6,7-tetrahydropyrrolo[3,2-c]pyridin-4-one,
(6*R*)-3-(3-chloro-2-methoxy-anilino)-2-[3-[(3,3-difluorotetrahydropyran-2-yl)methoxy]-4-pyridyl]-6-methyl-1,5,6,7-tetrahydropyrrolo[3,2-c]pyridin-4-one,
(6*R*)-3-(3-chloro-2-methoxy-anilino)-2-(3-{[(2*S*)-3,3-difluorotetrahydropyran-2-yl]methoxy}-4-pyridyl)-6-methyl-1,5,6,7-tetrahydropyrrolo[3,2-c]pyridin-4-one,
(6*R*)-3-(3-chloro-2-methoxy-anilino)-2-(3-{[(2*R*)-3,3-difluorotetrahydropyran-2-yl]methoxy}-4-pyridyl)-6-methyl-1,5,6,7-tetrahydropyrrolo[3,2-c]pyridin-4-one,
(6*R*)-3-(3-chloro-2-methoxy-anilino)-2-[3-[(5,5-dimethyl-1,4-dioxan-2-yl)methoxy]-4-pyridyl]-6-methyl-1,5,6,7-tetrahydropyrrolo[3,2-c]pyridin-4-one,
(6*R*)-3-(3-chloro-2-methoxy-anilino)-2(3-{[(2*S*)-5,5-dimethyl-1,4-dioxan-2-yl]methoxy}-4-pyridyl)-6-methyl-1,5,6,7-tetrahydropyrrolo[3,2-c]pyridin-4-one,
(6*R*)-3-(3-chloro-2-methoxy-anilino)-2(3-{[(2*R*)-5,5-dimethyl-1,4-dioxan-2-yl]methoxy}-4-pyridyl)-6-methyl-1,5,6,7-tetrahydropyrrolo[3,2-c]pyridin-4-one,
(6*R*)-3-(3-chloro-2-methoxy-anilino)-2-[3-(2-methoxypropoxy)-4-pyridyl]-6-methyl-1,5,6,7-tetrahydropyrrolo[3,2-c]pyridin-4-one,
(6*R*)-3-(3-chloro-2-methoxy-anilino)-2-{3-[(2*R*)-2-methoxypropoxy]-4-pyridyl}-6-methyl-1,5,6,7-tetrahydropyrrolo[3,2-c]pyridin-4-one,
(6*R*)-3-(3-chloro-2-methoxy-anilino)-2-{3-[(2*S*)-2-methoxypropoxy]-4-pyridyl}-6-methyl-1,5,6,7-tetrahydropyrrolo[3,2-c]pyridin-4-one,
(6*R*)-3-(3-chloro-2-methoxy-anilino)-6-methyl-2-[3-[(1-methyl-2-piperidyl)methoxy]-4-pyridyl]-1,5,6,7-tetrahydropyrrolo[3,2-c]pyridin-4-one,
(6*R*)-3-(3-chloro-2-methoxy-anilino)-6-methyl-2-(3-{[(2*S*)-1-methyl-2-piperidyl]methoxy}-4-pyridyl)-1,5,6,7-tetrahydropyrrolo[3,2-c]pyridin-4-one,
(6*R*)-3-(3-chloro-2-methoxy-anilino)-6-methyl-2-(3-{[(2*R*)-1-methyl-2-piperidyl]methoxy}-4-pyridyl)-1,5,6,7-tetrahydropyrrolo[3,2-c]pyridin-4-one,
(6*R*)-3-(3-chloro-2-methoxy-anilino)-6-methyl-2-[3-(2-tetrahydropyran-2-ylethoxy)-4-pyridyl]-1,5,6,7-tetrahydropyrrolo[3,2-c]pyridin-4-one,
(6*R*)-3-(3-chloro-2-methoxy-anilino)-6-methyl-2-(3-{2-[(2*S*)-tetrahydropyran-2-yl]ethoxy}-4-pyridyl)-1,5,6,7-tetrahydropyrrolo[3,2-c]pyridin-4-one,
(6*R*)-3-(3-chloro-2-methoxy-anilino)-6-methyl-2-(3-{2-[(2*R*)-tetrahydropyran-2-yl]ethoxy}-4-pyridyl)-1,5,6,7-tetrahydropyrrolo[3,2-c]pyridin-4-one,
(7*S*)-3-(3-chloro-2-methoxyanilino)-7-(2-fluoroethyl)-2-(3-{[(3*S*)-4-methylmorpholin-3-yl]methoxy}pyridin-4-yl)-1,5,6,7-tetrahydro-4*H*-pyrrolo[3,2-c]pyridin-4-one,
(7*R*)-3-(3-chloro-2-methoxyanilino)-7-(2-fluoroethyl)-2-(3-{[(3*S*)-4-methylmorpholin-3-yl]methoxy}pyridin-4-yl)-1,5,6,7-tetrahydro-4*H*-pyrrolo[3,2-c]pyridin-4-one,
(6*R*)-3-(3-chloro-2-ethylanilino)-6-methyl-2-(3-{[(2*S*)-4-methylmorpholin-2-yl]methoxy}pyridin-4-yl)-1,5,6,7-tetrahydro-4*H*-pyrrolo[3,2-c]pyridin-4-one,
(6*R*)-3-(3-chloro-2-methoxyanilino)-6-methyl-2-(3-{2-[(2*S*)-oxolan-2-yl]ethoxy}pyridin-4-yl)-1,5,6,7-tetrahydro-4*H*-pyrrolo[3,2-c]pyridin-4-one,
(6*R*)-3-(3-chloro-2-methoxyanilino)-6-methyl-2-(3-{[(3*S*)-4-methylmorpholin-3-yl]methoxy}pyridin-4-yl)-1,5,6,7-tetrahydro-4*H*-pyrrolo[3,2-c]pyridin-4-one,
(7*S*)-3-(3-chloro-2-methoxy-anilino)-2-(3-{[(2*S*)-5,5-dimethyl-1,4-dioxan-2-yl]methoxy}-4-pyridyl)-7-methyl-1,5,6,7-tetrahydropyrrolo[3,2-c]pyridin-4-one,
(7*S*)-3-(3-chloro-2-methoxy-anilino)-2-(3-{[(2*R*)-5,5-dimethyl-1,4-dioxan-2-yl]methoxy}-4-pyridyl)-7-methyl-1,5,6,7-tetrahydropyrrolo[3,2-c]pyridin-4-one,
(7*R*)-3-(3-chloro-2-methoxy-anilino)-2-(3-{[(2S)-5,5-dimethyl-1,4-dioxan-2-yl]methoxy}-4-pyridyl)-7-methyl-1,5,6,7-tetrahydropyrrolo[3,2-c]pyridin-4-one,
(7*R*)-3-(3-chloro-2-methoxy-anilino)-2-(3-{[(2*R*)-5,5-dimethyl-1,4-dioxan-2-yl]methoxy}-4-pyridyl)-7-methyl-1,5,6,7-tetrahydropyrrolo[3,2-c]pyridin-4-one,
(7*S*)-3-(3-chloro-2-methoxy-anilino)-2-(3-{[(2*S*)-tetrahydrofuran-2-yl]methoxy}-4-pyridyl)-7-(3,3,3-trifluoropropyl)-1,5,6,7-tetrahydropyrrolo[3,2-c]pyridin-4-one,
(7*R*)-3-(3-chloro-2-methoxy-anilino)-2-(3-{[(2*S*)-tetrahydrofuran-2-yl]methoxy}-4-pyridyl)-7-(3,3,3-trifluoropropyl)-1,5,6,7-tetrahydropyrrolo[3,2-c]pyridin-4-one,
(7*S*)-3-(3-chloro-2-methoxy-anilino)-7-(3,3-difluoroallyl)-2-(3-{[(2*S*)-tetrahydrofuran-2-yl]methoxy}-4-pyridyl)-1,5,6,7-tetrahydropyrrolo[3,2-c]pyridin-4-one,
(7*R*)-3-(3-chloro-2-methoxy-anilino)-7-(3,3-difluoroallyl)-2-(3-{[(2*S*)-tetrahydrofuran-2-yl]methoxy}-4-pyridyl)-1,5,6,7-tetrahydropyrrolo[3,2-c]pyridin-4-one,
(6*R*)-3-(3-chloro-2-methylanilino)-2-{3-[(1,4-dioxan-2-yl)methoxy]pyridin-4-yl}-6-methyl-1,5,6,7-tetrahydro-4*H*-pyrrolo[3,2-c]pyridin-4-one,
(6*R*)-3-(3-chloro-2-methylanilino)-2-(3-{[(2*S*)-1,4-dioxan-2-yl]methoxy}pyridin-4-yl)-6-methyl-1,5,6,7-tetrahydro-4*H*-pyrrolo[3,2-c]pyridin-4-one,
(6*R*)-3-(3-chloro-2-methylanilino)-6-methyl-2-(3-{[(2*S*)-oxolan-2-yl]methoxy}pyridin-4-yl)-1,5,6,7-tetrahydro-4*H*-pyrrolo[3,2-c]pyridin-4-one,
(6*R*)-3-(3-chloro-2-methylanilino)-2-{3-[(1-methoxypropan-2-yl)oxy]pyridin-4-yl}-6-methyl-1,5,6,7-tetrahydro-4*H*-pyrrolo[3,2-c]pyridin-4-one,
(6*R*)-3-(3-chloro-2-methylanilino)-6-methyl-2-(3-{[(2*R*)-4-methylmorpholin-2-yl]methoxy}pyridin-4-yl)-1,5,6,7-tetrahydro-4*H*-pyrrolo[3,2-c]pyridin-4-one,
(6*R*)-3-(3-chloro-2-methylanilino)-6-methyl-2-(3-{[(2*S*)-4-methylmorpholin-2-yl]methoxy}pyridin-4-yl)-1,5,6,7-tetrahydro-4*H*-pyrrolo[3,2-c]pyridin-4-one,
(6*R*)-3-[2-(2,2-difluoroethyl)-3-fluoro-anilino]-2-[3-(1,4-dioxan-2-ylmethoxy)-4-pyridyl]-6-methyl-1,5,6,7-tetrahydropyrrolo[3,2-c]pyridin-4-one,
(6*R*)-3-(3-chloro-2-ethylanilino)-6-methyl-2-(3-{[(2*S*)-oxolan-2-yl]methoxy}pyridin-4-yl)-1,5,6,7-tetrahydro-4*H*-pyrrolo[3,2-c]pyridin-4-one,
(6*R*)-3-(3-chloro-2-ethylanilino)-2-(3-{[(2*S*)-1,4-dioxan-2-yl]methoxylpyridin-4-yl)-6-methyl-1,5,6,7-tetrahydro-4*H*-pyrrolo[3,2-c]pyridin-4-one,
3'-(3-chloro-2-methoxyanilino)-2'-(3-{[(2*S*)-1,4-dioxan-2-yl]methoxy}pyridin-4-yl)-1',7'-dihydrospiro[cyclopropane-1,6'-pyrrolo[3,2-c]pyridin]-4'(5'*H*)-one,
3'-(3-chloro-2-methoxyanilino)-2'-(3-{[(2*S*)-4-methylmorpholin-2-yl]methoxy}pyridin-4-yl)-1',7'-dihydrospiro[cyclopropane-1,6'-pyrrolo[3,2-c]pyridin]-4'(5'H)-one,
3'-(3-chloro-2-methoxyanilino)-2'-(3-{[(2*S*)-oxolan-2-yl]methoxy}pyridin-4-yl)-1',7'-dihydrospiro[cyclopropane-1,6'-pyrrolo[3,2-c]pyridin]-4'(5'*H*)-one,
7-allyl-2-[3-(2-allyloxyethoxy)-4-pyridyl]-3-(3-chloro-2-methoxy-anilino)-1,5,6,7-tetrahydropyrrolo[3,2-c]pyridin-4-one,
(7*S*)-3-(3-chloro-2-methoxyanilino)-7-(2-hydroxyethyl)-2-(3-{[(3*S*)-4-methylmorpholin-3-yl]methoxy}pyridin-4-yl)-1,5,6,7-tetrahydro-4H-pyrrolo[3,2-c]pyridin-4-one,
(7*R*)-3-(3-chloro-2-methoxyanilino)-7-(2-hydroxyethyl)-2-(3-{[(3*S*)-4-methylmorpholin-3-yl]methoxy}pyridin-4-yl)-1,5,6,7-tetrahydro-4H-pyrrolo[3,2-c]pyridin-4-one,
3-(3-chloro-2-methyl-anilino)-7-methyl-2-[3-(tetrahydropyran-2-ylmethoxy)-4-pyridyl]-1,5,6,7-tetrahydropyrrolo[3,2-c]pyridin-4-one,
(7*S*)-3-(3-chloro-2-methyl-anilino)-7-methyl-2-(3-{[(2*S*)-tetrahydropyran-2-yl]methoxy}-4-pyridyl)-1,5,6,7-tetrahydropyrrolo[3,2-c]pyridin-4-one,
(7*R*)-3-(3-chloro-2-methyl-anilino)-7-methyl-2-(3-{[(2*S*)-tetrahydropyran-2-yl]methoxy}-4-pyridyl)-1,5,6,7-tetrahydropyrrolo[3,2-c]pyridin-4-one,
(6*R*)-3-(3-chloro-2-methoxy-anilino)-2-[3-(3-methoxybutoxy)-4-pyridyl]-6-methyl-1,5,6,7-tetrahydropyrrolo[3,2-c]pyridin-4-one,
(6*R*)-3-(3-chloro-2-methoxy-anilino)-2-{3-[(3*S*)-3-methoxybutoxy]-4-pyridyl}-6-methyl-1,5,6,7-tetrahydropyrrolo[3,2-c]pyridin-4-one,
(6*R*)-3-(3-chloro-2-methoxy-anilino)-2-{3-[(3*R*)-3-methoxybutoxy]-4-pyridyl}-6-methyl-1,5,6,7-tetrahydropyrrolo[3,2-c]pyridin-4-one,
3-(3-chloro-2-methoxy-anilino)-2-[3-(1,4-dioxan-2-ylmethoxy)-4-pyridyl]spiro[5,7-dihydro-1H-pyrrolo[3,2-c]pyridine-6,1'-cyclobutane]-4-one,
3-(3-chloro-2-methoxy-anilino)-2-{3-[(2*S*)-1,4-dioxan-2-ylmethoxy]-4-pyridyl}spiro[5,7-dihydro-1*H*-pyrrolo[3,2-c]pyridine-6,1'-cyclobutane]-4-one,
3-(3-chloro-2-methoxy-anilino)-2-{3-[(2*R*)-1,4-dioxan-2-ylmethoxy]-4-pyridyl}spiro[5,7-dihydro-1*H*-pyrrolo[3,2-c]pyridine-6,1'-cyclobutane]-4-one,
3'-(3-chloro-2-methoxyanilino)-2'-(3-{[(2*S*)-oxolan-2-yl]methoxy}pyridin-4-yl)-1',7'-dihydrospiro[cyclobutane-1,6'-pyrrolo[3,2-c]pyridin]-4'(5'*H*)-one,
3'-(3-chloro-2-methoxyanilino)-2'-(3-{[(2*S*)-oxan-2-yl]methoxy}pyridin-4-yl)-1',7'-dihydrospiro[cyclobutane-1,6'-pyrrolo[3,2-c]pyridin]-4'(5'*H*)-one,
3-(3-chloro-2-methoxy-anilino)-2-[3-(2-tetrahydropyran-2-ylethoxy)-4-pyridyl]spiro[5,7-dihydro-1*H*-pyrrolo[3,2-c]pyridine-6,1'-cyclobutane]-4-one,
3'-(3-chloro-2-methoxyanilino)-2'-{3-[(5,5-dimethyl-1,4-dioxan-2-yl)methoxy]pyridin-4-yl}-1',7'-dihydrospiro[cyclobutane-1,6'-pyrrolo[3,2-c]pyridin]-4'(5'*H*)-one,
3'-(3-chloro-2-methoxyanilino)-2'-(3-{[(2*R*)-5,5-dimethyl-1,4-dioxan-2-yl]methoxy}pyridin-4-yl)-1',7'-dihydrospiro[cyclobutane-1,6'-pyrrolo[3,2-c]pyridin]-4'(5'*H*)-one,
3'-(3-chloro-2-methoxyanilino)-2'-(3-{[(2*S*)-5,5-dimethyl-1,4-dioxan-2-yl]methoxy}pyridin-4-yl)-1',7'-dihydrospiro[cyclobutane-1,6'-pyrrolo[3,2-c]pyridin]-4'(5'*H*)-one,
3'-(3-chloro-2-methoxyanilino)-2'-{3-[(5,5-dimethyl-1,4-dioxan-2-yl)methoxy]pyridin-4-yl}-1',7'-dihydrospiro[cyclopropane-1,6'-pyrrolo[3,2-c]pyridin]-4'(5'*H*)-one,
3'-(3-chloro-2-methoxyanilino)-2'-(3-{[(2*R*)-5,5-dimethyl-1,4-dioxan-2-yl]methoxy}pyridin-4-yl)-1',7'-dihydrospiro[cyclopropane-1,6'-pyrrolo[3,2-c]pyridin]-4'(5'*H*)-one, and
3'-(3-chloro-2-methoxyanilino)-2'-(3-{[(2*S*)-5,5-dimethyl-1,4-dioxan-2-yl]methoxy}pyridin-4-yl)-1',7'-dihydrospiro[cyclopropane-1,6'-pyrrolo[3,2-c]pyridin]-4'(5'*H*)-one.

5. A compound of general formula (I) according to any of claims 1 to 4 for use in the treatment or prophylaxis of diseases.

6. The compound for use according to claim 5, wherein the diseases are hyperproliferative diseases and/or disorders responsive to induction of cell death.

7. The compound for use according to claim 6, wherein the hyperproliferative diseases and/or disorders responsive to induction of cell death are haematological tumours, solid tumours and/or metastases thereof.

8. The compound for use according to claim 7, wherein the tumour harbors a mutant EGFR and/or metastases thereof.

9. The compound for use according to claim 7, wherein the tumour is lung cancer, particularly lung cancer harboring a mutant EGFR with exon 20 insertion mutation, and/or metastases thereof.

10. The compound for use according to claim 7, wherein the tumour is lung cancer, particularly lung cancer harboring a mutant EGFR with in-frame deletions in exon 19 (such as EGFR E746_A750del) or point mutations in exon 21 (e.g. L858R), and/or metastases thereof.

11. The compound for use according to claim 7, wherein the tumour is lung cancer, particularly lung cancer harboring a mutant EGFR with a D770_N771insSVD C797S, E746_A750del C797S, or L858R C797S acquired resistance mutation, and/or metastases thereof.

12. The compound for use according to claim 7, wherein the tumour is lung cancer, particularly lung cancer harboring a mutant ERBB2 with exon 20 insertion mutations (such as ERBB2 A775_G776insYVMA), and/or metastases thereof.

13. A pharmaceutical composition comprising at least one compound of general formula (I) according to any of claims 1 to 4, together with at least one pharmaceutically acceptable auxiliary.

14. The composition according to claim 13 for the treatment of haematological tumours, solid tumours and/or metastases thereof.

15. A combination comprising one or more first active ingredients selected from a compound of general formula (I) according to any of claims 1 to 4, and one or more second active ingredients selected from chemotherapeutic anti-cancer agents and target-specific anti-cancer agents.

## Patentansprüche

1. Eine Verbindung der Formel (I) 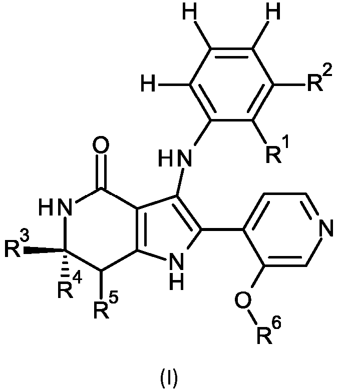 in welcher:
R¹ stellt Methyl, Ethyl, Trifluormethyl, 2,2-Difluorethyl, Cyano, Chlor, Brom, Methoxy oder Difluormethoxy dar;
R² stellt Methyl, Ethyl, Fluor, Chlor oder Brom dar;
R³ stellt Wasserstoff, Methyl oder Trifluormethyl dar;
R⁴ stellt Wasserstoff dar; oder
R³ und R⁴ bilden zusammen mit dem Kohlenstoffatom, an das sie gebunden sind, einen 3-bis 5-gliedrigen Cycloalkylring;
R⁵ stellt Wasserstoff, C₁-C₃-Alkyl oder C₂-C₃-Alkenyl dar, wobei die Alkylgruppen gegebenenfalls ein- oder mehrfach, unabhängig voneinander, mit Fluor, Hydroxy oder Methoxy substituiert sind und die Alkenylgruppen gegebenenfalls ein- oder mehrfach mit Fluor substituiert sind; oder
R³ und R⁵ zusammen mit den Kohlenstoffatomen, an die sie gebunden sind, einen 4- bis 6-gliedrigen Cycloalkylring bilden;
Mit der Maßgabe, dass mindestens einer der Reste R³ und R⁵ von Wasserstoff verschieden ist;
R⁶ stellt C₂-C₅-Alkyl dar, das einmal mit Hydroxy, C₁-C₄-Alkoxy oder C₂-C₃-Alkenyloxy substituiert ist,
oder eine Gruppe, ausgewählt aus der Gruppe: 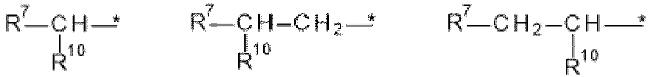
wobei * den Bindungspunkt der Gruppe mit dem Rest des Moleküls angibt;
R⁷ stellt eine Gruppe dar, die ausgewählt ist aus der Gruppe: 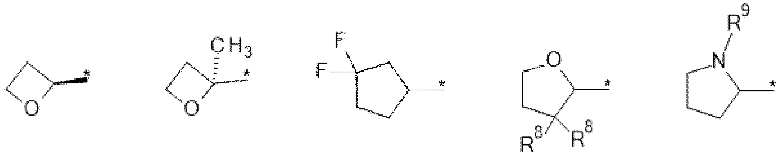 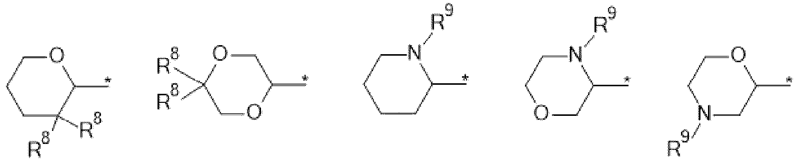 wobei * den Punkt der Bindung der Gruppe an den Rest des Moleküls angibt;
R⁸ stellt unabhängig voneinander bei jedem Auftreten Wasserstoff, Methyl oder Fluor dar;
R⁹ stellt C₁-C₃-Alkyl oder C₂-C₃-Fluoralkyl dar;
R¹⁰ stellt Wasserstoff oder Methyl dar;
oder ein N-Oxid, ein Salz, ein Tautomer oder ein Stereoisomer der genannten Verbindung oder ein Salz des genannten N-Oxids, Tautomers oder Stereoisomers.

2. Die Verbindung der Formel (I) gemäß Anspruch 1, wobei:
R¹ stellt Methyl, Ethyl, Trifluormethyl, 2,2-Difluorethyl, Methoxy oder Difluormethoxy dar;
R² stellt Methyl, Fluor, Chlor oder Brom dar;
R³ stellt Wasserstoff, Methyl oder Trifluormethyl dar;
R⁴ stellt Wasserstoff dar; oder
R³ und R⁴ bilden zusammen mit dem Kohlenstoffatom, an das sie gebunden sind, einen 3-bis 5-gliedrigen Cycloalkylring;
R⁵ stellt Wasserstoff, C₁-C₃-Alkyl oder C₂-C₃-Alkenyl dar, wobei die Alkylgruppen gegebenenfalls ein-, zwei- oder dreifach, unabhängig voneinander, mit Fluor, Hydroxy oder Methoxy substituiert sind und die Alkenylgruppen gegebenenfalls ein-, zwei- oder dreifach mit Fluor substituiert sind; oder
R³ und R⁵ zusammen mit den Kohlenstoffatomen, an die sie gebunden sind, einen 5- bis 6-gliedrigen Cycloalkylring bilden;
Mit der Maßgabe, dass mindestens einer der Reste R³ und R⁵ von Wasserstoff verschieden ist;
R⁶ stellt C₂-C₅-Alkyl dar, das einmal mit Hydroxy, C₁-C₃-Alkoxy oder C₂-C₃-Alkenyloxy substituiert ist,
oder eine Gruppe, ausgewählt aus der Gruppe: 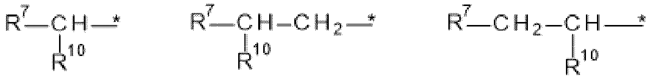
wobei * den Bindungspunkt der Gruppe mit dem Rest des Moleküls angibt;
R⁷ stellt eine Gruppe dar, die ausgewählt ist aus der Gruppe: 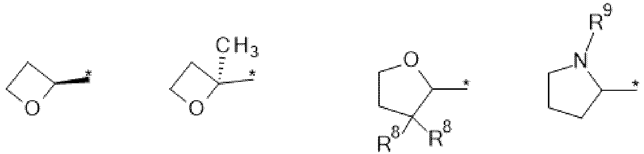 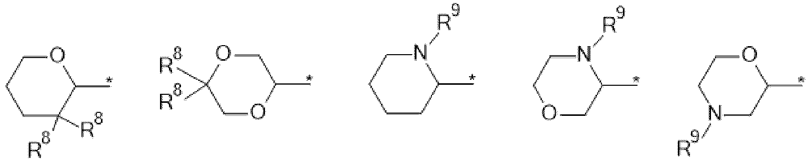 wobei * den Punkt der Bindung der Gruppe an den Rest des Moleküls angibt;
R⁸ stellt Wasserstoff, Methyl oder Fluor dar;
R⁹ stellt C₁-C₃-Alkyl oder C₂-C₃-Fluoralkyl dar;
R¹⁰ stellt Wasserstoff oder Methyl dar;
oder ein N-Oxid, ein Salz, ein Tautomer oder ein Stereoisomer der genannten Verbindung oder ein Salz des genannten N-Oxids, Tautomers oder Stereoisomers.

3. Die Verbindung der Formel (I) gemäß Anspruch 1 oder 2, wobei:
R¹ stellt Methyl, Ethyl, 2,2-Difluorethyl oder Methoxy dar;
R² stellt Fluor oder Chlor dar;
R³ stellt Wasserstoff, Methyl oder Trifluormethyl dar;
R⁴ stellt Wasserstoff dar; oder
R³ und R⁴ bilden zusammen mit dem Kohlenstoffatom, an das sie gebunden sind, einen 3-bis 4-gliedrigen Cycloalkylring;
R⁵ stellt Wasserstoff, C₁-C₃-Alkyl, Prop-2-en-1-yl oder 3,3-Difluorprop-2-en-1-yl dar, wobei die Alkylgruppen gegebenenfalls ein-, zwei- oder dreifach mit Fluor oder einmal mit Hydroxy substituiert sind; oder
R³ und R⁵ zusammen mit den Kohlenstoffatomen, an die sie gebunden sind, einen 5-gliedrigen Cycloalkylring bilden;
Mit der Maßgabe, dass mindestens einer der Reste R³ und R⁵ von Wasserstoff verschieden ist;
R⁶ stellt C₂-C₄-Alkyl dar, das einmal mit Hydroxy, Methoxy oder (Prop-2-en-1-yl)oxy substituiert ist,
oder eine Gruppe, ausgewählt aus der Gruppe: 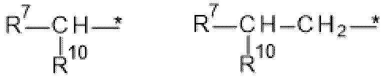
wobei * die Bindungsstelle der Gruppe mit dem Rest des Moleküls angibt;
R⁷ stellt eine Gruppe dar, die ausgewählt ist aus der Gruppe: 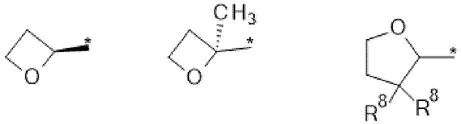 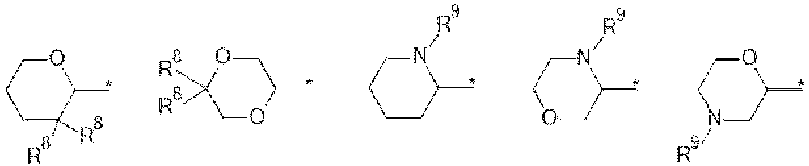 wobei * den Punkt der Bindung der Gruppe an den Rest des Moleküls angibt;
R⁸ stellt Wasserstoff, Methyl oder Fluor dar;
R⁹ stellt Methyl oder 2,2-Difluorethyl dar;
R¹⁰ stellt Wasserstoff dar;
oder ein N-Oxid, ein Salz, ein Tautomer oder ein Stereoisomer der genannten Verbindung oder ein Salz des genannten N-Oxids, Tautomers oder Stereoisomers.

4. Die Verbindung der Formel (I) nach einem der Ansprüche 1 bis 3, die ausgewählt ist aus der Gruppe bestehend aus:
(*6R*)-3-(3-chloro-2-methoxyanilino)-2-[3-(2-methoxy-2-methylpropoxy)pyridin-4-yl]-6-methyl-1,5,6,7-tetrahydro-4H-pyrrolo[3,2-c]pyridin-4-one,
3-(3-chloro-2-methoxyanilino)-6-methyl-2-(3-{[(*2S*)-oxolan-2-yl]methoxy}pyridin-4-yl)-1,5,6,7-*tetrahydro-4H-pyrrolo*[3,2-c]pyridin-4-one,
(*6R*)-3-(3-chloro-2-methoxyanilino)-6-methyl-2-(3-{[(2S)-oxolan-2-yl]methoxy}pyridin-4-yl)-1,5,6,7-tetrahydro-4H-pyrrolo[3,2-c]pyridin-4-one,
(*6R*)-3-(3-chloro-2-methoxyanilino)-6-methyl-2-(3-{[(*2S*)-oxetan-2-yl]methoxy}pyridin-4-yl)-1,5,6,7-tetrahydro-4H-pyrrolo[3,2-c]pyridin-4-one,
3-(3-fluoro-2-methoxyanilino)-6-methyl-2-(3-{[(*2S*)-oxolan-2-yl]methoxy}pyridin-4-yl)-1,5,6,7-*tetrahydro-4H-pyrrolo*[3,2-c]pyridin-4-one,
(*6R*)-3-(3-fluoro-2-methoxyanilino)-6-methyl-2-(3-{[(*2S*)-oxolan-2-yl]methoxy}pyridin-4-yl)-1,5,6,*7-tetrahydro*-*4H-pyrrolo*[3,2-c]pyridin-4-one*,*
(*6R*)-3-(3-chloro-2-methoxyanilino)-6-methyl-2-(3-{[(*2S*)-4-methylmorpholin-2-yl]methoxy}pyridin-4-yl)-1,5,6,*7-tetrahydro-4H-pyrrolo*[3,2-c]pyridin-4-one,
(*6R*)-3-(3-chloro-2-methoxyanilino)-2-(3-{[(*2S*)-4-(2,2-difluoroethyl)morpholin-2-yl]methoxy}pyridin-4-yl)-6-methyl-1,5,6*,7-tetrahydro-4H-pyrrolo*[3,2-c]pyridin-4-one,
3-(3-chloro-2-methoxyanilino)-7-(2-hydroxyethyl)-2-[3-(2-methoxy-2-methylpropoxy)pyridin-4-yl]-1,5,6,7-tetrahydro-4H-pyrrolo[3,2-c]pyridin-4-one,
(*7R*)-3-(3-chloro-2-methoxyanilino)-7-(2-hydroxyethyl)-2-[3-(2-methoxy-2-methylpropoxy)pyridin-4-yl]-1,5,6,*7*-tetrahydro-*4H*-*pyrrolo*[3,2-c]pyridin-4-one,
(*7S*)-3-(3-chloro-2-methoxyanilino)-7-(2-hydroxyethyl)-2-[3-(2-methoxy-2-methylpropoxy)pyridin-4-yl]-1,5,6,*7-tetrahydro-4H-pyrrolo*[3,2-c]pyridin-4-one,
3-(3-chloro-2-methoxyanilino)-7-(2-hydroxyethyl)-2-(3-{[(*2S*)-oxolan-2-yl]methoxy}pyridin-4-yl)-1,5,6,*7*-*tetrahydro-4H-pyrrolo*[3,2-c]pyridin-4-one,
(*7R*)-3-(3-chloro-2-methoxyanilino)-7-(2-hydroxyethyl)-2-(3-{[(*2S*)-oxolan-2-yl]methoxy}pyridin-4-yl)-1,5,6*,7-tetrahydro-4H-pyrrolo*[3,2-c]pyridin-4-one,
(*7S*)-3-(3-chloro-2-methoxyanilino)-7-(2-hydroxyethyl)-2-(3-{[(*2S*)-oxolan-2-yl]methoxy}pyridin-4-yl)-1,5,6*,7-tetrahydro-4H-pyrrolo*[3,2-c]pyridin-4-one,
3-(3-chloro-2-methoxyanilino)-7-(2-hydroxyethyl)-2-{3-[(2-methyloxetan-2-yl)methoxy]pyridin-4-yl}-1,5,6,*7-tetrahydro-4H-pyrrolo*[3,2-c]pyridin-4-one,
(*7S*)-3-(3-chloro-2-methoxyanilino)-7-(2-hydroxyethyl)-2-(3-{[(*2S)*-2-methyloxetan-2-yl]methoxy}pyridin-4-yl)-1,5,6,*7-tetrahydro-4H-pyrrolo*[3,2-c]pyridin-4-one,
(*7R*)-3-(3-chloro-2-methoxyanilino)-7-(2-hydroxyethyl)-2-(3-{[(*2S*)-2-methyloxetan-2-yl]methoxy}pyridin-4-yl)-1,5,6,*7-tetrahydro-4H-pyrrolo*[3,2-c]pyridin-4-one,
(*7S*)-3-(3-chloro-2-methoxyanilino)-7-(2-hydroxyethyl)-2-(3-{[(*2R*)-2-methyloxetan-2-yl]methoxy}pyridin-4-yl)-1,5,6,*7-tetrahydro-4H-pyrrolo*[3,2-c]pyridin-4-one,
(*7R*)-3-(3-chloro-2-methoxyanilino)-7-(2-hydroxyethyl)-2-(3-{[(*2R*)-2-methyloxetan-2-yl]methoxy}pyridin-4-yl)-1,5,6,*7-tetrahydro-4H-pyrrolo*[3,2-c]pyridin-4-one,
(*6R*)-3-(3-chloro-2-methoxy-anilino)-2-[3-(1,4-dioxan-2-ylmethoxy)-4-pyridyl]-6-methyl-1,5,6,7-tetrahydropyrrolo[3,2-c]pyridin-4-one,
(*6R*)-3-(3-chloro-2-methoxy-anilino)-2-{3-[(*2S*)-1,4-dioxan-2-ylmethoxy]-4-pyridyl}-6-methyl-1,5,6,7-tetrahydropyrrolo[3,2-c]pyridin-4-one,
(*6R*)-3-(3-chloro-2-methoxy-anilino)-2-{3-[(*2R*)-1,4-dioxan-2-ylmethoxy]-4-pyridyl}-6-methyl-1,5,6,7-tetrahydropyrrolo[3,2-c]pyridin-4-one,
(*6R*)-3-(3-chloro-2-methoxy-anilino)-6-methyl-2-[3-(tetrahydropyran-2-ylmethoxy)-4-pyridyl]-1,5,6,7-tetrahydropyrrolo[3,2-c]pyridin-4-one,
(*6R*)-3-(3-chloro-2-methoxy-anilino)-6-methyl-2-{3-[(*2S*)-tetrahydropyran-2-ylmethoxy]-4-pyridyl}-1,5,6,7-tetrahydropyrrolo[3,2-c]pyridin-4-one,
(*6R*)-3-(3-chloro-2-methoxy-anilino)-6-methyl-2-{3-[(*2R*)-tetrahydropyran-2-ylmethoxy]-4-pyridyl}-1,5,6,7-tetrahydropyrrolo[3,2-c]pyridin-4-one,
3-(3-chloro-2-methoxy-anilino)-7-methyl-2-[3-[[(*2S*)-tetrahydrofuran-2-yl]methoxy]-4-pyridyl]-1,5,6,7-tetrahydropyrrolo[3,2-c]pyridin-4-one,
(*7S*)-3-(3-chloro-2-methoxy-anilino)-7-methyl-2-[3-[[(*2S*)-tetrahydrofuran-2-yl]methoxy]-4-pyridyl]-1,5,6,7-tetrahydropyrrolo[3,2-c]pyridin-4-one,
(*7R*)-3-(3-chloro-2-methoxy-anilino)-7-methyl-2-[3-[[(*2S*)-tetrahydrofuran-2-yl]methoxy]-4-pyridyl]-1,5,6,7-tetrahydropyrrolo[3,2-c]pyridin-4-one,
3-(3-chloro-2-methoxy-anilino)-2-[3-(1,4-dioxan-2-ylmethoxy)-4-pyridyl]-7-methyl-1,5,6,7-tetrahydropyrrolo[3,2-c]pyridin-4-one,
(*7S*)-3-(3-chloro-2-methoxy-anilino)-2-{3-[(*2S*)-1,4-dioxan-2-ylmethoxy]-4-pyridyl}-7-methyl-1,5,6,7-tetrahydropyrrolo[3,2-c]pyridin-4-one,
(*7S*)-3-(3-chloro-2-methoxy-anilino)-2-{3-[(*2R*)-1,4-dioxan-2-ylmethoxy]-4-pyridyl}-7-methyl-1,5,6,7-tetrahydropyrrolo[3,2-c]pyridin-4-one,
(*7R*)-3-(3-chloro-2-methoxy-anilino)-2-{3-[(*2S*)-1,4-dioxan-2-ylmethoxy]-4-pyridyl}-7-methyl-1,5,6,7-tetrahydropyrrolo[3,2-c]pyridin-4-one,
(*7R*)-3-(3-chloro-2-methoxy-anilino)-2-{3-[(*2R*)-1,4-dioxan-2-ylmethoxy]-4-pyridyl}-7-methyl-1,5,6,7-tetrahydropyrrolo[3,2-c]pyridin-4-one,
3-(3-chloro-2-methoxy-anilino)-2-[3-[[(*2S*)-tetrahydrofuran-2-yl]methoxy]-4-pyridyl]-6-(trifluoromethyl)-1,5,6,7-tetrahydropyrrolo[3,2-c]pyridin-4-one,
(*6S*)-3-(3-chloro-2-methoxy-anilino)-2-[3-[[(*2S*)-tetrahydrofuran-2-yl]methoxy]-4-pyridyl]-6-(trifluoromethyl)-1,5,6,7-tetrahydropyrrolo[3,2-c]pyridin-4-one,
(*6R*)-3-(3-chloro-2-methoxy-anilino)-2-{3-[(1-methoxypropan-2-yl)oxy]-4-pyridyl}-6-methyl-1,5,6,7-tetrahydropyrrolo[3,2-c]pyridin-4-one,
(*6R*)-3-(3-chloro-2-methoxy-anilino)-2-(3-{[(*2S*)-1-methoxypropan-2-yl]oxy}-4-pyridyl)-6-methyl-1,5,6,7-tetrahydropyrrolo[3,2-c]pyridin-4-one,
(*6R*)-3-(3-chloro-2-methoxy-anilino)-2-(3-{[(*2R*)-1-methoxypropan-2-yl]oxy}-4-pyridyl)-6-methyl-1,5,6,7-tetrahydropyrrolo[3,2-c]pyridin-4-one,
3-(3-chloro-2-methoxy-anilino)-7-methyl-2-[3-(tetrahydropyran-2-ylmethoxy)-4-pyridyl]-1,5,6,7-tetrahydropyrrolo[3,2-c]pyridin-4-one,
(*7S*)-3-(3-chloro-2-methoxy-anilino)-7-methyl-2-{3-[(*2S*)-tetrahydropyran-2-ylmethoxy]-4-pyridyl}-1,5,6,7-tetrahydropyrrolo[3,2-c]pyridin-4-one,
(*7S*)-3-(3-chloro-2-methoxy-anilino)-7-methyl-2-{3-[(*2R*)-tetrahydropyran-2-ylmethoxy]-4-pyridyl}-1,5,6,7-tetrahydropyrrolo[3,2-c]pyridin-4-one,
(*7R*)-3-(3-chloro-2-methoxy-anilino)-7-methyl-2-{3-[(*2S*)-tetrahydropyran-2-ylmethoxy]-4-pyridyl}-1,5,6,7-tetrahydropyrrolo[3,2-c]pyridin-4-one,
(*7R*)-3-(3-chloro-2-methoxy-anilino)-7-methyl-2-{3-[(*2R*)-tetrahydropyran-2-ylmethoxy]-4-pyridyl}-1,5,6,7-tetrahydropyrrolo[3,2-c]pyridin-4-one,
(*5aR,8aS*)-3-(3-chloro-2-methoxyanilino)-2-{3-[(1-methoxypropan-2-yl)oxy]-4-pyridyl}-5,5a,6,7,8,8a-hexahydrocyclopenta[b]pyrrolo[2,3-d]pyridin-4(*1H*)-one,
(*5aR,8aS*)-3-(3-chloro-2-methoxyanilino)-2-(3-{[(*2S*)-1-methoxypropan-2-yl]oxy}-4-pyridyl)-5,5a,6,7,8,8a-hexahydrocyclopenta[b]pyrrolo[2,3-d]pyridin-4(*1H*)-one,
(*5aR,8aS*)-3-(3-chloro-2-methoxyanilino)-2-(3-{[(*2R*)-1-methoxypropan-2-yl]oxy}-4-pyridyl)-5,5a,6,7,8,8a-hexahydrocyclopenta[b]pyrrolo[2,3-d]pyridin-4(*1H*)-one,
(*5aR,8aS*)-3-(3-chloro-2-methoxyanilino)-2-[3-(1,4-dioxan-2-ylmethoxy)-4-pyridyl]-5,5a,6,7,8,8a-hexahydrocyclopenta[b]pyrrolo[2,3-d]pyridin-4(*1H*)-one,
(*5aR,8aS*)-3-(3-chloro-2-methoxyanilino)-2-{3-[(*2S*)-1,4-dioxan-2-ylmethoxy]-4-pyridyl}-5,5a,6,7,8,8a-hexahydrocyclopenta[b]pyrrolo[2,3-d]pyridin-4(*1H*)-one,
(*5aR,8aS*)-3-(3-chloro-2-methoxyanilino)-2-{3-[(*2R*)-1,4-dioxan-2-ylmethoxy]-4-pyridyl}-5,5a,6,7,8,8a-hexahydrocyclopenta[b]pyrrolo[2,3-d]pyridin-4(*1H*)-one,
(*5aR**,*8aS**)-3-(3-chloro-2-methoxyanilino)-2-(3-{[(2S)-oxolan-2-yl]methoxy}-4-pyridyl)-5,5a,6,7,8,8a-hexahydrocyclopenta[b]pyrrolo[2,3-d]pyridin-4(1*H*)-one,
(*5aR,8aS*)-3-(3-chloro-2-methoxyanilino)-2-(3-{[(*2S*)-oxolan-2-yl]methoxy}-4-pyridyl)-5,5a,6,7,8,8a-hexahydrocyclopenta[b]pyrrolo[2,3-d]pyridin-4(1*H*)-one,
3-(3-chloro-2-methyl-anilino)-7-methyl-2-(3-{[(*2S*)-oxolan-2-yl]methoxy}pyridin-4-yl)-1,5,6,7-tetrahydropyrrolo[3,2-c]pyridin-4-one,
(*7S*)-3-(3-chloro-2-methyl-anilino)-7-methyl-2-(3-{[(*2S*)-oxolan-2-yl]methoxy}pyridin-4-yl)-1,5,6,7-tetrahydropyrrolo[3,2-c]pyridin-4-one,
(*7R*)-3-(3-chloro-2-methyl-anilino)-7-methyl-2-(3-{[(*2S*)-oxolan-2-yl]methoxy}pyridin-4-yl)-1,5,6,7-tetrahydropyrrolo[3,2-c]pyridin-4-one,
3-(3-chloro-2-methyl-anilino)-2-[3-[(3,3-difluorotetrahydropyran-2-yl)methoxy]-4-pyridyl]-7-methyl-1,5,6,7-tetrahydropyrrolo[3,2-c]pyridin-4-one,
3-(3-chloro-2-methyl-anilino)-2-[3-[(5,5-dimethyl-1,4-dioxan-2-yl)methoxy]-4-pyridyl]-7-methyl-1,5,6,7-tetrahydropyrrolo[3,2-c]pyridin-4-one,
(6*R*)-3-[2-(2,2-difluoroethyl)-3-fluoroanilino]-6-methyl-2-(3-{[(*2S*)-oxolan-2-yl]methoxy}pyridin-4-yl)-1,5,6,*7-tetrahydro-4H-pyrrolo*[3,2-c]pyridin-4-one,
(*5aR,8aS*)-3-(3-chloro-2-methoxyanilino)-2-(3-{[(*2S*)-4-methylmorpholin-2-yl]methoxy}pyridin-4-yl)-5,5a,6,7,8,8a-hexahydrocyclopenta[b]pyrrolo[2,3-d]pyridin-4(*1H*)-one,
(*6R*)-3-[2-(2,2-difluoroethyl)-3-fluoroanilino]-2-[3-(2-methoxy-2-methylpropoxy)pyridin-4-yl]-6-methyl-1,5,6*,7-tetrahydro-4H-pyrrolo*[3,2-c]pyridin-4-one,
3-(3-chloro-2-methyl-anilino)-2-[3-(1,4-dioxan-2-ylmethoxy)-4-pyridyl]-7-methyl-1,5,6,7-tetrahydropyrrolo[3,2-c]pyridin-4-one,
(*7S*)-3-(3-chloro-2-methyl-anilino)-2-[3-[(*2S*)-1,4-dioxan-2-ylmethoxy]-4-pyridyl]-7-methyl-1,5,6,7-tetrahydropyrrolo[3,2-c]pyridin-4-one,
(*7R*)-3-(3-chloro-2-methyl-anilino)-2-[3-[(*2R*)-1,4-dioxan-2-ylmethoxy]-4-pyridyl]-7-methyl-1,5,6,7-tetrahydropyrrolo[3,2-c]pyridin-4-one,
(*7S*)-3-(3-chloro-2-methyl-anilino)-2-[3-[(*2R*)-1,4-dioxan-2-ylmethoxy]-4-pyridyl]-7-methyl-1,5,6,7-tetrahydropyrrolo[3,2-c]pyridin-4-one,
(*7R*)-3-(3-chloro-2-methyl-anilino)-2-[3-[(*2S*)-1,4-dioxan-2-ylmethoxy]-4-pyridyl]-7-methyl-1,5,6,7-tetrahydropyrrolo[3,2-c]pyridin-4-one,
(*6R*)-3-(3-chloro-2-methoxy-anilino)-2-[3-[(3,3-difluorotetrahydrofuran-2-yl)methoxy]-4-pyridyl]-6-methyl-1,5,6,7-tetrahydropyrrolo[3,2-c]pyridin-4-one,
(*6R*)-3-(3-chloro-2-methoxy-anilino)-2-(3-{[(*2S*)-3,3-difluorotetrahydrofuran-2-yl]methoxy}-4-pyridyl)-6-methyl-1,5,6,7-tetrahydropyrrolo[3,2-c]pyridin-4-one,
(*6R*)-3-(3-chloro-2-methoxy-anilino)-2-(3-{[(*2R*)-3,3-difluorotetrahydrofuran-2-yl]methoxy}-4-pyridyl)-6-methyl-1,5,6,7-tetrahydropyrrolo[3,2-c]pyridin-4-one,
(*6R*)-3-(3-chloro-2-methoxy-anilino)-2-[3-[(3,3-difluorotetrahydropyran-2-yl)methoxy]-4-pyridyl]-6-methyl-1,5,6,7-tetrahydropyrrolo[3,2-c]pyridin-4-one,
(*6R*)-3-(3-chloro-2-methoxy-anilino)-2-(3-{[(*2S*)-3,3-difluorotetrahydropyran-2-yl]methoxy}-4-pyridyl)-6-methyl-1,5,6,7-tetrahydropyrrolo[3,2-c]pyridin-4-one,
(*6R*)-3-(3-chloro-2-methoxy-anilino)-2-(3-{[(*2R*)-3,3-difluorotetrahydropyran-2-yl]methoxy}-4-pyridyl)-6-methyl-1,5,6,7-tetrahydropyrrolo[3,2-c]pyridin-4-one,
(*6R*)-3-(3-chloro-2-methoxy-anilino)-2-[3-[(5,5-dimethyl-1,4-dioxan-2-yl)methoxy]-4-pyridyl]-6-methyl-1,5,6,7-tetrahydropyrrolo[3,2-c]pyridin-4-one,
(*6R*)-3-(3-chloro-2-methoxy-anilino)-2(3-{[(*2S*)-5,5-dimethyl-1,4-dioxan-2-yl]methoxy}-4-pyridyl)-6-methyl-1,5,6,7-tetrahydropyrrolo[3,2-c]pyridin-4-one,
(*6R*)-3-(3-chloro-2-methoxy-anilino)-2(3-{[(*2R*)-5,5-dimethyl-1,4-dioxan-2-yl]methoxy}-4-pyridyl)-6-methyl-1,5,6,7-tetrahydropyrrolo[3,2-c]pyridin-4-one,
(*6R*)-3-(3-chloro-2-methoxy-anilino)-2-[3-(2-methoxypropoxy)-4-pyridyl]-6-methyl-1,5,6,7-tetrahydropyrrolo[3,2-c]pyridin-4-one,
(*6R*)-3-(3-chloro-2-methoxy-anilino)-2-{3-[(*2R*)-2-methoxypropoxy]-4-pyridyl}-6-methyl-1,5,6,7-tetrahydropyrrolo[3,2-c]pyridin-4-one,
(*6R*)-3-(3-chloro-2-methoxy-anilino)-2-{3-[(*2S*)-2-methoxypropoxy]-4-pyridyl}-6-methyl-1,5,6,7-tetrahydropyrrolo[3,2-c]pyridin-4-one,
(*6R*)-3-(3-chloro-2-methoxy-anilino)-6-methyl-2-[3-[(1-methyl-2-piperidyl)methoxy]-4-pyridyl]-1,5,6,7-tetrahydropyrrolo[3,2-c]pyridin-4-one,
(*6R*)-3-(3-chloro-2-methoxy-anilino)-6-methyl-2-(3-{[(*2S*)-1-methyl-2-piperidyl]methoxy}-4-pyridyl)-1,5,6,7-tetrahydropyrrolo[3,2-c]pyridin-4-one,
(*6R*)-3-(3-chloro-2-methoxy-anilino)-6-methyl-2-(3-{[(*2R*)-1-methyl-2-piperidyl]methoxy}-4-pyridyl)-1,5,6,7-tetrahydropyrrolo[3,2-c]pyridin-4-one,
(*6R*)-3-(3-chloro-2-methoxy-anilino)-6-methyl-2-[3-(2-tetrahydropyran-2-ylethoxy)-4-pyridyl]-1,5,6,7-tetrahydropyrrolo[3,2-c]pyridin-4-one,
(*6R*)-3-(3-chloro-2-methoxy-anilino)-6-methyl-2-(3-{2-[(*2S*)-tetrahydropyran-2-yl]ethoxy}-4-pyridyl)-1,5,6,7-tetrahydropyrrolo[3,2-c]pyridin-4-one,
(*6R*)-3-(3-chloro-2-methoxy-anilino)-6-methyl-2-(3-{2-[(*2R*)-tetrahydropyran-2-yl]ethoxy}-4-pyridyl)-1,5,6,7-tetrahydropyrrolo[3,2-c]pyridin-4-one,
(*7S*)-3-(3-chloro-2-methoxyanilino)-7-(2-fluoroethyl)-2-(3-{[(*3S*)-4-methylmorpholin-3-yl]methoxy}pyridin-4-yl)-1,5,6,*7-tetrahydro-4H-pyrrolo*[3,2-c]pyridin-4-one,
(*7R*)-3-(3-chloro-2-methoxyanilino)-7-(2-fluoroethyl)-2-(3-{[(*3S*)-4-methylmorpholin-3-yl]methoxy}pyridin-4-yl)-1,5,6,*7-tetrahydro-4H-pyrrolo*[3,2-c]pyridin-4-one,
(*6R*)-3-(3-chloro-2-ethylanilino)-6-methyl-2-(3-{[(*2S*)-4-methylmorpholin-2-yl]methoxy}pyridin-4-yl)-1,5,6,*7-tetrahydro-4H-pyrrolo*[3,2-c]pyridin-4-one,
(*6R*)-3-(3-chloro-2-methoxyanilino)-6-methyl-2-(3-{2-[(*2S*)-oxolan-2-yl]ethoxy}pyridin-4-yl)-1,5,6,7-*tetrahydro*-*4H-pyrrolo*[3,2-c]pyridin-4-one*,*
(*6R*)-3-(3-chloro-2-methoxyanilino)-6-methyl-2-(3-{[(*3S*)-4-methylmorpholin-3-yl]methoxy}pyridin-4-yl)-1,5,6,7-*tetrahydro-4H-pyrrolo*[3,2-c]pyridin-4-one,
(*7S*)-3-(3-chloro-2-methoxy-anilino)-2-(3-{[(*2S*)-5,5-dimethyl-1,4-dioxan-2-yl]methoxy}-4-pyridyl)-7-methyl-1,5,6,7-tetrahydropyrrolo[3,2-c]pyridin-4-one,
(*7S*)-3-(3-chloro-2-methoxy-anilino)-2-(3-{[(*2R*)-5,5-dimethyl-1,4-dioxan-2-yl]methoxy}-4-pyridyl)-7-methyl-1,5,6,7-tetrahydropyrrolo[3,2-c]pyridin-4-one,
(*7R*)-3-(3-chloro-2-methoxy-anilino)-2-(3-{[(*2S*)-5,5-dimethyl-1,4-dioxan-2-yl]methoxy}-4-pyridyl)-7-methyl-1,5,6,7-tetrahydropyrrolo[3,2-c]pyridin-4-one,
(*7R*)-3-(3-chloro-2-methoxy-anilino)-2-(3-{[(*2R*)-5,5-dimethyl-1,4-dioxan-2-yl]methoxy}-4-pyridyl)-7-methyl-1,5,6,7-tetrahydropyrrolo[3,2-c]pyridin-4-one,
(*7S*)-3-(3-chloro-2-methoxy-anilino)-2-(3-{[(*2S*)-tetrahydrofuran-2-yl]methoxy}-4-pyridyl)-7-(3,3,3-trifluoropropyl)-1,5,6,7-tetrahydropyrrolo[3,2-c]pyridin-4-one,
(*7R*)-3-(3-chloro-2-methoxy-anilino)-2-(3-{[(*2S*)-tetrahydrofuran-2-yl]methoxy}-4-pyridyl)-7-(3,3,3-trifluoropropyl)-1,5,6,7-tetrahydropyrrolo[3,2-c]pyridin-4-one,
(*7S*)-3-(3-chloro-2-methoxy-anilino)-7-(3,3-difluoroallyl)-2-(3-{[(*2S*)-tetrahydrofuran-2-yl]methoxy}-4-pyridyl)-1,5,6,7-tetrahydropyrrolo[3,2-c]pyridin-4-one,
(*7R*)-3-(3-chloro-2-methoxy-anilino)-7-(3,3-difluoroallyl)-2-(3-{[(*2S*)-tetrahydrofuran-2-yl]methoxy}-4-pyridyl)-1,5,6,7-tetrahydropyrrolo[3,2-c]pyridin-4-one,
(*6R*)-3-(3-chloro-2-methylanilino)-2-{3-[(1,4-dioxan-2-yl)methoxy]pyridin-4-yl}-6-methyl-1,5,6,*7-tetrahydro-4H-pyrrolo*[3,2-c]pyridin-4-one,
(*6R*)-3-(3-chloro-2-methylanilino)-2-(3-{[(*2S*)-1,4-dioxan-2-yl]methoxy}pyridin-4-yl)-6-methyl-1,5,6,7-*tetrahydro-4H-pyrrolo*[3,2-c]pyridin-4-one,
(*6R*)-3-(3-chloro-2-methylanilino)-6-methyl-2-(3-{[(*2S*)-oxolan-2-yl]methoxy}pyridin-4-yl)-1,5,6,7-*tetrahydro-4H-pyrrolo*[3,2-c]pyridin-4-one,
(*6R*)-3-(3-chloro-2-methylanilino)-2-{3-[(1-methoxypropan-2-yl)oxy]pyridin-4-yl}-6-methyl-1,5,6,7-*tetrahydro*-*4H-pyrrolo*[3,2-c]pyridin-4-one*,*
(*6R*)-3-(3-chloro-2-methylanilino)-6-methyl-2-(3-{[(*2R*)-4-methylmorpholin-2-yl]methoxy}pyridin-4-yl)-1,5,6*,7-tetrahydro-4H-pyrrolo*[3,2-c]pyridin-4-one*,*
(*6R*)-3-(3-chloro-2-methylanilino)-6-methyl-2-(3-{[(*2S*)-4-methylmorpholin-2-yl]methoxy}pyridin-4-yl)-1,5,6*,7-tetrahydro-4H-pyrrolo*[3,2-c]pyridin-4-one*,*
(*6R*)-3-[2-(2,2-difluoroethyl)-3-fluoro-anilino]-2-[3-(1,4-dioxan-2-ylmethoxy)-4-pyridyl]-6-methyl-1,5,6,7-tetrahydropyrrolo[3,2-c]pyridin-4-one,
(*6R*)-3-(3-chloro-2-ethylanilino)-6-methyl-2-(3-{[(*2S*)-oxolan-2-yl]methoxy}pyridin-4-yl)-1,5,6,7-*tetrahydro-4H-pyrrolo*[3,2-c]pyridin-4-one,
(*6R*)-3-(3-chloro-2-ethylanilino)-2-(3-{[(*2S*)-1,4-dioxan-2-yl]methoxy}pyridin-4-yl)-6-methyl-1,5,6,*7-tetrahydro-4H-pyrrolo*[3,2-c]pyridin-4-one,
3'-(3-chloro-2-methoxyanilino)-2'-(3-{[(*2S*)-1,4-dioxan-2-yl]methoxy}pyridin-4-yl)-1',7'-dihydrospiro[cyclopropane-1,6'-pyrrolo[3,2-c]pyridin]-4'(5'*H*)-one,
3'-(3-Chlor-2-methoxyanilino)-2'-(3-{[(*2S*)-4-Methylmorpholin-2-yl]methoxy}pyridin-4-yl)-1',7'-dihydrospiro[Cyclopropan-1,6'-pyrrolo[3,2-c]pyridin]-4'(5'H)-on,
3'-(3-chloro-2-methoxyanilino)-2'-(3-{[(*2S*)-oxolan-2-yl]methoxy}pyridin-4-yl)-1',7'-dihydrospiro[cyclopropane-1,6'-pyrrolo[3,2-c]pyridin]-4'(5'*H*)-one,
7-allyl-2-[3-(2-allyloxyethoxy)-4-pyridyl]-3-(3-chloro-2-methoxy-anilino)-1,5,6,7-tetrahydropyrrolo[3,2-c]pyridin-4-one,
(*7S*)-3-(3-chloro-2-methoxyanilino)-7-(2-hydroxyethyl)-2-(3-{[(*3S*)-4-methylmorpholin-3-yl]methoxy}pyridin-4-yl)-1,5,6,7-tetrahydro-4H-pyrrolo[3,2-c]pyridin-4-one,
(*7R*)-3-(3-chloro-2-methoxyanilino)-7-(2-hydroxyethyl)-2-(3-{[(*3S*)-4-methylmorpholin-3-yl]methoxy}pyridin-4-yl)-1,5,6,7-tetrahydro-4H-pyrrolo[3,2-c]pyridin-4-one,
3-(3-chloro-2-methyl-anilino)-7-methyl-2-[3-(tetrahydropyran-2-ylmethoxy)-4-pyridyl]-1,5,6,7-tetrahydropyrrolo[3,2-c]pyridin-4-one,
(*7S*)-3-(3-chloro-2-methyl-anilino)-7-methyl-2-(3-{[(*2S*)-tetrahydropyran-2-yl]methoxy}-4-pyridyl)-1,5,6,7-tetrahydropyrrolo[3,2-c]pyridin-4-one,
(*7R*)-3-(3-chloro-2-methyl-anilino)-7-methyl-2-(3-{[(*2S*)-tetrahydropyran-2-yl]methoxy}-4-pyridyl)-1,5,6,7-tetrahydropyrrolo[3,2-c]pyridin-4-one,
(*6R*)-3-(3-chloro-2-methoxy-anilino)-2-[3-(3-methoxybutoxy)-4-pyridyl]-6-methyl-1,5,6,7-tetrahydropyrrolo[3,2-c]pyridin-4-one,
(*6R*)-3-(3-chloro-2-methoxy-anilino)-2-{3-[(*3S*)-3-methoxybutoxy]-4-pyridyl}-6-methyl-1,5,6,7-tetrahydropyrrolo[3,2-c]pyridin-4-one,
(*6R*)-3-(3-chloro-2-methoxy-anilino)-2-{3-[(*3R*)-3-methoxybutoxy]-4-pyridyl}-6-methyl-1,5,6,7-tetrahydropyrrolo[3,2-c]pyridin-4-one,
3-(3-chloro-2-methoxy-anilino)-2-[3-(1,4-dioxan-2-ylmethoxy)-4-pyridyl]spiro[5,7-dihydro-1H-pyrrolo[3,2-c]pyridine-6,1'-cyclobutane]-4-one,
3-(3-chloro-2-methoxy-anilino)-2-{3-[(*2S*)-1,4-dioxan-2-ylmethoxy]-4-pyridyl}spiro[5,*7-dihydro-1H-pyrrolo*[3,2-c]pyridine-6,1'-cyclobutane]-4-one,
3-(3-chloro-2-methoxy-anilino)-2-{3-[(*2R*)-1,4-dioxan-2-ylmethoxy]-4-pyridyl}spiro[5,*7-dihydro-1H-pyrrolo*[3,2-c]pyridine-6,1'-cyclobutane]-4-one,
3'-(3-chloro-2-methoxyanilino)-2'-(3-{[(*2S*)-oxolan-2-yl]methoxy}pyridin-4-yl)-1',7'-dihydrospiro[cyclobutane-1,6'-pyrrolo[3,2-c]pyridin]-4'(5'*H*)-one,
3'-(3-chloro-2-methoxyanilino)-2'-(3-{[(2*S*)-oxan-2-yl]methoxy}pyridin-4-yl)-1',7'-dihydrospiro[cyclobutane-1,6'-pyrrolo[3,2-c]pyridin]-4'(5'*H*)-one,
3-(3-chloro-2-methoxy-anilino)-2-[3-(2-tetrahydropyran-2-ylethoxy)-4-pyridyl]spiro[5,7-*dihydro-1H-pyrrolo*[3,2-c]pyridine-6,1'-cyclobutane]-4-one*,*
3'-(3-chloro-2-methoxyanilino)-2'-{3-[(5,5-dimethyl-1,4-dioxan-2-yl)methoxy]pyridin-4-yl}-1',7'-dihydrospiro[cyclobutane-1,6'-pyrrolo[3,2-c]pyridin]-4'(5'*H*)-one,
3'-(3-chloro-2-methoxyanilino)-2'-(3-{[(*2R*)-5,5-dimethyl-1,4-dioxan-2-yl]methoxy}pyridin-4-yl)-1',7'-dihydrospiro[cyclobutane-1,6'-pyrrolo[3,2-c]pyridin]-4'(5'*H*)-one,
3'-(3-chloro-2-methoxyanilino)-2'-(3-{[(*2S*)-5,5-dimethyl-1,4-dioxan-2-yl]methoxy}pyridin-4-yl)-1',7'-dihydrospiro[cyclobutane-1,6'-pyrrolo[3,2-c]pyridin]-4'(5'*H*)-one,
3'-(3-chloro-2-methoxyanilino)-2'-{3-[(5,5-dimethyl-1,4-dioxan-2-yl)methoxy]pyridin-4-yl}-1',7'-dihydrospiro[cyclopropane-1,6'-pyrrolo[3,2-c]pyridin]-4'(5'*H*)-one,
3'-(3-chloro-2-methoxyanilino)-2'-(3-{[(*2R*)-5,5-dimethyl-1,4-dioxan-2-yl]methoxy}pyridin-4-yl)-1',7'-dihydrospiro[cyclopropane-1,6'-pyrrolo[3,2-c]pyridin]-4'(5'*H*)-one, and
3'-(3-chloro-2-methoxyanilino)-2'-(3-{[(2*S*)-5,5-dimethyl-1,4-dioxan-2-yl]methoxy}pyridin-4-yl)-1',7'-dihydrospiro[cyclopropane-1,6'-pyrrolo[3,2-c]pyridin]-4'(5'*H*)-one.

5. Eine Verbindung der allgemeinen Formel (I) nach einem der Ansprüche 1 bis 4 zur Verwendung bei der Behandlung oder Prophylaxe von Krankheiten.

6. Die Verbindung zur Verwendung gemäß Anspruch 5, wobei die Krankheiten hyperproliferative Krankheiten und/oder Störungen sind, die auf die Induktion von Zelltod ansprechen.

7. Die Verbindung zur Verwendung gemäß Anspruch 6, wobei die hyperproliferativen Krankheiten und/oder Störungen, die auf die Induktion des Zelltods ansprechen, hämatologische Tumore, solide Tumore und/oder Metastasen davon sind.

8. Die Verbindung zur Verwendung gemäß Anspruch 7, wobei der Tumor eine EGFR-Mutante und/oder Metastasen davon beherbergt.

9. Die Verbindung zur Verwendung gemäß Anspruch 7, wobei der Tumor Lungenkrebs ist, insbesondere Lungenkrebs, der eine mutierte EGFR mit Exon 20-Insertionsmutation aufweist, und/oder Metastasen davon.

10. Die Verbindung zur Verwendung gemäß Anspruch 7, wobei der Tumor Lungenkrebs ist, insbesondere Lungenkrebs, der eine EGFR-Mutante mit In-Frame-Deletionen in Exon 19 (wie EGFR E746_A750del) oder Punktmutationen in Exon 21 (z. B. L858R) aufweist, und/oder Metastasen davon.

11. Die Verbindung zur Verwendung gemäß Anspruch 7, wobei der Tumor Lungenkrebs ist, insbesondere Lungenkrebs, der eine EGFR-Mutation mit einer D770_N771insSVD C797S, E746_A750del C797S oder L858R C797S erworbenen Resistenzmutation aufweist, und/oder Metastasen davon.

12. Die Verbindung zur Verwendung gemäß Anspruch 7, wobei der Tumor Lungenkrebs ist, insbesondere Lungenkrebs, der eine mutierte ERBB2 mit Exon 20-Insertionsmutationen (wie ERBB2 A775_G776insYVMA) aufweist, und/oder Metastasen davon.

13. Eine pharmazeutische Zusammensetzung, die mindestens eine Verbindung der allgemeinen Formel (I) nach einem der Ansprüche 1 bis 4, zusammen mit mindestens einem pharmazeutisch akzeptablen Hilfsstoff, umfasst.

14. Die Zusammensetzung gemäß Anspruch 13 zur Behandlung von hämatologischen Tumoren, soliden Tumoren und/oder Metastasen davon.

15. Eine Kombination, die einen oder mehrere erste Wirkstoffe, ausgewählt aus einer Verbindung der allgemeinen Formel (I) gemäß einem der Ansprüche 1 bis 4, und einen oder mehrere zweite Wirkstoffe, ausgewählt aus chemotherapeutischen Anti-Krebsmitteln und zielspezifischen Anti-Krebsmitteln, umfasst.

## Revendications

1. Composé de formule (I) 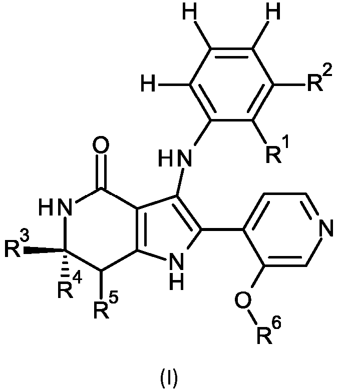 dans lequel :
R¹ représente méthyle, éthyle, trifluorométhyle, 2,2-difluoroéthyle, cyano, chloro, bromo, méthoxy ou difluorométhoxy ;
R² représente méthyle, éthyle, fluoro, chloro ou bromo ;
R³ représente hydrogène, méthyle ou trifluorométhyle ;
R⁴ représente hydrogène ; ou
R³ et R⁴ forment, avec l'atome de carbone auquel ils sont attachés, un cycle cycloalkyle de 3 à 5 chaînons ;
R⁵ représente hydrogène, un alkyle en C₁-C₄ ou un alcényle en C₂-C₃, dans lequel lesdits groupes alkyles sont éventuellement substitués une ou plusieurs fois, indépendamment l'un de l'autre, par fluoro, hydroxy ou méthoxy et lesdits groupes alcényles sont éventuellement substitués une ou plusieurs fois par fluoro ; ou
R³ et R⁵ forment, avec les atomes de carbone auxquels ils sont attachés, un cycle cycloalkyle de 4 à 6 chaînons ;
à condition qu'au moins l'un des R³ et R⁵ soit différent de hydrogène ;
R⁶ représente un alkyle en C₂-C₅, qui est substitué une fois par hydroxy, un alkoxy en C₁-C₄, ou un alcényloxy en C₂-C₃,
ou un groupe choisi dans le groupe : 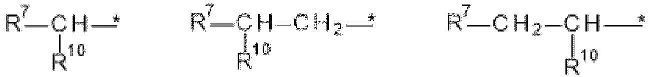
dans lequel * indique le point d'attache dudit groupe avec le reste de la molécule ;
R⁷ représente un groupe choisi dans le groupe : 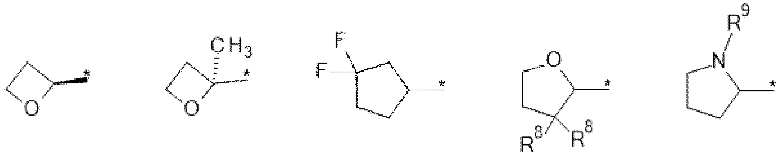 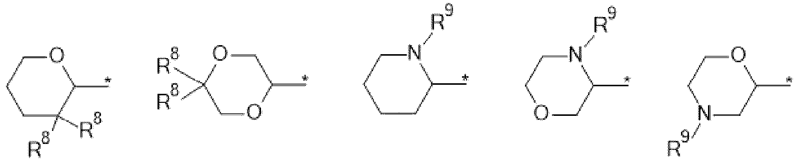 dans lequel * indique le point d'attache dudit groupe avec le reste de la molécule ;
R⁸ représente indépendamment hydrogène, méthyle ou fluoro à chaque occurrence ;
R⁹ représente un alkyle en C₁-C₃ ou un fluoroalkyle en C₂-C₃ ;
R¹⁰ représente hydrogène ou méthyle ;
ou un N-oxyde, un sel, un tautomère ou un stéréoisomère dudit composé, ou un sel dudit N-oxyde, tautomère ou stéréoisomère.

2. Composé de formule (I) selon la revendication 1, dans lequel :
R¹ représente méthyle, éthyle, trifluorométhyle, 2,2-difluoroéthyle, méthoxy ou difluorométhoxy ;
R² représente méthyle, fluoro, chloro ou bromo ;
R³ représente hydrogène, méthyle ou trifluorométhyle ;
R⁴ représente hydrogène ; ou
R³ et R⁴ forment, avec l'atome de carbone auquel ils sont attachés, un cycle cycloalkyle de 3 à 5 chaînons ;
R⁵ représente hydrogène, un alkyle en C₁-C₃ ou un alcényle en C₂-C₃, dans lequel lesdits groupes alkyles sont éventuellement substitués une, deux ou trois fois, indépendamment l'un de l'autre, par fluoro, hydroxy ou méthoxy et lesdits groupes alcényles sont éventuellement substitués une, deux ou trois fois par fluoro ; ou
R³ et R⁵ forment, avec les atomes de carbone auxquels ils sont attachés, un cycle cycloalkyle à 5 ou 6 chaînons ;
à condition qu'au moins l'un des R³ et R⁵ soit différent de hydrogène ;
R⁶ représente un alkyle en C₂-C₅, qui est substitué une fois par hydroxy, un alkoxy en C₁-C₃, ou un alcényloxy en C₂-C₃,
ou un groupe choisi dans le groupe : 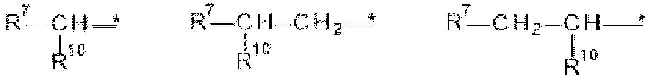
dans lequel * indique le point d'attache dudit groupe avec le reste de la molécule ;
R⁷ représente un groupe choisi dans le groupe : 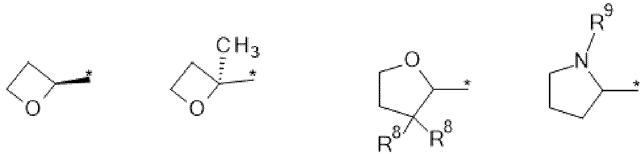 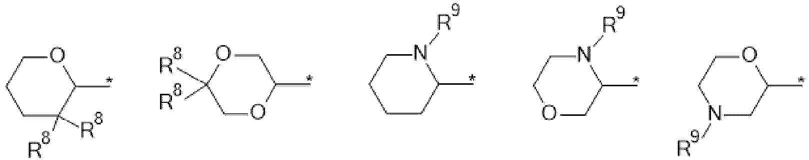 dans lequel * indique le point d'attache dudit groupe avec le reste de la molécule ;
R⁸ représente hydrogène, méthyle ou fluoro ;
R⁹ représente un alkyle en C₁-C₃ ou un fluoroalkyle en C₂-C₃ ;
R¹⁰ représente hydrogène ou méthyle ;
ou un N-oxyde, un sel, un tautomère ou un stéréoisomère dudit composé, ou un sel dudit N-oxyde, tautomère ou stéréoisomère.

3. Composé de formule (I) selon la revendication 1 ou 2, dans lequel :
R¹ représente méthyle, éthyle, 2,2-difluoroéthyle ou méthoxy ;
R² représente fluoro ou chloro ;
R³ représente hydrogène, méthyle ou trifluorométhyle ;
R⁴ représente hydrogène ; ou
R³ et R⁴ forment, avec l'atome de carbone auquel ils sont attachés, un cycle cycloalkyle à 3 ou 4 chaînons ;
R⁵ représente hydrogène, un alkyle en C₁-C₃, prop-2-én-1-yle ou 3,3-difluoroprop-2-én-1-yle,
dans lequel lesdits groupes alkyles sont éventuellement substitués une, deux ou trois fois par fluoro ou une fois par hydroxy ; ou
R³ et R⁵ forment, avec les atomes de carbone auxquels ils sont attachés, un cycle cycloalkyle à 5 chaînons ;
à condition qu'au moins l'un des R³ et R⁵ soit différent de hydrogène ;
R⁶ représente un alkyle en C₂-C₄ substitué une fois par hydroxy, méthoxy ou (prop-2-én-1-yl)oxy,
ou un groupe choisi dans le groupe : 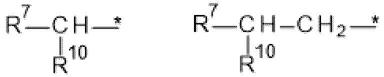
dans lequel * indique le point d'attache dudit groupe avec le reste de la molécule ;
R⁷ représente un groupe choisi dans le groupe : 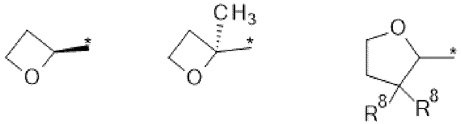 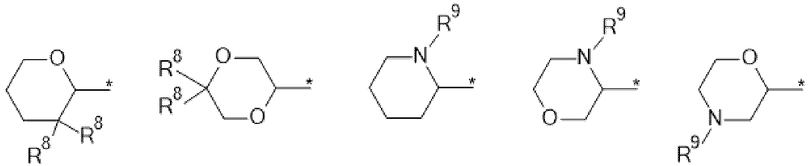 dans lequel * indique le point d'attache dudit groupe avec le reste de la molécule ;
R⁸ représente hydrogène, méthyle ou fluoro ;
R⁹ représente méthyle ou 2,2-difluoroéthyle ;
R¹⁰ représente hydrogène ;
ou un N-oxyde, un sel, un tautomère ou un stéréoisomère dudit composé, ou un sel dudit N-oxyde, tautomère ou stéréoisomère.

4. Composé de formule (I) selon l'une quelconque des revendications 1 à 3, qui est constitué par :
(*6R*)-3-(3-chloro-2-methoxyanilino)-2-[3-(2-methoxy-2-methylpropoxy)pyridin-4-yl]-6-methyl-1,5,6,7-tetrahydro-4H-pyrrolo[3,2-c]pyridin-4-one,
3-(3-chloro-2-methoxyanilino)-6-methyl-2-(3-{[(*2S*)-oxolan-2-yl]methoxy}pyridin-4-yl)-1,5,6,7-*tetrahydro-4H-pyrrolo*[3,2-c]pyridin-4-one,
(*6R*)-3-(3-chloro-2-methoxyanilino)-6-methyl-2-(3-{[(2S)-oxolan-2-yl]methoxy}pyridin-4-yl)-1,5,6,7-tetrahydro-4H-pyrrolo[3,2-c]pyridin-4-one,
(*6R*)-3-(3-chloro-2-methoxyanilino)-6-methyl-2-(3-{[(*2S*)-oxetan-2-yl]methoxy}pyridin-4-yl)-1,5,6,7-tetrahydro-4H-pyrrolo[3,2-c]pyridin-4-one,
3-(3-fluoro-2-methoxyanilino)-6-methyl-2-(3-{[(*2S*)-oxolan-2-yl]methoxy}pyridin-4-yl)-1,5,6,7-*tetrahydro-4H-pyrrolo*[3,2-c]pyridin-4-one,
(*6R*)-3-(3-fluoro-2-methoxyanilino)-6-methyl-2-(3-{[(*2S*)-oxolan-2-yl]methoxy}pyridin-4-yl)-1,5,6,*7-tetrahydro-4H-pyrrolo*[3,2-c]pyridin-4-one,
(*6R*)-3-(3-chloro-2-methoxyanilino)-6-methyl-2-(3-{[(*2S*)-4-methylmorpholin-2-yl]methoxy}pyridin-4-yl)-1,5,6,*7-tetrahydro-4H-pyrrolo*[3,2-c]pyridin-4-one,
(*6R*)-3-(3-chloro-2-methoxyanilino)-2-(3-{[(*2S*)-4-(2,2-difluoroethyl)morpholin-2-yl]methoxy}pyridin-4-yl)-6-methyl-1,5,6,*7-tetrahydro-4H-pyrrolo*[3,2-c]pyridin-4-one,
3-(3-chloro-2-methoxyanilino)-7-(2-hydroxyethyl)-2-[3-(2-methoxy-2-methylpropoxy)pyridin-4-yl]-1,5,6,7-tetrahydro-4H-pyrrolo[3,2-c]pyridin-4-one,
(*7R*)-3-(3-chloro-2-methoxyanilino)-7-(2-hydroxyethyl)-2-[3-(2-methoxy-2-methylpropoxy)pyridin-4-yl]-1,5,6,*7-tetrahydro-4H-pyrrolo*[3,2-c]pyridin-4-one,
(*7S*)-3-(3-chloro-2-methoxyanilino)-7-(2-hydroxyethyl)-2-[3-(2-methoxy-2-methylpropoxy)pyridin-4-yl]-1,5,6,*7-tetrahydro-4H-pyrrolo*[3,2-c]pyridin-4-one,
3-(3-chloro-2-methoxyanilino)-7-(2-hydroxyethyl)-2-(3-{[(*2S*)-oxolan-2-yl]methoxy}pyridin-4-yl)-1,5,6,*7-tetrahydro-4H-pyrrolo*[3,2-c]pyridin-4-one,
(*7R*)-3-(3-chloro-2-methoxyanilino)-7-(2-hydroxyethyl)-2-(3-{[(*2S*)-oxolan-2-yl]methoxy}pyridin-4-yl)-1,5,6*,7-tetrahydro-4H-pyrrolo*[3,2-c]pyridin-4-one,
(*7S*)-3-(3-chloro-2-methoxyanilino)-7-(2-hydroxyethyl)-2-(3-{[(*2S*)-oxolan-2-yl]methoxy}pyridin-4-yl)-1,5,6*,7-tetrahydro-4H-pyrrolo*[3,2-c]pyridin-4-one,
3-(3-chloro-2-methoxyanilino)-7-(2-hydroxyethyl)-2-{3-[(2-methyloxetan-2-yl)methoxy]pyridin-4-yl}-1,5,6,7-*tetrahydro-4H-pyrrolo*[3,2-c]pyridin-4-one,
(*7S*)-3-(3-chloro-2-methoxyanilino)-7-(2-hydroxyethyl)-2-(3-{[(*2S*)-2-methyloxetan-2-yl]methoxy}pyridin-4-yl)-1,5,6,*7-tetrahydro-4H-pyrrolo*[3,2-c]pyridin-4-one,
(*7R*)-3-(3-chloro-2-methoxyanilino)-7-(2-hydroxyethyl)-2-(3-{[(*2S*)-2-methyloxetan-2-yl]methoxy}pyridin-4-yl)-1,5,6,*7*-*tetrahydro-4H-pyrrolo*[3,2-c]pyridin-4-one,
(*7S*)-3-(3-chloro-2-methoxyanilino)-7-(2-hydroxyethyl)-2-(3-{[(*2R*)-2-methyloxetan-2-yl]methoxy}pyridin-4-yl)-1,5,6,*7-tetrahydro-4H-pyrrolo*[3,2-c]pyridin-4-one,
(*7R*)-3-(3-chloro-2-methoxyanilino)-7-(2-hydroxyethyl)-2-(3-{[(*2R*)-2-methyloxetan-2-yl]methoxy}pyridin-4-yl)-1,5,6,*7-tetrahydro-4H-pyrrolo*[3,2-c]pyridin-4-one,
(*6R*)-3-(3-chloro-2-methoxy-anilino)-2-[3-(1,4-dioxan-2-ylmethoxy)-4-pyridyl]-6-methyl-1,5,6,7-tetrahydropyrrolo[3,2-c]pyridin-4-one,
(*6R*)-3-(3-chloro-2-methoxy-anilino)-2-{3-[(*2S*)-1,4-dioxan-2-ylmethoxy]-4-pyridyl}-6-methyl-1,5,6,7-tetrahydropyrrolo[3,2-c]pyridin-4-one,
(*6R*)-3-(3-chloro-2-methoxy-anilino)-2-{3-[(*2R*)-1,4-dioxan-2-ylmethoxy]-4-pyridyl}-6-methyl-1,5,6,7-tetrahydropyrrolo[3,2-c]pyridin-4-one,
(*6R*)-3-(3-chloro-2-methoxy-anilino)-6-methyl-2-[3-(tetrahydropyran-2-ylmethoxy)-4-pyridyl]-1,5,6,7-tetrahydropyrrolo[3,2-c]pyridin-4-one,
(*6R*)-3-(3-chloro-2-methoxy-anilino)-6-methyl-2-{3-[(*2S*)-tetrahydropyran-2-ylmethoxy]-4-pyridyl}-1,5,6,7-tetrahydropyrrolo[3,2-c]pyridin-4-one,
(*6R*)-3-(3-chloro-2-methoxy-anilino)-6-methyl-2-{3-[(*2R*)-tetrahydropyran-2-ylmethoxy]-4-pyridyl}-1,5,6,7-tetrahydropyrrolo[3,2-c]pyridin-4-one,
3-(3-chloro-2-methoxy-anilino)-7-methyl-2-[3-[[(*2S*)-tetrahydrofuran-2-yl]methoxy]-4-pyridyl]-1,5,6,7-tetrahydropyrrolo[3,2-c]pyridin-4-one,
(*7S*)-3-(3-chloro-2-methoxy-anilino)-7-methyl-2-[3-[[(*2S*)-tetrahydrofuran-2-yl]methoxy]-4-pyridyl]-1,5,6,7-tetrahydropyrrolo[3,2-c]pyridin-4-one,
(*7R*)-3-(3-chloro-2-methoxy-anilino)-7-methyl-2-[3-[[(*2S*)-tetrahydrofuran-2-yl]methoxy]-4-pyridyl]-1,5,6,7-tetrahydropyrrolo[3,2-c]pyridin-4-one,
3-(3-chloro-2-methoxy-anilino)-2-[3-(1,4-dioxan-2-ylmethoxy)-4-pyridyl]-7-methyl-1,5,6,7-tetrahydropyrrolo[3,2-c]pyridin-4-one,
(*7S*)-3-(3-chloro-2-methoxy-anilino)-2-{3-[(*2S*)-1,4-dioxan-2-ylmethoxy]-4-pyridyl}-7-methyl-1,5,6,7-tetrahydropyrrolo[3,2-c]pyridin-4-one,
(*7S*)-3-(3-chloro-2-methoxy-anilino)-2-{3-[(*2R*)-1,4-dioxan-2-ylmethoxy]-4-pyridyl}-7-methyl-1,5,6,7-tetrahydropyrrolo[3,2-c]pyridin-4-one,
(*7R*)-3-(3-chloro-2-methoxy-anilino)-2-{3-[(*2S*)-1,4-dioxan-2-ylmethoxy]-4-pyridyl}-7-methyl-1,5,6,7-tetrahydropyrrolo[3,2-c]pyridin-4-one,
(*7R*)-3-(3-chloro-2-methoxy-anilino)-2-{3-[(*2R*)-1,4-dioxan-2-ylmethoxy]-4-pyridyl}-7-methyl-1,5,6,7-tetrahydropyrrolo[3,2-c]pyridin-4-one,
3-(3-chloro-2-methoxy-anilino)-2-[3-[[(*2S*)-tetrahydrofuran-2-yl]methoxy]-4-pyridyl]-6-(trifluoromethyl)-1,5,6,7-tetrahydropyrrolo[3,2-c]pyridin-4-one,
(*6S*)-3-(3-chloro-2-methoxy-anilino)-2-[3-[[(*2S*)-tetrahydrofuran-2-yl]methoxy]-4-pyridyl]-6-(trifluoromethyl)-1,5,6,7-tetrahydropyrrolo[3,2-c]pyridin-4-one,
(*6R*)-3-(3-chloro-2-methoxy-anilino)-2-{3-[(1-methoxypropan-2-yl)oxy]-4-pyridyl}-6-methyl-1,5,6,7-tetrahydropyrrolo[3,2-c]pyridin-4-one,
(*6R*)-3-(3-chloro-2-methoxy-anilino)-2-(3-{[(*2S*)-1-methoxypropan-2-yl]oxy}-4-pyridyl)-6-methyl-1,5,6,7-tetrahydropyrrolo[3,2-c]pyridin-4-one,
(*6R*)-3-(3-chloro-2-methoxy-anilino)-2-(3-{[(*2R*)-1-methoxypropan-2-yl]oxy}-4-pyridyl)-6-methyl-1,5,6,7-tetrahydropyrrolo[3,2-c]pyridin-4-one,
3-(3-chloro-2-methoxy-anilino)-7-methyl-2-[3-(tetrahydropyran-2-ylmethoxy)-4-pyridyl]-1,5,6,7-tetrahydropyrrolo[3,2-c]pyridin-4-one,
(*7S*)-3-(3-chloro-2-methoxy-anilino)-7-methyl-2-{3-[(*2S*)-tetrahydropyran-2-ylmethoxy]-4-pyridyl}-1,5,6,7-tetrahydropyrrolo[3,2-c]pyridin-4-one,
(*7S*)-3-(3-chloro-2-methoxy-anilino)-7-methyl-2-{3-[(*2R*)-tetrahydropyran-2-ylmethoxy]-4-pyridyl}-1,5,6,7-tetrahydropyrrolo[3,2-c]pyridin-4-one,
(*7R*)-3-(3-chloro-2-methoxy-anilino)-7-methyl-2-{3-[(*2S*)-tetrahydropyran-2-ylmethoxy]-4-pyridyl}-1,5,6,7-tetrahydropyrrolo[3,2-c]pyridin-4-one,
(*7R*)-3-(3-chloro-2-methoxy-anilino)-7-methyl-2-{3-[(*2R*)-tetrahydropyran-2-ylmethoxy]-4-pyridyl}-1,5,6,7-tetrahydropyrrolo[3,2-c]pyridin-4-one,
(*5aR,8aS*)-3-(3-chloro-2-methoxyanilino)-2-{3-[(1-methoxypropan-2-yl)oxy]-4-pyridyl}-5,5a,6,7,8,8a-hexahydrocyclopenta[b]pyrrolo[2,3-d]pyridin-4(*1H*)-one,
(*5aR,8aS*)-3-(3-chloro-2-methoxyanilino)-2-(3-{[(*2S*)-1-methoxypropan-2-yl]oxy}-4-pyridyl)-5,5a,6,7,8,8a-hexahydrocyclopenta[b]pyrrolo[2,3-d]pyridin-4(*1H*)-one,
(*5aR,8aS*)-3-(3-chloro-2-methoxyanilino)-2-(3-{[(*2R*)-1-methoxypropan-2-yl]oxy}-4-pyridyl)-5,5a,6,7,8,8a-hexahydrocyclopenta[b]pyrrolo[2,3-d]pyridin-4(*1H*)-one,
(*5aR,8aS*)-3-(3-chloro-2-methoxyanilino)-2-[3-(1,4-dioxan-2-ylmethoxy)-4-pyridyl]-5,5a,6,7,8,8a-hexahydrocyclopenta[b]pyrrolo[2,3-d]pyridin-4(*1H*)-one,
(*5aR,8aS*)-3-(3-chloro-2-methoxyanilino)-2-{3-[(*2S*)-1,4-dioxan-2-ylmethoxy]-4-pyridyl}-5,5a,6,7,8,8a-hexahydrocyclopenta[b]pyrrolo[2,3-d]pyridin-4(*1H*)-one,
(*5aR,8aS*)-3-(3-chloro-2-methoxyanilino)-2-{3-[(*2R*)-1,4-dioxan-2-ylmethoxy]-4-pyridyl}-5,5a,6,7,8,8a-hexahydrocyclopenta[b]pyrrolo[2,3-d]pyridin-4(*1H*)-one,
(*5aR*,8aS**)-3-(3-chloro-2-methoxyanilino)-2-(3-{[(*2S*)-oxolan-2-yl]methoxy}-4-pyridyl)-5,5a,6,7,8,8a-hexahydrocyclopenta[b]pyrrolo[2,3-d]pyridin-4(1H)-one,
(*5aR,8aS*)-3-(3-chloro-2-methoxyanilino)-2-(3-{[(*2S*)-oxolan-2-yl]methoxy}-4-pyridyl)-5,5a,6,7,8,8a-hexahydrocyclopenta[b]pyrrolo[2,3-d]pyridin-4(1H)-one,
3-(3-chloro-2-methyl-anilino)-7-methyl-2-(3-{[(*2S*)-oxolan-2-yl]methoxy}pyridin-4-yl)-1,5,6,7-tetrahydropyrrolo[3,2-c]pyridin-4-one,
(*7S*)-3-(3-chloro-2-methyl-anilino)-7-methyl-2-(3-{[(*2S*)-oxolan-2-yl]methoxy}pyridin-4-yl)-1,5,6,7-tetrahydropyrrolo[3,2-c]pyridin-4-one,
(*7R*)-3-(3-chloro-2-methyl-anilino)-7-methyl-2-(3-{[(*2S*)-oxolan-2-yl]methoxy}pyridin-4-yl)-1,5,6,7-tetrahydropyrrolo[3,2-c]pyridin-4-one,
3-(3-chloro-2-methyl-anilino)-2-[3-[(3,3-difluorotetrahydropyran-2-yl)methoxy]-4-pyridyl]-7-methyl-1,5,6,7-tetrahydropyrrolo[3,2-c]pyridin-4-one,
3-(3-chloro-2-methyl-anilino)-2-[3-[(5,5-dimethyl-1,4-dioxan-2-yl)methoxy]-4-pyridyl]-7-methyl-1,5,6,7-tetrahydropyrrolo[3,2-c]pyridin-4-one,
(*6R*)-3-[2-(2,2-difluoroethyl)-3-fluoroanilino]-6-methyl-2-(3-{[(*2S*)-oxolan-2-yl]methoxy}pyridin-4-yl)-1,5,6,*7-tetrahydro-4H-pyrrolo*[3,2-c]pyridin-4-one,
(*5aR,8aS*)-3-(3-chloro-2-methoxyanilino)-2-(3-{[(*2S*)-4-methylmorpholin-2-yl]methoxy}pyridin-4-yl)-5,5a,6,7,8,8a-hexahydrocyclopenta[b]pyrrolo[2,3-d]pyridin-4(*1H*)-one,
(*6R*)-3-[2-(2,2-difluoroethyl)-3-fluoroanilino]-2-[3-(2-methoxy-2-methylpropoxy)pyridin-4-yl]-6-methyl-1,5,6,*7-tetrahydro-4H-pyrrolo*[3,2-c]pyridin-4-one,
3-(3-chloro-2-methyl-anilino)-2-[3-(1,4-dioxan-2-ylmethoxy)-4-pyridyl]-7-methyl-1,5,6,7-tetrahydropyrrolo[3,2-c]pyridin-4-one,
(*7S*)-3-(3-chloro-2-methyl-anilino)-2-[3-[(*2S*)-1,4-dioxan-2-ylmethoxy]-4-pyridyl]-7-methyl-1,5,6,7-tetrahydropyrrolo[3,2-c]pyridin-4-one,
(*7R*)-3-(3-chloro-2-methyl-anilino)-2-[3-[(*2R*)-1,4-dioxan-2-ylmethoxy]-4-pyridyl]-7-methyl-1,5,6,7-tetrahydropyrrolo[3,2-c]pyridin-4-one,
(*7S*)-3-(3-chloro-2-methyl-anilino)-2-[3-[(*2R*)-1,4-dioxan-2-ylmethoxy]-4-pyridyl]-7-methyl-1,5,6,7-tetrahydropyrrolo[3,2-c]pyridin-4-one,
(*7R*)-3-(3-chloro-2-methyl-anilino)-2-[3-[(*2S*)-1,4-dioxan-2-ylmethoxy]-4-pyridyl]-7-methyl-1,5,6,7-tetrahydropyrrolo[3,2-c]pyridin-4-one,
(*6R*)-3-(3-chloro-2-methoxy-anilino)-2-[3-[(3,3-difluorotetrahydrofuran-2-yl)methoxy]-4-pyridyl]-6-methyl-1,5,6,7-tetrahydropyrrolo[3,2-c]pyridin-4-one,
(*6R*)-3-(3-chloro-2-methoxy-anilino)-2-(3-{[(*2S*)-3,3-difluorotetrahydrofuran-2-yl]methoxy}-4-pyridyl)-6-methyl-1,5,6,7-tetrahydropyrrolo[3,2-c]pyridin-4-one,
(*6R*)-3-(3-chloro-2-methoxy-anilino)-2-(3-{[(*2R*)-3,3-difluorotetrahydrofuran-2-yl]methoxy}-4-pyridyl)-6-methyl-1,5,6,7-tetrahydropyrrolo[3,2-c]pyridin-4-one,
(*6R*)-3-(3-chloro-2-methoxy-anilino)-2-[3-[(3,3-difluorotetrahydropyran-2-yl)methoxy]-4-pyridyl]-6-methyl-1,5,6,7-tetrahydropyrrolo[3,2-c]pyridin-4-one,
(*6R*)-3-(3-chloro-2-methoxy-anilino)-2-(3-{[(*2S*)-3,3-difluorotetrahydropyran-2-yl]methoxy}-4-pyridyl)-6-methyl-1,5,6,7-tetrahydropyrrolo[3,2-c]pyridin-4-one,
(*6R*)-3-(3-chloro-2-methoxy-anilino)-2-(3-{[(*2R*)-3,3-difluorotetrahydropyran-2-yl]methoxy}-4-pyridyl)-6-methyl-1,5,6,7-tetrahydropyrrolo[3,2-c]pyridin-4-one,
(*6R*)-3-(3-chloro-2-methoxy-anilino)-2-[3-[(5,5-dimethyl-1,4-dioxan-2-yl)methoxy]-4-pyridyl]-6-methyl-1,5,6,7-tetrahydropyrrolo[3,2-c]pyridin-4-one,
(*6R*)-3-(3-chloro-2-methoxy-anilino)-2(3-{[(*2S*)-5,5-dimethyl-1,4-dioxan-2-yl]methoxy}-4-pyridyl)-6-methyl-1,5,6,7-tetrahydropyrrolo[3,2-c]pyridin-4-one,
(*6R*)-3-(3-chloro-2-methoxy-anilino)-2(3-{[(*2R*)-5,5-dimethyl-1,4-dioxan-2-yl]methoxy}-4-pyridyl)-6-methyl-1,5,6,7-tetrahydropyrrolo[3,2-c]pyridin-4-one,
(*6R*)-3-(3-chloro-2-methoxy-anilino)-2-[3-(2-methoxypropoxy)-4-pyridyl]-6-methyl-1,5,6,7-tetrahydropyrrolo[3,2-c]pyridin-4-one,
(*6R*)-3-(3-chloro-2-methoxy-anilino)-2-{3-[(*2R*)-2-methoxypropoxy]-4-pyridyl}-6-methyl-1,5,6,7-tetrahydropyrrolo[3,2-c]pyridin-4-one,
(*6R*)-3-(3-chloro-2-methoxy-anilino)-2-{3-[(*2S*)-2-methoxypropoxy]-4-pyridyl}-6-methyl-1,5,6,7-tetrahydropyrrolo[3,2-c]pyridin-4-one,
(*6R*)-3-(3-chloro-2-methoxy-anilino)-6-methyl-2-[3-[(1-methyl-2-piperidyl)methoxy]-4-pyridyl]-1,5,6,7-tetrahydropyrrolo[3,2-c]pyridin-4-one,
(*6R*)-3-(3-chloro-2-methoxy-anilino)-6-methyl-2-(3-{[(*2S*)-1-methyl-2-piperidyl]methoxy}-4-pyridyl)-1,5,6,7-tetrahydropyrrolo[3,2-c]pyridin-4-one,
(*6R*)-3-(3-chloro-2-methoxy-anilino)-6-methyl-2-(3-{[(*2R*)-1-methyl-2-piperidyl]methoxy}-4-pyridyl)-1,5,6,7-tetrahydropyrrolo[3,2-c]pyridin-4-one,
(*6R*)-3-(3-chloro-2-methoxy-anilino)-6-methyl-2-[3-(2-tetrahydropyran-2-ylethoxy)-4-pyridyl]-1,5,6,7-tetrahydropyrrolo[3,2-c]pyridin-4-one,
(*6R*)-3-(3-chloro-2-methoxy-anilino)-6-methyl-2-(3-{2-[(*2S*)-tetrahydropyran-2-yl]ethoxy}-4-pyridyl)-1,5,6,7-tetrahydropyrrolo[3,2-c]pyridin-4-one,
(*6R*)-3-(3-chloro-2-methoxy-anilino)-6-methyl-2-(3-{2-[(*2R*)-tetrahydropyran-2-yl]ethoxy}-4-pyridyl)-1,5,6,7-tetrahydropyrrolo[3,2-c]pyridin-4-one,
(*7S*)-3-(3-chloro-2-methoxyanilino)-7-(2-fluoroethyl)-2-(3-{[(*3S*)-4-methylmorpholin-3-yl]methoxy}pyridin-4-yl)-1,5,6,*7-tetrahydro-4H-pyrrolo*[3,2-c]pyridin-4-one,
(*7R*)-3-(3-chloro-2-methoxyanilino)-7-(2-fluoroethyl)-2-(3-{[(*3S*)-4-methylmorpholin-3-yl]methoxy}pyridin-4-yl)-1,5,6,*7-tetrahydro-4H-pyrrolo*[3,2-c]pyridin-4-one,
(*6R*)-3-(3-chloro-2-ethylanilino)-6-methyl-2-(3-{[(*2S*)-4-methylmorpholin-2-yl]methoxy}pyridin-4-yl)-1,5,6*,7-tetrahydro-4H-pyrrolo*[3,2-c]pyridin-4-one,
(*6R*)-3-(3-chloro-2-methoxyanilino)-6-methyl-2-(3-{2-[(*2S*)-oxolan-2-yl]ethoxy}pyridin-4-yl)-1,5,6,*7-tetrahydro-4H-pyrrolo*[3,2-c]pyridin-4-one,
(*6R*)-3-(3-chloro-2-methoxyanilino)-6-methyl-2-(3-{[(*3S*)-4-methylmorpholin-3-yl]methoxy}pyridin-4-yl)-1,5,6,*7-tetrahydro-4H-pyrrolo*[3,2-c]pyridin-4-one,
(*7S*)-3-(3-chloro-2-methoxy-anilino)-2-(3-{[(*2S*)-5,5-dimethyl-1,4-dioxan-2-yl]methoxy}-4-pyridyl)-7-methyl-1,5,6,7-tetrahydropyrrolo[3,2-c]pyridin-4-one,
(*7S*)-3-(3-chloro-2-methoxy-anilino)-2-(3-{[(*2R*)-5,5-dimethyl-1,4-dioxan-2-yl]methoxy}-4-pyridyl)-7-methyl-1,5,6,7-tetrahydropyrrolo[3,2-c]pyridin-4-one,
(*7R*)-3-(3-chloro-2-methoxy-anilino)-2-(3-{[(*2S*)-5,5-dimethyl-1,4-dioxan-2-yl]methoxy}-4-pyridyl)-7-methyl-1,5,6,7-tetrahydropyrrolo[3,2-c]pyridin-4-one,
(*7R*)-3-(3-chloro-2-methoxy-anilino)-2-(3-{[(*2R*)-5,5-dimethyl-1,4-dioxan-2-yl]methoxy}-4-pyridyl)-7-methyl-1,5,6,7-tetrahydropyrrolo[3,2-c]pyridin-4-one,
(*7S*)-3-(3-chloro-2-methoxy-anilino)-2-(3-{[(*2S*)-tetrahydrofuran-2-yl]methoxy}-4-pyridyl)-7-(3,3,3-trifluoropropyl)-1,5,6,7-tetrahydropyrrolo[3,2-c]pyridin-4-one,
(*7R*)-3-(3-chloro-2-methoxy-anilino)-2-(3-{[(*2S*)-tetrahydrofuran-2-yl]methoxy}-4-pyridyl)-7-(3,3,3-trifluoropropyl)-1,5,6,7-tetrahydropyrrolo[3,2-c]pyridin-4-one,
(*7S*)-3-(3-chloro-2-methoxy-anilino)-7-(3,3-difluoroallyl)-2-(3-{[(*2S*)-tetrahydrofuran-2-yl]methoxy}-4-pyridyl)-1,5,6,7-tetrahydropyrrolo[3,2-c]pyridin-4-one,
(*7R*)-3-(3-chloro-2-methoxy-anilino)-7-(3,3-difluoroallyl)-2-(3-{[(*2S*)-tetrahydrofuran-2-yl]methoxy}-4-pyridyl)-1,5,6,7-tetrahydropyrrolo[3,2-c]pyridin-4-one,
(*6R*)-3-(3-chloro-2-methylanilino)-2-{3-[(1,4-dioxan-2-yl)methoxy]pyridin-4-yl}-6-methyl-1,5,6,*7-tetrahydro-4H-pyrrolo*[3,2-c]pyridin-4-one,
(*6R*)-3-(3-chloro-2-methylanilino)-2-(3-{[(*2S*)-1,4-dioxan-2-yl]methoxy}pyridin-4-yl)-6-methyl-1,5,6,*7-tetrahydro-4H-pyrrolo*[3,2-c]pyridin-4-one,
(*6R*)-3-(3-chloro-2-methylanilino)-6-methyl-2-(3-{[(*2S*)-oxolan-2-yl]methoxy}pyridin-4-yl)-1,5,6,*7-tetrahydro-4H-pyrrolo*[3,2-c]pyridin-4-one,
(*6R*)-3-(3-chloro-2-methylanilino)-2-{3-[(1-methoxypropan-2-yl)oxy]pyridin-4-yl}-6-methyl-1,5,6,*7-tetrahydro-4H-pyrrolo*[3,2-c]pyridin-4-one,
(*6R*)-3-(3-chloro-2-methylanilino)-6-methyl-2-(3-{[(*2R*)-4-methylmorpholin-2-yl]methoxy}pyridin-4-yl)-1,5,6,*7-tetrahydro-4H-pyrrolo*[3,2-c]pyridin-4-one,
(*6R*)-3-(3-chloro-2-methylanilino)-6-methyl-2-(3-{[(*2S*)-4-methylmorpholin-2-yl]methoxy}pyridin-4-yl)-1,5,6,*7-tetrahydro-4H-pyrrolo*[3,2-c]pyridin-4-one,
(*6R*)-3-[2-(2,2-difluoroethyl)-3-fluoro-anilino]-2-[3-(1,4-dioxan-2-ylmethoxy)-4-pyridyl]-6-methyl-1,5,6,7-tetrahydropyrrolo[3,2-c]pyridin-4-one,
(*6R*)-3-(3-chloro-2-ethylanilino)-6-methyl-2-(3-{[(*2S*)-oxolan-2-yl]methoxy}pyridin-4-yl)-1,5,6,7-*tetrahydro-4H-pyrrolo*[3,2-c]pyridin-4-one,
(*6R*)-3-(3-chloro-2-ethylanilino)-2-(3-{[(*2S*)-1,4-dioxan-2-yl]methoxy}pyridin-4-yl)-6-methyl-1,5,6,*7-tetrahydro-4H-pyrrolo*[3,2-c]pyridin-4-one,
3'-(3-chloro-2-methoxyanilino)-2'-(3-{[(*2S*)-1,4-dioxan-2-yl]methoxy}pyridin-4-yl)-1',7'-dihydrospiro[cyclopropane-1,6'-pyrrolo[3,2-c]pyridin]-4'(5'*H*)-one,
3'-(3-chloro-2-méthoxyanilino)-2'-(3-{[(*2S*)-4-méthylmorpholine-2-yl]méthoxy}pyridine-4-yl)-1',7'-dihydrospiro[cyclopropane-1,6'-pyrrolo[3,2-c]pyridine]-4'(5'H)-one,
3'-(3-chloro-2-methoxyanilino)-2'-(3-{[(*2S*)-oxolan-2-yl]methoxy}pyridin-4-yl)-1',7'-dihydrospiro[cyclopropane-1,6'-pyrrolo[3,2-c]pyridin]-4'(5'*H*)-one,
7-allyl-2-[3-(2-allyloxyethoxy)-4-pyridyl]-3-(3-chloro-2-methoxy-anilino)-1,5,6,7-tetrahydropyrrolo[3,2-c]pyridin-4-one,
(*7S*)-3-(3-chloro-2-methoxyanilino)-7-(2-hydroxyethyl)-2-(3-{[(*3S*)-4-methylmorpholin-3-yl]methoxy}pyridin-4-yl)-1,5,6,7-tetrahydro-4H-pyrrolo[3,2-c]pyridin-4-one,
(*7R*)-3-(3-chloro-2-methoxyanilino)-7-(2-hydroxyethyl)-2-(3-{[(*3S*)-4-methylmorpholin-3-yl]methoxy}pyridin-4-yl)-1,5,6,7-tetrahydro-4H-pyrrolo[3,2-c]pyridin-4-one,
3-(3-chloro-2-methyl-anilino)-7-methyl-2-[3-(tetrahydropyran-2-ylmethoxy)-4-pyridyl]-1,5,6,7-tetrahydropyrrolo[3,2-c]pyridin-4-one,
(*7S*)-3-(3-chloro-2-methyl-anilino)-7-methyl-2-(3-{[(*2S*)-tetrahydropyran-2-yl]methoxy}-4-pyridyl)-1,5,6,7-tetrahydropyrrolo[3,2-c]pyridin-4-one,
(*7R*)-3-(3-chloro-2-methyl-anilino)-7-methyl-2-(3-{[(*2S*)-tetrahydropyran-2-yl]methoxy}-4-pyridyl)-1,5,6,7-tetrahydropyrrolo[3,2-c]pyridin-4-one,
(*6R*)-3-(3-chloro-2-methoxy-anilino)-2-[3-(3-methoxybutoxy)-4-pyridyl]-6-methyl-1,5,6,7-tetrahydropyrrolo[3,2-c]pyridin-4-one,
(*6R*)-3-(3-chloro-2-methoxy-anilino)-2-{3-[(*3S*)-3-methoxybutoxy]-4-pyridyl}-6-methyl-1,5,6,7-tetrahydropyrrolo[3,2-c]pyridin-4-one,
(*6R*)-3-(3-chloro-2-methoxy-anilino)-2-{3-[(*3R*)-3-methoxybutoxy]-4-pyridyl}-6-methyl-1,5,6,7-tetrahydropyrrolo[3,2-c]pyridin-4-one,
3-(3-chloro-2-methoxy-anilino)-2-[3-(1,4-dioxan-2-ylmethoxy)-4-pyridyl]spiro[5,7-dihydro-1H-pyrrolo[3,2-c]pyridine-6,1'-cyclobutane]-4-one,
3-(3-chloro-2-methoxy-anilino)-2-{3-[(*2S*)-1,4-dioxan-2-ylmethoxy]-4-pyridyl}spiro[5,*7-dihydro-1H-pyrrolo*[3,2-c]pyridine-6,1'-cyclobutane]-4-one,
3-(3-chloro-2-methoxy-anilino)-2-{3-[(*2R*)-1,4-dioxan-2-ylmethoxy]-4-pyridyl}spiro[5,*7-dihydro-1H-pyrrolo*[3,2-c]pyridine-6,1'-cyclobutane]-4-one,
3'-(3-chloro-2-methoxyanilino)-2'-(3-{[(*2S*)-oxolan-2-yl]methoxy}pyridin-4-yl)-1',7'-dihydrospiro[cyclobutane-1,6'-pyrrolo[3,2-c]pyridin]-4'(5'*H*)-one,
3'-(3-chloro-2-methoxyanilino)-2'-(3-{[(2*S*)-oxan-2-yl]methoxy}pyridin-4-yl)-1',7'-dihydrospiro[cyclobutane-1,6'-pyrrolo[3,2-c]pyridin]-4'(5'*H*)-one,
3-(3-chloro-2-methoxy-anilino)-2-[3-(2-tetrahydropyran-2-ylethoxy)-4-pyridyl]spiro[5,7-*dihydro-1H-pyrrolo*[3,2-c]pyridine-6,1'-cyclobutane]-4-one,
3'-(3-chloro-2-methoxyanilino)-2'-{3-[(5,5-dimethyl-1,4-dioxan-2-yl)methoxy]pyridin-4-yl}-1',7'-dihydrospiro[cyclobutane-1,6'-pyrrolo[3,2-c]pyridin]-4'(5'*H*)-one,
3'-(3-chloro-2-methoxyanilino)-2'-(3-{[(*2R*)-5,5-dimethyl-1,4-dioxan-2-yl]methoxy}pyridin-4-yl)-1',7'-dihydrospiro[cyclobutane-1,6'-pyrrolo[3,2-c]pyridin]-4'(5'*H*)-one,
3'-(3-chloro-2-methoxyanilino)-2'-(3-{[(*2S*)-5,5-dimethyl-1,4-dioxan-2-yl]methoxy}pyridin-4-yl)-1',7'-dihydrospiro[cyclobutane-1,6'-pyrrolo[3,2-c]pyridin]-4'(5'*H*)-one,
3'-(3-chloro-2-methoxyanilino)-2'-{3-[(5,5-dimethyl-1,4-dioxan-2-yl)methoxy]pyridin-4-yl}-1',7'-dihydrospiro[cyclopropane-1,6'-pyrrolo[3,2-c]pyridin]-4'(5'*H*)-one,
3'-(3-chloro-2-methoxyanilino)-2'-(3-{[(*2R*)-5,5-dimethyl-1,4-dioxan-2-yl]methoxy}pyridin-4-yl)-1',7'-dihydrospiro[cyclopropane-1,6'-pyrrolo[3,2-c]pyridin]-4'(5'*H*)-one, and
3'-(3-chloro-2-methoxyanilino)-2'-(3-{[(*2S*)-5,5-dimethyl-1,4-dioxan-2-yl]methoxy}pyridin-4-yl)-1',7'-dihydrospiro[cyclopropane-1,6'-pyrrolo[3,2-c]pyridin]-4'(5'*H*)-one.

5. Composé de formule générale (I) selon l'une quelconque des revendications 1 à 4 pour utilisation dans le traitement ou la prophylaxie de maladies.

6. Composé pour utilisation selon la revendication 5, dans lequel les maladies sont des maladies hyperprolifératives et/ou des troubles répondant à l'induction de la mort cellulaire.

7. Composé pour utilisation selon la revendication 6, dans lequel les maladies hyperprolifératives et/ou les troubles répondant à l'induction de la mort cellulaire sont des tumeurs hématologiques, des tumeurs solides et/ou leurs métastases.

8. Composé pour utilisation selon la revendication 7, dans lequel la tumeur héberge un EGFR mutant et/ou ses métastases.

9. Composé pour utilisation selon la revendication 7, dans lequel la tumeur est un cancer du poumon, en particulier un cancer du poumon hébergeant un EGFR mutant avec une mutation d'insertion de l'exon 20, et/ou ses métastases.

10. Composé pour utilisation selon la revendication 7, dans lequel la tumeur est un cancer du poumon, en particulier un cancer du poumon hébergeant un EGFR mutant avec des délétions en phase dans l'exon 19 (tel que EGFR E746_A750del) ou des mutations ponctuelles dans l'exon 21 (par exemple L858R), et/ou ses métastases.

11. Composé pour utilisation selon la revendication 7, dans lequel la tumeur est un cancer du poumon, en particulier un cancer du poumon hébergeant un EGFR mutant avec une mutation de résistance acquise D770_N771insSVD C797S, E746_A750del C797S, ou L858R C797S, et/ou ses métastases.

12. Composé pour utilisation selon la revendication 7, dans lequel la tumeur est un cancer du poumon, en particulier un cancer du poumon hébergeant un ERBB2 mutant avec des mutations d'insertion de l'exon 20 (tel que ERBB2 A775_G776insYVMA), et/ou ses métastases.

13. Composition pharmaceutique comprenant au moins un composé de formule générale (I) selon l'une quelconque des revendications 1 à 4, ainsi qu'au moins un auxiliaire pharmaceutiquement acceptable.

14. Composition selon la revendication 13 pour le traitement des tumeurs hématologiques, des tumeurs solides et/ou de leurs métastases.

15. Combinaison comprenant un ou plusieurs premiers principes actifs choisis parmi un composé de formule générale (I) selon l'une quelconque des revendications 1 à 4, et un ou plusieurs seconds principes actifs choisis parmi les agents chimiothérapeutiques anticancéreux et les agents anticancéreux à cible spécifique.
